# EUROPEAN PATENT APPLICATION

(11) **EP 1 243 596 A2**
(43) Date of publication of application: **25.09.2002**
(21) Application number: 02006616.3
(22) Date of filing: 25.03.2002
(51) Int. Cl.: C07K 14/71, C12N 15/12, G01N 33/53

(54) **Catalytic domains of the human hepatocyte growth factor receptor tyrosine kinase and methods for identification of inhibitors thereof**

(30) Priority: 23.03.2001 US 277968 P
(71) Applicant: Agouron Pharmaceuticals, Inc., La Jolla, CA 92037 (US)
(72) Inventor: Mroczkowski, Barbara, Encinitas, CA 92024 (US); Hickey, Michael, San Diego, CA 92126 (US); McTigue, Michele Ann, Encinitas, CA 92024 (US); Murray, Brion William, San Diego, CA 92106 (US); Parge, Hans, San Diego, CA 92103 (US); Sarup, Jay, San Diego, CA 92122 (US); Zhu, Jeff, Carlsbad, CA 92009 (US)
(74) Representative: Keller, Günter, Dr.

(57) **Abstract**

The identification, isolation, purification, and characterization of the catalytic domain of the human hepatocyte growth factor receptor kinase (hHGFR) are described. A crystal structure of the hHGFR kinase domain is reported herein. This structure provides a three-dimensional description of the binding site of the hHGFR for structure-based design of small molecule inhibitors thereof as therapeutic agents. The kinase domain of human HGFR and its associated crystal structure is described for use in the discovery, identification and characeterization of modulators of human HGFR.

SEQUENCE LISTING

<110> Agouron, Pharmaceuticals, Inc.

<120> Catalytic Domains Of The Human Hepatocyte Growth Factor Receptor Tyrosine
Kinase, And Materials And Methods For Identification Of Inhibitors Thereof

<130> 0128-US

<140> 02.006616.3
<141> 2002-03-25

<160> 16

<170> PatentIn version 3.1

<210> 1
<211> 4620
<212> DNA
<213> full-length nucleotide of human HGFR

<400> 1
cgccctcgcc gcccgcggcg ccccgagcgc tttgtgagca gatgcggagc cgagtggagg 60

gcgcgagcca gatgcggggc gacagctgac ttgctgagag gaggcgggga ggcgcggagc 120

gcgcgtgtgg tccttgcgcc gctgacttct ccactggttc ctgggcaccg aaagataaac 180

ctctcataat gaaggccccc gctgtgcttg cacctggcat cctcgtgctc ctgtttacct 240

tggtgcagag gagcaatggg gagtgtaaag aggcactagc aaagtccgag atgaatgtga 300

atatgaagta tcagcttccc aacttcaccg cggaaacacc catccagaat gtcattctac 360

atgagcatca cattttcctt ggtgccacta actacattta tgttttaaat gaggaagacc 420

ttcagaaggt tgctgagtac aagactgggc ctgtgctgga acacccagat tgtttcccat 480

gtcaggactg cagcagcaaa gccaatttat caggaggtgt ttggaaagat aacatcaaca 540

tggctctagt tgtcgacacc tactatgatg atcaactcat tagctgtggc agcgtcaaca 600

gagggacctg ccagcgacat gtctttcccc acaatcatac tgctgacata cagtcggagg 660

ttcactgcat attctcccca cagatagaag agcccagcca gtgtcctgac tgtgtggtga 720

gcgccctggg agccaaagtc ctttcatctg taaaggaccg gttcatcaac ttctttgtag 780

gcaataccat aaattcttct tatttcccag atcatccatt gcattcgata tcagtgagaa 840

ggctaaagga aacgaaagat ggttttatgt ttttgacgga ccagtcctac attgatgttt 900

tacctgagtt cagagattct taccccatta agtatgtcca tgcctttgaa agcaacaatt 960

ttatttactt cttgacggtc caaagggaaa ctctagatgc tcagactttt cacacaagaa 1020

taatcaggtt ctgttccata aactctggat tgcattccta catggaaatg cctctggagt 1080

gtattctcac agaaaagaga aaaaagagat ccacaaagaa ggaagtgttt aatatacttc 1140
aggctgcgta tgtcagcaag cctggggccc agcttgctag acaaatagga gccagcctga 1200
atgatgacat tcttttcggg gtgttcgcac aaagcaagcc agattctgcc gaaccaatgg 1260
atcgatctgc catgtgtgca ttccctatca aatatgtcaa cgacttcttc aacaagatcg 1320
tcaacaaaaa caatgtgaga tgtctccagc atttttacgg acccaatcat gagcactgct 1380
ttaataggac acttctgaga aattcatcag gctgtgaagc gcgccgtgat gaatatcgaa 1440
cagagtttac cacagctttg cagcgcgttg acttattcat gggtcaattc agcgaagtcc 1500
tcttaacatc tatatccacc ttcattaaag gagacctcac catagctaat cttgggacat 1560
cagagggtcg cttcatgcag gttgtggttt ctcgatcagg accatcaacc cctcatgtga 1620
attttctcct ggactcccat ccagtgtctc cagaagtgat tgtggagcat acattaaacc 1680
aaaatggcta cacactggtt atcactggga agaagatcac gaagatccca ttgaatggct 1740
tgggctgcag acatttccag tcctgcagtc aatgcctctc tgccccaccc tttgttcagt 1800
gtggctggtg ccacgacaaa tgtgtgcgat cggaggaatg cctgagcggg acatggactc 1860
aacagatctg tctgcctgca atctacaagg ttttcccaaa tagtgcaccc cttgaaggag 1920
ggacaaggct gaccatatgt ggctgggact ttggatttcg gaggaataat aaatttgatt 1980
taaagaaaac tagagttctc cttggaaatg agagctgcac cttgacttta agtgagagca 2040
cgatgaatac attgaaatgc acagttggtc ctgccatgaa taagcatttc aatatgtcca 2100
taattatttc aaatggccac gggacaacac aatacagtac attctcctat gtggatcctg 2160
taataacaag tatttcgccg aaatacggtc ctatggctgg tggcacttta cttactttaa 2220
ctggaaatta cctaaacagt gggaattcta gacacatttc aattggtgga aaaacatgta 2280
ctttaaaaag tgtgtcaaac agtattcttg aatgttatac cccagcccaa accatttcaa 2340
ctgagtttgc tgttaaattg aaaattgact tagccaaccg agagacaagc atcttcagtt 2400
accgtgaaga tcccattgtc tatgaaattc atccaaccaa atcttttatt agtacttggt 2460
ggaaagaacc tctcaacatt gtcagttttc tattttgctt tgccagtggt gggagcacaa 2520
taacaggtgt tgggaaaaac ctgaattcag ttagtgtccc gagaatggtc ataaatgtgc 2580
atgaagcagg aaggaacttt acagtggcat gtcaacatcg ctctaattca gagataatct 2640
gttgtaccac tccttccctg caacagctga atctgcaact ccccctgaaa accaaagcct 2700
ttttcatgtt agatgggatc ctttccaaat actttgatct catttatgta cataatcctg 2760
tgtttaagcc ttttgaaaag ccagtgatga tctcaatggg caatgaaaat gtactggaaa 2820

ttaagggaaa tgatattgac cctgaagcag ttaaaggtga agtgttaaaa gttggaaata 2880

agagctgtga gaatatacac ttacattctg aagccgtttt atgcacggtc cccaatgacc 2940

tgctgaaatt gaacagcgag ctaaatatag agtggaagca agcaatttct tcaaccgtcc 3000

ttggaaaagt aatagttcaa ccagatcaga atttcacagg attgattgct ggtgttgtct 3060

caatatcaac agcactgtta ttactacttg ggtttttcct gtggctgaaa aagagaaagc 3120

aaattaaaga tctgggcagt gaattagttc gctacgatgc aagagtacac actcctcatt 3180

tggataggct tgtaagtgcc cgaagtgtaa gcccaactac agaaatggtt tcaaatgaat 3240

ctgtagacta ccgagctact tttccagaag atcagtttcc taattcatct cagaacggtt 3300

catgccgaca agtgcagtat cctctgacag acatgtcccc catcctaact agtggggact 3360

ctgatatatc cagtccatta ctgcaaaata ctgtccacat tgacctcagt gctctaaatc 3420

cagagctggt ccaggcagtg cagcatgtag tgattgggcc cagtagcctg attgtgcatt 3480

tcaatgaagt cataggaaga gggcattttg gttgtgtata tcatgggact ttgttggaca 3540

atgatggcaa gaaaattcac tgtgctgtga aatccttgaa cagaatcact gacataggag 3600

aagtttccca atttctgacc gagggaatca tcatgaaaga ttttagtcat cccaatgtcc 3660

tctcgctcct gggaatctgc ctgcgaagtg aagggtctcc gctggtggtc ctaccataca 3720

tgaaacatgg agatcttcga aatttcattc gaaatgagac tcataatcca actgtaaaag 3780

atcttattgg ctttggtctt caagtagcca aagcgatgaa atatcttgca agcaaaaagt 3840

ttgtccacag agacttggct gcaagaaact gtatgctgga tgaaaaattc acagtcaagg 3900

ttgctgattt tggtcttgcc agagacatgt atgataaaga atactatagt gtacacaaca 3960

aaacaggtgc aaagctgcca gtgaagtgga tggctttgga aagtctgcaa actcaaaagt 4020

ttaccaccaa gtcagatgtg tggtcctttg gcgtcgtcct ctgggagctg atgacaagag 4080

gagccccacc ttatcctgac gtaaacacct ttgatataac tgtttacttg ttgcaaggga 4140

gaagactcct acaacccgaa tactgcccag accccttata tgaagtaatg ctaaaatgct 4200

ggcaccctaa agccgaaatg cgcccatcct tttctgaact ggtgtcccgg atatcagcga 4260

tcttctctac tttcattggg gagcactatg tccatgtgaa cgctacttat gtgaacgtaa 4320

aatgtgtcgc tccgtatcct tctctgttgt catcagaaga taacgctgat gatgaggtgg 4380

acacacgacc agcctccttc tgggagacat catagtgcta gtactatgtc aaagcaacag 4440

tccacacttt gtccaatggt tttttcactg cctgaccttt aaaaggccat cgatattctt 4500

tgctccttgc cataggactt gtattgttat ttaaattact ggattctaag gaatttctta 4560

tctgacagag catcagaacc agaggcttgg tcccacaggc cagggaccaa tgcgctgcag 4620

<210> 2
<211> 1390
<212> PRT
<213> human HGFR

<400> 2

<210> 3
<211> 298
<212> PRT
<213> human catalytic domain wild-type HGFR

<400> 3

Val His Ile Asp Leu Ser Ala Leu Asn Pro Glu Leu Val Gln Ala Val
1               5                   10                  15

Gln His Val Val Ile Gly Pro Ser Ser Leu Ile Val His Phe Asn Glu
            20                  25                  30

Val Ile Gly Arg Gly His Phe Gly Cys Val Tyr His Gly Thr Leu Leu
        35                  40                  45

Asp Asn Asp Gly Lys Lys Ile His Cys Ala Val Lys Ser Leu Asn Arg
    50                  55                  60

Ile Thr Asp Ile Gly Glu Val Ser Gln Phe Leu Thr Glu Gly Ile Ile
65                  70                  75                  80

Met Lys Asp Phe Ser His Pro Asn Val Leu Ser Leu Leu Gly Ile Cys
                85                  90                  95

Leu Arg Ser Glu Gly Ser Pro Leu Val Val Leu Pro Tyr Met Lys His
            100                 105                 110

Gly Asp Leu Arg Asn Phe Ile Arg Asn Glu Thr His Asn Pro Thr Val
        115                 120                 125

Lys Asp Leu Ile Gly Phe Gly Leu Gln Val Ala Lys Gly Met Lys Tyr

    130                 135                 140

Leu Ala Ser Lys Lys Phe Val His Arg Asp Leu Ala Ala Arg Asn Cys
145                 150                 155                 160

Met Leu Asp Glu Lys Phe Thr Val Lys Val Ala Asp Phe Gly Leu Ala
                165                 170                 175

Arg Asp Met Tyr Asp Lys Glu Tyr Tyr Ser Val His Asn Lys Thr Gly
            180                 185                 190

Ala Lys Leu Pro Val Lys Trp Met Ala Leu Glu Ser Leu Gln Thr Gln
        195                 200                 205

Lys Phe Thr Thr Lys Ser Asp Val Trp Ser Phe Gly Val Val Leu Trp
    210                 215                 220

Glu Leu Met Thr Arg Gly Ala Pro Pro Tyr Pro Asp Val Asn Thr Phe
225                 230                 235                 240

Asp Ile Thr Val Tyr Leu Leu Gln Gly Arg Arg Leu Leu Gln Pro Glu
                245                 250                 255

Tyr Cys Pro Asp Pro Leu Tyr Glu Val Met Leu Lys Cys Trp His Pro
            260                 265                 270

Lys Ala Glu Met Arg Pro Ser Phe Ser Glu Leu Val Ser Arg Ile Ser
        275                 280                 285

Ala Ile Phe Ser Thr Phe Ile Gly Glu His
    290                 295

<210> 4
<211> 299
<212> PRT
<213> human catalytic domain wild-type HGFR

<400> 4

Met Val His Ile Asp Leu Ser Ala Leu Asn Pro Glu Leu Val Gln Ala
1               5                   10                  15

Val Gln His Val Val Ile Gly Pro Ser Ser Leu Ile Val His Phe Asn
            20                  25                  30

Glu Val Ile Gly Arg Gly His Phe Gly Cys Val Tyr His Gly Thr Leu
        35                  40                  45

Leu Asp Asn Asp Gly Lys Lys Ile His Cys Ala Val Lys Ser Leu Asn
    50                  55                  60

Arg Ile Thr Asp Ile Gly Glu Val Ser Gln Phe Leu Thr Glu Gly Ile
65                  70                  75                  80

Ile Met Lys Asp Phe Ser His Pro Asn Val Leu Ser Leu Leu Gly Ile
                85                  90                  95

Cys Leu Arg Ser Glu Gly Ser Pro Leu Val Val Leu Pro Tyr Met Lys
            100                 105                 110

His Gly Asp Leu Arg Asn Phe Ile Arg Asn Glu Thr His Asn Pro Thr
        115                 120                 125

Val Lys Asp Leu Ile Gly Phe Gly Leu Gln Val Ala Lys Ala Met Lys
    130                 135                 140

Tyr Leu Ala Ser Lys Lys Phe Val His Arg Asp Leu Ala Ala Arg Asn
145                 150                 155                 160

Cys Met Leu Asp Glu Lys Phe Thr Val Lys Val Ala Asp Phe Gly Leu
                165                 170                 175

Ala Arg Asp Met Tyr Asp Lys Glu Tyr Tyr Ser Val His Asn Lys Thr
            180                 185                 190

Gly Ala Lys Leu Pro Val Lys Trp Met Ala Leu Glu Ser Leu Gln Thr
        195                 200                 205

Gln Lys Phe Thr Thr Lys Ser Asp Val Trp Ser Phe Gly Val Leu Leu
    210                 215                 220

Trp Glu Leu Met Thr Arg Gly Ala Pro Pro Tyr Pro Asp Val Asn Thr
225                 230                 235                 240

Phe Asp Ile Thr Val Tyr Leu Leu Gln Gly Arg Arg Leu Leu Gln Pro
                245                 250                 255

Glu Tyr Cys Pro Asp Pro Leu Tyr Glu Val Met Leu Lys Cys Trp His
            260                 265                 270

Pro Lys Ala Glu Met Arg Pro Ser Phe Ser Glu Leu Val Ser Arg Ile
        275                 280                 285

Ser Ala Ile Phe Ser Thr Phe Ile Gly Glu His
    290                 295

<210> 5
<211> 299
<212> PRT
<213> human catalytic domain wild-type HGFR

<400> 5

Met Val His Ile Asp Leu Ser Ala Leu Asn Pro Glu Leu Val Gln Ala
1               5                   10                  15

Val Gln His Val Val Ile Gly Pro Ser Ser Leu Ile Val His Phe Asn
            20                  25                  30

Glu Val Ile Gly Arg Gly His Phe Gly Cys Val Tyr His Gly Thr Leu
        35                  40                  45

Leu Asp Asn Asp Gly Lys Lys Ile His Cys Ala Val Lys Ser Leu Asn
    50                  55                  60

Arg Ile Thr Asp Ile Gly Glu Val Ser Gln Phe Leu Thr Glu Gly Ile
65                  70                  75                  80

Ile Met Lys Asp Phe Ser His Pro Asn Val Leu Ser Leu Leu Gly Ile
                85                  90                  95

Cys Leu Arg Ser Glu Gly Ser Pro Leu Val Val Leu Pro Tyr Met Lys
            100                 105                 110

His Gly Asp Leu Arg Asn Phe Ile Arg Asn Glu Thr His Asn Pro Thr
        115                 120                 125

Val Lys Asp Leu Ile Gly Phe Gly Leu Gln Val Ala Lys Gly Met Lys
    130                 135                 140

Tyr Leu Ala Ser Lys Lys Phe Val His Arg Asp Leu Ala Ala Arg Asn
145                 150                 155                 160

Cys Met Leu Asp Glu Lys Phe Thr Val Lys Val Ala Asp Phe Gly Leu

165                 170                 175

Ala Arg Asp Met Tyr Asp Lys Glu Tyr Tyr Ser Val His Asn Lys Thr
        180                 185                 190

Gly Ala Lys Leu Pro Val Lys Trp Met Ala Leu Glu Ser Leu Gln Thr
    195                 200                 205

Gln Lys Phe Thr Thr Lys Ser Asp Val Trp Ser Phe Gly Val Leu Leu
   210                 215                 220

Trp Glu Leu Met Thr Arg Gly Ala Pro Pro Tyr Pro Asp Val Asn Thr
225                 230                 235                 240

Phe Asp Ile Thr Val Tyr Leu Leu Gln Gly Arg Arg Leu Leu Gln Pro
                245                 250                 255

Glu Tyr Cys Pro Asp Pro Leu Tyr Glu Val Met Leu Lys Cys Trp His
        260                 265                 270

Pro Lys Ala Glu Met Arg Pro Ser Phe Ser Glu Leu Val Ser Arg Ile
    275                 280                 285

Ser Ala Ile Phe Ser Thr Phe Ile Gly Glu His
   290                 295

<210>  6
<211>  31
<212>  DNA
<213>  synthetic nucleotide primer

<400>  6
gatcccatgg tccacattga cctcagtgct c                                31

<210>  7
<211>  40
<212>  DNA
<213>  synthetic nucleotide primer

<400>  7
ctagaagctt ctagtgctcc ccaatgaaag tagagaagat                       40

<210>  8
<211>  15
<212>  PRT
<213>  synthetic peptide

<400> 8

Ala Arg Asp Met Tyr Asp Lys Glu Tyr Tyr Ser Val His Asn Lys
1               5                   10                  15

<210> 9
<211> 299
<212> PRT
<213> HGFR M1250T Kinase

<400> 9

Met Val His Ile Asp Leu Ser Ala Leu Asn Pro Glu Leu Val Gln Ala
1               5                   10                  15

Val Gln His Val Val Ile Gly Pro Ser Ser Leu Ile Val His Phe Asn
            20                  25                  30

Glu Val Ile Gly Arg Gly His Phe Gly Cys Val Tyr His Gly Thr Leu
        35                  40                  45

Leu Asp Asn Asp Gly Lys Lys Ile His Cys Ala Val Lys Ser Leu Asn
    50                  55                  60

Arg Ile Thr Asp Ile Gly Glu Val Ser Gln Phe Leu Thr Glu Gly Ile
65                  70                  75                  80

Ile Met Lys Asp Phe Ser His Pro Asn Val Leu Ser Leu Leu Gly Ile
                85                  90                  95

Cys Leu Arg Ser Glu Gly Ser Pro Leu Val Val Leu Pro Tyr Met Lys
            100                 105                 110

His Gly Asp Leu Arg Asn Phe Ile Arg Asn Glu Thr His Asn Pro Thr
        115                 120                 125

Val Lys Asp Leu Ile Gly Phe Gly Leu Gln Val Ala Lys Ala Met Lys
    130                 135                 140

Tyr Leu Ala Ser Lys Lys Phe Val His Arg Asp Leu Ala Ala Arg Asn
145                 150                 155                 160

Cys Met Leu Asp Glu Lys Phe Thr Val Lys Val Ala Asp Phe Gly Leu
                165                 170                 175

Ala Arg Asp Met Tyr Asp Lys Glu Tyr Tyr Ser Val His Asn Lys Thr

            180                 185                 190

Gly Ala Lys Leu Pro Val Lys Trp Thr Ala Leu Glu Ser Leu Gln Thr
        195                 200                 205

Gln Lys Phe Thr Thr Lys Ser Asp Val Trp Ser Phe Gly Val Leu Leu
    210                 215                 220

Trp Glu Leu Met Thr Arg Gly Ala Pro Pro Tyr Pro Asp Val Asn Thr
225                 230                 235                 240

Phe Asp Ile Thr Val Tyr Leu Leu Gln Gly Arg Arg Leu Leu Gln Pro
                245                 250                 255

Glu Tyr Cys Pro Asp Pro Leu Tyr Glu Val Met Leu Lys Cys Trp His
            260                 265                 270

Pro Lys Ala Glu Met Arg Pro Ser Phe Ser Glu Leu Val Ser Arg Ile
        275                 280                 285

Ser Ala Ile Phe Ser Thr Phe Ile Gly Glu His
    290                 295

<210> 10
<211> 897
<212> DNA
<213> HGFR Y 1230C

<400> 10
atggtccaca ttgacctcag tgctctaaat ccagagctgg tccaggcagt gcagcatgta 60

gtgattgggc ccagtagcct gattgtgcat ttcaatgaag tcataggaag agggcatttt 120

ggttgtgtat atcatgggac tttgttggac aatgatggca agaaaattca ctgtgctgtg 180

aaatccttga acagaatcac tgacatagga gaagtttccc aatttctgac cgagggaatc 240

atcatgaaag attttagtca tcccaatgtc ctctcgctcc tgggaatctg cctgcgaagt 300

gaagggtctc cgctggtggt cctaccatac atgaaacatg gagatcttcg aaatttcatt 360

cgaaatgaga ctcataatcc aactgtaaaa gatcttattg gctttggtct tcaagtagcc 420

aaagggatga aatatcttgc aagcaaaaag tttgtccaca gagacttggc tgcaagaaac 480

tgtatgctgg atgaaaaatt cacagtcaag gttgctgatt ttggtcttgc cagagacatg 540

tgtgataaag aatactatag tgtacacaac aaaacaggtg caaagctgcc agtgaagtgg 600

atggctttgg aaagtctgca aactcaaaag tttaccacca agtcagatgt gtggtccttt 660

ggcgtcgtcc tctgggagct gatgacaaga ggagccccac cttatcctga cgtaaacacc 720

tttgatataa ctgtttactt gttgcaaggg agaagactcc tacaacccga atactgccca 780

gaccccttat atgaagtaat gctaaaatgc tggcacccta aagccgaaat gcgcccatcc 840

ttttctgaac tggtgtcccg gatatcagcg atcttctcta ctttcattgg ggagcac 897

<210> 11
<211> 897
<212> DNA
<213> HGFR M1250T

<400> 11
atggtccaca ttgacctcag tgctctaaat ccagagctgg tccaggcagt gcagcatgta 60

gtgattgggc ccagtagcct gattgtgcat ttcaatgaag tcataggaag agggcatttt 120

ggttgtgtat atcatgggac tttgttggac aatgatggca agaaaattca ctgtgctgtg 180

aaatccttga acagaatcac tgacatagga gaagtttccc aatttctgac cgagggaatc 240

atcatgaaag attttagtca tcccaatgtc ctctcgctcc tgggaatctg cctgcgaagt 300

gaagggtctc cgctggtggt cctaccatac atgaaacatg gagatcttcg aaatttcatt 360

cgaaatgaga ctcataatcc aactgtaaaa gatcttattg gctttggtct tcaagtagcc 420

aaaggcatga aatatcttgc gagcaaaaag tttgtccaca gagacttggc tgcaagaaac 480

tgtatgctgg atgaaaaatt cacagtcaag gttgctgatt ttggtcttgc cagagacatg 540

tatgataaag aatactatag tgtacacaac aaaacaggtg caaagctgcc agtgaagtgg 600

accgctttgg aaagtctgca aactcaaaag tttaccacca agtcagatgt gtggtccttt 660

ggcgtgctcc tctgggagct gatgacaaga ggagccccac cttatcctga tgtaaacacc 720

tttgatataa ctgtttactt gttgcaaggg agaagactcc tacaacccga atactgccca 780

gaccccttat atgaagtaat gctaaaatgc tggcacccta aagccgaaat gcgcccatcc 840

ttttctgaac tggtgtcccg gatatcagcg atcttctcta ctttcattgg ggagcac 897

<210> 12
<211> 1281
<212> DNA
<213> Human HGFR intracellular domain

<400> 12
atgggcagtg aattagttcg ctacgatgca agagtacaca ctcctcattt ggataggctt 60

gtaagtgccc gaagtgtaag cccaactaca gaaatggttt caaatgaatc tgtagactac 120

cgagctactt ttccagaaga tcagtttcct aattcatctc agaacggttc atgccgacaa 180

gtgcagtatc ctctgacaga catgtccccc atcctaacta gtggggactc tgatatatcc 240

agtccattac tgcaaaatac tgtccacatt gacctcagtg ctctaaatcc agagctggtc 300

caggcagtgc agcatgtagt gattgggccc agtagcctga ttgtgcattt caatgaagtc 360

ataggaagag ggcattttgg ttgtgtatat catgggactt tgttggacaa tgatggcaag 420

aaaattcact gtgctgtgaa atccttgaac agaatcactg acataggaga agtttcccaa 480

tttctggccg agggaatcat catgaaagat tttagtcatc ccaatgtcct ctcgctcctg 540

ggaatctgcc tgcgaagtga agggtctccg ctggtggtcc taccatacat gaaacatgga 600

gatcttcgaa atttcattcg aaatgagact cataatccaa ctgtaaaaga tcttattggc 660

tttggtcttc aagtagccaa aggcatgaaa tatcttgcaa gcaaaaagtt tgtccacaga 720

gacttggctg caagaaactg tatgctggat gaaaaattca cagtcaaggt tgctgatttt 780

ggtcttgcca gagacatgta tgataaagaa tactatagtg tacacaacaa aacaggtgca 840

aagctgccag tgaagtggat ggctttggaa agtctgcaaa ctcaaaagtt taccaccaag 900

tcagatgtgt ggtcctttgg cgtgctcctc tgggagctga tgacaagagg agccccacct 960

tatcctgatg taaacacctt tgatataact gtttacttgt tgcaagggag aagactccta 1020

caacccgaat actgcccaga ccccttatat gaagtaatgc taaaatgctg gcaccctaaa 1080

gccgaaatgc gcccatcctt ttctgaactg gtgtcccgga tatcagcaat cttctctact 1140

ttcattgggg agcactatgt ccatgtgaac gctacttatg tgaacgtaaa atgtgtcgct 1200

ccatatccat ctctgttgtc atcagaagat aacgctgatg atgaggtgga cacacgacca 1260

gcctccttct gggagacatc a 1281

<210> 13
<211> 427
<212> PRT
<213> Human HGFR intracellular domain protein

<400> 13

Met Gly Ser Glu Leu Val Arg Tyr Asp Ala Arg Val His Thr Pro His
1               5                   10                  15

Leu Asp Arg Leu Val Ser Ala Arg Ser Val Ser Pro Thr Thr Glu Met
            20                  25                  30

Val Ser Asn Glu Ser Val Asp Tyr Arg Ala Thr Phe Pro Glu Asp Gln
        35                  40                  45

Phe Pro Asn Ser Ser Gln Asn Gly Ser Cys Arg Gln Val Gln Tyr Pro
    50                  55                  60

Leu Thr Asp Met Ser Pro Ile Leu Thr Ser Gly Asp Ser Asp Ile Ser
65                  70                  75                  80

Ser Pro Leu Leu Gln Asn Thr Val His Ile Asp Leu Ser Ala Leu Asn
                85                  90                  95

Pro Glu Leu Val Gln Ala Val Gln His Val Val Ile Gly Pro Ser Ser
            100                 105                 110

Leu Ile Val His Phe Asn Glu Val Ile Gly Arg Gly His Phe Gly Cys
        115                 120                 125

Val Tyr His Gly Thr Leu Leu Asp Asn Asp Gly Lys Lys Ile His Cys
    130                 135                 140

Ala Val Lys Ser Leu Asn Arg Ile Thr Asp Ile Gly Glu Val Ser Gln
145                 150                 155                 160

Phe Leu Ala Glu Gly Ile Ile Met Lys Asp Phe Ser His Pro Asn Val
                165                 170                 175

Leu Ser Leu Leu Gly Ile Cys Leu Arg Ser Glu Gly Ser Pro Leu Val
            180                 185                 190

Val Leu Pro Tyr Met Lys His Gly Asp Leu Arg Asn Phe Ile Arg Asn
        195                 200                 205

Glu Thr His Asn Pro Thr Val Lys Asp Leu Ile Gly Phe Gly Leu Gln
    210                 215                 220

Val Ala Lys Gly Met Lys Tyr Leu Ala Ser Lys Lys Phe Val His Arg
225                 230                 235                 240

Asp Leu Ala Ala Arg Asn Cys Met Leu Asp Glu Lys Phe Thr Val Lys
                245                 250                 255

Val Ala Asp Phe Gly Leu Ala Arg Asp Met Tyr Asp Lys Glu Tyr Tyr
            260                 265                 270

Ser Val His Asn Lys Thr Gly Ala Lys Leu Pro Val Lys Trp Met Ala

275                 280                 285

Leu Glu Ser Leu Gln Thr Gln Lys Phe Thr Thr Lys Ser Asp Val Trp
    290                 295                 300

Ser Phe Gly Val Leu Leu Trp Glu Leu Met Thr Arg Gly Ala Pro Pro
305                 310                 315                 320

Tyr Pro Asp Val Asn Thr Phe Asp Ile Thr Val Tyr Leu Leu Gln Gly
                325                 330                 335

Arg Arg Leu Leu Gln Pro Glu Tyr Cys Pro Asp Pro Leu Tyr Glu Val
            340                 345                 350

Met Leu Lys Cys Trp His Pro Lys Ala Glu Met Arg Pro Ser Phe Ser
        355                 360                 365

Glu Leu Val Ser Arg Ile Ser Ala Ile Phe Ser Thr Phe Ile Gly Glu
    370                 375                 380

His Tyr Val His Val Asn Ala Thr Tyr Val Asn Val Lys Cys Val Ala
385                 390                 395                 400

Pro Tyr Pro Ser Leu Leu Ser Ser Glu Asp Asn Ala Asp Asp Glu Val
                405                 410                 415

Asp Thr Arg Pro Ala Ser Phe Trp Glu Thr Ser
            420                 425

<210> 14
<211> 897
<212> DNA
<213> HGFR H1094R DNA

<400> 14
atggtccaca ttgacctcag tgctctaaat ccagagctgg tccaggcagt gcagcatgta    60

gtgattgggc ccagtagcct gattgtgcat ttcaatgaag tcataggaag agggcatttt   120

ggttgtgtat atcgtgggac tttgttggac aatgatggca agaaaattca ctgtgctgtg   180

aaatccttga acagaatcac tgacatagga gaagtttccc aatttctgac cgagggaatc   240

atcatgaaag attttagtca tcccaatgtc ctctcgctcc tgggaatctg cctgcgaagt   300

gaagggtctc cgctggtggt cctaccatac atgaaacatg gagatcttcg aaatttcatt   360

cgaaatgaga ctcataatcc aactgtaaaa gatcttattg gctttggtct tcaagtagcc   420

aaagggatga aatatcttgc aagcaaaaag tttgtccaca gagacttggc tgcaagaaac 480

tgtatgctgg atgaaaaatt cacagtcaag gttgctgatt ttggtcttgc cagagacatg 540

tatgataaag aatactatag tgtacacaac aaaacaggtg caaagctgcc agtgaagtgg 600

atggctttgg aaagtctgca aactcaaaag tttaccacca agtcagatgt gtggtccttt 660

ggcgtcgtcc tctgggagct gatgacaaga ggagccccac cttatcctga cgtaaacacc 720

tttgatataa ctgtttactt gttgcaaggg agaagactcc tacaacccga atactgccca 780

gaccccttat atgaagtaat gctaaaatgc tggcacccta aagccgaaat gcgcccatcc 840

ttttctgaac tggtgtcccg gatatcagcg atcttctcta ctttcattgg ggagcac 897

<210> 15
<211> 299
<212> PRT
<213> HGFR H1094R Kinase

<400> 15

Met Val His Ile Asp Leu Ser Ala Leu Asn Pro Glu Leu Val Gln Ala
1               5                   10                  15

Val Gln His Val Val Ile Gly Pro Ser Ser Leu Ile Val His Phe Asn
            20                  25                  30

Glu Val Ile Gly Arg Gly His Phe Gly Cys Val Tyr Arg Gly Thr Leu
        35                  40                  45

Leu Asp Asn Asp Gly Lys Lys Ile His Cys Ala Val Lys Ser Leu Asn
    50                  55                  60

Arg Ile Thr Asp Ile Gly Glu Val Ser Gln Phe Leu Thr Glu Gly Ile
65                  70                  75                  80

Ile Met Lys Asp Phe Ser His Pro Asn Val Leu Ser Leu Leu Gly Ile
                85                  90                  95

Cys Leu Arg Ser Glu Gly Ser Pro Leu Val Val Leu Pro Tyr Met Lys
            100                 105                 110

His Gly Asp Leu Arg Asn Phe Ile Arg Asn Glu Thr His Asn Pro Thr
        115                 120                 125

Val Lys Asp Leu Ile Gly Phe Gly Leu Gln Val Ala Lys Ala Met Lys

    130                 135                 140

Tyr Leu Ala Ser Lys Lys Phe Val His Arg Asp Leu Ala Ala Arg Asn
145                 150                 155                 160

Cys Met Leu Asp Glu Lys Phe Thr Val Lys Val Ala Asp Phe Gly Leu
                165                 170                 175

Ala Arg Asp Met Tyr Asp Lys Glu Tyr Tyr Ser Val His Asn Lys Thr
            180                 185                 190

Gly Ala Lys Leu Pro Val Lys Trp Met Ala Leu Glu Ser Leu Gln Thr
        195                 200                 205

Gln Lys Phe Thr Thr Lys Ser Asp Val Trp Ser Phe Gly Val Leu Leu
    210                 215                 220

Trp Glu Leu Met Thr Arg Gly Ala Pro Pro Tyr Pro Asp Val Asn Thr
225                 230                 235                 240

Phe Asp Ile Thr Val Tyr Leu Leu Gln Gly Arg Arg Leu Leu Gln Pro
                245                 250                 255

Glu Tyr Cys Pro Asp Pro Leu Tyr Glu Val Met Leu Lys Cys Trp His
            260                 265                 270

Pro Lys Ala Glu Met Arg Pro Ser Phe Ser Glu Leu Val Ser Arg Ile
        275                 280                 285

Ser Ala Ile Phe Ser Thr Phe Ile Gly Glu His
    290                 295

<210> 16
<211> 299
<212> PRT
<213> HGFR Y1230C Kinase

<400> 16

Met Val His Ile Asp Leu Ser Ala Leu Asn Pro Glu Leu Val Gln Ala
1               5                   10                  15

Val Gln His Val Val Ile Gly Pro Ser Ser Leu Ile Val His Phe Asn
            20                  25                  30

Glu Val Ile Gly Arg Gly His Phe Gly Cys Val Tyr His Gly Thr Leu
        35                  40                  45

Leu Asp Asn Asp Gly Lys Lys Ile His Cys Ala Val Lys Ser Leu Asn
    50                  55                  60

Arg Ile Thr Asp Ile Gly Glu Val Ser Gln Phe Leu Thr Glu Gly Ile
65                  70                  75                  80

Ile Met Lys Asp Phe Ser His Pro Asn Val Leu Ser Leu Leu Gly Ile
                85                  90                  95

Cys Leu Arg Ser Glu Gly Ser Pro Leu Val Val Leu Pro Tyr Met Lys
            100                 105                 110

His Gly Asp Leu Arg Asn Phe Ile Arg Asn Glu Thr His Asn Pro Thr
        115                 120                 125

Val Lys Asp Leu Ile Gly Phe Gly Leu Gln Val Ala Lys Ala Met Lys
    130                 135                 140

Tyr Leu Ala Ser Lys Lys Phe Val His Arg Asp Leu Ala Ala Arg Asn
145                 150                 155                 160

Cys Met Leu Asp Glu Lys Phe Thr Val Lys Val Ala Asp Phe Gly Leu
                165                 170                 175

Ala Arg Asp Met Cys Asp Lys Glu Tyr Tyr Ser Val His Asn Lys Thr
            180                 185                 190

Gly Ala Lys Leu Pro Val Lys Trp Met Ala Leu Glu Ser Leu Gln Thr
        195                 200                 205

Gln Lys Phe Thr Thr Lys Ser Asp Val Trp Ser Phe Gly Val Leu Leu
    210                 215                 220

Trp Glu Leu Met Thr Arg Gly Ala Pro Pro Tyr Pro Asp Val Asn Thr
225                 230                 235                 240

Phe Asp Ile Thr Val Tyr Leu Leu Gln Gly Arg Arg Leu Leu Gln Pro
                245                 250                 255

Glu Tyr Cys Pro Asp Pro Leu Tyr Glu Val Met Leu Lys Cys Trp His
            260                 265                 270

Pro Lys Ala Glu Met Arg Pro Ser Phe Ser Glu Leu Val Ser Arg Ile
    275                 280                 285

Ser Ala Ile Phe Ser Thr Phe Ile Gly Glu His
    290                 295

### Annex to the application documents - subsequently filed sequences listing

SEQUENCE LISTING

<110> Agouron, Pharmaceuticals, Inc.

<120> Catalytic Domains Of The Human Hepatocyte Growth Factor Receptor Tyrosine
Kinase, And Materials And Methods For Identification Of Inhibitors Thereof

<130> 0128-US

<140> 02.006616.3
<141> 2002-03-25

<160> 16

<170> PatentIn version 3.1

<210> 1
<211> 4620
<212> DNA
<213> full-length nucleotide of human HGFR

<400> 1
cgccctcgcc gcccgcggcg ccccgagcgc tttgtgagca gatgcggagc cgagtggagg 60

gcgcgagcca gatgcggggc gacagctgac ttgctgagag gaggcgggga ggcgcggagc 120

gcgcgtgtgg tccttgcgcc gctgacttct ccactggttc ctgggcaccg aaagataaac 180

ctctcataat gaaggccccc gctgtgcttg cacctggcat cctcgtgctc ctgtttacct 240

tggtgcagag gagcaatggg gagtgtaaag aggcactagc aaagtccgag atgaatgtga 300

atatgaagta tcagcttccc aacttcaccg cggaaacacc catccagaat gtcattctac 360

atgagcatca cattttcctt ggtgccacta actacattta tgttttaaat gaggaagacc 420

ttcagaaggt tgctgagtac aagactgggc ctgtgctgga acacccagat tgtttcccat 480

gtcaggactg cagcagcaaa gccaatttat caggaggtgt ttggaaagat aacatcaaca 540

tggctctagt tgtcgacacc tactatgatg atcaactcat tagctgtggc agcgtcaaca 600

gagggacctg ccagcgacat gtctttcccc acaatcatac tgctgacata cagtcggagg 660

ttcactgcat attctcccca cagatagaag agcccagcca gtgtcctgac tgtgtggtga 720

gcgccctggg agccaaagtc ctttcatctg taaaggaccg gttcatcaac ttctttgtag 780

gcaataccat aaattcttct tatttcccag atcatccatt gcattcgata tcagtgagaa 840

ggctaaagga aacgaaagat ggttttatgt ttttgacgga ccagtcctac attgatgttt 900

tacctgagtt cagagattct taccccatta agtatgtcca tgcctttgaa agcaacaatt 960

ttatttactt cttgacggtc caaagggaaa ctctagatgc tcagactttt cacacaagaa 1020

taatcaggtt ctgttccata aactctggat tgcattccta catggaaatg cctctggagt 1080

gtattctcac agaaaagaga aaaaagagat ccacaaagaa ggaagtgttt aatatacttc 1140

aggctgcgta tgtcagcaag cctggggccc agcttgctag acaaatagga gccagcctga 1200

atgatgacat tcttttcggg gtgttcgcac aaagcaagcc agattctgcc gaaccaatgg 1260

atcgatctgc catgtgtgca ttccctatca aatatgtcaa cgacttcttc aacaagatcg 1320

tcaacaaaaa caatgtgaga tgtctccagc atttttacgg acccaatcat gagcactgct 1380

ttaataggac acttctgaga aattcatcag gctgtgaagc gcgccgtgat gaatatcgaa 1440

cagagtttac cacagctttg cagcgcgttg acttattcat gggtcaattc agcgaagtcc 1500

tcttaacatc tatatccacc ttcattaaag gagacctcac catagctaat cttgggacat 1560

cagagggtcg cttcatgcag gttgtggttt ctcgatcagg accatcaacc cctcatgtga 1620

attttctcct ggactcccat ccagtgtctc cagaagtgat tgtggagcat acattaaacc 1680

aaaatggcta cacactggtt atcactggga agaagatcac gaagatccca ttgaatggct 1740

tgggctgcag acatttccag tcctgcagtc aatgcctctc tgccccaccc tttgttcagt 1800

gtggctggtg ccacgacaaa tgtgtgcgat cggaggaatg cctgagcggg acatggactc 1860

aacagatctg tctgcctgca atctacaagg ttttcccaaa tagtgcaccc cttgaaggag 1920

ggacaaggct gaccatatgt ggctgggact ttggatttcg gaggaataat aaatttgatt 1980

taaagaaaac tagagttctc cttggaaatg agagctgcac cttgacttta agtgagagca 2040

cgatgaatac attgaaatgc acagttggtc ctgccatgaa taagcatttc aatatgtcca 2100

taattatttc aaatggccac gggacaacac aatacagtac attctcctat gtggatcctg 2160

taataacaag tatttcgccg aaatacggtc ctatggctgg tggcacttta cttactttaa 2220

ctggaaatta cctaaacagt gggaattcta gacacatttc aattggtgga aaaacatgta 2280

ctttaaaaag tgtgtcaaac agtattcttg aatgttatac cccagcccaa accatttcaa 2340

ctgagtttgc tgttaaattg aaaattgact tagccaaccg agagacaagc atcttcagtt 2400

accgtgaaga tcccattgtc tatgaaattc atccaaccaa atcttttatt agtacttggt 2460

ggaaagaacc tctcaacatt gtcagttttc tattttgctt tgccagtggt gggagcacaa 2520

taacaggtgt tgggaaaaac ctgaattcag ttagtgtccc gagaatggtc ataaatgtgc 2580

atgaagcagg aaggaacttt acagtggcat gtcaacatcg ctctaattca gagataatct 2640

gttgtaccac tccttccctg caacagctga atctgcaact ccccctgaaa accaaagcct 2700

ttttcatgtt agatgggatc ctttccaaat actttgatct catttatgta cataatcctg 2760

tgtttaagcc ttttgaaaag ccagtgatga tctcaatggg caatgaaaat gtactggaaa 2820

ttaagggaaa tgatattgac cctgaagcag ttaaaggtga agtgttaaaa gttggaaata 2880
agagctgtga gaatatacac ttacattctg aagccgtttt atgcacggtc cccaatgacc 2940
tgctgaaatt gaacagcgag ctaaatatag agtggaagca agcaatttct tcaaccgtcc 3000
ttggaaaagt aatagttcaa ccagatcaga atttcacagg attgattgct ggtgttgtct 3060
caatatcaac agcactgtta ttactacttg ggtttttcct gtggctgaaa aagagaaagc 3120
aaattaaaga tctgggcagt gaattagttc gctacgatgc aagagtacac actcctcatt 3180
tggataggct tgtaagtgcc cgaagtgtaa gcccaactac agaaatggtt tcaaatgaat 3240
ctgtagacta ccgagctact tttccagaag atcagtttcc taattcatct cagaacggtt 3300
catgccgaca agtgcagtat cctctgacag acatgtcccc catcctaact agtggggact 3360
ctgatatatc cagtccatta ctgcaaaata ctgtccacat tgacctcagt gctctaaatc 3420
cagagctggt ccaggcagtg cagcatgtag tgattgggcc cagtagcctg attgtgcatt 3480
tcaatgaagt cataggaaga gggcattttg gttgtgtata tcatgggact ttgttggaca 3540
atgatggcaa gaaaattcac tgtgctgtga aatccttgaa cagaatcact gacataggag 3600
aagtttccca atttctgacc gagggaatca tcatgaaaga ttttagtcat cccaatgtcc 3660
tctcgctcct gggaatctgc ctgcgaagtg aagggtctcc gctggtggtc ctaccataca 3720
tgaaacatgg agatcttcga aatttcattc gaaatgagac tcataatcca actgtaaaag 3780
atcttattgg ctttggtctt caagtagcca aagcgatgaa atatcttgca agcaaaaagt 3840
ttgtccacag agacttggct gcaagaaact gtatgctgga tgaaaaattc acagtcaagg 3900
ttgctgattt tggtcttgcc agagacatgt atgataaaga atactatagt gtacacaaca 3960
aaacaggtgc aaagctgcca gtgaagtgga tggctttgga aagtctgcaa actcaaaagt 4020
ttaccaccaa gtcagatgtg tggtcctttg gcgtcgtcct ctgggagctg atgacaagag 4080
gagccccacc ttatcctgac gtaaacacct ttgatataac tgtttacttg ttgcaaggga 4140
gaagactcct acaacccgaa tactgcccag accccttata tgaagtaatg ctaaaatgct 4200
ggcaccctaa agccgaaatg cgcccatcct tttctgaact ggtgtcccgg atatcagcga 4260
tcttctctac tttcattggg gagcactatg tccatgtgaa cgctacttat gtgaacgtaa 4320
aatgtgtcgc tccgtatcct tctctgttgt catcagaaga taacgctgat gatgaggtgg 4380
acacacgacc agcctccttc tgggagacat catagtgcta gtactatgtc aaagcaacag 4440
tccacacttt gtccaatggt tttttcactg cctgaccttt aaaaggccat cgatattctt 4500
tgctccttgc cataggactt gtattgttat ttaaattact ggattctaag gaatttctta 4560

tctgacagag catcagaacc agaggcttgg tcccacaggc cagggaccaa tgcgctgcag 4620

<210> 2
<211> 1390
<212> PRT
<213> human HGFR

<400> 2

<210> 3
<211> 298
<212> PRT
<213> human catalytic domain wild-type HGFR

<400> 3

Val His Ile Asp Leu Ser Ala Leu Asn Pro Glu Leu Val Gln Ala Val
1               5                   10                  15

Gln His Val Val Ile Gly Pro Ser Ser Leu Ile Val His Phe Asn Glu
            20                  25                  30

Val Ile Gly Arg Gly His Phe Gly Cys Val Tyr His Gly Thr Leu Leu
        35                  40                  45

Asp Asn Asp Gly Lys Lys Ile His Cys Ala Val Lys Ser Leu Asn Arg
    50                  55                  60

Ile Thr Asp Ile Gly Glu Val Ser Gln Phe Leu Thr Glu Gly Ile Ile
65                  70                  75                  80

Met Lys Asp Phe Ser His Pro Asn Val Leu Ser Leu Leu Gly Ile Cys
                85                  90                  95

Leu Arg Ser Glu Gly Ser Pro Leu Val Val Leu Pro Tyr Met Lys His
            100                 105                 110

Gly Asp Leu Arg Asn Phe Ile Arg Asn Glu Thr His Asn Pro Thr Val
        115                 120                 125

Lys Asp Leu Ile Gly Phe Gly Leu Gln Val Ala Lys Gly Met Lys Tyr

    130                 135                 140

Leu Ala Ser Lys Lys Phe Val His Arg Asp Leu Ala Ala Arg Asn Cys
145                 150                 155                 160

Met Leu Asp Glu Lys Phe Thr Val Lys Val Ala Asp Phe Gly Leu Ala
                165                 170                 175

Arg Asp Met Tyr Asp Lys Glu Tyr Tyr Ser Val His Asn Lys Thr Gly
            180                 185                 190

Ala Lys Leu Pro Val Lys Trp Met Ala Leu Glu Ser Leu Gln Thr Gln
        195                 200                 205

Lys Phe Thr Thr Lys Ser Asp Val Trp Ser Phe Gly Val Val Leu Trp
    210                 215                 220

Glu Leu Met Thr Arg Gly Ala Pro Pro Tyr Pro Asp Val Asn Thr Phe
225                 230                 235                 240

Asp Ile Thr Val Tyr Leu Leu Gln Gly Arg Arg Leu Leu Gln Pro Glu
                245                 250                 255

Tyr Cys Pro Asp Pro Leu Tyr Glu Val Met Leu Lys Cys Trp His Pro
            260                 265                 270

Lys Ala Glu Met Arg Pro Ser Phe Ser Glu Leu Val Ser Arg Ile Ser
        275                 280                 285

Ala Ile Phe Ser Thr Phe Ile Gly Glu His
    290                 295

<210> 4
<211> 299
<212> PRT
<213> human catalytic domain wild-type HGFR

<400> 4

Met Val His Ile Asp Leu Ser Ala Leu Asn Pro Glu Leu Val Gln Ala
1               5                   10                  15

Val Gln His Val Val Ile Gly Pro Ser Ser Leu Ile Val His Phe Asn
            20                  25                  30

Glu Val Ile Gly Arg Gly His Phe Gly Cys Val Tyr His Gly Thr Leu
        35                  40                  45

Leu Asp Asn Asp Gly Lys Lys Ile His Cys Ala Val Lys Ser Leu Asn
    50                  55                  60

Arg Ile Thr Asp Ile Gly Glu Val Ser Gln Phe Leu Thr Glu Gly Ile
65                  70                  75                  80

Ile Met Lys Asp Phe Ser His Pro Asn Val Leu Ser Leu Leu Gly Ile
                85                  90                  95

Cys Leu Arg Ser Glu Gly Ser Pro Leu Val Val Leu Pro Tyr Met Lys
            100                 105                 110

His Gly Asp Leu Arg Asn Phe Ile Arg Asn Glu Thr His Asn Pro Thr
        115                 120                 125

Val Lys Asp Leu Ile Gly Phe Gly Leu Gln Val Ala Lys Ala Met Lys
    130                 135                 140

Tyr Leu Ala Ser Lys Lys Phe Val His Arg Asp Leu Ala Ala Arg Asn
145                 150                 155                 160

Cys Met Leu Asp Glu Lys Phe Thr Val Lys Val Ala Asp Phe Gly Leu
                165                 170                 175

Ala Arg Asp Met Tyr Asp Lys Glu Tyr Tyr Ser Val His Asn Lys Thr
            180                 185                 190

Gly Ala Lys Leu Pro Val Lys Trp Met Ala Leu Glu Ser Leu Gln Thr
        195                 200                 205

Gln Lys Phe Thr Thr Lys Ser Asp Val Trp Ser Phe Gly Val Leu Leu
    210                 215                 220

Trp Glu Leu Met Thr Arg Gly Ala Pro Pro Tyr Pro Asp Val Asn Thr
225                 230                 235                 240

Phe Asp Ile Thr Val Tyr Leu Leu Gln Gly Arg Arg Leu Leu Gln Pro
                245                 250                 255

Glu Tyr Cys Pro Asp Pro Leu Tyr Glu Val Met Leu Lys Cys Trp His
            260                 265                 270

Pro Lys Ala Glu Met Arg Pro Ser Phe Ser Glu Leu Val Ser Arg Ile
        275                 280                 285

Ser Ala Ile Phe Ser Thr Phe Ile Gly Glu His
    290                 295

<210> 5
<211> 299
<212> PRT
<213> human catalytic domain wild-type HGFR

<400> 5

Met Val His Ile Asp Leu Ser Ala Leu Asn Pro Glu Leu Val Gln Ala
1               5                   10                  15

Val Gln His Val Val Ile Gly Pro Ser Ser Leu Ile Val His Phe Asn
            20                  25                  30

Glu Val Ile Gly Arg Gly His Phe Gly Cys Val Tyr His Gly Thr Leu
        35                  40                  45

Leu Asp Asn Asp Gly Lys Lys Ile His Cys Ala Val Lys Ser Leu Asn
    50                  55                  60

Arg Ile Thr Asp Ile Gly Glu Val Ser Gln Phe Leu Thr Glu Gly Ile
65                  70                  75                  80

Ile Met Lys Asp Phe Ser His Pro Asn Val Leu Ser Leu Leu Gly Ile
                85                  90                  95

Cys Leu Arg Ser Glu Gly Ser Pro Leu Val Val Leu Pro Tyr Met Lys
            100                 105                 110

His Gly Asp Leu Arg Asn Phe Ile Arg Asn Glu Thr His Asn Pro Thr
        115                 120                 125

Val Lys Asp Leu Ile Gly Phe Gly Leu Gln Val Ala Lys Gly Met Lys
    130                 135                 140

Tyr Leu Ala Ser Lys Lys Phe Val His Arg Asp Leu Ala Ala Arg Asn
145                 150                 155                 160

Cys Met Leu Asp Glu Lys Phe Thr Val Lys Val Ala Asp Phe Gly Leu

                165                 170                 175

Ala Arg Asp Met Tyr Asp Lys Glu Tyr Tyr Ser Val His Asn Lys Thr
            180                 185                 190

Gly Ala Lys Leu Pro Val Lys Trp Met Ala Leu Glu Ser Leu Gln Thr
        195                 200                 205

Gln Lys Phe Thr Thr Lys Ser Asp Val Trp Ser Phe Gly Val Leu Leu
    210                 215                 220

Trp Glu Leu Met Thr Arg Gly Ala Pro Pro Tyr Pro Asp Val Asn Thr
225                 230                 235                 240

Phe Asp Ile Thr Val Tyr Leu Leu Gln Gly Arg Arg Leu Leu Gln Pro
                245                 250                 255

Glu Tyr Cys Pro Asp Pro Leu Tyr Glu Val Met Leu Lys Cys Trp His
            260                 265                 270

Pro Lys Ala Glu Met Arg Pro Ser Phe Ser Glu Leu Val Ser Arg Ile
        275                 280                 285

Ser Ala Ile Phe Ser Thr Phe Ile Gly Glu His
    290                 295

<210> 6
<211> 31
<212> DNA
<213> synthetic nucleotide primer

<400> 6
gatcccatgg tccacattga cctcagtgct c 31

<210> 7
<211> 40
<212> DNA
<213> synthetic nucleotide primer

<400> 7
ctagaagctt ctagtgctcc ccaatgaaag tagagaagat 40

<210> 8
<211> 15
<212> PRT
<213> synthetic peptide

<400> 8

Ala Arg Asp Met Tyr Asp Lys Glu Tyr Tyr Ser Val His Asn Lys
1               5                   10                  15

<210> 9
<211> 299
<212> PRT
<213> HGFR M1250T Kinase

<400> 9

Met Val His Ile Asp Leu Ser Ala Leu Asn Pro Glu Leu Val Gln Ala
1               5                   10                  15

Val Gln His Val Val Ile Gly Pro Ser Ser Leu Ile Val His Phe Asn
            20                  25                  30

Glu Val Ile Gly Arg Gly His Phe Gly Cys Val Tyr His Gly Thr Leu
        35                  40                  45

Leu Asp Asn Asp Gly Lys Lys Ile His Cys Ala Val Lys Ser Leu Asn
    50                  55                  60

Arg Ile Thr Asp Ile Gly Glu Val Ser Gln Phe Leu Thr Glu Gly Ile
65                  70                  75                  80

Ile Met Lys Asp Phe Ser His Pro Asn Val Leu Ser Leu Leu Gly Ile
                85                  90                  95

Cys Leu Arg Ser Glu Gly Ser Pro Leu Val Val Leu Pro Tyr Met Lys
            100                 105                 110

His Gly Asp Leu Arg Asn Phe Ile Arg Asn Glu Thr His Asn Pro Thr
        115                 120                 125

Val Lys Asp Leu Ile Gly Phe Gly Leu Gln Val Ala Lys Ala Met Lys
    130                 135                 140

Tyr Leu Ala Ser Lys Lys Phe Val His Arg Asp Leu Ala Ala Arg Asn
145                 150                 155                 160

Cys Met Leu Asp Glu Lys Phe Thr Val Lys Val Ala Asp Phe Gly Leu
                165                 170                 175

Ala Arg Asp Met Tyr Asp Lys Glu Tyr Tyr Ser Val His Asn Lys Thr

180                 185                 190

Gly Ala Lys Leu Pro Val Lys Trp Thr Ala Leu Glu Ser Leu Gln Thr
    195                 200                 205

Gln Lys Phe Thr Thr Lys Ser Asp Val Trp Ser Phe Gly Val Leu Leu
  210                 215                 220

Trp Glu Leu Met Thr Arg Gly Ala Pro Pro Tyr Pro Asp Val Asn Thr
225                 230                 235                 240

Phe Asp Ile Thr Val Tyr Leu Leu Gln Gly Arg Arg Leu Leu Gln Pro
                245                 250                 255

Glu Tyr Cys Pro Asp Pro Leu Tyr Glu Val Met Leu Lys Cys Trp His
            260                 265                 270

Pro Lys Ala Glu Met Arg Pro Ser Phe Ser Glu Leu Val Ser Arg Ile
        275                 280                 285

Ser Ala Ile Phe Ser Thr Phe Ile Gly Glu His
    290                 295

<210>  10
<211>  897
<212>  DNA
<213>  HGFR Y 1230C

<400>  10
atggtccaca ttgacctcag tgctctaaat ccagagctgg tccaggcagt gcagcatgta    60

gtgattgggc ccagtagcct gattgtgcat ttcaatgaag tcataggaag agggcatttt   120

ggttgtgtat atcatgggac tttgttggac aatgatggca agaaaattca ctgtgctgtg   180

aaatccttga acagaatcac tgacatagga gaagtttccc aatttctgac cgagggaatc   240

atcatgaaag attttagtca tcccaatgtc ctctcgctcc tgggaatctg cctgcgaagt   300

gaagggtctc cgctggtggt cctaccatac atgaaacatg gagatcttcg aaatttcatt   360

cgaaatgaga ctcataatcc aactgtaaaa gatcttattg gctttggtct tcaagtagcc   420

aaagggatga aatatcttgc aagcaaaaag tttgtccaca gagacttggc tgcaagaaac   480

tgtatgctgg atgaaaaatt cacagtcaag gttgctgatt ttggtcttgc cagagacatg   540

tgtgataaag aatactatag tgtacacaac aaaacaggtg caaagctgcc agtgaagtgg   600

atggctttgg aaagtctgca aactcaaaag tttaccacca agtcagatgt gtggtccttt   660

ggcgtcgtcc tctgggagct gatgacaaga ggagccccac cttatcctga cgtaaacacc 720

tttgatataa ctgtttactt gttgcaaggg agaagactcc tacaacccga atactgccca 780

gaccccttat atgaagtaat gctaaaatgc tggcacccta aagccgaaat gcgcccatcc 840

ttttctgaac tggtgtcccg gatatcagcg atcttctcta ctttcattgg ggagcac 897

<210> 11
<211> 897
<212> DNA
<213> HGFR M1250T

<400> 11
atggtccaca ttgacctcag tgctctaaat ccagagctgg tccaggcagt gcagcatgta 60

gtgattgggc ccagtagcct gattgtgcat ttcaatgaag tcataggaag agggcatttt 120

ggttgtgtat atcatgggac tttgttggac aatgatggca agaaaattca ctgtgctgtg 180

aaatccttga acagaatcac tgacatagga gaagtttccc aatttctgac cgagggaatc 240

atcatgaaag attttagtca tcccaatgtc ctctcgctcc tgggaatctg cctgcgaagt 300

gaagggtctc cgctggtggt cctaccatac atgaaacatg gagatcttcg aaatttcatt 360

cgaaatgaga ctcataatcc aactgtaaaa gatcttattg gctttggtct tcaagtagcc 420

aaaggcatga aatatcttgc gagcaaaaag tttgtccaca gagacttggc tgcaagaaac 480

tgtatgctgg atgaaaaatt cacagtcaag gttgctgatt ttggtcttgc cagagacatg 540

tatgataaag aatactatag tgtacacaac aaaacaggtg caaagctgcc agtgaagtgg 600

accgctttgg aaagtctgca aactcaaaag tttaccacca agtcagatgt gtggtccttt 660

ggcgtgctcc tctgggagct gatgacaaga ggagccccac cttatcctga tgtaaacacc 720

tttgatataa ctgtttactt gttgcaaggg agaagactcc tacaacccga atactgccca 780

gaccccttat atgaagtaat gctaaaatgc tggcacccta aagccgaaat gcgcccatcc 840

ttttctgaac tggtgtcccg gatatcagcg atcttctcta ctttcattgg ggagcac 897

<210> 12
<211> 1281
<212> DNA
<213> Human HGFR intracellular domain

<400> 12
atgggcagtg aattagttcg ctacgatgca agagtacaca ctcctcattt ggataggctt 60

gtaagtgccc gaagtgtaag cccaactaca gaaatggttt caaatgaatc tgtagactac 120

cgagctactt ttccagaaga tcagtttcct aattcatctc agaacggttc atgccgacaa 180

gtgcagtatc ctctgacaga catgtccccc atcctaacta gtggggactc tgatatatcc 240

agtccattac tgcaaaatac tgtccacatt gacctcagtg ctctaaatcc agagctggtc 300

caggcagtgc agcatgtagt gattgggccc agtagcctga ttgtgcattt caatgaagtc 360

ataggaagag ggcattttgg ttgtgtatat catgggactt tgttggacaa tgatggcaag 420

aaaattcact gtgctgtgaa atccttgaac agaatcactg acataggaga agtttcccaa 480

tttctggccg agggaatcat catgaaagat tttagtcatc ccaatgtcct ctcgctcctg 540

ggaatctgcc tgcgaagtga agggtctccg ctggtggtcc taccatacat gaaacatgga 600

gatcttcgaa atttcattcg aaatgagact cataatccaa ctgtaaaaga tcttattggc 660

tttggtcttc aagtagccaa aggcatgaaa tatcttgcaa gcaaaaagtt tgtccacaga 720

gacttggctg caagaaactg tatgctggat gaaaaattca cagtcaaggt tgctgatttt 780

ggtcttgcca gagacatgta tgataaagaa tactatagtg tacacaacaa aacaggtgca 840

aagctgccag tgaagtggat ggctttggaa agtctgcaaa ctcaaaagtt taccaccaag 900

tcagatgtgt ggtcctttgg cgtgctcctc tgggagctga tgacaagagg agccccacct 960

tatcctgatg taaacacctt tgatataact gtttacttgt tgcaagggag aagactccta 1020

caacccgaat actgcccaga ccccttatat gaagtaatgc taaaatgctg gcaccctaaa 1080

gccgaaatgc gcccatcctt ttctgaactg gtgtcccgga tatcagcaat cttctctact 1140

ttcattgggg agcactatgt ccatgtgaac gctacttatg tgaacgtaaa atgtgtcgct 1200

ccatatccat ctctgttgtc atcagaagat aacgctgatg atgaggtgga cacacgacca 1260

gcctccttct gggagacatc a 1281

<210> 13
<211> 427
<212> PRT
<213> Human HGFR intracellular domain protein

<400> 13

Met Gly Ser Glu Leu Val Arg Tyr Asp Ala Arg Val His Thr Pro His
1               5                   10                  15

Leu Asp Arg Leu Val Ser Ala Arg Ser Val Ser Pro Thr Thr Glu Met
            20                  25                  30

Val Ser Asn Glu Ser Val Asp Tyr Arg Ala Thr Phe Pro Glu Asp Gln
        35                  40                  45

Phe Pro Asn Ser Ser Gln Asn Gly Ser Cys Arg Gln Val Gln Tyr Pro
    50                  55                  60

Leu Thr Asp Met Ser Pro Ile Leu Thr Ser Gly Asp Ser Asp Ile Ser
65                  70                  75                  80

Ser Pro Leu Leu Gln Asn Thr Val His Ile Asp Leu Ser Ala Leu Asn
                85                  90                  95

Pro Glu Leu Val Gln Ala Val Gln His Val Val Ile Gly Pro Ser Ser
            100                 105                 110

Leu Ile Val His Phe Asn Glu Val Ile Gly Arg Gly His Phe Gly Cys
        115                 120                 125

Val Tyr His Gly Thr Leu Leu Asp Asn Asp Gly Lys Lys Ile His Cys
    130                 135                 140

Ala Val Lys Ser Leu Asn Arg Ile Thr Asp Ile Gly Glu Val Ser Gln
145                 150                 155                 160

Phe Leu Ala Glu Gly Ile Ile Met Lys Asp Phe Ser His Pro Asn Val
                165                 170                 175

Leu Ser Leu Leu Gly Ile Cys Leu Arg Ser Glu Gly Ser Pro Leu Val
            180                 185                 190

Val Leu Pro Tyr Met Lys His Gly Asp Leu Arg Asn Phe Ile Arg Asn
        195                 200                 205

Glu Thr His Asn Pro Thr Val Lys Asp Leu Ile Gly Phe Gly Leu Gln
    210                 215                 220

Val Ala Lys Gly Met Lys Tyr Leu Ala Ser Lys Lys Phe Val His Arg
225                 230                 235                 240

Asp Leu Ala Ala Arg Asn Cys Met Leu Asp Glu Lys Phe Thr Val Lys
                245                 250                 255

Val Ala Asp Phe Gly Leu Ala Arg Asp Met Tyr Asp Lys Glu Tyr Tyr
            260                 265                 270

Ser Val His Asn Lys Thr Gly Ala Lys Leu Pro Val Lys Trp Met Ala

275                 280                 285

Leu Glu Ser Leu Gln Thr Gln Lys Phe Thr Thr Lys Ser Asp Val Trp
    290                 295                 300

Ser Phe Gly Val Leu Leu Trp Glu Leu Met Thr Arg Gly Ala Pro Pro
305                 310                 315                 320

Tyr Pro Asp Val Asn Thr Phe Asp Ile Thr Val Tyr Leu Leu Gln Gly
                325                 330                 335

Arg Arg Leu Leu Gln Pro Glu Tyr Cys Pro Asp Pro Leu Tyr Glu Val
            340                 345                 350

Met Leu Lys Cys Trp His Pro Lys Ala Glu Met Arg Pro Ser Phe Ser
        355                 360                 365

Glu Leu Val Ser Arg Ile Ser Ala Ile Phe Ser Thr Phe Ile Gly Glu
    370                 375                 380

His Tyr Val His Val Asn Ala Thr Tyr Val Asn Val Lys Cys Val Ala
385                 390                 395                 400

Pro Tyr Pro Ser Leu Leu Ser Ser Glu Asp Asn Ala Asp Asp Glu Val
                405                 410                 415

Asp Thr Arg Pro Ala Ser Phe Trp Glu Thr Ser
            420                 425

<210>  14
<211>  897
<212>  DNA
<213>  HGFR H1094R DNA

<400>  14
atggtccaca ttgacctcag tgctctaaat ccagagctgg tccaggcagt gcagcatgta      60

gtgattgggc ccagtagcct gattgtgcat ttcaatgaag tcataggaag agggcatttt     120

ggttgtgtat atcgtgggac tttgttggac aatgatggca agaaaattca ctgtgctgtg     180

aaatccttga acagaatcac tgacatagga gaagtttccc aatttctgac cgagggaatc     240

atcatgaaag attttagtca tcccaatgtc ctctcgctcc tgggaatctg cctgcgaagt     300

gaagggtctc cgctggtggt cctaccatac atgaaacatg gagatcttcg aaatttcatt     360

cgaaatgaga ctcataatcc aactgtaaaa gatcttattg gctttggtct tcaagtagcc     420

aaagggatga aatatcttgc aagcaaaaag tttgtccaca gagacttggc tgcaagaaac 480

tgtatgctgg atgaaaaatt cacagtcaag gttgctgatt ttggtcttgc cagagacatg 540

tatgataaag aatactatag tgtacacaac aaaacaggtg caaagctgcc agtgaagtgg 600

atggctttgg aaagtctgca aactcaaaag tttaccacca agtcagatgt gtggtccttt 660

ggcgtcgtcc tctgggagct gatgacaaga ggagccccac cttatcctga cgtaaacacc 720

tttgatataa ctgtttactt gttgcaaggg agaagactcc tacaacccga atactgccca 780

gaccccttat atgaagtaat gctaaaatgc tggcacccta aagccgaaat gcgcccatcc 840

ttttctgaac tggtgtcccg gatatcagcg atcttctcta ctttcattgg ggagcac 897

<210> 15
<211> 299
<212> PRT
<213> HGFR H1094R Kinase

<400> 15

Met Val His Ile Asp Leu Ser Ala Leu Asn Pro Glu Leu Val Gln Ala
1               5                   10                  15

Val Gln His Val Val Ile Gly Pro Ser Ser Leu Ile Val His Phe Asn
            20                  25                  30

Glu Val Ile Gly Arg Gly His Phe Gly Cys Val Tyr Arg Gly Thr Leu
        35                  40                  45

Leu Asp Asn Asp Gly Lys Lys Ile His Cys Ala Val Lys Ser Leu Asn
    50                  55                  60

Arg Ile Thr Asp Ile Gly Glu Val Ser Gln Phe Leu Thr Glu Gly Ile
65                  70                  75                  80

Ile Met Lys Asp Phe Ser His Pro Asn Val Leu Ser Leu Leu Gly Ile
                85                  90                  95

Cys Leu Arg Ser Glu Gly Ser Pro Leu Val Val Leu Pro Tyr Met Lys
            100                 105                 110

His Gly Asp Leu Arg Asn Phe Ile Arg Asn Glu Thr His Asn Pro Thr
        115                 120                 125

Val Lys Asp Leu Ile Gly Phe Gly Leu Gln Val Ala Lys Ala Met Lys

130                 135                 140

Tyr Leu Ala Ser Lys Lys Phe Val His Arg Asp Leu Ala Ala Arg Asn
145                 150                 155                 160

Cys Met Leu Asp Glu Lys Phe Thr Val Lys Val Ala Asp Phe Gly Leu
                165                 170                 175

Ala Arg Asp Met Tyr Asp Lys Glu Tyr Tyr Ser Val His Asn Lys Thr
            180                 185                 190

Gly Ala Lys Leu Pro Val Lys Trp Met Ala Leu Glu Ser Leu Gln Thr
        195                 200                 205

Gln Lys Phe Thr Thr Lys Ser Asp Val Trp Ser Phe Gly Val Leu Leu
    210                 215                 220

Trp Glu Leu Met Thr Arg Gly Ala Pro Pro Tyr Pro Asp Val Asn Thr
225                 230                 235                 240

Phe Asp Ile Thr Val Tyr Leu Leu Gln Gly Arg Arg Leu Leu Gln Pro
                245                 250                 255

Glu Tyr Cys Pro Asp Pro Leu Tyr Glu Val Met Leu Lys Cys Trp His
            260                 265                 270

Pro Lys Ala Glu Met Arg Pro Ser Phe Ser Glu Leu Val Ser Arg Ile
        275                 280                 285

Ser Ala Ile Phe Ser Thr Phe Ile Gly Glu His
    290                 295

<210>  16
<211>  299
<212>  PRT
<213>  HGFR Y1230C Kinase

<400>  16

Met Val His Ile Asp Leu Ser Ala Leu Asn Pro Glu Leu Val Gln Ala
1               5                   10                  15

Val Gln His Val Val Ile Gly Pro Ser Ser Leu Ile Val His Phe Asn
            20                  25                  30

Glu Val Ile Gly Arg Gly His Phe Gly Cys Val Tyr His Gly Thr Leu
        35                  40                  45

Leu Asp Asn Asp Gly Lys Lys Ile His Cys Ala Val Lys Ser Leu Asn
    50                  55                  60

Arg Ile Thr Asp Ile Gly Glu Val Ser Gln Phe Leu Thr Glu Gly Ile
65                  70                  75                  80

Ile Met Lys Asp Phe Ser His Pro Asn Val Leu Ser Leu Leu Gly Ile
                85                  90                  95

Cys Leu Arg Ser Glu Gly Ser Pro Leu Val Val Leu Pro Tyr Met Lys
            100                 105                 110

His Gly Asp Leu Arg Asn Phe Ile Arg Asn Glu Thr His Asn Pro Thr
        115                 120                 125

Val Lys Asp Leu Ile Gly Phe Gly Leu Gln Val Ala Lys Ala Met Lys
    130                 135                 140

Tyr Leu Ala Ser Lys Lys Phe Val His Arg Asp Leu Ala Ala Arg Asn
145                 150                 155                 160

Cys Met Leu Asp Glu Lys Phe Thr Val Lys Val Ala Asp Phe Gly Leu
                165                 170                 175

Ala Arg Asp Met Cys Asp Lys Glu Tyr Tyr Ser Val His Asn Lys Thr
            180                 185                 190

Gly Ala Lys Leu Pro Val Lys Trp Met Ala Leu Glu Ser Leu Gln Thr
        195                 200                 205

Gln Lys Phe Thr Thr Lys Ser Asp Val Trp Ser Phe Gly Val Leu Leu
    210                 215                 220

Trp Glu Leu Met Thr Arg Gly Ala Pro Pro Tyr Pro Asp Val Asn Thr
225                 230                 235                 240

Phe Asp Ile Thr Val Tyr Leu Leu Gln Gly Arg Arg Leu Leu Gln Pro
                245                 250                 255

Glu Tyr Cys Pro Asp Pro Leu Tyr Glu Val Met Leu Lys Cys Trp His
            260                 265                 270

Pro Lys Ala Glu Met Arg Pro Ser Phe Ser Glu Leu Val Ser Arg Ile
    275                 280                 285

Ser Ala Ile Phe Ser Thr Phe Ile Gly Glu His
    290                 295

## Description

### CROSS-REFERENCES TO RELATED APPLICATIONS

This application claims the benefit of U.S. provisional application Serial No. 60/277,968, filed March 23, 2001, which is hereby incorporated herein by reference in its entirety.

### FIELD OF THE INVENTION

The present invention generally relates to the isolation and purification of the catalytic domain of the human hepatocyte growth factor receptor kinase (the *Met* protooncogene product, *Met;* HGFR) and its use in the discovery, identification and characterization of inhibitors of same. The present invention further relates to the field of crystallography and, particularly, to X-ray crystallography data useful for identification and construction of therapeutic compounds in the treatment of various disease conditions associated with the *Met* receptor tyrosine kinase. More specifically, the invention relates to crystallized complexes of *Met.*

### BACKGROUND OF THE INVENTION

Hepatocyte growth factor (HGF), also known as scatter factor, is a mesenchymally derived cytokine capable of inducing a variety of pleiotropic effects in normal and neoplastic cells (Sonnenberg et al., *J*. *Cell Biol.* 123:223-235 (1993); Matsumato et al., *Crit. Rev. Oncog.* 3:27-54 (1992); and Stoker et al., *Nature* 327:239-242 (1987)). These include proliferation of different types of epithelial and endothelial cells, dissociation of epithelial colonies into individual cells, stimulation of the motility (scattering) of epithelial cells, induction of epithelial morphogenesis (Montesano et al., *Cell* 67:901-908 (1991)), angiogenesis (Bussolino et al., *J. Cell Biol.* 119:629-641 (1992)), and promotion of the invasion of extracellular matrices (Stella et al., *Int J. Biochem. Cell Biol.* 12:1357-62 (1999)and Stuart et al., *Int J. Exp Path.* 81:17-30 (2000)). *In vivo,* HGF is involved in tissue regeneration, tumor invasion, and embryonic processes, all of which are dependent on both cell motility and proliferation.

HGF initiates these physiologic processes through a high affinity receptor identified as the *c-Met* protooncogene product (Park et al., *Proc. Natl. Acad Sci USA* 84:6379-83 (1987); and Bottaro et al., *Science.* 251:802-4 (1991)). The mature form of the receptor (HGFR) consists of an extracellular α-subunit and a transmembrane β-subunit containing intrinsic tyrosine kinase activity. Engagement of the receptor induces dimerization which in turn up-regulates kinase activity. Activation of *Met* promotes transphosphorylation of several key tyrosine residues responsible for initiating downstream signaling cascades by recruiting multiple effectors (Furge et al., *Oncogene* 19:5582-9 (2000)). These include the p85 subunit of PI3-kinase, phospholipase Cγ (Gaul et al., *Oncogene* 19:1509-18 (2000)), Grb2 and Shc adaptor proteins, the protein phosphatase SHP2 and Gab1. The latter adapter has emerged as the major downstream docking molecule that becomes tyrosine phosphorylated in response to ligand occupancy (Schaeper et al., *J. Cell Biol.* 149:1419-32 (2000); Bardelli, et al., *Oncogene* 18:1139-46 (1999); and Sachs et al., *J. Cell Biol.* 150:1375-84 (2000)). Activation of other signaling molecules has been reported in HGF stimulated cells, most notably, Ras, MAP kinases and FAK (Tanimura et al., *Oncogene* 17:57-65 (1998); and Lai et al., *J*. *Biol. Chem.* 275:7474-80 (2000)). The role for many of these signaling molecules has been established in cell proliferation but is not as evident in cell dissociation and scattering.

The hepatocyte growth factor reecptor (HGFR) is expressed predominantly in epithelial cells but has also been detected in endothelial cells, myoblasts, hematopoietic cells and motor neurons. Inappropriate activation of the receptor is implicated in the onset and progression of a number of tumors and in the promotion of metastasis. A direct link between HGFR and cancer has been shown by the identification of missense mutations in the kinase domain which predispose individuals to papillary renal carcinomas (PRC) and hepatocellular carcinomas (HCC) (Giordano et al., *FASEB J* 14:399-406 (2000)).

Activation of this tyrosine kinase plays a key role in the regulation of migration, invasion and angiogenesis in cancer. The receptor is overexpressed in a significant percentage of human cancers and is amplified during the transition between primary tumors and metastasis. Missense mutations in the tyrosine kinase domain of the gene have been reported in the germline of affected members of PRC and HCC families (Park et al., *Cancer Res.* 59:307-10 (1999); Schmidt et al., *Nature Genetics* 16:68-73 (1997); and Schmidt et al., *Cancer Research* 58:1719-22 (1998)). Most of these genetic lesions represent disease-producing mutations that appear to accelerate carcinogenesis by constitutively activating the receptor. In addition, *in vivo* experiments indicate that autocrine HGF-*Met* signaling plays a significant role in the development and progression of certain malignancies (Bellusci et al., *Oncogene* 9:1091-99 (1994) and Rong et al., *Proc. Natl. Acad. Sci USA* 91:4731-4735 (1994)). It is becoming increasingly evident that the *Met* signaling pathway is involved in the invasive behavior of various tumors by promoting not only tumor spreading, but also neovascularization (Ramirez et al., *Clin. Endocrinol.* 53:635-44 (2000)). Thus, selective, small molecule kinase modulators are expected to have therapeutic potential for the treatment of cancers in which *Met* receptor activation plays a critical role in the development and progression of primary tumors and secondary metastases. Since HGF is also a known angiogenic, there is the potential for this class of modulators to impact angiogenesis-dependent diseases such as diabetic retinopathy.

A direct role for HGFR in the metastatic behavior of human malignancy has been documented in the literature since its initial identification as the cellular homologue of the *tpr-Met* oncogene (Cooper et al., *Nature* 311:29-33 (1984)). The receptor is overexpressed in various tumors including thyroid, ovarian and pancreatic carcinomas and is amplified in liver metastases of colorectal carcinomas (Rong et al., *Cancer Res.* 55:1963-1970 (1995); Rong et al., *Cancer Res.* 53:5355-5360 (1993); Kenworthy et al., *Br. J*. *Cancer* 66:243-247 (1992); and Scarpino et al., *J*. *Pathology* 189:570-575 (1999)). In patients with invasive breast carcinoma, expression of either the receptor or ligand is a predictor of decreased survival, further linking *Met* to tumor progression (Camp et al., *Cancer* 86:2259-65 (1999)). In general, most human tumors and tumor cell lines of mesenchymal origin inappropriately express HGFR and/or HGF. This observation supports the premise that HGFR plays a key role in sarcomagenesis and that both autocrine and paracrine signaling modes contribute to the development of human tumors of mesenchymal origin.

HGFR was originally identified as the cellular counterpart of the *tpr-Met* oncogene, the product of a chromosomal rearrangement generating a chimeric gene by fusing a leucine zipper motif to the tyrosine kinase domain of HGFR (Cooper et al., *Nature* 311:29-33 (1984)). The *tpr-Met* oncogene is activated via the leucine zipper interaction that is responsible for deregulating the enzymatic activity of the kinase domain. Accordingly, the *tpr-Met* oncogene efficiently transforms NIH-3T3 fibroblasts and transgenic expression of this oncogene leads to the development of hyperplasia and tumors in mice (Liang et al., *J. Clin. Invest.* 97:2872-2877 (1996)).

Almost a decade ago, Vande Woude's lab made the observation that mouse NIH 3T3 fibroblasts that overexpress *Met* can induce tumor formation in nude mice via an autocrine mechanism resulting in the interaction between recombinant *Met* receptor and endogenously expressed ligand (Rong et al., *Proc Natl Acad Sci USA* 91:4731-4735 (1994)). They also showed the transformed cells to be metastatic. Since then numerous reports have substantiated the initial observation that chronic *Met*-HGF signaling can induce tumor formation (Oda et al., *Human Pathology* 31:185-192 (2000)). For example, spontaneously transformed tumor cells, which express both ligand and receptor, routinely exhibit increased proliferation and motility. The co-expression of HGF and HGFR is common among non-small-cell lung cancers, especially adenocarcinoma. Not surprisingly, retroviral transduction of HGF in NCI-H358 lung adenocarcinoma cells that express HGFR endows these cells with enhanced capacity to colonize soft agar medium and to form xenograft tumors when implanted in immune-deficient mice (Seung et al., *Neoplasia* 2:226-234 (2000)).

The most compelling data linking HGFR signaling to human malignancies is the discovery of germline missense mutations that map to the kinase domain of HGFR in the majority of hereditary papillary renal cell carcinomas and the detection of somatic missense HGFR mutations in sporadic papillary kidney carcinomas and childhood hepatocellular carcinomas. These mutations which render the receptor constitutively active have been shown to confer an invasive phenotype to transfected cells (Jeffers et al., *Proc. Natl. Acad. Sci. USA* 94:11445-11450 (1997)).

The introduction of these mutations into wild-type *Met* cDNA results in transforming, tumorigenic, and metastatic properties in mouse cell lines. When these same mutations are introduced into mice as transgenes, the founders develop tumors that metastasize to secondary sites. Furthermore, it has been observed that cells carrying these *Met* mutations undergo clonal expansion during HNSCC (head and neck squamous cell carcinoma) progression, further correlating *Met* with the progression of primary cancers to metastasis (Renzo et al., *Oncogene* 19:1547-1555 (2000)).

Direct experimental evidence linking the *Met*-HGF signaling pathway to tumor cell metastasis has been validated in the mouse, using either transfected cells or transgenic animals (Takayama et al., *Proc. Natl. Acad. Sci. USA* 94: 701-706 (1997)). For example, in rigorously controlled experiments, anti-Met oligonucleotides inhibit the proliferation and invasiveness of human gastric cancer cells (Kaji et al., *Cancer Gene Therapy* 3:393-404 (1996)). Dominant negative *Met* has been shown by numerous laboratories to reduce tumorigenicity and spontaneous metastasis both *in vitro* and *in vivo* (Firon et al., *Oncogene* 19:2386-2397 (2000)). *In vivo* a dramatic reduction in tumor and metastasis formation, accompanied by improved survival, was noted. Furthermore, peptides corresponding to the multifunctional docking site at the carboxy terminal tail of the *Met* receptor, that bind the receptor and inhibit kinase activity, inhibit HGF-mediated invasive growth, as measured by cell migration, invasivness, and branched morphogenesis (Bardelli et al., J. *Biol. Chem.* 274:29274-29281 (1999)).

Naturally occurring splice variants of HGF have also been identified that behave as competitive antagonists of mature HGF (Date et al., *Oncogene* 17: 3045-3054 (1998)). These variants inhibit autophosphorylation of the receptor and consequently block HGF-induced migration of human umbilical vein endothelial cells in a migration assay and in an endothelial wounding assay (Jiang et al., *Clinical Cancer Research* **5**:3695-3703 (1999)). HGF antagonists have also been reported to inhibit the motility and invasion of colon, gallbladder and cervical carcinoma cells. Most significantly, infusion of these antagonists into nude mice implanted with tumor cells represses the invasion of tumorigenic cells into surrounding tissues (Kuba et al., *Cancer Research* 60: 6737-6743 (2000)). Similarly, a monoclonal antibody highly selective for HGF has also been found to block tumor progression *in vitro* and *in vivo.* Cao et al., *Proc. Nat.l Acad. Sci. USA* 98:7443-8 (2001).

Recently, the geldanamycin family of anisamycin antibiotics has been implicated in the down-regulation of the HGFR at nanomolar concentrations. The loss of HGFR expression observed at nanomolar concentrations not only inhibits HGF-induced cell motility and invasion but also reverts the Met-transformed phenotype (Webb et al., *Cancer Research* 60: 342-349 (2000)). This class of compounds are currently in clinical trials (NCI) as potential anti-invasive, anti-metastatic agents.

PCT International Publication No. WO 01/09159 discloses nucleic acid ligands to HGF and HGFR. These ligands were isolated using SELEX (Systematic Evolution of Ligands Exponential enrichment). U.S. Patent Nos. 5,686,292 and 6,099,841 report HGFR antagonists and agonists, respectively. U.S. Patent No. 6,174,889 discloses bicyclic heteroaromatic compounds as protein tyrosine kinase inhibitors. PCT International Publication Nos. WO 00/43373, WO 98/07695 and WO 99/15550 disclose protein kinase inhibitor compounds.

Several attempts have been made to elucidate three dimensional structures of proteins to design candidate drugs (See, e.g., Davis et al., *Science* 291:134-137 (2001); Zhu et al., *Structure* 7: 651-661 (1999); and Ymaguchi et al., *Nature* 384: 484-489 (1996)). Further, PCT International Publication No. WO 00/70030 discloses the three-dimensional crystal structure of Lck with its ligand.

### SUMMARY OF THE INVENTION

The generation, kinetic characterization, and structure determination of the kinase domain of the human HGFR protein is disclosed herein. The domain begins between residues 1051 and 1078 and terminates between residues 1341 and 1348 of the full-length protein [SEQ ID NO: 2]. The domain preferably extends from residues 1051-1341, and more preferably from residues 1051-1348. In one embodiment of the present invention, the domain has the sequence selected from the group of SEQ ID NOS: 3 through 5, 9, 13, 15, and 16.

In one of its aspects, the present invention relates to an isolated polynucleotide that encodes the human hepatocyte growth factor receptor or the human hepatocyte gowth factor receptor kinase domain, or a fragment or variant thereof. In one embodiment, the nucleotide sequence of the polynucleotide corresponds to at least bases 3342 to 4206 of SEQ ID NO: 1. In other embodiments, the nucleotide sequence of the polynucleotide corresponds to the sequence of SEQ ID NOS: 10, 11, 12, or 14.

In another of its aspects, the present invention relates to a crystal structure containing the human hepatocyte growth factor receptor kinase. In one embodiment, the amino acid sequence of the kinase corresponds to at least amino acids 1051 to 1348 of SEQ ID NO: 2. In other embodiments, the amino acid sequence of the kinase corresponds to the sequence of SEQ ID NOS: 3, 4, 5, 6, 7, 8, 9, 13, 15, or 16.

In still another of its aspects, the present invention relates to an isolated polypeptide containing the human hepatocyte growth factor receptor or human hepatocyte growth factor receptor kinase domain, or a variant thereof. In one embodiment, the human hepatocyte growth factor receptor or human hepatocyte growth factor receptor kinase domain contains a deletion that imparts favorable physical characteristics to the resulting polypeptide (e.g., suitability for analysis by nuclear magnetic resonance, suitability for high throughput screening, suitability for biochemical characterizations, suitability for x-ray crystallography, suitability for colorimetry and suitability for other diagnostic methods). In other embodiments, the polypeptide contains amino acids 1051 to 1341 of the sequence as set forth in SEQ ID NO. 2; amino acids 1051 to 1348 of the sequence as set forth in SEQ ID NO. 2; or the amino acid sequence as set forth in SEQ ID NOS. 3, 4, 5, 6, 7, 8, 9, 13, 15, or 16; or a conservatively substituted variant thereof.

In yet another of its aspects, the present invention relates to an isolated polynucleotide that encodes the catalytically active form of the human hepatocyte growth factor receptor or human hepatocyte growth factor receptor kinase domain, or a fragment or variant thereof.

A further aspect of the present invention relates to an isolated catalytically active polypeptide comprising the human hepatocyte growth factor receptor or human hepatocyte growth factor receptor kinase domain, or a variant thereof.

Another aspect of the present invention relates to an isolated polynucleotide which encodes the catalytic domain of the human hepatocyte growth factor receptor kinase, or a fragment or variant thereof.

Still another aspect of the present invention relates to an isolated catalytically active polypeptide containing the catalytic domain of the human hepatocyte growth factor receptor kinase or a variant thereof.

Yet another aspect of the present invention relates to an isolated soluble polypeptide comprising the catalytic domain of the human hepatocyte growth factor receptor kinase or a variant thereof.

The present invention also relates to an expression vector for producing the human hepatocyte growth factor receptor kinase in a host cell. The vector contains a polynucleotide encoding the human hepatocyte growth factor receptor kinase or a variant thereof; and regulatory sequences that are functional in the host cell and operably linked to the polynucleotide. In one embodiment, the polynucleotide encodes the active human hepatocyte growth factor receptor kinase containing bases 3342 to 4206 of SEQ ID NO: 1. In another embodiment, the vector is selected from the group consisting of pET28a, pAcSG2, and pFastBac. In a further embodiment, the host cell is *E. coli.*

In another aspect, the present invention relates to a host cell transformed or transfected with a polynucleotide encoding the human hepatocyte growth factor receptor kinase or a variant thereof. In one embodiment, the host cell is transformed or transfected with the polynucleotide via an expression vector containing the polynucleotide; a regulatory sequence functional in the host cell operably linked to the polynucleotide; and a selectable marker. The expression vector can be, for example, pET28a, pAcSG2, and pFastBac. In another embodiment, the polynucleotide encodes the human hepatocyte growth factor receptor kinase containing bases 3342 to 4206 of SEQ ID NO: 1. In a further embodiment, the host cell is *E. coli.* In yet another embodiment, the host cell is infected with a recombinant baculovirus. Additionally, the host cell can be is insect cell, such as Sf9.

The present invention additionally relates to a method of producing a polypeptide or variant thereof by culturing a host cell, transformed or transfected with a polynucleotide encoding the human hepatocyte growth factor receptor kinase or a variant thereof, under conditions such that the polypeptide or variant thereof is expressed; and recovering the polypeptide or variant.

In yet another of its aspects, the present invention relates to a method for assaying a candidate compound for its ability to interact with the human hepatocyte growth factor receptor. The method involves expressing an isolated DNA sequence or variant thereof encoding the kinase domain of the human hepatocyte growth factor receptor in a host capable of producing the kinase in a form which may be assayed for interaction with the candidate compound. The kinase is exposed to the candidate compound and the interaction of the kinase with the candidate compound is evaluated. In one embodiment, the interaction is evaluated by crystallizing the kinase in a condition suitable for x-ray crystallography; and conducting x-ray crystallography on the kinase. The results of the x-ray crystallography are optionally used to determine the three dimensional molecular structure of the configuration of human hepatocyte growth factor receptor kinase and the binding pockets thereof.

The present invention further relates to a crystal structure containing a polypeptide encoded by a polynucleotide which encodes the human hepatocyte growth factor receptor kinase domain, or a fragment or variant thereof.

In addition, the present invention relates to a crystal structure containing a polypeptide encoded by a polynucleotide which encodes the human hepatocyte growth factor receptor kinase domain, or a fragment or variant thereof, and a ligand complexed thereto. In one embodiment, the ligand modulates the activity of human hepatocyte growth factor kinase. In another embodiment, the ligand is a compound of the formula:

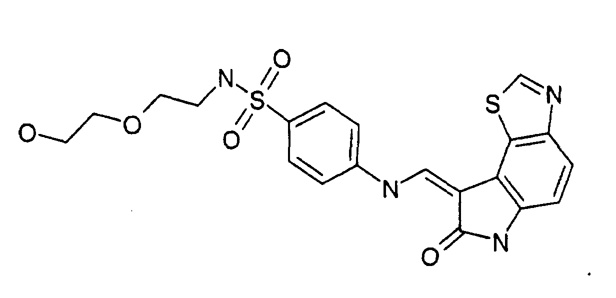

The present invention still further relates to a process of drug design for compounds which interact with the human hepatocyte growth factor receptor kinase. The process involves crystallizing the human hepatocyte growth factor receptor kinase and resolving the x-ray crystallography of the kinase. The data generated from resolving the x-ray crystallography of the kinase is then applied to a computer algorithm which generates a model of the kinase suitable for use in designing molecules that will act as agonists or antagonists to the polypeptide. An interative process is then applied whereby various molecular structures are applied to the computer-generated model to identify potential agonists or antagonists of the kinase. In one embodiment, the process is utilized to identify modulators of the active kinase, which serve as lead compounds for the design of potentially therapeutic compounds for the treatment of diseases or disorders associated with the hepatocyte growth factor receptor- hepatocyte growth factor signaling pathway.

In yet another aspect, the present invention relates to a method of rapidly screening large compound libraries to identify compounds that inhibit human hepatocyte growth factor receptor kinase containing a non-radioactive immunosorbent assay capable of robotic control. In one embodiment, the assay is DELFIA.

In still another aspect, the present invention relates to a method of assessing compounds which are agonists or antagonists of the activity of the hepatocyte growth factor receptor kinase by crystallizing the hepatocyte growth factor receptor kinase and obtaining crystallography coordinates for the crystallized hepatocyte growth factor receptor kinase. The crystallography coordinates are then applied to a computer algorithm such that the algorithm generates a model of the kinase suitable for use in designing molecules that will act as agonists or antagonists to the kinase. An iterative process is used to apply various molecular structures to the computer-generated model to identify potential agonists or antagonists to the kinase. The agonist or antagonist is then optionally synthesized or obtained, and contacted with the molecule to determine the ability of the potential agonist or antagonist to interact with the molecule.

The present invention also relates to a method for determining the three-dimensional structure of a complex of hepatocyte growth factor receptor kinase with a ligand, wherein x-ray diffraction data for crystals of the complex are obtained, and the set of atomic coordinates of Table 1 or portions thereof; and coordinates having a root mean square deviation therefrom with respect to conserved protein backbone atoms of not more than about 1.5 Å are used to define the three-dimensional structure of the complex.

In a further of its aspects, the present invention relates to a method of using a three-dimensional structure of a polypeptide encoded by a polynucleotide which encodes the human hepatocyte growth factor receptor and a compound of the formula: 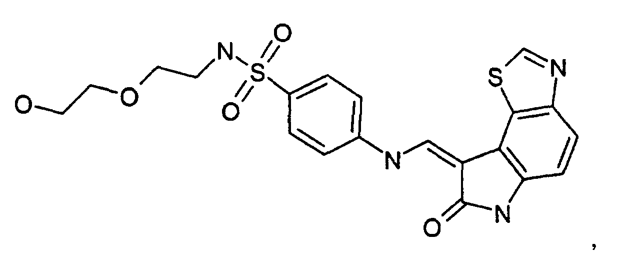 as defined by the structure coordinates of Table 1, or a portion thereof, in a drug-discovery strategy. A potential drug is selected, in conjunction with computer modeling, by performing rational drug design with the three-dimensional structure determined from one or more sets of atomic coordinates in Table 1. The potential drug is contacted with a polypeptide containing a functional human hepatocyte growth factor receptor and the binding of the potential drug with the polypeptide is determined.

The present invention still further relates to a method of using a three-dimensional structure of a polypeptide encoded by a polynucleotide which encodes the human hepatocyte growth factor receptor kinase domain and a compound of the formula: 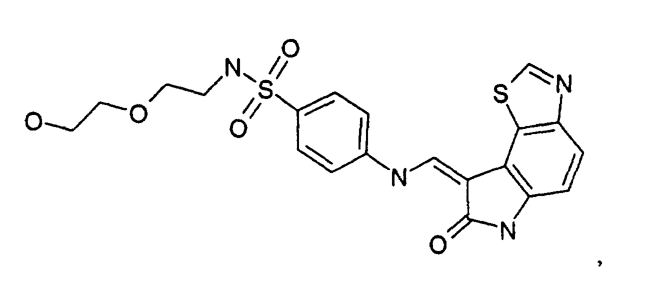 as defined by the structure coordinates of Table 1, or a portion thereof, in a drug-discovery strategy. A potential drug is selcted, in conjunction with computer modeling, by performing rational drug design with the three-dimensional structure determined from one or more sets of atomic coordinates in Table 1. The potential drug is contacted with a polypeptide containing a functional human hepatocyte growth factor receptor. Whether or not the potential drug modulates the activity of the polypeptide is then determined.

The present invention further relates to a method for evaluating the potential of a chemical entity to associate with either: (a) a molecule or molecular complex having a binding pocket defined by structure coordinates of human hepatocyte growth factor receptor amino acids 1082-1086, 1091-1094, 1107-1110, 1140-1142, 1155-1175, 1208-1213, and 1219-1231, according to Table 1, or (b) a homologue of the molecule or molecular complex having a binding pocket that has a root mean square deviation from the backbone atoms of the amino acids of not more than about 1.5 Å. The method involves employing computational means to perform a fitting operation between the chemical entity and a binding pocket defined by structure coordinates of hepatocyte growth factor receptor amino acids 1082-1086, 1091-1094, 1107-1110, 1140-1142, 1155-1175, 1208-1213, and 1219-1231which are within about a root mean square deviation of not more than about 1.5 Å from the backbone atoms of said amino acids according to Table 1; and analyzing the results of the fitting operation to quantify the association between the chemical entity and the binding pocket.

In yet another of its aspects, the present invention relates to a computer for producing a three-dimensional representation of: (a) a molecule or molecular complex, wherein said molecule or molecular complex comprises a binding pocket defined by the structure coordinates of hepatocyte growth factor receptor kinase amino acids 1082-1086, 1091-1094, 1107-1110, 1140-1142, 1155-1175, 1208-1213, and 1219-1231, according to Table 1; or (b) a homologue of said molecule or molecular complex, wherein said homologue comprises a binding pocket that has a root mean square deviation from the backbone atoms of said amino acids of not more than about 1.5 Å. The computer comprises includes a computer-readable data storage medium having a data storage material encoded with computer-readable data containing the structure coordinates of hepatocyte growth factor kinase amino acids 1082-1086, 1091-1094, 1107-1110, 1140-1142, 1155-1175, 1208-1213, and 1219-1231, according to Table 1. The computer also includes a working memory for storing instructions for processing the computer-readable data; a central-processing unit coupled to the working memory and to the computer-readable data storage medium for processing the computer-machine readable data into the three-dimensional representation; and a display coupled to the central-processing unit for displaying the three-dimensional representation. In one embodiment, the computer produces a three-dimensional representation of: (a) a molecule or molecular complex defined by structure coordinates of all of the hepatocyte growth factor kinase amino acids set forth in Table 1, or (b) a homologue of the molecule or molecular complex having a binding pocket that has a root mean square deviation from the backbone atoms of the amino acids of not more than 1.5 Å.

The present invention also relates to a computer for determining at least a portion of the structure coordinates corresponding to the x-ray diffraction data obtained from a molecule or molecular complex. The computer includes a computer-readable data storage medium having a data storage material encoded with machine-readable data, wherein the data includes at least a portion of the structural coordinates of hepatocyte growth factor receptor kinase according to Table 1. The computer also includes a computer-readable data storage medium having a data storage material encoded with computer-readable data including x-ray diffraction data obtained from the molecule or molecular complex; a working memory for storing instructions for processing the computer-readable data; a central-processing unit coupled to the working memory and to the computer-readable data storage medium for performing a Fourier transform of the machine readable data and for processing the computer-readable data into structure coordinates; and a display coupled to the central-processing unit for displaying the structure coordinates of the molecule or molecular complex.

In still another aspect, the present invention relates to a computer readable medium having stored thereon data of the structure coordinates of a *Met* ligand-binding site including 1082-1086, 1091-1094, 1107-1110, 1140-1142, 1155-1175, 1208-1213, and 1219-1231 according to Table 1.

### BRIEF DESCRIPTION OF THE DRAWINGS

The numerous objects and advantages of the present invention may be better understood by those skilled in the art by reference to the accompanying detailed description and the following drawings. The application file contains at least one drawing executed in color. Copies of this patent application publication with color drawings will be provided by the Office upon request and payment of the necessary fee.
Figure 1 is a purification scheme of the human HGFRkd;
Figure 2 is a ribbon representation of the kinase domain of the HGFR protein structure with Compound **1** bound thereto, wherein the N- and C-termini are indicated by N and C, respectively. Colors: Compound **1** (purple), Glycine-rich loop (orange), activation loop (yellow), alpha helix C (green), kinase insert domain (red), and remainder of protein (blue);
Figure 3 is a ribbon representation of the kinase domain of the HGFR kinase activation loop. Colors: activation loop (purple), Glycine-rich loop (yellow), alpha helix C (red), Phenylalanine 1089 (light blue), Aspartic acid 1228 (green), and Tyrosine-1230 (dark blue);
Figure 4 is an atomic representation of the Compound **1**-HGFR kinase domain binding area, wherein the positions of bound water molecules are shown as red crosshairs. Colors: carbon (green), nitrogen (blue), oxygen (red), and sulfur (yellow);
Figure 5(A) is a Coomassie stained isoelectric focussing (IEF) electrophoretic evaluation of a time-course (20 °C) of HGFR autophosphorylation;
Figure 5(B) is an autoradiogram of IEF gel;
Figure 5(C) is a kinetic evaluation of the activation time course at 4°C;
Figure 5(D) is a MALDI-TOF evaluation of HGFR and pHGFR peptides derived from an exhaustive tryptic digest;
Figure 5(E) is a parent ion scan by nano-ESI-MS trypsin proteolyzed HGFR;
Figure 6(A) is a graph showing inhibition of HGFR and pHGFR by Compound **1** as measured in the coupled enzymatic assay; and
Figure 6(B) is a graph showing double reciprocal analysis of Compound **1** inhibition of pHGFR.

### DETAILED DESCRIPTION OF THE INVENTION AND PREFERRED EMBODIMENTS

The terms "comprising" and "including" are used herein in their open, non-limiting sense.

The catalytic domain of the human HGFR receptor kinase facilitated crystallography, enzyme characterization, and high throughput screening of inhibitors. In particular, the catalytic domain of the HGFR kinase domain was used to determine its three-dimensional structure, which provides unique structural information useful for drug design.

As used herein, the abbreviation 'HGFR' or *'Met'* refers to the polynucleotide encoding the hepatocyte growth factor receptor tyrosine kinase, or the protein *per se.* Also, the abbreviation 'hHGFR' or 'human *Met',* as used herein, refers to the polynucleotide encoding the human hepatocyte growth factor receptor tyrosine kinase or the protein *per se.* The HGFR protein is sometimes referred to as HGFR tyrosine kinase or HGFR kinase throughout the application. The nucleic acid sequence of the polynucleotide encoding the full-length protein of hHGFR was published by Park et al. (*Proc. Natl. Acad. Sci.USA* 84: 6379-83 (1987)) and submitted to GenBank under the accession number NM_000245. The nucleic acid sequence described therein is provided herein, as SEQ ID NO: 1. The corresponding peptide sequence of the full-length protein is provided herein, as SEQ ID NO: 2. This peptide sequence was submitted to GenBank by Park et al. and assigned Accession number P08581. The intracellular domain of HGFR is provided herein as SEQ ID NO. 12.

SEQ ID NO. 1:
cgccctcgcc gcccgcggcg ccccgagcgc tttgtgagca gatgcggagc cgagtggagg
gcgcgagcca gatgcggggc gacagctgac ttgctgagag gaggcgggga ggcgcggagc
gcgcgtgtgg tccttgcgcc gctgacttct ccactggttc ctgggcaccg aaagataaac
ctctcataat gaaggccccc gctgtgcttg cacctggcat cctcgtgctc ctgtttacct
tggtgcagag gagcaatggg gagtgtaaag aggcactagc aaagtccgag atgaatgtga
atatgaagta tcagcttccc aacttcaccg cggaaacacc catccagaat gtcattctac
atgagcatca cattttcctt ggtgccacta actacattta tgttttaaat gaggaagacc
ttcagaaggt tgctgagtac aagactgggc ctgtgctgga acacccagat tgtttcccat
gtcaggactg cagcagcaaa gccaatttat caggaggtgt ttggaaagat aacatcaaca
tggctctagt tgtcgacacc tactatgatg atcaactcat tagctgtggc agcgtcaaca
gagggacctg ccagcgacat gtctttcccc acaatcatac tgctgacata cagtcggagg
ttcactgcat attctcccca cagatagaag agcccagcca gtgtcctgac tgtgtggtga
gcgccctggg agccaaagtc ctttcatctg taaaggaccg gttcatcaac ttctttgtag
gcaataccat aaattcttct tatttcccag atcatccatt gcattcgata tcagtgagaa
ggctaaagga aacgaaagat ggttttatgt ttttgacgga ccagtcctac attgatgttt
tacctgagtt cagagattct taccccatta agtatgtcca tgcctttgaa agcaacaatt

ttatttactt cttgacggtc caaagggaaa ctctagatgc tcagactttt cacacaagaa
taatcaggtt ctgttccata aactctggat tgcattccta catggaaatg cctctggagt
gtattctcac agaaaagaga aaaaagagat ccacaaagaa ggaagtgttt aatatacttc
aggctgcgta tgtcagcaag cctggggccc agcttgctag acaaatagga gccagcctga
atgatgacat tcttttcggg gtgttcgcac aaagcaagcc agattctgcc gaaccaatgg
atcgatctgc catgtgtgca ttccctatca aatatgtcaa cgacttcttc aacaagatcg
tcaacaaaaa caatgtgaga tgtctccagc atttttacgg acccaatcat gagcactgct
ttaataggac acttctgaga aattcatcag gctgtgaagc gcgccgtgat gaatatcgaa
cagagtttac cacagctttg cagcgcgttg acttattcat gggtcaattc agcgaagtcc
tcttaacatc tatatccacc ttcattaaag gagacctcac catagctaat cttgggacat
cagagggtcg cttcatgcag gttgtggttt ctcgatcagg accatcaacc cctcatgtga
attttctcct ggactcccat ccagtgtctc cagaagtgat tgtggagcat acattaaacc
aaaatggcta cacactggtt atcactggga agaagatcac gaagatccca ttgaatggct
tgggctgcag acatttccag tcctgcagtc aatgcctctc tgccccaccc tttgttcagt
gtggctggtg ccacgacaaa tgtgtgcgat cggaggaatg cctgagcggg acatggactc
aacagatctg tctgcctgca atctacaagg ttttcccaaa tagtgcaccc cttgaaggag
ggacaaggct gaccatatgt ggctgggact ttggatttcg gaggaataat aaatttgatt
taaagaaaac tagagttctc cttggaaatg agagctgcac cttgacttta agtgagagca
cgatgaatac attgaaatgc acagttggtc ctgccatgaa taagcatttc aatatgtcca
taattatttc aaatggccac gggacaacac aatacagtac attctcctat gtggatcctg
taataacaag tatttcgccg aaatacggtc ctatggctgg tggcacttta cttactttaa
ctggaaatta cctaaacagt gggaattcta gacacatttc aattggtgga aaaacatgta
ctttaaaaag tgtgtcaaac agtattcttg aatgttatac cccagcccaa accatttcaa
ctgagtttgc tgttaaattg aaaattgact tagccaaccg agagacaagc atcttcagtt
accgtgaaga tcccattgtc tatgaaattc atccaaccaa atcttttatt agtacttggt
ggaaagaacc tctcaacatt gtcagttttc tattttgctt tgccagtggt gggagcacaa
taacaggtgt tgggaaaaac ctgaattcag ttagtgtccc gagaatggtc ataaatgtgc
atgaagcagg aaggaacttt acagtggcat gtcaacatcg ctctaattca gagataatct
gttgtaccac tccttccctg caacagctga atctgcaact ccccctgaaa accaaagcct
ttttcatgtt agatgggatc ctttccaaat actttgatct catttatgta cataatcctg
tgtttaagcc ttttgaaaag ccagtgatga tctcaatggg caatgaaaat gtactggaaa
ttaagggaaa tgatattgac cctgaagcag ttaaaggtga agtgttaaaa gttggaaata
agagctgtga gaatatacac ttacattctg aagccgtttt atgcacggtc cccaatgacc
tgctgaaatt gaacagcgag ctaaatatag agtggaagca agcaatttct tcaaccgtcc
ttggaaaagt aatagttcaa ccagatcaga atttcacagg attgattgct ggtgttgtct
caatatcaac agcactgtta ttactacttg ggtttttcct gtggctgaaa aagagaaagc
aaattaaaga tctgggcagt gaattagttc gctacgatgc aagagtacac actcctcatt
tggataggct tgtaagtgcc cgaagtgtaa gcccaactac agaaatggtt tcaaatgaat
ctgtagacta ccgagctact tttccagaag atcagtttcc taattcatct cagaacggtt
catgccgaca agtgcagtat cctctgacag acatgtcccc catcctaact agtggggact
ctgatatatc cagtccatta ctgcaaaata ctgtccacat tgacctcagt gctctaaatc
cagagctggt ccaggcagtg cagcatgtag tgattgggcc cagtagcctg attgtgcatt
tcaatgaagt cataggaaga gggcattttg gttgtgtata tcatgggact ttgttggaca
atgatggcaa gaaaattcac tgtgctgtga aatccttgaa cagaatcact gacataggag
aagtttccca atttctgacc gagggaatca tcatgaaaga ttttagtcat cccaatgtcc

tctcgctcct gggaatctgc ctgcgaagtg aagggtctcc gctggtggtc ctaccataca
tgaaacatgg agatcttcga aatttcattc gaaatgagac tcataatcca actgtaaaag
atcttattgg ctttggtctt caagtagcca aagcgatgaa atatcttgca agcaaaaagt
ttgtccacag agacttggct gcaagaaact gtatgctgga tgaaaaattc acagtcaagg
ttgctgattt tggtcttgcc agagacatgt atgataaaga atactatagt gtacacaaca
aaacaggtgc aaagctgcca gtgaagtgga tggctttgga aagtctgcaa actcaaaagt
ttaccaccaa gtcagatgtg tggtcctttg gcgtcgtcct ctgggagctg atgacaagag
gagccccacc ttatcctgac gtaaacacct ttgatataac tgtttacttg ttgcaaggga
gaagactcct acaacccgaa tactgcccag accccttata tgaagtaatg ctaaaatgct
ggcaccctaa agccgaaatg cgcccatcct tttctgaact ggtgtcccgg atatcagcga
tcttctctac tttcattggg gagcactatg tccatgtgaa cgctacttat gtgaacgtaa
aatgtgtcgc tccgtatcct tctctgttgt catcagaaga taacgctgat gatgaggtgg
acacacgacc agcctccttc tgggagacat catagtgcta gtactatgtc aaagcaacag
tccacacttt gtccaatggt tttttcactg cctgaccttt aaaaggccat cgatattctt
tgctccttgc cataggactt gtattgttat ttaaattact ggattctaag gaatttctta
tctgacagag catcagaacc agaggcttgg tcccacaggc cagggaccaa tgcgctgcag

SEQ ID NO. 2:


























SEQ ID NO. 12:

atgggcagtgaattagttcgctacgatgcaagagtacacactcctcatttggataggcttgtaagtgcccgaagtgtaagcccaactaca
gaaatggtttcaaatgaatctgtagactaccgagctacttttccagaagatcagtttcctaattcatctcagaacggttcatgccgacaagt
gcagtatcctctgacagacatgtcccccatcctaactagtggggactctgatatatccagtccattactgcaaaatactgtccacattgacc
tcagtgctctaaatccagagctggtccaggcagtgcagcatgtagtgattgggcccagtagcctgattgtgcatttcaatgaagtcatag
gaagagggcattttggttgtgtatatcatgggactttgttggacaatgatggcaagaaaattcactgtgctgtgaaatccttgaacagaatc
actgacataggagaagtttcccaatttctggccgagggaatcatcatgaaagattttagtcatcccaatgtcctctcgctcctgggaatctg
cctgcgaagtgaagggtctccgctggtggtcctaccatacatgaaacatggagatcttcgaaatttcattcgaaatgagactcataatcca
actgtaaaagatcttattggctttggtcttcaagtagccaaaggcatgaaatatcttgcaagcaaaaagtttgtccacagagacttggctgc
aagaaactgtatgctggatgaaaaattcacagtcaaggttgctgattttggtcttgccagagacatgtatgataaagaatactatagtgtac
acaacaaaacaggtgcaaagctgccagtgaagtggatggctttggaaagtctgcaaactcaaaagtttaccaccaagtcagatgtgtg
gtcctttggcgtgctcctctgggagctgatgacaagaggagccccaccttatcctgatgtaaacacctttgatataactgtttacttgttgca
agggagaagactcctacaacccgaatactgcccagaccccttatatgaagtaatgctaaaatgctggcaccctaaagccgaaatgcgc
ccatccttttctgaactggtgtcccggatatcagcaatcttctctactttcattggggagcactatgtccatgtgaacgctacttatgtgaacg
taaaatgtgtcgctccatatccatctctgttgtcatcagaagataacgctgatgatgaggtggacacacgaccagcctccttctgggagac
atca

As used herein, the abbreviation 'HGFRkd' refers to the catalytic domain of the hHGFR, said domain beginning between residues 1051 and 1078 and terminating between residues 1341 and 1348 of the full-length protein [SEQ ID NO: 2]. According to certain embodiments of the present invention: (1) a methionine residue is added to the very N-terminal of the HGFRkd sequence [SEQ ID NO: 3]; (2) residues 1051-1349 of the hepatocyte growth factor receptor precursor wherein a methionine residue has been added to the very N-terminus of the sequence, the glycine at residue 1191 has been replaced by alanine, and the valine at position 1272 has been replaced by leucine [SEQ ID NO. 4]; (3) the valine at position 1272 is replaced by leucine [SEQ ID NO. 5]; (4) the methionine at residue 1250 is replaced by threonine [SEQ ID NO. 9; a naturally occurring variant in hepatocellular carcinoma (HPRC)]; (5) the histidine at residue 1094 is replaced by arginine [SEQ ID NO. 15]; and/or (6) the tyrosine at residue 1230 is replaced by cysteine [SEQ ID NO. 16].

SEQ ID NO. 3:

VHIDLSALN PELVQAVQHV VIGPSSLIVH FNEVIGRGHF GCVYHGTLLD
NDGKKIHCAV KSLNRITDIG EVSQFLTEGI IMKDFSHPNV LSLLGICLRS EGSPLVVLPY
MKHGDLRNFI RNETHNPTVK DLIGFGLQVA KGMKYLASKK FVHRDLAARN
CMLDEKFTVK VADFGLARDM YDKEYYSVHN KTGAKLPVKW MALESLQTQK
FTTKSDVWSF GVVLWELMTR GAPPYPDVNT FDITVYLLQG RRLLQPEYCP
DPLYEVMLKC WHPKAEMRPS FSELVSRISA IFSTFIGEH

SEQ ID NO.: 4

MVHIDLSALN PELVQAVQHV VIGPSSLIVH FNEVIGRGHF GCVYHGTLLD
NDGKKIHCAV KSLNRITDIG EVSQFLTEGI IMKDFSHPNV LSLLGICLRS
EGSPLVVLPY MKHGDLRNFI RNETHNPTVK DLIGFGLQVA KAMKYLASKK
FVHRDLAARN CMLDEKFTVK VADFGLARDM YDKEYYSVHN KTGAKLPVKW
MALESLQTQK FTTKSDVWSF GVLLWELMTR GAPPYPDVNT FDITVYLLQG
RRLLQPEYCP DPLYEVMLKC WHPKAEMRPS FSELVSRISA IFSTFIGEH

SEQ ID NO.: 5

MVHIDLSALN PELVQAVQHV VIGPSSLIVH FNEVIGRGHF GCVYHGTLLD
NDGKKIHCAV KSLNRITDIG EVSQFLTEGI IMKDFSHPNV LSLLGICLRS
EGSPLVVLPY MKHGDLRNFI RNETHNPTVK DLIGFGLQVA KGMKYLASKK
FVHRDLAARN CMLDEKFTVK VADFGLARDM YDKEYYSVHN KTGAKLPVKW
MALESLQTQK FTTKSDVWSF GVLLWELMTR GAPPYPDVNT FDITVYLLQG
RRLLQPEYCP DPLYEVMLKC WHPKAEMRPS FSELVSRISA IFSTFIGEH

SEQ ID NO.: 9

MVHIDLSALN PELVQAVQHV VIGPSSLIVH FNEVIGRGHF GCVYHGTLLD
NDGKKIHCAV KSLNRITDIG EVSQFLTEGI IMKDFSHPNV LSLLGICLRS
EGSPLVVLPY MKHGDLRNFI RNETHNPTVK DLIGFGLQVA KAMKYLASKK
FVHRDLAARN CMLDEKFTVK VADFGLARDM YDKEYYSVHN KTGAKLPVKW
TALESLQTQK FTTKSDVWSF GVLLWELMTR GAPPYPDVNT FDITVYLLQG
RRLLQPEYCP DPLYEVMLKC WHPKAEMRPS FSELVSRISA IFSTFIGEH

SEQ ID NO.: 15:
MVHIDLSALN PELVQAVQHV VIGPSSLIVH FNEVIGRGHF GCVYRGTLLD
NDGKKIHCAV KSLNRITDIG EVSQFLTEGI IMKDFSHPNV LSLLGICLRS
EGSPLVVLPY MKHGDLRNFI RNETHNPTVK DLIGFGLQVA KAMKYLASKK
FVHRDLAARN CMLDEKFTVK VADFGLARDM YDKEYYSVHN KTGAKLPVKW
MALESLQTQK FTTKSDVWSF GVLLWELMTR GAPPYPDVNT FDITVYLLQG
RRLLQPEYCP DPLYEVMLKC WHPKAEMRPS FSELVSRISA IFSTFIGEH

SEQ ID NO.: 16

MVHIDLSALN PELVQAVQHV VIGPSSLIVH FNEVIGRGHF GCVYHGTLLD
NDGKKIHCAV KSLNRITDIG EVSQFLTEGI IMKDFSHPNV LSLLGICLRS
EGSPLVVLPY MKHGDLRNFI RNETHNPTVK DLIGFGLQVA KAMKYLASKK
FVHRDLAARN CMLDEKFTVK VADFGLARDM CDKEYYSVHN KTGAKLPVKW
MALESLQTQK FTTKSDVWSF GVLLWELMTR GAPPYPDVNT FDITVYLLQG
RRLLQPEYCP DPLYEVMLKC WHPKAEMRPS FSELVSRISA IFSTFIGEH

As used herein, the abbreviation 'pHGFR' refers to phosphorylated HGFR.

### A. Peptides, Proteins and Antibodies

The present invention provides isolated peptide and protein molecules that are comprised of, consist of, or consist essentially of the amino acid sequences of the kinase peptides encoded by the nucleic acid sequence disclosed in the SEQ ID NO: 1, as well as all obvious variants of these peptides that are within the art to make and use. Some of these variants are described in detail below.

As used herein, the terms "kinase", "kinase peptide" and "protein kinase" refer to enzymes that catalyze the transfer of a phosphate residue from a nucleoside triphosphate to an amino acid side chain in selected targets. The covalent phosphorylation in turn regulates the activity of the target protein. In addition, phosphorylation frequently acts as the signal that triggers a particular process or reaction, playing an integral part in cellular regulation and control mechanisms. Inappropriate or unregulated phosphorylation can result in errors in cell signaling and the associated cell cycle and regulation processes. Most protein kinases are highly substrate specific. Those that have the ability to phosphorylate numerous substrates frequently turn out to be oncogenes, genes that are associated with neoplastic transformation of a cell.

As used herein, the term "catalytically active form" refers to any form of peptides or proteins exhibiting intrinsic enzymatic activity. Preferably, the term "catalytically active form" refers to peptides or proteins capable of autophosphorylation.

As used herein, a peptide is said to be "isolated" or "purified" when it is free or substantially free of cellular material and/or free or substantially free of chemical precursors or other chemicals. The peptides of the present invention can be purified to homogeneity or other degrees of purity. The level of purification will be based primarily on the intended use. The critical feature is that the preparation allows for the desired function of the peptide, even if in the presence of considerable amounts of other components.

In some uses, "substantially free of cellular material" includes preparations of the peptide having less than about 30% (by dry weight) other proteins (i.e., contaminating protein), preferably less than about 20% other proteins, more preferably less than about 10% other proteins, or even more preferably less than about 5% other proteins. When the peptide is recombinantly produced, it can also be substantially free of culture medium, i.e., culture medium represents less than about 20% of the volume of the protein preparation.

The language "substantially free of chemical precursors or other chemicals" includes preparations of the peptide in which it is separated from chemical precursors or other chemicals that are involved in its synthesis. In one embodiment, the language "substantially free of chemical precursors or other chemicals" includes preparations of the kinase peptide having less than about 30% (by dry weight) chemical precursors or other chemicals, preferably less than about 20% chemical precursors or other chemicals, more preferably less than about 10% chemical precursors or other chemicals, or even more preferably less than about 5% chemical precursors or other chemicals.

The isolated kinase described herein can be purified from cells that naturally express it, purified from cells that have been altered to express it (recombination), or synthesized using known protein synthesis methods. For example, a nucleic acid molecule encoding the protein kinase is cloned into an expression vector, the expression vector introduced into a host cell and the protein expressed in the host cell. The protein can then be isolated from the cells by an appropriate purification scheme using standard protein purification techniques. Many of these techniques are described in detail below.

As mentioned above, the present invention also provides variants of the amino acid sequence of the peptides of the present invention, such as naturally occurring mature forms of the peptides, allelic/sequence variants of the peptides, non-naturally occurring recombinantly derived variants of the peptides, and orthologs and paralogs of the peptides. Such variants can be generated using techniques that are known by those skilled in the fields of recombinant nucleic acid technology and protein biochemistry. In addition, such variants can readily be identified/made using molecular techniques and the sequence information disclosed herein. Further, such variants can readily be distinguished from other peptides based on sequence and/or structural homology to the peptides of the present invention. The degree of homology/identity present will be based primarily on whether the peptide is a functional variant or non-functional variant, the amount of divergence present in the paralog family and the evolutionary distance between the orthologs.

To determine the percent identity of two amino acid sequences or two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid 'identity' is equivalent to amino acid or nucleic acid 'homology'). The percent identity between the two sequences is a function of the number of identical positions shared by the sequences, taking into account the number of gaps, and the length of each gap, which need to be introduced for optimal alignment of the two sequences. In a preferred embodiment, the length of a reference sequence aligned for comparison purposes is at least about 40%, preferably about 50%, more preferably about 60%, even more preferably about 70%, still more preferably about 80%, and yet more preferably about 90% or more of the length of the reference sequence.

The comparison of sequences and determination of percent identity and similarity between two sequences can be accomplished using a mathematical algorithm. See, e.g., *Computational Molecular Biology,* Lesk, A.M., ed., Oxford University Press, New York (1988); *Biocomputing: Informatics and Genome Projects,* Smith, D.W., ed., Academic Press, New York (1993); *Computer Analysis of Sequence Data, Part 1*, Griffin, A.M., and Griffin, H.G., eds., Humana Press, New Jersey (1994); *Sequence Analysis in Molecular Biology,* von Heinje, G., Academic Press (1987); and *Sequence Analysis Primer,* Gribskov, M. and Devereux, J., eds., M Stockton Press, New York (1991). In one embodiment, the percent identity between two amino acid sequences is determined using the Needleman and Wunsch (*J. Mol. Biol.* (48):444-453 (1970)) algorithm which has been incorporated into commercially available computer programs, such as GAP in the GCG software package, using either a Blossom 62 matrix or a PAM250 matrix, and a gap weight of 16, 14, 12, 10, 8, 6, or 4 and a length weight of 1, 2, 3, 4, 5, or 6. In another embodiment, the percent identity between two nucleotide sequences is determined using the commercially available computer programs including the GAP program in the GCG software package (Devereux, J., et al., *Nucleic Acids Res.* 12(1):387 (1984)), the NWS gap DNA CMP matrix and a gap weight of 40, 50, 60, 70, or 80 and a length weight of 1, 2, 3, 4, 5, or 6. In yet another embodiment, the percent identity between two amino acid or nucleotide sequences is determined using the algorithm of E. Meyers and W. Miller (CABIOS, 4:11-17 (1989)) which has been incorporated into commercially available computer programs, such as ALIGN (version 2.0), using a PAM120 weight residue table, a gap length penalty of 12 and a gap penalty of 4.

The nucleic acid and protein sequences of the present invention can further be used as a "query sequence" to perform a search against sequence databases to, for example, identify other family members or related sequences. Such searches can be performed using commercially available search engines, such as the NBLAST and XBLAST programs (version 2.0) of Altschul et al., *J. Mol. Biol.* 215:403-10 (1990). BLAST nucleotide searches can be performed, for example, with the NBLAST program, score = 100, wordlength = 12, to obtain nucleotide sequences homologous to the nucleic acid molecules of the invention. BLAST protein searches can be performed, for example, with the XBLAST program, score = 50, wordlength = 3, to obtain amino acid sequences homologous to the proteins of the invention. To obtain gapped alignments for comparison purposes, Gapped BLAST can be utilized as described in Altschul et al. *(Nucleic Acids Res.* 25(17):3389-3402 (1997)). When utilizing BLAST programs, the default parameters of the respective programs (e.g., XBLAST and NBLAST) can be used. See, e.g., http://www.ncbi.nlm.nih.gov.

As used herein, two proteins (or a region of the proteins) have significant homology when the amino acid sequences are preferably at least about 70-75%, more preferably at least about 80-85%, and even more preferably at least about 90-95% or more homologous. A significantly homologous amino acid sequence, according to the present invention, will be encoded by a nucleic acid sequence that will hybridize to a peptide encoding nucleic acid molecule under stringent conditions as more fully described below. Peptides can readily be identified as having a high degree of (i.e., significant) sequence homology/identity to the peptides of the present invention. Full-length clones comprising one of the peptides of the present invention can readily be identified as having complete sequence identity to one of the kinases of the present invention as well as being encoded by the same genetic locus as the kinase provided herein. Allelic variants of a peptide can readily be identified as having a high degree of sequence homology/identity to at least a portion of the peptide as well as being encoded by the same genetic locus as the kinase peptide provided herein.

Paralogs of a hepatocyte growth factor receptor kinase can readily be identified as having some degree of significant sequence homology/identity to at least a portion of the HGFR, as being encoded by a gene from humans, and as having similar activity or function. Two proteins will typically be considered paralogs when the amino acid sequences are preferably at least about 60% or greater, and more preferably at least about 70% or greater homology through a given region or domain. Such paralogs will be encoded by a nucleic acid sequence that will hybridize to a HGFR encoding nucleic acid molecule under moderate to stringent conditions as more fully described below.

Orthologs of a kinase peptide can readily be identified as having some degree of significant sequence homology/identity to at least a portion of the kinase peptide as well as being encoded by a gene from another organism. Preferred orthologs will be isolated from mammals. Such orthologs will be encoded by a nucleic acid sequence that will hybridize to a kinase peptide encoding nucleic acid molecule under moderate to stringent conditions, as more fully described below, depending on the degree of relatedness of the two organisms yielding the proteins.

Non-naturally occurring variants of the kinases of the present invention can readily be generated using recombinant techniques. Such variants include, but are not limited to deletions, additions and substitutions in the amino acid sequence of the kinase. For example, one class of substitutions are conserved amino acid substitutions. Such substitutions are those that substitute a given amino acid in a kinase peptide by another amino acid of like characteristics. Typically seen as conservative substitutions are the replacements, one for another, among the aliphatic amino acids Ala, Val, Leu, and Ile; interchange of the hydroxyl residues Ser and Thr; exchange of the acidic residues Asp and Glu; substitution between the amide residues Asn and Gln; exchange of the basic residues Lys and Arg; and replacements among the aromatic residues Phe and Tyr. Guidance concerning which amino acid changes are likely to be phenotypically silent are found in Bowie et al., *Science 247*:1306-1310 (1990).

Variant kinases can be fully functional or may have reduced or decreased activity when compared to the wild-type protein. Fully functional variants may contain only conservative variations or variations in non-critical residues or in non-critical regions. Functional variants can also contain substitutions of similar amino acids, which result in no change or an insignificant change in function. Alternatively, such substitutions may positively or negatively affect function to some degree. Non-functional variants typically contain one or more non-conservative amino acid substitutions, deletions, insertions, inversions, or truncations or a substitution, insertion, inversion, or deletion in a critical residue or critical region.

Amino acids that are essential for function can be identified by methods known in the art, such as site-directed mutagenesis or alanine-scanning mutagenesis (Cunningham et al., *Science* 244:1081-1085 (1989)). The latter procedure introduces single alanine mutations at every residue in the molecule. The resulting mutant molecules are then tested for biological activity, for example by measuring enzymatic activity. Sites that are critical for binding can also be determined by structural analysis such as X-ray crystallography, nuclear magnetic resonance or photoaffinity labeling (Smith et al., *J. Mol. Biol.* 224:899-904 (1992); de Vos et al., *Science* 255:306-312 (1992)). Accordingly, the peptides of the present invention also encompass derivatives or analogs in which a substituted amino acid residue is not one encoded by the genetic code; in which a substituent group is included; in which the polypeptide is fused with another compound, such as a compound to increase the half-life of the polypeptide (for example, polyethylene glycol); or in which the additional amino acids are fused to the polypeptide, such as a leader or secretory sequence or a sequence for purification of the mature polypeptide or a pro-protein sequence.

The present invention further provides for functional, active fragments of the kinase peptides, in addition to proteins and peptides that comprise and consist of such fragments. As used herein, a fragment comprises at least about 8 or more contiguous amino acid residues from the protein kinase. Such fragments can be chosen based on the ability to retain one or more of the biological activities of the kinase or could be chosen for the ability to perform a function, e.g. act as an immunogen. Preferred are fragments that are catalytically active and that have improved crystallographic properties as compared to the full-length, wild-type kinase. Such fragments will typically comprise a domain or motif of the kinase, e.g., active site. Further, possible fragments include, but are not limited to, domain or motif containing fragments, soluble peptide fragments, and fragments containing immunogenic structures. Predicted domains and functional sites are readily identifiable by computer programs known and readily available to those of skill in the art (e.g., by PROSITE analysis- Hofmann et al., *Nucleic Acids Res.* 27:215-219 (1999); Bucher et al., *Proceedings 2nd International Conference on Intelligent Systems for Molecular Biology* AAAI Press, Menlo Park, 53-61 (1994)). For example, the fragment can comprise the HGFR
intracellular domain [SEQ ID NO. 13].

SEQ ID NO. 13:
MGSELVRYDARVHTPHLDRLVSARSVSPTTEMVSNESVDYRATFPEDQFPNSSQNGS
CRQVQYPLTDMSPILTSGDSDISSPLLQNTVHIDLSALNPELVQAVQHVVIGPSSLIVHF
NEVIGRGHFGCVYHGTLLDNDGKKIHCAVKSLNRITDIGEVSQFLAEGIIMKDFSHPN
VLSLLGICLRSEGSPLVVLPYMKHGDLRNFIRNETHNPTVKDLIGFGLQVAKGMKYL

ASKKFVHRDLAARNCMLDEKFTVKVADFGLARDMYDKEYYSVHNKTGAKLPVKW
MALESLQTQKFTTKSDVWSFGVLLWELMTRGAPPYPDVNTFDITVYLLQGRRLLQPE
YCPDPLYEVMLKCWHPKAEMRPSFSELVSRISAIFSTFIGEHYVHVNATYVNVKCVA
PYPSLLSSEDNADDEVDTRPASFWETS

A fragment is a variant peptide having an amino acid sequence that is entirely the same as part, but not all, of any amino acid sequence of any peptide of the invention. Fragments may be free standing or comprised within a larger peptide.

Polypeptides of the present invention also optionally contain amino acids other than the 20 amino acids commonly referred to as the 20 naturally-occurring amino acids. Further, amino acids, including the terminal amino acids, may be modified by natural processes, such as processing and other post-translational modifications, or by chemical modification techniques known in the art. Common modifications that occur naturally in polypeptides are described in basic texts, detailed monographs, and the research literature. Known modifications include, but are not limited to, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristoylation, oxidation, proteolytic processing, phosphorylation, phenylation, racemization, selenoylation, sulfation, transfer-RNA mediated addition of amino acids to proteins such as arginylation, and ubiquitination. Several particularly common modifications, glycosylation, lipid attachment, sulfation, gamma-carboxylation of glutamic acid residues, hydroxylation and ADP-ribosylation, for instance, are described in most basic texts, such as *Proteins - Structure and Molecular Properties,* 2nd Ed., T.E. Creighton, W. H. Freeman and Company, New York (1993). Many detailed reviews are available on this subject, such as by Wold, F., *Posttranslational Covalent Modification of Proteins,* B.C. Johnson, Ed., Academic Press, New York 1-12 (1983); Seifter et al., *Meth. Enzymol. 182:* 626-646 (1990) and Rattan et al., *Ann. N.Y. Acad. Sci. 663*:48-62 (1992).

As used herein, "polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. The terms "peptide," "polypeptide," and "protein" are used interchangeably herein.

The peptides of the present invention can be attached to heterologous sequences to form chimeric or fusion proteins. Such chimeric and fusion proteins comprise a peptide operatively linked to a heterologous protein. "Operatively linked" indicates that the peptide and the heterologous protein are fused in-frame. The heterologous protein can be fused to the N-terminus or C-terminus of the kinase peptide. The two peptides linked in a fusion peptide are typically derived from two independent sources. Therefore, a fusion peptide comprises two linked peptides not normally found linked in nature. The two peptides may be from the same or different genome. In some uses, the fusion protein does not affect the activity of the peptide *per se.* For example, the fusion protein can include, but is not limited to, enzymatic fusion proteins, for example beta-galactosidase fusions, yeast two-hybrid GAL fusions, poly-His fusions (i.e., HI-tagged), MYC-tagged, and Ig fusions. Such fusion proteins, particularly poly-His fusions, can facilitate the purification of recombinant kinase peptides. In certain host cells (e.g., mammalian host cells), expression and/or secretion of a protein can be increased by using a heterologous signal sequence.

A chimeric or fusion protein can be produced by standard recombinant DNA techniques. For example, DNA fragments coding for the different protein sequences are ligated together in-frame in accordance with conventional techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments, which can subsequently be annealed and re-amplified to generate a chimeric gene sequence (see, Ausubel et al., *Current Protocols in Molecular Biology,* (1992)). Moreover, many expression vectors are commercially available that already encode a fusion moiety (e.g., a GST protein, His-tag, or green fluorescent protein). A kinase peptide-encoding nucleic acid can be cloned into such an expression vector such that the fusion moiety is linked in-frame to the kinase peptide.

Herein, the term "antibody" refers to a polypeptide or group of polypeptides which are comprised of at least one antibody combining site or binding domain, said binding domain or combining site formed from the folding of variable domains of an antibody molecule to form three dimensional binding spaces with an internal surface shape and charge distribution complementary to the features of an antigen epitope. The term encompasses immunoglobulin molecules and immunologically active portions of immunoglobulin molecules, such as molecules that contain an antibody combining site or paratope. Exemplary antibody molecules are intact immunoglobulin molecules, substantially intact immunoglobulin molecules and portions of an immunoglobulin molecule, including those known in the art as Fab, FabB, F(abB)₂ and F(v).

### B. Nucleic Acids and Polynucleotides

The present invention provides isolated nucleic acid molecules that encode the functional or active kinases of the present invention. Such nucleic acid molecules will consist of, consist essentially of, or comprise a nucleotide sequence that encodes one of the kinase peptides of the present invention, an allelic variant thereof, or an ortholog or paralog thereof.

The terms "nucleic acid molecule" and "polynucleotide" are used interchangeable in this application. These terms generally refer to any polyribonucleotide or polydeoxribonucleotide, which may be unmodified RNA or DNA or modified RNA or DNA. These terms are intended to include DNA molecules (e.g., cDNA) and RNA molecules (e.g., mRNA) and analogs of the DNA or RNA generated using nucleotide analogs. These terms include, without limitation, single- and double-stranded DNA, DNA that is a mixture of single- and double-stranded regions or single-, double- and triple-stranded regions, single- and double-stranded RNA, and RNA that is mixture of single- and double-stranded regions, hybrid molecules comprising DNA and RNA that may be single-stranded or, more typically, double-stranded, or triple-stranded regions, or a mixture of single- and double-stranded regions. In addition, "polynucleotide" and "nucleic acid molecule" as used herein refer to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. As used herein, the terms "polynucleotide(s)" and "nucleic acid molecule" also include DNAs or RNAs as described above that contain one or more modified bases. Thus, DNAs or RNAs with backbones modified for stability or for other reasons are "polynucleotide(s)" as that term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The terms "polynucleotide(s)" and "nucleic acid molecules" as employed herein embraces such chemically, enzymatically or metabolically modified forms of polynucleotides, as well as the chemical forms of DNA and RNA characteristic of viruses and cells, including, for example, simple and complex cells. "Polynucleotide(s)" also embraces short polynucleotides often referred to as oligonucleotide(s).

As used herein, an "isolated" nucleic acid molecule is one that is separated from other nucleic acid present in the natural source of the nucleic acid. Preferably, an "isolated" nucleic acid is free of sequences which naturally flank the nucleic acid (i.e., sequences located at the 5' and 3' ends of the nucleic acid) in the genomic DNA or cDNA of the organism from which the nucleic acid is derived. However, there can be some flanking nucleotide sequences, for example up to about 5KB, particularly contiguous peptide encoding sequences and peptide encoding sequences within the same gene but separated by introns in the genomic sequence. The important point is that the nucleic acid is isolated from remote and unimportant flanking sequences such that it can be subjected to the specific manipulations described herein, such as recombinant expression, preparation of probes and primers, and other uses specific to the nucleic acid sequences. Moreover, an "isolated" nucleic acid molecule, such as a cDNA molecule, is preferably substantially free of other cellular material, or culture medium when produced by recombinant techniques, or chemical precursors or other chemicals when chemically synthesized. However, the nucleic acid molecule can be fused to other coding or regulatory sequences and still be considered isolated. In some instances, the isolated material will form part of a composition (for example, a crude extract containing other substances), buffer system or reagent mix. In other circumstances, the material may be purified to essential homogeneity, for example as determined by PAGE or column chromatograph such as HPLC.

For example, recombinant DNA molecules contained in a vector are considered isolated. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the isolated DNA molecules of the present invention. Isolated nucleic acid molecules according to the present invention further include such molecules produced synthetically.

The preferred classes of nucleic acid molecules that are comprised of the nucleotide sequences of the present invention are the full-length cDNA molecules and genes and genomic clones since some of the nucleic acid molecules provided herein are fragments of the complete gene that exists in nature. A description of how various types of these nucleic acid molecules can be readily made/isolated is provided herein.

Full-length genes or portions thereof may be cloned from known sequences using any one of a number of methods known in the art. For example, a method which employs XL-PCR (Perkin-Elmer, Foster City, Calif.) to amplify long pieces of DNA may be used. Other methods for obtaining full-length sequences are known in the art.

The isolated nucleic acid molecules can encode the active protein plus additional amino or carboxyl-terminal amino acids, or amino acids interior to the mature peptide (when the mature form has more than one peptide chain, for instance). Such sequences may play a role in processing of a protein from precursor to an active form, facilitate protein trafficking, prolong or shorten protein half-life or facilitate manipulation of a protein for assay or production, among other things. As generally is the case *in situ,* the additional amino acids may be processed away from the mature active protein by cellular enzymes.

Once a full-length gene is cloned, portions of the gene can be obtained using techniques known to those of ordinary skill in the art. The isolated nucleic acid molecules include, but are not limited to, the sequence encoding the active kinase alone or in combination with coding sequences, such as a leader or secretory sequence (e.g., a pre-pro or pro-protein sequence), the sequence encoding the active kinase, with or without the additional coding sequences, plus additional non-coding sequences, for example introns and non-coding 5' and 3' sequences such as transcribed but non-translated sequences that play a role in transcription, mRNA processing (including splicing and polyadenylation signals), ribosome binding and stability of mRNA. In addition, the nucleic acid molecule may be fused to a marker sequence encoding, for example, a peptide that facilitates purification.

Isolated nucleic acid molecules can be in the form of RNA, such as mRNA, or in the form DNA, including cDNA and genomic DNA, obtained by cloning or produced by chemical synthetic techniques or by a combination thereof. The nucleic acid, especially DNA, can be double-stranded or single-stranded. Single-stranded nucleic acid can be the coding strand (sense strand) or the non-coding strand (anti-sense strand).

The invention further provides nucleic acid molecules that encode fragments of the peptides of the present invention and that encode obvious variants of the kinase proteins of the present invention that are described above. Such nucleic acid molecules may be naturally occurring, such as allelic variants (same locus), paralogs (different locus), and orthologs (different organism), or may be constructed by recombinant DNA methods or by chemical synthesis. Such non-naturally occurring variants may be made by mutagenesis techniques, including those applied to nucleic acid molecules, cells, or organisms. Accordingly, as discussed above, the variants can contain nucleotide substitutions, deletions, inversions and insertions. Variation can occur in either or both the coding and non-coding regions. The variations can produce both conservative and non-conservative amino acid substitutions.

According to certain embodiments of the present invention, mutations to HGFR are utilized. For example, the tyrosine at residue 12130 is replaced with cysteine [SEQ ID NO. 10; a germline mutation in HPRC]; the methionine 1250 at residue 1250 is replaced with threonine [SEQ ID NO. 11]; and/or the histidine at residue 1094 is replaced with argenine [SEQ ID NO. 14].

SEQ ID NO. 10:
atggtccaca ttgacctcag tgctctaaat ccagagctgg tccaggcagt gcagcatgta
gtgattgggc ccagtagcct gattgtgcat ttcaatgaag tcataggaag agggcatttt
ggttgtgtat atcatgggac tttgttggac aatgatggca agaaaattca ctgtgctgtg
aaatccttga acagaatcac tgacatagga gaagtttccc aatttctgac cgagggaatc
atcatgaaag attttagtca tcccaatgtc ctctcgctcc tgggaatctg cctgcgaagt
gaagggtctc cgctggtggt cctaccatac atgaaacatg gagatcttcg aaatttcatt
cgaaatgaga ctcataatcc aactgtaaaa gatcttattg gctttggtct tcaagtagcc
aaagggatga aatatcttgc aagcaaaaag tttgtccaca gagacttggc tgcaagaaac
tgtatgctgg atgaaaaatt cacagtcaag gttgctgatt ttggtcttgc cagagacatg
tgtgataaag aatactatag tgtacacaac aaaacaggtg caaagctgcc agtgaagtgg
atggctttgg aaagtctgca aactcaaaag tttaccacca agtcagatgt gtggtccttt
ggcgtcgtcc tctgggagct gatgacaaga ggagccccac cttatcctga cgtaaacacc
tttgatataa ctgtttactt gttgcaaggg agaagactcc tacaacccga atactgccca
gaccccttat atgaagtaat gctaaaatgc tggcacccta aagccgaaat gcgcccatcc
ttttctgaac tggtgtcccg gatatcagcg atcttctcta ctttcattgg ggagcac

SEQ ID NO 11:
atggtccaca ttgacctcag tgctctaaat ccagagctgg tccaggcagt gcagcatgta
gtgattgggc ccagtagcct gattgtgcat ttcaatgaag tcataggaag agggcatttt
ggttgtgtat atcatgggac tttgttggac aatgatggca agaaaattca ctgtgctgtg
aaatccttga acagaatcac tgacatagga gaagtttccc aatttctgac cgagggaatc
atcatgaaag attttagtca tcccaatgtc ctctcgctcc tgggaatctg cctgcgaagt
gaagggtctc cgctggtggt cctaccatac atgaaacatg gagatcttcg aaatttcatt
cgaaatgaga ctcataatcc aactgtaaaa gatcttattg gctttggtct tcaagtagcc
aaaggcatga aatatcttgc gagcaaaaag tttgtccaca gagacttggc tgcaagaaac
tgtatgctgg atgaaaaatt cacagtcaag gttgctgatt ttggtcttgc cagagacatg
tatgataaag aatactatag tgtacacaac aaaacaggtg caaagctgcc agtgaagtgg
accgctttgg aaagtctgca aactcaaaag tttaccacca agtcagatgt gtggtccttt
ggcgtgctcc tctgggagct gatgacaaga ggagccccac cttatcctga tgtaaacacc
tttgatataa ctgtttactt gttgcaaggg agaagactcc tacaacccga atactgccca
gaccccttat atgaagtaat gctaaaatgc tggcacccta aagccgaaat gcgcccatcc
ttttctgaac tggtgtcccg gatatcagcg atcttctcta ctttcattgg ggagcac

SEQ ID NO 14:

atggtccaca ttgacctcag tgctctaaat ccagagctgg tccaggcagt gcagcatgta
gtgattgggc ccagtagcct gattgtgcat ttcaatgaag tcataggaag agggcatttt
ggttgtgtat atcgtgggac tttgttggac aatgatggca agaaaattca ctgtgctgtg
aaatccttga acagaatcac tgacatagga gaagtttccc aatttctgac cgagggaatc
atcatgaaag attttagtca tcccaatgtc ctctcgctcc tgggaatctg cctgcgaagt
gaagggtctc cgctggtggt cctaccatac atgaaacatg gagatcttcg aaatttcatt
cgaaatgaga ctcataatcc aactgtaaaa gatcttattg gctttggtct tcaagtagcc
aaagggatga aatatcttgc aagcaaaaag tttgtccaca gagacttggc tgcaagaaac
tgtatgctgg atgaaaaatt cacagtcaag gttgctgatt ttggtcttgc cagagacatg
tatgataaag aatactatag tgtacacaac aaaacaggtg caaagctgcc agtgaagtgg
atggctttgg aaagtctgca aactcaaaag tttaccacca agtcagatgt gtggtccttt
ggcgtcgtcc tctgggagct gatgacaaga ggagccccac cttatcctga cgtaaacacc
tttgatataa ctgtttactt gttgcaaggg agaagactcc tacaacccga atactgccca
gaccccttat atgaagtaat gctaaaatgc tggcacccta aagccgaaat gcgcccatcc
ttttctgaac tggtgtcccg gatatcagcg atcttctcta ctttcattgg ggagcac

A fragment comprises a contiguous nucleotide sequence greater than about 8 or more nucleotides. Further, a fragment could be at least about 30, preferably at least about 40, more preferably at least about 50, and even more preferably at least about 100 or more nucleotides in length. The length of the fragment will be based on its intended use. For example, the fragment can encode epitope bearing regions of the peptide, or can be useful as DNA probes and primers. Such fragments can be isolated using the known nucleotide sequence to synthesize an oligonucleotide probe. A labeled probe can then be used to screen a cDNA library, genomic DNA library, or mRNA to isolate nucleic acid corresponding to the coding region. Further, primers can be used in PCR reactions to clone specific regions of gene.

A probe/primer typically comprises substantially a purified oligonucleotide or oligonucleotide pair. The oligonucleotide typically comprises a region of nucleotide sequence that hybridizes under stringent conditions to at least about 12 or more consecutive nucleotides.

Orthologs, homologs, and allelic variants can be identified using methods known in the art. As described above, these variants comprise a nucleotide sequence encoding a peptide that is preferably about 60-65%, more preferably about 70-75%, and even more preferably at least about 90-95% or more homologous to the nucleotide sequence provided in SEQ ID NO: 1 or a fragment of this sequence. Such nucleic acid molecules can readily be identified as being able to hybridize under moderate to stringent conditions to the nucleotide sequence shown in SEQ ID NO: 1 or a fragment of the sequence.

As used herein, the term "hybridizes under stringent conditions" is intended to describe conditions for hybridization and washing under which nucleotide sequences encoding a peptide at least about 50%, and more preferably at least about 55% or more, homologous to each other typically remain hybridized to each other. The conditions can be such that sequences at least about 65%, preferably at least about 70%, and more preferably at least about 75% or more homologous to each other typically remains hybridized to each other. Such stringent conditions are known to those skilled in the art and can be found in *Current Protocols in Molecular Biology,* John Wiley & Sons, N.Y., 6.3.1-6.3.6 (1989), which is hereby incorporated by reference in its entirety. One example of stringent hybridization conditions is hybridization in 6X sodium chloride/sodium citrate (SSC) at about 45°C, followed by one or more washes in 0.2 X SSC, 0.1% SDS at 50-65°C.

As used herein, the term "hybridizes under moderate conditions" is intended to describe conditions for hybridization and washing which are less severe than those described above for stringent conditions. Such moderate conditions are known to those skilled in the art and can be found in *Molecular Cloning, A laboratory manual*, J.Sambrook, E.F. Fritisch, T. Maniatis , Cold Spring Harbor Press Book 2 Chapter 9. One example of moderate conditions is hybridization in 6x SSC at room temperature, followed by 2x SSc and 0.1% SDS at 37°C.

The nucleic acid molecules of the present invention are useful for probes, primers, and chemical intermediates, and in biological assays. For example, the nucleic acid molecules can be used as hybridization probes for cDNA and genomic DNA to isolate full-length cDNA and genomic clones encoding the peptide described herein and to isolate cDNA and genomic clones that correspond to variants (alleles, orthologs, etc.) producing the same or related peptides described herein. The probe can correspond to any sequence along the entire length of the nucleic acid molecules provided in SEQ ID NO: 1. Accordingly, it could be derived from 5' noncoding regions, the coding region, and 3' noncoding regions. However, as discussed, fragments are not to be construed as those which may encompass fragments disclosed prior to the present invention. Probes can be used as a part of a diagnostic test kit for identifying cells or tissues that express a kinase protein, such as by measuring a level of a receptor-encoding nucleic acid in a sample of cells from a subject e.g., mRNA or genomic DNA, or determining if a receptor gene has been mutated.

The nucleic acid molecules of the present invention are useful for producing peptides for use in crystallization studies, drug discovery, and drug design. The nucleic acid molecules are also useful as primers for PCR to amplify any given region of a nucleic acid molecule and are useful to synthesize anti-sense molecules of desired length and sequence. The nucleic acid molecules are also useful for constructing recombinant vectors. Such vectors include expression vectors that express a portion of, or all of, the peptide sequences. Vectors also include insertion vectors, used to integrate into another nucleic acid molecule sequence, such as into the cellular genome, to alter *in situ* expression of a gene and/or gene product. For example, an endogenous coding sequence can be replaced via homologous recombination with all or part of the coding region containing one or more specifically introduced mutations. In addition, the nucleic acid molecules are useful for expressing antigenic portions of the proteins; for determining the chromosomal positions of the nucleic acid molecules by means of *in situ* hybridization methods; for designing ribozymes corresponding to all, or a part, of the mRNA produced from the nucleic acid molecules described herein; for constructing host cells expressing a part, or all, of the nucleic acid molecules and peptides; for constructing transgenic animals expressing all, or a part, of the nucleic acid molecules and peptides; and for making vectors that express part, or all, of the peptides.

Further, the nucleic acid molecules are useful as hybridization probes for determining the presence, level, form and distribution of nucleic acid expression. Accordingly, the probes can be used to detect the presence of, or to determine levels of, a specific nucleic acid molecule in cells, tissues, and in organisms. The nucleic acid whose level is determined can be DNA or RNA. Accordingly, probes corresponding to the peptides described herein can be used to assess expression and/or gene copy number in a given cell, tissue, or organism. These uses are relevant for diagnosis of disorders involving an increase or decrease in kinase protein expression relative to normal results.

*In vitro* techniques for detection of mRNA include Northern hybridizations and *in situ* hybridizations. *In vitro* techniques for detecting DNA include Southern hybridizations and *in situ* hybridization.

### C. Vectors and Host Cells

The invention also provides vectors containing the nucleic acid molecules described herein. The term "vector" refers to a vehicle, preferably a nucleic acid molecule, that can transport the nucleic acid molecules. When the vector is a nucleic acid molecule, the nucleic acid molecules are covalently linked to the vector nucleic acid. With this aspect of the invention, the vector includes a plasmid, single or double stranded phage, a single or double stranded RNA or DNA viral vector, or artificial chromosome, such as a BAC, PAC, YAC, OR MAC. Various expression vectors can be used to express polynucleotides encoding an hHGFR kinase, such as (but not limited to) pET and pProEX. A vector can be maintained in the host cell as an extrachromosomal element where it replicates and produces additional copies of the nucleic acid molecules. Alternatively, the vector may integrate into the host cell genome and produce additional copies of the nucleic acid molecules when the host cell replicates. The invention provides vectors for the maintenance (cloning vectors) or vectors for expression (expression vectors) of the nucleic acid molecules. The vectors can function in prokaryotic or eukaryotic cells or in both (shuttle vectors).

Expression vectors contain cis-acting regulatory regions that are operably linked in the vector to the nucleic acid molecules such that transcription of the nucleic acid molecules is allowed in a host cell. The nucleic acid molecules can be introduced into the host cell with a separate nucleic acid molecule capable of affecting transcription. Thus, the second nucleic acid molecule may provide a trans-acting factor interacting with the cis-regulatory control region to allow transcription of the nucleic acid molecules from the vector. Alternatively, a trans-acting factor may be supplied by the host cell. Finally, a trans-acting factor can be produced from the vector itself. It is understood, however, that in some embodiments, transcription and/or translation of the nucleic acid molecules can occur in a cell-free system.

The regulatory sequence to which the nucleic acid molecules described herein can be operably linked include promoters for directing mRNA transcription. These include, but are not limited to, the left promoter from bacteriophage λ, the lac, TRP, and TAC promoters from E. *coli,* the early and late promoters from SV40, the CMV immediate early promoter, the adenovirus early and late promoters, and retrovirus long-terminal repeats. In addition to control regions that promote transcription, expression vectors may also include regions that modulate transcription, such as repressor binding sites and enhancers. Examples include the SV40 enhancer, the cytomegalovirus immediate early enhancer, polyoma enhancer, adenovirus enhancers, and retrovirus LTR enhancers. The term "operably linked" as used herein indicates that a gene and a regulatory sequence(s), such as a promoter, are connected in such a way as to permit gene expression when the appropriate molecules (e.g., transcriptional activator proteins or proteins which include transcriptional activation domains) are bound to the regulatory sequence(s).

In addition to containing sites for transcription initiation and control, expression vectors can also contain sequences necessary for transcription termination and, in the transcribed region, a ribosome binding site for translation. Other regulatory control elements for expression include initiation and termination codons as well as polyadenylation signals. The person of ordinary skill in the art would be aware of the numerous regulatory sequences that are useful in expression vectors. Such regulatory sequences are described, for example, in Sambrook et al., *Molecular Cloning: A Laboratory Manual.* 3rd. *ed.,* Cold Spring Harbor Laboratory Press, Cold Spring Harbor, NY, (2001), which is hereby incorporated by reference in its entirety.

A variety of expression vectors can be used to express a nucleic acid molecule. Such vectors include chromosomal, episomal, and virus-derived vectors, for example vectors derived from bacterial plasmids, from bacteriophage, from yeast episomes, from yeast chromosomal elements, including yeast artificial chromosomes, from viruses such as baculoviruses, papovaviruses such as SV40, Vaccinia viruses, adenoviruses, poxviruses, pseudorabies viruses, and retroviruses. Vectors may also be derived from combinations of these sources such as those derived from plasmid and bacteriophage genetic elements, e.g., cosmids and phagemids. Appropriate cloning and expression vectors for prokaryotic and eukaryotic hosts are described in Sambrook et al., *supra.*

The regulatory sequence may provide constitutive expression in one or more host cells (i.e., tissue specific) or may provide for inducible expression in one or more cell types such as by temperature, nutrient additive, or exogenous factor such as a hormone or other ligand. A variety of vectors providing for constitutive and inducible expression in prokaryotic and eukaryotic hosts are known to those of ordinary skill in the art.

The nucleic acid molecules can be inserted into the vector nucleic acid by well-known methodology. Generally, the DNA sequence that will ultimately be expressed is joined to an expression vector by cleaving the DNA sequence and the expression vector with one or more restriction enzymes and then ligating the fragments together. Procedures for restriction enzyme digestion and ligation are known to those of ordinary skill in the art.

The vector containing the appropriate nucleic acid molecule can be introduced into an appropriate host cell for propagation or expression using techniques known to those of ordinary skill in the art. Bacterial cells include, but are not limited to, *E. coli, Streptomyces,* and *Salmonella typhimurium.* Eukaryotic cells include, but are not limited to, yeast, insect cells such as *Drosophila,* animal cells such as COS and CHO cells, and plant cells.

As described herein, it may be desirable to express a peptide of the present invention as a fusion protein. Accordingly, the invention provides fusion vectors that allow for the production of such peptides. Fusion vectors can increase the expression of a recombinant protein, increase the solubility of the recombinant protein, and aid in the purification of the protein by acting for example as a ligand for affinity purification. A proteolytic cleavage site may be introduced at the junction of the fusion moiety so that the desired peptide can ultimately be separated from the fusion moiety. Proteolytic enzymes include, but are not limited to, factor Xa, thrombin, and enterokinase. Typical fusion expression vectors include pGEX (Smith et al., *Gene* 67:31-40 (1988)), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein. Examples of suitable inducible non-fusion *E. coli* expression vectors include pTrc (Amann et al., *Gene* 69:301-315 (1988)) and pET 11d (Studier et al., *Gene Expression Technology: Methods in Enzymology* 185:60-89 (1990)).

Recombinant protein expression can be maximized in a host bacteria by providing a genetic background wherein the host cell has an impaired capacity to proteolytically cleave the recombinant protein (Gottesman, S., *Gene Expression Technology: Methods in Enzymology* 185, Academic Press, San Diego, California 119-128 (1990)). Alternatively, the sequence of the nucleic acid molecule of interest can be altered to provide preferential codon usage for a specific host cell, for example *E*. *coli*. (Wada et al., *Nucleic Acids Res.* 20:2111-2118 (1992)).

The nucleic acid molecules can also be expressed by expression vectors that are operative in yeast. Examples of vectors for expression in yeast, e.g., *S*. *cerevisiae*, include pYepSec1 (Baldari, et al., *EMBO J*. 6:229-234 (1987)), pMFa (Kurjan et al., *Cell* 30:933-943 (1982)), pJRY88 (Schultz et al., *Gene* 54:113-123 (1987)), and pYES2 (Invitrogen Corporation, San Diego, CA). The nucleic acid molecules can also be expressed in insect cells using, for example, baculovirus expression vectors. Baculovirus vectors available for expression of proteins in cultured insect cells (e.g., Sf 9 cells) include the pAc series (Smith et al., *Mol. Cell Biol.* 3:2156-2165 (1983)) and the pVL series (Lucklow et al., *Virology* 170:31-39 (1989)). In certain embodiments of the invention, the nucleic acid molecules described herein are expressed in mammalian cells using mammalian expression vectors. Examples of mammalian expression vectors include pCDM8 (Seed, B., *Nature* 329:840 (1987)) and pMT2PC (Kaufman et al., *EMBO J.* 6:187-195 (1987)). Each of the foregoing references is hereby incorporated by reference in its entirety.

The expression vectors listed herein are provided by way of example only of the well-known vectors available to those of ordinary skill in the art that would be useful to express the nucleic acid molecules. Preferred vectors include pET28a (Novagen, Madison, WI), pAcSG2 (Pharmingen, San Diego, CA), and pFastBac (Life Technologies, Gaithersburg, MD). The person of ordinary skill in the art would be aware of other vectors suitable for maintenance propagation or expression of the nucleic acid molecules described herein. These are found, for example, in Sambrook et al., *supra.*

The invention also encompasses vectors in which the nucleic acid sequences described herein are cloned into the vector in reverse orientation, but operably linked to a regulatory sequence that permits transcription of anti-sense RNA. Thus, an anti-sense transcript can be produced to all, or to a portion, of the nucleic acid molecule sequences described herein, including both coding and non-coding regions. Expression of this anti-sense RNA is subject to each of the parameters described above in relation to expression of the sense RNA (regulatory sequences, constitutive or inducible expression, tissue-specific expression).

The invention also relates to recombinant host cells containing the vectors described herein. Host cells therefore include prokaryotic cells, lower eukaryotic cells such as yeast, other eukaryotic cells such as insect cells, and higher eukaryotic cells such as mammalian cells. Preferred host cells of the instant invention include *E*. *coli* and Sf9.

The recombinant host cells are prepared by introducing the vector constructs described herein into the cells by techniques readily available to the person of ordinary skill in the art. These include, but are not limited to, calcium phosphate transfection, DEAE-dextran-mediated transfection, cationic lipid-mediated transfection, electroporation, transduction, infection, lipofection, and other techniques such as those found in Sambrook et al., *supra.*

Host cells can contain more than one vector. Thus, different nucleotide sequences can be introduced on different vectors of the same cell. Similarly, the nucleic acid molecules can be introduced either alone or with other nucleic acid molecules that are not related to the nucleic acid molecules such as those providing trans-acting factors for expression vectors. When more than one vector is introduced into a cell, the vectors can be introduced independently, co-introduced or joined to the nucleic acid molecule vector.

In the case of bacteriophage and viral vectors, these can be introduced into cells as packaged or encapsulated virus by standard procedures for infection and transduction. Viral vectors can be replication-competent or replication-defective. In the case in which viral replication is defective, replication will occur in host cells providing functions that complement the defects.

Vectors generally include selectable markers that enable the selection of the subpopulation of cells that contain the recombinant vector constructs. The marker can be contained in the same vector that contains the nucleic acid molecules described herein or may be on a separate vector. Markers include tetracycline or ampicillin-resistance genes for prokaryotic host cells and dihydrofolate reductase or neomycin resistance for eukaryotic host cells. However, any marker that provides selection for a phenotypic trait will be effective.

While the active protein kinases can be produced in bacteria, yeast, mammalian cells, and other cells under the control of the appropriate regulatory sequences, cell- free transcription and translation systems can also be used to produce these proteins using RNA derived from the DNA constructs described herein.

Where secretion of the peptide is desired, appropriate secretion signals are incorporated into the vector. The signal sequence can be endogenous to the peptides or heterologous to these peptides.

It is also understood that depending upon the host cell used for the recombinant production of the peptides described herein, the peptides can have various glycosylation patterns, depending upon the cell, or maybe non-glycosylated as when produced in bacteria. In addition, the peptides may include an initial modified methionine in some cases as a result of a host-mediated process.

The recombinant host cells expressing the peptides described herein have a variety of uses. First, the cells are useful for producing a kinase protein or peptide that can be further purified to produce desired amounts of kinase protein or fragments. Thus, host cells containing expression vectors are useful for peptide production. Host cells are also useful for conducting cell-based assays involving the kinase protein or kinase protein fragments. Thus, a recombinant host cell expressing a kinase polypeptide of the invention is useful for assaying compounds that stimulate or inhibit kinase protein function. Host cells are also useful for identifying kinase protein mutants in which these functions are affected. If the mutants naturally occur and give rise to a pathology, host cells containing the mutations are useful to assay compounds that have a desired effect on the mutant kinase protein (for example, stimulating or inhibiting function) which may not be indicated by their effect on the native kinase protein.

### D. Crystallization and Computer Methods for Model Building and Drug Design

Crystals of the polypeptides of the invention or ligand complexes of such polypeptides can be grown by a number of techniques including batch crystallization, vapor diffusion (either by sitting drop or hanging drop) and by microdialysis. Seeding of the crystals in some instances is required to obtain X-ray quality crystals. Standard micro and/or macro seeding of crystals may therefore be used. The HGFR-Compound **1** complex can be prepared as described below in reference to Example 2.

Once a crystal of the present invention is grown, X-ray diffraction data can be collected. X-ray diffraction data collection can be obtained using, for example, a MAR imaging plate detector. Crystals can be characterized by using X-rays produced in a conventional source (such as a sealed tube or a rotating anode) or using a synchrotron source.

Data processing and reduction can be carried out using programs such as HKL, DENZO, and SCALEPACK (Otwinowski and Minor, 1997, *Meth. Enymol*. 276:307-326 (1997)). In addition, X-PLOR, (Bruger, X-PLOR v.3.1 Manual, New Haven: Yale University, (1993)) or Heavy (T. Terwilliger, Los Alamos National Laboratory) may be utilized for bulk solvent correction and B-factor scaling. Electron density maps can be calculated using SHARP (La Fortelle, E. D. and Bricogne G., *Meth. Enzymol.* 276:472-494 (1997)) and SOLOMON. Molecular models can be built into this map using O (Jones, T. et al., *ACTA Crystallogr*. A47:110-119 (1991)), XTALVIEW (Scripps Research) or QUANTA96 (Accelrys, Inc. San Diego). Refinement can be done using XPLOR (Brunger, "X-PLOR:A System for X-ray Crystallography and NMR," Yale University Press, New Haven, Conn), using the free R-value to monitor the course of refinement.

Once the three-dimensional structure of a crystal comprising HGFR or an HGFR-complex is determined, a potential ligand (antagonist or agonist) is examined through the use of computer modeling using a docking program such as FelxiDock (Tripos, St. Louis, MO), GRAM (Medical Univ. Of South Carolina), DOCK3.5 and 4.0 (Univ. Calif. San Francisco), Glide (Schrödinger, Portland, OR), Gold (Cambridge Crystallographic Data Centre, UK), FLEX-X (BioSolveIT GmbH, Germany); AGDOCK (in-house software from Agouron Pharmaceuticals; Gehlhaar et al., *Chemistry & Biol.* 2:317-324), Hex (Ritchie, D and Kemp, G., *Proteins: Struct. Funct. & Genet.* 39:178-194), or AUTODOCK (Scripps Research Institute). This procedure can include computer fitting of potential ligands to the HGFR substrate-binding domain to ascertain how well the shape and the chemical structure of the potential ligand will complement or interfere with the HGFR substrate-binding domain (Bugg et al., *Scientific American* Dec.:92-98 (1993); West et al., *TIPS* 16:67-74 (1995)). Computer programs can also be employed to estimate the attraction, repulsion, and steric hindrance of the ligand to the HGFR binding domain. Generally the tighter the fit (e.g., the lower the steric hindrance, and/or the greater the attractive force) the more potent the potential drug will be since these properties are consistent with a tighter-binding constant.

"Binding domain" also referred to as "binding site", "binding pocket", "substrate-binding site," "catalytic domain," or "substrate-binding domain," refers to a region or regions of a molecule or molecular complex, that, as a result of its shape, can associate with another chemical entity or compound. Such regions are of significant utility in fields such as drug discovery. The association of natural ligands or substrates with binding pockets of their corresponding receptors or enzymes is the basis of many biological mechanisms of action. Similarly, many drugs exert their biological effects via an interaction with the binding pockets of a receptor or enzyme. Such interactions may occur with all or part of the binding pocket. An understanding of such interactions can lead to the design of drugs having more favorable and specific interactions with their target receptor or enzyme, and thus, improved biological effects. Therefore, information related to ligand binding with the HGFR substrate-binding site is valuable in designing potential modulators of HGFR. Further, the more specificity in the design of a potential drug the more likely that the drug will not interact with other similar proteins, thus, minimizing potential side effects due to unwanted cross interactions.

Initially, a potential ligand could be obtained by screening a random chemical library. A ligand selected in this manner could be then be systematically modified by computer-modeling programs until one or more promising potential ligands are identified. Such analysis has been shown to be effective in the development of HIV protease inhibitors (Larn et al., *Science* 263:380-384 (1994); Wlodawer et al., *Ann. Rev. Biochem.* 62:543-585 (1993); Appelt, *Perspectives in Drug Discovery and Design* 1:23-48 (1993); Erickson, *Perspectives in Drug Discovery and Design* 1: 109-128 (1993). Such computer modeling allows the selection of a finite number of rational chemical modifications, as opposed to the countless number of essentially random chemical modifications that could be made, and of which any one might lead to a useful drug. Each chemical modification requires additional chemical steps, which while being reasonable for the synthesis of a finite number of compounds, quickly becomes overwhelming if all possible modifications needed to be synthesized. Thus, through the use of the structure coordinates disclosed herein and computer modeling, a large number of these compounds can be rapidly screened on the computer monitor screen, and a few likely candidates can be determined without the laborious synthesis of untold numbers of compounds.

Once a potential ligand (agonist or antagonist) is identified it can be either selected from commercial libraries of compounds or alternatively the potential ligand may be synthesized *de novo.* As mentioned above, the *de novo* synthesis of one or even a relatively small group of specific compounds is reasonable in the art of drug design. The prospective drug can be tested in the binding assay exemplified below to test its ability to bind to the HGFR substrate-binding domain. The effect of the prospective drug on HGFR activity can also be determined using the assay described herein or other HGFR assays known in the art.

When a suitable compound is identified, a supplemental crystal can be grown which comprises a protein-ligand complex formed between the HGFR domain and the compound. Preferably the crystal effectively diffracts X-rays allowing the determination of the atomic coordinates of the protein-ligand complex to a resolution value of about 3.0 Å or less, more preferably about 2.0 Å or less. Molecular Replacement Analysis can be used to determine the three-dimensional structure of the supplemental crystal.

Molecular replacement involves using a known three-dimensional structure as a search model to determine the structure of an identical or closely related molecule or protein-ligand complex in a new crystal form. The measured X-ray diffraction properties of the new crystal are compared with those calculated from a search model structure to compute the position and orientation of the protein in the new crystal. Computer programs that can be used for this purpose include: X-PLOR, EPMR (Kissinger et al. *Acta Cryst.* D55:484-491 (1999)), ProLSQ and AMORE (J. Navaza, *Acta Crysrallographics ASO,* 157-163 (1994)). Once the position and orientation are known an electron density map can be calculated using the search model to provide X-ray phases. Thereafter, the electron density is inspected for structural differences and the search model is modified to conform to the new structure. Using this approach, it is possible to use the claimed structure to solve the three-dimensional structures of any such HGFR polypeptide-ligand complex. Other computer programs that can be used to solve the structures of such HGFR crystals include X-site, QUANTA, INSIGHT, ARP/wARP, and ICM.

For all of the drug design strategies described herein further refinements to the structure of the drug will generally be necessary and can be made by the successive iterations of any and/or all of the steps provided by the aforementioned strategies.

Another aspect of the invention involves using the structure coordinates generated from the HGFR -ligand complex to generate a three-dimensional shape. This is achieved through the use of commercially available software that is capable of generating three-dimensional graphical representations of molecules or portions thereof from a set of structure coordinates.

It will be readily apparent to those of skill in the art that the numbering of amino acids in other isoforms of HGFR may be different than that set forth for herein. Corresponding amino acids in other isoforms of HGFR are easily identified by inspection of the amino acid sequences, for example, through the use of commercially available homology software programs.

The amino acids of the HGFR domain of the polypeptides of the invention are described herein and are defined by a set of structure coordinates set forth in Table 1. The terms "structure coordinates" or "atomic coordinates" refer to Cartesian coordinates derived from mathematical equations related to the patterns obtained on diffraction of a monochromatic beam of X-rays by the atoms (scattering centers) of a protein or protein-ligand complex in crystal form. The diffraction data are used to calculate an electron density map of the repeating unit of the crystal. The electron density maps are then used to establish the positions of the individual atoms of the enzyme or enzyme complex.

**Table 1**

| | | | | | | | | | | | |
|---|---|---|---|---|---|---|---|---|---|---|---|
| ATOM | 1 | CB | LEU | 1062 | -5.656 | 32.472 | 9.323 | 1.00 | 42.24 | | C |
| ANISOU | 1 | CB | LEU | 1062 | 4988 | 5484 | 5576 | -719 | -875 | 457 | C |
| ATOM | 2 | CG | LEU | 1062 | -4.343 | 31.747 | 9.028 | 1.00 | 45.82 | | C |
| ANISOU | 2 | CG | LEU | 1062 | 6417 | 5073 | 5918 | 773 | -2605 | -523 | C |
| ATOM | 3 | CD1 | LEU | 1062 | -3.291 | 31.922 | 10.117 | 1.00 | 49.80 | | C |
| ANISOU | 3 | CD1 | LEU | 1062 | 6543 | 6638 | 5741 | 1783 | -2327 | -3085 | C |
| ATOM | 4 | CD2 | LEU | 1062 | -4.608 | 30.252 | 8.793 | 1.00 | 61.21 | | C |
| ANISOU | 4 | CD2 | LEU | 1062 | 8940 | 5485 | 8834 | 2 | -3713 | -1422 | C |
| ATOM | 5 | C | LEU | 1062 | -4.927 | 34.835 | 9.088 | 1.00 | 32.29 | | C |
| ANISOU | 5 | C | LEU | 1062 | 2270 | 5351 | 4646 | 198 | -130 | 1309 | C |
| ATOM | 6 | O | LEU | 1062 | -3.746 | 35.184 | 9.314 | 1.00 | 35.90 | | O |
| ANISOU | 6 | O | LEU | 1062 | 3421 | 4477 | 5741 | -1230 | -770 | 879 | O |
| ATOM | 7 | N | LEU | 1062 | -6.957 | 34.298 | 10.421 | 1.00 | 35.32 | | N |
| ANISOU | 7 | N | LEU | 1062 | 2492 | 5438 | 5488 | 363 | -111 | 3338 | N |
| ATOM | 8 | CA | LEU | 1062 | -5.610 | 33.834 | 10.010 | 1.00 | 31.18 | | C |
| ANISOU | 8 | CA | LEU | 1062 | 1961 | 4568 | 5319 | -249 | -1111 | 1419 | C |
| ATOM | 9 | N | VAL | 1063 | -5.635 | 35.320 | 8.052 | 1.00 | 33.88 | | N |
| ANISOU | 9 | N | VAL | 1063 | 4004 | 4197 | 4671 | 563 | -652 | 829 | N |
| ATOM | 10 | CA | VAL | 1063 | -4.971 | 36.369 | 7.245 | 1.00 | 37.92 | | C |
| ANISOU | 10 | CA | VAL | 1063 | 4464 | 4071 | 5871 | 117 | -1511 | 1514 | C |
| ATOM | 11 | CB | VAL | 1063 | -5.771 | 36.763 | 6.002 | 1.00 | 40.52 | | C |
| ANISOU | 11 | CB | VAL | 1063 | 5622 | 4212 | 5561 | 183 | -1748 | 1365 | C |
| ATOM | 12 | CG1 | VAL | 1063 | -5.271 | 38.081 | 5.394 | 1.00 | 45.84 | | C |
| ANISOU | 12 | CG1 | VAL | 1063 | 5460 | 5756 | 6200 | -558 | -1740 | 2571 | C |
| ATOM | 13 | CG2 | VAL | 1063 | -5.720 | 35.689 | 4.922 | 1.00 | 49.72 | | C |
| ANISOU | 13 | CG2 | VAL | 1063 | 5692 | 6405 | 6795 | 826 | -2035 | -304 | C |
| ATOM | 14 | C | VAL | 1063 | -4.710 | 37.538 | 8.200 | 1.00 | 39.48 | | C |
| ANISOU | 14 | C | VAL | 1063 | 3451 | 5518 | 6030 | -897 | -1094 | 793 | C |
| ATOM | 15 | O | VAL | 1063 | -3.699 | 38.229 | 8.177 | 1.00 | 40.62 | | O |
| ANISOU | 15 | O | VAL | 1063 | 3403 | 5114 | 6917 | -825 | -1890 | 2272 | O |
| ATOM | 16 | N | GLN | 1064 | -5.682 | 37.725 | 9.084 | 1.00 | 37.95 | | N |
| ANISOU | 16 | N | GLN | 1064 | 3174 | 4947 | 6299 | 383 | -1356 | 1245 | N |
| ATOM | 17 | CA | GLN | 1064 | -5.705 | 38.664 | 10.170 | 1.00 | 39.27 | | C |
| ANISOU | 17 | CA | GLN | 1064 | 4283 | 4277 | 6360 | 527 | -858 | 1541 | C |
| ATOM | 18 | CB | GLN | 1064 | -7.053 | 38.571 | 10.905 | 1.00 | 46.57 | | C |
| ANISOU | 18 | CB | GLN | 1064 | 4345 | 5743 | 7608 | 867 | -356 | 955 | C |
| ATOM | 23 | C | GLN | 1064 | -4.558 | 38.406 | 11.133 | 1.00 | 35.57 | | C |
| ANISOU | 23 | C | GLN | 1064 | 4284 | 3605 | 5627 | -193 | -745 | 1294 | C |
| ATOM | 24 | O | GLN | 1064 | -3.767 | 39.306 | 11.437 | 1.00 | 45.66 | | O |
| ANISOU | 24 | O | GLN | 1064 | 5423 | 2722 | 9203 | 389 | -1986 | 262 | O |
| ATOM | 25 | N | ALA | 1065 | -4.518 | 37.159 | 11.580 | 1.00 | 29.57 | | N |
| ANISOU | 25 | N | ALA | 1065 | 3049 | 3646 | 4541 | -263 | -123 | 1171 | N |
| ATOM | 26 | CA | ALA | 1065 | -3.497 | 36.787 | 12.549 | 1.00 | 29.84 | | C |
| ANISOU | 26 | CA | ALA | 1065 | 3405 | 3848 | 4084 | -976 | -258 | 1486 | C |
| ATOM | 27 | CB | ALA | 1065 | -3.687 | 35.306 | 12.883 | 1.00 | 32.47 | | |
| ANISOU | 27 | CB | ALA | 1065 | 3146 | 3579 | 5612 | -273 | -333 | 1426 | C |
| ATOM | 28 | C | ALA | 1065 | -2.089 | 37.054 | 12.067 | 1.00 | 25.87 | | C |
| ANISOU | 28 | C | ALA | 1065 | 3124 | 3301 | 3403 | -419 | -315 | 292 | C |
| ATOM | 29 | O | ALA | 1065 | -1.180 | 37.393 | 12.848 | 1.00 | 26.64 | | O |
| ANISOU | 29 | O | ALA | 1065 | 2881 | 4358 | 2883 | 204 | -198 | 17 | O |
| ATOM | 30 | N | VAL | 1066 | -1.835 | 36.917 | 10.762 | 1.00 | 26.85 | | N |
| ANISOU | 30 | N | VAL | 1066 | 2739 | 4255 | 3206 | -70 | -872 | 397 | N |
| ATOM | 31 | CA | VAL | 1066 | -0.436 | 37.056 | 10.364 | 1.00 | 26.36 | | C |
| ANISOU | 31 | CA | VAL | 1066 | 2950 | 3462 | 3605 | 287 | -304 | 343 | C |
| ATOM | 32 | CB | VAL | 1066 | -0.083 | 36.006 | 9.295 | 1.00 | 28.45 | | C |
| ANISOU | 32 | CB | VAL | 1066 | 3464 | 3113 | 4233 | -338 | -285 | -36 | C |
| ATOM | 33 | CG1 | VAL | 1066 | -0.453 | 34.603 | 9.772 | 1.00 | 36.19 | | C |
| ANISOU | 33 | CG1 | VAL | 1066 | 4167 | 3284 | 6301 | -985 | -1217 | 473 | C |
| ATOM | 34 | CG2 | VAL | 1066 | -0.769 | 36.395 | 7.984 | 1.00 | 30.30 | | C |
| ANISOU | 34 | CG2 | VAL | 1066 | 3985 | 3335 | 4193 | 907 | -483 | -732 | C |
| ATOM | 35 | C | VAL | 1066 | -0.092 | 38.411 | 9.781 | 1.00 | 24.00 | | C |
| ANISOU | 35 | C | VAL | 1066 | 2887 | 3186 | 3046 | 177 | -724 | -37 | C |
| ATOM | 36 | O | VAL | 1066 | 1.077 | 38.661 | 9.417 | 1.00 | 23.42 | | O |
| ANISOU | 36 | O | VAL | 1066 | 3052 | 2885 | 2960 | -124 | -441 | -920 | O |
| ATOM | 37 | N | GLN | 1067 | -1.054 | 39.310 | 9.668 | 1.00 | 23.79 | | N |
| ANISOU | 37 | N | GLN | 1067 | 3069 | 3328 | 2642 | 275 | -1049 | -182 | N |
| ATOM | 38 | CA | GLN | 1067 | -0.829 | 40.504 | 8.828 | 1.00 | 25.42 | | C |
| ANISOU | 38 | CA | GLN | 1067 | 3390 | 3279 | 2991 | 208 | -1380 | -72 | C |
| ATOM | 39 | CB | GLN | 1067 | -2.099 | 41.362 | 8.853 | 1.00 | 32.36 | | C |
| ANISOU | 39 | CB | GLN | 1067 | 3545 | 3823 | 4926 | 539 | -1139 | 626 | C |
| ATOM | 40 | CG | GLN | 1067 | -2.506 | 41.771 | 10.269 | 1.00 | 33.75 | | C |
| ANISOU | 40 | CG | GLN | 1067 | 3125 | 4206 | 5494 | 982 | -364 | 560 | C |
| ATOM | 41 | CD | GLN | 1067 | -3.599 | 42.819 | 10.158 | 1.00 | 39.88 | | C |
| ANISOU | 41 | CD | GLN | 1067 | 4158 | 4663 | 6334 | 1659 | -406 | 1208 | C |
| ATOM | 42 | OE1 | GLN | 1067 | -3.432 | 43.710 | 9.330 | 1.00 | 52.77 | | O |
| ANISOU | 42 | OE1 | GLN | 1067 | 5684 | 5689 | 8676 | 1385 | -832 | 2880 | O |
| ATOM | 43 | NE2 | GLN | 1067 | -4.650 | 42.688 | 10.963 | 1.00 | 38.22 | | N |
| ANISOU | 43 | NE2 | GLN | 1067 | 3384 | 4488 | 6650 | 1391 | -621 | -342 | N |
| ATOM | 44 | C | GLN | 1067 | 0.367 | 41.348 | 9.228 | 1.00 | 26.72 | | C |
| ANISOU | 44 | C | GLN | 1067 | 3500 | 3686 | 2968 | -132 | -932 | -91 | C |
| ATOM | 45 | O | GLN | 1067 | 0.971 | 41.896 | 8.291 | 1.00 | 30.88 | | O |
| ANISOU | 45 | O | GLN | 1067 | 4066 | 4260 | 3409 | -151 | 500 | -870 | O |
| ATOM | 46 | N | HIS | 1068 | 0.749 | 41.488 | 10.490 | 1.00 | 22.49 | | N |
| ANISOU | 46 | N | HIS | 1068 | 2696 | 2632 | 3219 | 765 | -1235 | -534 | N |
| ATOM | 47 | CA | HIS | 1068 | 1.878 | 42.369 | 10.817 | 1.00 | 20.79 | | C |
| ANISOU | 47 | CA | HIS | 1068 | 2761 | 2235 | 2903 | 499 | -528 | -167 | C |
| ATOM | 48 | CB | HIS | 1068 | 1.599 | 43.204 | 12.077 | 1.00 | 24.08 | | C |
| ANISOU | 48 | CB | HIS | 1068 | 2417 | 3415 | 3319 | -294 | -74 | -938 | C |
| ATOM | 49 | CG | HIS | 1068 | 0.326 | 44.002 | 12.029 | 1.00 | 23.87 | | C |
| ANISOU | 49 | CG | HIS | 1068 | 3189 | 3310 | 2570 | 239 | -466 | -1342 | C |
| ATOM | 50 | CD2 | HIS | 1068 | -0.836 | 43.766 | 12.720 | 1.00 | 24.71 | | C |
| ANISOU | 50 | CD2 | HIS | 1068 | 2664 | 3594 | 3129 | 683 | -612 | -1166 | C |
| ATOM | 51 | ND1 | HIS | 1068 | 0.072 | 45.120 | 11.276 | 1.00 | 26.89 | | N |
| ANISOU | 51 | ND1 | HIS | 1068 | 3883 | 3106 | 3226 | -228 | -1210 | -1263 | N |
| ATOM | 52 | CE1 | HIS | 1068 | -1.160 | 45.547 | 11.492 | 1.00 | 30.43 | | C |
| ANISOU | 52 | CE1 | HIS | 1068 | 3833 | 3687 | 4041 | 215 | -1728 | -845 | C |
| ATOM | 53 | NE2 | HIS | 1068 | -1.726 | 44.730 | 12.373 | 1.00 | 28.63 | | N |
| ANISOU | 53 | NE2 | HIS | 1068 | 3424 | 3481 | 3975 | 942 | -928 | -1207 | N |
| ATOM | 54 | C | HIS | 1068 | 3.158 | 41.560 | 11.002 | 1.00 | 21.05 | | C |
| ANISOU | 54 | C | HIS | 1068 | 2589 | 1916 | 3491 | 168 | -955 | -173 | C |
| ATOM | 55 | O | HIS | 1068 | 4.155 | 42.094 | 11.526 | 1.00 | 21.33 | | O |
| ANISOU | 55 | O | HIS | 1068 | 2663 | 2336 | 3105 | -43 | -627 | -326 | O |
| ATOM | 56 | N | VAL | 1069 | 3.167 | 40.288 | 10.587 | 1.00 | 19.97 | | N |
| ANISOU | 56 | N | VAL | 1069 | 2603 | 2209 | 2777 | 347 | -611 | -509 | N |
| ATOM | 57 | CA | VAL | 1069 | 4.419 | 39.516 | 10.717 | 1.00 | 19.24 | | C |
| ANISOU | 57 | CA | VAL | 1069 | 2820 | 2362 | 2129 | 558 | -190 | 143 | C |
| ATOM | 58 | CB | VAL | 1069 | 4.316 | 38.354 | 11.715 | 1.00 | 22.02 | | C |
| ANISOU | 58 | CB | VAL | 1069 | 3642 | 2341 | 2384 | 308 | -63 | 151 | C |
| ATOM | 59 | CG1 | VAL | 1069 | 4.023 | 38.874 | 13.117 | 1.00 | 28.55 | | C |
| ANISOU | 59 | CG1 | VAL | 1069 | 5153 | 3362 | 2334 | 403 | 598 | 264 | C |
| ATOM | 60 | CG2 | VAL | 1069 | 3.246 | 37.357 | 11.278 | 1.00 | 24.46 | | C |
| ANISOU | 60 | CG2 | VAL | 1069 | 3088 | 3407 | 2799 | -232 | -46 | 795 | C |
| ATOM | 61 | C | VAL | 1069 | 4.858 | 38.995 | 9.350 | 1.00 | 18.10 | | C |
| ANISOU | 61 | C | VAL | 1069 | 2737 | 2084 | 2054 | 419 | -635 | -116 | C |
| ATOM | 62 | O | VAL | 1069 | 5.775 | 38.193 | 9.242 | 1.00 | 18.22 | | O |
| ANISOU | 62 | O | VAL | 1069 | 2723 | 2181 | 2019 | 453 | -330 | 170 | O |
| ATOM | 63 | N | VAL | 1070 | 4.215 | 39.458 | 8.266 | 1.00 | 19.19 | | N |
| ANISOU | 63 | N | VAL | 1070 | 2697 | 2437 | 2157 | 385 | -602 | 44 | N |
| ATOM | 64 | CA | VAL | 1070 | 4.619 | 39.011 | 6.944 | 1.00 | 19.24 | | C |
| ANISOU | 64 | CA | VAL | 1070 | 2347 | 2944 | 2019 | 217 | -469 | 136 | C |
| ATOM | 65 | CB | VAL | 1070 | 3.503 | 39.187 | 5.900 | 1.00 | 22.58 | | C |
| ANISOU | 65 | CB | VAL | 1070 | 2536 | 3873 | 2170 | 58 | -613 | 325 | C |
| ATOM | 66 | CG1 | VAL | 1070 | 3.971 | 38.874 | 4.499 | 1.00 | 28.99 | | C |
| ANISOU | 66 | CG1 | VAL | 1070 | 3261 | 5784 | 1971 | 702 | -704 | 513 | C |
| ATOM | 67 | CG2 | VAL | 1070 | 2.330 | 38.275 | 6.266 | 1.00 | 24.98 | | C |
| ANISOU | 67 | CG2 | VAL | 1070 | 3104 | 3947 | 2440 | -713 | -721 | -585 | C |
| ATOM | 68 | C | VAL | 1070 | 5.874 | 39.792 | 6.541 | 1.00 | 19.01 | | C |
| ANISOU | 68 | C | VAL | 1070 | 2659 | 2193 | 2372 | 65 | -559 | 75 | C |
| ATOM | 69 | O | VAL | 1070 | 5.982 | 41.014 | 6.711 | 1.00 | 24.45 | | O |
| ANISOU | 69 | O | VAL | 1070 | 4066 | 2110 | 3114 | 249 | -535 | 149 | O |
| ATOM | 70 | N | ILE | 1071 | 6.825 | 39.063 | 6.003 | 1.00 | 17.21 | | N |
| ANISOU | 70 | N | ILE | 1071 | 2468 | 2035 | 2034 | 44 | -406 | 676 | N |
| ATOM | 71 | CA | ILE | 1071 | 8.101 | 39.583 | 5.519 | 1.00 | 17.62 | | C |
| ANISOU | 71 | CA | ILE | 1071 | 2679 | 2026 | 1989 | -295 | -480 | 388 | C |
| ATOM | 72 | CB | ILE | 1071 | 9.289 | 38.803 | 6.101 | 1.00 | 20.55 | | C |
| ANISOU | 72 | CB | ILE | 1071 | 2449 | 2963 | 2398 | -146 | -535 | 340 | C |
| ATOM | 73 | CG2 | ILE | 1071 | 10.570 | 39.163 | 5.366 | 1.00 | 24.19 | | C |
| ANISOU | 73 | CG2 | ILE | 1071 | 2854 | 2460 | 3876 | 178 | 188 | 899 | C |
| ATOM | 74 | CG1 | ILE | 1071 | 9.381 | 38.973 | 7.635 | 1.00 | 21.09 | | C |
| ANISOU | 74 | CG1 | ILE | 1071 | 3261 | 2347 | 2407 | -491 | -1080 | 417 | C |
| ATOM | 75 | CD1 | ILE | 1071 | 10.243 | 37.970 | 8.359 | 1.00 | 21.91 | | C |
| ANISOU | 75 | CD1 | ILE | 1071 | 3147 | 2946 | 2233 | 348 | 127 | 799 | C |
| ATOM | 76 | C | ILE | 1071 | 8.133 | 39.519 | 3.993 | 1.00 | 19.93 | | C |
| ANISOU | 76 | C | ILE | 1071 | 3336 | 2256 | 1980 | 182 | -104 | 412 | C |
| ATOM | 77 | O | ILE | 1071 | 7.976 | 38.429 | 3.412 | 1.00 | 21.78 | | O |
| ANISOU | 77 | O | ILE | 1071 | 3638 | 2597 | 2039 | -228 | -477 | 213 | O |
| ATOM | 78 | N | GLY | 1072 | 8.324 | 40.654 | 3.328 | 1.00 | 20.86 | | N |
| ANISOU | 78 | N | GLY | 1072 | 3232 | 2498 | 2196 | 246 | -255 | 751 | N |
| ATOM | 79 | CA | GLY | 1072 | 8.367 | 40.662 | 1.868 | 1.00 | 24.04 | | C |
| ANISOU | 79 | CA | GLY | 1072 | 3777 | 3106 | 2250 | 169 | 160 | 780 | C |
| ATOM | 80 | C | GLY | 1072 | 9.644 | 40.009 | 1.360 | 1.00 | 20.97 | | C |
| ANISOU | 80 | C | GLY | 1072 | 3001 | 2894 | 2074 | -549 | -441 | 61 | C |
| ATOM | 81 | O | GLY | 1072 | 10.716 | 40.053 | 1.976 | 1.00 | 19.86 | | O |
| ANISOU | 81 | O | GLY | 1072 | 3387 | 2343 | 1815 | -788 | -788 | 590 | O |
| ATOM | 82 | N | PRO | 1073 | 9.510 | 39.381 | 0.195 | 1.00 | 21.09 | | N |
| ANISOU | 82 | N | PRO | 1073 | 3042 | 2647 | 2324 | -460 | -767 | -114 | N |
| ATOM | 83 | CD | PRO | 1073 | 8.346 | 39.297 | -0.664 | 1.00 | 26.42 | | C |
| ANISOU | 83 | CD | PRO | 1073 | 3184 | 3883 | 2972 | -309 | -1038 | -617 | C |
| ATOM | 84 | CA | PRO | 1073 | 10.659 | 38.651 | -0.329 | 1.00 | 20.61 | | C |
| ANISOU | 84 | CA | PRO | 1073 | 3246 | 2358 | 2227 | -393 | -649 | 95 | C |
| ATOM | 85 | CB | PRO | 1073 | 10.211 | 38.125 | -1.706 | 1.00 | 24.26 | | C |
| ANISOU | 85 | CB | PRO | 1073 | 3874 | 2924 | 2419 | -259 | -783 | -305 | C |
| ATOM | 86 | CG | PRO | 1073 | 8.817 | 38.584 | -1.906 | 1.00 | 29.68 | | C |
| ANISOU | 86 | CG | PRO | 1073 | 3674 | 4569 | 3035 | -339 | -1211 | -1081 | C |
| ATOM | 87 | C | PRO | 1073 | 11.867 | 39.551 | -0.513 | 1.00 | 19.99 | | C |
| ANISOU | 87 | C | PRO | 1073 | 3129 | 2452 | 2014 | -290 | -657 | 592 | C |
| ATOM | 88 | O | PRO | 1073 | 12.984 | 39.039 | -0.367 | 1.00 | 23.20 | | O |
| ANISOU | 88 | O | PRO | 1073 | 3174 | 2549 | 3093 | -117 | -902 | -382 | O |
| ATOM | 89 | N | ASER | 1074 | 11.719 | 40.845 | -0.828 | 0.50 | 21.38 | | N |
| ANISOU | 89 | N | ASER | 1074 | 3775 | 2431 | 1919 | -444 | -1016 | 625 | N |
| ATOM | 90 | N | BSER | 1074 | 11.651 | 40.837 | -0.819 | 0.50 | 20.89 | | N |
| ANISOU | 90 | N | BSER | 1074 | 3635 | 2384 | 1917 | -418 | -1029 | 483 | N |
| ATOM | 91 | CA | ASER | 1074 | 12.963 | 41.605 | -1.046 | 0.50 | 21.22 | | C |
| ANISOU | 91 | CA | ASER | 1074 | 3769 | 2360 | 1933 | -309 | -825 | 840 | C |
| ATOM | 92 | CA | BSER | 1074 | 12.823 | 41.696 | -1.057 | 0.50 | 21.96 | | C |
| ANISOU | 92 | CA | BSER | 1074 | 3905 | 2426 | 2013 | -537 | -752 | 478 | C |
| ATOM | 93 | CB | ASER | 1074 | 12.713 | 42.890 | -1.841 | 0.50 | 23.69 | | C |
| ANISOU | 93 | CB | ASER | 1074 | 4389 | 2301 | 2312 | -186 | -719 | 878 | C |
| ATOM | 94 | CB | BSER | 1074 | 12.418 | 42.950 | -1.842 | 0.50 | 23.74 | | C |
| ANISOU | 94 | CB | BSER | 1074 | 4454 | 2561 | 2004 | -530 | -783 | 628 | C |
| ATOM | 95 | OG | ASER | 1074 | 11.826 | 43.718 | -1.106 | 0.50 | 23.29 | | O |
| ANISOU | 95 | OG | ASER | 1074 | 4566 | 2054 | 2230 | -329 | -972 | 514 | O |
| ATOM | 96 | OG | BSER | 1074 | 12.319 | 42.655 | -3.234 | 0.50 | 23.49 | | O |
| ANISOU | 96 | OG | BSER | 1074 | 4211 | 2616 | 2100 | -301 | -886 | 395 | O |
| ATOM | 97 | C | ASER | 1074 | 13.653 | 41.966 | 0.268 | 0.50 | 19.51 | | C |
| ANISOU | 97 | C | ASER | 1074 | 3408 | 2037 | 1968 | -397 | -513 | 454 | C |
| ATOM | 98 | C | BSER | 1074 | 13.527 | 42.075 | 0.244 | 0.50 | 19.59 | | C |
| ANISOU | 98 | C | BSER | 1074 | 3591 | 1874 | 1980 | -594 | -555 | 440 | C |
| ATOM | 99 | O | ASER | 1074 | 14.744 | 42.537 | 0.266 | 0.50 | 20.53 | | O |
| ANISOU | 99 | O | ASER | 1074 | 3631 | 1737 | 2434 | -552 | -421 | 543 | O |
| ATOM | 100 | O | BSER | 1074 | 14.544 | 42.770 | 0.213 | 0.50 | 21.14 | | O |
| ANISOU | 100 | O | BSER | 1074 | 3500 | 2093 | 2437 | -616 | -157 | -2 | O |
| ATOM | 101 | N | SER | 1075 | 13.007 | 41.625 | 1.386 | 1.00 | 18.05 | | N |
| ANISOU | 101 | N | SER | 1075 | 3116 | 1811 | 1930 | -27 | -533 | 486 | N |
| ATOM | 102 | CA | SER | 1075 | 13.591 | 41.840 | 2.704 | 1.00 | 16.60 | | C |
| ANISOU | 102 | CA | SER | 1075 | 2940 | 1414 | 1953 | -102 | -570 | 254 | C |
| ATOM | 103 | CB | SER | 1075 | 12.504 | 42.149 | 3.723 | 1.00 | 20.80 | | C |
| ANISOU | 103 | CB | SER | 1075 | 3261 | 2548 | 2094 | 635 | -567 | 87 | C |
| ATOM | 104 | OG | SER | 1075 | 11.780 | 43.313 | 3.385 | 1.00 | 24.33 | | O |
| ANISOU | 104 | OG | SER | 1075 | 2999 | 2848 | 3398 | 572 | 98 | 985 | O |
| ATOM | 105 | C | SER | 1075 | 14.363 | 40.604 | 3.154 | 1.00 | 17.00 | | C |
| ANISOU | 105 | C | SER | 1075 | 2930 | 1658 | 1871 | 82 | -579 | 154 | C |
| ATOM | 106 | O | SER | 1075 | 14.993 | 40.657 | 4.243 | 1.00 | 17.81 | | O |
| ANISOU | 106 | O | SER | 1075 | 3173 | 1499 | 2094 | -174 | -825 | 321 | O |
| ATOM | 107 | N | LEU | 1076 | 14.373 | 39.491 | 2.418 | 1.00 | 16.66 | | N |
| ANISOU | 107 | N | LEU | 1076 | 2809 | 1512 | 2010 | 53 | -486 | 183 | N |
| ATOM | 108 | CA | LEU | 1076 | 14.914 | 38.246 | 2.961 | 1.00 | 16.17 | | C |
| ANISOU | 108 | CA | LEU | 1076 | 2639 | 1649 | 1854 | 32 | -286 | 289 | C |
| ATOM | 109 | CB | LEU | 1076 | 13.784 | 37.248 | 3.285 | 1.00 | 18.47 | | C |
| ANISOU | 109 | CB | LEU | 1076 | 2876 | 1752 | 2390 | -219 | -595 | 467 | C |
| ATOM | 110 | CG | LEU | 1076 | 14.196 | 35.914 | 3.935 | 1.00 | 18.88 | | C |
| ANISOU | 110 | CG | LEU | 1076 | 3203 | 1798 | 2175 | -213 | -481 | 477 | C |
| ATOM | 111 | CD1 | LEU | 1076 | 14.861 | 36.139 | 5.283 | 1.00 | 18.69 | | C |
| ANISOU | 111 | CD1 | LEU | 1076 | 3510 | 1756 | 1834 | -176 | -228 | 480 | C |
| ATOM | 112 | CD2 | LEU | 1076 | 12.962 | 35.021 | 4.014 | 1.00 | 19.42 | | C |
| ANISOU | 112 | CD2 | LEU | 1076 | 3376 | 1913 | 2088 | -335 | -104 | 519 | C |
| ATOM | 113 | C | LEU | 1076 | 15.835 | 37.577 | 1.999 | 1.00 | 17.58 | | C |
| ANISOU | 113 | C | LEU | 1076 | 2935 | 1965 | 1781 | 424 | -486 | 134 | C |
| ATOM | 114 | O | LEU | 1076 | 15.358 | 37.347 | 0.857 | 1.00 | 19.61 | | O |
| ANISOU | 114 | O | LEU | 1076 | 3138 | 2208 | 2105 | 303 | -705 | -283 | O |
| ATOM | 115 | N | ILE | 1077 | 17.038 | 37.293 | 2.448 | 1.00 | 16.49 | | N |
| ANISOU | 115 | N | ILE | 1077 | 2899 | 1747 | 1620 | 341 | -334 | 308 | N |
| ATOM | 116 | CA | ILE | 1077 | 17.989 | 36.499 | 1.679 | 1.00 | 17.91 | | C |
| ANISOU | 116 | CA | ILE | 1077 | 2927 | 1728 | 2150 | 330 | -3 | 355 | C |
| ATOM | 117 | CB | ILE | 1077 | 19.315 | 37.249 | 1.550 | 1.00 | 19.48 | | C |
| ANISOU | 117 | CB | ILE | 1077 | 3117 | 2014 | 2268 | 99 | 132 | 333 | C |
| ATOM | 118 | CG2 | ILE | 1077 | 20.427 | 36.397 | 0.954 | 1.00 | 24.13 | | C |
| ANISOU | 118 | CG2 | ILE | 1077 | 2937 | 2291 | 3940 | 311 | 310 | 441 | C |
| ATOM | 119 | CG1 | ILE | 1077 | 19.155 | 38.514 | 0.730 | 1.00 | 24.54 | | C |
| ANISOU | 119 | CG1 | ILE | 1077 | 3840 | 1863 | 3619 | 181 | 368 | 672 | C |
| ATOM | 120 | CD1 | ILE | 1077 | 20.045 | 39.672 | 1.155 | 1.00 | 37.76 | | C |
| ANISOU | 120 | CD1 | ILE | 1077 | 6042 | 3146 | 5160 | -1742 | -416 | 1530 | C |
| ATOM | 121 | C | ILE | 1077 | 18.162 | 35.142 | 2.357 | 1.00 | 16.43 | | C |
| ANISOU | 121 | C | ILE | 1077 | 2982 | 1620 | 1640 | 209 | -301 | 61 | C |
| ATOM | 122 | O | ILE | 1077 | 18.505 | 35.125 | 3.554 | 1.00 | 20.58 | | O |
| ANISOU | 122 | O | ILE | 1077 | 3818 | 2134 | 1869 | 160 | -921 | 37 | O |
| ATOM | 123 | N | VAL | 1078 | 17.937 | 34.063 | 1.671 | 1.00 | 16.17 | | N |
| ANISOU | 123 | N | VAL | 1078 | 2951 | 1684 | *1509* | 130 | -255 | 111 | N |
| ATOM | 124 | CA | VAL | 1078 | 18.115 | 32.719 | 2.247 | 1.00 | 15.97 | | C |
| ANISOU | 124 | CA | VAL | 1078 | 2765 | 1675 | 1629 | 147 | -139 | 183 | C |
| ATOM | 125 | CB | VAL | 1078 | 16.869 | 31.839 | 2.048 | 1.00 | 17.27 | | C |
| ANISOU | 125 | CB | VAL | 1078 | 2873 | 1638 | 2050 | 152 | -117 | 101 | C |
| ATOM | 126 | CG1 | VAL | 1078 | 17.032 | 30.506 | 2.816 | 1.00 | 19.12 | | C |
| ANISOU | 126 | CG1 | VAL | 1078 | 3317 | 1751 | 2196 | 14 | -35 | 324 | C |
| ATOM | 127 | CG2 | VAL | 1078 | 15.607 | 32.560 | 2.479 | 1.00 | 18.41 | | C |
| ANISOU | 127 | CG2 | VAL | 1078 | 2684 | 2246 | 2065 | 215 | -259 | -22 | C |
| ATOM | 128 | C | VAL | 1078 | 19.340 | 32.062 | 1.631 | 1.00 | 16.01 | | C |
| ANISOU | 128 | C | VAL | 1078 | 2903 | 1693 | 1489 | 133 | -52 | 162 | C |
| ATOM | 129 | O | VAL | 1078 | 19.470 | 31.991 | 0.386 | 1.00 | 18.40 | | O |
| ANISOU | 129 | O | VAL | 1078 | 3189 | 2277 | 1524 | 150 | -103 | -36 | O |
| ATOM | 130 | N | AHIS | 1079 | 20.242 | 31.576 | 2.472 | 0.50 | 16.40 | | N |
| ANISOU | 130 | N | AHIS | 1079 | 2797 | 1848 | 1587 | 253 | -23 | 81 | N |
| ATOM | 131 | N | BHIS | 1079 | 20.261 | 31.553 | 2.440 | 0.50 | 16.51 | | N |
| ANISOU | 131 | N | BHIS | 1079 | 2765 | 1870 | 1638 | 195 | -94 | 52 | N |
| ATOM | 132 | CA | AHIS | 1079 | 21.430 | 30.839 | 2.028 | 0.50 | 17.26 | | C |
| ANISOU | 132 | CA | AHIS | 1079 | 2998 | 1873 | 1687 | 359 | 10 | -92 | C |
| ATOM | 133 | CA | BHIS | 1079 | 21.423 | 30.842 | 1.894 | 0.50 | 17.62 | | C |
| ANISOU | 133 | CA | BHIS | 1079 | 2873 | 1936 | 1885 | 236 | -56 | -113 | C |
| ATOM | 134 | CB | AHIS | 1079 | 22.609 | 31.159 | 2.920 | 0.50 | 19.02 | | C |
| ANISOU | 134 | CB | AHIS | 1079 | 2578 | 2285 | 2363 | -268 | 87 | 509 | C |
| ATOM | 135 | CB | BHIS | 1079 | 22.676 | 31.237 | 2.653 | 0.50 | 18.95 | | C |
| ANISOU | 135 | CB | BHIS | 1079 | 2648 | 2301 | 2253 | -231 | 33 | 566 | C |
| ATOM | 136 | CG | AHIS | 1079 | 22.965 | 32.591 | 3.166 | 0.50 | 22.35 | | C |
| ANISOU | 136 | CG | AHIS | 1079 | 3565 | 2384 | 2542 | -618 | -354 | 607 | C |
| ATOM | 137 | CG | BHIS | 1079 | 22.964 | 32.700 | 2.506 | 0.50 | 24.20 | | C |
| ANISOU | 137 | CG | BHIS | 1079 | 3723 | 2499 | 2973 | -792 | -78 | 599 | C |
| ATOM | 138 | CD2AHIS | | 1079 | 23.938 | 33.368 | 2.626 | 0.50 | 26.85 | | C |
| ANISOU | 138 | CD2AHIS | | 1079 | 4255 | 2688 | 3257 | -1127 | -63 | 545 | C |
| ATOM | 139 | CD2BHIS | | 1079 | 23.498 | 33.364 | 1.459 | 0.50 | 25.31 | | C |
| ANISOU | 139 | CD2BHIS | | 1079 | 4723 | 2566 | 2328 | -709 | -1039 | 1229 | C |
| ATOM | 140 | ND1AHIS | | 1079 | 22.316 | 33.420 | 4.042 | 0.50 | 24.01 | | N |
| ANISOU | 140 | ND1AHIS | | 1079 | 4560 | 2121 | 2443 | -751 | -239 | 487 | N |
| ATOM | 141 | ND1BHIS | | 1079 | 22.699 | 33.635 | 3.478 | 0.50 | 25.67 | | N |
| ANISOU | 141 | ND1BHIS | | 1079 | 4025 | 2397 | 3332 | -1141 | -218 | 316 | N |
| ATOM | 142 | CE1AHIS | | 1079 | 22.844 | 34.627 | 4.053 | 0.50 | 24.86 | | C |
| ANISOU | 142 | CE1AHIS | | 1079 | 4407 | 2522 | 2518 | -1179 | -730 | 371 | C |
| ATOM | 143 | CE1BHIS | | 1079 | 23.066 | 34.827 | 3.037 | 0.50 | 27.09 | | C |
| ANISOU | 143 | CE1BHIS | | 1079 | 4668 | 2395 | 3230 | -872 | -884 | 828 | C |
| ATOM | 144 | NE2AHIS | | 1079 | 23.854 | 34.620 | 3.182 | 0.50 | 27.29 | | N |
| ANISOU | 144 | NE2AHIS | | 1079 | 4446 | 2629 | 3293 | -1071 | -399 | 589 | N |
| ATOM | 145 | NE2BHIS | | 1079 | 23.553 | 34.684 | 1.820 | 0.50 | 26.51 | | N |
| ANISOU | 145 | NE2BHIS | | 1079 | 4897 | 2558 | 2616 | -722 | -1431 | 1250 | N |
| ATOM | 146 | C | AHIS | 1079 | 21.048 | 29.364 | 2.013 | 0.50 | 17.00 | | C |
| ANISOU | 146 | C | AHIS | 1079 | 2948 | 1929 | 1583 | 279 | 9 | -102 | C |
| ATOM | 147 | C | BHIS | 1079 | 21.164 | 29.347 | 1.955 | 0.50 | 16.82 | | C |
| ANISOU | 147 | C | BHIS | 1079 | 2883 | 1939 | 1567 | 227 | 3 | -32 | C |
| ATOM | 148 | O | AHIS | 1079 | 21.161 | 28.669 | 3.030 | 0.50 | 16.03 | | O |
| ANISOU | 148 | O | AHIS | 1079 | 2408 | 1917 | 1765 | 223 | -196 | -50 | O |
| ATOM | 149 | O | BHIS | 1079 | 21.480 | 28.687 | 2.946 | 0.50 | 17.29 | | O |
| ANISOU | 149 | O | BHIS | 1079 | 3081 | 2034 | 1454 | 718 | 227 | -161 | O |
| ATOM | 150 | N | PHE | 1080 | 20.579 | 28.836 | 0.882 | 1.00 | 17.39 | | N |
| ANISOU | 150 | N | PHE | 1080 | 2954 | 2085 | 1568 | 147 | 99 | -187 | N |
| ATOM | 151 | CA | PHE | 1080 | 20.081 | 27.430 | 0.904 | 1.00 | 16.92 | | C |
| ANISOU | 151 | CA | PHE | 1080 | 2460 | 2201 | 1769 | 113 | 247 | -233 | C |
| ATOM | 152 | CB | PHE | 1080 | 19.147 | 27.213 | -0.304 | 1.00 | 18.38 | | C |
| ANISOU | 152 | CB | PHE | 1080 | 2637 | 2391 | 1955 | -124 | 55 | 38 | C |
| ATOM | 153 | CG | PHE | 1080 | 17.754 | 27.797 | -0.090 | 1.00 | 17.89 | | C |
| ANISOU | 153 | CG | PHE | 1080 | 2753 | 2269 | 1773 | 17 | -128 | -72 | C |
| ATOM | 154 | CD1 | PHE | 1080 | 16.828 | 27.160 | 0.729 | 1.00 | 20.14 | | C |
| ANISOU | 154 | CD1 | PHE | 1080 | 2675 | 2920 | 2059 | 79 | 116 | 31 | C |
| ATOM | 155 | CD2 | PHE | 1080 | 17.379 | 28.984 | -0.711 | 1.00 | 19.48 | | C |
| ANISOU | 155 | CD2 | PHE | 1080 | 3443 | 1989 | 1971 | 203 | -99 | -384 | C |
| ATOM | 156 | CE1 | PHE | 1080 | 15.561 | 27.654 | 0.948 | 1.00 | 21.11 | | C |
| ANISOU | 156 | CE1 | PHE | 1080 | 2562 | 3022 | 2436 | 106 | -172 | 60 | C |
| ATOM | 157 | CE2 | PHE | 1080 | 16.101 | 29.489 | -0.510 | 1.00 | 19.43 | | C |
| ANISOU | 157 | CE2 | PHE | 1080 | 3349 | 2023 | 2012 | 247 | -336 | -589 | C |
| ATOM | 158 | CZ | PHE | 1080 | 15.197 | 28.833 | 0.312 | 1.00 | 22.31 | | C |
| ANISOU | 158 | CZ | PHE | 1080 | 2990 | 2438 | 3047 | -23 | -387 | -282 | C |
| ATOM | 159 | C | PHE | 1080 | 21.182 | 26.387 | 0.943 | 1.00 | 16.85 | | C |
| ANISOU | 159 | C | PHE | 1080 | 2541 | 2090 | 1770 | 65 | 292 | -117 | C |
| ATOM | 160 | O | PHE | 1080 | 20.882 | 25.213 | 1.136 | 1.00 | 20.88 | | O |
| ANISOU | 160 | O | PHE | 1080 | 3183 | 2038 | 2713 | 14 | 531 | -141 | O |
| ATOM | 161 | N | ASN | 1081 | 22.439 | 26.760 | 0.786 | 1.00 | 16.77 | | N |
| ANISOU | 161 | N | ASN | 1081 | 2418 | 2420 | 1535 | 126 | 75 | -223 | N |
| ATOM | 162 | CA | ASN | 1081 | 23.563 | 25.841 | 0.950 | 1.00 | 18.90 | | C |
| ANISOU | 162 | CA | ASN | 1081 | 2641 | 2753 | 1789 | 387 | 47 | -362 | C |
| ATOM | 163 | CB | ASN | 1081 | 24.627 | 26.074 | -0.125 | 1.00 | 21.87 | | C |
| ANISOU | 163 | CB | ASN | 1081 | 2589 | 3368 | 2353 | 507 | 327 | -140 | C |
| ATOM | 164 | CG | ASN | 1081 | 24.146 | 25.632 | -1.496 | 1.00 | 24.19 | | C |
| ANISOU | 164 | CG | ASN | 1081 | 3816 | 3383 | 1991 | 258 | 590 | -17 | C |
| ATOM | 165 | OD1 | ASN | 1081 | 24.726 | 26.169 | -2.454 | 1.00 | 29.42 | | O |
| ANISOU | 165 | OD1 | ASN | 1081 | 3861 | 4794 | 2524 | 356 | 1052 | 397 | O |
| ATOM | 166 | ND2 | ASN | 1081 | 23.177 | 24.733 | -1.679 | 1.00 | 24.15 | | N |
| ANISOU | 166 | ND2 | ASN | 1081 | 4914 | 2433 | 1831 | 227 | 260 | -254 | N |
| ATOM | 167 | C | ASN | 1081 | 24.180 | 25.968 | 2.342 | 1.00 | 19.43 | | C |
| ANISOU | 167 | C | ASN | 1081 | 2956 | 2350 | 2075 | 577 | -300 | -435 | C |
| ATOM | 168 | O | ASN | 1081 | 25.172 | 25.284 | 2.598 | 1.00 | 19.66 | | O |
| ANISOU | 168 | O | ASN | 1081 | 2514 | 2746 | 2210 | 437 | -132 | -400 | O |
| ATOM | 169 | N | AGLU | 1082 | 23.593 | 26.803 | 3.196 | 0.50 | 16.40 | | N |
| ANISOU | 169 | N | AGLU | 1082 | 2643 | 1940 | 1647 | 124 | -72 | -111 | N |
| ATOM | 170 | N | BGLU | 1082 | 23.633 | 26.800 | 3.230 | 0.50 | 16.73 | | N |
| ANISOU | 170 | N | BGLU | 1082 | 2713 | 1981 | 1663 | 177 | -98 | -123 | N |
| ATOM | 171 | CA | AGLU | 1082 | 24.002 | 26.914 | 4.590 | 0.50 | 18.05 | | C |
| ANISOU | 171 | CA | AGLU | 1082 | 2915 | 2211 | 1731 | -217 | -228 | -59 | C |
| ATOM | 172 | CA | BGLU | 1082 | 24.117 | 26.915 | 4.602 | 0.50 | 18.05 | | C |
| ANISOU | 172 | CA | BGLU | 1082 | 2897 | 2200 | 1762 | -201 | -244 | -68 | C |
| ATOM | 173 | CB | AGLU | 1082 | 24.268 | 28.368 | 4.942 | 0.50 | 20.25 | | C |
| ANISOU | 173 | CB | AGLU | 1082 | 3181 | 2425 | 2089 | -548 | -351 | -252 | C |
| ATOM | 174 | CB | BGLU | 1082 | 24.411 | 28.355 | 4.980 | 0.50 | 20.64 | | C |
| ANISOU | 174 | CB | BGLU | 1082 | 3301 | 2389 | 2151 | -559 | -283 | -215 | C |
| ATOM | 175 | CG | AGLU | 1082 | 25.527 | 28.908 | 4.279 | 0.50 | 24.08 | | C |
| ANISOU | 175 | CG | AGLU | 1082 | 3025 | 3060 | 3065 | -848 | -417 | -116 | C |
| ATOM | 176 | CG | BGLU | 1082 | 25.566 | 29.068 | 4.328 | 0.50 | 24.46 | | C |
| ANISOU | 176 | CG | BGLU | 1082 | 3148 | 3101 | 3045 | -1017 | -213 | -477 | C |
| ATOM | 177 | CD | AGLU | 1082 | 25.698 | 30.368 | 4.642 | 0.50 | 24.26 | | C |
| ANISOU | 177 | CD | AGLU | 1082 | 2477 | 3138 | 3602 | -840 | -315 | -386 | C |
| ATOM | 178 | CD | BGLU | 1082 | 26.921 | 28.451 | 4.590 | 0.50 | 28.74 | | C |
| ANISOU | 178 | CD | BGLU | 1082 | 3299 | 3660 | 3962 | -662 | -192 | -512 | C |
| ATOM | 179 | OE1AGLU | | 1082 | 25.478 | 30.696 | 5.818 | 0.50 | 31.54 | | O1- |
| ANISOU | 179 | OE1AGLU | | 1082 | 3548 | 3995 | 4440 | -926 | 857 | -1176 | O1- |
| ATOM | 180 | OE1BGLU | | 1082 | 27.156 | 27.855 | 5.662 | 0.50 | 31.80 | | O1- |
| ANISOU | 180 | OE1BGLU | | 1082 | 4268 | 2629 | 5187 | -603 | -805 | 44 | O1- |
| ATOM | 181 | OE2AGLU | | 1082 | 26.045 | 31.170 | 3.770 | 0.50 | 28.09 | | O |
| ANISOU | 181 | OE2AGLU | | 1082 | 3921 | 2887 | 3863 | -344 | -1290 | 353 | O |
| ATOM | 182 | OE2BGLU | | 1082 | 27.771 | 28.572 | 3.676 | 0.50 | 37.61 | | O |
| ANISOU | 182 | OE2BGLU | | 1082 | 3296 | 6069 | 4925 | -868 | 347 | -874 | O |
| ATOM | 183 | C | AGLU | 1082 | 22.927 | 26.322 | 5.485 | 0.50 | 17.58 | | C |
| ANISOU | 183 | C | AGLU | 1082 | 3158 | 1863 | 1658 | 88 | -36 | -22 | C |
| ATOM | 184 | C | BGLU | 1082 | 23.076 | 26.333 | 5.556 | 0.50 | 17.03 | | C |
| ANISOU | 184 | C | BGLU | 1082 | 2990 | 1850 | 1630 | 47 | -106 | -192 | C |
| ATOM | 185 | O | AGLU | 1082 | 22.025 | 26.997 | 5.975 | 0.50 | 16.90 | | O |
| ANISOU | 185 | 0 | AGLU | 1082 | 3084 | 1731 | 1606 | 46 | -135 | 5 | O |
| ATOM | 186 | O | BGLU | 1082 | 22.306 | 27.084 | 6.160 | 0.50 | 20.02 | | O |
| ANISOU | 186 | O | BGLU | 1082 | 3596 | 2197 | 1814 | 490 | 131 | -61 | O |
| ATOM | 187 | N | VAL | 1083 | 23.070 | 25.010 | 5.667 | 1.00 | 18.03 | | N |
| ANISOU | 187 | N | VAL | 1083 | 2939 | 1936 | 1975 | 225 | -167 | 174 | N |
| ATOM | 188 | CA | VAL | 1083 | 22.058 | 24.288 | 6.443 | 1.00 | 17.39 | | C |
| ANISOU | 188 | CA | VAL | 1083 | 3290 | 1673 | 1645 | 306 | 262 | -167 | C |
| ATOM | 189 | CB | VAL | 1083 | 21.922 | 22.854 | 5.916 | 1.00 | 24.18 | | C |
| ANISOU | 189 | CB | VAL | 1083 | 4950 | 1903 | 2336 | -349 | 296 | -475 | C |
| ATOM | 190 | CG1 | VAL | 1083 | 20.887 | 22.035 | 6.690 | 1.00 | 27.10 | | C |
| ANISOU | 190 | CG1 | VAL | 1083 | 5156 | 2112 | 3029 | -643 | 692 | -795 | C |
| ATOM | 191 | CG2 | VAL | 1083 | 21.556 | 22.897 | 4.434 | 1.00 | 26.37 | | C |
| ANISOU | 191 | CG2 | VAL | 1083 | 3906 | 3775 | 2338 | -430 | 344 | -1262 | C |
| ATOM | 192 | C | VAL | 1083 | 22.435 | 24.282 | 7.905 | 1.00 | 17.65 | | C |
| ANISOU | 192 | C | VAL | 1083 | 2873 | 2047 | 1787 | 522 | 63 | -188 | C |
| ATOM | 193 | O | VAL | 1083 | 23.521 | 23.861 | 8.266 | 1.00 | 21.70 | | O |
| ANISOU | 193 | O | VAL | 1083 | 2950 | 2986 | 2310 | 770 | 292 | 705 | 0 |
| ATOM | 194 | N | ILE | 1084 | 21.516 | 24.737 | 8.741 | 1.00 | 16.13 | | N |
| ANISOU | 194 | N | ILE | 1084 | 2806 | 1743 | 1579 | 73 | 216 | -40 | N |
| ATOM | 195 | CA | ILE | 1084 | 21.807 | 24.825 | 10.170 | 1.00 | 14.78 | | C |
| ANISOU | 195 | CA | ILE | 1084 | 2425 | 1560 | 1630 | 63 | 1 | 47 | C |
| ATOM | 196 | CB | ILE | 1084 | 21.807 | 26.300 | 10.623 | 1.00 | 15.08 | | C |
| ANISOU | 196 | CB | ILE | 1084 | 2611 | 1517 | 1603 | -103 | -60 | 134 | C |
| ATOM | 197 | CG2 | ILE | 1084 | 23.007 | 26.993 | 10.035 | 1.00 | 20.31 | | C |
| ANISOU | 197 | CG2 | ILE | 1084 | 3149 | 2256 | 2312 | -725 | 617 | -578 | C |
| ATOM | 198 | CG1 | ILE | 1084 | 20.506 | 27.059 | 10.305 | 1.00 | 15.80 | | C |
| ANISOU | 198 | CG1 | ILE | 1084 | 3022 | 1380 | 1601 | 163 | -227 | 50 | C |
| ATOM | 199 | CD1 | ILE | 1084 | 20.476 | 28.498 | 10.796 | 1.00 | 15.53 | | C |
| ANISOU | 199 | CD1 | ILE | 1084 | 3136 | 1428 | 1337 | -19 | -262 | 14 | C |
| ATOM | 200 | C | ILE | 1084 | 20.802 | 24.010 | 10.972 | 1.00 | 14.27 | | C |
| ATOM | 201 | O | ILE | 1084 | 20.741 | 24.062 | 12.181 | 1.00 | 17.54 | | O |
| ANISOU | 201 | O | ILE | 1084 | 3270 | 1960 | 1433 | 92 | -110 | 220 | O |
| ATOM | 202 | N | GLY | 1085 | 19.960 | 23.213 | 10.295 | 1.00 | 16.41 | | N |
| ANISOU | 202 | N | GLY | 1085 | 3083 | 1207 | 1944 | -198 | -352 | 274 | N |
| ATOM | 203 | CA | GLY | 1085 | 19.044 | 22.286 | 11.019 | 1.00 | 15.32 | | C |
| ANISOU | 203 | CA | GLY | 1085 | 2520 | 1655 | 1646 | -123 | -130 | -17 | C |
| ATOM | 204 | C | GLY | 1085 | 18.370 | 21.449 | 9.935 | 1.00 | 14.73 | | C |
| ANISOU | 204 | C | GLY | 1085 | 2698 | 1443 | 1455 | -126 | 12 | 22 | C |
| ATOM | 205 | O | GLY | 1085 | 18.127 | 21.918 | 8.815 | 1.00 | 16.10 | | O |
| ANISOU | 205 | O | GLY | 1085 | 2935 | 1677 | 1505 | 205 | -126 | 7 | O |
| ATOM | 206 | N | ARG | 1086 | 18.060 | 20.206 | 10.247 | 1.00 | 17.64 | | N |
| ANISOU | 206 | N | ARG | 1086 | 3312 | 1330 | 2059 | -90 | -38 | -65 | N |
| ATOM | 207 | CA | ARG | 1086 | 17.406 | 19.344 | 9.242 | 1.00 | 17.42 | | C |
| ANISOU | 207 | CA | ARG | 1086 | 3021 | 1287 | 2313 | 172 | -113 | -293 | C |
| ATOM | 208 | CB | ARG | 1086 | 18.364 | 18.297 | 8.682 | 1.00 | 22.28 | | C |
| ANISOU | 208 | CB | ARG | 1086 | 3468 | 1802 | 3196 | 540 | 31 | -684 | C |
| ATOM | 209 | CG | ARG | 1086 | 19.674 | 18.872 | 8.120 | 1.00 | 29.91 | | C |
| ANISOU | 209 | CG | ARG | 1086 | 3963 | 2412 | 4991 | 833 | 1200 | -221 | C |
| ATOM | 210 | CD | ARG | 1086 | 20.619 | 17.705 | 7.779 | 1.00 | 40.67 | | C |
| ANISOU | 210 | CD | ARG | 1086 | 4824 | 4062 | 6569 | 1782 | 1725 | -1122 | C |
| ATOM | 211 | NE | ARG | 1086 | 21.926 | 18.207 | 7.327 | 1.00 | 43.96 | | N |
| ANISOU | 211 | NE | ARG | 1086 | 4898 | 5021 | 6784 | 1621 | 1993 | -1342 | N |
| ATOM | 212 | CZ | ARG | 1086 | 22.036 | 18.516 | 6.023 | 1.00 | 47.16 | | C |
| ANISOU | 212 | CZ | ARG | 1086 | 5928 | 4990 | 7000 | 731 | 1928 | -878 | C |
| ATOM | 213 | NH1 | ARG | 1086 | 20.931 | 18.330 | 5.293 | 1.00 | 44.75 | | N1+ |
| ANISOU | 213 | NH1 | ARG | 1086 | 6568 | 3804 | 6630 | -216 | 1733 | -274 | N1+ |
| ATOM | 214 | NH2 | ARG | 1086 | 23.150 | 18.978 | 5.488 | 1.00 | 44.27 | | N |
| ANISOU | 214 | NH2 | ARG | 1086 | 6041 | 3106 | 7674 | 752 | 1478 | 103 | N |
| ATOM | 215 | C | ARG | 1086 | 16.177 | 18.761 | 9.933 | 1.00 | 17.19 | | C |
| ANISOU | 215 | C | ARG | 1086 | 3285 | 1237 | 2009 | 16 | -169 | -58 | C |
| ATOM | 216 | O | ARG | 1086 | 16.315 | 18.095 | 10.977 | 1.00 | 22.86 | | O |
| ANISOU | 216 | O | ARG | 1086 | 3969 | 1937 | 2780 | 38 | -359 | 713 | O |
| ATOM | 217 | N | GLY | 1087 | 15.007 | 18.996 | 9.400 | 1.00 | 18.31 | | N |
| ANISOU | 217 | N | GLY | 1087 | 3157 | 1959 | 1842 | -293 | -68 | 66 | N |
| ATOM | 218 | CA | GLY | 1087 | 13.741 | 18.509 | 9.873 | 1.00 | 20.31 | | C |
| ANISOU | 218 | CA | GLY | 1087 | 3473 | 2621 | 1622 | -927 | -181 | 19 | C |
| ATOM | 219 | C | GLY | 1087 | 13.052 | 17.627 | 8.859 | 1.00 | 21.07 | | C |
| ANISOU | 219 | C | GLY | 1087 | 3779 | 2560 | 1666 | -818 | -73 | -218 | C |
| ATOM | 220 | O | GLY | 1087 | 13.523 | 17.538 | 7.734 | 1.00 | 26.79 | | O |
| ANISOU | 220 | O | GLY | 1087 | 4866 | 3372 | 1940 | -1021 | 403 | -608 | O |
| ATOM | 221 | N | HIS | 1088 | 11.974 | 16.990 | 9.231 | 1.00 | 24.91 | | N |
| ANISOU | 221 | N | HIS | 1088 | 4177 | 3355 | 1930 | -1526 | -177 | -459 | N |
| ATOM | 222 | CA | HIS | 1088 | 11.174 | 16.158 | 8.326 | 1.00 | 27.00 | | C |
| ANISOU | 222 | CA | HIS | 1088 | 4895 | 2928 | 2436 | -1464 | -413 | -654 | C |
| ATOM | 223 | CB | HIS | 1088 | 10.283 | 15.232 | 9.183 | 1.00 | 32.58 | | C |
| ANISOU | 223 | CB | HIS | 1088 | 5242 | 2763 | 4373 | -1747 | -240 | -82 | C |
| ATOM | 224 | CG | HIS | 1088 | 9.353 | 14.421 | 8.338 | 1.00 | 38.61 | | C |
| ANISOU | 224 | CG | HIS | 1088 | 5086 | 4382 | 5204 | -2254 | 433 | -1268 | C |
| ATOM | 225 | CD2 | HIS | 1088 | 9.506 | 13.244 | 7.716 | 1.00 | 42.66 | | C |
| ANISOU | 225 | CD2 | HIS | 1088 | 5506 | 4958 | 5745 | -2299 | 282 | -2026 | C |
| ATOM | 226 | ND1 | HIS | 1088 | 8.066 | 14.836 | 8.050 | 1.00 | 36.87 | | N |
| ANISOU | 226 | ND1 | HIS | 1088 | 4674 | 4633 | 4703 | -2762 | 693 | -1056 | N |
| ATOM | 227 | CE1 | HIS | 1088 | 7.453 | 13.943 | 7.282 | 1.00 | 41.93 | | C |
| ANISOU | 227 | CE1 | HIS | 1088 | 5515 | 5480 | 4938 | -2234 | 305 | -2015 | C |
| ATOM | 228 | NE2 | HIS | 1088 | 8.321 | 12.972 | 7.074 | 1.00 | 45.01 | | N |
| ANISOU | 228 | NE2 | HIS | 1088 | 6037 | 6002 | 5064 | -1683 | -231 | -2354 | N |
| ATOM | 229 | C | HIS | 1088 | 10.388 | 17.034 | 7.376 | 1.00 | 26.78 | | C |
| ANISOU | 229 | C | HIS | 1088 | 4785 | 3207 | 2183 | -1394 | -586 | -907 | C |
| ATOM | 230 | O | HIS | 1088 | 10.230 | 16.763 | 6.187 | 1.00 | 31.07 | | O |
| ANISOU | 230 | O | HIS | 1088 | 5705 | 3986 | 2115 | -1251 | -463 | -961 | O |
| ATOM | 231 | N | PHE | 1089 | 9.865 | 18.160 | 7.886 | 1.00 | 26.90 | | N |
| ANISOU | 231 | N | PHE | 1089 | 4805 | 3450 | 1966 | -1068 | -112 | -545 | N |
| ATOM | 232 | CA | PHE | 1089 | 8.942 | 18.987 | 7.088 | 1.00 | 29.25 | | C |
| ANISOU | 232 | CA | PHE | 1089 | 3618 | 4246 | 3252 | -1186 | 5 | -52 | C |
| ATOM | 233 | CB | PHE | 1089 | 7.917 | 19.633 | 8.033 | 1.00 | 36.34 | | C |
| ANISOU | 233 | CB | PHE | 1089 | 4195 | 6183 | 3428 | -427 | 358 | 74 | C |
| ATOM | 234 | CG | PHE | 1089 | 7.082 | 18.497 | 8.654 | 1.00 | 43.24 | | C |
| ANISOU | 234 | CG | PHE | 1089 | 4275 | 7650 | 4504 | -619 | 935 | 1061 | C |
| ATOM | 235 | CD1 | PHE | 1089 | 7.541 | 17.686 | 9.681 | 1.00 | 43.64 | | C |
| ANISOU | 235 | CD1 | PHE | 1089 | 4510 | 7517 | 4553 | -662 | 1001 | 1135 | C |
| ATOM | 236 | CD2 | PHE | 1089 | 5.809 | 18.256 | 8.175 | 1.00 | 47.16 | | C |
| ANISOU | 236 | CD2 | PHE | 1089 | 4148 | 7847 | 5925 | -764 | 766 | 1789 | C |
| ATOM | 237 | CE1 | PHE | 1089 | 6.817 | 16.657 | 10.241 | 1.00 | 41.41 | | C |
| ANISOU | 237 | CE1 | PHE | 1089 | 4062 | 7287 | 4386 | -378 | 1386 | 755 | C |
| ATOM | 238 | CE2 | PHE | 1089 | 5.074 | 17.235 | 8.723 | 1.00 | 47.41 | | C |
| ANISOU | 238 | CE2 | PHE | 1089 | 4067 | 8514 | 5433 | -633 | 1203 | 2104 | C |
| ATOM | 239 | CZ | PHE | 1089 | 5.553 | 16.435 | 9.736 | 1.00 | 44.74 | | C |
| ANISOU | 239 | CZ | PHE | 1089 | 3958 | 8168 | 4873 | -400 | 1249 | 1517 | C |
| ATOM | 240 | C | PHE | 1089 | 9.731 | 19.982 | 6.241 | 1.00 | 25.55 | | C |
| ANISOU | 240 | C | PHE | 1089 | 3424 | 2951 | 3334 | -675 | -157 | -233 | C |
| ATOM | 241 | O | PHE | 1089 | 9.219 | 20.500 | 5.250 | 1.00 | 27.22 | | O |
| ANISOU | 241 | O | PHE | 1089 | 3389 | 3627 | 3324 | -282 | -25 | -117 | O |
| ATOM | 242 | N | GLY | 1090 | 10.983 | 20.211 | 6.663 | 1.00 | 24.18 | | N |
| ANISOU | 242 | N | GLY | 1090 | 3300 | 2739 | 3149 | -537 | 39 | -268 | N |
| ATOM | 243 | CA | GLY | 1090 | 11.847 | 21.203 | 6.055 | 1.00 | 23.73 | | C |
| ANISOU | 243 | CA | GLY | 1090 | 2849 | 2964 | 3203 | -134 | -306 | 549 | C |
| ATOM | 244 | C | GLY | 1090 | 13.161 | 21.340 | 6.786 | 1.00 | 19.57 | | C |
| ANISOU | 244 | C | GLY | 1090 | 3470 | 1938 | 2028 | -485 | -407 | -24 | C |
| ATOM | 245 | O | GLY | 1090 | 13.348 | 20.827 | 7.875 | 1.00 | 25.58 | | O |
| ANISOU | 245 | O | GLY | 1090 | 3696 | 3588 | 2436 | -426 | -167 | 944 | O |
| ATOM | 246 | N | CYS | 1091 | 14.109 | 22.041 | 6.175 | 1.00 | 19.42 | | N |
| ANISOU | 246 | N | CYS | 1091 | 3428 | 1834 | 2116 | -572 | -653 | 258 | N |
| ATOM | 247 | CA | CYS | 1091 | 15.373 | 22.343 | 6.817 | 1.00 | 17.64 | | C |
| ANISOU | 247 | CA | CYS | 1091 | 3299 | 1566 | 1837 | -219 | -515 | 4 | C |
| ATOM | 248 | CB | CYS | 1091 | 16.567 | 22.051 | 5.895 | 1.00 | 20.05 | | C |
| ANISOU | 248 | CB | CYS | 1091 | 3493 | 2093 | 2031 | -44 | -430 | -411 | C |
| ATOM | 249 | SG | CYS | 1091 | 16.694 | 20.266 | 5.604 | 1.00 | 30.65 | | S |
| ANISOU | 249 | SG | CYS | 1091 | 6394 | 2454 | 2796 | 968 | -663 | -835 | S |
| ATOM | 250 | C | CYS | 1091 | 15.368 | 23.804 | 7.291 | 1.00 | 17.15 | | C |
| ANISOU | 250 | C | CYS | 1091 | 3197 | 1589 | 1730 | -74 | -582 | 34 | C |
| ATOM | 251 | O | CYS | 1091 | 14.496 | 24.577 | 6.875 | 1.00 | 16.97 | | O |
| ANISOU | 251 | O | CYS | 1091 | 2763 | 1788 | 1898 | 32 | -305 | 43 | O |
| ATOM | 252 | N | VAL | 1092 | 16.345 | 24.075 | 8.153 | 1.00 | 16.59 | | N |
| ANISOU | 252 | N | VAL | 1092 | 3244 | 1473 | 1588 | -269 | -581 | 198 | N |
| ATOM | 253 | CA | VAL | 1092 | 16.601 | 25.407 | 8.647 | 1.00 | 15.83 | | C |
| ANISOU | 253 | CA | VAL | 1092 | 2994 | 1441 | 1580 | -199 | -431 | 168 | C |
| ATOM | 254 | CB | VAL | 1092 | 16.792 | 25.461 | 10.164 | 1.00 | 15.56 | | C |
| ANISOU | 254 | CB | VAL | 1092 | 2766 | 1575 | 1572 | -125 | -170 | -29 | C |
| ATOM | 255 | CG1 | VAL | 1092 | 16.789 | 26.919 | 10.637 | 1.00 | 16.56 | | C |
| ANISOU | 255 | CG1 | VAL | 1092 | 3357 | 1410 | 1525 | 248 | -162 | 182 | C |
| ATOM | 256 | CG2 | VAL | 1092 | 15.719 | 24.682 | 10.891 | 1.00 | 16.19 | | C |
| ANISOU | 256 | CG2 | VAL | 1092 | 2466 | 1933 | 1751 | -58 | -368 | 326 | C |
| ATOM | 257 | C | VAL | 1092 | 17.864 | 25.906 | 7.945 | 1.00 | 14.33 | | C |
| ANISOU | 257 | C | VAL | 1092 | 2817 | 1305 | 1321 | -11 | -508 | 48 | C |
| ATOM | 258 | O | VAL | 1092 | 18.877 | 25.175 | 7.918 | 1.00 | 16.95 | | O |
| ANISOU | 258 | O | VAL | 1092 | 3252 | 1416 | 1772 | 306 | -217 | 41 | O |
| ATOM | 259 | N | TYR | 1093 | 17.816 | 27.112 | 7.390 | 1.00 | 15.58 | | N |
| ANISOU | 259 | N | TYR | 1093 | 3167 | 1295 | 1456 | 122 | -89 | 136 | N |
| ATOM | 260 | CA | TYR | 1093 | 18.930 | 27.678 | 6.650 | 1.00 | 16.16 | | C |
| ANISOU | 260 | CA | TYR | 1093 | 3015 | 1522 | 1602 | 101 | -136 | 183 | C |
| ATOM | 261 | CB | TYR | 1093 | 18.536 | 27.951 | 5.187 | 1.00 | 15.50 | | C |
| ANISOU | 261 | CB | TYR | 1093 | 2732 | 1745 | 1412 | 179 | 36 | -14 | C |
| ATOM | 262 | CG | TYR | 1093 | 18.038 | 26.704 | 4.475 | 1.00 | 16.97 | | C |
| ANISOU | 262 | CG | TYR | 1093 | 2938 | 1839 | 1670 | 9 | 64 | -75 | C |
| ATOM | 263 | CD1 | TYR | 1093 | 18.944 | 25.830 | 3.878 | 1.00 | 15.90 | | C |
| ANISOU | 263 | CD1 | TYR | 1093 | 3007 | 1572 | 1463 | 175 | -174 | 74 | C |
| ATOM | 264 | CE1 | TYR | 1093 | 18.471 | 24.699 | 3.232 | 1.00 | 18.28 | | C |
| ANISOU | 264 | CE1 | TYR | 1093 | 3439 | 1850 | 1658 | -266 | 289 | -113 | C |
| ATOM | 265 | CD2 | TYR | 1093 | 16.672 | 26.404 | 4.405 | 1.00 | 17.46 | | C |
| ANISOU | 265 | CD2 | TYR | 1093 | 2990 | 1793 | 1852 | -185 | 252 | -45 | C |
| ATOM | 266 | CE2 | TYR | 1093 | 16.224 | 25.271 | 3.758 | 1.00 | 19.35 | | C |
| ANISOU | 266 | CE2 | TYR | 1093 | 3238 | 2177 | 1937 | -47 | -322 | -340 | C |
| ATOM | 267 | CZ | TYR | 1093 | 17.123 | 24.411 | 3.168 | 1.00 | 18.75 | | C |
| ANISOU | 267 | CZ | TYR | 1093 | 3484 | 1933 | 1705 | -308 | 23 | -237 | C |
| ATOM | 268 | OH | TYR | 1093 | 16.694 | 23.277 | 2.521 | 1.00 | 23.14 | | O |
| ANISOU | 268 | OH | TYR | 1093 | 4049 | 2391 | 2351 | -564 | 57 | -798 | O |
| ATOM | 269 | C | TYR | 1093 | 19.379 | 29.003 | 7.220 | 1.00 | 14.74 | | C |
| ANISOU | 269 | C | TYR | 1093 | 2748 | 1556 | 1298 | 153 | 38 | 157 | C |
| ATOM | 270 | O | TYR | 1093 | 18.558 | 29.723 | 7.797 | 1.00 | 15.45 | | O |
| ANISOU | 270 | O | TYR | 1093 | 2673 | 1544 | 1652 | 161 | 95 | 200 | O |
| ATOM | 271 | N | HIS | 1094 | 20.658 | 29.333 | 7.063 | 1.00 | 15.95 | | N |
| ANISOU | 271 | N | HIS | 1094 | 2687 | 1595 | 1779 | 232 | -53 | -4 | N |
| ATOM | 272 | CA | HIS | 1094 | 21.106 | 30.675 | 7.400 | 1.00 | 14.41 | | C |
| ANISOU | 272 | CA | HIS | 1094 | 2397 | 1538 | 1540 | 360 | 137 | -113 | C |
| ATOM | 273 | CB | HIS | 1094 | 22.615 | 30.781 | 7.241 | 1.00 | 17.27 | | C |
| ANISOU | 273 | CB | HIS | 1094 | 2202 | 2042 | 2318 | 464 | -119 | -180 | C |
| ATOM | 274 | CG | HIS | 1094 | 23.180 | 31.996 | 7.879 | 1.00 | 23.52 | | C |
| ANISOU | 274 | CG | HIS | 1094 | 3006 | 2472 | 3458 | -266 | -129 | -343 | C |
| ATOM | 275 | CD2 | HIS | 1094 | 22.660 | 32.992 | 8.647 | 1.00 | 28.73 | | C |
| ANISOU | 275 | CD2 | HIS | 1094 | 3606 | 2741 | 4568 | -125 | -1100 | -1414 | C |
| ATOM | 276 | ND1 | HIS | 1094 | 24.497 | 32.314 | 7.755 | 1.00 | 36.95 | | N |
| ANISOU | 276 | ND1 | HIS | 1094 | 3184 | 4394 | 6461 | -831 | -465 | -1739 | N |
| ATOM | 277 | CE1 | HIS | 1094 | 24.777 | 33.440 | 8.406 | 1.00 | 41.24 | | C |
| ANISOU | 277 | CE1 | HIS | 1094 | 4025 | 5022 | 6623 | -1290 | -724 | -2210 | C |
| ATOM | 278 | NE2 | HIS | 1094 | 23.657 | 33.874 | 8.963 | 1.00 | 36.80 | | N |
| ANISOU | 278 | NE2 | HIS | 1094 | 4282 | 3613 | 6088 | -1106 | -647 | -1597 | N |
| ATOM | 279 | C | HIS | 1094 | 20.434 | 31.716 | 6.513 | 1.00 | 13.89 | | C |
| ANISOU | 279 | C | HIS | 1094 | 2051 | 1579 | 1647 | 141 | -41 | -81 | C |
| ATOM | 280 | O | HIS | 1094 | 20.329 | 31.492 | 5.314 | 1.00 | 16.32 | | O |
| ANISOU | 280 | O | HIS | 1094 | 2862 | 1648 | 1691 | -1 | -332 | -66 | O |
| ATOM | 281 | N | GLY | 1095 | 19.997 | 32.825 | 7.101 | 1.00 | 14.85 | | N |
| ANISOU | 281 | N | GLY | 1095 | 2291 | 1495 | 1858 | 244 | 157 | 99 | N |
| ATOM | 282 | CA | GLY | 1095 | 19.413 | 33.873 | 6.281 | 1.00 | 14.77 | | C |
| ANISOU | 282 | CA | GLY | 1095 | 2245 | 1364 | 2004 | 15 | -181 | 3 | C |
| ATOM | 283 | C | GLY | 1095 | 19.863 | 35.240 | 6.763 | 1.00 | 14.17 | | C |
| ANISOU | 283 | C | GLY | 1095 | 2094 | 1503 | 1785 | -10 | -477 | 35 | C |
| ATOM | 284 | O | GLY | 1095 | 20.500 | 35.396 | 7.795 | 1.00 | 13.82 | | O |
| ANISOU | 284 | O | GLY | 1095 | 1817 | 1780 | 1656 | 136 | -282 | 63 | O |
| ATOM | 285 | N | THR | 1096 | 19.482 | 36.228 | 5.960 | 1.00 | 14.93 | | N |
| ANISOU | 285 | N | THR | 1096 | 2591 | 1370 | 1710 | -68 | -380 | 138 | N |
| ATOM | 286 | CA | THR | 1096 | 19.766 | 37.624 | 6.240 | 1.00 | 15.41 | | C |
| ANISOU | 286 | CA | THR | 1096 | 2350 | 1508 | 1999 | -159 | -225 | -80 | C |
| ATOM | 287 | CB | THR | 1096 | 20.907 | 38.156 | 5.328 | 1.00 | 18.97 | | C |
| ANISOU | 287 | CB | THR | 1096 | 2355 | 2191 | 2661 | -535 | -170 | 193 | C |
| ATOM | 288 | OG1 | THR | 1096 | 22.104 | 37.394 | 5.564 | 1.00 | 20.86 | | O |
| ANISOU | 288 | OG1 | THR | 1096 | 2422 | 2917 | 2587 | -173 | 70 | 200 | O |
| ATOM | 289 | CG2 | THR | 1096 | 21.261 | 39.576 | 5.649 | 1.00 | 23.05 | | C |
| ANISOU | 289 | CG2 | THR | 1096 | 3735 | 2186 | 2837 | -866 | 239 | 222 | C |
| ATOM | 290 | C | THR | 1096 | 18.504 | 38.443 | 6.026 | 1.00 | 14.55 | | C |
| ANISOU | 290 | C | THR | 1096 | 2581 | 1420 | 1528 | -27 | -196 | 113 | C |
| ATOM | 291 | O | THR | 1096 | 17.902 | 38.352 | 4.954 | 1.00 | 16.83 | | O |
| ANISOU | 291 | O | THR | 1096 | 2925 | 1870 | 1601 | 17 | -370 | 9 | O |
| ATOM | 292 | N | LEU | 1097 | 18.065 | 39.238 | 7.001 | 1.00 | 15.49 | | N |
| ANISOU | 292 | N | LEU | 1097 | 2796 | 1239 | 1848 | -158 | -14 | -42 | N |
| ATOM | 293 | CA | LEU | 1097 | 17.003 | 40.211 | 6.858 | 1.00 | 16.19 | | C |
| ANISOU | 293 | CA | LEU | 1097 | 2515 | 1496 | 2142 | -152 | 313 | 163 | C |
| ATOM | 294 | CB | LEU | 1097 | 16.170 | 40.409 | 8.135 | 1.00 | 17.06 | | C |
| ANISOU | 294 | CB | LEU | 1097 | 2356 | 2357 | 1768 | -397 | 26 | -99 | C |
| ATOM | 295 | CG | LEU | 1097 | 15.241 | 39.210 | 8.381 | 1.00 | 17.74 | | C |
| ANISOU | 295 | CG | LEU | 1097 | 2592 | 2281 | 1867 | -267 | -82 | 739 | C |
| ATOM | 296 | CD1 | LEU | 1097 | 14.818 | 39.199 | 9.834 | 1.00 | 24.35 | | C |
| ANISOU | 296 | CD1 | LEU | 1097 | 3560 | 3494 | 2198 | -69 | 591 | 801 | C |
| ATOM | 297 | CD2 | LEU | 1097 | 14.067 | 39.291 | 7.418 | 1.00 | 20.72 | | C |
| ANISOU | 297 | CD2 | LEU | 1097 | 2478 | 2467 | 2928 | -509 | -497 | 274 | C |
| ATOM | 298 | C | LEU | 1097 | 17.646 | 41.542 | 6.466 | 1.00 | 15.26 | | C |
| ANISOU | 298 | C | LEU | 1097 | 2304 | 1381 | 2112 | -122 | -179 | 174 | C |
| ATOM | 299 | O | LEU | 1097 | 18.637 | 41.938 | 7.063 | 1.00 | 17.75 | | O |
| ANISOU | 299 | O | LEU | 1097 | 2907 | 1532 | 2304 | -270 | -591 | 41 | O |
| ATOM | 300 | N | LEU | 1098 | 17.091 | 42.205 | 5.464 | 1.00 | 17.52 | | N |
| ANISOU | 300 | N | LEU | 1098 | 3184 | 1514 | 1959 | -383 | -511 | 201 | N |
| ATOM | 301 | CA | LEU | 1098 | 17.640 | 43.414 | 4.866 | 1.00 | 16.97 | | C |
| ANISOU | 301 | CA | LEU | 1098 | 2749 | 1566 | 2133 | -124 | -49 | 258 | C |
| ATOM | 302 | CB | LEU | 1098 | 18.114 | 43.120 | 3.436 | 1.00 | 19.15 | | C |
| ANISOU | 302 | CB | LEU | 1098 | 2742 | 2390 | 2146 | 64 | -77 | 150 | C |
| ATOM | 303 | CG | LEU | 1098 | 18.607 | 44.302 | 2.601 | 1.00 | 21.77 | | C |
| ANISOU | 303 | CG | LEU | 1098 | 4274 | 2339 | 1657 | 218 | 191 | 73 | C |
| ATOM | 304 | CD1 | LEU | 1098 | 19.880 | 44.867 | 3.248 | 1.00 | 26.19 | | C |
| ANISOU | 304 | CD1 | LEU | 1098 | 3755 | 2516 | 3678 | -437 | 514 | 515 | C |
| ATOM | 305 | CD2 | LEU | 1098 | 18.897 | 43.992 | 1.133 | 1.00 | 30.96 | | C |
| ANISOU | 305 | CD2 | LEU | 1098 | 6253 | 3706 | 1805 | 2293 | 790 | 351 | C |
| ATOM | 306 | C | LEU | 1098 | 16.592 | 44.501 | 4.845 | 1.00 | 17.65 | | C |
| ANISOU | 306 | C | LEU | 1098 | 2624 | 1546 | 2538 | -144 | -140 | 99 | C |
| ATOM | 307 | O | LEU | 1098 | 15.539 | 44.265 | 4.250 | 1.00 | 19.16 | | O |
| ANISOU | 307 | O | LEU | 1098 | 3027 | 1715 | 2539 | 90 | -429 | -145 | O |
| ATOM | 308 | N | ASP | 1099 | 16.823 | 45.667 | 5.449 | 1.00 | 16.43 | | N |
| ANISOU | 308 | N | ASP | 1099 | 2638 | 1647 | 1957 | -231 | 131 | 160 | N |
| ATOM | 309 | CA | ASP | 1099 | 15.824 | 46.729 | 5.343 | 1.00 | 16.01 | | C |
| ANISOU | 309 | CA | ASP | 1099 | 2561 | 1901 | 1620 | 11 | 267 | -167 | C |
| ATOM | 310 | CB | ASP | 1099 | 15.683 | 47.494 | 6.660 | 1.00 | 22.02 | | C |
| ANISOU | 310 | CB | ASP | 1099 | 4017 | 2355 | 1996 | -345 | 470 | -593 | C |
| ATOM | 311 | CG | ASP | 1099 | 16.716 | 48.503 | 7.068 | 1.00 | 23.16 | | C |
| ANISOU | 311 | CG | ASP | 1099 | 4684 | 1960 | 2156 | -577 | 670 | -659 | C |
| ATOM | 312 | OD1 | ASP | 1099 | 17.616 | 48.827 | 6.252 | 1.00 | 23.23 | | O |
| ANISOU | 312 | OD1 | ASP | 1099 | 4876 | 1831 | 2119 | -581 | 598 | -139 | O |
| ATOM | 313 | OD2 | ASP | 1099 | 16.616 | 48.978 | 8.241 | 1.00 | 26.73 | | O1- |
| ANISOU | 313 | OD2 | ASP | 1099 | 5007 | 3114 | 2036 | -744 | 428 | -880 | O1- |
| ATOM | 314 | C | ASP | 1099 | 16.152 | 47.661 | 4.167 | 1.00 | 17.27 | | C |
| ANISOU | 314 | C | ASP | 1099 | 2528 | 2086 | 1949 | 39 | -5 | 213 | C |
| ATOM | 315 | O | ASP | 1099 | 17.156 | 47.528 | 3.454 | 1.00 | 16.53 | | O |
| ANISOU | 315 | O | ASP | 1099 | 2985 | 1593 | 1704 | 43 | 220 | 187 | O |
| ATOM | 316 | N | ASN | 1100 | 15.288 | 48.643 | 3.906 | 1.00 | 20.88 | | N |
| ANISOU | 316 | N | ASN | 1100 | 3575 | 1876 | 2484 | 527 | 532 | 114 | N |
| ATOM | 317 | CA | ASN | 1100 | 15.424 | 49.422 | 2.678 | 1.00 | 19.64 | | C |
| ANISOU | 317 | CA | ASN | 1100 | 2972 | 2021 | 2468 | 30 | -299 | 250 | C |
| ATOM | 318 | CB | ASN | 1100 | 14.156 | 50.244 | 2.400 | 1.00 | 23.92 | | C |
| ANISOU | 318 | CB | ASN | 1100 | 3128 | 3246 | 2713 | 518 | -438 | 205 | C |
| ATOM | 319 | CG | ASN | 1100 | 14.055 | 50.811 | 1.000 | 1.00 | 24.41 | | C |
| ANISOU | 319 | CG | ASN | 1100 | 3968 | 2514 | 2792 | 1195 | -80 | 198 | C |
| ATOM | 320 | OD1 | ASN | 1100 | 14.243 | 50.122 | -0.011 | 1.00 | 22.03 | | O |
| ANISOU | 320 | OD1 | ASN | 1100 | 3360 | 2202 | 2807 | 37 | 109 | 85 | O |
| ATOM | 321 | ND2 | ASN | 1100 | 13.750 | 52.093 | 0.901 | 1.00 | 31.01 | | N |
| ANISOU | 321 | ND2 | ASN | 1100 | 5196 | 2646 | 3939 | 1704 | 1842 | 557 | N |
| ATOM | 322 | C | ASN | 1100 | 16.600 | 50.361 | 2.763 | 1.00 | 17.79 | | C |
| ANISOU | 322 | C | ASN | 1100 | 2954 | 1691 | 2113 | 260 | 184 | -398 | C |
| ATOM | 323 | O | ASN | 1100 | 17.017 | 50.903 | 1.742 | 1.00 | 20.12 | | O |
| ANISOU | 323 | O | ASN | 1100 | 3289 | 1945 | 2411 | -91 | -65 | 68 | O |
| ATOM | 324 | N | ASP | 1101 | 17.114 | 50.526 | 3.976 | 1.00 | 20.09 | | N |
| ANISOU | 324 | N | ASP | 1101 | 3400 | 1859 | 2374 | 80 | -288 | -246 | N |
| ATOM | 325 | CA | ASP | 1101 | 18.291 | 51.372 | 4.122 | 1.00 | 20.77 | | C |
| ANISOU | 325 | CA | ASP | 1101 | 3646 | 1932 | 2316 | -276 | 6 | -255 | C |
| ATOM | 326 | CB | ASP | 1101 | 18.233 | 52.124 | 5.453 | 1.00 | 26.26 | | C |
| ANISOU | 326 | CB | ASP | 1101 | 4661 | 2746 | 2572 | -823 | 90 | -695 | C |
| ATOM | 327 | CG | ASP | 1101 | 17.015 | 53.047 | 5.478 | 1.00 | 34.17 | | C |
| ANISOU | 327 | CG | ASP | 1101 | 7156 | 2780 | 3045 | 832 | 395 | -937 | C |
| ATOM | 328 | OD1 | ASP | 1101 | 16.760 | 53.624 | 4.402 | 1.00 | 32.73 | | O |
| ANISOU | 328 | OD1 | ASP | 1101 | 6770 | 2635 | 3031 | 700 | 599 | -824 | O |
| ATOM | 329 | OD2 | ASP | 1101 | 16.330 | 53.193 | 6.518 | 1.00 | 30.97 | | O1- |
| ANISOU | 329 | OD2 | ASP | 1101 | 6291 | 2452 | 3025 | 553 | 199 | -592 | O1- |
| ATOM | 330 | C | ASP | 1101 | 19.562 | 50.558 | 4.082 | 1.00 | 20.72 | | C |
| ANISOU | 330 | C | ASP | 1101 | 3420 | 2302 | 2149 | -357 | -317 | 465 | C |
| ATOM | 331 | O | ASP | 1101 | 20.679 | 51.045 | 4.185 | 1.00 | 25.71 | | O |
| ANISOU | 331 | O | ASP | 1101 | 3586 | 2977 | 3205 | -501 | -1050 | 658 | O |
| ATOM | 332 | N | GLY | 1102 | 19.460 | 49.231 | 3.932 | 1.00 | 21.33 | | N |
| ANISOU | 332 | N | GLY | 1102 | 3335 | 2231 | 2540 | 117 | -237 | 314 | N |
| ATOM | 333 | CA | GLY | 1102 | 20.598 | 48.352 | 3.817 | 1.00 | 22.38 | | C |
| ANISOU | 333 | CA | GLY | 1102 | 3131 | 2950 | 2421 | 284 | 384 | 1278 | C |
| ATOM | 334 | C | GLY | 1102 | 21.044 | 47.769 | 5.141 | 1.00 | 24.59 | | C |
| ANISOU | 334 | C | GLY | 1102 | 3891 | 2884 | 2567 | 197 | 22 | 1321 | C |
| ATOM | 335 | O | GLY | 1102 | 22.090 | 47.103 | 5.229 | 1.00 | 27.76 | | O |
| ANISOU | 335 | O | GLY | 1102 | 3674 | 3376 | 3497 | 183 | -565 | 1286 | O |
| ATOM | 336 | N | LYS | 1103 | 20.263 | 48.010 | 6.204 | 1.00 | 21.29 | | N |
| ANISOU | 336 | N | LYS | 1103 | 4055 | 1997 | 2037 | -852 | -314 | 562 | N |
| ATOM | 337 | CA | LYS | 1103 | 20.766 | 47.379 | 7.463 | 1.00 | 23.81 | | C |
| ANISOU | 337 | CA | LYS | 1103 | 5015 | 1938 | 2095 | -994 | -758 | 486 | C |
| ATOM | 338 | CB | LYS | 1103 | 20.174 | 48.115 | 8.638 | 1.00 | 29.64 | | C |
| ANISOU | 338 | CB | LYS | 1103 | 6249 | 2897 | 2115 | -1122 | -540 | 16 | C |
| ATOM | 343 | C | LYS | 1103 | 20.420 | 45.916 | 7.444 | 1.00 | 23.84 | | C |
| ANISOU | 343 | C | LYS | 1103 | 4449 | 1974 | 2636 | -889 | -1236 | 667 | C |
| ATOM | 344 | O | LYS | 1103 | 19.374 | 45.537 | 6.933 | 1.00 | 21.10 | | O |
| ANISOU | 344 | O | LYS | 1103 | 4038 | 2074 | 1906 | -692 | -681 | 390 | O |
| ATOM | 345 | N | LYS | 1104 | 21.280 | 45.078 | 7.998 | 1.00 | 24.29 | | N |
| ANISOU | 345 | N | LYS | 1104 | 4202 | 2066 | 2960 | -786 | -1089 | 626 | N |
| ATOM | 346 | CA | LYS | 1104 | 20.992 | 43.654 | 7.970 | 1.00 | 23.84 | | C |
| ANISOU | 346 | CA | LYS | 1104 | 4493 | 2000 | 2564 | -605 | -962 | 507 | C |
| ATOM | 347 | CB | LYS | 1104 | 21.975 | 42.987 | 7.003 | 1.00 | 28.84 | | C |
| ANISOU | 347 | CB | LYS | 1104 | 4843 | 2666 | 3450 | -371 | -432 | 347 | C |
| ATOM | 348 | CG | LYS | 1104 | 21.803 | 43.566 | 5.593 | 1.00 | 29.67 | | C |
| ANISOU | 348 | CG | LYS | 1104 | 4346 | 3490 | 3439 | -342 | 342 | 816 | C |
| ATOM | 349 | CD | LYS | 1104 | 22.788 | 42.910 | 4.622 | 1.00 | 33.82 | | C |
| ANISOU | 349 | CD | LYS | 1104 | 4578 | 4403 | 3867 | 398 | 108 | 405 | C |
| ATOM | 350 | CE | LYS | 1104 | 24.212 | 43.385 | 4.884 | 1.00 | 37.41 | | C |
| ANISOU | 350 | CE | LYS | 1104 | 4710 | 5159 | 4345 | -320 | 1026 | -293 | C |
| ATOM | 351 | NZ | LYS | 1104 | 25.065 | 43.442 | 3.676 | 1.00 | 40.31 | | N1+ |
| ANISOU | 351 | NZ | LYS | 1104 | 5578 | 5814 | 3924 | -95 | 922 | 1260 | N1+ |
| ATOM | 352 | C | LYS | 1104 | 21.111 | 42.977 | 9.317 | 1.00 | 24.33 | | C |
| ANISOU | 352 | C | LYS | 1104 | 4239 | 2235 | 2771 | -1171 | -1438 | 695 | C |
| ATOM | 353 | O | LYS | 1104 | 21.886 | 43.413 | 10.174 | 1.00 | 24.94 | | O |
| ANISOU | 353 | O | LYS | 1104 | 3928 | 2262 | 3287 | -982 | -1742 | 758 | O |
| ATOM | 354 | N | ILE | 1105 | 20.318 | 41.892 | 9.455 | 1.00 | 20.58 | | N |
| ANISOU | 354 | N | ILE | 1105 | 3608 | 1487 | 2725 | -505 | -917 | 204 | N |
| ATOM | 355 | CA | ILE | 1105 | 20.590 | 41.049 | 10.626 | 1.00 | 22.02 | | C |
| ANISOU | 355 | CA | ILE | 1105 | 4488 | 1711 | 2169 | -684 | -641 | 83 | C |
| ATOM | 356 | CB | ILE | 1105 | 19.620 | 41.231 | 11.780 | 1.00 | 25.56 | | C |
| ANISOU | 356 | CB | ILE | 1105 | 4854 | 2160 | 2696 | 2 | -339 | -206 | C |
| ATOM | 357 | CG2 | ILE | 1105 | 19.736 | 42.592 | 12.465 | 1.00 | 33.79 | | C |
| ANISOU | 357 | CG2 | ILE | 1105 | 6745 | 2782 | 3311 | -825 | 246 | -910 | C |
| ATOM | 358 | CG1 | ILE | 1105 | 18.185 | 40.968 | 11.324 | 1.00 | 24.34 | | C |
| ANISOU | 358 | CG1 | ILE | 1105 | 4595 | 2304 | 2350 | 412 | -239 | -29 | C |
| ATOM | 359 | CD1 | ILE | 1105 | 17.424 | 40.521 | 12.575 | 1.00 | 28.23 | | C |
| ANISOU | 359 | CD1 | ILE | 1105 | 5098 | 3402 | 2224 | -443 | -505 | 299 | C |
| ATOM | 360 | C | ILE | 1105 | 20.520 | 39.608 | 10.180 | 1.00 | 18.60 | | C |
| ANISOU | 360 | C | ILE | 1105 | 3389 | 1597 | 2080 | 444 | -677 | 206 | C |
| ATOM | 361 | O | ILE | 1105 | 19.781 | 39.242 | 9.248 | 1.00 | 19.32 | | O |
| ANISOU | 361 | O | ILE | 1105 | 3491 | 1563 | 2286 | -345 | -939 | 249 | O |
| ATOM | 362 | N | HIS | 1106 | 21.319 | 38.779 | 10.867 | 1.00 | 18.20 | | N |
| ANISOU | 362 | N | HIS | 1106 | 3056 | 2013 | 1846 | -526 | -671 | 189 | N |
| ATOM | 363 | CA | HIS | 1106 | 21.224 | 37.357 | 10.624 | 1.00 | 16.97 | | C |
| ANISOU | 363 | CA | HIS | 1106 | 2576 | 1862 | 2010 | -61 | -336 | 229 | C |
| ATOM | 364 | CB | HIS | 1106 | 22.248 | 36.488 | 11.375 | 1.00 | 22.26 | | C |
| ANISOU | 364 | CB | HIS | 1106 | 3188 | 2598 | 2671 | 43 | -1259 | 258 | C |
| ATOM | 365 | CG | HIS | 1106 | 23.632 | 36.932 | 11.124 | 1.00 | 29.93 | | C |
| ANISOU | 365 | CG | HIS | 1106 | 2773 | 5051 | 3547 | 248 | -1011 | -798 | C |
| ATOM | 366 | CD2 | HIS | 1106 | 24.422 | 37.798 | 11.793 | 1.00 | 37.58 | | C |
| ANISOU | 366 | CD2 | HIS | 1106 | 3127 | 6178 | 4972 | -1142 | 235 | -1730 | C |
| ATOM | 367 | ND1 | HIS | 1106 | 24.373 | 36.470 | 10.065 | 1.00 | 35.04 | | N |
| ANISOU | 367 | ND1 | HIS | 1106 | 3693 | 5182 | 4437 | 590 | -337 | -1019 | N |
| ATOM | 368 | CE1 | HIS | 1106 | 25.575 | 37.032 | 10.081 | 1.00 | 36.29 | | C |
| ANISOU | 368 | CE1 | HIS | 1106 | 3932 | 5372 | 4486 | 220 | 251 | -453 | C |
| ATOM | 369 | NE2 | HIS | 1106 | 25.627 | 37.842 | 11.123 | 1.00 | 38.30 | | N |
| ANISOU | 369 | NE2 | HIS | 1106 | 3359 | 5806 | 5387 | -502 | 637 | -1207 | N |
| ATOM | 370 | C | HIS | 1106 | 19.901 | 36.797 | 11.107 | 1.00 | 15.47 | | C |
| ANISOU | 370 | C | HIS | 1106 | 2905 | 1407 | 1566 | 96 | 89 | 349 | C |
| ATOM | 371 | O | HIS | 1106 | 19.306 | 37.353 | 12.007 | 1.00 | 20.63 | | O |
| ANISOU | 371 | O | HIS | 1106 | 4193 | 1513 | 2134 | -41 | 852 | 130 | O |
| ATOM | 372 | N | CYS | 1107 | 19.486 | 35.692 | 10.504 | 1.00 | 15.17 | | N |
| ANISOU | 372 | N | CYS | 1107 | 2373 | 1579 | 1814 | 186 | -242 | 221 | N |
| ATOM | 373 | CA | CYS | 1107 | 18.299 | 35.001 | 10.980 | 1.00 | 15.24 | | C |
| ANISOU | 373 | CA | CYS | 1107 | 2596 | 1560 | 1633 | 157 | 55 | 315 | C |
| ATOM | 374 | CB | CYS | 1107 | 17.012 | 35.611 | 10.446 | 1.00 | 16.18 | | C |
| ANISOU | 374 | CB | CYS | 1107 | 2338 | 1974 | 1834 | 255 | 418 | 504 | C |
| ATOM | 375 | SG | CYS | 1107 | 16.865 | 35.601 | 8.615 | 1.00 | 16.14 | | S |
| ANISOU | 375 | SG | CYS | 1107 | 2271 | 1948 | 1913 | 199 | -45 | 516 | S |
| ATOM | 376 | C | CYS | 1107 | 18.449 | 33.532 | 10.562 | 1.00 | 14.82 | | C |
| ANISOU | 376 | C | CYS | 1107 | 2259 | 1745 | 1627 | 105 | -99 | 24 | C |
| ATOM | 377 | O | CYS | 1107 | 19.413 | 33.158 | 9.894 | 1.00 | 14.60 | | O |
| ANISOU | 377 | O | CYS | 1107 | 2318 | 1851 | 1380 | 253 | -148 | 257 | O |
| ATOM | 378 | N | ALA | 1108 | 17.464 | 32.760 | 11.000 | 1.00 | 15.07 | | N |
| ANISOU | 378 | N | ALA | 1108 | 2576 | 1492 | 1658 | 171 | 180 | 117 | N |
| ATOM | 379 | CA | ALA | 1108 | 17.348 | 31.367 | 10.614 | 1.00 | 13.52 | | C |
| ANISOU | 379 | CA | ALA | 1108 | 2375 | 1529 | 1232 | 345 | -16 | -8 | C |
| ATOM | 380 | CB | ALA | 1108 | 17.367 | 30.505 | 11.862 | 1.00 | 15.06 | | C |
| ANISOU | 380 | CB | ALA | 1108 | 2968 | 1369 | 1387 | 203 | -304 | 15 | C |
| ATOM | 381 | C | ALA | 1108 | 16.080 | 31.163 | 9.833 | 1.00 | 14.94 | | C |
| ANISOU | 381 | C | ALA | 1108 | 2349 | 1854 | 1474 | 399 | -87 | 48 | C |
| ATOM | 382 | O | ALA | 1108 | 15.035 | 31.700 | 10.251 | 1.00 | 19.41 | | O |
| ANISOU | 382 | O | ALA | 1108 | 2542 | 2949 | 1884 | 784 | -238 | -499 | O |
| ATOM | 383 | N | VAL | 1109 | 16.079 | 30.444 | 8.733 | 1.00 | 13.81 | | N |
| ANISOU | 383 | N | VAL | 1109 | 2237 | 1499 | 1512 | 137 | -187 | 54 | N |
| ATOM | 384 | CA | VAL | 1109 | 14.890 | 30.344 | 7.898 | 1.00 | 15.47 | | C |
| ANISOU | 384 | CA | VAL | 1109 | 2539 | 1515 | 1824 | 26 | -362 | 289 | C |
| ATOM | 385 | CB | VAL | 1109 | 15.149 | 30.928 | 6.496 | 1.00 | 16.40 | | C |
| ANISOU | 385 | CB | VAL | 1109 | 2607 | 1990 | 1633 | -407 | -429 | 275 | C |
| ATOM | 386 | CG1 | VAL | 1109 | 13.897 | 30.883 | 5.665 | 1.00 | 17.25 | | C |
| ANISOU | 386 | CG1 | VAL | 1109 | 2613 | 2194 | 1747 | -300 | -469 | 90 | C |
| ATOM | 387 | CG2 | VAL | 1109 | 15.659 | 32.365 | 6.632 | 1.00 | 16.33 | | C |
| ANISOU | 387 | CG2 | VAL | 1109 | 2060 | 1845 | 2298 | -188 | -308 | 437 | C |
| ATOM | 388 | C | VAL | 1109 | 14.466 | 28.882 | 7.821 | 1.00 | 14.66 | | C |
| ANISOU | 388 | C | VAL | 1109 | 2433 | 1474 | 1664 | 110 | -324 | 105 | C |
| ATOM | 389 | O | VAL | 1109 | 15.240 | 28.088 | 7.286 | 1.00 | 16.65 | | O |
| ANISOU | 389 | O | VAL | 1109 | 2852 | 1651 | 1823 | 131 | 23 | -27 | O |
| ATOM | 390 | N | LYS | 1110 | 13.288 | 28.599 | 8.364 | 1.00 | 15.46 | | N |
| ANISOU | 390 | N | LYS | 1110 | 2347 | 1547 | 1979 | -13 | -340 | 85 | N |
| ATOM | 391 | CA | LYS | 1110 | 12.808 | 27.208 | 8.327 | 1.00 | 17.72 | | C |
| ANISOU | 391 | CA | LYS | 1110 | 2658 | 1549 | 2528 | -150 | -514 | -123 | C |
| ATOM | 392 | CB | LYS | 1110 | 12.111 | 26.845 | 9.620 | 1.00 | 21.03 | | C |
| ANISOU | 392 | CB | LYS | 1110 | 3139 | 1834 | 3018 | -291 | -312 | 612 | C |
| ATOM | 393 | CG | LYS | 1110 | 11.338 | 25.507 | 9.662 | 1.00 | 20.82 | | C |
| ANISOU | 393 | CG | LYS | 1110 | 2720 | 1928 | 3261 | -327 | -778 | 339 | C |
| ATOM | 394 | CD | LYS | 1110 | 12.291 | 24.345 | 9.457 | 1.00 | 23.85 | | C |
| ANISOU | 394 | CD | LYS | 1110 | 3221 | 1921 | 3921 | -132 | -876 | 133 | C |
| ATOM | 395 | CE | LYS | 1110 | 11.673 | 22.978 | 9.329 | 1.00 | 29.36 | | C |
| ANISOU | 395 | CE | LYS | 1110 | 3758 | 2067 | 5329 | -365 | 226 | -274 | C |
| ATOM | 396 | NZ | LYS | 1110 | 10.456 | 22.736 | 10.084 | 1.00 | 29.17 | | N1+ |
| ANISOU | 396 | NZ | LYS | 1110 | 4720 | 1822 | 4541 | -444 | 694 | -151 | N1+ |
| ATOM | 397 | C | LYS | 1110 | 11.879 | 27.063 | 7.134 | 1.00 | 20.76 | | C |
| ANISOU | 397 | C | LYS | 1110 | 3013 | 1951 | 2922 | 221 | -879 | -555 | C |
| ATOM | 398 | O | LYS | 1110 | 10.856 | 27.780 | 7.110 | 1.00 | 20.82 | | O |
| ANISOU | 398 | O | LYS | 1110 | 2678 | 2302 | 2931 | 101 | -766 | -630 | O |
| ATOM | 399 | N | SER | 1111 | 12.221 | 26.187 | 6.195 | 1.00 | 20.32 | | N |
| ANISOU | 399 | N | SER | 1111 | 3454 | 1748 | 2518 | 338 | -765 | -228 | N |
| ATOM | 400 | CA | SER | 1111 | 11.364 | 25.888 | 5.045 | 1.00 | 23.57 | | C |
| ANISOU | 400 | CA | SER | 1111 | 3806 | 2299 | 2849 | 315 | -1075 | -512 | C |
| ATOM | 401 | CB | SER | 1111 | 12.201 | 25.396 | 3.878 | 1.00 | 27.76 | | C |
| ANISOU | 401 | CB | SER | 1111 | 3824 | 3662 | 3062 | 0 | -1020 | -1266 | C |
| ATOM | 402 | OG | SER | 1111 | 11.498 | 25.189 | 2.676 | 1.00 | 42.14 | | O |
| ANISOU | 402 | OG | SER | 1111 | 5683 | 7063 | 3265 | 797 | -1657 | -1928 | O |
| ATOM | 403 | C | SER | 1111 | 10.340 | 24.845 | 5.439 | 1.00 | 24.98 | | C |
| ANISOU | 403 | C | SER | 1111 | 3971 | 2663 | 2858 | 46 | -1352 | -279 | C |
| ATOM | 404 | O | SER | 1111 | 10.624 | 23.979 | 6.292 | 1.00 | 27.49 | | O |
| ANISOU | 404 | O | SER | 1111 | 4330 | 2449 | 3667 | -116 | -1758 | -69 | O |
| ATOM | 405 | N | LEU | 1112 | 9.162 | 24.891 | 4.853 | 1.00 | 25.97 | | N |
| ANISOU | 405 | N | LEU | 1112 | 3459 | 2928 | 3481 | 453 | -935 | 55 | N |
| ATOM | 406 | CA | LEU | 1112 | 8.099 | 23.958 | 5.140 | 1.00 | 26.54 | | C |
| ANISOU | 406 | CA | LEU | 1112 | 3871 | 3329 | 2885 | 15 | -1123 | 49 | C |
| ATOM | 407 | CB | LEU | 1112 | 6.778 | 24.566 | 5.617 | 1.00 | 28.64 | | C |
| ANISOU | 407 | CB | LEU | 1112 | 4042 | 4028 | 2811 | 75 | -416 | 702 | C |
| ATOM | 408 | CG | LEU | 1112 | 6.673 | 25.263 | 6.981 | 1.00 | 35.42 | | C |
| ANISOU | 408 | CG | LEU | 1112 | 5411 | 5109 | 2938 | 445 | 61 | 411 | C |
| ATOM | 409 | CD1 | LEU | 1112 | 5.275 | 25.868 | 7.146 | 1.00 | 42.56 | | C |
| ANISOU | 409 | CD1 | LEU | 1112 | 5747 | 6294 | 4129 | 885 | 889 | 276 | C |
| ATOM | 410 | CD2 | LEU | 1112 | 6.982 | 24.340 | 8.152 | 1.00 | 35.29 | | C |
| ANISOU | 410 | CD2 | LEU | 1112 | 4756 | 5792 | 2859 | -1573 | -713 | 897 | C |
| ATOM | 411 | C | LEU | 1112 | 7.826 | 23.161 | 3.848 | 1.00 | 33.48 | | C |
| ANISOU | 411 | C | LEU | 1112 | 4834 | 4490 | 3397 | -320 | -1158 | -747 | C |
| ATOM | 412 | O | LEU | 1112 | 6.662 | 22.930 | 3.579 | 1.00 | 39.94 | | O |
| ANISOU | 412 | O | LEU | 1112 | 5053 | 5312 | 4810 | 368 | -2203 | -1565 | O |
| ATOM | 1 | N | VAL | 1121 | 1.340 | 25.478 | 5.568 | 1.00 | 72.47 | | N |
| ANISOU | 1 | N | VAL | 1121 | 5592 | 8899 | 13045 | -1574 | 1973 | 1002 | N |
| ATOM | 2 | CA | VAL | 1121 | 0.038 | 24.833 | 5.597 | 1.00 | 61.07 | | C |
| ANISOU | 2 | CA | VAL | 1121 | 4968 | 6693 | 11545 | -487 | 1166 | 221 | C |
| ATOM | 3 | CB | VAL | 1121 | 0.069 | 23.454 | 6.300 | 1.00 | 67.25 | | C |
| ANISOU | 3 | CB | VAL | 1121 | 5568 | 6327 | 13657 | -237 | 1373 | 448 | C |
| ATOM | 4 | CG1 | VAL | 1121 | -1.353 | 22.969 | 6.508 | 1.00 | 55.98 | | C |
| ANISOU | 4 | CG1 | VAL | 1121 | 4761 | 1994 | 14516 | -1430 | -2727 | -1648 | C |
| ATOM | 5 | CG2 | VAL | 1121 | 0.871 | 22.431 | 5.512 | 1.00 | 85.79 | | C |
| ANISOU | 5 | CG2 | VAL | 1121 | 10094 | 7587 | 14915 | 1698 | 1696 | -299 | C |
| ATOM | 6 | C | VAL | 1121 | -1.002 | 25.674 | 6.330 | 1.00 | 49.24 | | C |
| ANISOU | 6 | C | VAL | 1121 | 4242 | 6355 | 8112 | -477 | -1276 | -419 | C |
| ATOM | 7 | O | VAL | 1121 | -0.657 | 26.225 | 7.377 | 1.00 | 54.11 | | O |
| ANISOU | 7 | O | VAL | 1121 | 5031 | 5585 | 9943 | -2387 | -2393 | -733 | O |
| ATOM | 8 | N | SER | 1122 | -2.208 | 25.733 | 5.764 | 1.00 | 43.40 | | N |
| ANISOU | 8 | N | SER | 1122 | 4244 | 6060 | 6187 | -1134 | -864 | 553 | N |
| ATOM | 9 | CA | SER | 1122 | -3.232 | 26.566 | 6.404 | 1.00 | 39.26 | | C |
| ANISOU | 9 | CA | SER | 1122 | 3836 | 5398 | 5683 | -1070 | -1940 | 375 | C |
| ATOM | 10 | CB | SER | 1122 | -4.525 | 26.528 | 5.577 | 1.00 | 43.90 | | C |
| ANISOU | 10 | CB | SER | 1122 | 4667 | 5623 | 6391 | -1026 | -2749 | 721 | C |
| ATOM | 11 | OG | SER | 1122 | -5.212 | 27.745 | 5.795 | 1.00 | 48.91 | | O |
| ANISOU | 11 | OG | SER | 1122 | 5138 | 6421 | 7023 | -227 | -3402 | 115 | O |
| ATOM | 12 | C | SER | 1122 | -3.479 | 26.141 | 7.848 | 1.00 | 35.32 | | C |
| ANISOU | 12 | C | SER | 1122 | 3546 | 4298 | 5578 | -1165 | -1820 | -44 | C |
| ATOM | 13 | O | SER | 1122 | -3.483 | 26.972 | 8.760 | 1.00 | 34.40 | | O |
| ANISOU | 13 | O | SER | 1122 | 3128 | 4035 | 5906 | -685 | -1858 | -101 | O |
| ATOM | 14 | N | GLN | 1123 | -3.682 | 24.845 | 8.117 | 1.00 | 36.12 | | N |
| ANISOU | 14 | N | GLN | 1123 | 3588 | 4270 | 5865 | -1432 | -2053 | -234 | N |
| ATOM | 15 | CA | GLN | 1123 | -3.861 | 24.395 | 9.495 | 1.00 | 35.36 | | C |
| ANISOU | 15 | CA | GLN | 1123 | 3438 | 3931 | 6067 | -1313 | -2133 | 51 | C |
| ATOM | 16 | CB | GLN | 1123 | -4.369 | 22.959 | 9.615 | 1.00 | 39.93 | | C |
| ANISOU | 16 | CB | GLN | 1123 | 4128 | 4338 | 6704 | -2111 | -2580 | 40 | C |
| ATOM | 17 | CG | GLN | 1123 | -3.776 | 22.071 | 8.538 | 1.00 | 51.55 | | C |
| ANISOU | 17 | CG | GLN | 1123 | 6432 | 4402 | 8751 | -1059 | -2502 | -1038 | C |
| ATOM | 18 | CD | GLN | 1123 | -3.183 | 20.773 | 9.058 | 1.00 | 62.86 | | C |
| ANISOU | 18 | CD | GLN | 1123 | 8215 | 5518 | 10152 | 133 | -2114 | -54 | C |
| ATOM | 19 | OE1 | GLN | 1123 | -3.830 | 19.713 | 8.974 | 1.00 | 73.59 | | O |
| ANISOU | 19 | OE1 | GLN | 1123 | 10215 | 4943 | 12804 | -98 | -3418 | 1053 | O |
| ATOM | 20 | NE2 | GLN | 1123 | -1.965 | 20.857 | 9.599 | 1.00 | 65.98 | | N |
| ANISOU | 20 | NE2 | GLN | 1123 | 7443 | 7441 | 10184 | 150 | -1368 | 2052 | N |
| ATOM | 21 | C | GLN | 1123 | -2.538 | 24.494 | 10.264 | 1.00 | 32.25 | | C |
| ANISOU | 21 | C | GLN | 1123 | 3233 | 3442 | 5579 | -1228 | -1763 | -661 | C |
| ATOM | 22 | O | GLN | 1123 | -2.545 | 24.850 | 11.437 | 1.00 | 31.57 | | O |
| ANISOU | 22 | O | GLN | 1123 | 3776 | 2974 | 5244 | -1094 | -1562 | -81 | O |
| ATOM | 23 | N | PHE | 1124 | -1.410 | 24.182 | 9.627 | 1.00 | 32.94 | | N |
| ANISOU | 23 | N | PHE | 1124 | 3591 | 2949 | 5975 | -149 | -2046 | -1123 | N |
| ATOM | 24 | CA | PHE | 1124 | -0.147 | 24.339 | 10.360 | 1.00 | 32.07 | | C |
| ANISOU | 24 | CA | PHE | 1124 | 3378 | 2467 | 6340 | -353 | -2007 | -607 | C |
| ATOM | 25 | CB | PHE | 1124 | 1.005 | 23.944 | 9.439 | 1.00 | 33.05 | | C |
| ANISOU | 25 | CB | PHE | 1124 | 3549 | 3324 | 5684 | -110 | -2427 | -1263 | C |
| ATOM | 26 | CG | PHE | 1124 | 2.347 | 23.846 | 10.151 | 1.00 | 29.57 | | C |
| ANISOU | 26 | CG | PHE | 1124 | 3290 | 2972 | 4972 | -582 | -1941 | -228 | C |
| ATOM | 27 | CD1 | PHE | 1124 | 2.570 | 22.757 | 10.992 | 1.00 | 33.21 | | C |
| ANISOU | 27 | CD1 | PHE | 1124 | 4327 | 3300 | 4994 | -836 | -1991 | 99 | C |
| ATOM | 28 | CD2 | PHE | 1124 | 3.322 | 24.800 | 9.976 | 1.00 | 27.33 | | C |
| ANISOU | 28 | CD2 | PHE | 1124 | 3152 | 3024 | 4207 | -396 | -1038 | -544 | C |
| ATOM | 29 | CE1 | PHE | 1124 | 3.774 | 22.653 | 11.643 | 1.00 | 33.03 | | C |
| ANISOU | 29 | CE1 | PHE | 1124 | 4111 | 2984 | 5456 | -464 | -1788 | 583 | C |
| ATOM | 30 | CE2 | PHE | 1124 | 4.541 | 24.713 | 10.623 | 1.00 | 25.55 | | C |
| ANISOU | 30 | CE2 | PHE | 1124 | 2951 | 3074 | 3684 | -675 | -756 | -444 | C |
| ATOM | 31 | CZ | PHE | 1124 | 4.760 | 23.623 | 11.462 | 1.00 | 31.68 | | C |
| ANISOU | 31 | CZ | PHE | 1124 | 3734 | 3435 | 4868 | -454 | -1270 | 110 | C |
| ATOM | 32 | C | PHE | 1124 | 0.046 | 25.755 | 10.866 | 1.00 | 30.72 | | C |
| ANISOU | 32 | C | PHE | 1124 | 4081 | 2478 | 5115 | -267 | -2101 | -521 | C |
| ATOM | 33 | O | PHE | 1124 | 0.464 | 26.024 | 11.996 | 1.00 | 29.09 | | O |
| ANISOU | 33 | O | PHE | 1124 | 3054 | 3316 | 4683 | -796 | -1561 | -141 | O |
| ATOM | 34 | N | LEU | 1125 | -0.274 | 26.708 | 9.968 | 1.00 | 29.88 | | N |
| ANISOU | 34 | N | LEU | 1125 | 4302 | 2539 | 4511 | -1310 | -1801 | -326 | N |
| ATOM | 35 | CA | LEU | 1125 | -0.186 | 28.106 | 10.385 | 1.00 | 30.03 | | C |
| ANISOU | 35 | CA | LEU | 1125 | 4964 | 2455 | 3992 | -1151 | -1550 | 39 | C |
| ATOM | 36 | CB | LEU | 1125 | -0.424 | 29.022 | 9.166 | 1.00 | 34.53 | | C |
| ANISOU | 36 | CB | LEU | 1125 | 5602 | 3163 | 4355 | -956 | -1921 | 447 | C |
| ATOM | 37 | CG | LEU | 1125 | 0.637 | 28.998 | 8.092 | 1.00 | 32.22 | | C |
| ANISOU | 37 | CG | LEU | 1125 | 5087 | 3371 | 3785 | -1554 | -2444 | 708 | C |
| ATOM | 38 | CD1 | LEU | 1125 | 0.217 | 29.913 | 6.947 | 1.00 | 38.65 | | C |
| ANISOU | 38 | CD1 | LEU | 1125 | 6495 | 4256 | 3933 | -1442 | -3149 | 909 | C |
| ATOM | 39 | CD2 | LEU | 1125 | 1.987 | 29.426 | 8.639 | 1.00 | 34.23 | | C |
| ANISOU | 39 | CD2 | LEU | 1125 | 5157 | 2590 | 5258 | -1091 | -3031 | 603 | C |
| ATOM | 40 | C | LEU | 1125 | -1.175 | 28.456 | 11.473 | 1.00 | 28.26 | | C |
| ANISOU | 40 | C | LEU | 1125 | 4081 | 2268 | 4390 | -948 | -1854 | 58 | C |
| ATOM | 41 | O | LEU | 1125 | -0.836 | 29.105 | 12.463 | 1.00 | 28.07 | | O |
| ANISOU | 41 | O | LEU | 1125 | 4000 | 3035 | 3632 | -1170 | -1627 | 313 | O |
| ATOM | 42 | N | THR | 1126 | -2.458 | 28.069 | 11.362 | 1.00 | 29.89 | | N |
| ANISOU | 42 | N | THR | 1126 | 3787 | 2436 | 5134 | -373 | -2603 | 401 | N |
| ATOM | 43 | CA | THR | 1126 | -3.243 | 28.600 | 12.518 | 1.00 | 40.40 | | C |
| ANISOU | 43 | CA | THR | 1126 | 3953 | 5618 | 5780 | -31 | -1707 | 457 | C |
| ATOM | 44 | CB | THR | 1126 | -4.746 | 28.680 | 12.170 | 1.00 | 45.32 | | C |
| ANISOU | 44 | CB | THR | 1126 | 3928 | 7468 | 5824 | -327 | -1709 | 753 | C |
| ATOM | 45 | OG1 | THR | 1126 | -5.596 | 28.227 | 13.250 | 1.00 | 59.39 | | O |
| ANISOU | 45 | OG1 | THR | 1126 | 5038 | 11238 | 6290 | -886 | 132 | -1180 | O |
| ATOM | 46 | CG2 | THR | 1126 | -5.011 | 27.796 | 10.956 | 1.00 | 50.18 | | C |
| ANISOU | 46 | CG2 | THR | 1126 | 2655 | 10678 | 5735 | -411 | -874 | -625 | C |
| ATOM | 47 | C | THR | 1126 | -2.915 | 27.768 | 13.755 | 1.00 | 37.03 | | C |
| ANISOU | 47 | C | THR | 1126 | 3372 | 5091 | 5608 | -575 | -1324 | 412 | C |
| ATOM | 48 | O | THR | 1126 | -3.200 | 28.196 | 14.866 | 1.00 | 37.84 | | O |
| ANISOU | 48 | O | THR | 1126 | 3645 | 4985 | 5748 | -1285 | -689 | 487 | O |
| ATOM | 49 | N | GLU | 1127 | -2.309 | 26.605 | 13.577 | 1.00 | 34.12 | | N |
| ANISOU | 49 | N | GLU | 1127 | 2797 | 4193 | 5974 | -1567 | -1491 | 396 | N |
| ATOM | 50 | CA | GLU | 1127 | -1.744 | 25.774 | 14.629 | 1.00 | 38.13 | | C |
| ANISOU | 50 | CA | GLU | 1127 | 3272 | 4634 | 6583 | -1134 | -1544 | 741 | C |
| ATOM | 51 | CB | GLU | 1127 | -1.171 | 24.484 | 14.025 | 1.00 | 48.00 | | C |
| ANISOU | 51 | CB | GLU | 1127 | 5125 | 4605 | 8509 | -558 | -2123 | 208 | C |
| ATOM | 56 | C | GLU | 1127 | -0.633 | 26.471 | 15.391 | 1.00 | 36.99 | | C |
| ANISOU | 56 | C | GLU | 1127 | 3557 | 5093 | 5407 | -1331 | -1479 | 1100 | C |
| ATOM | 57 | O | GLU | 1127 | -0.502 | 26.448 | 16.628 | 1.00 | 48.39 | | O |
| ANISOU | 57 | O | GLU | 1127 | 4063 | 9169 | 5153 | -2634 | -499 | 679 | O |
| ATOM | 58 | N | GLY | 1128 | 0.211 | 27.120 | 14.591 | 1.00 | 25.15 | | N |
| ANISOU | 58 | N | GLY | 1128 | 2581 | 2498 | 4475 | 60 | -1241 | 6 | N |
| ATOM | 59 | CA | GLY | 1128 | 1.465 | 27.602 | 15.152 | 1.00 | 22.65 | | C |
| ANISOU | 59 | CA | GLY | 1128 | 2748 | 2081 | 3775 | 177 | -1292 | 33 | C |
| ATOM | 60 | C | GLY | 1128 | 1.599 | 29.102 | 15.329 | 1.00 | 20.93 | | C |
| ANISOU | 60 | C | GLY | 1128 | 2744 | 2096 | 3110 | 240 | -994 | 145 | C |
| ATOM | 61 | O | GLY | 1128 | 2.546 | 29.575 | 15.961 | 1.00 | 19.89 | | O |
| ANISOU | 61 | O | GLY | 1128 | 2496 | 2105 | 2956 | 143 | -651 | 30 | O |
| ATOM | 62 | N | ILE | 1129 | 0.692 | 29.919 | 14.798 | 1.00 | 22.67 | | N |
| ANISOU | 62 | N | ILE | 1129 | 3052 | 2102 | 3460 | 452 | -1123 | 94 | N |
| ATOM | 63 | CA | ILE | 1129 | 0.820 | 31.368 | 14.858 | 1.00 | 20.75 | | C |
| ANISOU | 63 | CA | ILE | 1129 | 3180 | 2117 | 2589 | -72 | -1156 | 634 | C |
| ATOM | 64 | CB | ILE | 1129 | -0.243 | 32.007 | 13.898 | 1.00 | 19.51 | | C |
| ANISOU | 64 | CB | ILE | 1129 | 2081 | 2274 | 3058 | 50 | -591 | 714 | C |
| ATOM | 65 | CG2 | ILE | 1129 | -1.700 | 31.731 | 14.229 | 1.00 | 21.61 | | C |
| ANISOU | 65 | CG2 | ILE | 1129 | 2453 | 2437 | 3320 | 37 | 237 | 735 | C |
| ATOM | 66 | CG1 | ILE | 1129 | -0.018 | 33.529 | 13.747 | 1.00 | 19.42 | | C |
| ANISOU | 66 | CG1 | ILE | 1129 | 2164 | 2293 | 2922 | 37 | -733 | 889 | C |
| ATOM | 67 | CD1 | ILE | 1129 | 1.421 | 33.802 | 13.305 | 1.00 | 20.99 | | C |
| ANISOU | 67 | CD1 | ILE | 1129 | 2634 | 2162 | 3180 | -110 | 192 | 72 | C |
| ATOM | 68 | C | ILE | 1129 | 0.730 | 31.878 | 16.279 | 1.00 | 20.07 | | C |
| ANISOU | 68 | C | ILE | 1129 | 2615 | 2165 | 2845 | 81 | -436 | 295 | C |
| ATOM | 69 | O | ILE | 1129 | 1.163 | 33.014 | 16.552 | 1.00 | 22.80 | | O |
| ANISOU | 69 | O | ILE | 1129 | 3052 | 2177 | 3433 | 221 | -878 | 63 | O |
| ATOM | 70 | N | ILE | 1130 | 0.222 | 31.086 | 17.202 | 1.00 | 21.50 | | N |
| ANISOU | 70 | N | ILE | 1130 | 2186 | 2904 | 3080 | 292 | -322 | 639 | N |
| ATOM | 71 | CA | ILE | 1130 | 0.184 | 31.498 | 18.608 | 1.00 | 21.81 | | C |
| ANISOU | 71 | CA | ILE | 1130 | 2392 | 2901 | 2993 | 135 | -365 | 890 | C |
| ATOM | 72 | CB | ILE | 1130 | -0.387 | 30.340 | 19.450 | 1.00 | 23.97 | | C |
| ANISOU | 72 | CB | ILE | 1130 | 2219 | 3242 | 3645 | -147 | -74 | 1068 | C |
| ATOM | 73 | CG2 | ILE | 1130 | 0.528 | 29.133 | 19.520 | 1.00 | 32.46 | | C |
| ANISOU | 73 | CG2 | ILE | 1130 | 3977 | 3002 | 5356 | 310 | -376 | 1457 | C |
| ATOM | 74 | CG1 | ILE | 1130 | -0.736 | 30.739 | 20.881 | 1.00 | 29.64 | | C |
| ANISOU | 74 | CG1 | ILE | 1130 | 3929 | 3684 | 3648 | -1053 | 371 | 1093 | C |
| ATOM | 75 | CD1 | ILE | 1130 | -1.361 | 29.577 | 21.653 | 1.00 | 35.98 | | C |
| ANISOU | 75 | CD1 | ILE | 1130 | 5169 | 4705 | 3798 | -2202 | -388 | 1712 | C |
| ATOM | 76 | C | ILE | 1130 | 1.537 | 31.912 | 19.124 | 1.00 | 21.45 | | C |
| ANISOU | 76 | C | ILE | 1130 | 2383 | 2827 | 2942 | -159 | 18 | 462 | C |
| ATOM | 77 | O | ILE | 1130 | 1.701 | 32.732 | 20.041 | 1.00 | 20.97 | | O |
| ANISOU | 77 | O | ILE | 1130 | 2412 | 2393 | 3164 | 75 | 131 | 476 | O |
| ATOM | 78 | N | MET | 1131 | 2.603 | 31.369 | 18.556 | 1.00 | 19.58 | | N |
| ANISOU | 78 | N | MET | 1131 | 2341 | 2260 | 2839 | -267 | -295 | 196 | N |
| ATOM | 79 | CA | MET | 1131 | 3.939 | 31.724 | 19.004 | 1.00 | 18.04 | | C |
| ANISOU | 79 | CA | MET | 1131 | 2364 | 1634 | 2855 | -28 | -487 | -1 | C |
| ATOM | 80 | CB | MET | 1131 | 4.984 | 30.859 | 18.289 | 1.00 | 19.41 | | C |
| ANISOU | 80 | CB | MET | 1131 | 2535 | 1882 | 2958 | 96 | -471 | -188 | C |
| ATOM | 81 | CG | MET | 1131 | 5.273 | 31.261 | 16.862 | 1.00 | 22.00 | | C |
| ANISOU | 81 | CG | MET | 1131 | 2426 | 2954 | 2979 | 236 | -368 | -17 | C |
| ATOM | 82 | SD | MET | 1131 | 6.430 | 30.202 | 15.919 | 1.00 | 21.10 | | S |
| ANISOU | 82 | SD | MET | 1131 | 3230 | 1778 | 3010 | -57 | 39 | 266 | S |
| ATOM | 83 | CE | MET | 1131 | 7.801 | 30.100 | 17.020 | 1.00 | 22.86 | | C |
| ANISOU | 83 | CE | MET | 1131 | 2535 | 1581 | 4569 | 194 | -191 | -319 | C |
| ATOM | 84 | C | MET | 1131 | 4.234 | 33.230 | 18.801 | 1.00 | 17.51 | | C |
| ANISOU | 84 | C | MET | 1131 | 2547 | 1647 | 2459 | -41 | -323 | 50 | C |
| ATOM | 85 | O | MET | 1131 | 5.141 | 33.738 | 19.474 | 1.00 | 17.77 | | O |
| ANISOU | 85 | O | MET | 1131 | 2017 | 1699 | 3036 | 72 | -152 | -252 | O |
| ATOM | 86 | N | LYS | 1132 | 3.504 | 33.922 | 17.927 | 1.00 | 17.69 | | N |
| ANISOU | 86 | N | LYS | 1132 | 2663 | 1790 | 2267 | -50 | -147 | 120 | N |
| ATOM | 87 | CA | LYS | 1132 | 3.771 | 35.365 | 17.772 | 1.00 | 16.74 | | C |
| ANISOU | 87 | CA | LYS | 1132 | 2007 | 1830 | 2525 | 18 | -536 | 251 | C |
| ATOM | 88 | CB | LYS | 1132 | 2.994 | 35.937 | 16.571 | 1.00 | 20.03 | | C |
| ANISOU | 88 | CB | LYS | 1132 | 2429 | 2341 | 2840 | 216 | -779 | 416 | C |
| ATOM | 89 | CG | LYS | 1132 | 1.553 | 36.289 | 17.024 | 1.00 | 21.87 | | C |
| ANISOU | 89 | CG | LYS | 1132 | 2372 | 2620 | 3316 | 365 | -774 | 819 | C |
| ATOM | 90 | CD | LYS | 1132 | 0.782 | 36.818 | 15.823 | 1.00 | 25.78 | | C |
| ANISOU | 90 | CD | LYS | 1132 | 3024 | 3290 | 3480 | 869 | -1143 | 612 | C |
| ATOM | 91 | CE | LYS | 1132 | -0.506 | 37.490 | 16.241 | 1.00 | 29.34 | | C |
| ANISOU | 91 | CE | LYS | 1132 | 3071 | 4937 | 3140 | 1348 | -1285 | 447 | C |
| ATOM | 92 | NZ | LYS | 1132 | -0.308 | 38.628 | 17.160 | 1.00 | 36.92 | | N1+ |
| ANISOU | 92 | NZ | LYS | 1132 | 4640 | 4017 | 5370 | 1264 | -100 | -57 | N1+ |
| ATOM | 93 | C | LYS | 1132 | 3.423 | 36.127 | 19.036 | 1.00 | 17.59 | | C |
| ANISOU | 93 | C | LYS | 1132 | 2235 | 1641 | 2807 | 151 | -584 | 126 | C |
| ATOM | 94 | O | LYS | 1132 | 3.919 | 37.245 | 19.237 | 1.00 | 20.41 | | O |
| ANISOU | 94 | O | LYS | 1132 | 2989 | 1644 | 3124 | 49 | -1083 | 155 | O |
| ATOM | 95 | N | ASP | 1133 | 2.588 | 35.567 | 19.908 | 1.00 | 17.71 | | N |
| ANISOU | 95 | N | ASP | 1133 | 1687 | 2341 | 2701 | 114 | -561 | -250 | N |
| ATOM | 96 | CA | ASP | 1133 | 2.180 | 36.209 | 21.123 | 1.00 | 19.73 | | C |
| ANISOU | 96 | CA | ASP | 1133 | 2170 | 2501 | 2824 | 611 | -567 | -233 | C |
| ATOM | 97 | CB | ASP | 1133 | 0.759 | 35.768 | 21.475 | 1.00 | 22.85 | | C |
| ANISOU | 97 | CB | ASP | 1133 | 2009 | 3708 | 2966 | 875 | -335 | -123 | C |
| ATOM | 98 | CG | ASP | 1133 | -0.299 | 36.124 | 20.466 | 1.00 | 25.69 | | C |
| ANISOU | 98 | CG | ASP | 1133 | 2404 | 3583 | 3774 | 474 | -1087 | -337 | C |
| ATOM | 99 | OD1 | ASP | 1133 | -0.222 | 37.154 | 19.779 | 1.00 | 31.92 | | O |
| ANISOU | 99 | OD1 | ASP | 1133 | 4537 | 3878 | 3714 | 1791 | -909 | 5 | O |
| ATOM | 100 | OD2 | ASP | 1133 | -1.258 | 35.354 | 20.333 | 1.00 | 37.84 | | O1- |
| ANISOU | 100 | OD2 | ASP | 1133 | 2583 | 6074 | 5721 | -557 | -862 | -1513 | O1- |
| ATOM | 101 | C | ASP | 1133 | 3.057 | 35.874 | 22.327 | 1.00 | 19.57 | | C |
| ANISOU | 101 | C | ASP | 1133 | 2285 | 2386 | 2766 | 726 | -605 | -446 | C |
| ATOM | 102 | O | ASP | 1133 | 2.814 | 36.321 | 23.462 | 1.00 | 23.20 | | O |
| ANISOU | 102 | O | ASP | 1133 | 3363 | 2582 | 2869 | 730 | -692 | -724 | O |
| ATOM | 103 | N | PHE | 1134 | 4.084 | 35.058 | 22.104 | 1.00 | 15.45 | | N |
| ANISOU | 103 | N | PHE | 1134 | 1547 | 1477 | 2846 | -81 | -326 | 130 | N |
| ATOM | 104 | CA | PHE | 1134 | 4.985 | 34.723 | 23.190 | 1.00 | 15.56 | | C |
| ANISOU | 104 | CA | PHE | 1134 | 1599 | 1325 | 2989 | -123 | -474 | -23 | C |
| ATOM | 105 | CB | PHE | 1134 | 5.407 | 33.251 | 23.135 | 1.00 | 17.33 | | C |
| ANISOU | 105 | CB | PHE | 1134 | 2217 | 1187 | 3182 | -231 | -354 | 359 | C |
| ATOM | 106 | CG | PHE | 1134 | 4.268 | 32.244 | 23.266 | 1.00 | 15.66 | | C |
| ANISOU | 106 | CG | PHE | 1134 | 2001 | 1279 | 2671 | -132 | -653 | 255 | C |
| ATOM | 107 | CD1 | PHE | 1134 | 3.054 | 32.498 | 23.882 | 1.00 | 19.74 | | C |
| ANISOU | 107 | CD1 | PHE | 1134 | 2421 | 2117 | 2962 | -711 | -91 | -374 | C |
| ATOM | 108 | CD2 | PHE | 1134 | 4.434 | 30.969 | 22.743 | 1.00 | 17.42 | | C |
| ANISOU | 108 | CD2 | PHE | 1134 | 2393 | 1245 | 2981 | -183 | -859 | 203 | C |
| ATOM | 109 | CE1 | PHE | 1134 | 2.034 | 31.562 | 23.980 | 1.00 | 17.15 | | C |
| ANISOU | 109 | CE1 | PHE | 1134 | 2683 | 1666 | 2167 | -708 | -213 | -40 | C |
| ATOM | 110 | CE2 | PHE | 1134 | 3.446 | 30.022 | 22.829 | 1.00 | 16.40 | | C |
| ANISOU | 110 | CE2 | PHE | 1134 | 2113 | 1416 | 2703 | -190 | -931 | 262 | C |
| ATOM | 111 | CZ | PHE | 1134 | 2.245 | 30.287 | 23.448 | 1.00 | 16.42 | | C |
| ANISOU | 111 | CZ | PHE | 1134 | 2551 | 1545 | 2144 | -66 | -647 | 182 | C |
| ATOM | 112 | C | PHE | 1134 | 6.216 | 35.628 | 23.134 | 1.00 | 13.98 | | C |
| ANISOU | 112 | C | PHE | 1134 | 1660 | 1277 | 2375 | -176 | -154 | -183 | C |
| ATOM | 113 | O | PHE | 1134 | 6.825 | 35.747 | 22.062 | 1.00 | 17.67 | | O |
| ANISOU | 113 | O | PHE | 1134 | 2131 | 2319 | 2263 | -328 | -109 | -313 | O |
| ATOM | 114 | N | SER | 1135 | 6.552 | 36.243 | 24.262 | 1.00 | 12.93 | | N |
| ANISOU | 114 | N | SER | 1135 | 1498 | 1285 | 2129 | -57 | -253 | 108 | N |
| ATOM | 115 | CA | SER | 1135 | 7.684 | 37.146 | 24.343 | 1.00 | 11.74 | | C |
| ANISOU | 115 | CA | SER | 1135 | 1360 | 1055 | 2047 | 137 | -332 | -90 | C |
| ATOM | 116 | CB | SER | 1135 | 7.266 | 38.636 | 24.273 | 1.00 | 13.92 | | C |
| ANISOU | 116 | CB | SER | 1135 | 1784 | 1136 | 2369 | 315 | -380 | 281 | C |
| ATOM | 117 | OG | SER | 1135 | 6.582 | 38.923 | 23.097 | 1.00 | 18.16 | | O |
| ANISOU | 117 | OG | SER | 1135 | 2313 | 1925 | 2662 | 293 | -688 | 425 | O |
| ATOM | 118 | C | SER | 1135 | 8.378 | 36.876 | 25.651 | 1.00 | 11.79 | | C |
| ANISOU | 118 | C | SER | 1135 | 1598 | 1029 | 1850 | 296 | -230 | 8 | C |
| ATOM | 119 | O | SER | 1135 | 8.077 | 37.530 | 26.668 | 1.00 | 13.31 | | O |
| ANISOU | 119 | O | SER | 1135 | 1956 | 1198 | 1906 | 461 | -40 | 9 | O |
| ATOM | 120 | N | HIS | 1136 | 9.299 | 35.924 | 25.645 | 1.00 | 10.04 | | N |
| ANISOU | 120 | N | HIS | 1136 | 1434 | 806 | 1577 | 38 | -173 | 66 | N |
| ATOM | 121 | CA | HIS | 1136 | 10.042 | 35.588 | 26.861 | 1.00 | 9.82 | | C |
| ANISOU | 121 | CA | HIS | 1136 | 1276 | 799 | 1655 | 12 | -95 | 86 | C |
| ATOM | 122 | CB | HIS | 1136 | 9.376 | 34.424 | 27.591 | 1.00 | 9.93 | | C |
| ANISOU | 122 | CB | HIS | 1136 | 1182 | 939 | 1654 | -12 | -2 | 221 | C |
| ATOM | 123 | CG | HIS | 1136 | 10.034 | 34.102 | 28.896 | 1.00 | 9.54 | | C |
| ANISOU | 123 | CG | HIS | 1136 | 1092 | 976 | 1555 | 21 | 99 | 33 | C |
| ATOM | 124 | CD2 | HIS | 1136 | 9.676 | 34.443 | 30.165 | 1.00 | 9.85 | | C |
| ANISOU | 124 | CD2 | HIS | 1136 | 1480 | 655 | 1607 | 78 | 147 | -71 | C |
| ATOM | 125 | ND1 | HIS | 1136 | 11.205 | 33.365 | 28.946 | 1.00 | 8.72 | | N |
| ANISOU | 125 | ND1 | HIS | 1136 | 1160 | 717 | 1435 | 18 | 74 | 61 | N |
| ATOM | 126 | CE1 | HIS | 1136 | 11.519 | 33.280 | 30.224 | 1.00 | 9.37 | | C |
| ANISOU | 126 | CE1 | HIS | 1136 | 1322 | 860 | 1380 | -22 | 133 | 67 | C |
| ATOM | 127 | NE2 | HIS | 1136 | 10.616 | 33.917 | 30.998 | 1.00 | 10.35 | | N |
| ANISOU | 127 | NE2 | HIS | 1136 | 1412 | 957 | 1563 | 125 | 87 | -90 | N |
| ATOM | 128 | C | HIS | 1136 | 11.482 | 35.288 | 26.468 | 1.00 | 9.66 | | C |
| ANISOU | 128 | C | HIS | 1136 | 1255 | 952 | 1463 | -181 | -20 | 89 | C |
| ATOM | 129 | O | HIS | 1136 | 11.673 | 34.636 | 25.416 | 1.00 | 9.82 | | O |
| ANISOU | 129 | O | HIS | 1136 | 1318 | 934 | 1481 | -18 | -65 | -26 | O |
| ATOM | 130 | N | PRO | 1137 | 12.471 | 35.711 | 27.237 | 1.00 | 10.09 | | N |
| ANISOU | 130 | N | PRO | 1137 | 1324 | 961 | 1548 | -160 | -91 | -15 | N |
| ATOM | 131 | CD | PRO | 1137 | 12.370 | 36.474 | 28.512 | 1.00 | 11.92 | | C |
| ANISOU | 131 | CD | PRO | 1137 | 1736 | 1171 | 1623 | -102 | -172 | -116 | C |
| ATOM | 132 | CA | PRO | 1137 | 13.862 | 35.492 | 26.871 | 1.00 | 10.67 | | C |
| ANISOU | 132 | CA | PRO | 1137 | 1256 | 962 | 1837 | -276 | -93 | 58 | C |
| ATOM | 133 | CB | PRO | 1137 | 14.694 | 36.051 | 28.062 | 1.00 | 12.67 | | C |
| ANISOU | 133 | CB | PRO | 1137 | 1568 | 1245 | 2001 | -371 | -272 | -102 | C |
| ATOM | 134 | CG | PRO | 1137 | 13.736 | 36.240 | 29.164 | 1.00 | 17.19 | | C |
| ANISOU | 134 | CG | PRO | 1137 | 1670 | 3026 | 1836 | -511 | -324 | -304 | C |
| ATOM | 135 | C | PRO | 1137 | 14.242 | 34.024 | 26.695 | 1.00 | 9.58 | | C |
| ANISOU | 135 | C | PRO | 1137 | 1324 | 1011 | 1306 | -111 | -64 | 181 | C |
| ATOM | 136 | O | PRO | 1137 | 15.255 | 33.758 | 25.992 | 1.00 | 11.79 | | O |
| ANISOU | 136 | O | PRO | 1137 | 1196 | 1328 | 1957 | 25 | 131 | 381 | O |
| ATOM | 137 | N | ASN | 1138 | 13.512 | 33.101 | 27.287 | 1.00 | 9.06 | | N |
| ANISOU | 137 | N | ASN | 1138 | 1272 | 893 | 1277 | -144 | -119 | 138 | N |
| ATOM | 138 | CA | ASN | 1138 | 13.902 | 31.681 | 27.137 | 1.00 | 9.51 | | C |
| ANISOU | 138 | CA | ASN | 1138 | 1304 | 876 | 1434 | 35 | -437 | 152 | C |
| ATOM | 139 | CB | ASN | 1138 | 14.029 | 31.046 | 28.537 | 1.00 | 8.44 | | C |
| ANISOU | 139 | CB | ASN | 1138 | 887 | 991 | 1329 | 18 | -144 | 116 | C |
| ATOM | 140 | CG | ASN | 1138 | 15.059 | 31.798 | 29.353 | 1.00 | 8.48 | | C |
| ANISOU | 140 | CG | ASN | 1138 | 1035 | 896 | 1291 | 50 | -163 | -12 | C |
| ATOM | 141 | OD1 | ASN | 1138 | 14.733 | 32.372 | 30.392 | 1.00 | 10.22 | | O |
| ANISOU | 141 | OD1 | ASN | 1138 | 1271 | 1170 | 1442 | -86 | 55 | -126 | O |
| ATOM | 142 | ND2 | ASN | 1138 | 16.322 | 31.761 | 28.876 | 1.00 | 9.05 | | N |
| ANISOU | 142 | ND2 | ASN | 1138 | 983 | 1039 | 1417 | 34 | -101 | 77 | N |
| ATOM | 143 | C | ASN | 1138 | 12.930 | 30.923 | 26.246 | 1.00 | 8.10 | | C |
| ANISOU | 143 | C | ASN | 1138 | 881 | 948 | 1247 | 13 | -31 | -4 | C |
| ATOM | 144 | O | ASN | 1138 | 12.849 | 29.690 | 26.333 | 1.00 | 8.68 | | O |
| ANISOU | 144 | O | ASN | 1138 | 1021 | 976 | 1302 | 59 | -87 | -57 | O |
| ATOM | 145 | N | VAL | 1139 | 12.234 | 31.617 | 25.380 | 1.00 | 8.68 | | N |
| ANISOU | 145 | N | VAL | 1139 | 1013 | 1074 | 1212 | -114 | -133 | 11 | N |
| ATOM | 146 | CA | VAL | 1139 | 11.317 | 31.040 | 24.393 | 1.00 | 7.91 | | C |
| ANISOU | 146 | CA | VAL | 1139 | 897 | 993 | 1116 | -72 | 0 | -17 | C |
| ATOM | 147 | CB | VAL | 1139 | 9.856 | 31.349 | 24.768 | 1.00 | 9.51 | | C |
| ANISOU | 147 | CB | VAL | 1139 | 880 | 1335 | 1398 | -35 | -24 | 108 | C |
| ATOM | 148 | CG1 | VAL | 1139 | 8.882 | 30.831 | 23.691 | 1.00 | 9.96 | | C |
| ANISOU | 148 | CG1 | VAL | 1139 | 1074 | 1196 | 1513 | -87 | -229 | 157 | C |
| ATOM | 149 | CG2 | VAL | 1139 | 9.533 | 30.782 | 26.149 | 1.00 | 9.84 | | C |
| ANISOU | 149 | CG2 | VAL | 1139 | 1032 | 1365 | 1340 | -32 | 129 | 25 | C |
| ATOM | 150 | C | VAL | 1139 | 11.675 | 31.603 | 23.038 | 1.00 | 8.34 | | C |
| ANISOU | 150 | C | VAL | 1139 | 1108 | 912 | 1149 | -17 | -36 | 23 | C |
| ATOM | 151 | O | VAL | 1139 | 11.768 | 32.833 | 22.884 | 1.00 | 9.75 | | O |
| ANISOU | 151 | O | VAL | 1139 | 1403 | 892 | 1411 | -35 | 28 | 59 | O |
| ATOM | 152 | N | LEU | 1140 | 11.889 | 30.732 | 22.038 | 1.00 | 8.67 | | N |
| ANISOU | 152 | N | LEU | 1140 | 1182 | 1064 | 1048 | 36 | -120 | -20 | N |
| ATOM | 153 | CA | LEU | 1140 | 12.237 | 31.217 | 20.715 | 1.00 | 9.13 | | C |
| ANISOU | 153 | CA | LEU | 1140 | 1333 | 1073 | 1062 | 146 | -28 | 43 | C |
| ATOM | 154 | CB | LEU | 1140 | 12.512 | 30.000 | 19.814 | 1.00 | 10.25 | | C |
| ANISOU | 154 | CB | LEU | 1140 | 1631 | 1054 | 1210 | 3 | 79 | -81 | C |
| ATOM | 155 | CG | LEU | 1140 | 13.111 | 30.363 | 18.446 | 1.00 | 11.11 | | C |
| ANISOU | 155 | CG | LEU | 1140 | 1562 | 1457 | 1202 | 14 | 103 | -155 | C |
| ATOM | 156 | CD1 | LEU | 1140 | 14.565 | 30.728 | 18.637 | 1.00 | 14.60 | | C |
| ANISOU | 156 | CD1 | LEU | 1140 | 1475 | 1896 | 2178 | -99 | 247 | -78 | C |
| ATOM | 157 | CD2 | LEU | 1140 | 12.877 | 29.194 | 17.482 | 1.00 | 12.76 | | C |
| ANISOU | 157 | CD2 | LEU | 1140 | 2174 | 1544 | 1131 | 247 | -172 | -167 | C |
| ATOM | 158 | C | LEU | 1140 | 11.126 | 32.067 | 20.133 | 1.00 | 10.00 | | C |
| ANISOU | 158 | C | LEU | 1140 | 1345 | 1259 | 1195 | 179 | -59 | 138 | C |
| ATOM | 159 | O | LEU | 1140 | 9.951 | 31.694 | 20.100 | 1.00 | 12.73 | | O |
| ANISOU | 159 | O | LEU | 1140 | 1376 | 1689 | 1773 | 88 | -121 | 374 | O |
| ATOM | 160 | N | SER | 1141 | 11.484 | 33.260 | 19.643 | 1.00 | 10.90 | | N |
| ANISOU | 160 | N | SER | 1141 | 1642 | 1190 | 1309 | 241 | -76 | 132 | N |
| ATOM | 161 | CA | SER | 1141 | 10.447 | 34.120 | 19.039 | 1.00 | 13.26 | | C |
| ANISOU | 161 | CA | SER | 1141 | 2361 | 1267 | 1410 | 528 | -421 | 114 | C |
| ATOM | 162 | CB | SER | 1141 | 10.817 | 35.559 | 19.220 | 1.00 | 19.24 | | C |
| ANISOU | 162 | CB | SER | 1141 | 3068 | 1328 | 2913 | 447 | -1004 | -104 | C |
| ATOM | 163 | OG | SER | 1141 | 12.016 | 35.843 | 18.584 | 1.00 | 33.36 | | O |
| ANISOU | 163 | OG | SER | 1141 | 3664 | 2550 | 6463 | -736 | -133 | 736 | O |
| ATOM | 164 | C | SER | 1141 | 10.298 | 33.817 | 17.567 | 1.00 | 11.99 | | C |
| ANISOU | 164 | C | SER | 1141 | 1499 | 1685 | 1372 | 30 | -91 | 309 | C |
| ATOM | 165 | O | SER | 1141 | 11.122 | 33.170 | 16.930 | 1.00 | 13.68 | | O |
| ANISOU | 165 | O | SER | 1141 | 1870 | 1697 | 1630 | 103 | -77 | -35 | O |
| ATOM | 166 | N | LEU | 1142 | 9.208 | 34.311 | 17.034 | 1.00 | 13.20 | | N |
| ANISOU | 166 | N | LEU | 1142 | 1798 | 1629 | 1587 | 106 | -393 | 257 | N |
| ATOM | 167 | CA | LEU | 1142 | 8.959 | 34.364 | 15.613 | 1.00 | 13.00 | | C |
| ANISOU | 167 | CA | LEU | 1142 | 1751 | 1708 | 1482 | 19 | -262 | 430 | C |
| ATOM | 168 | CB | LEU | 1142 | 7.467 | 34.101 | 15.343 | 1.00 | 14.74 | | C |
| ATOM | 169 | CG | LEU | 1142 | 7.026 | 34.223 | 13.891 | 1.00 | 14.30 | | C |
| ANISOU | 169 | CG | LEU | 1142 | 1728 | 2027 | 1678 | -28 | -345 | -11 | C |
| ATOM | 170 | CD1 | LEU | 1142 | 7.679 | 33.152 | 13.024 | 1.00 | 15.60 | | C |
| ANISOU | 170 | CD1 | LEU | 1142 | 2045 | 1582 | 2300 | -219 | -267 | -234 | C |
| ATOM | 171 | CD2 | LEU | 1142 | 5.505 | 34.132 | 13.787 | 1.00 | 17.38 | | C |
| ANISOU | 171 | CD2 | LEU | 1142 | 1703 | 2264 | 2636 | 65 | -670 | 38 | C |
| ATOM | 172 | C | LEU | 1142 | 9.364 | 35.716 | 15.044 | 1.00 | 12.80 | | C |
| ANISOU | 172 | C | LEU | 1142 | 1808 | 1398 | 1658 | 43 | -280 | 166 | C |
| ATOM | 173 | O | LEU | 1142 | 8.843 | 36.726 | 15.533 | 1.00 | 14.64 | | O |
| ANISOU | 173 | O | LEU | 1142 | 1903 | 1690 | 1969 | 281 | -204 | 125 | O |
| ATOM | 174 | N | LEU | 1143 | 10.254 | 35.782 | 14.051 | 1.00 | 12.49 | | N |
| ANISOU | 174 | N | LEU | 1143 | 1918 | 1334 | 1493 | -21 | -292 | 303 | N |
| ATOM | 175 | CA | LEU | 1143 | 10.535 | 37.074 | 13.436 | 1.00 | 12.91 | | C |
| ANISOU | 175 | CA | LEU | 1143 | 1908 | 1206 | 1791 | -28 | -328 | 257 | C |
| ATOM | 176 | CB | LEU | 1143 | 11.922 | 37.108 | 12.786 | 1.00 | 14.81 | | C |
| ANISOU | 176 | CB | LEU | 1143 | 1935 | 1567 | 2126 | -57 | -133 | 488 | C |
| ATOM | 177 | CG | LEU | 1143 | 13.059 | 36.879 | 13.779 | 1.00 | 16.06 | | C |
| ANISOU | 177 | CG | LEU | 1143 | 1959 | 1832 | 2311 | 94 | -247 | 147 | C |
| ATOM | 178 | CD1 | LEU | 1143 | 14.428 | 37.113 | 13.126 | 1.00 | 16.05 | | C |
| ANISOU | 178 | CD1 | LEU | 1143 | 1970 | 1619 | 2508 | -96 | -256 | 355 | C |
| ATOM | 179 | CD2 | LEU | 1143 | 12.893 | 37.771 | 14.994 | 1.00 | 23.19 | | C |
| ANISOU | 179 | CD2 | LEU | 1143 | 3172 | 3313 | 2326 | -89 | -85 | -462 | C |
| ATOM | 180 | C | LEU | 1143 | 9.503 | 37.363 | 12.373 | 1.00 | 14.41 | | C |
| ANISOU | 180 | C | LEU | 1143 | 2058 | 1382 | 2035 | -63 | -502 | 491 | C |
| ATOM | 181 | O | LEU | 1143 | 9.104 | 38.520 | 12.218 | 1.00 | 17.67 | | O |
| ANISOU | 181 | O | LEU | 1143 | 2912 | 1655 | 2147 | 453 | -717 | 292 | O |
| ATOM | 182 | N | GLY | 1144 | 9.050 | 36.383 | 11.613 | 1.00 | 14.23 | | N |
| ANISOU | 182 | N | GLY | 1144 | 2053 | 1709 | 1645 | -29 | -436 | 362 | N |
| ATOM | 183 | CA | GLY | 1144 | 7.930 | 36.619 | 10.702 | 1.00 | 17.26 | | C |
| ANISOU | 183 | CA | GLY | 1144 | 2812 | 1857 | 1889 | 472 | -929 | 148 | C |
| ATOM | 184 | C | GLY | 1144 | 7.844 | 35.456 | 9.715 | 1.00 | 16.18 | | C |
| ANISOU | 184 | C | GLY | 1144 | 2303 | 1670 | 2173 | 59 | -867 | 196 | C |
| ATOM | 185 | O | GLY | 1144 | 8.522 | 34.433 | 9.864 | 1.00 | 15.88 | | O |
| ANISOU | 185 | O | GLY | 1144 | 2444 | 1547 | 2044 | 38 | -588 | 343 | O |
| ATOM | 186 | N | ILE | 1145 | 6.995 | 35.653 | 8.705 | 1.00 | 16.06 | | N |
| ANISOU | 186 | N | ILE | 1145 | 1921 | 2244 | 1936 | -3 | -661 | 173 | N |
| ATOM | 187 | CA | ILE | 1145 | 6.720 | 34.638 | 7.698 | 1.00 | 15.81 | | C |
| ANISOU | 187 | CA | ILE | 1145 | 2276 | 1985 | 1746 | -42 | -547 | 358 | C |
| ATOM | 188 | CB | ILE | 1145 | 5.312 | 34.045 | 7.892 | 1.00 | 20.34 | | C |
| ANISOU | 188 | CB | ILE | 1145 | 2944 | 2930 | 1854 | -1027 | -298 | 154 | C |
| ATOM | 189 | CG2 | ILE | 1145 | 4.940 | 32.994 | 6.861 | 1.00 | 23.35 | | C |
| ANISOU | 189 | CG2 | ILE | 1145 | 3106 | 3362 | 2403 | -938 | -356 | -338 | C |
| ATOM | 190 | CG1 | ILE | 1145 | 5.147 | 33.476 | 9.311 | 1.00 | 20.25 | | C |
| ANISOU | 190 | CG1 | ILE | 1145 | 2579 | 3064 | 2050 | -570 | -490 | 588 | C |
| ATOM | 191 | CD1 | ILE | 1145 | 3.699 | 33.400 | 9.788 | 1.00 | 24.08 | | C |
| ANISOU | 191 | CD1 | ILE | 1145 | 3186 | 2686 | 3279 | -834 | 533 | 535 | C |
| ATOM | 192 | C | ILE | 1145 | 6.834 | 35.200 | 6.279 | 1.00 | 16.04 | | C |
| ANISOU | 192 | C | ILE | 1145 | 2383 | 1854 | 1857 | -311 | -530 | 397 | C |
| ATOM | 193 | O | ILE | 1145 | 6.263 | 36.254 | 6.005 | 1.00 | 19.29 | | O |
| ANISOU | 193 | O | ILE | 1145 | 3771 | 1665 | 1893 | -90 | -630 | 394 | O |
| ATOM | 194 | N | CYS | 1146 | 7.544 | 34.512 | 5.419 | 1.00 | 17.58 | | N |
| ANISOU | 194 | N | CYS | 1146 | 2340 | 2492 | 1847 | -124 | -369 | 467 | N |
| ATOM | 195 | CA | CYS | 1146 | 7.620 | 35.005 | 4.036 | 1.00 | 18.21 | | C |
| ANISOU | 195 | CA | CYS | 1146 | 2687 | 2460 | 1772 | 304 | -712 | 350 | C |
| ATOM | 196 | CB | CYS | 1146 | 9.073 | 35.113 | 3.618 | 1.00 | 21.06 | | C |
| ANISOU | 196 | CB | CYS | 1146 | 3163 | 2514 | 2326 | -294 | 190 | 365 | C |
| ATOM | 197 | SG | CYS | 1146 | 9.273 | 35.635 | 1.864 | 1.00 | 27.17 | | S |
| ANISOU | 197 | SG | CYS | 1146 | 3967 | 3707 | 2651 | 324 | 97 | 935 | S |
| ATOM | 198 | C | CYS | 1146 | 6.768 | 34.057 | 3.206 | 1.00 | 20.26 | | C |
| ANISOU | 198 | C | CYS | 1146 | 2772 | 2881 | 2044 | 296 | -581 | -36 | C |
| ATOM | 199 | O | CYS | 1146 | 7.037 | 32.847 | 3.101 | 1.00 | 21.86 | | O |
| ANISOU | 199 | O | CYS | 1146 | 3105 | 2863 | 2337 | 65 | -564 | -208 | O |
| ATOM | 200 | N | LEU | 1147 | 5.722 | 34.608 | 2.603 | 1.00 | 25.84 | | N |
| ANISOU | 200 | N | LEU | 1147 | 3324 | 3164 | 3329 | -256 | -1659 | 573 | N |
| ATOM | 201 | CA | LEU | 1147 | 4.730 | 33.851 | 1.836 | 1.00 | 30.79 | | C |
| ANISOU | 201 | CA | LEU | 1147 | 3619 | 4302 | 3778 | -789 | -1616 | -1 | C |
| ATOM | 202 | CB | LEU | 1147 | 3.320 | 34.416 | 2.066 | 1.00 | 33.11 | | C |
| ANISOU | 202 | CB | LEU | 1147 | 3544 | 4697 | 4339 | -498 | -2144 | 281 | C |
| ATOM | 203 | CG | LEU | 1147 | 2.882 | 34.388 | 3.540 | 1.00 | 33.72 | | C |
| ANISOU | 203 | CG | LEU | 1147 | 3081 | 4708 | 5023 | -1047 | -1079 | 565 | C |
| ATOM | 204 | CD1 | LEU | 1147 | 1.502 | 34.986 | 3.730 | 1.00 | 45.99 | | C |
| ANISOU | 204 | CD1 | LEU | 1147 | 3244 | 8470 | 5761 | 110 | -1226 | 1641 | C |
| ATOM | 205 | CD2 | LEU | 1147 | 2.918 | 32.963 | 4.072 | 1.00 | 44.69 | | C |
| ANISOU | 205 | CD2 | LEU | 1147 | 5782 | 5089 | 6109 | -1294 | -1077 | 1276 | C |
| ATOM | 206 | C | LEU | 1147 | 5.070 | 33.862 | 0.359 | 1.00 | 39.93 | | C |
| ANISOU | 206 | C | LEU | 1147 | 5100 | 6313 | 3757 | -1526 | -1509 | -554 | C |
| ATOM | 207 | O | LEU | 1147 | 4.554 | 33.091 | -0.466 | 1.00 | 47.45 | | O |
| ANISOU | 207 | O | LEU | 1147 | 6055 | 7604 | 4369 | -1710 | -1748 | -1357 | O |
| ATOM | 208 | N | ARG | 1148 | 5.981 | 34.747 | -0.059 | 1.00 | 46.27 | | N |
| ANISOU | 208 | N | ARG | 1148 | 6720 | 7485 | 3377 | -2429 | -1417 | 400 | N |
| ATOM | 209 | CA | ARG | 1148 | 6.602 | 34.494 | -1.363 | 1.00 | 56.44 | | C |
| ANISOU | 209 | CA | ARG | 1148 | 8079 | 8903 | 4463 | -2251 | -99 | -10 | C |
| ATOM | 210 | CB | ARG | 1148 | 7.113 | 35.762 | -2.013 | 1.00 | 57.07 | | C |
| ANISOU | 210 | CB | ARG | 1148 | 9237 | 9419 | 3027 | -2936 | -268 | -147 | C |
| ATOM | 211 | C | ARG | 1148 | 7.702 | 33.451 | -1.129 | 1.00 | 69.14 | | C |
| ANISOU | 211 | C | ARG | 1148 | 9984 | 10125 | 6161 | -508 | 503 | -86 | C |
| ATOM | 212 | O | ARG | 1148 | 7.644 | 32.849 | -0.011 | 1.00 | 77.51 | | O |
| ANISOU | 212 | O | ARG | 1148 | 9835 | 13187 | 6428 | 3652 | 2875 | 1106 | O |
| ATOM | 213 | OXT | ARG | 1148 | 8.578 | 33.240 | -2.008 | 1.00 | 68.86 | | O1- |
| ANISOU | 213 | OXT | ARG | 1148 | 11502 | 9299 | 5363 | 718 | 1004 | 1658 | O1- |
| ATOM | 1 | N | PRO | 1153 | 5.973 | 28.468 | 2.246 | 1.00 | 34.94 | | N |
| ANISOU | I | N | PRO | 1153 | 4567 | 4013 | 4697 | -1777 | -2050 | -152 | N |
| ATOM | 2 | CD | PRO | 1153 | 4.864 | 27.579 | 2.655 | 1.00 | 41.86 | | C |
| ANISOU | 2 | CD | PRO | 1153 | 4433 | 5580 | 5895 | -2041 | -1191 | -180 | C |
| ATOM | 3 | CA | PRO | 1153 | 6.143 | 29.558 | 3.217 | 1.00 | 36.18 | | C |
| ANISOU | 3 | CA | PRO | 1153 | 4538 | 5364 | 3844 | -1397 | -2079 | -548 | C |
| ATOM | 4 | CB | PRO | 1153 | 5.021 | 29.438 | 4.223 | 1.00 | 40.59 | | C |
| ANISOU | 4 | CB | PRO | 1153 | 3893 | 7055 | 4475 | -1319 | -2157 | -464 | C |
| ATOM | 5 | CG | PRO | 1153 | 4.143 | 28.343 | 3.725 | 1.00 | 45.43 | | C |
| ANISOU | 5 | CG | PRO | 1153 | 4877 | 6509 | 5875 | -1832 | -1322 | -630 | C |
| ATOM | 6 | C | PRO | 1153 | 7.491 | 29.345 | 3.908 | 1.00 | 28.05 | | C |
| ANISOU | 6 | C | PRO | 1153 | 4166 | 3634 | 2857 | -1103 | -1285 | -28 | C |
| ATOM | 7 | O | PRO | 1153 | 7.925 | 28.199 | 4.077 | 1.00 | 30.49 | | O |
| ANISOU | 7 | O | PRO | 1153 | 4729 | 3156 | 3699 | -1443 | -802 | -341 | O |
| ATOM | 8 | N | LEU | 1154 | 8.061 | 30.479 | 4.259 | 1.00 | 20.88 | | N |
| ANISOU | 8 | N | LEU | 1154 | 2824 | 2973 | 2136 | -184 | -824 | -56 | N |
| ATOM | 9 | CA | LEU | 1154 | 9.326 | 30.434 | 5.009 | 1.00 | 19.08 | | C |
| ANISOU | 9 | CA | LEU | 1154 | 2664 | 2951 | 1636 | -12 | -538 | -65 | C |
| ATOM | 10 | CB | LEU | 1154 | 10.435 | 31.205 | 4.303 | 1.00 | 19.26 | | C |
| ANISOU | 10 | CB | LEU | 1154 | 2677 | 2742 | 1899 | 18 | -225 | -280 | C |
| ATOM | 11 | CG | LEU | 1154 | 10.818 | 30.672 | 2.924 | 1.00 | 21.16 | | C |
| ANISOU | 11 | CG | LEU | 1154 | 3090 | 3024 | 1926 | 553 | -137 | -123 | C |
| ATOM | 12 | CD1 | LEU | 1154 | 11.825 | 31.632 | 2.312 | 1.00 | 23.75 | | C |
| ANISOU | 12 | CD1 | LEU | 1154 | 3584 | 3487 | 1953 | 250 | 220 | -195 | C |
| ATOM | 13 | CD2 | LEU | 1154 | 11.341 | 29.227 | 3.004 | 1.00 | 22.18 | | C |
| ANISOU | 13 | CD2 | LEU | 1154 | 2823 | 3068 | 2535 | 634 | -676 | -500 | C |
| ATOM | 14 | C | LEU | 1154 | 9.074 | 30.989 | 6.401 | 1.00 | 17.48 | | C |
| ANISOU | 14 | C | LEU | 1154 | 2736 | 2229 | 1677 | 218 | -410 | 139 | C |
| ATOM | 15 | O | LEU | 1154 | 8.455 | 32.050 | 6.535 | 1.00 | 22.55 | | O |
| ANISOU | 15 | O | LEU | 1154 | 3564 | 2757 | 2245 | 932 | -667 | 65 | O |
| ATOM | 16 | N | VAL | 1155 | 9.536 | 30.307 | 7.432 | 1.00 | 17.71 | | N |
| ANISOU | 16 | N | VAL | 1155 | 3125 | 1989 | 1613 | 54 | -530 | 73 | N |
| ATOM | 17 | CA | VAL | 1155 | 9.355 | 30.724 | 8.810 | 1.00 | 15.48 | | C |
| ANISOU | 17 | CA | VAL | 1155 | 2446 | 1774 | 1664 | -136 | -422 | 80 | C |
| ATOM | 18 | CB | VAL | 1155 | 8.958 | 29.541 | 9.706 | 1.00 | 15.52 | | C |
| ANISOU | 18 | CB | VAL | 1155 | 2399 | 1717 | 1781 | -38 | -402 | 136 | C |
| ATOM | 19 | CG1 | VAL | 1155 | 8.909 | 30.049 | 11.164 | 1.00 | 16.52 | | C |
| ANISOU | 19 | CG1 | VAL | 1155 | 2743 | 1823 | 1711 | -33 | -268 | 103 | C |
| ATOM | 20 | CG2 | VAL | 1155 | 7.645 | 28.890 | 9.320 | 1.00 | 22.78 | | C |
| ANISOU | 20 | CG2 | VAL | 1155 | 3543 | 2117 | 2996 | -1084 | -557 | -356 | C |
| ATOM | 21 | C | VAL | 1155 | 10.666 | 31.371 | 9.220 | 1.00 | 15.43 | | C |
| ANISOU | 21 | C | VAL | 1155 | 2364 | 1757 | 1743 | -122 | -75 | -99 | C |
| ATOM | 22 | O | VAL | 1155 | 11.711 | 30.709 | 9.235 | 1.00 | 18.49 | | O |
| ANISOU | 22 | O | VAL | 1155 | 2407 | 1769 | 2851 | -157 | -467 | 172 | O |
| ATOM | 23 | N | VAL | 1156 | 10.651 | 32.656 | 9.547 | 1.00 | 15.66 | | N |
| ANISOU | 23 | N | VAL | 1156 | 2505 | 1702 | 1742 | -219 | -296 | 23 | N |
| ATOM | 24 | CA | VAL | 1156 | 11.884 | 33.357 | 9.865 | 1.00 | 13.62 | | C |
| ANISOU | 24 | CA | VAL | 1156 | 2240 | 1435 | 1500 | 89 | -419 | 311 | C |
| ATOM | 25 | CB | VAL | 1156 | 11.903 | 34.745 | 9.184 | 1.00 | 15.37 | | C |
| ANISOU | 25 | CB | VAL | 1156 | 2356 | 1699 | 1784 | -88 | -468 | 650 | C |
| ATOM | 26 | CG1 | VAL | 1156 | 13.225 | 35.448 | 9.396 | 1.00 | 15.89 | | C |
| ANISOU | 26 | CG1 | VAL | 1156 | 2062 | 2105 | 1871 | -149 | 85 | 445 | C |
| ATOM | 27 | CG2 | VAL | 1156 | 11.596 | 34.594 | 7.681 | 1.00 | 16.61 | | C |
| ANISOU | 27 | CG2 | VAL | 1156 | 2475 | 2235 | 1602 | 292 | -233 | 560 | C |
| ATOM | 28 | C | VAL | 1156 | 12.026 | 33.452 | 11.385 | 1.00 | 12.70 | | C |
| ANISOU | 28 | C | VAL | 1156 | 1899 | 1440 | 1487 | 40 | -207 | 304 | C |
| ATOM | 29 | O | VAL | 1156 | 11.123 | 33.958 | 12.052 | 1.00 | 12.84 | | O |
| ANISOU | 29 | O | VAL | 1156 | 1724 | 1388 | 1768 | -118 | -252 | 42 | O |
| ATOM | 30 | N | LEU | 1157 | 13.172 | 32.954 | 11.869 | 1.00 | 12.85 | | N |
| ANISOU | 30 | N | LEU | 1157 | 1910 | 1461 | 1509 | 95 | -313 | 233 | N |
| ATOM | 31 | CA | LEU | 1157 | 13.436 | 32.801 | 13.289 | 1.00 | 12.63 | | C |
| ANISOU | 31 | CA | LEU | 1157 | 1992 | 1162 | 1644 | -46 | -403 | 410 | C |
| ATOM | 32 | CB | LEU | 1157 | 13.516 | 31.299 | 13.647 | 1.00 | 12.41 | | C |
| ANISOU | 32 | CB | LEU | 1157 | 1722 | 1055 | 1941 | 84 | -129 | 225 | C |
| ATOM | 33 | CG | LEU | 1157 | 12.268 | 30.492 | 13.208 | 1.00 | 12.51 | | C |
| ANISOU | 33 | CG | LEU | 1157 | 1720 | 1128 | 1904 | 58 | -126 | 172 | C |
| ATOM | 34 | CD1 | LEU | 1157 | 12.511 | 29.008 | 13.207 | 1.00 | 13.62 | | C |
| ANISOU | 34 | CD1 | LEU | 1157 | 2202 | 1119 | 1855 | -17 | -163 | 54 | C |
| ATOM | 35 | CD2 | LEU | 1157 | 11.119 | 30.833 | 14.128 | 1.00 | 14.35 | | C |
| ANISOU | 35 | CD2 | LEU | 1157 | 1857 | 1272 | 2321 | 14 | 190 | 284 | C |
| ATOM | 36 | C | LEU | 1157 | 14.726 | 33.483 | 13.666 | 1.00 | 11.30 | | C |
| ANISOU | 36 | C | LEU | 1157 | 1816 | 1005 | 1472 | 88 | -59 | -65 | C |
| ATOM | 37 | O | LEU | 1157 | 15.591 | 33.748 | 12.807 | 1.00 | 12.72 | | O |
| ANISOU | 37 | O | LEU | 1157 | 1963 | 1305 | 1564 | 178 | 90 | 155 | O |
| ATOM | 38 | N | PRO | 1158 | 14.917 | 33.785 | 14.953 | 1.00 | 11.95 | | N |
| ANISOU | 38 | N | PRO | 1158 | 1820 | 1283 | 1438 | 120 | -117 | 91 | N |
| ATOM | 39 | CD | PRO | 1158 | 13.969 | 33.659 | 16.068 | 1.00 | 15.87 | | C |
| ANISOU | 39 | CD | PRO | 1158 | 2092 | 2392 | 1546 | -278 | 112 | -318 | C |
| ATOM | 40 | CA | PRO | 1158 | 16.224 | 34.332 | 15.381 | 1.00 | 12.73 | | C |
| ANISOU | 40 | CA | PRO | 1158 | 1756 | 1653 | 1430 | 203 | -105 | -177 | C |
| ATOM | 41 | CB | PRO | 1158 | 16.063 | 34.517 | 16.890 | 1.00 | 14.09 | | C |
| ANISOU | 41 | CB | PRO | 1158 | 1998 | 1899 | 1458 | 223 | -77 | -288 | C |
| ATOM | 42 | CG | PRO | 1158 | 14.633 | 34.462 | 17.157 | 1.00 | 18.50 | | C |
| ANISOU | 42 | CG | PRO | 1158 | 2273 | 2913 | 1843 | -931 | 405 | -660 | C |
| ATOM | 43 | C | PRO | 1158 | 17.366 | 33.366 | 15.098 | 1.00 | 11.34 | | C |
| ANISOU | 43 | C | PRO | 1158 | 1711 | 1250 | 1345 | -43 | 90 | 135 | C |
| ATOM | 44 | O | PRO | 1158 | 17.220 | 32.136 | 15.195 | 1.00 | 12.10 | | O |
| ANISOU | 44 | O | PRO | 1158 | 1884 | 1177 | 1537 | -105 | 63 | 213 | O |
| ATOM | 45 | N | TYR | 1159 | 18.502 | 33.964 | 14.747 | 1.00 | 11.59 | | N |
| ANISOU | 45 | N | TYR | 1159 | 1682 | 1257 | 1466 | -61 | 9 | 286 | N |
| ATOM | 46 | CA | TYR | 1159 | 19.734 | 33.223 | 14.588 | 1.00 | 11.92 | | C |
| ANISOU | 46 | CA | TYR | 1159 | 1614 | 1324 | 1592 | -82 | 12 | 340 | C |
| ATOM | 47 | CB | TYR | 1159 | 20.765 | 34.024 | 13.788 | 1.00 | 14.71 | | C |
| ANISOU | 47 | CB | TYR | 1159 | 2023 | 1648 | 1917 | 22 | 383 | 704 | C |
| ATOM | 48 | CG | TYR | 1159 | 22.000 | 33.192 | 13.525 | 1.00 | 14.40 | | C |
| ANISOU | 48 | CG | TYR | 1159 | 1930 | 1618 | 1922 | -126 | 392 | 544 | C |
| ATOM | 49 | CD1 | TYR | 1159 | 21.918 | 32.121 | 12.625 | 1.00 | 16.77 | | C |
| ANISOU | 49 | CD1 | TYR | 1159 | 2279 | 1981 | 2112 | -142 | 587 | 238 | C |
| ATOM | 50 | CE1 | TYR | 1159 | 23.050 | 31.339 | 12.372 | 1.00 | 19.27 | | C |
| ANISOU | 50 | CE1 | TYR | 1159 | 2504 | 2370 | 2450 | -3 | 924 | 75 | C |
| ATOM | 51 | CD2 | TYR | 1159 | 23.200 | 33.460 | 14.154 | 1.00 | 16.12 | | C |
| ANISOU | 51 | CD2 | TYR | 1159 | 1858 | 2234 | 2030 | -168 | 429 | 461 | C |
| ATOM | 52 | CE2 | TYR | 1159 | 24.322 | 32.688 | 13.903 | 1.00 | 18.57 | | C |
| ANISOU | 52 | CE2 | TYR | 1159 | 1780 | 3012 | 2264 | -79 | 754 | 364 | C |
| ATOM | 53 | CZ | TYR | 1159 | 24.232 | 31.641 | 13.017 | 1.00 | 19.08 | | C |
| ANISOU | 53 | CZ | TYR | 1159 | 2265 | 2712 | 2275 | 208 | 946 | 527 | C |
| ATOM | 54 | OH | TYR | 1159 | 25.371 | 30.872 | 12.776 | 1.00 | 25.18 | | O |
| ANISOU | 54 | OH | TYR | 1159 | 2490 | 3626 | 3451 | 495 | 1329 | 209 | O |
| ATOM | 55 | C | TYR | 1159 | 20.310 | 32.885 | 15.961 | 1.00 | 11.18 | | C |
| ANISOU | 55 | C | TYR | 1159 | 1564 | 1226 | 1457 | -132 | 29 | 189 | C |
| ATOM | 56 | O | TYR | 1159 | 20.416 | 33.766 | 16.835 | 1.00 | 14.01 | | O |
| ANISOU | 56 | O | TYR | 1159 | 2319 | 1255 | 1749 | -305 | 36 | 37 | O |
| ATOM | 57 | N | AMET | 1160 | 20.661 | 31.631 | 16.167 | 0.50 | 11.05 | | N |
| ANISOU | 57 | N | AMET | 1160 | 1505 | 1227 | 1466 | -184 | -191 | 154 | N |
| ATOM | 58 | N | BMET | 1160 | 20.662 | 31.621 | 16.088 | 0.50 | 10.59 | | N |
| ANISOU | 58 | N | BMET | 1160 | 1504 | 1165 | 1354 | -274 | 151 | 309 | N |
| ATOM | 59 | CA | AMET | 1160 | 21.137 | 31.112 | 17.455 | 0.50 | 11.05 | | C |
| ANISOU | 59 | CA | AMET | 1160 | 1377 | 1378 | 1444 | -252 | 42 | 334 | C |
| ATOM | 60 | CA | BMET | 1160 | 21.149 | 30.985 | 17.308 | 0.50 | 10.92 | | C |
| ANISOU | 60 | CA | BMET | 1160 | 1472 | 1169 | 1509 | -344 | -52 | 265 | C |
| ATOM | 61 | CB | AMET | 1160 | 20.178 | 30.071 | 18.030 | 0.50 | 10.42 | | C |
| ANISOU | 61 | CB | AMET | 1160 | 1296 | 1002 | 1663 | -239 | -6 | 80 | C |
| ATOM | 62 | CB | BMET | 1160 | 20.243 | 29.812 | 17.689 | 0.50 | 10.00 | | C |
| ANISOU | 62 | CB | BMET | 1160 | 1379 | 986 | 1435 | -219 | 7 | 279 | C |
| ATOM | 63 | CG | AMET | 1160 | 18.738 | 30.563 | 18.173 | 0.50 | 11.25 | | C |
| ANISOU | 63 | CG | AMET | 1160 | 1267 | 1367 | 1640 | -213 | 56 | 106 | C |
| ATOM | 64 | CG | BMET | 1160 | 18.822 | 30.248 | 18.059 | 0.50 | 9.96 | | C |
| ANISOU | 64 | CG | BMET | 1160 | 1392 | 1044 | 1350 | -125 | -10 | 312 | C |
| ATOM | 65 | SD | AMET | 1160 | 18.509 | 31.858 | 19.425 | 0.50 | 12.21 | | S |
| ANISOU | 65 | SD | AMET | 1160 | 1407 | 1589 | 1645 | 4 | 40 | 22 | S |
| ATOM | 66 | SD | BMET | 1160 | 18.767 | 31.013 | 19.708 | 0.50 | 10.37 | | S |
| ANISOU | 66 | SD | BMET | 1160 | 1365 | 1141 | 1434 | -125 | -55 | 218 | S |
| ATOM | 67 | CE | AMET | 1160 | 18.488 | 30.897 | 20.925 | 0.50 | 14.21 | | C |
| ANISOU | 67 | CE | AMET | 1160 | 2250 | 1461 | 1686 | 36 | 250 | 49 | C |
| ATOM | 68 | CE | BMET | 1160 | 18.006 | 32.605 | 19.307 | 0.50 | 11.82 | | C |
| ANISOU | 68 | CE | BMET | 1160 | 1717 | 1316 | 1459 | 162 | -144 | 129 | C |
| ATOM | 69 | C | AMET | 1160 | 22.534 | 30.577 | 17.202 | 0.50 | 10.25 | | C |
| ANISOU | 69 | C | AMET | 1160 | 1388 | 1263 | 1245 | -232 | 40 | 331 | C |
| ATOM | 70 | C | BMET | 1160 | 22.583 | 30.561 | 17.081 | 0.50 | 11.37 | | C |
| ANISOU | 70 | C | BMET | 1160 | 1472 | 1298 | 1551 | -311 | 101 | 321 | C |
| ATOM | 71 | O | AMET | 1160 | 22.707 | 29.436 | 16.752 | 0.50 | 11.53 | | O |
| ANISOU | 71 | O | AMET | 1160 | 1688 | 1307 | 1386 | -182 | 31 | 238 | O |
| ATOM | 72 | O | BMET | 1160 | 22.833 | 29.475 | 16.539 | 0.50 | 12.74 | | O |
| ANISOU | 72 | O | BMET | 1160 | 1714 | 1477 | 1648 | -283 | 266 | 111 | O |
| ATOM | 73 | N | LYS | 1161 | 23.533 | 31.411 | 17.482 | 1.00 | 12.71 | | N |
| ANISOU | 73 | N | LYS | 1161 | 1418 | 1380 | 2031 | -379 | 389 | 166 | N |
| ATOM | 74 | CA | LYS | 1161 | 24.930 | 31.154 | 17.093 | 1.00 | 14.68 | | C |
| ANISOU | 74 | CA | LYS | 1161 | 1441 | 1604 | 2535 | -322 | 498 | 227 | C |
| ATOM | 75 | CB | LYS | 1161 | 25.825 | 32.258 | 17.695 | 1.00 | 17.98 | | C |
| ANISOU | 75 | CB | LYS | 1161 | 1617 | 1957 | 3256 | -659 | 509 | 83 | C |
| ATOM | 76 | CG | LYS | 1161 | 27.322 | 32.073 | 17.511 | 1.00 | 23.11 | | C |
| ANISOU | 76 | CG | LYS | 1161 | 1689 | 3236 | 3856 | -1082 | 974 | -407 | C |
| ATOM | 77 | CD | LYS | 1161 | 28.078 | 33.235 | 18.154 | 1.00 | 26.09 | | C |
| ANISOU | 77 | CD | LYS | 1161 | 2294 | 3233 | 4387 | -1786 | 608 | 239 | C |
| ATOM | 78 | CE | LYS | 1161 | 29.563 | 32.987 | 18.274 | 1.00 | 33.81 | | C |
| ANISOU | 78 | CE | LYS | 1161 | 2423 | 4184 | 6239 | -1770 | -318 | 779 | C |
| ATOM | 79 | NZ | LYS | 1161 | 30.275 | 33.924 | 19.209 | 1.00 | 47.25 | | N1+ |
| ANISOU | 79 | NZ | LYS | 1161 | 2553 | 6700 | 8699 | -1608 | -615 | -1584 | N1+ |
| ATOM | 80 | C | LYS | 1161 | 25.450 | 29.797 | 17.500 | 1.00 | 14.28 | | C |
| ANISOU | 80 | C | LYS | 1161 | 1466 | 1806 | 2154 | -120 | 423 | 207 | C |
| ATOM | 81 | O | LYS | 1161 | 26.236 | 29.180 | 16.741 | 1.00 | 14.87 | | O |
| ANISOU | 81 | O | LYS | 1161 | 1571 | 1960 | 2118 | -69 | 370 | 162 | O |
| ATOM | 82 | N | HIS | 1162 | 25.070 | 29.285 | 18.674 | 1.00 | 12.76 | | N |
| ANISOU | 82 | N | HIS | 1162 | 1296 | 1588 | 1965 | -303 | 166 | -19 | N |
| ATOM | 83 | CA | HIS | 1162 | 25.648 | 28.044 | 19.164 | 1.00 | 12.90 | | C |
| ANISOU | 83 | CA | HIS | 1162 | 1220 | 1832 | 1848 | -200 | -92 | 50 | C |
| ATOM | 84 | CB | HIS | 1162 | 25.962 | 28.163 | 20.682 | 1.00 | 14.67 | | C |
| ANISOU | 84 | CB | HIS | 1162 | 1073 | 2652 | 1849 | -227 | -91 | -166 | C |
| ATOM | 85 | CG | HIS | 1162 | 27.043 | 29.176 | 20.880 | 1.00 | 16.06 | | C |
| ANISOU | 85 | CG | HIS | 1162 | 1125 | 2543 | 2434 | -163 | -219 | -385 | C |
| ATOM | 86 | CD2 | HIS | 1162 | 27.067 | 30.393 | 21.432 | 1.00 | 19.23 | | C |
| ANISOU | 86 | CD2 | HIS | 1162 | 1722 | 3221 | 2365 | -337 | -316 | -1044 | C |
| ATOM | 87 | ND1 | HIS | 1162 | 28.333 | 28.909 | 20.414 | 1.00 | 18.56 | | N |
| ANISOU | 87 | ND1 | HIS | 1162 | 1033 | 2552 | 3467 | -128 | -199 | -259 | N |
| ATOM | 88 | CE1 | HIS | 1162 | 29.094 | 29.953 | 20.698 | 1.00 | 21.11 | | C |
| ANISOU | 88 | CE1 | HIS | 1162 | 1453 | 3128 | 3440 | -580 | -243 | -339 | C |
| ATOM | 89 | NE2 | HIS | 1162 | 28.373 | 30.873 | 21.317 | 1.00 | 22.12 | | N |
| ANISOU | 89 | NE2 | HIS | 1162 | 1972 | 3233 | 3200 | -704 | -458 | -931 | N |
| ATOM | 90 | C | HIS | 1162 | 24.774 | 26.825 | 18.896 | 1.00 | 11.89 | | C |
| ANISOU | 90 | C | HIS | 1162 | 1121 | 1648 | 1751 | -189 | 25 | 252 | C |
| ATOM | 91 | O | HIS | 1162 | 25.092 | 25.750 | 19.399 | 1.00 | 14.32 | | O |
| ANISOU | 91 | O | HIS | 1162 | 1257 | 1797 | 2388 | 8 | 68 | 460 | O |
| ATOM | 92 | N | GLY | 1163 | 23.701 | 27.010 | 18.121 | 1.00 | 10.70 | | N |
| ANISOU | 92 | N | GLY | 1163 | 1060 | 1502 | 1503 | -129 | 124 | 58 | N |
| ATOM | 93 | CA | GLY | 1163 | 22.905 | 25.849 | 17.704 | 1.00 | 10.95 | | C |
| ANISOU | 93 | CA | GLY | 1163 | 1332 | 1383 | 1447 | -200 | 108 | 157 | C |
| ATOM | 94 | C | GLY | 1163 | 22.146 | 25.221 | 18.855 | 1.00 | 9.41 | | C |
| ANISOU | 94 | C | GLY | 1163 | 1123 | 1129 | 1323 | -45 | -29 | 69 | C |
| ATOM | 95 | O | GLY | 1163 | 21.821 | 25.844 | 19.866 | 1.00 | 10.12 | | O |
| ANISOU | 95 | O | GLY | 1163 | 1212 | 1259 | 1375 | -19 | 29 | -80 | O |
| ATOM | 96 | N | ASP | 1164 | 21.855 | 23.917 | 18.669 | 1.00 | 9.68 | | N |
| ANISOU | 96 | N | ASP | 1164 | 1259 | 1011 | 1407 | 133 | -71 | 167 | N |
| ATOM | 97 | CA | ASP | 1164 | 20.992 | 23.250 | 19.652 | 1.00 | 9.15 | | C |
| ANISOU | 97 | CA | ASP | 1164 | 1083 | 1168 | 1225 | -136 | -146 | 62 | C |
| ATOM | 98 | CB | ASP | 1164 | 20.319 | 21.986 | 19.073 | 1.00 | 10.27 | | C |
| ANISOU | 98 | CB | ASP | 1164 | 1166 | 995 | 1741 | 85 | -264 | -56 | C |
| ATOM | 99 | CG | ASP | 1164 | 21.269 | 20.792 | 18.966 | 1.00 | 10.32 | | C |
| ANISOU | 99 | CG | ASP | 1164 | 1268 | 1178 | 1476 | 218 | 11 | 154 | C |
| ATOM | 100 | OD1 | ASP | 1164 | 21.970 | 20.638 | 17.934 | 1.00 | 13.19 | | O |
| ANISOU | 100 | OD1 | ASP | 1164 | 1717 | 1792 | 1502 | 408 | 60 | -13 | O |
| ATOM | 101 | OD2 | ASP | 1164 | 21.342 | 19.997 | 19.924 | 1.00 | 10.33 | | O1- |
| ANISOU | 101 | OD2 | ASP | 1164 | 1262 | 1139 | 1524 | 57 | -272 | 162 | O1- |
| ATOM | 102 | C | ASP | 1164 | 21.805 | 22.949 | 20.920 | 1.00 | 8.82 | | C |
| ANISOU | 102 | C | ASP | 1164 | 884 | 1106 | 1363 | 177 | -116 | 148 | C |
| ATOM | 103 | O | ASP | 1164 | 23.008 | 22.721 | 20.904 | 1.00 | 9.29 | | O |
| ANISOU | 103 | O | ASP | 1164 | 923 | 1108 | 1498 | 120 | 5 | 50 | O |
| ATOM | 104 | N | LEU | 1165 | 21.084 | 22.957 | 22.052 | 1.00 | 8.75 | | N |
| ANISOU | 104 | N | LEU | 1165 | 1098 | 1030 | 1199 | 58 | -77 | 17 | N |
| ATOM | 105 | CA | LEU | 1165 | 21.728 | 22.781 | 23.363 | 1.00 | 8.89 | | C |
| ANISOU | 105 | CA | LEU | 1165 | 1025 | 1051 | 1300 | 120 | -20 | 20 | C |
| ATOM | 106 | CB | LEU | 1165 | 20.617 | 22.986 | 24.418 | 1.00 | 9.14 | | C |
| ANISOU | 106 | CB | LEU | 1165 | 883 | 1367 | 1225 | 5 | -72 | -115 | C |
| ATOM | 107 | CG | LEU | 1165 | 21.060 | 22.907 | 25.895 | 1.00 | 9.61 | | C |
| ANISOU | 107 | CG | LEU | 1165 | 894 | 1481 | 1277 | 114 | -116 | -81 | C |
| ATOM | 108 | CD1 | LEU | 1165 | 22.088 | 23.964 | 26.240 | 1.00 | 12.03 | | C |
| ANISOU | 108 | CD1 | LEU | 1165 | 1092 | 1704 | 1775 | 74 | -451 | -382 | C |
| ATOM | 109 | CD2 | LEU | 1165 | 19.823 | 23.039 | 26.760 | 1.00 | 11.09 | | C |
| ANISOU | 109 | CD2 | LEU | 1165 | 1129 | 1788 | 1296 | 317 | -1 | -217 | C |
| ATOM | 110 | C | LEU | 1165 | 22.400 | 21.460 | 23.515 | 1.00 | 8.39 | | C |
| ANISOU | 110 | C | LEU | 1165 | 805 | 954 | 1428 | -71 | 12 | 185 | C |
| ATOM | 111 | O | LEU | 1165 | 23.451 | 21.383 | 24.165 | 1.00 | 9.20 | | O |
| ANISOU | 111 | O | LEU | 1165 | 849 | 1243 | 1402 | 16 | -22 | 3 | O |
| ATOM | 112 | N | ARG | 1166 | 21.845 | 20.378 | 22.945 | 1.00 | 8.83 | | N |
| ANISOU | 112 | N | ARG | 1166 | 884 | 1003 | 1469 | 61 | 51 | -25 | N |
| ATOM | 113 | CA | ARG | 1166 | 22.511 | 19.078 | 23.102 | 1.00 | 9.27 | | C |
| ANISOU | 113 | CA | ARG | 1166 | 838 | 1032 | 1653 | 71 | 104 | -33 | C |
| ATOM | 114 | CB | ARG | 1166 | 21.625 | 17.938 | 22.575 | 1.00 | 8.73 | | C |
| ANISOU | 114 | CB | ARG | 1166 | 955 | 1020 | 1341 | 35 | -76 | -4 | C |
| ATOM | 115 | CG | ARG | 1166 | 22.229 | 16.601 | 22.998 | 1.00 | 10.57 | | C |
| ANISOU | 115 | CG | ARG | 1166 | 1559 | 1031 | 1428 | 163 | -345 | -67 | C |
| ATOM | 116 | CD | ARG | 1166 | 21.594 | 15.428 | 22.256 | 1.00 | 10.93 | | C |
| ANISOU | 116 | CD | ARG | 1166 | 1479 | 1015 | 1658 | 13 | -208 | -43 | C |
| ATOM | 117 | NE | ARG | 1166 | 22.235 | 14.192 | 22.683 | 1.00 | 10.73 | | N |
| ANISOU | 117 | NE | ARG | 1166 | 1545 | 1013 | 1518 | 124 | 17 | -6 | N |
| ATOM | 118 | CZ | ARG | 1166 | 22.522 | 13.160 | 21.904 | 1.00 | 10.46 | | C |
| ANISOU | 118 | CZ | ARG | 1166 | 1331 | 1187 | 1458 | 109 | -1 | -54 | C |
| ATOM | 119 | NH1 | ARG | 1166 | 22.242 | 13.138 | 20.597 | 1.00 | 12.64 | | N1+ |
| ANISOU | 119 | NH1 | ARG | 1166 | 1796 | 1565 | 1442 | 344 | -97 | -74 | N1+ |
| ATOM | 120 | NH2 | ARG | 1166 | 23.110 | 12.113 | 22.493 | 1.00 | 11.73 | | N |
| ANISOU | 120 | NH2 | ARG | 1166 | 1497 | 1291 | 1667 | 378 | -258 | -154 | N |
| ATOM | 121 | C | ARG | 1166 | 23.857 | 19.056 | 22.395 | 1.00 | 8.56 | | C |
| ANISOU | 121 | C | ARG | 1166 | 850 | 965 | 1439 | 128 | 14 | 102 | C |
| ATOM | 122 | O | ARG | 1166 | 24.874 | 18.672 | 22.971 | 1.00 | 9.40 | | O |
| ANISOU | 122 | O | ARG | 1166 | 903 | 1166 | 1504 | 144 | -49 | 66 | O |
| ATOM | 123 | N | ASN | 1167 | 23.889 | 19.474 | 21.116 | 1.00 | 9.82 | | N |
| ANISOU | 123 | N | ASN | 1167 | 1065 | 1280 | 1385 | 37 | 38 | 48 | N |
| ATOM | 124 | CA | ASN | 1167 | 25.197 | 19.469 | 20.428 | 1.00 | 10.15 | | C |
| ANISOU | 124 | CA | ASN | 1167 | 1038 | 1443 | 1376 | -22 | 66 | 41 | C |
| ATOM | 125 | CB | ASN | 1167 | 25.028 | 19.756 | 18.952 | 1.00 | 12.52 | | C |
| ANISOU | 125 | CB | ASN | 1167 | 1780 | 1677 | 1300 | -39 | 81 | 47 | C |
| ATOM | 126 | CG | ASN | 1167 | 24.663 | 18.496 | 18.194 | 1.00 | 15.95 | | C |
| ANISOU | 126 | CG | ASN | 1167 | 2272 | 1979 | 1809 | 391 | -350 | -454 | C |
| ATOM | 127 | OD1 | ASN | 1167 | 25.311 | 17.444 | 18.360 | 1.00 | 19.03 | | O |
| ANISOU | 127 | OD1 | ASN | 1167 | 2346 | 2692 | 2193 | -270 | -527 | -131 | O |
| ATOM | 128 | ND2 | ASN | 1167 | 23.652 | 18.531 | 17.383 | 1.00 | 18.84 | | N |
| ANISOU | 128 | ND2 | ASN | 1167 | 3193 | 2002 | 1964 | 722 | -108 | -370 | N |
| ATOM | 129 | C | ASN | 1167 | 26.128 | 20.468 | 21.105 | 1.00 | 9.92 | | C |
| ANISOU | 129 | C | ASN | 1167 | 1012 | 1251 | 1506 | 76 | -71 | 134 | C |
| ATOM | 130 | O | ASN | 1167 | 27.344 | 20.222 | 21.086 | 1.00 | 10.86 | | O |
| ANISOU | 130 | O | ASN | 1167 | 1007 | 1518 | 1603 | 56 | 76 | 116 | O |
| ATOM | 131 | N | PHE | 1168 | 25.640 | 21.552 | 21.688 | 1.00 | 9.56 | | N |
| ANISOU | 131 | N | PHE | 1168 | 949 | 1363 | 1321 | -7 | -126 | 60 | N |
| ATOM | 132 | CA | PHE | 1168 | 26.523 | 22.491 | 22.353 | 1.00 | 9.90 | | C |
| ANISOU | 132 | CA | PHE | 1168 | 1107 | 1206 | 1447 | -39 | -122 | 176 | C |
| ATOM | 133 | CB | PHE | 1168 | 25.751 | 23.738 | 22.820 | 1.00 | 10.48 | | C |
| ANISOU | 133 | CB | PHE | 1168 | 1108 | 1169 | 1705 | 46 | -105 | 124 | C |
| ATOM | 134 | CG | PHE | 1168 | 26.692 | 24.705 | 23.545 | 1.00 | 11.24 | | C |
| ANISOU | 134 | CG | PHE | 1168 | 1305 | 1106 | 1858 | 37 | -183 | 211 | C |
| ATOM | 135 | CD1 | PHE | 1168 | 27.481 | 25.549 | 22.794 | 1.00 | 14.47 | | C |
| ANISOU | 135 | CD1 | PHE | 1168 | 1773 | 1238 | 2488 | -358 | 226 | 42 | C |
| ATOM | 136 | CD2 | PHE | 1168 | 26.774 | 24.756 | 24.953 | 1.00 | 11.95 | | C |
| ANISOU | 136 | CD2 | PHE | 1168 | 1300 | 1357 | 1884 | 260 | -362 | -80 | C |
| ATOM | 137 | CE1 | PHE | 1168 | 28.340 | 26.413 | 23.454 | 1.00 | 17.10 | | C |
| ANISOU | 137 | CE1 | PHE | 1168 | 2233 | 1871 | 2392 | -771 | 17 | 148 | C |
| ATOM | 138 | CE2 | PHE | 1168 | 27.635 | 25.622 | 25.602 | 1.00 | 12.86 | | C |
| ANISOU | 138 | CE2 | PHE | 1168 | 1346 | 1497 | 2042 | 26 | -342 | 92 | C |
| ATOM | 139 | CZ | PHE | 1168 | 28.416 | 26.462 | 24.827 | 1.00 | 17.13 | | C |
| ANISOU | 139 | CZ | PHE | 1168 | 1938 | 2149 | 2422 | -438 | -180 | 287 | C |
| ATOM | 140 | C | PHE | 1168 | 27.228 | 21.840 | 23.532 | 1.00 | 9.35 | | C |
| ANISOU | 140 | C | PHE | 1168 | 842 | 1194 | 1518 | -134 | -163 | 201 | C |
| ATOM | 141 | O | PHE | 1168 | 28.452 | 21.915 | 23.677 | 1.00 | 10.84 | | O |
| ANISOU | 141 | O | PHE | 1168 | 833 | 1534 | 1751 | -115 | -55 | 159 | O |
| ATOM | 142 | N | ILE | 1169 | 26.460 | 21.157 | 24.415 | 1.00 | 9.26 | | N |
| ANISOU | 142 | N | ILE | 1169 | 895 | 1178 | 1445 | 66 | 10 | 158 | N |
| ATOM | 143 | CA | ILE | 1169 | 27.087 | 20.594 | 25.606 | 1.00 | 9.31 | | C |
| ANISOU | 143 | CA | ILE | 1169 | 1044 | 1226 | 1269 | 114 | 4 | 56 | C |
| ATOM | 144 | CB | ILE | 1169 | 26.058 | 20.237 | 26.671 | 1.00 | 8.97 | | C |
| ANISOU | 144 | CB | ILE | 1169 | 918 | 1098 | 1391 | 81 | -1 | -18 | C |
| ATOM | 145 | CG2 | ILE | 1169 | 25.344 | 21.514 | 27.158 | 1.00 | 10.44 | | C |
| ANISOU | 145 | CG2 | ILE | 1169 | 1252 | 1243 | 1470 | 311 | -108 | -186 | C |
| ATOM | 146 | CG1 | ILE | 1169 | 25.073 | 19.144 | 26.241 | 1.00 | 9.61 | | C |
| ANISOU | 146 | CG1 | ILE | 1169 | 973 | 1192 | 1488 | 12 | 37 | -55 | C |
| ATOM | 147 | CD1 | ILE | 1169 | 23.942 | 18.876 | 27.210 | 1.00 | 9.89 | | C |
| ANISOU | 147 | CD1 | ILE | 1169 | 951 | 1125 | 1681 | 170 | 219 | 250 | C |
| ATOM | 148 | C | ILE | 1169 | 27.933 | 19.370 | 25.228 | 1.00 | 8.58 | | C |
| ANISOU | 148 | C | ILE | 1169 | 876 | 1235 | 1151 | 64 | 36 | 125 | C |
| ATOM | 149 | O | ILE | 1169 | 28.808 | 18.986 | 26.005 | 1.00 | 9.69 | | O |
| ANISOU | 149 | O | ILE | 1169 | 899 | 1301 | 1480 | 46 | -152 | 83 | O |
| ATOM | 150 | N | ARG | 1170 | 27.722 | 18.752 | 24.067 | 1.00 | 9.58 | | N |
| ANISOU | 150 | N | ARG | 1170 | 1083 | 1244 | 1311 | 205 | -15 | -23 | N |
| ATOM | 151 | CA | ARG | 1170 | 28.502 | 17.609 | 23.589 | 1.00 | 10.03 | | C |
| ANISOU | 151 | CA | ARG | 1170 | 873 | 1465 | 1475 | 214 | -2 | -130 | C |
| ATOM | 152 | CB | ARG | 1170 | 27.745 | 16.759 | 22.556 | 1.00 | 10.30 | | C |
| ANISOU | 152 | CB | ARG | 1170 | 1284 | 1118 | 1510 | -31 | 6 | 11 | C |
| ATOM | 153 | CG | ARG | 1170 | 26.623 | 15.941 | 23.241 | 1.00 | 10.67 | | C |
| ANISOU | 153 | CG | ARG | 1170 | 1187 | 1432 | 1434 | 34 | 7 | 166 | C |
| ATOM | 154 | CD | ARG | 1170 | 25.835 | 15.188 | 22.205 | 1.00 | 12.14 | | C |
| ANISOU | 154 | CD | ARG | 1170 | 1121 | 1575 | 1917 | -124 | 157 | -198 | C |
| ATOM | 155 | NE | ARG | 1170 | 26.631 | 14.213 | 21.488 | 1.00 | 14.53 | | N |
| ANISOU | 155 | NE | ARG | 1170 | 1576 | 1897 | 2048 | 23 | 273 | -329 | N |
| ATOM | 156 | CZ | ARG | 1170 | 26.909 | 12.990 | 21.864 | 1.00 | 19.72 | | C |
| ANISOU | 156 | CZ | ARG | 1170 | 2604 | 1954 | 2933 | 449 | 826 | -136 | C |
| ATOM | 157 | NH1 | ARG | 1170 | 26.476 | 12.462 | 23.017 | 1.00 | 22.85 | | N1+ |
| ANISOU | 157 | NH1 | ARG | 1170 | 2993 | 2144 | 3544 | 101 | 1126 | 338 | N1+ |
| ATOM | 158 | NH2 | ARG | 1170 | 27.662 | 12.265 | 21.041 | 1.00 | 23.38 | | N |
| ANISOU | 158 | NH2 | ARG | 1170 | 3028 | 2152 | 3704 | 513 | 1139 | -459 | N |
| ATOM | 159 | C | ARG | 1170 | 29.825 | 18.068 | 22.958 | 1.00 | 10.85 | | C |
| ANISOU | 159 | C | ARG | 1170 | 881 | 1540 | 1699 | 201 | 34 | -5 | C |
| ATOM | 160 | O | ARG | 1170 | 30.680 | 17.226 | 22.741 | 1.00 | 13.39 | | O |
| ANISOU | 160 | O | ARG | 1170 | 1052 | 1676 | 2360 | 362 | 138 | -254 | O |
| ATOM | 161 | N | ASN | 1171 | 29.985 | 19.353 | 22.645 | 1.00 | 10.72 | | N |
| ANISOU | 161 | N | ASN | 1171 | 1041 | 1551 | 1480 | 77 | -105 | 33 | N |
| ATOM | 162 | CA | ASN | 1171 | 31.201 | 19.813 | 22.000 | 1.00 | 11.35 | | C |
| ANISOU | 162 | CA | ASN | 1171 | 992 | 1685 | 1636 | 120 | -176 | 274 | C |
| ATOM | 163 | CB | ASN | 1171 | 30.911 | 21.192 | 21.364 | 1.00 | 13.78 | | C |
| ANISOU | 163 | CB | ASN | 1171 | 1040 | 1973 | 2223 | 227 | 40 | 600 | C |
| ATOM | 164 | CG | ASN | 1171 | 32.049 | 21.700 | 20.502 | 1.00 | 15.68 | | C |
| ANISOU | 164 | CG | ASN | 1171 | 1250 | 2415 | 2291 | -271 | -21 | 778 | C |
| ATOM | 165 | OD1 | ASN | 1171 | 31.860 | 22.550 | 19.635 | 1.00 | 23.60 | | O |
| ANISOU | 165 | OD1 | ASN | 1171 | 2345 | 3350 | 3270 | -269 | -279 | 1759 | O |
| ATOM | 166 | ND2 | ASN | 1171 | 33.242 | 21.224 | 20.675 | 1.00 | 13.78 | | N |
| ANISOU | 166 | ND2 | ASN | 1171 | 1210 | 2520 | 1506 | -106 | 209 | 283 | N |
| ATOM | 167 | C | ASN | 1171 | 32.342 | 19.855 | 23.024 | 1.00 | 10.27 | | C |
| ANISOU | 167 | C | ASN | 1171 | 1015 | 1336 | 1551 | 154 | -77 | 22 | C |
| ATOM | 168 | O | ASN | 1171 | 32.387 | 20.776 | 23.831 | 1.00 | 11.56 | | O |
| ANISOU | 168 | O | ASN | 1171 | 1266 | 1422 | 1702 | 202 | -69 | -109 | O |
| ATOM | 169 | N | GLU | 1172 | 33.239 | 18.896 | 22.898 | 1.00 | 10.88 | | N |
| ANISOU | 169 | N | GLU | 1172 | 984 | 1672 | 1478 | 378 | -170 | -139 | N |
| ATOM | 170 | CA | GLU | 1172 | 34.254 | 18.810 | 23.960 | 1.00 | 11.57 | | C |
| ANISOU | 170 | CA | GLU | 1172 | 1254 | 1313 | 1830 | 17 | -538 | 185 | C |
| ATOM | 171 | CB | GLU | 1172 | 34.841 | 17.376 | 23.901 | 1.00 | 14.01 | | C |
| ANISOU | 171 | CB | GLU | 1172 | 1720 | 1624 | 1981 | 554 | -640 | -132 | C |
| ATOM | 172 | CG | GLU | 1172 | 35.599 | 17.068 | 22.655 | 1.00 | 17.13 | | C |
| ANISOU | 172 | CG | GLU | 1172 | 1913 | 1948 | 2648 | 276 | 155 | 136 | C |
| ATOM | 173 | CD | GLU | 1172 | 36.166 | 15.642 | 22.617 | 1.00 | 19.35 | | C |
| ANISOU | 173 | CD | GLU | 1172 | 2582 | 2013 | 2759 | 554 | 429 | -85 | C |
| ATOM | 174 | OE1 | GLU | 1172 | 35.822 | 14.842 | 23.565 | 1.00 | 14.74 | | O1- |
| ANISOU | 174 | OE1 | GLU | 1172 | 1770 | 1720 | 2110 | 1 | -510 | -294 | O1- |
| ATOM | 175 | OE2 | GLU | 1172 | 36.940 | 15.369 | 21.635 | 1.00 | 17.91 | | O |
| ANISOU | 175 | OE2 | GLU | 1172 | 1718 | 2706 | 2380 | 214 | -208 | -566 | O |
| ATOM | 176 | C | GLU | 1172 | 35.329 | 19.849 | 23.870 | 1.00 | 11.22 | | C |
| ANISOU | 176 | C | GLU | 1172 | 1105 | 1618 | 1541 | 115 | -148 | 12 | C |
| ATOM | 177 | O | GLU | 1172 | 36.157 | 19.948 | 24.778 | 1.00 | 13.09 | | O |
| ANISOU | 177 | O | GLU | 1172 | 1204 | 1750 | 2021 | -170 | -412 | 215 | O |
| ATOM | 178 | N | THR | 1173 | 35.363 | 20.642 | 22.802 | 1.00 | 11.29 | | N |
| ANISOU | 178 | N | THR | 1173 | 1170 | 1492 | 1629 | 132 | 73 | -34 | N |
| ATOM | 179 | CA | THR | 1173 | 36.369 | 21.730 | 22.767 | 1.00 | 11.99 | | C |
| ANISOU | 179 | CA | THR | 1173 | 1018 | 1784 | 1753 | 79 | 228 | 47 | C |
| ATOM | 180 | CB | THR | 1173 | 36.549 | 22.287 | 21.360 | 1.00 | 13.91 | | C |
| ANISOU | 180 | CB | THR | 1173 | 1424 | 2064 | 1798 | 122 | 252 | 235 | C |
| ATOM | 181 | OG1 | THR | 1173 | 35.346 | 22.942 | 20.917 | 1.00 | 17.61 | | O |
| ANISOU | 181 | OG1 | THR | 1173 | 1662 | 3107 | 1922 | 259 | 69 | 555 | O |
| ATOM | 182 | CG2 | THR | 1173 | 36.861 | 21.132 | 20.402 | 1.00 | 17.96 | | C |
| ANISOU | 182 | CG2 | THR | 1173 | 2577 | 2554 | 1693 | 99 | 200 | -31 | C |
| ATOM | 183 | C | THR | 1173 | 35.985 | 22.858 | 23.728 | 1.00 | 10.25 | | C |
| ANISOU | 183 | C | THR | 1173 | 905 | 1313 | 1676 | 79 | -94 | 286 | C |
| ATOM | 184 | O | THR | 1173 | 36.819 | 23.741 | 24.003 | 1.00 | 12.25 | | O |
| ANISOU | 184 | O | THR | 1173 | 1111 | 1694 | 1850 | -95 | -282 | 310 | O |
| ATOM | 185 | N | HIS | 1174 | 34.752 | 22.852 | 24.227 | 1.00 | 9.98 | | N |
| ANISOU | 185 | N | HIS | 1174 | 1056 | 1444 | 1291 | 147 | 87 | 243 | N |
| ATOM | 186 | CA | HIS | 1174 | 34.322 | 23.727 | 25.286 | 1.00 | 10.45 | | C |
| ANISOU | 186 | CA | HIS | 1174 | 1170 | 1436 | 1363 | 33 | 0 | 165 | C |
| ATOM | 187 | CB | HIS | 1174 | 33.037 | 24.451 | 24.814 | 1.00 | 13.42 | | C |
| ANISOU | 187 | CB | HIS | 1174 | 1528 | 1368 | 2201 | 426 | -148 | 18 | C |
| ATOM | 188 | CG | HIS | 1174 | 32.414 | 25.395 | 25.810 | 1.00 | 14.01 | | C |
| ANISOU | 188 | CG | HIS | 1174 | 1402 | 1626 | 2296 | 209 | -59 | -80 | C |
| ATOM | 189 | CD2 | HIS | 1174 | 32.924 | 26.591 | 26.230 | 1.00 | 14.54 | | C |
| ANISOU | 189 | CD2 | HIS | 1174 | 1341 | 1694 | 2491 | 367 | -355 | -394 | C |
| ATOM | 190 | ND1 | HIS | 1174 | 31.216 | 25.235 | 26.468 | 1.00 | 17.05 | | N |
| ANISOU | 190 | ND1 | HIS | 1174 | 1772 | 2329 | 2376 | -29 | 199 | -378 | N |
| ATOM | 191 | CE1 | HIS | 1174 | 30.983 | 26.269 | 27.266 | 1.00 | 14.39 | | C |
| ANISOU | 191 | CE1 | HIS | 1174 | 1490 | 1995 | 1982 | 519 | -282 | -72 | C |
| ATOM | 192 | NE2 | HIS | 1174 | 32.012 | 27.114 | 27.140 | 1.00 | 18.69 | | N |
| ANISOU | 192 | NE2 | HIS | 1174 | 1989 | 2098 | 3012 | 319 | 104 | -499 | N |
| ATOM | 193 | C | HIS | 1174 | 34.064 | 22.915 | 26.536 | 1.00 | 9.30 | | C |
| ANISOU | 193 | C | HIS | 1174 | 828 | 1307 | 1397 | -172 | 181 | 49 | C |
| ATOM | 194 | O | HIS | 1174 | 33.655 | 21.746 | 26.424 | 1.00 | 11.98 | | O |
| ANISOU | 194 | O | HIS | 1174 | 1520 | 1399 | 1632 | -368 | 208 | -137 | O |
| ATOM | 195 | N | ASN | 1175 | 34.272 | 23.492 | 27.713 | 1.00 | 8.83 | | N |
| ANISOU | 195 | N | ASN | 1175 | 805 | 1194 | 1357 | 61 | 49 | 98 | N |
| ATOM | 196 | CA | ASN | 1175 | 33.947 | 22.790 | 28.969 | 1.00 | 9.09 | | C |
| ANISOU | 196 | CA | ASN | 1175 | 845 | 1198 | 1409 | 151 | 71 | 208 | C |
| ATOM | 197 | CB | ASN | 1175 | 35.220 | 22.409 | 29.701 | 1.00 | 10.21 | | C |
| ANISOU | 197 | CB | ASN | 1175 | 939 | 1224 | 1718 | 220 | -30 | 333 | C |
| ATOM | 198 | CG | ASN | 1175 | 35.045 | 21.331 | 30.761 | 1.00 | 9.50 | | C |
| ANISOU | 198 | CG | ASN | 1175 | 989 | 1125 | 1495 | 337 | 148 | 174 | C |
| ATOM | 199 | OD1 | ASN | 1175 | 35.985 | 20.555 | 30.990 | 1.00 | 11.90 | | O |
| ANISOU | 199 | OD1 | ASN | 1175 | 1151 | 1415 | 1956 | 476 | 328 | 513 | O |
| ATOM | 200 | ND2 | ASN | 1175 | 33.916 | 21.206 | 31.438 | 1.00 | 10.96 | | N |
| ANISOU | 200 | ND2 | ASN | 1175 | 1022 | 1293 | 1850 | 109 | 214 | 104 | N |
| ATOM | 201 | C | ASN | 1175 | 33.053 | 23.689 | 29.802 | 1.00 | 8.54 | | C |
| ANISOU | 201 | C | ASN | 1175 | 856 | 916 | 1473 | 44 | 111 | 206 | C |
| ATOM | 202 | O | ASN | 1175 | 33.540 | 24.502 | 30.574 | 1.00 | 9.66 | | O |
| ANISOU | 202 | O | ASN | 1175 | 838 | 1141 | 1693 | 100 | -116 | 78 | O |
| ATOM | 203 | N | PRO | 1176 | 31.715 | 23.530 | 29.672 | 1.00 | 9.16 | | N |
| ANISOU | 203 | N | PRO | 1176 | 874 | 1167 | 1437 | 43 | 57 | -13 | N |
| ATOM | 204 | CD | PRO | 1176 | 30.974 | 22.648 | 28.762 | 1.00 | 11.31 | | C |
| ANISOU | 204 | CD | PRO | 1176 | 944 | 1742 | 1611 | 46 | -62 | -255 | C |
| ATOM | 205 | CA | PRO | 1176 | 30.843 | 24.271 | 30.573 | 1.00 | 8.68 | | C |
| ANISOU | 205 | CA | PRO | 1176 | 703 | 1197 | 1399 | 66 | 31 | 85 | C |
| ATOM | 206 | CB | PRO | 1176 | 29.432 | 23.796 | 30.212 | 1.00 | 12.91 | | C |
| ANISOU | 206 | CB | PRO | 1176 | 836 | 2114 | 1956 | -1 | -76 | -630 | C |
| ATOM | 207 | CG | PRO | 1176 | 29.579 | 23.200 | 28.839 | 1.00 | 13.63 | | C |
| ANISOU | 207 | CG | PRO | 1176 | 851 | 2605 | 1725 | 58 | 14 | -474 | C |
| ATOM | 208 | C | PRO | 1176 | 31.191 | 23.838 | 31.990 | 1.00 | 9.66 | | C |
| ANISOU | 208 | C | PRO | 1176 | 929 | 1272 | 1469 | 268 | 122 | 153 | C |
| ATOM | 209 | O | PRO | 1176 | 31.615 | 22.709 | 32.207 | 1.00 | 10.84 | | O |
| ANISOU | 209 | O | PRO | 1176 | 1145 | 1308 | 1664 | 255 | 142 | 336 | O |
| ATOM | 210 | N | THR | 1177 | 31.005 | 24.722 | 32.960 | 1.00 | 9.73 | | N |
| ANISOU | 210 | N | THR | 1177 | 793 | 1434 | 1470 | 63 | 141 | 4 | N |
| ATOM | 211 | CA | THR | 1177 | 31.110 | 24.283 | 34.334 | 1.00 | 10.10 | | C |
| ANISOU | 211 | CA | THR | 1177 | 955 | 1367 | 1514 | -17 | 72 | 7 | C |
| ATOM | 212 | CB | THR | 1177 | 31.374 | 25.488 | 35.285 | 1.00 | 11.37 | | C |
| ANISOU | 212 | CB | THR | 1177 | 764 | 1661 | 1893 | -38 | -119 | -343 | C |
| ATOM | 213 | OG1 | THR | 1177 | 30.261 | 26.381 | 35.119 | 1.00 | 11.62 | | O |
| ANISOU | 213 | OG1 | THR | 1177 | 932 | 1606 | 1879 | 34 | -37 | -384 | O |
| ATOM | 214 | CG2 | THR | 1177 | 32.663 | 26.170 | 34.898 | 1.00 | 13.40 | | C |
| ANISOU | 214 | CG2 | THR | 1177 | 958 | 2076 | 2058 | -342 | -191 | -64 | C |
| ATOM | 215 | C | THR | 1177 | 29.841 | 23.569 | 34.754 | 1.00 | 9.08 | | C |
| ANISOU | 215 | C | THR | 1177 | 818 | 1246 | 1387 | 83 | -51 | 15 | C |
| ATOM | 216 | O | THR | 1177 | 28.808 | 23.648 | 34.074 | 1.00 | 9.66 | | O |
| ANISOU | 216 | O | THR | 1177 | 945 | 1360 | 1365 | 35 | -83 | -19 | O |
| ATOM | 217 | N | VAL | 1178 | 29.877 | 22.884 | 35.893 | 1.00 | 10.69 | | N |
| ANISOU | 217 | N | VAL | 1178 | 1074 | 1578 | 1408 | -8 | -1 | 177 | N |
| ATOM | 218 | CA | VAL | 1178 | 28.653 | 22.333 | 36.451 | 1.00 | 9.95 | | C |
| ANISOU | 218 | CA | VAL | 1178 | 1022 | 1513 | 1248 | 81 | 39 | 29 | C |
| ATOM | 219 | CB | VAL | 1178 | 28.903 | 21.572 | 37.778 | 1.00 | 12.30 | | C |
| ANISOU | 219 | CB | VAL | 1178 | 1150 | 1902 | 1622 | 538 | 222 | 510 | C |
| ATOM | 220 | CG1 | VAL | 1178 | 27.552 | 21.255 | 38.411 | 1.00 | 15.49 | | C |
| ANISOU | 220 | CG1 | VAL | 1178 | 1523 | 2066 | 2297 | 161 | 581 | 712 | C |
| ATOM | 221 | CG2 | VAL | 1178 | 29.690 | 20.307 | 37.529 | 1.00 | 15.45 | | C |
| ANISOU | 221 | CG2 | VAL | 1178 | 1933 | 1708 | 2231 | 577 | 494 | 410 | C |
| ATOM | 222 | C | VAL | 1178 | 27.655 | 23.456 | 36.644 | 1.00 | 9.15 | | C |
| ANISOU | 222 | C | VAL | 1178 | 998 | 1241 | 1238 | -15 | -223 | 45 | C |
| ATOM | 223 | O | VAL | 1178 | 26.471 | 23.277 | 36.334 | 1.00 | 9.57 | | O |
| ANISOU | 223 | O | VAL | 1178 | 972 | 1407 | 1257 | -87 | -123 | -51 | O |
| ATOM | 224 | N | LYS | 1179 | 28.078 | 24.598 | 37.142 | 1.00 | 9.80 | | N |
| ANISOU | 224 | N | *LYS* | 1179 | 970 | 1477 | 1276 | 24 | -201 | -221 | N |
| ATOM | 225 | CA | LYS | 1179 | 27.194 | 25.741 | 37.336 | 1.00 | 10.25 | | C |
| ANISOU | 225 | CA | LYS | 1179 | 1140 | 1482 | 1273 | 76 | -262 | -325 | C |
| ATOM | 226 | CB | LYS | 1179 | 27.997 | 26.923 | 37.906 | 1.00 | 11.45 | | C |
| ANISOU | 226 | CB | LYS | 1179 | 1163 | 1477 | 1710 | -67 | -341 | -265 | C |
| ATOM | 227 | CG | LYS | 1179 | 27.044 | 28.071 | 38.321 | 1.00 | 12.33 | | C |
| ANISOU | 227 | CG | LYS | 1179 | 1238 | 1446 | 2002 | -53 | -295 | -429 | C |
| ATOM | 228 | CD | LYS | 1179 | 27.909 | 29.254 | 38.803 | 1.00 | 16.72 | | C |
| ANISOU | 228 | CD | LYS | 1179 | 1435 | 2073 | 2845 | -233 | -384 | -1210 | C |
| ATOM | 229 | CE | LYS | 1179 | 27.041 | 30.445 | 39.212 | 1.00 | 17.61 | | C |
| ANISOU | 229 | CE | LYS | 1179 | 2035 | 1848 | 2807 | -438 | 502 | -1052 | C |
| ATOM | 230 | NZ | LYS | 1179 | 26.482 | 30.163 | 40.568 | 1.00 | 16.98 | | N1+ |
| ANISOU | 230 | NZ | LYS | 1179 | 1332 | 1952 | 3167 | -99 | 394 | -155 | N1+ |
| ATOM | 231 | C | LYS | 1179 | 26.524 | 26.135 | 36.024 | 1.00 | 9.59 | | C |
| ANISOU | 231 | C | LYS | 1179 | 798 | 1492 | 1352 | -25 | -154 | -122 | C |
| ATOM | 232 | O | LYS | 1179 | 25.322 | 26.379 | 35.975 | 1.00 | 9.69 | | O |
| ANISOU | 232 | O | LYS | 1179 | 835 | 1330 | 1517 | -10 | -142 | -223 | O |
| ATOM | 233 | N | ASP | 1180 | 27.312 | 26.198 | 34.953 | 1.00 | 9.18 | | N |
| ANISOU | 233 | N | ASP | 1180 | 1041 | 1100 | 1346 | -117 | -82 | -97 | N |
| ATOM | 234 | CA | ASP | 1180 | 26.762 | 26.551 | 33.653 | 1.00 | 8.85 | | C |
| ANISOU | 234 | CA | ASP | 1180 | 942 | 979 | 1440 | -185 | -75 | 41 | C |
| ATOM | 235 | CB | ASP | 1180 | 27.812 | 26.499 | 32.547 | 1.00 | 9.53 | | C |
| ANISOU | 235 | CB | ASP | 1180 | 813 | 1409 | 1401 | -156 | -95 | 30 | C |
| ATOM | 236 | CG | ASP | 1180 | 28.865 | 27.597 | 32.622 | 1.00 | 11.54 | | C |
| ANISOU | 236 | CG | ASP | 1180 | 1045 | 1140 | 2200 | -130 | 256 | 152 | C |
| ATOM | 237 | OD1 | ASP | 1180 | 28.600 | 28.651 | 33.216 | 1.00 | 14.06 | | O |
| ANISOU | 237 | OD1 | ASP | 1180 | 1169 | 1307 | 2867 | -285 | 368 | -255 | O |
| ATOM | 238 | OD2 | ASP | 1180 | 29.944 | 27.320 | 32.053 | 1.00 | 12.80 | | O1- |
| ANISOU | 238 | OD2 | ASP | 1180 | 1119 | 1429 | 2318 | -208 | 474 | 162 | O1- |
| ATOM | 239 | C | ASP | 1180 | 25.667 | 25.584 | 33.216 | 1.00 | 8.59 | | C |
| ANISOU | 239 | C | ASP | 1180 | 832 | 953 | 1477 | -55 | -122 | -11 | C |
| ATOM | 240 | O | ASP | 1180 | 24.646 | 25.988 | 32.697 | 1.00 | 8.86 | | O |
| ANISOU | 240 | O | ASP | 1180 | 803 | 1097 | 1466 | 79 | -96 | -134 | O |
| ATOM | 241 | N | LEU | 1181 | 25.912 | 24.282 | 33.426 | 1.00 | 8.71 | | N |
| ANISOU | 241 | N | LEU | 1181 | 1044 | 883 | 1381 | -65 | -68 | -116 | N |
| ATOM | 242 | CA | LEU | 1181 | 24.940 | 23.273 | 32.997 | 1.00 | 8.08 | | C |
| ANISOU | 242 | CA | LEU | 1181 | 783 | 945 | 1342 | -8 | -56 | -169 | C |
| ATOM | 243 | CB | LEU | 1181 | 25.539 | 21.872 | 33.168 | 1.00 | 8.70 | | C |
| ANISOU | 243 | CB | LEU | 1181 | 823 | 890 | 1592 | 7 | 25 | -133 | C |
| ATOM | 244 | CG | LEU | 1181 | 26.739 | 21.570 | 32.264 | 1.00 | 9.75 | | C |
| ANISOU | 244 | CG | LEU | 1181 | 892 | 1322 | 1491 | 220 | 1 | -157 | C |
| ATOM | 245 | CD1 | LEU | 1181 | 27.383 | 20.260 | 32.714 | 1.00 | 11.15 | | C |
| ANISOU | 245 | CD1 | LEU | 1181 | 1049 | 1337 | 1852 | 366 | -72 | -289 | C |
| ATOM | 246 | CD2 | LEU | 1181 | 26.353 | 21.502 | 30.791 | 1.00 | 10.62 | | C |
| ANISOU | 246 | CD2 | LEU | 1181 | 1169 | 1351 | 1514 | -41 | -35 | -188 | C |
| ATOM | 247 | C | LEU | 1181 | 23.628 | 23.430 | 33.767 | 1.00 | 8.45 | | C |
| ANISOU | 247 | C | LEU | 1181 | 886 | 1058 | 1266 | 109 | -76 | -117 | C |
| ATOM | 248 | O | LEU | 1181 | 22.519 | 23.364 | 33.215 | 1.00 | 8.81 | | O |
| ANISOU | 248 | O | LEU | 1181 | 733 | 1196 | 1417 | -5 | -137 | 3 | O |
| ATOM | 249 | N | ILE | 1182 | 23.750 | 23.645 | 35.073 | 1.00 | 8.10 | | N |
| ANISOU | 249 | N | ILE | 1182 | 859 | 1001 | 1219 | 45 | -39 | -36 | N |
| ATOM | 250 | CA | ILE | 1182 | 22.546 | 23.880 | 35.901 | 1.00 | 8.12 | | C |
| ANISOU | 250 | CA | ILE | 1182 | 847 | 1007 | 1230 | 108 | -2 | 29 | C |
| ATOM | 251 | CB | ILE | 1182 | 22.936 | 23.922 | 37.388 | 1.00 | 8.56 | | C |
| ANISOU | 251 | CB | ILE | 1182 | 950 | 1092 | 1209 | 110 | 0 | -121 | C |
| ATOM | 252 | CG2 | ILE | 1182 | 21.709 | 24.249 | 38.242 | 1.00 | 9.90 | | C |
| ANISOU | 252 | CG2 | ILE | 1182 | 1131 | 1214 | 1417 | 140 | 182 | -143 | C |
| ATOM | 253 | CG1 | ILE | 1182 | 23.611 | 22.623 | 37.829 | 1.00 | 9.54 | | C |
| ANISOU | 253 | CG1 | ILE | 1182 | 968 | 1169 | 1488 | 82 | -272 | 79 | C |
| ATOM | 254 | CD1 | ILE | 1182 | 24.161 | 22.659 | 39.251 | 1.00 | 12.91 | | C |
| ANISOU | 254 | CD1 | ILE | 1182 | 1229 | 2447 | 1231 | 417 | 26 | 309 | C |
| ATOM | 255 | C | ILE | 1182 | 21.887 | 25.140 | 35.436 | 1.00 | 8.58 | | C |
| ANISOU | 255 | C | ILE | 1182 | 841 | 942 | 1475 | 38 | -125 | -6 | C |
| ATOM | 256 | O | ILE | 1182 | 20.642 | 25.207 | 35.353 | 1.00 | 8.60 | | O |
| ANISOU | 256 | O | ILE | 1182 | 857 | 1108 | 1301 | 80 | -126 | 32 | O |
| ATOM | 257 | N | GLY | 1183 | 22.683 | 26.170 | 35.117 | 1.00 | 8.35 | | N |
| ANISOU | 257 | N | GLY | 1183 | 1064 | 865 | 1245 | -32 | -93 | -117 | N |
| ATOM | 258 | CA | GLY | 1183 | 22.101 | 27.428 | 34.635 | 1.00 | 8.61 | | C |
| ANISOU | 258 | CA | GLY | 1183 | 1002 | 1045 | 1226 | -167 | -255 | 143 | C |
| ATOM | 259 | C | GLY | 1183 | 21.338 | 27.250 | 33.337 | 1.00 | 7.73 | | C |
| ANISOU | 259 | C | GLY | 1183 | 817 | 906 | 1212 | -181 | -71 | 21 | C |
| ATOM | 260 | O | GLY | 1183 | 20.274 | 27.869 | 33.161 | 1.00 | 8.93 | | O |
| ANISOU | 260 | O | GLY | 1183 | 886 | 1016 | 1492 | 32 | -182 | -50 | O |
| ATOM | 261 | N | PHE | 1184 | 21.826 | 26.416 | 32.414 | 1.00 | 8.29 | | N |
| ANISOU | 261 | N | PHE | 1184 | 1041 | 807 | 1304 | -96 | -143 | -55 | N |
| ATOM | 262 | CA | PHE | 1184 | 21.033 | 26.154 | 31.215 | 100 | 8.14 | | C |
| ANISOU | 262 | CA | PHE | 1184 | 847 | 1033 | 1211 | -87 | -52 | -25 | C |
| ATOM | 263 | CB | PHE | 1184 | 21.822 | 25.243 | 30.283 | 1.00 | 9.26 | | C |
| ANISOU | 263 | CB | PHE | 1184 | 1013 | 1269 | 1235 | -78 | 68 | -104 | C |
| ATOM | 264 | CG | PHE | 1184 | 23.093 | 25.842 | 29.709 | 1.00 | 9.41 | | C |
| ANISOU | 264 | CG | PHE | 1184 | 931 | 1277 | 1368 | 8 | 32 | 192 | C |
| ATOM | 265 | CD1 | PHE | 1184 | 23.247 | 27.181 | 29.424 | 1.00 | 10.96 | | C |
| ANISOU | 265 | CD1 | PHE | 1184 | 995 | 1332 | 1837 | -109 | 42 | 172 | C |
| ATOM | 266 | CD2 | PHE | 1184 | 24.155 | 24.967 | 29.456 | 1.00 | 10.30 | | C |
| ANISOU | 266 | CD2 | PHE | 1184 | 901 | 1454 | 1558 | 9 | 64 | 92 | C |
| ATOM | 267 | CE1 | PHE | 1184 | 24.444 | 27.650 | 28.871 | 1.00 | 12.83 | | C |
| ANISOU | 267 | CE1 | PHE | 1184 | 1173 | 1641 | 2060 | -349 | 233 | 94 | C |
| ATOM | 268 | CE2 | PHE | 1184 | 25.359 | 25.417 | 28.916 | 1.00 | 12.09 | | C |
| ANISOU | 268 | CE2 | PHE | 1184 | 875 | 1840 | 1880 | -171 | 106 | 94 | C |
| ATOM | 269 | CZ | PHE | 1184 | 25.488 | 26.773 | 28.631 | 1.00 | 13.14 | | C |
| ANISOU | 269 | CZ | PHE | 1184 | 1082 | 1833 | 2076 | -319 | 215 | 30 | C |
| ATOM | 270 | C | PHE | 1184 | 19.703 | 25.510 | 31.559 | 1.00 | 7.61 | | C |
| ANISOU | 270 | C | PHE | 1184 | 882 | 777 | 1233 | -56 | -70 | -34 | C |
| ATOM | 271 | O | PHE | 1184 | 18.649 | 25.829 | 30.982 | 1.00 | 8.03 | | O |
| ANISOU | 271 | O | PHE | 1184 | 822 | 966 | 1265 | -8 | -54 | -14 | O |
| ATOM | 272 | N | GLY | 1185 | 19.703 | 24.613 | 32.540 | 1.00 | 7.78 | | N |
| ANISOU | 272 | N | GLY | 1185 | 914 | 828 | 1215 | -78 | -9 | -7 | N |
| ATOM | 273 | CA | GLY | 1185 | 18.469 | 23.994 | 32.979 | 1.00 | 7.93 | | C |
| ANISOU | 273 | CA | GLY | 1185 | 757 | 889 | 1365 | 19 | 128 | 25 | C |
| ATOM | 274 | C | GLY | 1185 | 17.536 | 24.996 | 33.624 | 1.00 | 7.29 | | C |
| ANISOU | 274 | C | GLY | 1185 | 763 | 721 | 1285 | 15 | -148 | 53 | C |
| ATOM | 275 | O | GLY | 1185 | 16.299 | 24.956 | 33.419 | 1.00 | 8.20 | | O |
| ANISOU | 275 | O | GLY | 1185 | 731 | 830 | 1554 | 0 | -108 | 137 | O |
| ATOM | 276 | N | LEU | 1186 | 18.080 | 25.923 | 34.413 | 1.00 | 8.00 | | N |
| ANISOU | 276 | N | LEU | 1186 | 1023 | 774 | 1244 | -34 | 3 | -6 | N |
| ATOM | 277 | CA | LEU | 1186 | 17.273 | 27.025 | 34.995 | 1.00 | 7.57 | | C |
| ANISOU | 277 | CA | LEU | 1186 | 896 | 827 | 1153 | 11 | -192 | -46 | C |
| ATOM | 278 | CB | LEU | 1186 | 18.216 | 27.806 | 35.914 | 1.00 | 9.06 | | C |
| ANISOU | 278 | CB | LEU | 1186 | 1064 | 1152 | 1226 | 73 | -279 | -261 | C |
| ATOM | 279 | CG | LEU | 1186 | 17.562 | 29.042 | 36.588 | 1.00 | 9.84 | | C |
| ANISOU | 279 | CG | LEU | 1186 | 983 | 1292 | 1464 | -178 | 40 | -469 | C |
| ATOM | 280 | CD1 | LEU | 1186 | 16.314 | 28.696 | 37.373 | 1.00 | 9.74 | | C |
| ANISOU | 280 | CD1 | LEU | 1186 | 994 | 1271 | 1437 | -90 | 43 | -44 | C |
| ATOM | 281 | CD2 | LEU | 1186 | 18.613 | 29.718 | 37.467 | 1.00 | 10.69 | | C |
| ANISOU | 281 | CD2 | LEU | 1186 | 1084 | 1524 | 1454 | -275 | -196 | -469 | C |
| ATOM | 282 | C | LEU | 1186 | 16.645 | 27.880 | 33.922 | 1.00 | 7.32 | | C |
| ANISOU | 282 | C | LEU | 1186 | 828 | 757 | 1197 | -98 | -164 | -40 | C |
| ATOM | 283 | O | LEU | 1186 | 15.465 | 28.227 | 34.010 | 1.00 | 7.86 | | O |
| ANISOU | 283 | O | LEU | 1186 | 909 | 937 | 1139 | 38 | -202 | -42 | O |
| ATOM | 284 | N | GLN | 1187 | 17.424 | 28.211 | 32.884 | 1.00 | 7.78 | | N |
| ANISOU | 284 | N | GLN | 1187 | 982 | 771 | 1205 | -145 | -128 | 56 | N |
| ATOM | 285 | CA | GLN | 1187 | 16.838 | 28.986 | 31.777 | 1.00 | 7.95 | | C |
| ANISOU | 285 | CA | GLN | 1187 | 990 | 999 | 1030 | -119 | -148 | -85 | C |
| ATOM | 286 | CB | GLN | 1187 | 17.926 | 29.326 | 30.729 | 1.00 | 8.51 | | C |
| ANISOU | 286 | CB | GLN | 1187 | 1003 | 923 | 1308 | -277 | -20 | -37 | C |
| ATOM | 287 | CG | GLN | 1187 | 18.991 | 30.293 | 31.300 | 1.00 | 9.79 | | C |
| ANISOU | 287 | CG | GLN | 1187 | 1319 | 1045 | 1355 | -421 | -70 | -152 | C |
| ATOM | 288 | CD | GLN | 1187 | 19.837 | 30.774 | 30.143 | 1.00 | 9.38 | | C |
| ANISOU | 288 | CD | GLN | 1187 | 988 | 947 | 1630 | -243 | -101 | 17 | C |
| ATOM | 289 | OE1 | GLN | 1187 | 19.281 | 31.298 | 29.162 | 1.00 | 10.71 | | O |
| ANISOU | 289 | OE1 | GLN | 1187 | 1365 | 1193 | 1513 | -185 | -54 | 120 | O |
| ATOM | 290 | NE2 | GLN | 1187 | 21.140 | 30.613 | 30.212 | 1.00 | 12.77 | | N |
| ANISOU | 290 | NE2 | GLN | 1187 | 982 | 1468 | 2401 | -85 | -31 | 37 | N |
| ATOM | 291 | C | GLN | 1187 | 15.702 | 28.246 | 31.102 | 1.00 | 7.84 | | C |
| ANISOU | 291 | C | GLN | 1187 | 788 | 852 | 1338 | -71 | -104 | -31 | C |
| ATOM | 292 | O | GLN | 1187 | 14.664 | 28.831 | 30.786 | 1.00 | 8.11 | | O |
| ANISOU | 292 | O | GLN | 1187 | 809 | 965 | 1307 | 36 | -38 | -124 | O |
| ATOM | 293 | N | VAL | 1188 | 15.871 | 26.936 | 30.837 | 1.00 | 7.53 | | N |
| ANISOU | 293 | N | VAL | 1188 | 944 | 858 | 1058 | -61 | -108 | -125 | N |
| ATOM | 294 | CA | VAL | 1188 | 14.741 | 26.164 | 30.246 | 1.00 | 8.06 | | C |
| ANISOU | 294 | CA | VAL | 1188 | 926 | 944 | 1194 | -69 | -68 | -231 | C |
| ATOM | 295 | CB | VAL | 1188 | 15.202 | 24.728 | 29.939 | 1.00 | 8.44 | | C |
| ANISOU | 295 | CB | VAL | 1188 | 961 | 904 | 1341 | -117 | 47 | -232 | C |
| ATOM | 296 | CG1 | VAL | 1188 | 13.999 | 23.891 | 29.493 | 1.00 | 8.98 | | C |
| ANISOU | 296 | CG1 | VAL | 1188 | 983 | 1003 | 1425 | -90 | -141 | -263 | C |
| ATOM | 297 | CG2 | VAL | 1188 | 16.288 | 24.736 | 28.871 | 1.00 | 9.31 | | C |
| ANISOU | 297 | CG2 | VAL | 1188 | 1087 | 1300 | 1150 | 32 | 51 | -163 | C |
| ATOM | 298 | C | VAL | 1188 | 13.557 | 26.187 | 31.179 | 1.00 | 7.39 | | C |
| ANISOU | 298 | C | VAL | 1188 | 855 | 750 | 1202 | 9 | -148 | -50 | C |
| ATOM | 299 | O | VAL | 1188 | 12.406 | 26.370 | 30.734 | 1.00 | 8.19 | | O |
| ANISOU | 299 | O | VAL | 1188 | 848 | 805 | 1460 | 41 | -249 | -88 | O |
| ATOM | 300 | N | ALA | 1189 | 13.778 | 26.009 | 32.479 | 1.00 | 7.39 | | N |
| ANISOU | 300 | N | ALA | 1189 | 857 | 797 | 1155 | -12 | -51 | 57 | N |
| ATOM | 301 | CA | ALA | 1189 | 12.653 | 26.049 | 33.421 | 1.00 | 8.04 | | C |
| ANISOU | 301 | CA | ALA | 1189 | 930 | 923 | 1203 | 85 | -41 | 53 | C |
| ATOM | 302 | CB | ALA | 1189 | 13.145 | 25.786 | 34.847 | 1.00 | 8.53 | | C |
| ANISOU | 302 | CB | ALA | 1189 | 1201 | 912 | 1126 | 84 | -134 | -9 | C |
| ATOM | 303 | C | ALA | 1189 | 11.910 | 27.385 | 33.361 | 1.00 | 7.11 | | C |
| ANISOU | 303 | C | ALA | 1189 | 769 | 924 | 1007 | -32 | -44 | 88 | C |
| ATOM | 304 | O | ALA | 1189 | 10.704 | 27.424 | 33.448 | 1.00 | 7.76 | | O |
| ANISOU | 304 | O | ALA | 1189 | 804 | 958 | 1188 | 36 | -66 | -9 | O |
| ATOM | 305 | N | LYS | 1190 | 12.635 | 28.507 | 33.194 | 1.00 | 7.78 | | N |
| ANISOU | 305 | N | LYS | 1190 | 963 | 842 | 1150 | -32 | -93 | 62 | N |
| ATOM | 306 | CA | LYS | 1190 | 11.978 | 29.807 | 33.121 | 1.00 | 8.03 | | C |
| ANISOU | 306 | CA | LYS | 1190 | 983 | 864 | 1204 | 18 | -186 | -30 | C |
| ATOM | 307 | CB | LYS | 1190 | 13.021 | 30.920 | 33.178 | 1.00 | 8.26 | | C |
| ANISOU | 307 | CB | LYS | 1190 | 966 | 851 | 1322 | -21 | -103 | 62 | C |
| ATOM | 308 | CG | LYS | 1190 | 13.672 | 31.051 | 34.569 | 1.00 | 9.20 | | C |
| ANISOU | 308 | CG | LYS | 1190 | 1204 | 991 | 1301 | -72 | -197 | -136 | C |
| ATOM | 309 | CD | LYS | 1190 | 14.763 | 32.108 | 34.640 | 1.00 | 11.45 | | C |
| ANISOU | 309 | CD | LYS | 1190 | 1560 | 1198 | 1594 | -254 | -184 | -203 | C |
| ATOM | 310 | CE | LYS | 1190 | 15.239 | 32.250 | 36.093 | 1.00 | 13.21 | | C |
| ANISOU | 310 | CE | LYS | 1190 | 1812 | 1385 | 1820 | -385 | -572 | -204 | C |
| ATOM | 311 | NZ | LYS | 1190 | 16.396 | 33.160 | 36.167 | 1.00 | 19.39 | | N1+ |
| ANISOU | 311 | NZ | LYS | 1190 | 2447 | 2201 | 2719 | -1072 | -709 | -250 | N1+ |
| ATOM | 312 | C | LYS | 1190 | 11.170 | 29.941 | 31.830 | 1.00 | 7.84 | | C |
| ANISOU | 312 | C | LYS | 1190 | 916 | 865 | 1197 | 102 | -104 | -62 | C |
| ATOM | 313 | O | LYS | 1190 | 10.074 | 30.487 | 31.845 | 1.00 | 8.49 | | O |
| ANISOU | 313 | O | LYS | 1190 | 865 | 884 | 1474 | 8 | -81 | 143 | O |
| ATOM | 314 | N | GLY | 1191 | 11.700 | 29.438 | 30.715 | 1.00 | 7.81 | | N |
| ANISOU | 314 | N | GLY | 1191 | 1125 | 725 | 1119 | -101 | -159 | -52 | N |
| ATOM | 315 | CA | GLY | 1191 | 10.906 | 29.481 | 29.453 | 1.00 | 7.93 | | C |
| ANISOU | 315 | CA | GLY | 1191 | 1000 | 917 | 1097 | -247 | -151 | 4 | C |
| ATOM | 316 | C | GLY | 1191 | 9.669 | 28.622 | 29.587 | 1.00 | 8.03 | | C |
| ANISOU | 316 | C | GLY | 1191 | 877 | 728 | 1447 | -74 | -80 | 59 | C |
| ATOM | 317 | O | GLY | 1191 | 8.567 | 28.986 | 29.154 | 1.00 | 8.42 | | O |
| ANISOU | 317 | O | GLY | 1191 | 964 | 875 | 1360 | -27 | -140 | 74 | O |
| ATOM | 318 | N | MET | 1192 | 9.798 | 27.435 | 30.207 | 1.00 | 8.56 | | N |
| ANISOU | 318 | N | MET | 1192 | 874 | 774 | 1603 | -35 | -71 | 99 | N |
| ATOM | 319 | CA | MET | 1192 | 8.643 | 26.546 | 30.388 | 1.00 | 8.01 | | C |
| ANISOU | 319 | CA | MET | 1192 | 915 | 711 | 1418 | -95 | 5 | 6 | C |
| ATOM | 320 | CB | MET | 1192 | 9.120 | 25.161 | 30.827 | 1.00 | 881 | | C |
| ANISOU | 320 | CB | MET | 1192 | 1100 | 715 | 1534 | 55 | 17 | -40 | C |
| ATOM | 321 | CG | MET | 1192 | 9.884 | 24.386 | 29.740 | 1.00 | 9.44 | | C |
| ANISOU | 321 | CG | MET | 1192 | 1020 | 1089 | 1479 | 95 | -191 | -307 | C |
| ATOM | 322 | SD | MET | 1192 | 8.997 | 24.234 | 28.167 | 1.00 | 9.74 | | S |
| ANISOU | 322 | SD | MET | 1192 | 1286 | 1030 | 1386 | -148 | -77 | 32 | S |
| ATOM | 323 | CE | MET | 1192 | 7.489 | 23.426 | 28.713 | 1.00 | 12.16 | | C |
| ANISOU | 323 | CE | MET | 1192 | 1287 | 1585 | 1748 | -556 | -126 | -158 | C |
| ATOM | 324 | C | MET | 1192 | 7.686 | 27.121 | 31.412 | 1.00 | 7.76 | | C |
| ANISOU | 324 | C | MET | 1192 | 848 | 761 | 1340 | 95 | -143 | 95 | C |
| ATOM | 325 | O | MET | 1192 | 6.452 | 26.937 | 31.242 | 1.00 | 9.20 | | O |
| ANISOU | 325 | O | MET | 1192 | 872 | 908 | 1714 | 54 | -61 | 49 | O |
| ATOM | 326 | N | LYS | 1193 | 8.167 | 27.815 | 32.459 | 1.00 | 8.29 | | N |
| ANISOU | 326 | N | LYS | 1193 | 1079 | 711 | 1360 | 106 | -19 | -24 | N |
| ATOM | 327 | CA | LYS | 1193 | 7.227 | 28.501 | 33.366 | 1.00 | 9.03 | | C |
| ANISOU | 327 | CA | LYS | 1193 | 1115 | 981 | 1334 | 212 | -79 | 11 | C |
| ATOM | 328 | CB | LYS | 1193 | 8.059 | 29.182 | 34.466 | 1.00 | 9.23 | | C |
| ANISOU | 328 | CB | LYS | 1193 | 1232 | 892 | 1382 | 44 | -119 | -63 | C |
| ATOM | 329 | CG | LYS | 1193 | 7.250 | 30.053 | 35.443 | 1.00 | 11.20 | | C |
| ANISOU | 329 | CG | LYS | 1193 | 1336 | 1316 | 1604 | 120 | 93 | -211 | C |
| ATOM | 330 | CD | LYS | 1193 | 8.219 | 30.662 | 36.465 | 1.00 | 13.40 | | C |
| ANISOU | 330 | CD | LYS | 1193 | 2106 | 1577 | 1407 | 39 | -89 | -262 | C |
| ATOM | 331 | CE | LYS | 1193 | 7.746 | 31.796 | 37.323 | 1.00 | 20.83 | | C |
| ANISOU | 331 | CE | LYS | 1193 | 3758 | 1953 | 2204 | 313 | -271 | -873 | C |
| ATOM | 332 | NZ | LYS | 1193 | 8.837 | 32.282 | 38.290 | 1.00 | 18.80 | | N1+ |
| ANISOU | 332 | NZ | LYS | 1193 | 3621 | 1556 | 1967 | -374 | 209 | -563 | N1+ |
| ATOM | 333 | C | LYS | 1193 | 6.380 | 29.472 | 32.596 | 1.00 | 8.41 | | C |
| ANISOU | 333 | C | LYS | 1193 | 871 | 948 | 1378 | 114 | 25 | 21 | C |
| ATOM | 334 | O | LYS | 1193 | 5.163 | 29.588 | 32.794 | 1.00 | 10.02 | | O |
| ANISOU | 334 | O | LYS | 1193 | 928 | 982 | 1897 | 162 | 23 | 55 | O |
| ATOM | 335 | N | TYR | 1194 | 6.996 | 30.230 | 31.675 | 1.00 | 8.89 | | N |
| ANISOU | 335 | N | TYR | 1194 | 1192 | 739 | 1446 | 88 | -87 | 17 | N |
| ATOM | 336 | CA | TYR | 1194 | 6.232 | 31.172 | 30.853 | 1.00 | 9.37 | | C |
| ANISOU | 336 | CA | TYR | 1194 | 1118 | 987 | 1457 | -86 | -182 | 150 | C |
| ATOM | 337 | CB | TYR | 1194 | 7.203 | 31.994 | 30.038 | 1.00 | 9.58 | | C |
| ANISOU | 337 | CB | TYR | 1194 | 1071 | 913 | 1657 | -105 | -122 | 170 | C |
| ATOM | 338 | CG | TYR | 1194 | 6.528 | 32.970 | 29.073 | 1.00 | 10.84 | | C |
| ANISOU | 338 | CG | TYR | 1194 | 1244 | 1040 | 1835 | 2 | -35 | 354 | C |
| ATOM | 339 | CD1 | TYR | 1194 | 6.197 | 34.242 | 29.576 | 1.00 | 12.01 | | C |
| ANISOU | 339 | CD1 | TYR | 1194 | 1464 | 1044 | 2055 | 144 | 101 | 382 | C |
| ATOM | 340 | CE1 | TYR | 1194 | 5.576 | 35.181 | 28.722 | 1.00 | 13.41 | | C |
| ANISOU | 340 | CE1 | TYR | 1194 | 1614 | 1143 | 2339 | 146 | 130 | 585 | C |
| ATOM | 341 | CD2 | TYR | 1194 | 6.256 | 32.639 | 27.749 | 1.00 | 11.11 | | C |
| ANISOU | 341 | CD2 | TYR | 1194 | 1251 | 1227 | 1745 | -204 | -95 | 459 | C |
| ATOM | 342 | CE2 | TYR | 1194 | 5.647 | 33.568 | 26.909 | 1.00 | 12.83 | | C |
| ANISOU | 342 | CE2 | TYR | 1194 | 1303 | 1639 | 1930 | 57 | -94 | 548 | C |
| ATOM | 343 | CZ | TYR | 1194 | 5.314 | 34.821 | 27.410 | 1.00 | 14.24 | | C |
| ANISOU | 343 | CZ | TYR | 1194 | 1283 | 1648 | 2481 | 303 | -364 | 515 | C |
| ATOM | 344 | OH | TYR | 1194 | 4.730 | 35.731 | 26.592 | 1.00 | 16.96 | | O |
| ANISOU | 344 | OH | TYR | 1194 | 1616 | 2189 | 2640 | 650 | 59 | 995 | O |
| ATOM | 345 | C | TYR | 1194 | 5.235 | 30.436 | 29.977 | 1.00 | 8.98 | | C |
| ANISOU | 345 | C | TYR | 1194 | 908 | 868 | 1636 | -74 | -59 | 67 | C |
| ATOM | 346 | O | TYR | 1194 | 4.049 | 30.785 | 29.944 | 1.00 | 10.09 | | O |
| ANISOU | 346 | O | TYR | 1194 | 912 | 896 | 2025 | 69 | -188 | 77 | O |
| ATOM | 347 | N | LEU | 1195 | 5.679 | 29.407 | 29.260 | 1.00 | 9.32 | | N |
| ANISOU | 347 | N | LEU | 1195 | 1026 | 862 | 1654 | -53 | -113 | 73 | N |
| ATOM | 348 | CA | LEU | 1195 | 4.723 | 28.673 | 28.384 | 1.00 | 9.50 | | C |
| ANISOU | 348 | CA | LEU | 1195 | 1029 | 905 | 1675 | -143 | -88 | 23 | C |
| ATOM | 349 | CB | LEU | 1195 | 5.488 | 27.594 | 27.598 | 1.00 | 9.69 | | C |
| ANISOU | 349 | CB | LEU | 1195 | 1043 | 1047 | 1593 | 51 | -85 | 66 | C |
| ATOM | 350 | CG | LEU | 1195 | 6.436 | 28.134 | 26.519 | 1.00 | 11.10 | | C |
| ANISOU | 350 | CG | LEU | 1195 | 981 | 1490 | 1747 | -21 | -60 | 219 | C |
| ATOM | 351 | CD1 | LEU | 1195 | 7.197 | 26.966 | 25.904 | 1.00 | 12.70 | | C |
| ANISOU | 351 | CD1 | LEU | 1195 | 1421 | 1917 | 1486 | 263 | 34 | 159 | C |
| ATOM | 352 | CD2 | LEU | 1195 | 5.714 | 28.961 | 25.433 | 1.00 | 12.45 | | C |
| ANISOU | 352 | CD2 | LEU | 1195 | 1421 | 1546 | 1763 | -159 | -347 | 311 | C |
| ATOM | 353 | C | LEU | 1195 | 3.586 | 28.075 | 29.162 | 1.00 | 8.80 | | C |
| ANISOU | 353 | C | LEU | 1195 | 812 | 876 | 1656 | 87 | -178 | 163 | C |
| ATOM | 354 | O | LEU | 1195 | 2.438 | 28.124 | 28.757 | 1.00 | 9.90 | | O |
| ANISOU | 354 | O | LEU | 1195 | 837 | 1114 | 1811 | 124 | -250 | 99 | O |
| ATOM | 355 | N | ALA | 1196 | 3.873 | 27.490 | 30.317 | 1.00 | 10.24 | | N |
| ANISOU | 355 | N | ALA | 1196 | 1012 | 1218 | 1660 | -81 | -241 | 287 | N |
| ATOM | 356 | CA | ALA | 1196 | 2.825 | 26.895 | 31.150 | 1.00 | 10.58 | | C |
| ANISOU | 356 | CA | ALA | 1196 | 1186 | 1223 | 1612 | -50 | -99 | 256 | C |
| ATOM | 357 | CB | ALA | 1196 | 3.452 | 26.131 | 32.305 | 1.00 | 11.69 | | C |
| ANISOU | 357 | CB | ALA | 1196 | 1232 | 1381 | 1829 | -203 | -238 | 473 | C |
| ATOM | 358 | C | ALA | 1196 | 1.880 | 27.977 | 31.620 | 1.00 | 11.27 | | C |
| ANISOU | 358 | C | ALA | 1196 | 1150 | 1330 | 1802 | -23 | -61 | 215 | C |
| ATOM | 359 | O | ALA | 1196 | 0.673 | 27.725 | 31.766 | 1.00 | 12.21 | | O |
| ANISOU | 359 | O | ALA | 1196 | 1068 | 1682 | 1889 | -82 | -258 | 437 | O |
| ATOM | 360 | N | SER | 1197 | 2.365 | 29.198 | 31.882 | 1.00 | 11.07 | | N |
| ANISOU | 360 | N | SER | 1197 | 1230 | 1251 | 1725 | 30 | -131 | 169 | N |
| ATOM | 361 | CA | SER | 1197 | 1.512 | 30.299 | 32.338 | 1.00 | 13.17 | | C |
| ANISOU | 361 | CA | SER | 1197 | 1514 | 1472 | 2017 | 260 | -315 | 65 | C |
| ATOM | 362 | CB | SER | 1197 | 2.342 | 31.484 | 32.823 | 1.00 | 13.50 | | C |
| ANISOU | 362 | CB | SER | 1197 | 1466 | 1673 | 1990 | 130 | -71 | -388 | C |
| ATOM | 363 | OG | SER | 1197 | 2.824 | 32.310 | 31.732 | 1.00 | 14.87 | | O |
| ANISOU | 363 | OG | SER | 1197 | 1644 | 1832 | 2175 | -136 | -14 | -369 | O |
| ATOM | 364 | C | SER | 1197 | 0.559 | 30.710 | 31.206 | 1.00 | 12.00 | | C |
| ANISOU | 364 | C | SER | 1197 | 939 | 1600 | 2022 | 74 | -115 | 102 | C |
| ATOM | 365 | O | SER | 1197 | -0.521 | 31.234 | 31.500 | 1.00 | 14.85 | | O |
| ANISOU | 365 | O | SER | 1197 | 1100 | 2130 | 2412 | 279 | -110 | -81 | O |
| ATOM | 366 | N | LYS | 1198 | 0.945 | 30.476 | 29.969 | 1.00 | 10.88 | | N |
| ANISOU | 366 | N | LYS | 1198 | 1132 | 1026 | 1974 | -63 | -141 | 175 | N |
| ATOM | 367 | CA | LYS | 1198 | 0.100 | 30.685 | 28.800 | 1.00 | 11.12 | | C |
| ANISOU | 367 | CA | LYS | 1198 | 1275 | 1010 | 1941 | -42 | -142 | 149 | C |
| ATOM | 368 | CB | LYS | 1198 | 0.980 | 31.129 | 27.631 | 1.00 | 12.15 | | C |
| ANISOU | 368 | CB | LYS | 1198 | 1335 | 1235 | 2045 | -280 | -202 | 391 | C |
| ATOM | 369 | CG | LYS | 1198 | 1.783 | 32.383 | 27.855 | 1.00 | 13.20 | | C |
| ANISOU | 369 | CG | LYS | 1198 | 1313 | 1282 | 2419 | -276 | -211 | 183 | C |
| ATOM | 370 | CD | LYS | 1198 | 0.944 | 33.589 | 28.193 | 1.00 | 19.28 | | C |
| ANISOU | 370 | CD | LYS | 1198 | 1770 | 1267 | 4287 | -30 | -347 | 127 | C |
| ATOM | 371 | CE | LYS | 1198 | 1.883 | 34.780 | 28.433 | 1.00 | 26.75 | | C |
| ANISOU | 371 | CE | LYS | 1198 | 2458 | 1398 | 6306 | -254 | -51 | -598 | C |
| ATOM | 372 | NZ | LYS | 1198 | 1.049 | 35.986 | 28.782 | 1.00 | 35.73 | | N1+ |
| ANISOU | 372 | NZ | LYS | 1198 | 3366 | 1490 | 8720 | -45 | 568 | -920 | N1+ |
| ATOM | 373 | C | LYS | 1198 | -0.702 | 29.435 | 28.453 | 1.00 | 10.31 | | C |
| ANISOU | 373 | C | LYS | 1198 | 1138 | 1057 | 1724 | -10 | -108 | 192 | C |
| ATOM | 374 | O | LYS | 1198 | -1.386 | 29.475 | 27.418 | 1.00 | 11.86 | | O |
| ANISOU | 374 | O | LYS | 1198 | 1175 | 1284 | 2047 | -26 | -368 | 291 | O |
| ATOM | 375 | N | LYS | 1199 | -0.623 | 28.403 | 29.273 | 1.00 | 9.86 | | N |
| ANISOU | 375 | N | LYS | 1199 | 980 | 1060 | 1706 | -87 | -22 | 220 | N |
| ATOM | 376 | CA | LYS | 1199 | -1.400 | 27.161 | 29.120 | 1.00 | 9.82 | | C |
| ANISOU | 376 | CA | LYS | 1199 | 860 | 1069 | 1802 | -36 | -69 | 58 | C |
| ATOM | 377 | CB | LYS | 1199 | -2.908 | 27.423 | 29.220 | 1.00 | 11.06 | | C |
| ANISOU | 377 | CB | LYS | 1199 | 872 | 1665 | 1664 | -212 | -4 | 189 | C |
| ATOM | 378 | CG | LYS | 1199 | -3.217 | 28.025 | 30.584 | 1.00 | 13.03 | | C |
| ANISOU | 378 | CG | LYS | 1199 | 1111 | 1697 | 2144 | -188 | 203 | -240 | C |
| ATOM | 379 | CD | LYS | 1199 | -4.691 | 28.361 | 30.724 | 1.00 | 13.57 | | C |
| ANISOU | 379 | CD | LYS | 1199 | 989 | 1796 | 2371 | -330 | 290 | -38 | C |
| ATOM | 380 | CE | LYS | 1199 | -4.876 | 29.201 | 31.983 | 1.00 | 16.73 | | C |
| ANISOU | 380 | CE | LYS | 1199 | 1158 | 2064 | 3134 | -417 | 609 | -645 | C |
| ATOM | 381 | NZ | LYS | 1199 | -6.336 | 29.651 | 32.041 | 1.00 | 18.90 | | N1+ |
| ANISOU | 381 | NZ | LYS | 1199 | 1165 | 2344 | 3671 | -341 | 882 | -315 | N1+ |
| ATOM | 382 | C | LYS | 1199 | -1.028 | 26.453 | 27.816 | 1.00 | 9.55 | | C |
| ANISOU | 382 | C | LYS | 1199 | 966 | 1087 | 1577 | -74 | -107 | 266 | C |
| ATOM | 383 | O | LYS | 1199 | -1.825 | 25.741 | 27.205 | 1.00 | 10.78 | | O |
| ANISOU | 383 | O | LYS | 1199 | 1110 | 1287 | 1700 | -226 | -67 | 132 | O |
| ATOM | 384 | N | PHE | 1200 | 0.236 | 26.662 | 27.417 | 1.00 | 9.65 | | N |
| ANISOU | 384 | N | PHE | 1200 | 881 | 1046 | 1739 | 59 | -12 | 228 | N |
| ATOM | 385 | CA | PHE | 1200 | 0.783 | 26.007 | 26.224 | 1.00 | 9.51 | | C |
| ANISOU | 385 | CA | PHE | 1200 | 1033 | 914 | 1667 | -38 | -96 | 131 | C |
| ATOM | 386 | CB | PHE | 1200 | 1.677 | 27.031 | 25.481 | 1.00 | 10.90 | | C |
| ANISOU | 386 | CB | PHE | 1200 | 1238 | 1013 | 1890 | -40 | 246 | 70 | C |
| ATOM | 387 | CG | PHE | 1200 | 2.242 | 26.496 | 24.176 | 1.00 | 11.13 | | C |
| ANISOU | 387 | CG | PHE | 1200 | 1111 | 1354 | 1764 | -206 | 0 | 65 | C |
| ATOM | 388 | CD1 | PHE | 1200 | 1.504 | 26.583 | 23.025 | 1.00 | 13.16 | | C |
| ANISOU | 388 | CD1 | PHE | 1200 | 1644 | 1518 | 1837 | -197 | -174 | 97 | C |
| ATOM | 389 | CD2 | PHE | 1200 | 3.520 | 25.916 | 24.164 | 1.00 | 11.71 | | C |
| ANISOU | 389 | CD2 | PHE | 1200 | 1019 | 1445 | 1985 | -237 | 300 | -86 | C |
| ATOM | 390 | CE1 | PHE | 1200 | 2.012 | 26.103 | 21.831 | 1.00 | 14.89 | | C |
| ANISOU | 390 | CE1 | PHE | 1200 | 2050 | 1726 | 1882 | -335 | -70 | -90 | C |
| ATOM | 391 | CE2 | PHE | 1200 | 4.027 | 25.420 | 22.956 | 1.00 | 13.84 | | C |
| ANISOU | 391 | CE2 | PHE | 1200 | 1660 | 1688 | 1910 | -191 | 415 | 38 | C |
| ATOM | 392 | CZ | PHE | 1200 | 3.263 | 25.517 | 21.813 | 1.00 | 17.15 | | C |
| ANISOU | 392 | CZ | PHE | 1200 | 1992 | 2511 | 2013 | -215 | 175 | -275 | C |
| ATOM | 393 | C | PHE | 1200 | 1.545 | 24.755 | 26.564 | 1.00 | 9.79 | | C |
| ANISOU | 393 | C | PHE | 1200 | 975 | 1110 | 1633 | 73 | -176 | 70 | C |
| ATOM | 394 | O | PHE | 1200 | 2.324 | 24.831 | 27.494 | 1.00 | 13.32 | | O |
| ANISOU | 394 | O | PHE | 1200 | 1605 | 1310 | 2145 | 294 | -796 | -144 | O |
| ATOM | 395 | N | VAL | 1201 | 1.350 | 23.665 | 25.846 | 1.00 | 8.69 | | N |
| ANISOU | 395 | N | VAL | 1201 | 837 | 1037 | 1428 | 103 | -10 | 165 | N |
| ATOM | 396 | CA | VAL | 1201 | 2.021 | 22.385 | 26.073 | 1.00 | 8.97 | | C |
| ANISOU | 396 | CA | VAL | 1201 | 730 | 1034 | 1643 | 25 | 69 | 125 | C |
| ATOM | 397 | CB | VAL | 1201 | 0.993 | 21.264 | 26.211 | 1.00 | 8.90 | | C |
| ANISOU | 397 | CB | VAL | 1201 | 768 | 1076 | 1539 | -8 | -56 | 146 | C |
| ATOM | 398 | CG1 | VAL | 1201 | 1.727 | 19.953 | 26.491 | 1.00 | 10.20 | | C |
| ANISOU | 398 | CG1 | VAL | 1201 | 1124 | 979 | 1770 | -35 | -110 | 165 | C |
| ATOM | 399 | CG2 | VAL | 1201 | -0.024 | 21.623 | 27.297 | 1.00 | 10.67 | | C |
| ANISOU | 399 | CG2 | VAL | 1201 | 877 | 1412 | 1764 | -94 | 163 | 160 | C |
| ATOM | 400 | C | VAL | 1201 | 2.971 | 22.140 | 24.921 | 1.00 | 8.28 | | C |
| ANISOU | 400 | C | VAL | 1201 | 765 | 986 | 1396 | 42 | -112 | 55 | C |
| ATOM | 401 | O | VAL | 1201 | 2.552 | 22.135 | 23.781 | 1.00 | 9.87 | | O |
| ANISOU | 401 | O | VAL | 1201 | 853 | 1454 | 1443 | 40 | -199 | 265 | O |
| ATOM | 402 | N | HIS | 1202 | 4.252 | 21.929 | 25.260 | 1.00 | 8.26 | | N |
| ANISOU | 402 | N | HIS | 1202 | 612 | 1041 | 1487 | 9 | -64 | -6 | N |
| ATOM | 403 | CA | HIS | 1202 | 5.267 | 21.752 | 24.232 | 1.00 | 8.05 | | C |
| ANISOU | 403 | CA | HIS | 1202 | 780 | 960 | 1317 | -173 | -40 | 58 | C |
| ATOM | 404 | CB | HIS | 1202 | 6.644 | 22.014 | 24.915 | 1.00 | 8.45 | | C |
| ANISOU | 404 | CB | HIS | 1202 | 573 | 1054 | 1586 | -47 | 55 | -56 | C |
| ATOM | 405 | CG | HIS | 1202 | 7.731 | 22.060 | 23.878 | 1.00 | 8.34 | | C |
| ANISOU | 405 | CG | HIS | 1202 | 778 | 1031 | 1361 | 3 | -2 | 51 | C |
| ATOM | 406 | CD2 | HIS | 1202 | 8.419 | 23.162 | 23.434 | 1.00 | 9.11 | | C |
| ANISOU | 406 | CD2 | HIS | 1202 | 686 | 1054 | 1721 | -130 | 103 | -111 | C |
| ATOM | 407 | ND1 | HIS | 1202 | 8.166 | 20.947 | 23.193 | 1.00 | 8.57 | | N |
| ANISOU | 407 | ND1 | HIS | 1202 | 891 | 933 | 1433 | 31 | -4 | -8 | N |
| ATOM | 408 | CE1 | HIS | 1202 | 9.112 | 21.356 | 22.358 | 1.00 | 9.28 | | C |
| ANISOU | 408 | CE1 | HIS | 1202 | 970 | 1014 | 1542 | -87 | 50 | -69 | C |
| ATOM | 409 | NE2 | HIS | 1202 | 9.294 | 22.675 | 22.478 | 1.00 | 9.03 | | N |
| ANISOU | 409 | NE2 | HIS | 1202 | 874 | 1004 | 1555 | -38 | 112 | 35 | N |
| ATOM | 410 | C | HIS | 1202 | 5.219 | 20.388 | 23.570 | 1.00 | 8.20 | | C |
| ANISOU | 410 | C | HIS | 1202 | 748 | 1076 | 1292 | 4 | -132 | 21 | C |
| ATOM | 411 | O | HIS | 1202 | 5.258 | 20.290 | 22.331 | 1.00 | 9.39 | | O |
| ANISOU | 411 | O | HIS | 1202 | 1006 | 1207 | 1357 | -44 | -143 | -12 | O |
| ATOM | 412 | N | ARG | 1203 | 5.134 | 19.318 | 24.367 | 1.00 | 8.50 | | N |
| ANISOU | 412 | N | ARG | 1203 | 800 | 895 | 1535 | -80 | -189 | 81 | N |
| ATOM | 413 | CA | ARG | 1203 | 5.038 | 17.905 | 23.939 | 1.00 | 8.97 | | C |
| ANISOU | 413 | CA | ARG | 1203 | 904 | 1079 | 1427 | -185 | -179 | -164 | C |
| ATOM | 414 | CB | ARG | 1203 | 3.994 | 17.653 | 22.830 | 1.00 | 9.19 | | C |
| ANISOU | 414 | CB | ARG | 1203 | 992 | 1199 | 1299 | -88 | -196 | -46 | C |
| ATOM | 415 | CG | ARG | 1203 | 2.627 | 18.191 | 23.233 | 1.00 | 11.55 | | C |
| ANISOU | 415 | CG | ARG | 1203 | 803 | 1682 | 1905 | -185 | -267 | -109 | C |
| ATOM | 416 | CD | ARG | 1203 | 1.545 | 17.734 | 22.217 | 1.00 | 15.13 | | C |
| ANISOU | 416 | CD | ARG | 1203 | 1218 | 2704 | 1827 | -315 | -535 | -157 | C |
| ATOM | 417 | NE | ARG | 1203 | 1.821 | 18.378 | 20.941 | 1.00 | 16.96 | | N |
| ANISOU | 417 | NE | ARG | 1203 | 1331 | 2979 | 2136 | -11 | -601 | 285 | N |
| ATOM | 418 | CZ | ARG | 1203 | 1.095 | 18.134 | 19.845 | 1.00 | 19.71 | | C |
| ANISOU | 418 | CZ | ARG | 1203 | 1880 | 3714 | 1895 | 61 | -542 | 136 | C |
| ATOM | 419 | NH1 | ARG | 1203 | 0.085 | 17.273 | 19.920 | 1.00 | 21.13 | | N1+ |
| ANISOU | 419 | NH1 | ARG | 1203 | 2108 | 2943 | 2977 | 183 | -1113 | -537 | N1+ |
| ATOM | 420 | NH2 | ARG | 1203 | 1.455 | 18.790 | 18.758 | 1.00 | 29.90 | | N |
| ANISOU | 420 | NH2 | ARG | 1203 | 4222 | 5014 | 2126 | 135 | -249 | 784 | N |
| ATOM | 421 | C | ARG | 1203 | 6.358 | 17.312 | 23.436 | 1.00 | 9.24 | | C |
| ANISOU | 421 | C | ARG | 1203 | 1016 | 1026 | 1469 | 28 | -226 | 14 | C |
| ATOM | 422 | O | ARG | 1203 | 6.398 | 16.092 | 23.222 | 1.00 | 10.31 | | O |
| ANISOU | 422 | O | ARG | 1203 | 1136 | 1043 | 1738 | -27 | -133 | -12 | O |
| ATOM | 423 | N | ASP | 1204 | 7.418 | 18.077 | 23.261 | 1.00 | 8.51 | | N |
| ANISOU | 423 | N | ASP | 1204 | 881 | 1088 | 1265 | 94 | -140 | -6 | N |
| ATOM | 424 | CA | ASP | 1204 | 8.685 | 17.478 | 22.823 | 1.00 | 8.33 | | C |
| ANISOU | 424 | CA | ASP | 1204 | 865 | 1079 | 1222 | 70 | -222 | -41 | C |
| ATOM | 425 | CB | ASP | 1204 | 8.742 | 17.401 | 21.281 | 1.00 | 9.88 | | C |
| ANISOU | 425 | CB | ASP | 1204 | 1312 | 1223 | 1221 | 8 | -130 | -57 | C |
| ATOM | 426 | CG | ASP | 1204 | 9.837 | 16.494 | 20.766 | 1.00 | 9.76 | | C |
| ANISOU | 426 | CG | ASP | 1204 | 1197 | 1169 | 1342 | -94 | -52 | -136 | C |
| ATOM | 427 | OD1 | ASP | 1204 | 10.528 | 15.776 | 21.515 | 1.00 | 10.39 | | O |
| ANISOU | 427 | OD1 | ASP | 1204 | 1384 | 987 | 1575 | -35 | -18 | -154 | O |
| ATOM | 428 | OD2 | ASP | 1204 | 10.046 | 16.516 | 19.521 | 1.00 | 12.40 | | O1- |
| ANISOU | 428 | OD2 | ASP | 1204 | 1458 | 1799 | 1455 | -48 | -53 | -193 | O1- |
| ATOM | 429 | C | ASP | 1204 | 9.870 | 18.233 | 23.406 | 1.00 | 8.15 | | C |
| ANISOU | 429 | C | ASP | 1204 | 967 | 800 | 1329 | -65 | -33 | -106 | C |
| ATOM | 430 | O | ASP | 1204 | 10.831 | 18.547 | 22.712 | 1.00 | 9.16 | | O |
| ANISOU | 430 | O | ASP | 1204 | 1077 | 1059 | 1346 | -78 | 89 | -126 | O |
| ATOM | 431 | N | LEU | 1205 | 9.803 | 18.514 | 24.721 | 1.00 | 7.83 | | N |
| ANISOU | 431 | N | LEU | 1205 | 948 | 767 | 1260 | 31 | -162 | -19 | N |
| ATOM | 432 | CA | LEU | 1205 | 10.948 | 19.175 | 25.332 | 1.00 | 7.58 | | C |
| ANISOU | 432 | CA | LEU | 1205 | 745 | 879 | 1257 | -27 | -46 | -56 | C |
| ATOM | 433 | CB | LEU | 1205 | 10.526 | 19.716 | 26.691 | 1.00 | 8.62 | | C |
| ANISOU | 433 | CB | LEU | 1205 | 908 | 1196 | 1169 | -77 | -58 | -71 | C |
| ATOM | 434 | CG | LEU | 1205 | 11.597 | 20.490 | 27.458 | 1.00 | 8.64 | | C |
| ANISOU | 434 | CG | LEU | 1205 | 1103 | 898 | 1283 | -162 | -169 | 44 | C |
| ATOM | 435 | CD1 | LEU | 1205 | 12.057 | 21.746 | 26.699 | 1.00 | 10.45 | | C |
| ANISOU | 435 | CD1 | LEU | 1205 | 1488 | 929 | 1555 | -37 | -62 | 245 | C |
| ATOM | 436 | CD2 | LEU | 1205 | 11.065 | 20.883 | 28.815 | 1.00 | 9.85 | | C |
| ANISOU | 436 | CD2 | LEU | 1205 | 1513 | 995 | 1236 | -94 | -157 | -45 | C |
| ATOM | 437 | C | LEU | 1205 | 12.103 | 18.187 | 25.445 | 1.00 | 7.78 | | C |
| ANISOU | 437 | C | LEU | 1205 | 847 | 915 | 1195 | -77 | -48 | -69 | C |
| ATOM | 438 | O | LEU | 1205 | 11.929 | 17.053 | 25.896 | 1.00 | 8.78 | | O |
| ANISOU | 438 | O | LEU | 1205 | 1016 | 969 | 1353 | 69 | -6 | 129 | O |
| ATOM | 439 | N | ALA | 1206 | 13.284 | 18.613 | 25.050 | 1.00 | 7.99 | | N |
| ANISOU | 439 | N | ALA | 1206 | 763 | 987 | 1284 | 85 | -59 | -66 | N |
| ATOM | 440 | CA | ALA | 1206 | 14.508 | 17.815 | 25.031 | 1.00 | 7.84 | | C |
| ANISOU | 440 | CA | ALA | 1206 | 825 | 920 | 1233 | 64 | 57 | -173 | C |
| ATOM | 441 | CB | ALA | 1206 | 14.422 | 16.697 | 23.983 | 1.00 | 8.71 | | C |
| ANISOU | 441 | CB | ALA | 1206 | 990 | 977 | 1343 | 33 | -67 | -280 | C |
| ATOM | 442 | C | ALA | 1206 | 15.639 | 18.765 | 24.744 | 1.00 | 7.56 | | C |
| ANISOU | 442 | C | ALA | 1206 | 886 | 909 | 1079 | -19 | -202 | -64 | C |
| ATOM | 443 | O | ALA | 1206 | 15.400 | 19.828 | 24.142 | 1.00 | 8.39 | | O |
| ANISOU | 443 | O | ALA | 1206 | 949 | 946 | 1291 | 79 | -133 | 30 | O |
| ATOM | 444 | N | ALA | 1207 | 16.887 | 18.448 | 25.103 | 1.00 | 7.51 | | N |
| ANISOU | 444 | N | ALA | 1207 | 791 | 913 | 1151 | 4 | -19 | -36 | N |
| ATOM | 445 | CA | ALA | 1207 | 17.985 | 19.371 | 24.821 | 1.00 | 8.23 | | C |
| ANISOU | 445 | CA | ALA | 1207 | 822 | 1046 | 1260 | -65 | -204 | 138 | C |
| ATOM | 446 | CB | ALA | 1207 | 19.280 | 18.819 | 25.417 | 1.00 | 8.64 | | C |
| ANISOU | 446 | CB | ALA | 1207 | 827 | 1297 | 1158 | 101 | -87 | -18 | C |
| ATOM | 447 | C | ALA | 1207 | 18.126 | 19.644 | 23.334 | 1.00 | 7.72 | | C |
| ANISOU | 447 | C | ALA | 1207 | 837 | 823 | 1272 | 44 | -33 | 78 | C |
| ATOM | 448 | O | ALA | 1207 | 18.480 | 20.756 | 22.948 | 1.00 | 8.46 | | O |
| ANISOU | 448 | O | ALA | 1207 | 884 | 977 | 1354 | -138 | -244 | 158 | O |
| ATOM | 449 | N | ARG | 1208 | 17.854 | 18.654 | 22.450 | 1.00 | 8.02 | | N |
| ANISOU | 449 | N | ARG | 1208 | 882 | 896 | 1271 | 81 | -93 | -24 | N |
| ATOM | 450 | CA | ARG | 1208 | 17.970 | 18.859 | 21.024 | 1.00 | 8.31 | | C |
| ANISOU | 450 | CA | ARG | 1208 | 972 | 914 | 1273 | 137 | -19 | -8 | C |
| ATOM | 451 | CB | ARG | 1208 | 17.704 | 17.520 | 20.279 | 1.00 | 9.14 | | C |
| ANISOU | 451 | CB | ARG | 1208 | 1153 | 1034 | 1287 | 87 | 55 | -123 | C |
| ATOM | 452 | CG | ARG | 1208 | 16.295 | 16.977 | 20.491 | 1.00 | 8.97 | | C |
| ANISOU | 452 | CG | ARG | 1208 | 1218 | 1107 | 1082 | -22 | 28 | -102 | C |
| ATOM | 453 | CD | ARG | 1208 | 16.010 | 15.687 | 19.637 | 1.00 | 10.16 | | C |
| ANISOU | 453 | CD | ARG | 1208 | 1579 | 861 | 1420 | 47 | 55 | -148 | C |
| ATOM | 454 | NE | ARG | 1208 | 14.668 | 15.224 | 20.123 | 1.00 | 10.44 | | N |
| ANISOU | 454 | NE | ARG | 1208 | 1579 | 945 | 1442 | -97 | -145 | 64 | N |
| ATOM | 455 | CZ | ARG | 1208 | 14.463 | 14.444 | 21.146 | 1.00 | 9.46 | | C |
| ANISOU | 455 | CZ | ARG | 1208 | 1314 | 609 | 1671 | -164 | -283 | -37 | C |
| ATOM | 456 | NH1 | ARG | 1208 | 15.533 | 13.958 | 21.837 | 1.00 | 9.90 | | N1+ |
| ANISOU | 456 | NH1 | ARG | 1208 | 1388 | 1079 | 1296 | -20 | -196 | -48 | N1+ |
| ATOM | 457 | NH2 | ARG | 1208 | 13.214 | 14.133 | 21.501 | 1.00 | 11.17 | | N |
| ANISOU | 457 | NH2 | ARG | 1208 | 1318 | 1052 | 1874 | -312 | -143 | -132 | N |
| ATOM | 458 | C | ARG | 1208 | 17.012 | 19.904 | 20.500 | 1.00 | 8.35 | | C |
| ANISOU | 458 | C | ARG | 1208 | 878 | 941 | 1352 | 12 | 29 | 149 | C |
| ATOM | 459 | O | ARG | 1208 | 17.207 | 20.425 | 19.402 | 1.00 | 8.95 | | O |
| ANISOU | 459 | O | ARG | 1208 | 1118 | 1110 | 1172 | 73 | 14 | 13 | O |
| ATOM | 460 | N | ASN | 1209 | 15.949 | 20.212 | 21.296 | 1.00 | 7.95 | | N |
| ANISOU | 460 | N | ASN | 1209 | 938 | 854 | 1228 | 60 | -37 | 4 | N |
| ATOM | 461 | CA | ASN | 1209 | 14.881 | 21.099 | 20.862 | 1.00 | 8.64 | | C |
| ANISOU | 461 | CA | ASN | 1209 | 851 | 894 | 1538 | 37 | -232 | -102 | C |
| ATOM | 462 | CB | ASN | 1209 | 13.519 | 20.416 | 21.139 | 1.00 | 8.68 | | C |
| ANISOU | 462 | CB | ASN | 1209 | 966 | 935 | 1395 | -38 | 5 | -217 | C |
| ATOM | 463 | CG | ASN | 1209 | 13.264 | 19.300 | 20.134 | 1.00 | 8.67 | | C |
| ANISOU | 463 | CG | ASN | 1209 | 1028 | 966 | 1301 | 3 | -156 | -154 | C |
| ATOM | 464 | OD1 | ASN | 1209 | 13.698 | 19.404 | 18.971 | 1.00 | 10.43 | | O |
| ANISOU | 464 | OD1 | ASN | 1209 | 1434 | 1224 | 1306 | -224 | -74 | -219 | O |
| ATOM | 465 | ND2 | ASN | 1209 | 12.593 | 18.252 | 20.582 | 1.00 | 9.67 | | N |
| ANISOU | 465 | ND2 | ASN | 1209 | 1118 | 1017 | 1539 | -120 | -59 | -146 | N |
| ATOM | 466 | C | ASN | 1209 | 14.958 | 22.490 | 21.525 | 1.00 | 7.70 | | C |
| ANISOU | 466 | C | ASN | 1209 | 1021 | 816 | 1087 | 43 | -230 | 29 | C |
| ATOM | 467 | O | ASN | 1209 | 13.991 | 23.253 | 21.454 | 1.00 | 8.89 | | O |
| ANISOU | 467 | O | ASN | 1209 | 1018 | 907 | 1453 | 95 | -116 | 0 | O |
| ATOM | 468 | N | CYS | 1210 | 16.102 | 22.774 | 22.131 | 1.00 | 7.98 | | N |
| ANISOU | 468 | N | CYS | 1210 | 1022 | 846 | 1163 | -64 | -172 | 22 | N |
| ATOM | 469 | CA | CYS | 1210 | 16.409 | 24.089 | 22.689 | 1.00 | 7.90 | | C |
| ANISOU | 469 | CA | CYS | 1210 | 965 | 881 | 1157 | -100 | -60 | -82 | C |
| ATOM | 470 | CB | CYS | 1210 | 16.730 | 24.063 | 24.188 | 1.00 | 8.44 | | C |
| ANISOU | 470 | CB | CYS | 1210 | 1084 | 1012 | 1110 | -129 | -45 | 51 | C |
| ATOM | 471 | SG | CYS | 1210 | 15.336 | 23.400 | 25.174 | 1.00 | 9.02 | | S |
| ANISOU | 471 | SG | CYS | 1210 | 1127 | 996 | 1304 | -70 | -28 | 34 | S |
| ATOM | 472 | C | CYS | 1210 | 17.616 | 24.640 | 21.940 | 1.00 | 7.69 | | C |
| ANISOU | 472 | C | CYS | 1210 | 911 | 922 | 1088 | -24 | -108 | -15 | C |
| ATOM | 473 | O | CYS | 1210 | 18.462 | 23.813 | 21.570 | 1.00 | 8.85 | | O |
| ANISOU | 473 | O | CYS | 1210 | 924 | 1014 | 1424 | 91 | 16 | 106 | O |
| ATOM | 474 | N | MET | 1211 | 17.671 | 25.946 | 21.748 | 1.00 | 8.01 | | N |
| ANISOU | 474 | N | MET | 1211 | 890 | 894 | 1261 | -52 | 40 | 11 | N |
| ATOM | 475 | CA | MET | 1211 | 18.786 | 26.581 | 21.036 | 1.00 | 8.30 | | C |
| ANISOU | 475 | CA | MET | 1211 | 953 | 1059 | 1141 | -136 | -99 | 99 | C |
| ATOM | 476 | CB | MET | 1211 | 18.263 | 27.341 | 19.806 | 1.00 | 9.78 | | C |
| ANISOU | 476 | CB | MET | 1211 | 1426 | 935 | 1355 | -94 | -266 | 182 | C |
| ATOM | 477 | CG | MET | 1211 | 17.450 | 26.482 | 18.851 | 1.00 | 9.86 | | C |
| ANISOU | 477 | CG | MET | 1211 | 1203 | 1205 | 1337 | -39 | -188 | -64 | C |
| ATOM | 478 | SD | MET | 1211 | 18.419 | 25.175 | 18.068 | 1.00 | 10.93 | | S |
| ANISOU | 478 | SD | MET | 1211 | 1372 | 1318 | 1462 | 111 | -138 | -36 | S |
| ATOM | 479 | CE | MET | 1211 | 17.287 | 23.785 | 18.116 | 1.00 | 13.56 | | C |
| ANISOU | 479 | CE | MET | 1211 | 1959 | 1293 | 1902 | -163 | -462 | -123 | C |
| ATOM | 480 | C | MET | 1211 | 19.529 | 27.531 | 21.945 | 1.00 | 8.50 | | C |
| ANISOU | 480 | C | MET | 1211 | 903 | 1025 | 1303 | -31 | -21 | -9 | C |
| ATOM | 481 | O | MET | 1211 | 19.002 | 27.946 | 22.983 | 1.00 | 10.04 | | O |
| ANISOU | 481 | O | MET | 1211 | 1000 | 1342 | 1474 | -2 | -35 | -371 | O |
| ATOM | 482 | N | LEU | 1212 | 20.765 | 27.865 | 21.554 | 1.00 | 9.06 | | N |
| ANISOU | 482 | N | LEU | 1212 | 1026 | 1033 | 1385 | -221 | -41 | 45 | N |
| ATOM | 483 | CA | LEU | 1212 | 21.597 | 28.737 | 22.391 | 1.00 | 10.03 | | C |
| ANISOU | 483 | CA | LEU | 1212 | 1156 | 1213 | 1442 | -256 | -132 | -87 | C |
| ATOM | 484 | CB | LEU | 1212 | 22.705 | 27.878 | 23.035 | 1.00 | 13.63 | | C |
| ANISOU | 484 | CB | LEU | 1212 | 1011 | 1958 | 2211 | 237 | -266 | -304 | C |
| ATOM | 485 | CG | LEU | 1212 | 23.637 | 28.423 | 24.087 | 1.00 | 15.76 | | C |
| ANISOU | 485 | CG | LEU | 1212 | 1424 | 2489 | 2077 | 368 | -418 | -348 | C |
| ATOM | 486 | CD1 | LEU | 1212 | 22.863 | 28.789 | 25.349 | 1.00 | 15.92 | | C |
| ANISOU | 486 | CD1 | LEU | 1212 | 1866 | 2489 | 1695 | -122 | -140 | 207 | C |
| ATOM | 487 | CD2 | LEU | 1212 | 24.717 | 27.429 | 24.478 | 1.00 | 16.72 | | C |
| ANISOU | 487 | CD2 | LEU | 1212 | 1874 | 2414 | 2065 | 231 | -606 | 407 | C |
| ATOM | 488 | C | LEU | 1212 | 22.142 | 29.882 | 21.552 | 1.00 | 10.90 | | C |
| ANISOU | 488 | C | LEU | 1212 | 1049 | 1456 | 1636 | -516 | 100 | -268 | C |
| ATOM | 489 | O | LEU | 1212 | 22.746 | 29.611 | 20.516 | 1.00 | 12.77 | | O |
| ANISOU | 489 | O | LEU | 1212 | 1273 | 1775 | 1805 | -560 | 323 | -209 | O |
| ATOM | 490 | N | ASP | 1213 | 21.951 | 31.117 | 21.979 | 1.00 | 11.62 | | N |
| ANISOU | 490 | N | ASP | 1213 | 1331 | 1292 | 1792 | -419 | -136 | -64 | N |
| ATOM | 491 | CA | ASP | 1213 | 22.420 | 32.266 | 21.164 | 1.00 | 12.65 | | C |
| ANISOU | 491 | CA | ASP | 1213 | 1574 | 1373 | 1860 | -431 | 146 | -36 | C |
| ATOM | 492 | CB | ASP | 1213 | 21.380 | 33.379 | 21.233 | 1.00 | 13.17 | | C |
| ANISOU | 492 | CB | ASP | 1213 | 1760 | 1411 | 1833 | -273 | -87 | -12 | C |
| ATOM | 493 | CG | ASP | 1213 | 21.315 | 34.180 | 22.519 | 1.00 | 12.24 | | C |
| ANISOU | 493 | CG | ASP | 1213 | 1512 | 1333 | 1805 | -256 | 13 | 75 | C |
| ATOM | 494 | OD1 | ASP | 1213 | 22.175 | 34.055 | 23.416 | 1.00 | 12.12 | | O |
| ANISOU | 494 | OD1 | ASP | 1213 | 1625 | 1254 | 1725 | -294 | 54 | 9 | O |
| ATOM | 495 | OD2 | ASP | 1213 | 20.355 | 34.995 | 22.642 | 1.00 | 14.98 | | O1- |
| ANISOU | 495 | OD2 | ASP | 1213 | 1920 | 1420 | 2351 | 19 | 32 | -35 | O1- |
| ATOM | 496 | C | ASP | 1213 | 23.804 | 32.701 | 21.594 | 1.00 | 13.67 | | C |
| ANISOU | 496 | C | ASP | 1213 | 1627 | 1406 | 2160 | -495 | 70 | 5 | C |
| ATOM | 497 | O | ASP | 1213 | 24.487 | 32.112 | 22.424 | 1.00 | 13.82 | | O |
| ANISOU | 497 | O | ASP | 1213 | 1410 | 1557 | 2282 | -255 | 184 | -96 | O |
| ATOM | 498 | N | GLU | 1214 | 24.252 | 33.811 | 20.976 | 1.00 | 14.40 | | N |
| ANISOU | 498 | N | GLU | 1214 | 2006 | 1555 | 1911 | -843 | 182 | -164 | N |
| ATOM | 499 | CA | GLU | 1214 | 25.646 | 34.223 | 21.170 | 1.00 | 15.62 | | C |
| ANISOU | 499 | CA | GLU | 1214 | 1872 | 1843 | 2218 | -824 | 393 | -273 | C |
| ATOM | 500 | CB | GLU | 1214 | 25.995 | 35.297 | 20.115 | 1.00 | 21.19 | | C |
| ANISOU | 500 | CB | GLU | 1214 | 2885 | 2457 | 2710 | -1232 | 895 | 76 | C |
| ATOM | 501 | CG | GLU | 1214 | 25.488 | 36.672 | 20.466 | 1.00 | 25.38 | | C |
| ANISOU | 501 | CG | GLU | 1214 | 4622 | 2231 | 2789 | -893 | 588 | 425 | C |
| ATOM | 502 | CD | GLU | 1214 | 24.041 | 36.878 | 20.079 | 1.00 | 31.60 | | C |
| ANISOU | 502 | CD | GLU | 1214 | 4746 | 3140 | 4121 | 178 | 586 | -131 | C |
| ATOM | 503 | OE1 | GLU | 1214 | 23.623 | 38.060 | 19.972 | 1.00 | 55.89 | | O1- |
| ANISOU | 503 | OE1 | GLU | 1214 | 7816 | 4393 | 9025 | 2739 | 465 | -1249 | O1- |
| ATOM | 504 | OE2 | GLU | 1214 | 23.347 | 35.869 | 19.889 | 1.00 | 42.70 | | O |
| ANISOU | 504 | OE2 | GLU | 1214 | 4924 | 5355 | 5944 | -1189 | -279 | -923 | O |
| ATOM | 505 | C | GLU | 1214 | 25.921 | 34.728 | 22.573 | 1.00 | 15.46 | | C |
| ANISOU | 505 | C | GLU | 1214 | 1486 | 1966 | 2423 | -638 | 235 | -488 | C |
| ATOM | 506 | O | GLU | 1214 | 27.115 | 34.824 | 22.956 | 1.00 | 17.86 | | O |
| ANISOU | 506 | O | GLU | 1214 | 1554 | 2303 | 2931 | -681 | 149 | -569 | O |
| ATOM | 507 | N | LYS | 1215 | 24.903 | 35.052 | 23.340 | 1.00 | 13.55 | | N |
| ANISOU | 507 | N | LYS | 1215 | 1606 | 1388 | 2154 | -368 | 167 | -147 | N |
| ATOM | 508 | CA | LYS | 1215 | 25.049 | 35.440 | 24.752 | 1.00 | 13.47 | | C |
| ANISOU | 508 | CA | LYS | 1215 | 1539 | 1432 | 2145 | -288 | -38 | -115 | C |
| ATOM | 509 | CB | LYS | 1215 | 24.091 | 36.594 | 25.093 | 1.00 | 15.71 | | C |
| ANISOU | 509 | CB | LYS | 1215 | 2050 | 1671 | 2247 | -78 | 255 | -192 | C |
| ATOM | 510 | CG | LYS | 1215 | 24.427 | 37.817 | 24.234 | 1.00 | 22.20 | | C |
| ANISOU | 510 | CG | LYS | 1215 | 3128 | 1481 | 3825 | 65 | 607 | 217 | C |
| ATOM | 511 | CD | LYS | 1215 | 23.463 | 38.948 | 24.570 | 1.00 | 25.70 | | C |
| ANISOU | 511 | CD | LYS | 1215 | 3764 | 2273 | 3727 | 952 | 231 | 323 | C |
| ATOM | 512 | CE | LYS | 1215 | 23.947 | 40.229 | 23.889 | 1.00 | 28.08 | | C |
| ANISOU | 512 | CE | LYS | 1215 | 4177 | 2139 | 4353 | 1324 | 167 | 675 | C |
| ATOM | 513 | NZ | LYS | 1215 | 22.996 | 41.318 | 24.282 | 1.00 | 33.64 | | N1+ |
| ANISOU | 513 | NZ | LYS | 1215 | 5337 | 2734 | 4710 | 2102 | 636 | 789 | N1+ |
| ATOM | 514 | C | LYS | 1215 | 24.802 | 34.275 | 25.709 | 1.00 | 12.61 | | C |
| ANISOU | 514 | C | LYS | 1215 | 1099 | 1551 | 2141 | -591 | 95 | -133 | C |
| ATOM | 515 | O | LYS | 1215 | 24.725 | 34.464 | 26.927 | 1.00 | 14.30 | | O |
| ANISOU | 515 | O | LYS | 1215 | 1611 | 1719 | 2103 | -355 | -114 | -116 | O |
| ATOM | 516 | N | PHE | 1216 | 24.692 | 33.063 | 25.167 | 1.00 | 12.96 | | N |
| ANISOU | 516 | N | PHE | 1216 | 1206 | 1457 | 2260 | -504 | -243 | -69 | N |
| ATOM | 517 | CA | PHE | 1216 | 24.436 | 31.824 | 25.908 | 1.00 | 13.16 | | C |
| ANISOU | 517 | CA | PHE | 1216 | 1140 | 1515 | 2346 | -170 | -204 | 218 | C |
| ATOM | 518 | CB | PHE | 1216 | 25.569 | 31.516 | 26.895 | 1.00 | 15.70 | | C |
| ANISOU | 518 | CB | PHE | 1216 | 1201 | 2058 | 2707 | -161 | -467 | 46 | C |
| ATOM | 519 | CG | PHE | 1216 | 26.891 | 31.449 | 26.127 | 1.00 | 17.27 | | C |
| ANISOU | 519 | CG | PHE | 1216 | 1156 | 2576 | 2829 | -321 | -414 | -411 | C |
| ATOM | 520 | CD1 | PHE | 1216 | 27.208 | 30.377 | 25.303 | 1.00 | 19.84 | | C |
| ANISOU | 520 | CD1 | PHE | 1216 | 1545 | 2210 | 3783 | -190 | -90 | -450 | C |
| ATOM | 521 | CD2 | PHE | 1216 | 27.808 | 32.490 | 26.240 | 1.00 | 18.93 | | C |
| ANISOU | 521 | CD2 | PHE | 1216 | 1561 | 2467 | 3166 | -531 | 25 | -375 | C |
| ATOM | 522 | CE1 | PHE | 1216 | 28.408 | 30.319 | 24.593 | 1.00 | 20.84 | | C |
| ANISOU | 522 | CE1 | PHE | 1216 | 1873 | 2345 | 3701 | -152 | 202 | -370 | C |
| ATOM | 523 | CE2 | PHE | 1216 | 29.030 | 32.455 | 25.542 | 1.00 | 21.11 | | C |
| ANISOU | 523 | CE2 | PHE | 1216 | 1538 | 2657 | 3825 | -400 | 237 | -531 | C |
| ATOM | 524 | CZ | PHE | 1216 | 29.326 | 31.366 | 24.720 | 1.00 | 21.59 | | C |
| ANISOU | 524 | CZ | PHE | 1216 | 1842 | 2564 | 3796 | -294 | 237 | -448 | C |
| ATOM | 525 | C | PHE | 1216 | 23.079 | 31.886 | 26.620 | 1.00 | 12.67 | | C |
| ANISOU | 525 | C | PHE | 1216 | 1148 | 1864 | 1801 | -282 | -339 | 80 | C |
| ATOM | 526 | O | PHE | 1216 | 22.887 | 31.344 | 27.697 | 1.00 | 13.17 | | O |
| ANISOU | 526 | O | PHE | 1216 | 1345 | 1716 | 1942 | -203 | -295 | 174 | O |
| ATOM | 527 | N | THR | 1217 | 22.139 | 32.577 | 25.942 | 1.00 | 11.34 | | N |
| ANISOU | 527 | N | THR | 1217 | 1117 | 1405 | 1785 | -227 | -235 | -109 | N |
| ATOM | 528 | CA | THR | 1217 | 20.748 | 32.489 | 26.326 | 1.00 | 11.25 | | C |
| ANISOU | 528 | CA | THR | 1217 | 1099 | 1214 | 1962 | -306 | -189 | -135 | C |
| ATOM | 529 | CB | THR | 1217 | 19.964 | 33.743 | 25.918 | 1.00 | 13.02 | | C |
| ANISOU | 529 | CB | THR | 1217 | 1167 | 1141 | 2640 | -295 | -151 | 17 | C |
| ATOM | 530 | OG1 | THR | 1217 | 20.534 | 34.910 | 26.615 | 1.00 | 17.48 | | O |
| ANISOU | 530 | OG1 | THR | 1217 | 1641 | 1318 | 3683 | -382 | -414 | -354 | O |
| ATOM | 531 | CG2 | THR | 1217 | 18.511 | 33.697 | 26.348 | 1.00 | 12.25 | | C |
| ANISOU | 531 | CG2 | THR | 1217 | 1251 | 1291 | 2111 | -139 | 22 | -72 | C |
| ATOM | 532 | C | THR | 1217 | 20.134 | 31.272 | 25.660 | 1.00 | 9.65 | | C |
| ANISOU | 532 | C | THR | 1217 | 1056 | 1138 | 1474 | -192 | -75 | -12 | C |
| ATOM | 533 | O | THR | 1217 | 20.224 | 31.123 | 24.451 | 1.00 | 10.32 | | O |
| ANISOU | 533 | O | THR | 1217 | 1177 | 1239 | 1504 | -176 | -86 | 0 | O |
| ATOM | 534 | N | VAL | 1218 | 19.507 | 30.431 | 26.484 | 1.00 | 9.35 | | N |
| ANISOU | 534 | N | VAL | 1218 | 1051 | 1058 | 1444 | -168 | -235 | 24 | N |
| ATOM | 535 | CA | VAL | 1218 | 18.792 | 29.248 | 25.986 | 1.00 | 9.25 | | C |
| ANISOU | 535 | CA | VAL | 1218 | 982 | 878 | 1655 | -27 | -247 | 40 | C |
| ATOM | 536 | CB | VAL | 1218 | 18.754 | 28.133 | 27.040 | 1.00 | 9.89 | | C |
| ANISOU | 536 | CB | VAL | 1218 | 1344 | 996 | 1418 | -61 | -137 | 65 | C |
| ATOM | 537 | CG1 | VAL | 1218 | 18.078 | 26.863 | 26.439 | 1.00 | 11.25 | | C |
| ANISOU | 537 | CG1 | VAL | 1218 | 1510 | 1138 | 1626 | -369 | 123 | 59 | C |
| ATOM | 538 | CG2 | VAL | 1218 | 20.128 | 27.780 | 27.554 | 1.00 | 12.65 | | C |
| ANISOU | 538 | CG2 | VAL | 1218 | 1693 | 1280 | 1833 | 55 | -536 | 232 | C |
| ATOM | 539 | C | VAL | 1218 | 17.368 | 29.612 | 25.619 | 1.00 | 7.89 | | C |
| ANISOU | 539 | C | VAL | 1218 | 870 | 833 | 1295 | -12 | -6 | -52 | C |
| ATOM | 540 | O | VAL | 1218 | 16.692 | 30.239 | 26.418 | 1.00 | 10.44 | | O |
| ANISOU | 540 | O | VAL | 1218 | 1137 | 1341 | 1489 | -71 | 79 | -310 | O |
| ATOM | 541 | N | LYS | 1219 | 16.969 | 29.195 | 24.425 | 1.00 | 8.44 | | N |
| ANISOU | 541 | N | LYS | 1219 | 834 | 1019 | 1352 | -121 | -40 | -95 | N |
| ATOM | 542 | CA | LYS | 1219 | 15.569 | 29.383 | 23.995 | 1.00 | 9.19 | | C |
| ANISOU | 542 | CA | LYS | 1219 | 890 | 1015 | 1587 | -91 | -152 | -186 | C |
| ATOM | 543 | CB | LYS | 1219 | 15.514 | 30.233 | 22.731 | 1.00 | 12.40 | | C |
| ANISOU | 543 | CB | LYS | 1219 | 1210 | 1276 | 2223 | -239 | -501 | 362 | C |
| ATOM | 544 | CG | LYS | 1219 | 16.165 | 31.598 | 23.150 | 1.00 | 22.15 | | C |
| ANISOU | 544 | CG | LYS | 1219 | 2301 | 1279 | 4838 | -814 | 116 | 300 | C |
| ATOM | 545 | CD | LYS | 1219 | 15.729 | 32.750 | 22.403 | 1.00 | 20.39 | | C |
| ANISOU | 545 | CD | LYS | 1219 | 2107 | 1248 | 4393 | -473 | 65 | -144 | C |
| ATOM | 546 | CE | LYS | 1219 | 16.685 | 33.919 | 22.727 | 1.00 | 15.19 | | C |
| ANISOU | 546 | CE | LYS | 1219 | 2066 | 1127 | 2578 | -496 | 556 | -9 | C |
| ATOM | 547 | NZ | LYS | 1219 | 15.983 | 34.885 | 23.611 | 1.00 | 12.39 | | N1+ |
| ANISOU | 547 | NZ | LYS | 1219 | 1581 | 1505 | 1621 | -25 | 29 | 277 | N1+ |
| ATOM | 548 | C | LYS | 1219 | 14.948 | 28.031 | 23.765 | 1.00 | 8.58 | | C |
| ANISOU | 548 | C | LYS | 1219 | 888 | 1007 | 1366 | -69 | -151 | -139 | C |
| ATOM | 549 | O | LYS | 1219 | 15.477 | 27.209 | 22.996 | 1.00 | 9.41 | | O |
| ANISOU | 549 | O | LYS | 1219 | 1008 | 1061 | 1507 | -29 | 10 | -168 | O |
| ATOM | 550 | N | VAL | 1220 | 13.817 | 27.787 | 24.424 | 1.00 | 8.56 | | N |
| ANISOU | 550 | N | VAL | 1220 | 896 | 944 | 1415 | -25 | -140 | 91 | N |
| ATOM | 551 | CA | VAL | 1220 | 12.976 | 26.631 | 24.091 | 1.00 | 8.95 | | C |
| ANISOU | 551 | CA | VAL | 1220 | 1058 | 1062 | 1281 | -184 | -148 | 116 | C |
| ATOM | 552 | CB | VAL | 1220 | 11.800 | 26.498 | 25.067 | 1.00 | 9.18 | | C |
| ANISOU | 552 | CB | VAL | 1220 | 1095 | 1171 | 1223 | -171 | -82 | 79 | C |
| ATOM | 553 | CG1 | VAL | 1220 | 10.862 | 25.383 | 24.638 | 1.00 | 10.40 | | C |
| ANISOU | 553 | CG1 | VAL | 1220 | 1225 | 1186 | 1541 | -313 | -185 | 271 | C |
| ATOM | 554 | CG2 | VAL | 1220 | 12.339 | 26.259 | 26.507 | 1.00 | 10.54 | | C |
| ANISOU | 554 | CG2 | VAL | 1220 | 1542 | 1195 | 1266 | -49 | -273 | 303 | C |
| ATOM | 555 | C | VAL | 1220 | 12.512 | 26.846 | 22.676 | 1.00 | 8.31 | | C |
| ANISOU | 555 | C | VAL | 1220 | 957 | 950 | 1249 | 29 | -101 | 37 | C |
| ATOM | 556 | O | VAL | 1220 | 11.917 | 27.897 | 22.370 | 1.00 | 9.16 | | O |
| ANISOU | 556 | O | VAL | 1220 | 1224 | 872 | 1384 | 93 | -109 | 94 | O |
| ATOM | 557 | N | ALA | 1221 | 12.762 | 25.896 | 21.785 | 1.00 | 8.29 | | N |
| ANISOU | 557 | N | ALA | 1221 | 934 | 963 | 1253 | 14 | -153 | 47 | N |
| ATOM | 558 | CA | ALA | 1221 | 12.457 | 26.025 | 20.371 | 1.00 | 8.20 | | C |
| ANISOU | 558 | CA | ALA | 1221 | 973 | 956 | 1188 | -83 | -107 | -31 | C |
| ATOM | 559 | CB | ALA | 1221 | 13.744 | 26.054 | 19.557 | 1.00 | 9.29 | | C |
| ANISOU | 559 | CB | ALA | 1221 | 1045 | 1024 | 1460 | 29 | 17 | 109 | C |
| ATOM | 560 | C | ALA | 1221 | 11.494 | 24.901 | 19.979 | 1.00 | 8.97 | | C |
| ANISOU | 560 | C | ALA | 1221 | 1151 | 1011 | 1247 | -162 | -187 | 131 | C |
| ATOM | 561 | O | ALA | 1221 | 10.791 | 24.379 | 20.854 | 1.00 | 9.47 | | O |
| ANISOU | 561 | O | ALA | 1221 | 1172 | 1067 | 1358 | -169 | -133 | 140 | O |
| ATOM | 562 | N | ASP | 1222 | 11.430 | 24.531 | 18.709 | 1.00 | 9.14 | | N |
| ANISOU | 562 | N | ASP | 1222 | 1233 | 980 | 1258 | -45 | -271 | -65 | N |
| ATOM | 563 | CA | ASP | 1222 | 10.669 | 23.351 | 18.299 | 1.00 | 9.81 | | C |
| ANISOU | 563 | CA | ASP | 1222 | 1107 | 1014 | 1605 | -113 | -209 | 0 | C |
| ATOM | 564 | CB | ASP | 1222 | 11.309 | 22.059 | 18.844 | 1.00 | 10.51 | | C |
| ANISOU | 564 | CB | ASP | 1222 | 1228 | 987 | 1779 | 71 | -56 | 22 | C |
| ATOM | 565 | CG | ASP | 1222 | 10.606 | 20.850 | 18.310 | 1.00 | 11.12 | | C |
| ANISOU | 565 | CG | ASP | 1222 | 1573 | 1064 | 1589 | 85 | -305 | 33 | C |
| ATOM | 566 | OD1 | ASP | 1222 | 10.236 | 20.848 | 17.133 | 1.00 | 12.27 | | O |
| ANISOU | 566 | OD1 | ASP | 1222 | 1786 | 1256 | 1620 | 159 | -236 | 16 | O |
| ATOM | 567 | OD2 | ASP | 1222 | 10.381 | 19.934 | 19.132 | 1.00 | 10.87 | | O1- |
| ANISOU | 567 | OD2 | ASP | 1222 | 1495 | 1099 | 1534 | -134 | -149 | -28 | O1- |
| ATOM | 568 | C | ASP | 1222 | 9.227 | 23.398 | 18.765 | 1.00 | 9.36 | | C |
| ANISOU | 568 | C | ASP | 1222 | 1104 | 1166 | 1286 | 9 | -188 | 52 | C |
| ATOM | 569 | O | ASP | 1222 | 8.670 | 22.447 | 19.286 | 1.00 | 10.04 | | O |
| ANISOU | 569 | O | ASP | 1222 | 1166 | 1153 | 1497 | -107 | -211 | -7 | O |
| ATOM | 570 | N | PHE | 1223 | 8.554 | 24.546 | 18.566 | 1.00 | 9.78 | | N |
| ANISOU | 570 | N | PHE | 1223 | 1249 | 1167 | 1301 | 19 | -301 | -58 | N |
| ATOM | 571 | CA | PHE | 1223 | 7.126 | 24.596 | 18.848 | 1.00 | 11.22 | | C |
| ANISOU | 571 | CA | PHE | 1223 | 1270 | 1327 | 1665 | 242 | -190 | 129 | C |
| ATOM | 572 | CB | PHE | 1223 | 6.800 | 25.003 | 20.296 | 1.00 | 12.10 | | C |
| ANISOU | 572 | CB | PHE | 1223 | 1404 | 1370 | 1822 | 132 | 28 | 4 | C |
| ATOM | 573 | CG | PHE | 1223 | 7.141 | 26.462 | 20.545 | 1.00 | 12.79 | | C |
| ANISOU | 573 | CG | PHE | 1223 | 1373 | 1503 | 1983 | 20 | -21 | -60 | C |
| ATOM | 574 | CD1 | PHE | 1223 | 8.413 | 26.895 | 20.858 | 1.00 | 15.18 | | C |
| ANISOU | 574 | CD1 | PHE | 1223 | 1630 | 1870 | 2268 | -16 | -400 | -538 | C |
| ATOM | 575 | CD2 | PHE | 1223 | 6.158 | 27.442 | 20.458 | 1.00 | 14.32 | | C |
| ANISOU | 575 | CD2 | PHE | 1223 | 1937 | 1363 | 2141 | 226 | -587 | -39 | C |
| ATOM | 576 | CE1 | PHE | 1223 | 8.694 | 28.271 | 21.060 | 1.00 | 16.28 | | C |
| ANISOU | 576 | CE1 | PHE | 1223 | 1717 | 1810 | 2658 | 97 | -63 | -858 | C |
| ATOM | 577 | CE2 | PHE | 1223 | 6.384 | 28.780 | 20.632 | 1.00 | 13.39 | | C |
| ANISOU | 577 | CE2 | PHE | 1223 | 1758 | 1379 | 1949 | -153 | -177 | 91 | C |
| ATOM | 578 | CZ | PHE | 1223 | 7.672 | 29.202 | 20.937 | 1.00 | 14.68 | | C |
| ANISOU | 578 | CZ | PHE | 1223 | 1540 | 1837 | 2203 | -63 | 13 | 100 | C |
| ATOM | 579 | C | PHE | 1223 | 6.499 | 25.557 | 17.832 | 1.00 | 12.18 | | C |
| ANISOU | 579 | C | PHE | 1223 | 1353 | 1299 | 1976 | 68 | -414 | 190 | C |
| ATOM | 580 | O | PHE | 1223 | 7.186 | 26.344 | 17.185 | 1.00 | 12.98 | | O |
| ANISOU | 580 | O | PHE | 1223 | 1629 | 1376 | 1927 | 38 | -313 | 236 | O |
| ATOM | 581 | N | GLY | 1224 | 5.178 | 25.486 | 17.727 | 1.00 | 12.75 | | N |
| ANISOU | 581 | N | GLY | 1224 | 1288 | 1506 | 2051 | 282 | -397 | 14 | N |
| ATOM | 582 | CA | GLY | 1224 | 4.472 | 26.347 | 16.791 | 1.00 | 15.36 | | C |
| ANISOU | 582 | CA | GLY | 1224 | 1505 | 1773 | 2560 | 161 | -569 | 446 | C |
| ATOM | 583 | C | GLY | 1224 | 4.994 | 26.137 | 15.380 | 1.00 | 15.88 | | C |
| ANISOU | 583 | C | GLY | 1224 | 1935 | 1810 | 2290 | -162 | -798 | 422 | C |
| ATOM | 584 | O | GLY | 1224 | 5.197 | 25.010 | 14.894 | 1.00 | 15.10 | | O |
| ANISOU | 584 | O | GLY | 1224 | 1937 | 1777 | 2024 | -281 | -825 | 432 | O |
| ATOM | 585 | N | LEU | 1225 | 5.223 | 27.257 | 14.691 | 1.00 | 16.25 | | N |
| ANISOU | 585 | N | LEU | 1225 | 1960 | 1782 | 2435 | 16 | -873 | 500 | N |
| ATOM | 586 | CA | LEU | 1225 | 5.678 | 27.210 | 13.299 | 1.00 | 16.56 | | C |
| ANISOU | 586 | CA | LEU | 1225 | 2361 | 1615 | 2314 | 32 | -1013 | 735 | C |
| ATOM | 587 | CB | LEU | 1225 | 5.665 | 28.616 | 12.672 | 1.00 | 18.65 | | C |
| ANISOU | 587 | CB | LEU | 1225 | 2170 | 1571 | 3347 | -358 | -939 | 990 | C |
| ATOM | 588 | CG | LEU | 1225 | 4.313 | 29.329 | 12.779 | 1.00 | 20.58 | | C |
| ANISOU | 588 | CG | LEU | 1225 | 2539 | 1648 | 3634 | 49 | -1527 | 840 | C |
| ATOM | 589 | CD1 | LEU | 1225 | 4.459 | 30.677 | 12.053 | 1.00 | 23.87 | | C |
| ANISOU | 589 | CD1 | LEU | 1225 | 3188 | 2090 | 3792 | 25 | -1392 | 1309 | C |
| ATOM | 590 | CD2 | LEU | 1225 | 3.140 | 28.533 | 12.235 | 1.00 | 21.01 | | C |
| ANISOU | 590 | CD2 | LEU | 1225 | 2162 | 2220 | 3601 | 98 | -1081 | 290 | C |
| ATOM | 591 | C | LEU | 1225 | 7.067 | 26.601 | 13.187 | 1.00 | 14.92 | | C |
| ANISOU | 591 | C | LEU | 1225 | 2249 | 1610 | 1808 | -188 | -765 | 743 | C |
| ATOM | 592 | O | LEU | 1225 | 7.453 | 26.251 | 12.063 | 1.00 | 18.36 | | O |
| ANISOU | 592 | O | LEU | 1225 | 2890 | 2237 | 1848 | 206 | -1051 | 283 | O |
| ATOM | 593 | N | ALA | 1226 | 7.784 | 26.482 | 14.297 | 1.00 | 13.46 | | N |
| ANISOU | 593 | N | ALA | 1226 | 2067 | 1314 | 1733 | -21 | -735 | 382 | N |
| ATOM | 594 | CA | ALA | 1226 | 9.141 | 25.913 | 14.271 | 1.00 | 14.72 | | C |
| ANISOU | 594 | CA | ALA | 1226 | 1934 | 1506 | 2151 | -107 | -469 | 469 | C |
| ATOM | 595 | CB | ALA | 1226 | 10.008 | 26.733 | 15.208 | 1.00 | 17.51 | | C |
| ANISOU | 595 | CB | ALA | 1226 | 1649 | 1965 | 3039 | -789 | -314 | 500 | C |
| ATOM | 596 | C | ALA | 1226 | 9.176 | 24.424 | 14.663 | 1.00 | 13.09 | | C |
| ANISOU | 596 | C | ALA | 1226 | 1739 | 1553 | 1680 | 27 | -474 | 404 | C |
| ATOM | 597 | O | ALA | 1226 | 10.254 | 23.844 | 14.810 | 1.00 | 13.67 | | O |
| ANISOU | 597 | O | ALA | 1226 | 1791 | 1643 | 1761 | 159 | -341 | 229 | O |
| ATOM | 598 | N | AARG | 1227 | 8.007 | 23.781 | 14.834 | 0.70 | 12.96 | | N |
| ANISOU | 598 | N | AARG | 1227 | 1867 | 1510 | 1547 | -237 | -607 | 260 | N |
| ATOM | 599 | N | BARG | 1227 | 8.009 | 23.783 | 14.837 | 0.30 | 12.98 | | N |
| ANISOU | 599 | N | BRAG | 1227 | 1842 | 1493 | 1595 | -143 | -491 | 263 | N |
| ATOM | 600 | CA | AARG | 1227 | 7.916 | 22.391 | 15.255 | 0.70 | 13.35 | | C |
| ANISOU | 600 | CA | AARG | 1227 | 1947 | 1444 | 1680 | 111 | -102 | 229 | C |
| ATOM | 601 | CA | BRAG | 1227 | 7.879 | 22.379 | 15.220 | 0.30 | 14.10 | | C |
| ANISOU | 601 | CA | BRAG | 1227 | 2066 | 1436 | 1856 | 70 | -48 | 210 | C |
| ATOM | 602 | CB | AARG | 1227 | 6.476 | 22.060 | 15.661 | 0.70 | 18.56 | | C |
| ANISOU | 602 | CB | AARG | 1227 | 2435 | 2038 | 2580 | 11 | 633 | 768 | C |
| ATOM | 603 | CB | BARG | 1227 | 6.430 | 21.973 | 15.517 | 0.30 | 17.88 | | C |
| ANISOU | 603 | CB | BARG | 1227 | 2394 | 1541 | 2859 | -154 | 521 | 165 | C |
| ATOM | 604 | CG | AARG | 1227 | 6.225 | 20.627 | 16.034 | 0.70 | 22.87 | | C |
| ANISOU | 604 | CG | AARG | 1227 | 2869 | 1892 | 3927 | -735 | 273 | 116 | C |
| ATOM | 605 | CG | BARG | 1227 | 6.192 | 20.861 | 16.521 | 0.30 | 21.30 | | C |
| ANISOU | 605 | CG | BARG | 1227 | 2833 | 1802 | 3457 | -103 | 772 | 523 | C |
| ATOM | 606 | CD | AARG | 1227 | 4.769 | 20.276 | 16.112 | 0.70 | 25.14 | | C |
| ANISOU | 606 | CD | AARG | 1227 | 2759 | 2719 | 4073 | -404 | 724 | 894 | C |
| ATOM | 607 | CD | BARG | 1227 | 4.795 | 20.264 | 16.442 | 0.30 | 22.46 | | C |
| ANISOU | 607 | CD | BARG | 1227 | 2809 | 1970 | 3756 | -98 | 466 | 1270 | C |
| ATOM | 608 | NE | AARG | 1227 | 3.873 | 21.164 | 15.357 | 0.70 | 31.62 | | N |
| ANISOU | 608 | NE | AARG | 1227 | 3492 | 3426 | 5097 | 118 | -325 | 524 | N |
| ATOM | 609 | NE | BARG | 1227 | 4.705 | 18.886 | 16.922 | 0.30 | 15.60 | | N |
| ANISOU | 609 | NE | BARG | 1227 | 1815 | 1434 | 2680 | 448 | 275 | 501 | N |
| ATOM | 610 | CZ | AARG | 1227 | 2.754 | 20.560 | 14.919 | 0.70 | 37.82 | | C |
| ANISOU | 610 | CZ | AARG | 1227 | 4182 | 4963 | 5224 | -650 | -908 | 773 | C |
| ATOM | 611 | CZ | BRAG | 1227 | 3.806 | 17.974 | 16.608 | 0.30 | 13.18 | | C |
| ANISOU | 611 | CZ | BRAG | 1227 | 1294 | 1721 | 1992 | 490 | -91 | 1253 | C |
| ATOM | 612 | NH1AARG | | 1227 | 2.646 | 19.273 | 15.234 | 0.70 | 38.45 | | N1+ |
| ANISOU | 612 | NH1AARG | | 1227 | 4662 | 5574 | 4371 | -1905 | -1517 | 1503 | N1+ |
| ATOM | 613 | NH1BARG | | 1227 | 2.823 | 18.268 | 15.753 | 0.30 | 20.09 | | N1+ |
| ANISOU | 613 | NH1BARG | | 1227 | 2683 | 2678 | 2274 | 796 | -896 | 1484 | N1+ |
| ATOM | 614 | NH2AARG | | 1227 | 1.836 | 21.187 | 14.230 | 0.70 | 42.04 | | N |
| ANISOU | 614 | NH2AARG | | 1227 | 3623 | 7166 | 5186 | -1835 | -479 | 3431 | N |
| ATOM | 615 | NH2BARG | | 1227 | 3.811 | 16.729 | 17.106 | 0.30 | 11.30 | | N |
| ANISOU | 615 | NH2BARG | | 1227 | 1212 | 1798 | 1284 | 300 | 131 | 1246 | N |
| ATOM | 616 | C | AARG | 1227 | 8.329 | 21.438 | 14.150 | 0.70 | 13.98 | | C |
| ANISOU | 616 | C | AARG | 1227 | 1836 | 1758 | 1720 | -57 | -432 | -124 | C |
| ATOM | 617 | C | BRAG | 1227 | 8.412 | 21.474 | 14.115 | 0.30 | 14.02 | | C |
| ANISOU | 617 | C | BRAG | 1227 | 1947 | 1667 | 1712 | -69 | -287 | 13 | C |
| ATOM | 618 | O | AARG | 1227 | 7.915 | 21.637 | 13.004 | 0.70 | 16.02 | | O |
| ANISOU | 618 | O | AARG | 1227 | 2321 | 1990 | 1777 | -205 | -711 | -18 | O |
| ATOM | 619 | O | BRAG | 1227 | 8.174 | 21.719 | 12.932 | 0.30 | 16.70 | | O |
| ANISOU | 619 | O | BARG | 1227 | 2516 | 2050 | 1781 | -23 | -754 | -97 | O |
| ATOM | 620 | N | ASP | 1228 | 9.136 | 20.419 | 14.508 | 1.00 | 14.10 | | N |
| ANISOU | 620 | N | ASP | 1228 | 1981 | 1697 | 1680 | 74 | -248 | -132 | N |
| ATOM | 621 | CA | ASP | 1228 | 9.529 | 19.404 | 13.544 | 1.00 | 15.18 | | C |
| ANISOU | 621 | CA | ASP | 1228 | 2764 | 1480 | 1525 | -137 | 70 | 21 | C |
| ATOM | 622 | CB | ASP | 1228 | 10.502 | 19.943 | 12.486 | 1.00 | 16.06 | | C |
| ANISOU | 622 | CB | ASP | 1228 | 2709 | 1991 | 1404 | -614 | -57 | -200 | C |
| ATOM | 623 | CG | ASP | 1228 | 10.393 | 19.298 | 11.119 | 1.00 | 18.55 | | C |
| ANISOU | 623 | CG | ASP | 1228 | 3652 | 1938 | 1458 | -961 | 117 | -210 | C |
| ATOM | 624 | OD1 | ASP | 1228 | 10.147 | 18.076 | 11.050 | 1.00 | 18.61 | | O |
| ANISOU | 624 | OD1 | ASP | 1228 | 3446 | 1850 | 1776 | -725 | -476 | -187 | O |
| ATOM | 625 | OD2 | ASP | 1228 | 10.561 | 20.019 | 10.103 | 1.00 | 17.47 | | O1- |
| ANISOU | 625 | OD2 | ASP | 1228 | 3409 | 1803 | 1426 | -344 | 76 | -159 | O1- |
| ATOM | 626 | C | ASP | 1228 | 10.175 | 18.245 | 14.299 | 1.00 | 14.24 | | C |
| ANISOU | 626 | C | ASP | 1228 | 2207 | 1621 | 1583 | 6 | -94 | -163 | C |
| ATOM | 627 | O | ASP | 1228 | 10.201 | 18.283 | 15.537 | 1.00 | 15.70 | | O |
| ANISOU | 627 | O | ASP | 1228 | 2652 | 1753 | 1560 | 109 | -284 | -164 | O |
| ATOM | 628 | N | MET | 1229 | 10.689 | 17.248 | 13.590 | 1.00 | 16.18 | | N |
| ANISOU | 628 | N | MET | 1229 | 2186 | 2048 | 1914 | 176 | -83 | -451 | N |
| ATOM | 629 | CA | MET | 1229 | 11.591 | 16.243 | 14.124 | 1.00 | 14.87 | | C |
| ANISOU | 629 | CA | MET | 1229 | 2211 | 1808 | 1633 | 112 | -88 | -433 | C |
| ATOM | 630 | CB | MET | 1229 | 11.271 | 14.790 | 13.649 | 1.00 | 17.55 | | C |
| ANISOU | 630 | CB | MET | 1229 | 2673 | 2068 | 1928 | -65 | 432 | -1018 | C |
| ATOM | 631 | CG | MET | 1229 | 9.875 | 14.483 | 14.150 | 1.00 | 23.83 | | C |
| ANISOU | 631 | CG | MET | 1229 | 2394 | 3061 | 3599 | -418 | 369 | -1656 | C |
| ATOM | 632 | SD | MET | 1229 | 9.387 | 12.758 | 13.822 | 1.00 | 26.58 | | S |
| ANISOU | 632 | SD | MET | 1229 | 3407 | 2962 | 3730 | -746 | -480 | -273 | S |
| ATOM | 633 | CE | MET | 1229 | 11.066 | 12.176 | 13.470 | 1.00 | 14.36 | | C |
| ANISOU | 633 | CE | MET | 1229 | 3344 | 1016 | 1096 | -932 | -684 | -101 | C |
| ATOM | 634 | C | MET | 1229 | 12.994 | 16.564 | 13.677 | 1.00 | 14.49 | | C |
| ANISOU | 634 | C | MET | 1229 | 2273 | 1829 | 1404 | 175 | -82 | -234 | C |
| ATOM | 635 | O | MET | 1229 | 13.213 | 16.412 | 12.472 | 1.00 | 17.29 | | O |
| ANISOU | 635 | O | MET | 1229 | 2995 | 2172 | 1401 | -416 | 132 | -193 | O |
| ATOM | 636 | N | TYR | 1230 | 13.916 | 16.982 | 14.526 | 1.00 | 12.95 | | N |
| ANISOU | 636 | N | TYR | 1230 | 2158 | 1296 | 1466 | 58 | -52 | -56 | N |
| ATOM | 637 | CA | TYR | 1230 | 15.215 | 17.476 | 14.087 | 1.00 | 13.52 | | C |
| ANISOU | 637 | CA | TYR | 1230 | 2254 | 1382 | 1500 | 100 | 89 | -6 | C |
| ATOM | 638 | CB | TYR | 1230 | 15.566 | 18.760 | 14.847 | 1.00 | 13.61 | | C |
| ANISOU | 638 | CB | TYR | 1230 | 2075 | 1242 | 1856 | 62 | 53 | 50 | C |
| ATOM | 639 | CG | TYR | 1230 | 14.670 | 19.906 | 14.441 | 1.00 | 14.14 | | C |
| ANISOU | 639 | CG | TYR | 1230 | 2268 | 1291 | 1816 | 100 | 134 | 135 | C |
| ATOM | 640 | CD1 | TYR | 1230 | 14.882 | 20.541 | 13.207 | 1.00 | 13.27 | | C |
| ANISOU | 640 | CD1 | TYR | 1230 | 2066 | 1294 | 1684 | -115 | -74 | 50 | C |
| ATOM | 641 | CE1 | TYR | 1230 | 14.100 | 21.590 | 12.778 | 1.00 | 13.63 | | C |
| ANISOU | 641 | CE1 | TYR | 1230 | 2067 | 1133 | 1979 | -210 | -49 | 141 | C |
| ATOM | 642 | CD2 | TYR | 1230 | 13.628 | 20.395 | 15.224 | 1.00 | 12.86 | | C |
| ANISOU | 642 | CD2 | TYR | 1230 | 2011 | 1161 | 1714 | -25 | -83 | 61 | C |
| ATOM | 643 | CE2 | TYR | 1230 | 12.821 | 21.460 | 14.808 | 1.00 | 13.21 | | C |
| ANISOU | 643 | CE2 | TYR | 1230 | 2018 | 1314 | 1690 | 11 | -273 | 38 | C |
| ATOM | 644 | CZ | TYR | 1230 | 13.074 | 22.041 | 13.590 | 1.00 | 13.80 | | C |
| ANISOU | 644 | CZ | TYR | 1230 | 1926 | 1568 | 1750 | 33 | -278 | 211 | C |
| ATOM | 645 | OH | TYR | 1230 | 12.296 | 23.104 | 13.151 | 1.00 | 15.16 | | O |
| ANISOU | 645 | OH | TYR | 1230 | 2094 | 1770 | 1895 | 114 | -195 | 379 | O |
| ATOM | 646 | C | TYR | 1230 | 16.296 | 16.434 | 14.294 | 1.00 | 13.45 | | C |
| ANISOU | 646 | C | TYR | 1230 | 2248 | 1363 | 1500 | 126 | 148 | -44 | C |
| ATOM | 647 | O | TYR | 1230 | 17.428 | 16.628 | 13.857 | 1.00 | 14.68 | | O |
| ANISOU | 647 | O | TYR | 1230 | 2233 | 1500 | 1845 | 45 | 122 | 138 | O |
| ATOM | 648 | N | ASP | 1231 | 15.988 | 15.309 | 14.969 | 1.00 | 13.12 | | N |
| ANISOU | 648 | N | ASP | 1231 | 2049 | 1322 | 1615 | 67 | -14 | -88 | N |
| ATOM | 649 | CA | ASP | 1231 | 16.912 | 14.199 | 15.087 | 1.00 | 12.85 | | C |
| ANISOU | 649 | CA | ASP | 1231 | 1938 | 1356 | 1586 | 74 | 20 | -101 | C |
| ATOM | 650 | CB | ASP | 1231 | 17.800 | 14.300 | 16.335 | 1.00 | 12.11 | | C |
| ANISOU | 650 | CB | ASP | 1231 | 1649 | 1305 | 1646 | -42 | 111 | -64 | C |
| ATOM | 651 | CG | ASP | 1231 | 18.852 | 13.201 | 16.328 | 1.00 | 13.54 | | C |
| ANISOU | 651 | CG | ASP | 1231 | 1656 | 1517 | 1971 | 59 | -22 | -303 | C |
| ATOM | 652 | OD1 | ASP | 1231 | 18.764 | 12.239 | 15.552 | 1.00 | 15.12 | | O |
| ANISOU | 652 | OD1 | ASP | 1231 | 2183 | 1760 | 1801 | 280 | 21 | -481 | O |
| ATOM | 653 | OD2 | ASP | 1231 | 19.785 | 13.319 | 17.152 | 1.00 | 13.05 | | O1- |
| ANISOU | 653 | OD2 | ASP | 1231 | 1696 | 1605 | 1659 | 18 | 90 | -37 | O1- |
| ATOM | 654 | C | ASP | 1231 | 16.048 | 12.961 | 15.096 | 1.00 | 12.14 | | C |
| ANISOU | 654 | C | ASP | 1231 | 1766 | 1352 | 1494 | 96 | -30 | -169 | C |
| ATOM | 655 | O | ASP | 1231 | 15.519 | 12.559 | 16.128 | 1.00 | 12.72 | | O |
| ANISOU | 655 | O | ASP | 1231 | 1935 | 1373 | 1526 | 46 | 11 | -218 | O |
| ATOM | 656 | N | LYS | 1232 | 15.897 | 12.335 | 13.919 | 1.00 | 13.64 | | N |
| ANISOU | 656 | N | LYS | 1232 | 2003 | 1548 | 1633 | 3 | 159 | -325 | N |
| ATOM | 657 | CA | LYS | 1232 | 14.976 | 11.218 | 13.777 | 1.00 | 15.00 | | C |
| ANISOU | 657 | CA | LYS | 1232 | 2373 | 1828 | 1498 | -257 | -382 | -208 | C |
| ATOM | 658 | CB | LYS | 1232 | 14.963 | 10.750 | 12.305 | 1.00 | 24.66 | | C |
| ANISOU | 658 | CB | LYS | 1232 | 4099 | 3634 | 1636 | -1025 | -286 | -793 | C |
| ATOM | 659 | CG | LYS | 1232 | 16.313 | 10.884 | 11.660 | 1.00 | 37.13 | | C |
| ANISOU | 659 | CG | LYS | 1232 | 5161 | 5661 | 3287 | 104 | 1519 | -1586 | C |
| ATOM | 660 | CD | LYS | 1232 | 17.199 | 9.697 | 11.980 | 1.00 | 51.93 | | C |
| ANISOU | 660 | CD | LYS | 1232 | 5536 | 7179 | 7014 | 1055 | 969 | -916 | C |
| ATOM | 661 | CE | LYS | 1232 | 18.605 | 10.028 | 11.464 | 1.00 | 55.57 | | C |
| ANISOU | 661 | CE | LYS | 1232 | 5575 | 7144 | 8395 | 1196 | 1136 | 406 | C |
| ATOM | 662 | NZ | LYS | 1232 | 18.593 | 11.387 | 10.827 | 1.00 | 68.08 | | N1+ |
| ANISOU | 662 | NZ | LYS | 1232 | 7038 | 8533 | 10294 | 953 | 146 | 2605 | N1+ |
| ATOM | 663 | C | LYS | 1232 | 15.310 | 10.019 | 14.656 | 1.00 | 14.39 | | C |
| ANISOU | 663 | C | LYS | 1232 | 2054 | 1359 | 2055 | 47 | 155 | -359 | C |
| ATOM | 664 | O | LYS | 1232 | 14.401 | 9.225 | 14.897 | 1.00 | 16.41 | | O |
| ANISOU | 664 | O | LYS | 1232 | 2571 | 1703 | 1960 | -411 | 102 | -364 | O |
| ATOM | 665 | N | GLU | 1233 | 16.559 | 9.901 | 15.103 | 1.00 | 14.43 | | N |
| ANISOU | 665 | N | GLU | 1233 | 2151 | 1510 | 1820 | 341 | 161 | -366 | N |
| ATOM | 666 | CA | GLU | 1233 | 16.941 | 8.733 | 15.897 | 1.00 | 15.23 | | C |
| ANISOU | 666 | CA | GLU | 1233 | 2326 | 1757 | 1702 | 418 | 207 | -380 | C |
| ATOM | 667 | CB | GLU | 1233 | 18.437 | 8.874 | 16.290 | 1.00 | 18.97 | | C |
| ANISOU | 667 | CB | GLU | 1233 | 1909 | 2450 | 2847 | 901 | 527 | -133 | C |
| ATOM | 668 | CG | GLU | 1233 | 18.842 | 7.727 | 17.196 | 1.00 | 26.78 | | C |
| ANISOU | 668 | CG | GLU | 1233 | 3100 | 3308 | 3768 | 1543 | -77 | 291 | C |
| ATOM | 669 | CD | GLU | 1233 | 20.294 | 7.670 | 17.587 | 1.00 | 30.62 | | C |
| ANISOU | 669 | CD | GLU | 1233 | 3000 | 3794 | 4840 | 1169 | -189 | 1158 | C |
| ATOM | 670 | OE1 | GLU | 1233 | 21.067 | 8.508 | 17.036 | 1.00 | 35.64 | | O1- |
| ANISOU | 670 | OE1 | GLU | 1233 | 3758 | 3910 | 5874 | 582 | -2 | 1131 | O1- |
| ATOM | 671 | OE2 | GLU | 1233 | 20.638 | 6.777 | 18.436 | 1.00 | 30.66 | | O |
| ANISOU | 671 | OE2 | GLU | 1233 | 3221 | 3965 | 4464 | 1353 | -251 | 918 | O |
| ATOM | 672 | C | GLU | 1233 | 16.067 | 8.535 | 17.127 | 1.00 | 15.49 | | C |
| ANISOU | 672 | C | GLU | 1233 | 2147 | 1451 | 2287 | 521 | 557 | -138 | C |
| ATOM | 673 | O | GLU | 1233 | 15.845 | 7.409 | 17.599 | 1.00 | 18.33 | | O |
| ANISOU | 673 | O | GLU | 1233 | 3286 | 1388 | 2292 | 673 | 546 | -102 | O |
| ATOM | 674 | N | TYR | 1234 | 15.540 | 9.630 | 17.688 | 1.00 | 12.26 | | N |
| ANISOU | 674 | N | TYR | 1234 | 1826 | 1347 | 1486 | 374 | -27 | -203 | N |
| ATOM | 675 | CA | TYR | 1234 | 14.885 | 9.565 | 18.996 | 1.00 | 12.57 | | C |
| ANISOU | 675 | CA | TYR | 1234 | 1769 | 1451 | 1555 | 157 | -75 | -424 | C |
| ATOM | 676 | CB | TYR | 1234 | 15.388 | 10.772 | 19.860 | 1.00 | 11.97 | | C |
| ANISOU | 676 | CB | TYR | 1234 | 1811 | 1185 | 1552 | 69 | 5 | -233 | C |
| ATOM | 677 | CG | TYR | 1234 | 16.871 | 10.571 | 20.112 | 1.00 | 11.31 | | C |
| ANISOU | 677 | CG | TYR | 1234 | 1826 | 1246 | 1226 | 52 | 25 | -229 | C |
| ATOM | 678 | CD1 | TYR | 1234 | 17.857 | 11.285 | 19.432 | 1.00 | 12.23 | | C |
| ANISOU | 678 | CD1 | TYR | 1234 | 1874 | 1066 | 1707 | 40 | 45 | -118 | C |
| ATOM | 679 | CE1 | TYR | 1234 | 19.185 | 11.082 | 19.674 | 1.00 | 12.38 | | C |
| ANISOU | 679 | CE1 | TYR | 1234 | 1849 | 1262 | 1591 | -90 | -125 | -278 | C |
| ATOM | 680 | CD2 | TYR | 1234 | 17.286 | 9.625 | 21.060 | 1.00 | 11.77 | | C |
| ANISOU | 680 | CD2 | TYR | 1234 | 1788 | 1244 | 1438 | -50 | -99 | -158 | C |
| ATOM | 681 | CE2 | TYR | 1234 | 18.612 | 9.385 | 21.334 | 1.00 | 13.00 | | C |
| ANISOU | 681 | CE2 | TYR | 1234 | 1781 | 1343 | 1817 | -56 | -220 | -134 | C |
| ATOM | 682 | CZ | TYR | 1234 | 19.542 | 10.136 | 20.621 | 1.00 | 12.66 | | C |
| ANISOU | 682 | CZ | TYR | 1234 | 1774 | 1313 | 1722 | -101 | -217 | -234 | C |
| ATOM | 683 | OH | TYR | 1234 | 20.878 | 9.923 | 20.871 | 1.00 | 15.18 | | O |
| ANISOU | 683 | OH | TYR | 1234 | 1788 | 1614 | 2366 | -255 | -461 | -100 | O |
| ATOM | 684 | C | TYR | 1234 | 13.374 | 9.508 | 18.919 | 1.00 | 12.83 | | C |
| ANISOU | 684 | C | TYR | 1234 | 1771 | 1465 | 1639 | -15 | -52 | -92 | C |
| ATOM | 685 | O | TYR | 1234 | 12.694 | 9.715 | 19.923 | 1.00 | 13.00 | | O |
| ANISOU | 685 | O | TYR | 1234 | 1857 | 1492 | 1588 | 140 | -37 | 17 | O |
| ATOM | 686 | N | TYR | 1235 | 12.821 | 9.202 | 17.720 | 1.00 | 12.60 | | N |
| ANISOU | 686 | N | TYR | 1235 | 1743 | 1314 | 1729 | 106 | -105 | -279 | N |
| ATOM | 687 | CA | TYR | 1235 | 11.402 | 9.112 | 17.512 | 1.00 | 12.07 | | C |
| ANISOU | 687 | CA | TYR | 1235 | 1736 | 1100 | 1748 | -129 | 12 | -294 | C |
| ATOM | 688 | CB | TYR | 1235 | 10.884 | 10.156 | 16.487 | 1.00 | 12.71 | | C |
| ANISOU | 688 | CB | TYR | 1235 | 1774 | 1210 | 1846 | 33 | -202 | -321 | C |
| ATOM | 689 | CG | TYR | 1235 | 11.148 | 11.536 | 17.075 | 1.00 | 11.57 | | C |
| ANISOU | 689 | CG | TYR | 1235 | 1706 | 1155 | 1537 | -9 | -102 | -161 | C |
| ATOM | 690 | CD1 | TYR | 1235 | 12.408 | 12.122 | 16.930 | 1.00 | 11.96 | | C |
| ANISOU | 690 | CD1 | TYR | 1235 | 1647 | 1172 | 1724 | 48 | -92 | -137 | C |
| ATOM | 691 | CE1 | TYR | 1235 | 12.719 | 13.381 | 17.446 | 1.00 | 11.68 | | C |
| ANISOU | 691 | CE1 | TYR | 1235 | 1579 | 1025 | 1836 | 150 | -162 | -62 | C |
| ATOM | 692 | CD2 | TYR | 1235 | 10.157 | 12.229 | 17.761 | 1.00 | 10.34 | | C |
| ANISOU | 692 | CD2 | TYR | 1235 | 1482 | 1062 | 1385 | 98 | -356 | -93 | C |
| ATOM | 693 | CE2 | TYR | 1235 | 10.452 | 13.488 | 18.289 | 1.00 | 10.27 | | C |
| ANISOU | 693 | CE2 | TYR | 1235 | 1491 | 1189 | 1222 | 13 | -448 | -127 | C |
| ATOM | 694 | CZ | TYR | 1235 | 11.711 | 14.025 | 18.120 | 1.00 | 10.64 | | C |
| ANISOU | 694 | CZ | TYR | 1235 | 1478 | 988 | 1577 | 120 | -396 | -98 | C |
| ATOM | 695 | OH | TYR | 1235 | 12.013 | 15.268 | 18.631 | 1.00 | 11.37 | | O |
| ANISOU | 695 | OH | TYR | 1235 | 1391 | 1234 | 1694 | -53 | -285 | -288 | O |
| ATOM | 696 | C | TYR | 1235 | 11.061 | 7.709 | 17.024 | 1.00 | 14.28 | | C |
| ANISOU | 696 | C | TYR | 1235 | 1965 | 1116 | 2345 | -38 | 180 | -509 | C |
| ATOM | 697 | O | TYR | 1235 | 11.752 | 7.225 | 16.102 | 1.00 | 18.57 | | O |
| ANISOU | 697 | O | TYR | 1235 | 2741 | 1768 | 2546 | -490 | 578 | -1034 | O |
| ATOM | 698 | N | SER | 1236 | 10.063 | 7.095 | 17.637 | 1.00 | 13.08 | | N |
| ANISOU | 698 | N | SER | 1236 | 1914 | 1044 | 2010 | -218 | -212 | -354 | N |
| ATOM | 699 | CA | SER | 1236 | 9.636 | 5.763 | 17.231 | 1.00 | 13.74 | | C |
| ANISOU | 699 | CA | SER | 1236 | 1892 | 1028 | 2301 | -34 | -532 | -419 | C |
| ATOM | 700 | CB | SER | 1236 | 9.928 | 4.783 | 18.376 | 1.00 | 17.45 | | C |
| ANISOU | 700 | CB | SER | 1236 | 2501 | 1156 | 2974 | 65 | -858 | 17 | C |
| ATOM | 701 | OG | SER | 1236 | 11.342 | 4.632 | 18.468 | 1.00 | 20.19 | | O |
| ANISOU | 701 | OG | SER | 1236 | 2533 | 1802 | 3338 | 280 | -900 | -74 | O |
| ATOM | 702 | C | SER | 1236 | 8.161 | 5.783 | 16.871 | 1.00 | 13.57 | | C |
| ANISOU | 702 | C | SER | 1236 | 1827 | 1185 | 2144 | -74 | -440 | -416 | C |
| ATOM | 703 | O | SER | 1236 | 7.394 | 6.482 | 17.512 | 1.00 | 15.73 | | O |
| ANISOU | 703 | O | SER | 1236 | 1899 | 1730 | 2346 | 34 | -319 | -601 | O |
| ATOM | 704 | N | VAL | 1237 | 7.810 | 5.006 | 15.850 | 1.00 | 15.64 | | N |
| ANISOU | 704 | N | VAL | 1237 | 2087 | 1740 | 2113 | -247 | -565 | -517 | N |
| ATOM | 705 | CA | VAL | 1237 | 6.430 | 4.956 | 15.390 | 1.00 | 17.22 | | C |
| ANISOU | 705 | CA | VAL | 1237 | 2015 | 2341 | 2188 | -262 | -558 | -431 | C |
| ATOM | 706 | CB | VAL | 1237 | 6.375 | 4.486 | 13.924 | 1.00 | 22.66 | | C |
| ANISOU | 706 | CB | VAL | 1237 | 2719 | 3622 | 2268 | -810 | -709 | -718 | C |
| ATOM | 707 | CG1 | VAL | 1237 | 4.962 | 4.362 | 13.439 | 1.00 | 24.29 | | C |
| ANISOU | 707 | CG1 | VAL | 1237 | 2459 | 4451 | 2318 | 183 | -655 | -1374 | C |
| ATOM | 708 | CG2 | VAL | 1237 | 7.135 | 5.482 | 13.055 | 1.00 | 26.34 | | C |
| ANISOU | 708 | CG2 | VAL | 1237 | 3574 | 3944 | 2488 | -227 | 117 | -196 | C |
| ATOM | 709 | C | VAL | 1237 | 5.628 | 4.037 | 16.302 | 1.00 | 17.00 | | C |
| ANISOU | 709 | C | VAL | 1237 | 2126 | 1717 | 2614 | -188 | -419 | -539 | C |
| ATOM | 710 | O | VAL | 1237 | 5.974 | 2.856 | 16.517 | 1.00 | 21.11 | | O |
| ANISOU | 710 | O | VAL | 1237 | 2819 | 1873 | 3329 | 165 | -502 | -307 | O |
| ATOM | 711 | N | HIS | 1238 | 4.547 | 4.549 | 16.854 | 1.00 | 16.83 | | N |
| ANISOU | 711 | N | HIS | 1238 | 2093 | 2136 | 2166 | -102 | -464 | -478 | N |
| ATOM | 712 | CA | HIS | 1238 | 3.588 | 3.754 | 17.613 | 1.00 | 17.94 | | C |
| ANISOU | 712 | CA | HIS | 1238 | 2508 | 2060 | 2250 | -425 | -336 | -701 | C |
| ATOM | 713 | CB | HIS | 1238 | 2.644 | 4.602 | 18.465 | 1.00 | 20.86 | | C |
| ANISOU | 713 | CB | HIS | 1238 | 2177 | 2698 | 3050 | -584 | -59 | -1171 | C |
| ATOM | 714 | CG | HIS | 1238 | 1.963 | 3.741 | 19.485 | 1.00 | 23.58 | | C |
| ANISOU | 714 | CG | HIS | 1238 | 2246 | 3890 | 2824 | -565 | -123 | -785 | C |
| ATOM | 715 | CD2 | HIS | 1238 | 0.865 | 2.951 | 19.298 | 1.00 | 19.89 | | C |
| ANISOU | 715 | CD2 | HIS | 1238 | 2255 | 2533 | 2770 | -250 | 295 | -993 | C |
| ATOM | 716 | ND1 | HIS | 1238 | 2.308 | 3.585 | 20.812 | 1.00 | 30.42 | | N |
| ANISOU | 716 | ND1 | HIS | 1238 | 3426 | 5093 | 3039 | -685 | -593 | -473 | N |
| ATOM | 717 | CE1 | HIS | 1238 | 1.479 | 2.752 | 21.398 | 1.00 | 27.54 | | C |
| ANISOU | 717 | CE1 | HIS | 1238 | 3319 | 4300 | 2844 | 280 | 207 | -515 | C |
| ATOM | 718 | NE2 | HIS | 1238 | 0.606 | 2.368 | 20.475 | 1.00 | 27.61 | | N |
| ANISOU | 718 | NE2 | HIS | 1238 | 3500 | 3807 | 3183 | -527 | 304 | -313 | N |
| ATOM | 719 | C | HIS | 1238 | 2.781 | 2.926 | 16.640 | 1.00 | 19.32 | | C |
| ANISOU | 719 | C | HIS | 1238 | 2881 | 2059 | 2400 | -428 | -761 | -533 | C |
| ATOM | 720 | O | HIS | 1238 | 2.169 | 3.445 | 15.706 | 1.00 | 21.41 | | O |
| ANISOU | 720 | O | HIS | 1238 | 2729 | 2522 | 2883 | -383 | -974 | -325 | O |
| ATOM | 721 | N | ASN | 1239 | 2.792 | 1.606 | 16.882 | 1.00 | 18.74 | | N |
| ANISOU | 721 | N | ASN | 1239 | 2583 | 2072 | 2465 | -573 | -546 | -554 | N |
| ATOM | 722 | CA | ASN | 1239 | 2.197 | 0.775 | 15.812 | 1.00 | 18.67 | | C |
| ANISOU | 722 | CA | ASN | 1239 | 2517 | 2120 | 2456 | -305 | -764 | -639 | C |
| ATOM | 723 | CB | ASN | 1239 | 2.932 | -0.595 | 15.747 | 1.00 | 22.78 | | C |
| ANISOU | 723 | CB | ASN | 1239 | 3617 | 1967 | 3070 | -141 | 29 | -465 | C |
| ATOM | 724 | CG | ASN | 1239 | 2.854 | -1.483 | 16.974 | 1.00 | 25.87 | | C |
| ANISOU | 724 | CG | ASN | 1239 | 3747 | 2456 | 3625 | 111 | -123 | 85 | C |
| ATOM | 725 | ODI | ASN | 1239 | 1.879 | -1.485 | 17.724 | 1.00 | 26.32 | | O |
| ANISOU | 725 | OD1 | ASN | 1239 | 4194 | 2582 | 3224 | -534 | 52 | -132 | O |
| ATOM | 726 | ND2 | ASN | 1239 | 3.939 | -2.273 | 17.181 | 1.00 | 27.62 | | N |
| ANISOU | 726 | ND2 | ASN | 1239 | 4208 | 2441 | 3846 | 274 | -1437 | -934 | N |
| ATOM | 727 | C | ASN | 1239 | 0.691 | 0.618 | 15.949 | 1.00 | 23.47 | | C |
| ANISOU | 727 | C | ASN | 1239 | 2573 | 3650 | 2695 | -602 | -666 | -1115 | C |
| ATOM | 728 | O | ASN | 1239 | 0.084 | -0.201 | 15.218 | 1.00 | 28.82 | | O |
| ANISOU | 728 | O | ASN | 1239 | 2889 | 3944 | 4117 | -1169 | -315 | -1838 | O |
| ATOM | 729 | N | LYS | 1240 | 0.045 | 1.375 | 16.824 | 1.00 | 24.60 | | N |
| ANISOU | 729 | N | LYS | 1240 | 2564 | 3477 | 3306 | -751 | -235 | -1148 | N |
| ATOM | 730 | CA | LYS | 1240 | -1.424 | 1.344 | 16.754 | 1.00 | 25.90 | | C |
| ANISOU | 730 | CA | LYS | 1240 | 2590 | 3777 | 3474 | -666 | -462 | -1239 | C |
| ATOM | 731 | CB | LYS | 1240 | -1.975 | 1.075 | 18.144 | 1.00 | 27.11 | | C |
| ANISOU | 731 | CB | LYS | 1240 | 2595 | 3814 | 3891 | -437 | -23 | -868 | C |
| ATOM | 736 | C | LYS | 1240 | -1.811 | 2.654 | 16.114 | 1.00 | 27.03 | | C |
| ANISOU | 736 | C | LYS | 1240 | 2832 | 4028 | 3412 | -387 | -319 | -1091 | C |
| ATOM | 737 | O | LYS | 1240 | -2.725 | 2.625 | 15.283 | 1.00 | 33.81 | | O |
| ANISOU | 737 | O | LYS | 1240 | 2800 | 3811 | 6236 | 457 | -1583 | -1740 | O |
| ATOM | 738 | N | THR | 1241 | -1.126 | 3.755 | 16.478 | 1.00 | 24.37 | | N |
| ANISOU | 738 | N | THR | 1241 | 2705 | 3733 | 2820 | -177 | -308 | -785 | N |
| ATOM | 739 | CA | THR | 1241 | -1.554 | 5.027 | 15.901 | 1.00 | 23.34 | | C |
| ANISOU | 739 | CA | THR | 1241 | 1883 | 4000 | 2983 | 49 | -636 | -722 | C |
| ATOM | 740 | CB | THR | 1241 | -1.567 | 6.099 | 17.029 | 1.00 | 23.24 | | C |
| ANISOU | 740 | CB | THR | 1241 | 2119 | 3754 | 2958 | 28 | -364 | -590 | C |
| ATOM | 741 | OG1 | THR | 1241 | -0.185 | 6.266 | 17.412 | 1.00 | 21.67 | | O |
| ANISOU | 741 | OG1 | THR | 1241 | 2081 | 3752 | 2402 | 419 | -389 | -827 | O |
| ATOM | 742 | CG2 | THR | 1241 | -2.386 | 5.681 | 18.224 | 1.00 | 27.29 | | C |
| ANISOU | 742 | CG2 | THR | 1241 | 2620 | 4356 | 3391 | -127 | 166 | -682 | C |
| ATOM | 743 | C | THR | 1241 | -0.717 | 5.626 | 14.784 | 1.00 | 23.51 | | C |
| ANISOU | 743 | C | THR | 1241 | 2511 | 3547 | 2875 | 313 | -513 | -703 | C |
| ATOM | 744 | O | THR | 1241 | -1.158 | 6.581 | 14.115 | 1.00 | 24.60 | | O |
| ANISOU | 744 | O | THR | 1241 | 2529 | 3830 | 2990 | 444 | -656 | -633 | O |
| ATOM | 745 | N | GLY | 1242 | 0.478 | 5.165 | 14.520 | 1.00 | 19.74 | | N |
| ANISOU | 745 | N | GLY | 1242 | 2190 | 2967 | 2342 | -124 | -606 | -528 | N |
| ATOM | 746 | CA | GLY | 1242 | 1.396 | 5.703 | 13.515 | 1.00 | 18.56 | | C |
| ANISOU | 746 | CA | GLY | 1242 | 2512 | 2485 | 2054 | -252 | -854 | -282 | C |
| ATOM | 747 | C | GLY | 1242 | 2.149 | 6.934 | 13.987 | 1.00 | 17.81 | | C |
| ANISOU | 747 | C | GLY | 1242 | 2096 | 2277 | 2396 | 192 | -975 | -440 | C |
| ATOM | 748 | O | GLY | 1242 | 2.996 | 7.478 | 13.277 | 1.00 | 21.23 | | O |
| ANISOU | 748 | O | GLY | 1242 | 2961 | 2019 | 3087 | -156 | -610 | -221 | O |
| ATOM | 749 | N | ALA | 1243 | 1.871 | 7.430 | 15.207 | 1.00 | 20.71 | | N |
| ANISOU | 749 | N | ALA | 1243 | 2312 | 2910 | 2645 | 441 | -1145 | -923 | N |
| ATOM | 750 | CA | ALA | 1243 | 2.493 | 8.659 | 15.691 | 1.00 | 18.68 | | C |
| ANISOU | 750 | CA | ALA | 1243 | 2004 | 2740 | 2352 | 355 | -685 | -752 | C |
| ATOM | 751 | CB | ALA | 1243 | 1.851 | 9.097 | 17.001 | 1.00 | 22.28 | | C |
| ANISOU | 751 | CB | ALA | 1243 | 2406 | 3134 | 2924 | 561 | -275 | -1121 | C |
| ATOM | 752 | C | ALA | 1243 | 3.989 | 8.481 | 15.904 | 1.00 | 17.19 | | C |
| ANISOU | 752 | C | ALA | 1243 | 1993 | 2362 | 2178 | 325 | -515 | -190 | C |
| ATOM | 753 | O | ALA | 1243 | 4.355 | 7.416 | 16.428 | 1.00 | 16.95 | | O |
| ANISOU | 753 | O | ALA | 1243 | 2233 | 2139 | 2070 | 220 | -714 | -425 | O |
| ATOM | 754 | N | LYS | 1244 | 4.830 | 9.446 | 15.543 | 1.00 | 17.19 | | N |
| ANISOU | 754 | N | LYS | 1244 | 2226 | 2174 | 2132 | 173 | -748 | -409 | N |
| ATOM | 755 | CA | LYS | 1244 | 6.279 | 9.399 | 15.755 | 1.00 | 16.51 | | C |
| ANISOU | 755 | CA | LYS | 1244 | 2242 | 2452 | 1579 | -113 | -561 | -278 | C |
| ATOM | 756 | CB | LYS | 1244 | 6.996 | 10.115 | 14.619 | 1.00 | 24.37 | | C |
| ANISOU | 756 | CB | LYS | 1244 | 3095 | 4447 | 1718 | -450 | -252 | 233 | C |
| ATOM | 761 | C | LYS | 1244 | 6.570 | 10.019 | 17.107 | 1.00 | 13.96 | | C |
| ANISOU | 761 | C | LYS | 1244 | 2093 | 1609 | 1602 | 188 | -527 | -145 | C |
| ATOM | 762 | O | LYS | 1244 | 6.432 | 11.228 | 17.285 | 1.00 | 16.00 | | O |
| ANISOU | 762 | O | LYS | 1244 | 2436 | 1569 | 2072 | 207 | -586 | 2 | O |
| ATOM | 763 | N | LEU | 1245 | 6.966 | 9.237 | 18.106 | 1.00 | 12.09 | | N |
| ANISOU | 763 | N | LEU | 1245 | 1707 | 1395 | 1491 | -42 | -436 | -234 | N |
| ATOM | 764 | CA | LEU | 1245 | 6.978 | 9.680 | 19.503 | 1.00 | 11.64 | | C |
| ANISOU | 764 | CA | LEU | 1245 | 1527 | 1426 | 1471 | -70 | -365 | -386 | C |
| ATOM | 765 | CB | LEU | 1245 | 6.119 | 8.694 | 20.288 | 1.00 | 12.13 | | C |
| ANISOU | 765 | CB | LEU | 1245 | 1376 | 1696 | 1537 | 48 | -288 | -297 | C |
| ATOM | 766 | CG | LEU | 1245 | 4.616 | 8.695 | 19.963 | 1.00 | 12.80 | | C |
| ANISOU | 766 | CG | LEU | 1245 | 1394 | 1889 | 1579 | 40 | -337 | -326 | C |
| ATOM | 767 | CD1 | LEU | 1245 | 3.943 | 7.569 | 20.729 | 1.00 | 16.49 | | C |
| ANISOU | 767 | CD1 | LEU | 1245 | 1800 | 2257 | 2209 | -329 | -94 | -208 | C |
| ATOM | 768 | CD2 | LEU | 1245 | 3.979 | 10.052 | 20.252 | 1.00 | 15.04 | | C |
| ANISOU | 768 | CD2 | LEU | 1245 | 1360 | 2084 | 2269 | 232 | -539 | -668 | C |
| ATOM | 769 | C | LEU | 1245 | 8.367 | 9.703 | 20.091 | 1.00 | 10.74 | | C |
| ANISOU | 769 | C | LEU | 1245 | 1492 | 1165 | 1422 | 28 | -278 | -354 | C |
| ATOM | 770 | O | LEU | 1245 | 9.161 | 8.773 | 19.848 | 1.00 | 11.72 | | O |
| ANISOU | 770 | O | LEU | 1245 | 1643 | 1174 | 1638 | 37 | -155 | -268 | O |
| ATOM | 771 | N | PRO | 1246 | 8.707 | 10.736 | 20.856 | 1.00 | 10.15 | | N |
| ANISOU | 771 | N | PRO | 1246 | 1212 | 1173 | 1471 | 70 | -370 | -251 | N |
| ATOM | 772 | CD | PRO | 1246 | 7.886 | 11.940 | 21.084 | 1.00 | 10.19 | | C |
| ANISOU | 772 | CD | PRO | 1246 | 1492 | 890 | 1490 | 52 | -337 | -168 | C |
| ATOM | 773 | CA | PRO | 1246 | 10.010 | 10.826 | 21.531 | 1.00 | 10.05 | | C |
| ANISOU | 773 | CA | PRO | 1246 | 1275 | 959 | 1586 | -102 | -367 | -74 | C |
| ATOM | 774 | CB | PRO | 1246 | 10.161 | 12.336 | 21.800 | 1.00 | 10.20 | | C |
| ANISOU | 774 | CB | PRO | 1246 | 1443 | 968 | 1465 | -121 | -250 | -20 | C |
| ATOM | 775 | CG | PRO | 1246 | 8.739 | 12.771 | 22.016 | 1.00 | 10.24 | | C |
| ANISOU | 775 | CG | PRO | 1246 | 1396 | 863 | 1631 | -86 | -359 | 21 | C |
| ATOM | 776 | C | PRO | 1246 | 10.026 | 10.021 | 22.832 | 1.00 | 9.89 | | C |
| ANISOU | 776 | C | PRO | 1246 | 1289 | 913 | 1555 | 33 | -396 | -103 | C |
| ATOM | 777 | O | PRO | 1246 | 10.047 | 10.591 | 23.915 | 1.00 | 9.47 | | O |
| ANISOU | 777 | O | PRO | 1246 | 1217 | 877 | 1505 | 58 | -104 | -54 | O |
| ATOM | 778 | N | VAL | 1247 | 10.024 | 8.683 | 22.677 | 1.00 | 9.82 | | N |
| ANISOU | 778 | N | VAL | 1247 | 1192 | 904 | 1633 | -48 | -359 | -76 | N |
| ATOM | 779 | CA | VAL | 1247 | 9.611 | 7.834 | 23.802 | 1.00 | 10.27 | | C |
| ANISOU | 779 | CA | VAL | 1247 | 1460 | 955 | 1488 | -163 | -351 | -154 | C |
| ATOM | 780 | CB | VAL | 1247 | 9.573 | 6.338 | 23.375 | 1.00 | 12.33 | | C |
| ANISOU | 780 | CB | VAL | 1247 | 1750 | 965 | 1971 | -111 | -538 | -179 | C |
| ATOM | 781 | CG1 | VAL | 1247 | 8.429 | 6.166 | 22.327 | 1.00 | 15.23 | | C |
| ANISOU | 781 | CG1 | VAL | 1247 | 2107 | 1238 | 2444 | -242 | -930 | -264 | C |
| ATOM | 782 | CG2 | VAL | 1247 | 10.901 | 5.842 | 22.880 | 1.00 | 13.87 | | C |
| ANISOU | 782 | CG2 | VAL | 1247 | 1962 | 1116 | 2193 | 113 | -359 | -333 | C |
| ATOM | 783 | C | VAL | 1247 | 10.492 | 8.001 | 25.012 | 1.00 | 9.46 | | C |
| ANISOU | 783 | C | VAL | 1247 | 1157 | 834 | 1602 | -90 | -239 | -14 | C |
| ATOM | 784 | O | VAL | 1247 | 9.931 | 7.873 | 26.117 | 1.00 | 10.78 | | O |
| ANISOU | 784 | O | VAL | 1247 | 1355 | 1237 | 1505 | -98 | -236 | -28 | O |
| ATOM | 785 | N | ALYS | 1248 | 11.788 | 8.270 | 24.880 | 0.50 | 9.63 | | N |
| ANISOU | 785 | N | ALYS | 1248 | 1101 | 891 | 1667 | 133 | -289 | -68 | N |
| ATOM | 786 | N | BLYS | 1248 | 11.793 | 8.266 | 24.918 | 0.50 | 9.92 | | N |
| ANISOU | 786 | N | BLYS | 1248 | 1149 | 950 | 1671 | 24 | -278 | -120 | N |
| ATOM | 787 | CA | ALYS | 1248 | 12.677 | 8.321 | 26.042 | 0.50 | 9.14 | | C |
| ANISOU | 787 | CA | ALYS | 1248 | 1082 | 921 | 1469 | 47 | -196 | 20 | C |
| ATOM | 788 | CA | BLYS | 1248 | 12.582 | 8.279 | 26.157 | 0.50 | 9.81 | | C |
| ANISOU | 788 | CA | BLYS | 1248 | 1106 | 1029 | 1594 | 157 | -256 | -131 | C |
| ATOM | 789 | CB | ALYS | 1248 | 14.135 | 8.170 | 25.558 | 0.50 | 10.58 | | C |
| ANISOU | 789 | CB | ALYS | 1248 | 1079 | 1334 | 1605 | 171 | -182 | -307 | C |
| ATOM | 790 | CB | BLYS | 1248 | 14.049 | 7.934 | 25.823 | 0.50 | 8.28 | | C |
| ANISOU | 790 | CB | BLYS | 1248 | 1154 | 703 | 1289 | 94 | -24 | 405 | C |
| ATOM | 791 | CG | ALYS | 1248 | 14.419 | 6.809 | 24.943 | 0.50 | 11.97 | | C |
| ANISOU | 791 | CG | ALYS | 1248 | 1628 | 1363 | 1556 | 386 | -310 | -248 | C |
| ATOM | 792 | CG | BLYS | 1248 | 14.148 | 6.453 | 25.465 | 0.50 | 8.15 | | C |
| ANISOU | 792 | CG | BLYS | 1248 | 955 | 625 | 1517 | -73 | 116 | 438 | C |
| ATOM | 793 | CD | ALYS | 1248 | 14.091 | 5.630 | 25.815 | 0.50 | 21.74 | | C |
| ANISOU | 793 | CD | ALYS | 1248 | 3542 | 1552 | 3168 | -377 | -1023 | 532 | C |
| ATOM | 794 | CD | BLYS | 1248 | 15.560 | 6.139 | 24.948 | 0.50 | 7.63 | | C |
| ANISOU | 794 | CD | BLYS | 1248 | 725 | 981 | 1193 | -81 | -205 | 69 | C |
| ATOM | 795 | CE | ALYS | 1248 | 14.672 | 4.302 | 25.357 | 0.50 | 27.88 | | C |
| ANISOU | 795 | CE | ALYS | 1248 | 5033 | 1399 | 4160 | -399 | -699 | 285 | C |
| ATOM | 796 | CE | BLYS | 1248 | 15.634 | 4.681 | 24.574 | 0.50 | 13.62 | | C |
| ANISOU | 796 | CE | BLYS | 1248 | 1605 | 1165 | 2406 | -106 | 707 | -306 | C |
| ATOM | 797 | NZ | ALYS | 1248 | 15.884 | 4.359 | 24.463 | 0.50 | 23.18 | | N1+ |
| ANISOU | 797 | NZ | ALYS | 1248 | 6338 | 557 | 1914 | -680 | -709 | -628 | N1+ |
| ATOM | 798 | NZ | BLYS | 1248 | 15.029 | 4.402 | 23.255 | 0.50 | 31.75 | | N1+ |
| ANISOU | 798 | NZ | BLYS | 1248 | 7203 | 2562 | 2300 | -312 | 72 | -1528 | N1+ |
| ATOM | 799 | C | ALYS | 1248 | 12.511 | 9.591 | 26.861 | 0.50 | 9.28 | | C |
| ANISOU | 799 | C | ALYS | 1248 | 1189 | 991 | 1345 | 133 | -55 | 14 | C |
| ATOM | 800 | C | BLYS | 1248 | 12.503 | 9.602 | 26.893 | 0.50 | 9.31 | | C |
| ANISOU | 800 | C | BLYS | 1248 | 1240 | 937 | 1362 | 85 | -117 | 9 | C |
| ATOM | 801 | O | ALYS | 1248 | 13.099 | 9.712 | 27.940 | 0.50 | 9.42 | | O |
| ANISOU | 801 | O | ALYS | 1248 | 1099 | 1016 | 1465 | 107 | -142 | -17 | O |
| ATOM | 802 | O | BLYS | 1248 | 13.149 | 9.768 | 27.931 | 0.50 | 9.14 | | O |
| ANISOU | 802 | O | BLYS | 1248 | 1037 | 904 | 1533 | -1 | -181 | 9 | O |
| ATOM | 803 | N | TRP | 1249 | 11.715 | 10.551 | 26.350 | 1.00 | 8.58 | | N |
| ANISOU | 803 | N | TRP | 1249 | 1004 | 903 | 1351 | 47 | -12 | -24 | N |
| ATOM | 804 | CA | TRP | 1249 | 11.465 | 11.838 | 27.057 | 1.00 | 8.48 | | C |
| ANISOU | 804 | CA | TRP | 1249 | 1030 | 752 | 1440 | -181 | 79 | -49 | C |
| ATOM | 805 | CB | TRP | 1249 | 11.708 | 13.061 | 26.125 | 1.00 | 8.69 | | C |
| ANISOU | 805 | CB | TRP | 1249 | 957 | 974 | 1372 | -51 | -108 | 170 | C |
| ATOM | 806 | CG | TRP | 1249 | 13.193 | 13.367 | 26.018 | 1.00 | 7.75 | | C |
| ANISOU | 806 | CG | TRP | 1249 | 957 | 673 | 1314 | -22 | -37 | 86 | C |
| ATOM | 807 | CD2 | TRP | 1249 | 14.147 | 12.647 | 25.209 | 1.00 | 8.70 | | C |
| ANISOU | 807 | CD2 | TRP | 1249 | 1083 | 879 | 1345 | 67 | -135 | -9 | C |
| ATOM | 808 | CE2 | TRP | 1249 | 15.402 | 13.266 | 25.414 | 1.00 | 8.28 | | C |
| ANISOU | 808 | CE2 | TRP | 1249 | 987 | 772 | 1387 | 53 | -121 | 65 | C |
| ATOM | 809 | CE3 | TRP | 1249 | 14.074 | 11.544 | 24.333 | 1.00 | 8.54 | | C |
| ANISOU | 809 | CE3 | TRP | 1249 | 1124 | 984 | 1139 | 152 | -232 | -74 | C |
| ATOM | 810 | CD1 | TRP | 1249 | 13.873 | 14.357 | 26.653 | 1.00 | 7.93 | | C |
| ANISOU | 810 | CD1 | TRP | 1249 | 953 | 798 | 1264 | -45 | -95 | 110 | C |
| ATOM | 811 | NE1 | TRP | 1249 | 15.199 | 14.305 | 26.300 | 1.00 | 8.43 | | N |
| ANISOU | 811 | NE1 | TRP | 1249 | 945 | 966 | 1292 | 40 | -115 | -47 | N |
| ATOM | 812 | CZ2 | TRP | 1249 | 16.593 | 12.854 | 24.803 | 1.00 | 9.43 | | C |
| ANISOU | 812 | CZ2 | TRP | 1249 | 1161 | 1024 | 1398 | 213 | -128 | -14 | C |
| ATOM | 813 | CZ3 | TRP | 1249 | 15.265 | 11.145 | 23.735 | 1.00 | 9.35 | | C |
| ANISOU | 813 | CZ3 | TRP | 1249 | 1172 | 1021 | 1358 | -3 | 23 | 48 | C |
| ATOM | 814 | CH2 | TRP | 1249 | 16.492 | 11.769 | 23.952 | 1.00 | 8.87 | | C |
| ANISOU | 814 | CH2 | TRP | 1249 | 1243 | 887 | 1240 | -34 | -48 | 92 | C |
| ATOM | 815 | C | TRP | 1249 | 10.052 | 11.867 | 27.576 | 1.00 | 8.29 | | C |
| ANISOU | 815 | C | TRP | 1249 | 928 | 880 | 1340 | -130 | -91 | -189 | C |
| ATOM | 816 | O | TRP | 1249 | 9.658 | 12.790 | 28.276 | 1.00 | 9.05 | | O |
| ANISOU | 816 | O | TRP | 1249 | 1129 | 964 | 1347 | -116 | 14 | -184 | O |
| ATOM | 817 | N | MET | 1250 | 9.213 | 10.862 | 27.244 | 1.00 | 8.66 | | N |
| ANISOU | 817 | N | MET | 1250 | 921 | 961 | 1406 | -146 | -220 | -41 | N |
| ATOM | 818 | CA | MET | 1250 | 7.778 | 10.926 | 27.573 | 1.00 | 8.65 | | C |
| ANISOU | 818 | CA | MET | 1250 | 881 | 997 | 1409 | -142 | -90 | -110 | C |
| ATOM | 819 | CB | MET | 1250 | 7.015 | 10.063 | 26.551 | 1.00 | 9.17 | | C |
| ANISOU | 819 | CB | MET | 1250 | 1047 | 965 | 1472 | -73 | -217 | -93 | C |
| ATOM | 820 | CO | MET | 1250 | 6.915 | 10.787 | 25.213 | 1.00 | 9.81 | | C |
| ANISOU | 820 | CG | MET | 1250 | 1272 | 1034 | 1419 | -31 | -212 | -106 | C |
| ATOM | 821 | SD | MET | 1250 | 6.299 | 9.746 | 23.864 | 1.00 | 11.09 | | S |
| ANISOU | 821 | SD | MET | 1250 | 1384 | 1297 | 1532 | -47 | -241 | -180 | S |
| ATOM | 822 | CE | MET | 1250 | 4.668 | 9.277 | 24.509 | 1.00 | 13.16 | | C |
| ANISOU | 822 | CE | MET | 1250 | 1122 | 1553 | 2324 | -68 | -312 | -187 | C |
| ATOM | 823 | C | MET | 1250 | 7.460 | 10.447 | 28.954 | 1.00 | 8.39 | | C |
| ANISOU | 823 | C | MET | 1250 | 912 | 836 | 1439 | -144 | -200 | -102 | C |
| ATOM | 824 | O | MET | 1250 | 8.046 | 9.519 | 29.537 | 1.00 | 9.38 | | O |
| ANISOU | 824 | O | MET | 1250 | 925 | 1087 | 1552 | -4 | -115 | 49 | O |
| ATOM | 825 | N | ALA | 1251 | 6.438 | 11.094 | 29.531 | 1.00 | 8.78 | | N |
| ANISOU | 825 | N | ALA | 1251 | 1069 | 892 | 1376 | -176 | -61 | -153 | N |
| ATOM | 826 | CA | ALA | 1251 | 5.923 | 10.619 | 30.811 | 1.00 | 8.54 | | C |
| ANISOU | 826 | CA | ALA | 1251 | 1053 | 802 | 1391 | -26 | -140 | -36 | C |
| ATOM | 827 | CB | ALA | 1251 | 4.892 | 11.612 | 31.359 | 1.00 | 9.51 | | C |
| ANISOU | 827 | CB | ALA | 1251 | 1141 | 782 | 1690 | -103 | 119 | -59 | C |
| ATOM | 828 | C | ALA | 1251 | 5.286 | 9.262 | 30.688 | 1.00 | 8.58 | | C |
| ANISOU | 828 | C | ALA | 1251 | 968 | 768 | 1524 | 26 | -184 | 4 | C |
| ATOM | 829 | O | ALA | 1251 | 4.736 | 8.885 | 29.620 | 1.00 | 9.32 | | O |
| ANISOU | 829 | O | ALA | 1251 | 1077 | 919 | 1547 | -78 | -152 | -37 | O |
| ATOM | 830 | N | LEU | 1252 | 5.296 | 8.482 | 31.771 | 1.00 | 9.16 | | N |
| ANISOU | 830 | N | LEU | 1252 | 1016 | 908 | 1558 | -96 | -109 | 48 | N |
| ATOM | 831 | CA | LEU | 1252 | 4.687 | 7.155 | 31.775 | 1.00 | 10.01 | | C |
| ANISOU | 831 | CA | LEU | 1252 | 1071 | 922 | 1812 | -131 | -31 | 143 | C |
| ATOM | 832 | CB | LEU | 1252 | 4.776 | 6.596 | 33.208 | 1.00 | 12.06 | | C |
| ANISOU | 832 | CB | LEU | 1252 | 1405 | 1239 | 1940 | -242 | -154 | 351 | C |
| ATOM | 833 | CG | LEU | 1252 | 3.998 | 5.268 | 33.395 | 1.00 | 13.40 | | C |
| ANISOU | 833 | CG | LEU | 1252 | 2008 | 1121 | 1962 | -385 | -196 | 278 | C |
| ATOM | 834 | CD1 | LEU | 1252 | 4.521 | 4.208 | 32.441 | 1.00 | 14.50 | | C |
| ANISOU | 834 | CD1 | LEU | 1252 | 1388 | 1196 | 2926 | 10 | 64 | 180 | C |
| ATOM | 835 | CD2 | LEU | 1252 | 4.067 | 4.880 | 34.868 | 1.00 | 19.82 | | C |
| ANISOU | 835 | CD2 | LEU | 1252 | 3335 | 1959 | 2236 | -591 | -259 | 871 | C |
| ATOM | 836 | C | LEU | 1252 | 3.264 | 7.225 | 31.270 | 1.00 | 10.09 | | C |
| ANISOU | 836 | C | LEU | 1252 | 1084 | 940 | 1810 | -211 | -75 | 14 | C |
| ATOM | 837 | O | LEU | 1252 | 2.882 | 6.401 | 30.412 | 1.00 | 11.71 | | O |
| ANISOU | 837 | O | LEU | 1252 | 1402 | 1028 | 2021 | -177 | -179 | -164 | O |
| ATOM | 838 | N | GLU | 1253 | 2.461 | 8.184 | 31.764 | 1.00 | 10.22 | | N |
| ANISOU | 838 | N | GLU | 1253 | 1098 | 1072 | 1713 | -70 | -80 | 73 | N. |
| ATOM | 839 | CA | GLU | 1253 | 1.062 | 8.178 | 31.285 | 1.00 | 10.56 | | C |
| ANISOU | 839 | CA | GLU | 1253 | 1015 | 1191 | 1804 | -192 | -79 | -87 | C |
| ATOM | 840 | CB | GLU | 1253 | 0.201 | 9.137 | 32.127 | 1.00 | 10.89 | | C |
| ANISOU | 840 | CB | GLU | 1253 | 1176 | 952 | 2011 | -34 | -217 | -164 | C |
| ATOM | 841 | CG | GLU | 1253 | 0.474 | 10.637 | 31.937 | 1.00 | 11.20 | | C |
| ANISOU | 841 | CG | GLU | 1253 | 1042 | 1050 | 2166 | -305 | -334 | 101 | C |
| ATOM | 842 | CD | GLU | 1253 | 1.617 | 11.161 | 32.766 | 1.00 | 10.23 | | C |
| ANISOU | 842 | CD | GLU | 1253 | 970 | 1027 | 1891 | -39 | -267 | 8 | C |
| ATOM | 843 | OE1 | GLU | 1253 | 2.374 | 10.378 | 33.400 | 1.00 | 9.77 | | O1- |
| ANISOU | 843 | OE1 | GLU | 1253 | 1080 | 977 | 1656 | -10 | -226 | -123 | O1- |
| ATOM | 844 | OE2 | GLU | 1253 | 1.769 | 12.415 | 32.774 | 1.00 | 9.75 | | O |
| ANISOU | 844 | OE2 | GLU | 1253 | 1054 | 1036 | 1617 | -9 | -345 | -46 | O |
| ATOM | 845 | C | GLU | 1253 | 0.981 | 8.473 | 29.784 | 1.00 | 10.97 | | C |
| ANISOU | 845 | C | GLU | 1253 | 1117 | 1195 | 1855 | -123 | -206 | -28 | C |
| ATOM | 846 | O | GLU | 1253 | 0.063 | 7.923 | 29.125 | 1.00 | 13.27 | | O |
| ANISOU | 846 | O | GLU | 1253 | 1399 | 1701 | 1943 | -518 | -290 | 39 | O |
| ATOM | 847 | N | SER | 1254 | 1.850 | 9.287 | 29.221 | 1.00 | 10.26 | | N |
| ANISOU | 847 | N | SER | 1254 | 1237 | 991 | 1672 | -143 | -257 | -69 | N |
| ATOM | 848 | CA | SER | 1254 | 1.807 | 9.558 | 27.789 | 1.00 | 10.71 | | C |
| ANISOU | 848 | CA | SER | 1254 | 1341 | 1005 | 1724 | -59 | -280 | -44 | C |
| ATOM | 849 | CB | SER | 1254 | 2.744 | 10.707 | 27.417 | 1.00 | 10.39 | | C |
| ANISOU | 849 | CB | SER | 1254 | 1341 | 980 | 1627 | -103 | -176 | -156 | C |
| ATOM | 850 | OG | SER | 1254 | 2.429 | 11.869 | 28.160 | 1.00 | 10.02 | | O |
| ANISOU | 850 | OG | SER | 1254 | 1180 | 1045 | 1582 | 37 | -205 | -149 | O |
| ATOM | 851 | C | SER | 1254 | 2.207 | 8.343 | 26.984 | 1.00 | 10.47 | | C |
| ANISOU | 851 | C | SER | 1254 | 1159 | 1045 | 1773 | -43 | -326 | -131 | C |
| ATOM | 852 | O | SER | 1254 | 1.714 | 8.104 | 25.867 | 1.00 | 11.49 | | O |
| ANISOU | 852 | O | SER | 1254 | 1533 | 1155 | 1678 | -37 | -322 | -78 | O |
| ATOM | 853 | N | LEU | 1255 | 3.142 | 7.531 | 27.514 | 1.00 | 10.60 | | N |
| ANISOU | 853 | N | LEU | 1255 | 1348 | 984 | 1694 | -16 | -328 | -168 | N |
| ATOM | 854 | CA | LEU | 1255 | 3.426 | 6.252 | 26.846 | 1.00 | 11.75 | | C |
| ANISOU | 854 | CA | LEU | 1255 | 1418 | 872 | 2173 | -4 | -392 | -191 | C |
| ATOM | 855 | CB | LEU | 1255 | 4.612 | 5.589 | 27.552 | 1.00 | 11.19 | | C |
| ANISOU | 855 | CB | LEU | 1255 | 1396 | 946 | 1910 | -103 | -333 | -85 | C |
| ATOM | 856 | CG | LEU | 1255 | 5.949 | 6.310 | 27.337 | 1.00 | 11.29 | | C |
| ANISOU | 856 | CG | LEU | 1255 | 1349 | 1000 | 1940 | -75 | -146 | 13 | C |
| ATOM | 857 | CD1 | LEU | 1255 | 6.924 | 5.851 | 28.433 | 1.00 | 13.05 | | C |
| ANISOU | 857 | CD1 | LEU | 1255 | 1529 | 1118 | 2311 | -99 | -479 | -71 | C |
| ATOM | 858 | CD2 | LEU | 1255 | 6.512 | 6.078 | 25.958 | 1.00 | 13.40 | | C |
| ANISOU | 858 | CD2 | LEU | 1255 | 1525 | 1468 | 2098 | 83 | -34 | -183 | C |
| ATOM | 859 | C | LEU | 1255 | 2.217 | 5.357 | 26.847 | 1.00 | 11.82 | | C |
| ANISOU | 859 | C | LEU | 1255 | 1463 | 1052 | 1974 | -104 | -428 | -175 | C |
| ATOM | 860 | O | LEU | 1255 | 2.002 | 4.562 | 25.902 | 1.00 | 16.25 | | O |
| ANISOU | 860 | O | LEU | 1255 | 2075 | 1654 | 2445 | -310 | -339 | -736 | O |
| ATOM | 861 | N | GLN | 1256 | 1.379 | 5.441 | 27.868 | 1.00 | 12.35 | | N |
| ANISOU | 861 | N | GLN | 1256 | 1400 | 1097 | 2195 | -177 | -339 | -212 | N |
| ATOM | 862 | CA | GLN | 1256 | 0.241 | 4.536 | 27.950 | 1.00 | 14.25 | | C |
| ANISOU | 862 | CA | GLN | 1256 | 1413 | 1306 | 2695 | -287 | -429 | 49 | C |
| ATOM | 863 | CB | GLN | 1256 | -0.158 | 4.360 | 29.406 | 1.00 | 15.54 | | C |
| ANISOU | 863 | CB | GLN | 1256 | 1577 | 1638 | 2689 | -420 | -360 | 130 | C |
| ATOM | 864 | CG | GLN | 1256 | 0.898 | 3.595 | 30.211 | 1.00 | 17.67 | | C |
| ANISOU | 864 | CG | GLN | 1256 | 1628 | 2052 | 3032 | -488 | -434 | 550 | C |
| ATOM | 865 | CD | GLN | 1256 | 0.579 | 3.640 | 31.702 | 1.00 | 25.26 | | C |
| ANISOU | 865 | CD | GLN | 1256 | 3689 | 2920 | 2989 | 236 | -247 | 1030 | C |
| ATOM | 866 | OE1 | GLN | 1256 | 0.571 | 2.651 | 32.409 | 1.00 | 33.40 | | O |
| ANISOU | 866 | OE1 | GLN | 1256 | 5253 | 3444 | 3991 | 371 | 457 | 1764 | O |
| ATOM | 867 | NE2 | GLN | 1256 | 0.274 | 4.834 | 32.270 | 1.00 | 20.31 | | N |
| ANISOU | 867 | NE2 | GLN | 1256 | 2108 | 2958 | 2653 | -988 | -268 | 488 | N |
| ATOM | 868 | C | GLN | 1256 | -0.979 | 5.040 | 27.162 | 1.00 | 14.41 | | C |
| ANISOU | 868 | C | GLN | 1256 | 1496 | 1276 | 2704 | -221 | -565 | -290 | C |
| ATOM | 869 | O | GLN | 1256 | -1.744 | 4.212 | 26.666 | 1.00 | 19.15 | | O |
| ANISOU | 869 | O | GLN | 1256 | 1968 | 1471 | 3839 | -449 | -1153 | -302 | O |
| ATOM | 870 | N | THR | 1257 | -1.161 | 6.367 | 27.054 | 1.00 | 13.58 | | N |
| ANISOU | 870 | N | THR | 1257 | 1400 | 1266 | 2493 | -202 | -545 | -226 | N |
| ATOM | 871 | CA | THR | 1257 | -2.367 | 6.945 | 26.491 | 1.00 | 13.00 | | C |
| ANISOU | 871 | CA | THR | 1257 | 1102 | 1487 | 2350 | -205 | -258 | -199 | C |
| ATOM | 872 | CB | THR | 1257 | -3.095 | 7.860 | 27.520 | 1.00 | 14.02 | | C |
| ANISOU | 872 | CB | THR | 1257 | 1477 | 1687 | 2164 | -192 | -263 | -287 | C |
| ATOM | 873 | OG1 | THR | 1257 | -2.289 | 9.015 | 27.788 | 1.00 | 15.04 | | O |
| ANISOU | 873 | OG1 | THR | 1257 | 1625 | 1493 | 2599 | -61 | -332 | -289 | O |
| ATOM | 874 | CG2 | THR | 1257 | -3.328 | 7.100 | 28.825 | 1.00 | 17.43 | | C |
| ANISOU | 874 | CG2 | THR | 1257 | 1797 | 2560 | 2265 | -614 | -154 | -53 | C |
| ATOM | 875 | C | THR | 1257 | -2.121 | 7.799 | 25.260 | 1.00 | 12.77 | | C |
| ANISOU | 875 | C | THR | 1257 | 1453 | 1369 | 2029 | -169 | -389 | -452 | C |
| ATOM | 876 | O | THR | 1257 | -3.102 | 8.153 | 24.589 | 1.00 | 14.50 | | O |
| ANISOU | 876 | O | THR | 1257 | 1622 | 1472 | 2415 | 14 | -567 | -386 | O |
| ATOM | 877 | N | GLN | 1258 | -0.866 | 8.123 | 24.996 | 1.00 | 12.61 | | N |
| ANISOU | 877 | N | GLN | 1258 | 1460 | 1390 | 1940 | -295 | -206 | -461 | N |
| ATOM | 878 | CA | GLN | 1258 | -0.475 | 9.058 | 23.933 | 1.00 | 13.06 | | C |
| ANISOU | 878 | CA | GLN | 1258 | 1506 | 1582 | 1874 | 34 | -302 | -327 | C |
| ATOM | 879 | CB | GLN | 1258 | -0.763 | 8.510 | 22.508 | 1.00 | 15.10 | | C |
| ANISOU | 879 | CB | GLN | 1258 | 2218 | 1586 | 1934 | 247 | -720 | -361 | C |
| ATOM | 880 | CG | GLN | 1258 | 0.195 | 7.368 | 22.136 | 1.00 | 27.52 | | C |
| ANISOU | 880 | CG | GLN | 1258 | 4054 | 3442 | 2959 | 1580 | -257 | -1265 | C |
| ATOM | 881 | CD | GLN | 1258 | 0.030 | 6.833 | 20.720 | 1.00 | 29.05 | | C |
| ANISOU | 881 | CD | GLN | 1258 | 4243 | 3526 | 3268 | 575 | 167 | -1667 | C |
| ATOM | 882 | OE1 | GLN | 1258 | 0.056 | 7.547 | 19.703 | 1.00 | 29.12 | | O |
| ANISOU | 882 | OE1 | GLN | 1258 | 2551 | 5558 | 2956 | 338 | -369 | -1137 | O |
| ATOM | 883 | NE2 | GLN | 1258 | -0.144 | 5.520 | 20.642 | 1.00 | 50.54 | | N |
| ANISOU | 883 | NE2 | GLN | 1258 | 9112 | 3861 | 6229 | -732 | -858 | -2302 | N |
| ATOM | 884 | C | GLN | 1258 | -1.111 | 10.436 | 24.128 | 1.00 | 12.97 | | C |
| ANISOU | 884 | C | GLN | 1258 | 1335 | 1540 | 2053 | 38 | -421 | -330 | C |
| ATOM | 885 | O | GLN | 1258 | -1.295 | 11.139 | 23.139 | 1.00 | 17.50 | | O |
| ANISOU | 885 | O | GLN | 1258 | 2771 | 1703 | 2175 | 332 | -305 | -211 | O |
| ATOM | 886 | N | LYS | 1259 | -1.399 | 10.818 | 25.358 | 1.00 | 12.05 | | N |
| ANISOU | 886 | N | LYS | 1259 | 1110 | 1372 | 2096 | -30 | -432 | -361 | N |
| ATOM | 887 | CA | LYS | 1259 | -1.856 | 12.156 | 25.682 | 1.00 | 13.46 | | C |
| ANISOU | 887 | CA | LYS | 1259 | 1412 | 1410 | 2290 | -81 | -500 | -552 | C |
| ATOM | 888 | CB | LYS | 1259 | -3.134 | 12.102 | 26.533 | 1.00 | 13.68 | | C |
| ANISOU | 888 | CB | LYS | 1259 | 1378 | 1409 | 2413 | 7 | -435 | -561 | C |
| ATOM | 889 | CG | LYS | 1259 | -4.299 | 11.378 | 25.839 | 1.00 | 17.48 | | C |
| ANISOU | 889 | CG | LYS | 1259 | 1572 | 1747 | 3323 | -424 | -570 | -618 | C |
| ATOM | 890 | CD | LYS | 1259 | -5.560 | 11.502 | 26.639 | 1.00 | 27.58 | | C |
| ANISOU | 890 | CD | LYS | 1259 | 1733 | 3758 | 4986 | -613 | 57 | -489 | C |
| ATOM | 891 | CE | LYS | 1259 | -5.581 | 10.720 | 27.941 | 1.00 | 39.60 | | C |
| ANISOU | 891 | CE | LYS | 1259 | 4356 | 4331 | 6358 | -482 | 2233 | 823 | C |
| ATOM | 892 | NZ | LYS | 1259 | -6.900 | 10.847 | 28.634 | 1.00 | 55.65 | | N1+ |
| ANISOU | 892 | NZ | LYS | 1259 | 6166 | 7166 | 7811 | -848 | 4140 | -1006 | N1+ |
| ATOM | 893 | C | LYS | 1259 | -0.788 | 12.950 | 26.438 | 1.00 | 10.47 | | C |
| ANISOU | 893 | C | LYS | 1259 | 1208 | 1064 | 1706 | 104 | -423 | -121 | C |
| ATOM | 894 | O | LYS | 1259 | -0.118 | 12.374 | 27.302 | 1.00 | 12.68 | | O |
| ANISOU | 894 | O | LYS | 1259 | 1335 | 1228 | 2256 | -10 | -523 | 233 | O |
| ATOM | 895 | N | PHE | 1260 | -0.652 | 14.221 | 26.105 | 1.00 | 9.90 | | N |
| ANISOU | 895 | N | PHE | 1260 | 1071 | 1101 | 1590 | 61 | -293 | -118 | N |
| ATOM | 896 | CA | PHE | 1260 | 0.368 | 15.094 | 26.671 | 1.00 | 9.35 | | C |
| ANISOU | 896 | CA | PHE | 1260 | 846 | 1223 | 1482 | -10 | -226 | -65 | C |
| ATOM | 897 | CB | PHE | 1260 | 1.205 | 15.610 | 25.518 | 1.00 | 9.78 | | C |
| ANISOU | 897 | CB | PHE | 1260 | 1015 | 1258 | 1444 | 239 | -100 | 100 | C |
| ATOM | 898 | CG | PHE | 1260 | 2.037 | 14.570 | 24.764 | 1.00 | 9.93 | | C |
| ANISOU | 898 | CG | PHE | 1260 | 1091 | 1189 | 1493 | 117 | -131 | 30 | C |
| ATOM | 899 | CD1 | PHE | 1260 | 1.426 | 13.739 | 23.827 | 1.00 | 12.20 | | C |
| ANISOU | 899 | CD1 | PHE | 1260 | 1442 | 1429 | 1763 | -47 | -31 | -248 | C |
| ATOM | 900 | CD2 | PHE | 1260 | 3.403 | 14.445 | 25.016 | 1.00 | 9.98 | | C |
| ANISOU | 900 | CD2 | PHE | 1260 | 1030 | 1132 | 1629 | 290 | 7 | 199 | C |
| ATOM | 901 | CE1 | PHE | 1260 | 2.185 | 12.787 | 23.150 | 1.00 | 12.36 | | C |
| ANISOU | 901 | CE1 | PHE | 1260 | 1117 | 2008 | 1569 | 148 | -134 | -387 | C |
| ATOM | 902 | CE2 | PHE | 1260 | 4.145 | 13.515 | 24.317 | 1.00 | 9.86 | | C |
| ANISOU | 902 | CE2 | PHE | 1260 | 1131 | 998 | 1618 | 52 | -106 | -70 | C |
| ATOM | 903 | CZ | PHE | 1260 | 3.547 | 12.668 | 23.357 | 1.00 | 11.24 | | C |
| ANISOU | 903 | CZ | PHE | 1260 | 1120 | 1418 | 1733 | -165 | -259 | -42 | C |
| ATOM | 904 | C | PHE | 1260 | -0.225 | 16.264 | 27.418 | 1.00 | 9.28 | | C |
| ANISOU | 904 | C | PHE | 1260 | 871 | 1209 | 1447 | -47 | -157 | -64 | C |
| ATOM | 905 | O | PHE | 1260 | -1.337 | 16.736 | 27.026 | 1.00 | 10.47 | | O |
| ANISOU | 905 | O | PHE | 1260 | 924 | 1259 | 1793 | 40 | -303 | -96 | O |
| ATOM | 906 | N | THR | 1261 | 0.443 | 16.749 | 28.454 | 1.00 | 9.04 | | N |
| ANISOU | 906 | N | THR | 1261 | 1043 | 909 | 1482 | -23 | -249 | 20 | N |
| ATOM | 907 | CA | THR | 1261 | -0.016 | 17.823 | 29.305 | 1.00 | 8.91 | | C |
| ANISOU | 907 | CA | THR | 1261 | 942 | 1087 | 1357 | -35 | -77 | 13 | C |
| ATOM | 908 | CB | THR | 1261 | -0.725 | 17.340 | 30.585 | 1.00 | 9.86 | | C |
| ANISOU | 908 | CB | THR | 1261 | 989 | 1226 | 1531 | -57 | -116 | 240 | C |
| ATOM | 909 | OG1 | THR | 1261 | 0.289 | 16.658 | 31.382 | 1.00 | 9.74 | | O |
| ANISOU | 909 | OG1 | THR | 1261 | 993 | 1265 | 1441 | -60 | -226 | 120 | O |
| ATOM | 910 | CG2 | THR | 1261 | -1.887 | 16.398 | 30.267 | 1.00 | 10.68 | | C |
| ANISOU | 910 | CG2 | THR | 1261 | 838 | 1633 | 1588 | -132 | -151 | 173 | C |
| ATOM | 911 | C | THR | 1261 | 1.182 | 18.639 | 29.764 | 1.00 | 7.61 | | C |
| ANISOU | 911 | C | THR | 1261 | 817 | 841 | 1233 | 144 | -203 | 165 | C |
| ATOM | 912 | O | THR | 1261 | 2.349 | 18.273 | 29.550 | 1.00 | 8.44 | | O |
| ANISOU | 912 | O | THR | 1261 | 838 | 968 | 1400 | 24 | -121 | 21 | O |
| ATOM | 913 | N | THR | 1262 | 0.909 | 19.760 | 30.453 | 1.00 | 8.82 | | N |
| ANISOU | 913 | N | THR | 1262 | 1028 | 949 | 1374 | 106 | -117 | 28 | N |
| ATOM | 914 | CA | THR | 1262 | 2.019 | 20.496 | 31.050 | 1.00 | 9.61 | | C |
| ANISOU | 914 | CA | THR | 1262 | 1246 | 1075 | 1332 | 136 | -216 | -52 | C |
| ATOM | 915 | CB | THR | 1262 | 1.514 | 21.803 | 31.709 | 1.00 | 10.84 | | C |
| ANISOU | 915 | CB | THR | 1262 | 1416 | 1102 | 1600 | 280 | 27 | -244 | C |
| ATOM | 916 | OG1 | THR | 1262 | 1.244 | 22.765 | 30.710 | 1.00 | 15.47 | | O |
| ANISOU | 916 | OG1 | THR | 1262 | 2206 | 1297 | 2374 | 325 | 441 | 180 | O |
| ATOM | 917 | CG2 | THR | 1262 | 2.583 | 22.456 | 32.507 | 1.00 | 13.18 | | C |
| ANISOU | 917 | CG2 | THR | 1262 | 1070 | 1106 | 2831 | -219 | 400 | -612 | C |
| ATOM | 918 | C | THR | 1262 | 2.759 | 19.604 | 32.050 | 1.00 | 8.07 | | C |
| ANISOU | 918 | C | THR | 1262 | 897 | 905 | 1262 | -41 | -28 | 43 | C |
| ATOM | 919 | O | THR | 1262 | 3.977 | 19.743 | 32.175 | 1.00 | 8.72 | | O |
| ANISOU | 919 | O | THR | 1262 | 1046 | 951 | 1317 | -130 | -169 | 73 | O |
| ATOM | 920 | N | LYS | 1263 | 2.094 | 18.698 | 32.767 | 1.00 | 8.94 | | N |
| ANISOU | 920 | N | LYS | 1263 | 985 | 858 | 1553 | 45 | 0 | 98 | N |
| ATOM | 921 | CA | LYS | 1263 | 2.813 | 17.825 | 33.712 | 1.00 | 8.70 | | C |
| ANISOU | 921 | CA | LYS | 1263 | 982 | 908 | 1416 | 104 | 43 | 96 | C |
| ATOM | 922 | CB | LYS | 1263 | 1.832 | 17.135 | 34.678 | 1.00 | 10.07 | | C |
| ANISOU | 922 | CB | LYS | 1263 | 1076 | 1477 | 1275 | 96 | 96 | 145 | C |
| ATOM | 923 | CG | LYS | 1263 | 1.227 | 18.072 | 35.713 | 1.00 | 10.66 | | C |
| ANISOU | 923 | CG | LYS | 1263 | 1224 | 1377 | 1448 | 237 | 79 | 99 | C |
| ATOM | 924 | CD | LYS | 1263 | 2.288 | 18.805 | 36.545 | 1.00 | 11.75 | | C |
| ANISOU | 924 | CD | LYS | 1263 | 1599 | 1241 | 1624 | 181 | 6 | 9 | C |
| ATOM | 925 | CE | LYS | 1263 | 1.583 | 19.329 | 37.814 | 1.00 | 13.94 | | C |
| ANISOU | 925 | CE | LYS | 1263 | 1812 | 1617 | 1869 | -43 | 217 | -304 | C |
| ATOM | 926 | NZ | LYS | 1263 | 2.476 | 20.303 | 38.497 | 1.00 | 14.77 | | N1+ |
| ANISOU | 926 | NZ | LYS | 1263 | 1949 | 1712 | 1952 | 359 | -493 | -284 | N1+ |
| ATOM | 927 | C | LYS | 1263 | 3.645 | 16.782 | 32.960 | 1.00 | 8.04 | | C |
| ANISOU | 927 | C | LYS | 1263 | 876 | 835 | 1342 | 65 | -42 | 70 | C |
| ATOM | 928 | O | LYS | 1263 | 4.644 | 16.330 | 33.528 | 1.00 | 8.67 | | O |
| ANISOU | 928 | O | LYS | 1263 | 843 | 851 | 1600 | -18 | -154 | 90 | O |
| ATOM | 929 | N | SER | 1264 | 3.285 | 16.377 | 31.730 | 1.00 | 8.43 | | N |
| ANISOU | 929 | N | SER | 1264 | 1062 | 820 | 1322 | 35 | -54 | 110 | N |
| ATOM | 930 | CA | SER | 1264 | 4.239 | 15.520 | 31.006 | 1.00 | 8.11 | | C |
| ANISOU | 930 | CA | SER | 1264 | 892 | 900 | 1288 | -134 | -31 | 10 | C |
| ATOM | 931 | CB | SER | 1264 | 3.545 | 14.650 | 29.938 | 1.00 | 8.33 | | C |
| ANISOU | 931 | CB | SER | 1264 | 977 | 883 | 1305 | -55 | -189 | 29 | C |
| ATOM | 932 | OG | SER | 1264 | 2.862 | 15.382 | 28.920 | 1.00 | 8.85 | | O |
| ANISOU | 932 | OG | SER | 1264 | 941 | 936 | 1486 | 87 | -185 | -29 | O |
| ATOM | 933 | C | SER | 1264 | 5.386 | 16.367 | 30.451 | 1.00 | 7.34 | | C |
| ANISOU | 933 | C | SER | 1264 | 816 | 797 | 1176 | 45 | -216 | 122 | C |
| ATOM | 934 | O | SER | 1264 | 6.478 | 15.830 | 30.253 | 1.00 | 8.07 | | O |
| ANISOU | 934 | O | SER | 1264 | 777 | 930 | 1358 | 93 | -147 | -61 | O |
| ATOM | 935 | N | ASP | 1265 | 5.201 | 17.695 | 30.212 | 1.00 | 7.88 | | N |
| ANISOU | 935 | N | ASP | 1265 | 818 | 733 | 1441 | -20 | -55 | 56 | N |
| ATOM | 936 | CA | ASP | 1265 | 6.348 | 18.545 | 29.911 | 1.00 | 7.40 | | C |
| ANISOU | 936 | CA | ASP | 1265 | 844 | 754 | 1214 | -67 | -221 | 20 | C |
| ATOM | 937 | CB | ASP | 1265 | 5.925 | 19.972 | 29.593 | 1.00 | 8.17 | | C |
| ANISOU | 937 | CB | ASP | 1265 | 946 | 802 | 1356 | -47 | -329 | 154 | C |
| ATOM | 938 | CG | ASP | 1265 | 5.376 | 20.206 | 28.183 | 1.00 | 7.76 | | C |
| ANISOU | 938 | CG | ASP | 1265 | 774 | 897 | 1276 | 71 | -65 | 161 | C |
| ATOM | 939 | OD1 | ASP | 1265 | 5.492 | 19.321 | 27.291 | 1.00 | 7.98 | | O |
| ANISOU | 939 | OD1 | ASP | 1265 | 870 | 958 | 1203 | -115 | -99 | 38 | O |
| ATOM | 940 | OD2 | ASP | 1265 | 4.841 | 21.320 | 27.975 | 1.00 | 8.32 | | O1- |
| ANISOU | 940 | OD2 | ASP | 1265 | 958 | 892 | 1311 | 77 | -163 | 188 | O1- |
| ATOM | 941 | C | ASP | 1265 | 7.299 | 18.585 | 31.103 | 1.00 | 6.76 | | C |
| ANISOU | 941 | C | ASP | 1265 | 746 | 647 | 1174 | 129 | -82 | -42 | C |
| ATOM | 942 | O | ASP | 1265 | 8.520 | 18.634 | 30.897 | 1.00 | 7.39 | | O |
| ANISOU | 942 | O | ASP | 1265 | 749 | 916 | 1144 | 54 | -79 | 2 | O |
| ATOM | 943 | N | VAL | 1266 | 6.753 | 18.579 | 32.336 | 1.00 | 7.24 | | N |
| ANISOU | 943 | N | VAL | 1266 | 904 | 721 | 1124 | 25 | -16 | 46 | N |
| ATOM | 944 | CA | VAL | 1266 | 7.633 | 18.521 | 33.526 | 1.00 | 7.58 | | C |
| ANISOU | 944 | CA | VAL | 1266 | 886 | 831 | 1160 | 201 | -39 | -33 | C |
| ATOM | 945 | CB | VAL | 1266 | 6.784 | 18.670 | 34.809 | 1.00 | 7.71 | | C |
| ANISOU | 945 | CB | VAL | 1266 | 955 | 838 | 1136 | 178 | -55 | -7 | C |
| ATOM | 946 | CG1 | VAL | 1266 | 7.629 | 18.366 | 36.036 | 1.00 | 8.14 | | C |
| ANISOU | 946 | CG1 | VAL | 1266 | 999 | 958 | 1135 | 140 | -84 | -59 | C |
| ATOM | 947 | CG2 | VAL | 1266 | 6.170 | 20.085 | 34.890 | 1.00 | 8.87 | | C |
| ANISOU | 947 | CG2 | VAL | 1266 | 1004 | 919 | 1447 | 314 | 22 | -114 | C |
| ATOM | 948 | C | VAL | 1266 | 8.458 | 17.268 | 33.523 | 1.00 | 7.30 | | C |
| ANISOU | 948 | C | VAL | 1266 | 788 | 781 | 1207 | 45 | -120 | -86 | C |
| ATOM | 949 | O | VAL | 1266 | 9.671 | 17.313 | 33.812 | 1.00 | 7.44 | | O |
| ANISOU | 949 | O | VAL | 1266 | 711 | 864 | 1252 | 48 | -95 | 12 | O |
| ATOM | 950 | N | TRP | 1267 | 7.853 | 16.108 | 33.200 | 1.00 | 7.36 | | N |
| ANISOU | 950 | N | TRP | 1267 | 799 | 706 | 1291 | -35 | 28 | 101 | N |
| ATOM | 951 | CA | TRP | 1267 | 8.630 | 14.883 | 33.089 | 1.00 | 7.73 | | C |
| ANISOU | 951 | CA | TRP | 1267 | 967 | 731 | 1238 | -62 | 68 | -181 | C |
| ATOM | 952 | CB | TRP | 1267 | 7.673 | 13.725 | 32.643 | 1.00 | 8.01 | | C |
| ANISOU | 952 | CB | TRP | 1267 | 832 | 780 | 1431 | -71 | -137 | -29 | C |
| ATOM | 953 | CG | TRP | 1267 | 8.413 | 12.393 | 32.544 | 1.00 | 7.77 | | C |
| ANISOU | 953 | CG | TRP | 1267 | 899 | 699 | 1356 | -70 | -125 | 2 | C |
| ATOM | 954 | CD2 | TRP | 1267 | 8.136 | 11.215 | 33.337 | 1.00 | 8.17 | | C |
| ANISOU | 954 | CD2 | TRP | 1267 | 813 | 854 | 1439 | -75 | -191 | 53 | C |
| ATOM | 955 | CE2 | TRP | 1267 | 9.045 | 10.219 | 32.923 | 1.00 | 7.97 | | C |
| ATOM | 956 | CE3 | TRP | 1267 | 7.217 | 10.914 | 34.336 | 1.00 | 8.27 | | C |
| ANISOU | 956 | CE3 | TRP | 1267 | 1093 | 793 | 1256 | -282 | -154 | -40 | C |
| ATOM | 957 | CD1 | TRP | 1267 | 9.450 | 11.997 | 31.730 | 1.00 | 7.98 | | C |
| ANISOU | 957 | CD1 | TRP | 1267 | 851 | 741 | 1439 | -53 | -136 | -21 | C |
| ATOM | 958 | NE1 | TRP | 1267 | 9.835 | 10.749 | 31.944 | 1.00 | 8.02 | | N |
| ANISOU | 958 | NE1 | TRP | 1267 | 1153 | 583 | 1311 | -19 | -153 | -16 | N |
| ATOM | 959 | CZ2 | TRP | 1267 | 9.066 | 8.931 | 33.488 | 1.00 | 9.37 | | C |
| ANISOU | 959 | CZ2 | TRP | 1267 | 1231 | 792 | 1539 | -133 | -285 | 120 | C |
| ATOM | 960 | CZ3 | TRP | 1267 | 7.220 | 9.645 | 34.904 | 1.00 | 9.43 | | C |
| ANISOU | 960 | CZ3 | TRP | 1267 | 1135 | 868 | 1582 | -195 | -352 | 128 | C |
| ATOM | 961 | CH2 | TRP | 1267 | 8.147 | 8.658 | 34.471 | 1.00 | 9.55 | | C |
| ANISOU | 961 | CH2 | TRP | 1267 | 1061 | 931 | 1635 | -162 | -290 | 147 | C |
| ATOM | 962 | C | TRP | 1267 | 9.788 | 15.067 | 32.135 | 1.00 | 7.32 | | C |
| ANISOU | 962 | C | TRP | 1267 | 739 | 753 | 1291 | -18 | -76 | 25 | C |
| ATOM | 963 | O | TRP | 1267 | 10.953 | 14.743 | 32.421 | 1.00 | 7.52 | | O |
| ANISOU | 963 | O | TRP | 1267 | 807 | 744 | 1306 | 58 | -86 | 18 | O |
| ATOM | 964 | N | SER | 1268 | 9.488 | 15.533 | 30.906 | 1.00 | 7.16 | | N |
| ANISOU | 964 | N | SER | 1268 | 836 | 704 | 1180 | -59 | -59 | -99 | N |
| ATOM | 965 | CA | SER | 1268 | 10.514 | 15.718 | 29.883 | 1.00 | 7.21 | | C |
| ANISOU | 965 | CA | SER | 1268 | 814 | 943 | 983 | -96 | -157 | -158 | C |
| ATOM | 966 | CB | SER | 1268 | 9.934 | 16.279 | 28.588 | 1.00 | 8.08 | | C |
| ANISOU | 966 | CB | SER | 1268 | 940 | 1030 | 1100 | -32 | -288 | -132 | C |
| ATOM | 967 | OG | SER | 1268 | 8.923 | 15.395 | 28.105 | 1.00 | 8.68 | | O |
| ANISOU | 967 | OG | SER | 1268 | 933 | 1281 | 1083 | -106 | -185 | -291 | O |
| ATOM | 968 | C | SER | 1268 | 11.598 | 16.669 | 30.373 | 1.00 | 6.52 | | C |
| ANISOU | 968 | C | SER | 1268 | 775 | 747 | 955 | -60 | -99 | -25 | C |
| ATOM | 969 | O | SER | 1268 | 12.760 | 16.452 | 30.090 | 1.00 | 7.61 | | O |
| ANISOU | 969 | O | SER | 1268 | 729 | 890 | 1273 | -16 | -93 | -55 | O |
| ATOM | 970 | N | PHE | 1269 | 11.204 | 17.719 | 31.093 | 1.00 | 7.11 | | N |
| ANISOU | 970 | N | PHE | 1269 | 928 | 738 | 1036 | -63 | -114 | -59 | N |
| ATOM | 971 | CA | PHE | 1269 | 12.197 | 18.622 | 31.664 | 1.00 | 7.37 | | C |
| ANISOU | 971 | CA | PHE | 1269 | 912 | 796 | 1091 | -38 | -181 | -96 | C |
| ATOM | 972 | CB | PHE | 1269 | 11.443 | 19.747 | 32.412 | 1.00 | 7.74 | | C |
| ANISOU | 972 | CB | PHE | 1269 | 908 | 674 | 1359 | 11 | -131 | -131 | C |
| ATOM | 973 | CG | PHE | 1269 | 12.443 | 20.664 | 33.096 | 1.00 | 7.06 | | C |
| ANISOU | 973 | CG | PHE | 1269 | 727 | 735 | 1222 | 90 | -116 | -48 | C |
| ATOM | 974 | CD1 | PHE | 1269 | 13.211 | 21.577 | 32.359 | 1.00 | 8.64 | | C |
| ANISOU | 974 | CD1 | PHE | 1269 | 1030 | 794 | 1459 | -123 | -45 | -94 | C |
| ATOM | 975 | CD2 | PHE | 1269 | 12.635 | 20.611 | 34.465 | 1.00 | 8.40 | | C |
| ANISOU | 975 | CD2 | PHE | 1269 | 1050 | 926 | 1216 | -10 | -115 | -222 | C |
| ATOM | 976 | CE1 | PHE | 1269 | 14.147 | 22.417 | 32.983 | 1.00 | 8.28 | | C |
| ANISOU | 976 | CE1 | PHE | 1269 | 871 | 882 | 1394 | 0 | -82 | -117 | C |
| ATOM | 977 | CE2 | PHE | 1269 | 13.546 | 21.435 | 35.071 | 1.00 | 9.50 | | C |
| ANISOU | 977 | CE2 | PHE | 1269 | 1144 | 1048 | 1417 | -234 | -251 | -38 | C |
| ATOM | 978 | CZ | PHE | 1269 | 14.313 | 22.316 | 34.363 | 1.00 | 8.75 | | C |
| ANISOU | 978 | CZ | PHE | 1269 | 1129 | 863 | 1332 | -29 | -81 | -71 | C |
| ATOM | 979 | C | PHE | 1269 | 13.143 | 17.892 | 32.575 | 1.00 | 6.90 | | C |
| ANISOU | 979 | C | PHE | 1269 | 703 | 771 | 1148 | 41 | 8 | -57 | C |
| ATOM | 980 | O | PHE | 1269 | 14.345 | 18.158 | 32.565 | 1.00 | 7.15 | | O |
| ANISOU | 980 | O | PHE | 1269 | 776 | 764 | 1178 | 27 | -34 | -100 | O |
| ATOM | 981 | N | GLY | 1270 | 12.657 | 16.947 | 33.403 | 1.00 | 7.57 | | N |
| ANISOU | 981 | N | GLY | 1270 | 1118 | 633 | 1126 | -8 | -144 | -83 | N |
| ATOM | 982 | CA | GLY | 1270 | 13.594 | 16.170 | 34.225 | 1.00 | 7.83 | | C |
| ANISOU | 982 | CA | GLY | 1270 | 962 | 772 | 1241 | 156 | 0 | -12 | C |
| ATOM | 983 | C | GLY | 1270 | 14.597 | 15.408 | 33.386 | 1.00 | 7.57 | | C |
| ANISOU | 983 | C | GLY | 1270 | 913 | 789 | 1174 | 29 | -29 | -36 | C |
| ATOM | 984 | O | GLY | 1270 | 15.776 | 15.324 | 33.736 | 1.00 | 7.73 | | O |
| ANISOU | 984 | O | GLY | 1270 | 917 | 887 | 1133 | 84 | -108 | 40 | O |
| ATOM | 985 | N | VAL | 1271 | 14.126 | 14.821 | 32.251 | 1.00 | 7.23 | | N |
| ANISOU | 985 | N | VAL | 1271 | 818 | 715 | 1215 | -31 | 38 | -55 | N |
| ATOM | 986 | CA | VAL | 1271 | 15.097 | 14.135 | 31.367 | 1.00 | 7.44 | | C |
| ANISOU | 986 | CA | VAL | 1271 | 927 | 778 | 1123 | -35 | 5 | -7 | C |
| ATOM | 987 | CB | VAL | 1271 | 14.351 | 13.356 | 30.259 | 1.00 | 7.77 | | C |
| ANISOU | 987 | CB | VAL | 1271 | 1007 | 896 | 1050 | -119 | 33 | -43 | C |
| ATOM | 988 | CG1 | VAL | 1271 | 15.389 | 12.624 | 29.387 | 1.00 | 8.65 | | C |
| ANISOU | 988 | CG1 | VAL | 1271 | 997 | 1157 | 1131 | 93 | -62 | -99 | C |
| ATOM | 989 | CG2 | VAL | 1271 | 13.332 | 12.384 | 30.846 | 1.00 | 8.36 | | C |
| ANISOU | 989 | CG2 | VAL | 1271 | 1066 | 729 | 1381 | -76 | 51 | 43 | C |
| ATOM | 990 | C | VAL | 1271 | 16.087 | 15.131 | 30.795 | 1.00 | 6.88 | | C |
| ANISOU | 990 | C | VAL | 1271 | 806 | 703 | 1104 | 13 | 52 | -66 | C |
| ATOM | 991 | O | VAL | 1271 | 17.268 | 14.845 | 30.710 | 1.00 | 7.71 | | O |
| ANISOU | 991 | O | VAL | 1271 | 824 | 805 | 1302 | 46 | -69 | -83 | O |
| ATOM | 992 | N | LEU | 1272 | 15.620 | 16.326 | 30.397 | 1.00 | 7.21 | | N |
| ANISOU | 992 | N | LEU | 1272 | 940 | 683 | 1119 | -85 | -32 | 39 | N |
| ATOM | 993 | CA | LEU | 1272 | 16.534 | 17.350 | 29.901 | 1.00 | 7.04 | | C |
| ANISOU | 993 | CA | LEU | 1272 | 838 | 621 | 1216 | -81 | -184 | 8 | C |
| ATOM | 994 | CB | LEU | 1272 | 15.653 | 18.556 | 29.495 | 1.00 | 7.68 | | C |
| ANISOU | 994 | CB | LEU | 1272 | 857 | 783 | 1279 | 58 | -68 | 198 | C |
| ATOM | 995 | CG | LEU | 1272 | 16.433 | 19.702 | 28.801 | 1.00 | 8.64 | | C |
| ANISOU | 995 | CG | LEU | 1272 | 994 | 782 | 1508 | -9 | -92 | 256 | C |
| ATOM | 996 | CD1 | LEU | 1272 | 15.489 | 20.459 | 27.866 | 1.00 | 9.57 | | C |
| ANISOU | 996 | CD1 | LEU | 1272 | 1351 | 990 | 1297 | 219 | -203 | 185 | C |
| ATOM | 997 | CD2 | LEU | 1272 | 17.031 | 20.708 | 29.753 | 1.00 | 11.08 | | C |
| ANISOU | 997 | CD2 | LEU | 1272 | 1282 | 1065 | 1863 | -304 | -528 | 365 | C |
| ATOM | 998 | C | LEU | 1272 | 17.568 | 17.706 | 30.939 | 1.00 | 7.19 | | C |
| ANISOU | 998 | C | LEU | 1272 | 772 | 835 | 1126 | 56 | -36 | -145 | C |
| ATOM | 999 | O | LEU | 1272 | 18.756 | 17.884 | 30.611 | 1.00 | 7.61 | | O |
| ANISOU | 999 | O | LEU | 1272 | 745 | 863 | 1281 | 59 | -51 | 77 | O |
| ATOM | 1000 | N | LEU | 1273 | 17.180 | 17.808 | 32.230 | 1.00 | 8.06 | | N |
| ANISOU | 1000 | N | LEU | 1273 | 1018 | 966 | 1080 | -54 | -68 | -115 | N |
| ATOM | 1001 | CA | LEU | 1273 | 18.221 | 18.053 | 33.239 | 1.00 | 7.63 | | C |
| ANISOU | 1001 | CA | LEU | 1273 | 882 | 940 | 1076 | -14 | -36 | 34 | C |
| ATOM | 1002 | CB | LEU | 1273 | 17.571 | 18.155 | 34.654 | 1.00 | 8.61 | | C |
| ANISOU | 1002 | CB | LEU | 1273 | 1131 | 1003 | 1137 | 37 | 40 | -11 | C |
| ATOM | 1003 | CG | LEU | 1273 | 16.765 | 19.385 | 34.970 | 1.00 | 8.37 | | C |
| ANISOU | 1003 | CG | LEU | 1273 | 902 | 1042 | 1236 | -24 | 7 | -97 | C |
| ATOM | 1004 | CD1 | LEU | 1273 | 16.153 | 19.237 | 36.370 | 1.00 | 10.51 | | C |
| ANISOU | 1004 | CD1 | LEU | 1273 | 1428 | 1265 | 1301 | 168 | 245 | 173 | C |
| ATOM | 1005 | CD2 | LEU | 1273 | 17.627 | 20.637 | 34.874 | 1.00 | 10.89 | | C |
| ANISOU | 1005 | CD2 | LEU | 1273 | 1779 | 858 | 1501 | -156 | 60 | 116 | C |
| ATOM | 1006 | C | LEU | 1273 | 19.254 | 16.973 | 33.300 | 1.00 | 7.53 | | C |
| ANISOU | 1006 | C | LEU | 1273 | 856 | 835 | 1171 | -73 | 21 | 29 | C |
| ATOM | 1007 | O | LEU | 1273 | 20.444 | 17.215 | 33.484 | 1.00 | 8.12 | | O |
| ANISOU | 1007 | O | LEU | 1273 | 880 | 933 | 1273 | -73 | 3 | -56 | O |
| ATOM | 1008 | N | TRP | 1274 | 18.795 | 15.693 | 33.166 | 1.00 | 7.61 | | N |
| ANISOU | 1008 | N | TRP | 1274 | 834 | 853 | 1205 | -9 | -20 | 141 | N |
| ATOM | 1009 | CA | TRP | 1274 | 19.748 | 14.576 | 33.136 | 1.00 | 7.75 | | C |
| ANISOU | 1009 | CA | TRP | 1274 | 892 | 805 | 1249 | -1 | 91 | 121 | C |
| ATOM | 1010 | CB | TRP | 1274 | 18.947 | 13.271 | 33.155 | 1.00 | 7.89 | | C |
| ANISOU | 1010 | CB | TRP | 1274 | 856 | 823 | 1319 | -14 | -80 | 144 | C |
| ATOM | 1011 | CG | TRP | 1274 | 19.754 | 12.014 | 33.261 | 1.00 | 7.84 | | C |
| ANISOU | 1011 | CG | TRP | 1274 | 930 | 775 | 1276 | -61 | -107 | 62 | C |
| ATOM | 1012 | CD2 | TRP | 1274 | 20.410 | 11.289 | 32.183 | 1.00 | 8.33 | | C |
| ANISOU | 1012 | CD2 | TRP | 1274 | 1022 | 812 | 1332 | -54 | -96 | -17 | C |
| ATOM | 1013 | CE2 | TRP | 1274 | 21.033 | 10.174 | 32.795 | 1.00 | 8.78 | | C |
| ANISOU | 1013 | CE2 | TRP | 1274 | 847 | 1031 | 1457 | 79 | -55 | 4 | C |
| ATOM | 1014 | CE3 | TRP | 1274 | 20.507 | 11.480 | 30.792 | 1.00 | 8.97 | | C |
| ANISOU | 1014 | CE3 | TRP | 1274 | 873 | 1170 | 1366 | -71 | 17 | 63 | C |
| ATOM | 1015 | CD1 | TRP | 1274 | 20.012 | 11.329 | 34.417 | 1.00 | 7.88 | | C |
| ANISOU | 1015 | CD1 | TRP | 1274 | 794 | 870 | 1331 | 60 | -126 | 146 | C |
| ATOM | 1016 | NE1 | TRP | 1274 | 20.781 | 10.225 | 34.141 | 1.00 | 8.38 | | N |
| ANISOU | 1016 | NE1 | TRP | 1274 | 908 | 909 | 1366 | 86 | -183 | -22 | N |
| ATOM | 1017 | CZ2 | TRP | 1274 | 21.755 | 9.256 | 32.050 | 1.00 | 9.41 | | C |
| ANISOU | 1017 | CZ2 | TRP | 1274 | 877 | 1066 | 1632 | 107 | -65 | -164 | C |
| ATOM | 1018 | CZ3 | TRP | 1274 | 21.240 | 10.538 | 30.056 | 1.00 | 9.83 | | C |
| ANISOU | 1018 | CZ3 | TRP | 1274 | 1083 | 1183 | 1468 | -96 | -115 | -208 | C |
| ATOM | 1019 | CH2 | TRP | 1274 | 21.859 | 9.434 | 30.676 | 1.00 | 10.08 | | C |
| ANISOU | 1019 | CH2 | TRP | 1274 | 1230 | 1022 | 1579 | -52 | -20 | -210 | C |
| ATOM | 1020 | C | TRP | 1274 | 20.678 | 14.690 | 31.930 | 1.00 | 7.63 | | C |
| ANISOU | 1020 | C | TRP | 1274 | 783 | 947 | 1169 | -92 | -56 | 35 | C |
| ATOM | 1021 | O | TRP | 1274 | 21.883 | 14.448 | 32.008 | 1.00 | 7.94 | | O |
| ANISOU | 1021 | O | TRP | 1274 | 789 | 776 | 1450 | 25 | -17 | 34 | O |
| ATOM | 1022 | N | GLU | 1275 | 20.118 | 15.079 | 30.765 | 1.00 | 7.60 | | N |
| ANISOU | 1022 | N | GLU | 1275 | 951 | 844 | 1093 | 25 | 17 | -42 | N |
| ATOM | 1023 | CA | GLU | 1275 | 20.958 | 15.335 | 29.613 | 1.00 | 7.25 | | C |
| ANISOU | 1023 | CA | GLU | 1275 | 745 | 906 | 1104 | -62 | -37 | 31 | C |
| ATOM | 1024 | CB | GLU | 1275 | 20.124 | 15.857 | 28.453 | 1.00 | 7.91 | | C |
| ANISOU | 1024 | CB | GLU | 1275 | 874 | 962 | 1171 | -28 | -115 | 35 | C |
| ATOM | 1025 | CG | GLU | 1275 | 19.102 | 14.852 | 27.910 | 1.00 | 8.53 | | C |
| ANISOU | 1025 | CG | GLU | 1275 | 1005 | 965 | 1271 | -83 | -196 | -55 | C |
| ATOM | 1026 | CD | GLU | 1275 | 18.490 | 15.454 | 26.631 | 1.00 | 7.60 | | C |
| ANISOU | 1026 | CD | GLU | 1275 | 806 | 868 | 1214 | -84 | -69 | -109 | C |
| ATOM | 1027 | OE1 | GLU | 1275 | 17.415 | 16.099 | 26.727 | 1.00 | 8.22 | | O1- |
| ANISOU | 1027 | OE1 | GLU | 1275 | 930 | 833 | 1360 | -38 | -1 | -28 | O1- |
| ATOM | 1028 | OE2 | GLU | 1275 | 19.120 | 15.278 | 25.564 | 1.00 | 8.89 | | O |
| ANISOU | 1028 | OE2 | GLU | 1275 | 1031 | 1115 | 1232 | 197 | 20 | 34 | O |
| ATOM | 1029 | C | GLU | 1275 | 22.006 | 16.386 | 29.913 | 1.00 | 7.37 | | C |
| ANISOU | 1029 | C | GLU | 1275 | 796 | 820 | 1184 | 17 | -9 | -38 | C |
| ATOM | 1030 | O | GLU | 1275 | 23.188 | 16.254 | 29.564 | 1.00 | 8.10 | | O |
| ANISOU | 1030 | O | GLU | 1275 | 723 | 1057 | 1297 | 8 | 79 | 107 | O |
| ATOM | 1031 | N | LEU | 1276 | 21.612 | 17.473 | 30.566 | 1.00 | 8.03 | | N |
| ANISOU | 1031 | N | LEU | 1276 | 1009 | 792 | 1251 | 24 | -33 | -19 | N |
| ATOM | 1032 | CA | LEU | 1276 | 22.585 | 18.522 | 30.882 | 1.00 | 7.69 | | C |
| ANISOU | 1032 | CA | LEU | 1276 | 923 | 794 | 1205 | 69 | -74 | -41 | C |
| ATOM | 1033 | CB | LEU | 1276 | 21.907 | 19.717 | 31.573 | 1.00 | 8.36 | | C |
| ANISOU | 1033 | CB | LEU | 1276 | 1039 | 860 | 1277 | 130 | -96 | -146 | C |
| ATOM | 1034 | CG | LEU | 1276 | 21.092 | 20.576 | 30.619 | 1.00 | 8.55 | | C |
| ANISOU | 1034 | CG | LEU | 1276 | 1039 | 742 | 1468 | 71 | 69 | 78 | C |
| ATOM | 1035 | CD1 | LEU | 1276 | 20.186 | 21.509 | 31.374 | 1.00 | 9.54 | | C |
| ANISOU | 1035 | CD1 | LEU | 1276 | 934 | 1007 | 1684 | 92 | 71 | -223 | C |
| ATOM | 1036 | CD2 | LEU | 1276 | 22.051 | 21.376 | 29.727 | 1.00 | 12.65 | | C |
| ANISOU | 1036 | CD2 | LEU | 1276 | 1597 | 1480 | 1728 | 167 | 503 | 392 | C |
| ATOM | 1037 | C | LEU | 1276 | 23.702 | 18.001 | 31.770 | 1.00 | 7.87 | | C |
| ANISOU | 1037 | C | LEU | 1276 | 868 | 886 | 1238 | 116 | -2 | 12 | C |
| ATOM | 1038 | O | LEU | 1276 | 24.879 | 18.265 | 31.496 | 1.00 | 8.92 | | O |
| ANISOU | 1038 | O | LEU | 1276 | 906 | 1139 | 1344 | 29 | -3 | 36 | O |
| ATOM | 1039 | N | AMET | 1277 | 23.352 | 17.251 | 32.833 | 0.60 | 8.18 | | N |
| ANISOU | 1039 | N | AMET | 1277 | 937 | 959 | 1214 | 21 | -145 | 43 | N |
| ATOM | 1040 | N | BMET | 1277 | 23.364 | 17.253 | 32.826 | 0.40 | 7.97 | | N |
| ANISOU | 1040 | N | BMET | 1277 | 922 | 899 | 1207 | 70 | -106 | 24 | N |
| ATOM | 1041 | CA | AMET | 1277 | 24.340 | 16.831 | 33.820 | 0.60 | 8.67 | | C |
| ANISOU | 1041 | CA | AMET | 1277 | 971 | 1016 | 1308 | 131 | -179 | 22 | C |
| ATOM | 1042 | CA | BMET | 1277 | 24.401 | 16.878 | 33.786 | 0.40 | 8.78 | | C |
| ANISOU | 1042 | CA | BMET | 1277 | 903 | 1320 | 1114 | 200 | -34 | -21 | C |
| ATOM | 1043 | CB | AMET | 1277 | 23.677 | 16.267 | 35.069 | 0.60 | 10.87 | | C |
| ANISOU | 1043 | CB | AMET | 1277 | 1256 | 1542 | 1331 | 329 | -18 | 274 | C |
| ATOM | 1044 | CB | BMET | 1277 | 23.752 | 16.593 | 35.138 | 0.40 | 6.87 | | C |
| ANISOU | 1044 | CB | BMET | 1277 | 803 | 714 | 1094 | -191 | -127 | -161 | C |
| ATOM | 1045 | CG | AMET | 1277 | 23.025 | 17.256 | 36.035 | 0.60 | 11.92 | | C |
| ANISOU | 1045 | CG | AMET | 1277 | 1374 | 1557 | 1597 | 143 | 109 | 101 | C |
| ATOM | 1046 | CG | BMET | 1277 | 22.837 | 17.708 | 35.625 | 0.40 | 4.28 | | C |
| ANISOU | 1046 | CG | BMET | 1277 | 468 | 534 | 626 | -226 | -324 | 170 | C |
| ATOM | 1047 | SD | AMET | 1277 | 24.207 | 18.553 | 36.585 | 0.60 | 17.60 | | S |
| ANISOU | 1047 | SD | AMET | 1277 | 2384 | 1895 | 2409 | -246 | -4 | -173 | S |
| ATOM | 1048 | SD | BMET | 1277 | 23.523 | 19.354 | 35.545 | 0.40 | 5.59 | | S |
| ANISOU | 1048 | SD | BMET | 1277 | 678 | 529 | 918 | -69 | -134 | 200 | S |
| ATOM | 1049 | CE | AMET | 1277 | 23.669 | 19.698 | 35.309 | 0.60 | 11.76 | | C |
| ANISOU | 1049 | CE | AMET | 1277 | 1219 | 1332 | 1917 | -224 | 477 | -533 | C |
| ATOM | 1050 | CE | BMET | 1277 | 25.057 | 19.280 | 36.518 | 0.40 | 8.21 | | C |
| ANISOU | 1050 | CE | BMET | 1277 | 623 | 863 | 1633 | -786 | -326 | 453 | C |
| ATOM | 1051 | C | AMET | 1277 | 25.314 | 15.782 | 33.227 | 0.60 | 8.81 | | C |
| ANISOU | 1051 | C | AMET | 1277 | 864 | 1002 | 1481 | 50 | -98 | 48 | C |
| ATOM | 1052 | C | BMET | 1277 | 25.236 | 15.673 | 33.318 | 0.40 | 7.75 | | C |
| ANISOU | 1052 | C | BMET | 1277 | 642 | 1088 | 1216 | 31 | 28 | 178 | C |
| ATOM | 1053 | O | AMET | 1277 | 26.424 | 15.653 | 33.733 | 0.60 | 8.20 | | O |
| ANISOU | 1053 | O | AMET | 1277 | 802 | 1116 | 1196 | -25 | -17 | 139 | O |
| ATOM | 1054 | O | BMET | 1277 | 26.183 | 15.324 | 34.020 | 0.40 | 11.13 | | O |
| ANISOU | 1054 | O | BMET | 1277 | 887 | 1741 | 1599 | 284 | -278 | 120 | O |
| ATOM | 1055 | N | THR | 1278 | 24.900 | 15.071 | 32.182 | 1.00 | 8.18 | | N |
| ANISOU | 1055 | N | THR | 1278 | 853 | 937 | 1317 | 38 | 8 | 71 | N |
| ATOM | 1056 | CA | THR | 1278 | 25.754 | 14.106 | 31.513 | 1.00 | 8.29 | | C |
| ANISOU | 1056 | CA | THR | 1278 | 713 | 1099 | 1340 | 48 | 29 | 89 | C |
| ATOM | 1057 | CB | THR | 1278 | 24.975 | 12.866 | 31.074 | 1.00 | 8.60 | | C |
| ANISOU | 1057 | CB | THR | 1278 | 976 | 934 | 1359 | -22 | -122 | 192 | C |
| ATOM | 1058 | OG1 | THR | 1278 | 23.935 | 13.261 | 30.113 | 1.00 | 8.76 | | O |
| ANISOU | 1058 | OG1 | THR | 1278 | 1064 | 1066 | 1199 | 5 | -180 | 162 | O |
| ATOM | 1059 | CG2 | THR | 1278 | 24.296 | 12.171 | 32.253 | 1.00 | 10.52 | | C |
| ANISOU | 1059 | CG2 | THR | 1278 | 1376 | 1231 | 1390 | 33 | 26 | 440 | C |
| ATOM | 1060 | C | THR | 1278 | 26.402 | 14.708 | 30.263 | 1.00 | 8.50 | | C |
| ANISOU | 1060 | C | THR | 1278 | 916 | 1088 | 1227 | -9 | -1 | 47 | C |
| ATOM | 1061 | O | THR | 1278 | 27.090 | 13.989 | 29.540 | 1.00 | 9.69 | | O |
| ANISOU | 1061 | O | THR | 1278 | 988 | 1280 | 1413 | 87 | 126 | -44 | O |
| ATOM | 1062 | N | ARG | 1279 | 26.206 | 15.980 | 29.966 | 1.00 | 8.52 | | N |
| ANISOU | 1062 | N | ARG | 1279 | 737 | 1088 | 1414 | -77 | -72 | 173 | N |
| ATOM | 1063 | CA | ARG | 1279 | 26.688 | 16.652 | 28.759 | 1.00 | 8.82 | | C |
| ANISOU | 1063 | CA | ARG | 1279 | 780 | 1266 | 1305 | -121 | -45 | 152 | C |
| ATOM | 1064 | CB | ARG | 1279 | 28.226 | 16.834 | 28.758 | 1.00 | 9.24 | | C |
| ANISOU | 1064 | CB | ARG | 1279 | 763 | 1114 | 1632 | -23 | 35 | 83 | C |
| ATOM | 1065 | CG | ARG | 1279 | 28.597 | 17.935 | 29.774 | 1.00 | 9.79 | | C |
| ANISOU | 1065 | CG | ARG | 1279 | 786 | 1608 | 1326 | -87 | -18 | -87 | C |
| ATOM | 1066 | CD | ARG | 1279 | 30.079 | 18.355 | 29.742 | 1.00 | 10.02 | | C |
| ANISOU | 1066 | CD | ARG | 1279 | 772 | 1494 | 1542 | -61 | -62 | 249 | C |
| ATOM | 1067 | NE | ARG | 1279 | 30.393 | 18.852 | 28.406 | 1.00 | 8.98 | | N |
| ANISOU | 1067 | NE | ARG | 1279 | 922 | 1019 | 1470 | 25 | 142 | -97 | N |
| ATOM | 1068 | CZ | ARG | 1279 | 31.618 | 19.153 | 27.986 | 1.00 | 9.26 | | C |
| ANISOU | 1068 | CZ | ARG | 1279 | 983 | 1136 | 1399 | -52 | 14 | -59 | C |
| ATOM | 1069 | NH1 | ARG | 1279 | 32.680 | 19.032 | 28.805 | 1.00 | 10.05 | | N1+ |
| ANISOU | 1069 | NH1 | ARG | 1279 | 1004 | 1147 | 1668 | 19 | -156 | -89 | N1+ |
| ATOM | 1070 | NH2 | ARG | 1279 | 31.757 | 19.589 | 26.727 | 1.00 | 9.97 | | N |
| ANISOU | 1070 | NH2 | ARG | 1279 | 1207 | 1150 | 1431 | -150 | 83 | -6 | N |
| ATOM | 1071 | C | ARG | 1279 | 26.234 | 15.916 | 27.506 | 1.00 | 9.12 | | C |
| ANISOU | 1071 | C | ARG | 1279 | 934 | 1113 | 1418 | -112 | 83 | 58 | C |
| ATOM | 1072 | O | ARG | 1279 | 26.975 | 15.714 | 26.525 | 1.00 | 9.81 | | O |
| ANISOU | 1072 | O | ARG | 1279 | 943 | 1422 | 1363 | -131 | 63 | 18 | O |
| ATOM | 1073 | N | GLY | 1280 | 24.936 | 15.532 | 27.520 | 1.00 | 8.86 | | N |
| ANISOU | 1073 | N | GLY | 1280 | 930 | 1081 | 1356 | -106 | -84 | 40 | N |
| ATOM | 1074 | CA | GLY | 1280 | 24.319 | 15.059 | 26.277 | 1.00 | 9.18 | | C |
| ANISOU | 1074 | CA | GLY | 1280 | 1073 | 1062 | 1351 | 11 | -23 | -99 | C |
| ATOM | 1075 | C | GLY | 1280 | 24.429 | 13.576 | 26.031 | 1.00 | 9.09 | | C |
| ANISOU | 1075 | C | GLY | 1280 | 981 | 1065 | 1409 | 29 | -101 | -60 | C |
| ATOM | 1076 | O | GLY | 1280 | 24.359 | 13.127 | 24.891 | 1.00 | 10.18 | | O |
| ANISOU | 1076 | O | GLY | 1280 | 1074 | 1331 | 1463 | -45 | -31 | -205 | O |
| ATOM | 1077 | N | ALA | 1281 | 24.586 | 12.781 | 27.104 | 1.00 | 9.80 | | N |
| ANISOU | 1077 | N | ALA | 1281 | 981 | 1116 | 1627 | 82 | -140 | 88 | N |
| ATOM | 1078 | CA | ALA | 1281 | 24.481 | 11.344 | 26.926 | 1.00 | 9.73 | | C |
| ANISOU | 1078 | CA | ALA | 1281 | 1043 | 1060 | 1596 | 104 | -133 | 65 | C |
| ATOM | 1079 | CB | ALA | 1281 | 24.842 | 10.630 | 28.213 | 1.00 | 10.50 | | C |
| ANISOU | 1079 | CB | ALA | 1281 | 1370 | 1153 | 1466 | 398 | -149 | -43 | C |
| ATOM | 1080 | C | ALA | 1281 | 23.068 | 10.975 | 26.512 | 1.00 | 9.44 | | C |
| ANISOU | 1080 | C | ALA | 1281 | 968 | 1074 | 1544 | 171 | -1 | -36 | C |
| ATOM | 1081 | O | ALA | 1281 | 22.089 | 11.608 | 26.956 | 1.00 | 9.61 | | O |
| ANISOU | 1081 | O | ALA | 1281 | 1061 | 1187 | 1403 | 217 | 173 | 170 | O |
| ATOM | 1082 | N | PRO | 1282 | 22.884 | 9.973 | 25.678 | 1.00 | 9.87 | | N |
| ANISOU | 1082 | N | PRO | 1282 | 1095 | 1136 | 1519 | 138 | -22 | -35 | N |
| ATOM | 1083 | CD | PRO | 1282 | 23.927 | 9.187 | 24.997 | 1.00 | 11.71 | | C |
| ANISOU | 1083 | CD | PRO | 1282 | 1407 | 1346 | 1696 | 124 | 184 | -328 | C |
| ATOM | 1084 | CA | PRO | 1282 | 21.525 | 9.532 | 25.369 | 1.00 | 10.23 | | C |
| ANISOU | 1084 | CA | PRO | 1282 | 1258 | 1232 | 1395 | -11 | -104 | -29 | C |
| ATOM | 1085 | CB | PRO | 1282 | 21.723 | 8.547 | 24.209 | 1.00 | 12.85 | | C |
| ANISOU | 1085 | CB | PRO | 1282 | 1537 | 1524 | 1820 | -39 | 121 | -407 | C |
| ATOM | 1086 | CG | PRO | 1282 | 23.135 | 8.044 | 24.391 | 1.00 | 13.75 | | C |
| ANISOU | 1086 | CG | PRO | 1282 | 1582 | 1478 | 2167 | 19 | 56 | -502 | C |
| ATOM | 1087 | C | PRO | 1282 | 20.913 | 8.822 | 26.559 | 1.00 | 10.02 | | C |
| ANISOU | 1087 | C | PRO | 1282 | 986 | 1306 | 1515 | 67 | -221 | 120 | C |
| ATOM | 1088 | O | PRO | 1282 | 21.531 | 7.932 | 27.134 | 1.00 | 10.89 | | O |
| ANISOU | 1088 | O | PRO | 1282 | 1289 | 1315 | 1532 | 168 | -142 | 136 | O |
| ATOM | 1089 | N | PRO | 1283 | 19.679 | 9.166 | 26.932 | 1.00 | 9.85 | | N |
| ANISOU | 1089 | N | PRO | 1283 | 1192 | 1088 | 1463 | 79 | -83 | 4 | N |
| ATOM | 1090 | CD | PRO | 1283 | 18.790 | 10.166 | 26.298 | 1.00 | 11.10 | | C |
| ANISOU | 1090 | CD | PRO | 1283 | 960 | 1434 | 1824 | 215 | 29 | 184 | C |
| ATOM | 1091 | CA | PRO | 1283 | 19.102 | 8.514 | 28.097 | 1.00 | 9.94 | | C |
| ANISOU | 1091 | CA | PRO | 1283 | 1162 | 991 | 1623 | -25 | 20 | -115 | C |
| ATOM | 1092 | CB | PRO | 1283 | 17.848 | 9.384 | 28.396 | 1.00 | 10.86 | | C |
| ANISOU | 1092 | CB | PRO | 1283 | 1321 | 1019 | 1787 | 243 | 105 | 94 | C |
| ATOM | 1093 | CG | PRO | 1283 | 17.477 | 9.956 | 27.036 | 1.00 | 12.81 | | C |
| ANISOU | 1093 | CG | PRO | 1283 | 1193 | 1779 | 1894 | 165 | 181 | 365 | C |
| ATOM | 1094 | C | PRO | 1283 | 18.716 | 7.078 | 27.797 | 1.00 | 9.02 | | C |
| ANISOU | 1094 | C | PRO | 1283 | 903 | 1025 | 1499 | 83 | -50 | -109 | C |
| ATOM | 1095 | O | PRO | 1283 | 18.275 | 6.736 | 26.685 | 1.00 | 11.14 | | O |
| ANISOU | 1095 | O | PRO | 1283 | 1608 | 1111 | 1513 | 88 | -180 | -179 | O |
| ATOM | 1096 | N | TYR | 1284 | 18.861 | 6.224 | 28.820 | 1.00 | 9.82 | | N |
| ANISOU | 1096 | N | TYR | 1284 | 1282 | 921 | 1529 | 204 | -7 | -28 | N |
| ATOM | 1097 | CA | TYR | 1284 | 18.488 | 4.795 | 28.699 | 1.00 | 10.89 | | C |
| ANISOU | 1097 | CA | TYR | 1284 | 1184 | 1061 | 1893 | -187 | 227 | -175 | C |
| ATOM | 1098 | CB | TYR | 1284 | 16.993 | 4.630 | 28.709 | 1.00 | 10.86 | | C |
| ANISOU | 1098 | CB | TYR | 1284 | 1189 | 1141 | 1797 | 152 | 147 | 96 | C |
| ATOM | 1099 | CG | TYR | 1284 | 16.100 | 5.425 | 29.588 | 1.00 | 9.12 | | C |
| ANISOU | 1099 | CG | TYR | 1284 | 1052 | 873 | 1542 | -6 | -68 | 33 | C |
| ATOM | 1100 | CD1 | TYR | 1284 | 15.832 | 5.089 | 30.914 | 1.00 | 9.13 | | C |
| ANISOU | 1100 | CD1 | TYR | 1284 | 1094 | 818 | 1558 | -83 | -101 | -68 | C |
| ATOM | 1101 | CE1 | TYR | 1284 | 14.976 | 5.872 | 31.686 | 1.00 | 10.08 | | C |
| ANISOU | 1101 | CE1 | TYR | 1284 | 1264 | 999 | 1567 | 77 | -125 | -52 | C |
| ATOM | 1102 | CD2 | TYR | 1284 | 15.467 | 6.557 | 29.072 | 1.00 | 9.50 | | C |
| ANISOU | 1102 | CD2 | TYR | 1284 | 1061 | 981 | 1566 | 21 | -3 | 2 | C |
| ATOM | 1103 | CE2 | TYR | 1284 | 14.618 | 7.336 | 29.828 | 1.00 | 9.08 | | C |
| ANISOU | 1103 | CE2 | TYR | 1284 | 961 | 956 | 1531 | -78 | 97 | -7 | C |
| ATOM | 1104 | CZ | TYR | 1284 | 14.377 | 6.990 | 31.153 | 1.00 | 9.18 | | C |
| ANISOU | 1104 | CZ | TYR | 1284 | 1091 | 1075 | 1324 | 138 | -184 | -40 | C |
| ATOM | 1105 | OH | TYR | 1284 | 13.518 | 7.745 | 31.917 | 1.00 | 9.70 | | O |
| ANISOU | 1105 | OH | TYR | 1284 | 1099 | 1109 | 1477 | -38 | 27 | -130 | O |
| ATOM | 1106 | C | TYR | 1284 | 19.051 | 4.138 | 27.434 | 1.00 | 10.15 | | C |
| ANISOU | 1106 | C | TYR | 1284 | 1240 | 900 | 1718 | 35 | 160 | -20 | C |
| ATOM | 1107 | O | TYR | 1284 | 18.315 | 3.573 | 26.608 | 1.00 | 12.75 | | O |
| ANISOU | 1107 | O | TYR | 1284 | 1671 | 1127 | 2046 | 137 | -62 | -307 | O |
| ATOM | 1108 | N | PRO | 1285 | 20.373 | 4.177 | 27.278 | 1.00 | 12.27 | | N |
| ANISOU | 1108 | N | PRO | 1285 | 1300 | 1273 | 2088 | 157 | 229 | -39 | N |
| ATOM | 1109 | CD | PRO | 1285 | 21.376 | 4.664 | 28.228 | 1.00 | 12.83 | | C |
| ANISOU | 1109 | CD | PRO | 1285 | 1070 | 1399 | 2405 | 224 | 103 | -35 | C |
| ATOM | 1110 | CA | PRO | 1285 | 20.969 | 3.676 | 26.030 | 1.00 | 14.09 | | C |
| ANISOU | 1110 | CA | PRO | 1285 | 1375 | 1503 | 2474 | 157 | 459 | -299 | C |
| ATOM | 1111 | CB | PRO | 1285 | 22.470 | 3.981 | 26.220 | 1.00 | 15.60 | | C |
| ANISOU | 1111 | CB | PRO | 1285 | 1455 | 1820 | 2651 | -128 | 468 | -269 | C |
| ATOM | 1112 | CG | PRO | 1285 | 22.655 | 3.937 | 27.715 | 1.00 | 15.06 | | C |
| ANISOU | 1112 | CG | PRO | 1285 | 1323 | 1715 | 2686 | 462 | 288 | -231 | C |
| ATOM | 1113 | C | PRO | 1285 | 20.786 | 2.181 | 25.828 | 1.00 | 14.02 | | C |
| ANISOU | 1113 | C | PRO | 1285 | 1454 | 1539 | 2333 | 94 | 157 | -356 | C |
| ATOM | 1114 | O | PRO | 1285 | 20.875 | 1.671 | 24.703 | 1.00 | 17.93 | | O |
| ANISOU | 1114 | O | PRO | 1285 | 2295 | 2012 | 2504 | 0 | 523 | -620 | O |
| ATOM | 1115 | N | ASP | 1286 | 20.525 | 1.455 | 26.918 | 1.00 | 14.10 | | N |
| ANISOU | 1115 | N | ASP | 1286 | 1546 | 1380 | 2431 | 136 | -46 | -283 | N |
| ATOM | 1116 | CA | ASP | 1286 | 20.315 | 0.000 | 26.835 | 1.00 | 17.44 | | C |
| ANISOU | 1116 | CA | ASP | 1286 | 1836 | 1272 | 3517 | 213 | -259 | -202 | C |
| ATOM | 1117 | CB | ASP | 1286 | 20.965 | -0.669 | 28.044 | 1.00 | 24.27 | | C |
| ANISOU | 1117 | CB | ASP | 1286 | 2717 | 1955 | 4551 | 514 | -913 | 435 | C |
| ATOM | 1118 | CG | ASP | 1286 | 22.479 | -0.590 | 27.904 | 1.00 | 28.57 | | C |
| ANISOU | 1118 | CG | ASP | 1286 | 2638 | 3913 | 4303 | 332 | -1271 | -100 | C |
| ATOM | 1119 | OD1 | ASP | 1286 | 22.982 | -0.181 | 26.823 | 1.00 | 37.07 | | O |
| ANISOU | 1119 | OD1 | ASP | 1286 | 3849 | 3660 | 6576 | 1228 | 736 | 1149 | O |
| ATOM | 1120 | OD2 | ASP | 1286 | 23.144 | -0.941 | 28.900 | 1.00 | 47.33 | | O1- |
| ANISOU | 1120 | OD2 | ASP | 1286 | 4041 | 7871 | 6072 | 2284 | -2396 | 554 | O1- |
| ATOM | 1121 | C | ASP | 1286 | 18.844 | -0.406 | 26.749 | 1.00 | 17.94 | | C |
| ANISOU | 1121 | C | ASP | 1286 | 1867 | 1132 | 3817 | 64 | -86 | 228 | C |
| ATOM | 1122 | O | ASP | 1286 | 18.505 | -1.600 | 26.782 | 1.00 | 27.24 | | O |
| ANISOU | 1122 | O | ASP | 1286 | 2488 | 1203 | 6658 | -117 | 159 | -183 | O |
| ATOM | 1123 | N | VAL | 1287 | 17.940 | 0.546 | 26.638 | 1.00 | 14.90 | | N |
| ANISOU | 1123 | N | VAL | 1287 | 1697 | 1322 | 2641 | 17 | -351 | -189 | N |
| ATOM | 1124 | CA | VAL | 1287 | 16.528 | 0.284 | 26.472 | 1.00 | 15.30 | | C |
| ANISOU | 1124 | CA | VAL | 1287 | 1783 | 1575 | 2453 | -38 | -355 | -157 | C |
| ATOM | 1125 | CB | VAL | 1287 | 15.722 | 1.222 | 27.381 | 1.00 | 13.84 | | C |
| ANISOU | 1125 | CB | VAL | 1287 | 1627 | 1365 | 2267 | -165 | -282 | -11 | C |
| ATOM | 1126 | CG1 | VAL | 1287 | 14.234 | 1.030 | 27.137 | 1.00 | 17.31 | | C |
| ANISOU | 1126 | CG1 | VAL | 1287 | 1608 | 1773 | 3195 | -125 | -426 | 34 | C |
| ATOM | 1127 | CG2 | VAL | 1287 | 16.148 | 0.975 | 28.841 | 1.00 | 17.18 | | C |
| ANISOU | 1127 | CG2 | VAL | 1287 | 2438 | 1808 | 2281 | 53 | -446 | 147 | C |
| ATOM | 1128 | C | VAL | 1287 | 16.119 | 0.464 | 25.009 | 1.00 | 14.23 | | C |
| ANISOU | 1128 | C | VAL | 1287 | 1773 | 1246 | 2389 | 7 | -248 | -77 | C |
| ATOM | 1129 | O | VAL | 1287 | 16.454 | 1.536 | 24.446 | 1.00 | 17.83 | | O |
| ANISOU | 1129 | O | VAL | 1287 | 2402 | 1381 | 2990 | -115 | -199 | 234 | O |
| ATOM | 1130 | N | ASN | 1288 | 15.440 | -0.497 | 24.408 | 1.00 | 14.23 | | N |
| ANISOU | 1130 | N | ASN | 1288 | 1832 | 1235 | 2340 | 264 | -317 | -285 | N |
| ATOM | 1131 | CA | ASN | 1288 | 14.956 | -0.448 | 23.041 | 1.00 | 15.93 | | C |
| ANISOU | 1131 | CA | ASN | 1288 | 2209 | 1533 | 2311 | 329 | -352 | -228 | C |
| ATOM | 1132 | CB | ASN | 1288 | 14.823 | -1.882 | 22.491 | 1.00 | 18.95 | | C |
| ANISOU | 1132 | CB | ASN | 1288 | 2950 | 1732 | 2517 | 481 | -515 | -656 | C |
| ATOM | 1133 | CG | ASN | 1288 | 14.220 | -1.892 | 21.083 | 1.00 | 24.51 | | C |
| ANISOU | 1133 | CG | ASN | 1288 | 4244 | 2704 | 2366 | -713 | -568 | -357 | C |
| ATOM | 1134 | OD1 | ASN | 1288 | 14.576 | -1.077 | 20.195 | 1.00 | 34.15 | | O |
| ANISOU | 1134 | OD1 | ASN | 1288 | 7111 | 3404 | 2459 | -754 | 287 | -67 | O |
| ATOM | 1135 | ND2 | ASN | 1288 | 13.285 | -2.819 | 20.819 | 1.00 | 29.42 | | N |
| ANISOU | 1135 | ND2 | ASN | 1288 | 3722 | 3729 | 3727 | -717 | -1030 | -963 | N |
| ATOM | 1136 | C | ASN | 1288 | 13.630 | 0.274 | 22.978 | 1.00 | 14.34 | | C |
| ANISOU | 1136 | C | ASN | 1288 | 2093 | 1406 | 1950 | 217 | -191 | 213 | C |
| ATOM | 1137 | O | ASN | 1288 | 12.612 | -0.327 | 23.370 | 1.00 | 15.29 | | O |
| ANISOU | 1137 | O | ASN | 1288 | 2304 | 1335 | 2171 | 44 | -35 | -175 | O |
| ATOM | 1138 | N | THR | 1289 | 13.646 | 1.523 | 22.507 | 1.00 | 14.85 | | N |
| ANISOU | 1138 | N | THR | 1289 | 2399 | 1452 | 1792 | 206 | -81 | 191 | N |
| ATOM | 1139 | CA | THR | 1289 | 12.440 | 2.334 | 22.237 | 1.00 | 14.27 | | C |
| ANISOU | 1139 | CA | THR | 1289 | 2329 | 1177 | 1914 | 45 | -280 | 133 | C |
| ATOM | 1140 | CB | THR | 1289 | 11.947 | 2.131 | 20.793 | 1.00 | 17.15 | | C |
| ANISOU | 1140 | CB | THR | 1289 | 2537 | 1917 | 2063 | -132 | -506 | -36 | C |
| ATOM | 1141 | OG1 | THR | 1289 | 11.497 | 0.786 | 20.551 | 1.00 | 18.53 | | O |
| ANISOU | 1141 | OG1 | THR | 1289 | 3124 | 1705 | 2212 | 120 | -798 | -179 | O |
| ATOM | 1142 | CG2 | THR | 1289 | 13.112 | 2.394 | 19.826 | 1.00 | 19.29 | | C |
| ANISOU | 1142 | CG2 | THR | 1289 | 3187 | 2077 | 2066 | 473 | 3 | 165 | C |
| ATOM | 1143 | C | THR | 1289 | 11.267 | 2.080 | 23.210 | 1.00 | 13.76 | | C |
| ANISOU | 1143 | C | THR | 1289 | 2146 | 932 | 2148 | -82 | -340 | 109 | C |
| ATOM | 1144 | O | THR | 1289 | 11.455 | 2.451 | 24.381 | 1.00 | 13.13 | | O |
| ANISOU | 1144 | O | THR | 1289 | 1869 | 1017 | 2101 | 71 | -250 | -99 | O |
| ATOM | 1145 | N | PHE | 1290 | 10.146 | 1.495 | 22.804 | 1.00 | 14.90 | | N |
| ANISOU | 1145 | N | PHE | 1290 | 2056 | 1300 | 2308 | 26 | -674 | 170 | N |
| ATOM | 1146 | CA | PHE | 1290 | 8.945 | 1.402 | 23.664 | 1.00 | 13.95 | | C |
| ANISOU | 1146 | CA | PHE | 1290 | 1935 | 1139 | 2226 | -109 | -848 | 107 | C |
| ATOM | 1147 | CB | PHE | 1290 | 7.676 | 1.021 | 22.878 | 1.00 | 17.44 | | C |
| ANISOU | 1147 | CB | PHE | 1290 | 2222 | 1820 | 2584 | -500 | -1181 | 609 | C |
| ATOM | 1148 | CG | PHE | 1290 | 7.163 | 2.129 | 21.962 | 1.00 | 16.44 | | C |
| ANISOU | 1148 | CG | PHE | 1290 | 2105 | 1652 | 2491 | -346 | -1115 | 402 | C |
| ATOM | 1149 | CD1 | PHE | 1290 | 6.317 | 3.082 | 22.506 | 1.00 | 18.65 | | C |
| ANISOU | 1149 | CD1 | PHE | 1290 | 2029 | 1914 | 3145 | -328 | -937 | 344 | C |
| ATOM | 1150 | CD2 | PHE | 1290 | 7.519 | 2.203 | 20.617 | 1.00 | 19.19 | | C |
| ANISOU | 1150 | CD2 | PHE | 1290 | 2380 | 2460 | 2451 | -348 | -1120 | 579 | C |
| ATOM | 1151 | CE1 | PHE | 1290 | 5.801 | 4.125 | 21.728 | 1.00 | 18.42 | | C |
| ANISOU | 1151 | CE1 | PHE | 1290 | 2344 | 1887 | 2769 | 2 | -558 | 244 | C |
| ATOM | 1152 | CE2 | PHE | 1290 | 7.011 | 3.237 | 19.825 | 1.00 | 17.26 | | C |
| ANISOU | 1152 | CE2 | PHE | 1290 | 2400 | 1991 | 2166 | -256 | -948 | 167 | C |
| ATOM | 1153 | CZ | PHE | 1290 | 6.159 | 4.178 | 20.395 | 1.00 | 18.62 | | C |
| ANISOU | 1153 | CZ | PHE | 1290 | 2216 | 2050 | 2809 | -522 | -457 | 142 | C |
| ATOM | 1154 | C | PHE | 1290 | 9.128 | 0.415 | 24.806 | 1.00 | 13.58 | | C |
| ANISOU | 1154 | C | PHE | 1290 | 1834 | 1240 | 2085 | 12 | -740 | 22 | C |
| ATOM | 1155 | O | PHE | 1290 | 8.238 | 0.349 | 25.685 | 1.00 | 14.33 | | O |
| ANISOU | 1155 | O | PHE | 1290 | 1775 | 1086 | 2582 | -56 | -514 | 195 | O |
| ATOM | 1156 | N | ASP | 1291 | 10.257 | -0.322 | 24.864 | 1.00 | 12.85 | | N |
| ANISOU | 1156 | N | ASP | 1291 | 2087 | 818 | 1977 | 99 | -472 | 17 | N |
| ATOM | 1157 | CA | ASP | 1291 | 10.524 | -1.062 | 26.105 | 1.00 | 12.06 | | C |
| ANISOU | 1157 | CA | ASP | 1291 | 1764 | 854 | 1964 | 77 | -263 | 112 | C |
| ATOM | 1158 | CB | ASP | 1291 | 11.803 | -1.897 | 25.962 | 1.00 | 12.20 | | C |
| ANISOU | 1158 | CB | ASP | 1291 | 1820 | 879 | 1936 | 123 | -193 | 128 | C |
| ATOM | 1159 | CG | ASP | 1291 | 11.744 | -2.973 | 24.911 | 1.00 | 12.79 | | C |
| ANISOU | 1159 | CG | ASP | 1291 | 1856 | 1006 | 1998 | 84 | -138 | 50 | C |
| ATOM | 1160 | OD1 | ASP | 1291 | 10.728 | -3.106 | 24.228 | 1.00 | 15.16 | | O |
| ANISOU | 1160 | OD1 | ASP | 1291 | 1897 | 1210 | 2652 | -231 | -217 | -255 | O |
| ATOM | 1161 | OD2 | ASP | 1291 | 12.797 | -3.669 | 24.780 | 1.00 | 13.94 | | O1- |
| ANISOU | 1161 | OD2 | ASP | 1291 | 1902 | 961 | 2434 | 34 | 17 | -42 | O1- |
| ATOM | 1162 | C | ASP | 1291 | 10.656 | -0.156 | 27.312 | 1.00 | 10.93 | | C |
| ANISOU | 1162 | C | ASP | 1291 | 1373 | 912 | 1869 | 22 | -133 | 158 | C |
| ATOM | 1163 | O | ASP | 1291 | 10.598 | -0.630 | 28.465 | 1.00 | 12.35 | | O |
| ANISOU | 1163 | O | ASP | 1291 | 1708 | 1070 | 1915 | 22 | -175 | 206 | O |
| ATOM | 1164 | N | ILE | 1292 | 10.837 | 1.146 | 27.102 | 1.00 | 11.08 | | N |
| ANISOU | 1164 | N | ILE | 1292 | 1382 | 957 | 1870 | -33 | -234 | 44 | N |
| ATOM | 1165 | CA | ILE | 1292 | 10.905 | 2.109 | 28.203 | 1.00 | 11.68 | | C |
| ANISOU | 1165 | CA | ILE | 1292 | 1515 | 1051 | 1871 | -52 | 69 | -68 | C |
| ATOM | 1166 | CB | ILE | 1292 | 11.158 | 3.541 | 27.684 | 1.00 | 11.37 | | C |
| ANISOU | 1166 | CB | ILE | 1292 | 1459 | 1078 | 1782 | -183 | -145 | -5 | C |
| ATOM | 1167 | CG2 | ILE | 1292 | 10.001 | 4.079 | 26.875 | 1.00 | 14.15 | | C |
| ANISOU | 1167 | CG2 | ILE | 1292 | 1677 | 1164 | 2535 | -115 | -615 | -126 | C |
| ATOM | 1168 | CG1 | ILE | 1292 | 11.488 | 4.548 | 28.796 | 1.00 | 13.35 | | C |
| ANISOU | 1168 | CG1 | ILE | 1292 | 2080 | 1085 | 1907 | -49 | -391 | -77 | C |
| ATOM | 1169 | CD1 | ILE | 1292 | 12.724 | 4.220 | 29.630 | 1.00 | 17.87 | | C |
| ANISOU | 1169 | CD1 | ILE | 1292 | 2081 | 2579 | 2129 | -499 | -638 | 73 | C |
| ATOM | 1170 | C | ILE | 1292 | 9.629 | 2.034 | 29.033 | 1.00 | 11.11 | | C |
| ANISOU | 1170 | C | ILE | 1292 | 1484 | 1207 | 1530 | -115 | -192 | 179 | C |
| ATOM | 1171 | O | ILE | 1292 | 9.671 | 2.278 | 30.246 | 1.00 | 12.66 | | O |
| ANISOU | 1171 | O | ILE | 1292 | 1927 | 1316 | 1565 | -357 | -74 | 20 | O |
| ATOM | 1172 | N | THR | 1293 | 8.446 | 1.736 | 28.473 | 1.00 | 11.48 | | N |
| ANISOU | 1172 | N | THR | 1293 | 1499 | 900 | 1963 | -122 | -116 | -134 | N |
| ATOM | 1173 | CA | THR | 1293 | 7.242 | 1.805 | 29.278 | 1.00 | 12.74 | | C |
| ANISOU | 1173 | CA | THR | 1293 | 1481 | 1209 | 2149 | -154 | -40 | -37 | C |
| ATOM | 1174 | CB | THR | 1293 | 6.019 | 1.546 | 28.364 | 1.00 | 12.59 | | C |
| ANISOU | 1174 | CB | THR | 1293 | 1505 | 1187 | 2093 | 89 | -97 | -141 | C |
| ATOM | 1175 | OG1 | THR | 1293 | 6.126 | 2.416 | 27.231 | 1.00 | 13.51 | | O |
| ANISOU | 1175 | OG1 | THR | 1293 | 1771 | 1186 | 2177 | -26 | -233 | -107 | O |
| ATOM | 1176 | CG2 | THR | 1293 | 4.707 | 1.832 | 29.101 | 1.00 | 15.53 | | C |
| ANISOU | 1176 | CG2 | THR | 1293 | 1496 | 1807 | 2599 | -5 | 42 | -271 | C |
| ATOM | 1177 | C | THR | 1293 | 7.299 | 0.808 | 30.394 | 1.00 | 12.25 | | C |
| ANISOU | 1177 | C | THR | 1293 | 1652 | 1158 | 1844 | -148 | -93 | -232 | C |
| ATOM | 1178 | O | THR | 1293 | 7.003 | 1.152 | 31.554 | 1.00 | 13.63 | | O |
| ANISOU | 1178 | O | THR | 1293 | 1806 | 1340 | 2035 | -423 | 155 | -426 | O |
| ATOM | 1179 | N | AVAL | 1294 | 7.678 | -0.449 | 30.076 | 0.50 | 12.24 | | N |
| ANISOU | 1179 | N | AVAL | 1294 | 1764 | 1122 | 1764 | -296 | 189 | -130 | N |
| ATOM | 1180 | N | BVAL | 1294 | 7.680 | -0.449 | 30.101 | 0.50 | 12.62 | | N |
| ANISOU | 1180 | N | BVAL | 1294 | 1714 | 1207 | 1872 | -82 | 81 | -153 | N |
| ATOM | 1181 | CA | AVAL | 1294 | 7.750 | -1.477 | 31.112 | 0.50 | 13.37 | | C |
| ANISOU | 1181 | CA | AVAL | 1294 | 1870 | 1251 | 1960 | -35 | -61 | -30 | C |
| ATOM | 1182 | CA | BVAL | 1294 | 7.637 | -1.380 | 31.248 | 0.50 | 12.58 | | C |
| ANISOU | 1182 | CA | BVAL | 1294 | 1914 | 1005 | 1862 | -118 | 41 | -290 | C |
| ATOM | 1183 | CB | AVAL | 1294 | 8.106 | -2.885 | 30.603 | 0.50 | 16.96 | | C |
| ANISOU | 1183 | CB | AVAL | 1294 | 2971 | 1129 | 2344 | -292 | -108 | -172 | C |
| ATOM | 1184 | CB | BVAL | 1294 | 7.613 | -2.819 | 30.743 | 0.50 | 14.22 | | C |
| ANISOU | 1184 | CB | BVAL | 1294 | 2223 | 1102 | 2078 | 29 | 541 | -427 | C |
| ATOM | 1185 | CG1AVAL | | 1294 | 6.972 | -3.509 | 29.788 | 0.50 | 19.44 | | C |
| ANISOU | 1185 | CG1AVAL | | 1294 | 3434 | 1959 | 1996 | -893 | 5 | -255 | C |
| ATOM | 1186 | CG1BVAL | | 1294 | 8.995 | -3.263 | 30.287 | 0.50 | 14.49 | | C |
| ANISOU | 1186 | CG1BVAL | | 1294 | 1831 | 935 | 2739 | -462 | 578 | -567 | C |
| ATOM | 1187 | CG2AVAL | | 1294 | 9.369 | -2.830 | 29.778 | 0.50 | 21.70 | | C |
| ANISOU | 1187 | CG2AVAL | | 1294 | 2509 | 2236 | 3501 | 757 | 3 | -716 | C |
| ATOM | 1188 | CG2BVAL | | 1294 | 7.120 | -3.769 | 31.825 | 0.50 | 11.88 | | C |
| ANISOU | 1188 | CG2BVAL | | 1294 | 1330 | 965 | 2219 | -149 | 88 | -344 | C |
| ATOM | 1189 | C | AVAL | 1294 | 8.787 | -1.067 | 32.169 | 0.50 | 12.41 | | C |
| ANISOU | 1189 | C | AVAL | 1294 | 1677 | 1145 | 1892 | -2 | 152 | -112 | C |
| ATOM | 1190 | C | BVAL | 1294 | 8.793 | -1.067 | 32.195 | 0.50 | 12.52 | | C |
| ANISOU | 1190 | C | BVAL | 1294 | 1606 | 1233 | 1918 | 55 | 132 | -35 | C |
| ATOM | 1191 | O | AVAL | 1294 | 8.537 | -1.255 | 33.351 | 0.50 | 13.31 | | O |
| ANISOU | 1191 | O | AVAL | 1294 | 1949 | 1231 | 1878 | -251 | 67 | -71 | O |
| ATOM | 1192 | O | BVAL | 1294 | 8.659 | -1.322 | 33.389 | 0.50 | 13.03 | | O |
| ANISOU | 1192 | O | BVAL | 1294 | 1973 | 1099 | 1880 | -274 | 7 | -94 | O |
| ATOM | 1193 | N | TYR | 1295 | 9.912 | -0.521 | 31.732 | 1.00 | 12.59 | | N |
| ANISOU | 1193 | N | TYR | 1295 | 1548 | 1105 | 2130 | 121 | 181 | -45 | N |
| ATOM | 1194 | CA | TYR | 1295 | 10.946 | -0.077 | 32.663 | 1.00 | 12.93 | | C |
| ANISOU | 1194 | CA | TYR | 1295 | 1597 | 1055 | 2259 | 83 | 162 | 47 | C |
| ATOM | 1195 | CB | TYR | 1295 | 12.074 | 0.570 | 31.834 | 1.00 | 13.69 | | C |
| ANISOU | 1195 | CB | TYR | 1295 | 1529 | 1309 | 2362 | 65 | 231 | 84 | C |
| ATOM | 1196 | CG | TYR | 1295 | 13.306 | 1.008 | 32.560 | 1.00 | 16.70 | | C |
| ANISOU | 1196 | CG | TYR | 1295 | 1600 | 2363 | 2383 | -55 | 15 | 374 | C |
| ATOM | 1197 | CD1 | TYR | 1295 | 13.521 | 2.318 | 32.986 | 1.00 | 18.98 | | C |
| ANISOU | 1197 | CD1 | TYR | 1295 | 2027 | 2631 | 2553 | -866 | -278 | 405 | C |
| ATOM | 1198 | CE1 | TYR | 1295 | 14.688 | 2.689 | 33.664 | 1.00 | 23.05 | | C |
| ANISOU | 1198 | CE1 | TYR | 1295 | 1897 | 3650 | 3210 | -1215 | -327 | 731 | C |
| ATOM | 1199 | CD2 | TYR | 1295 | 14.286 | 0.033 | 32.812 | 1.00 | 22.87 | | C |
| ANISOU | 1199 | CD2 | TYR | 1295 | 1425 | 3782 | 3481 | 654 | 366 | 474 | C |
| ATOM | 1200 | CE2 | TYR | 1295 | 15.450 | 0.368 | 33.483 | 1.00 | 25.34 | | C |
| ANISOU | 1200 | CE2 | TYR | 1295 | 1451 | 4818 | 3358 | 456 | 298 | 974 | C |
| ATOM | 1201 | CZ | TYR | 1295 | 15.616 | 1.682 | 33.889 | 1.00 | 27.73 | | C |
| ANISOU | 1201 | CZ | TYR | 1295 | 2055 | 4873 | 3606 | -619 | -295 | 1321 | C |
| ATOM | 1202 | OH | TYR | 1295 | 16.791 | 1.946 | 34.540 | 1.00 | 41.40 | | O |
| ANISOU | 1202 | OH | TYR | 1295 | 2025 | 6425 | 7278 | -424 | -1235 | 534 | O |
| ATOM | 1203 | C | TYR | 1295 | 10.406 | 0.909 | 33.684 | 1.00 | 12.05 | | C |
| ANISOU | 1203 | C | TYR | 1295 | 1404 | 1075 | 2100 | 12 | 26 | 68 | C |
| ATOM | 1204 | O | TYR | 1295 | 10.609 | 0.806 | 34.901 | 1.00 | 12.19 | | O |
| ANISOU | 1204 | O | TYR | 1295 | 1386 | 1033 | 2212 | -7 | -57 | 54 | O |
| ATOM | 1205 | N | LEU | 1296 | 9.692 | 1.921 | 33.180 | 1.00 | 11.10 | | N |
| ANISOU | 1205 | N | LEU | 1296 | 1358 | 983 | 1875 | -27 | -111 | -163 | N |
| ATOM | 1206 | CA | LEU | 1296 | 9.104 | 2.947 | 34.075 | 1.00 | 11.39 | | C |
| ANISOU | 1206 | CA | LEU | 1296 | 1657 | 825 | 1844 | -104 | 37 | -31 | C |
| ATOM | 1207 | CB | LEU | 1296 | 8.602 | 4.108 | 33.226 | 1.00 | 11.71 | | C |
| ANISOU | 1207 | CB | LEU | 1296 | 1477 | 862 | 2111 | -88 | -250 | -25 | C |
| ATOM | 1208 | CG | LEU | 1296 | 9.695 | 4.840 | 32.445 | 1.00 | 11.33 | | C |
| ANISOU | 1208 | CG | LEU | 1296 | 1564 | 1000 | 1742 | -142 | -394 | 93 | C |
| ATOM | 1209 | CD1 | LEU | 1296 | 9.018 | 5.826 | 31.512 | 1.00 | 13.82 | | C |
| ANISOU | 1209 | CD1 | LEU | 1296 | 1853 | 1261 | 2138 | -23 | -580 | 337 | C |
| ATOM | 1210 | CD2 | LEU | 1296 | 10.697 | 5.535 | 33.370 | 1.00 | 12.80 | | C |
| ANISOU | 1210 | CD2 | LEU | 1296 | 1577 | 1224 | 2061 | -320 | -529 | 189 | C |
| ATOM | 1211 | C | LEU | 1296 | 7.997 | 2.380 | 34.948 | 1.00 | 12.19 | | C |
| ANISOU | 1211 | C | LEU | 1296 | 1413 | 1177 | 2041 | -120 | 58 | -98 | C |
| ATOM | 1212 | O | LEU | 1296 | 7.880 | 2.764 | 36.131 | 1.00 | 12.57 | | O |
| ANISOU | 1212 | O | LEU | 1296 | 1609 | 1139 | 2027 | -108 | 119 | -38 | O |
| ATOM | 1213 | N | LEU | 1297 | 7.175 | 1.468 | 34.425 | 1.00 | 12.98 | | N |
| ANISOU | 1213 | N | LEU | 1297 | 1359 | 1241 | 2332 | -163 | 2 | -110 | N |
| ATOM | 1214 | CA | LEU | 1297 | 6.085 | 0.876 | 35.190 | 1.00 | 13.37 | | C |
| ANISOU | 1214 | CA | LEU | 1297 | 1535 | 1174 | 2371 | -129 | 228 | -250 | C |
| ATOM | 1215 | CB | LEU | 1297 | 5.253 | -0.064 | 34.325 | 1.00 | 15.81 | | C |
| ANISOU | 1215 | CB | LEU | 1297 | 1672 | 1580 | 2754 | -408 | -129 | -224 | C |
| ATOM | 1216 | CG | LEU | 1297 | 4.137 | 0.522 | 33.496 | 1.00 | 24.97 | | C |
| ANISOU | 1216 | CG | LEU | 1297 | 2366 | 3080 | 4042 | 709 | -933 | -1004 | C |
| ATOM | 1217 | CD1 | LEU | 1297 | 3.535 | -0.491 | 32.499 | 1.00 | 29.70 | | C |
| ANISOU | 1217 | CD1 | LEU | 1297 | 3214 | 4972 | 3100 | -1962 | -776 | -124 | C |
| ATOM | 1218 | CD2 | LEU | 1297 | 3.036 | 1.088 | 34.412 | 1.00 | 25.27 | | C |
| ANISOU | 1218 | CD2 | LEU | 1297 | 1734 | 2293 | 5575 | -73 | 496 | 451 | C |
| ATOM | 1219 | C | LEU | 1297 | 6.630 | 0.090 | 36.353 | 1.00 | 13.35 | | C |
| ANISOU | 1219 | C | LEU | 1297 | 1487 | 1307 | 2277 | -284 | 204 | -238 | C |
| ATOM | 1220 | O | LEU | 1297 | 6.005 | -0.032 | 37.398 | 1.00 | 16.83 | | O |
| ANISOU | 1220 | O | LEU | 1297 | 1743 | 2265 | 2388 | -268 | 382 | -49 | O |
| ATOM | 1221 | N | GLN | 1298 | 7.826 | -0.475 | 36.126 | 1.00 | 13.14 | | N |
| ANISOU | 1221 | N | GLN | 1298 | 1547 | 1100 | 2344 | -189 | 102 | -165 | N |
| ATOM | 1222 | CA | GLN | 1298 | 8.514 | -1.213 | 37.187 | 1.00 | 14.79 | | C |
| ANISOU | 1222 | CA | GLN | 1298 | 1888 | 1320 | 2412 | -195 | -89 | -154 | C |
| ATOM | 1223 | CB | GLN | 1298 | 9.698 | -2.080 | 36.639 | 1.00 | 14.60 | | C |
| ANISOU | 1223 | CB | GLN | 1298 | 2129 | 1050 | 2370 | -10 | -235 | -115 | C |
| ATOM | 1224 | CG | GLN | 1298 | 9.137 | -3.284 | 35.839 | 1.00 | 16.14 | | C |
| ANISOU | 1224 | CG | GLN | 1298 | 2391 | 1439 | 2301 | -151 | -269 | -271 | C |
| ATOM | 1225 | CD | GLN | 1298 | 10.257 | -3.994 | 35.071 | 1.00 | 17.61 | | C |
| ANISOU | 1225 | CD | GLN | 1298 | 2519 | 1269 | 2902 | 111 | -280 | -403 | C |
| ATOM | 1226 | OE1 | GLN | 1298 | 11.399 | -3.520 | 35.017 | 1.00 | 20.58 | | O |
| ANISOU | 1226 | OE1 | GLN | 1298 | 2488 | 2013 | 3318 | -32 | -79 | -836 | O |
| ATOM | 1227 | NE2 | GLN | 1298 | 9.950 | -5.114 | 34.455 | 1.00 | 18.17 | | N |
| ANISOU | 1227 | NE2 | GLN | 1298 | 3259 | 1285 | 2362 | -145 | -214 | -165 | N |
| ATOM | 1228 | C | GLN | 1298 | 9.076 | -0.313 | 38.262 | 1.00 | 14.49 | | C |
| ANISOU | 1228 | C | GLN | 1298 | 1676 | 1420 | 2411 | -122 | -13 | -220 | C |
| ATOM | 1229 | O | GLN | 1298 | 9.613 | -0.807 | 39.270 | 1.00 | 16.52 | | O |
| ANISOU | 1229 | O | GLN | 1298 | 2394 | 1525 | 2356 | -188 | -161 | -191 | O |
| ATOM | 1230 | N | GLY | 1299 | 8.992 | 1.000 | 38.086 | 1.00 | 14.57 | | N |
| ANISOU | 1230 | N | GLY | 1299 | 1834 | 1359 | 2343 | -320 | -40 | -286 | N |
| ATOM | 1231 | CA | GLY | 1299 | 9.503 | 1.938 | 39.077 | 1.00 | 13.13 | | C |
| ANISOU | 1231 | CA | GLY | 1299 | 1660 | 1597 | 1731 | -240 | -83 | -151 | C |
| ATOM | 1232 | C | GLY | 1299 | 10.994 | 2.146 | 38.923 | 1.00 | 14.42 | | C |
| ANISOU | 1232 | C | GLY | 1299 | 1687 | 1478 | 2314 | -329 | 2 | -72 | C |
| ATOM | 1233 | O | GLY | 1299 | 11.634 | 2.495 | 39.943 | 1.00 | 19.79 | | O |
| ANISOU | 1233 | O | GLY | 1299 | 1909 | 2665 | 2945 | -587 | -200 | -1032 | O |
| ATOM | 1234 | N | ARG | 1300 | 11.538 | 1.956 | 37.722 | 1.00 | 12.49 | | N |
| ANISOU | 1234 | N | ARG | 1300 | 1443 | 985 | 2319 | -54 | -46 | 324 | N |
| ATOM | 1235 | CA | ARG | 1300 | 12.958 | 2.245 | 37.479 | 1.00 | 12.31 | | C |
| ANISOU | 1235 | CA | ARG | 1300 | 1442 | 878 | 2359 | -15 | -23 | 92 | C |
| ATOM | 1236 | CB | ARG | 1300 | 13.503 | 1.138 | 36.580 | 1.00 | 12.64 | | C |
| ANISOU | 1236 | CB | ARG | 1300 | 1733 | 1022 | 2048 | 15 | -88 | 54 | C |
| ATOM | 1237 | CG | ARG | 1300 | 13.324 | -0.231 | 37.232 | 1.00 | 15.33 | | C |
| ANISOU | 1237 | CG | ARG | 1300 | 2503 | 940 | 2380 | 198 | -38 | 65 | C |
| ATOM | 1238 | CD | ARG | 1300 | 13.770 | -1.361 | 36.288 | 1.00 | 15.58 | | C |
| ANISOU | 1238 | CD | ARG | 1300 | 2458 | 1169 | 2291 | 598 | -241 | 23 | C |
| ATOM | 1239 | NE | ARG | 1300 | 13.432 | -2.651 | 36.872 | 1.00 | 16.28 | | N |
| ANISOU | 1239 | NE | ARG | 1300 | 2495 | 1142 | 2550 | 428 | -430 | 0 | N |
| ATOM | 1240 | CZ | ARG | 1300 | 14.156 | -3.285 | 37.808 | 1.00 | 16.09 | | C |
| ANISOU | 1240 | CZ | ARG | 1300 | 2226 | 1481 | 2408 | 240 | -346 | 280 | C |
| ATOM | 1241 | NH1 | ARG | 1300 | 13.742 | -4.476 | 38.281 | 1.00 | 17.75 | | N1+ |
| ANISOU | 1241 | NH1 | ARG | 1300 | 3094 | 1528 | 2124 | -174 | -597 | 180 | N1+ |
| ATOM | 1242 | NH2 | ARG | 1300 | 15.293 | -2.742 | 38.272 | 1.00 | 15.38 | | N |
| ANISOU | 1242 | NH2 | ARG | 1300 | 2371 | 1576 | 1895 | 161 | -119 | -188 | N |
| ATOM | 1243 | C | ARG | 1300 | 13.084 | 3.641 | 36.895 | 1.00 | 11.27 | | C |
| ANISOU | 1243 | C | ARG | 1300 | 1213 | 918 | 2151 | -22 | -68 | 34 | C |
| ATOM | 1244 | O | ARG | 1300 | 12.148 | 4.125 | 36.239 | 1.00 | 10.63 | | O |
| ANISOU | 1244 | O | ARG | 1300 | 1401 | 909 | 1728 | 42 | -68 | -67 | O |
| ATOM | 1245 | N | ARG | 1301 | 14.211 | 4.284 | 37.132 | 1.00 | 10.94 | | N |
| ANISOU | 1245 | N | ARG | 1301 | 1320 | 939 | 1897 | -68 | -35 | 189 | N |
| ATOM | 1246 | CA | ARG | 1301 | 14.462 | 5.649 | 36.726 | 1.00 | 9.18 | | C |
| ANISOU | 1246 | CA | ARG | 1301 | 1214 | 732 | 1540 | 42 | 175 | 21 | C |
| ATOM | 1247 | CB | ARG | 1301 | 14.179 | 6.652 | 37.855 | 1.00 | 9.72 | | C |
| ANISOU | 1247 | CB | ARG | 1301 | 993 | 1255 | 1444 | 49 | -67 | -172 | C |
| ATOM | 1248 | CG | ARG | 1301 | 12.717 | 6.628 | 38.327 | 1.00 | 9.44 | | C |
| ANISOU | 1248 | CG | ARG | 1301 | 997 | 989 | 1600 | 63 | 27 | -9 | C |
| ATOM | 1249 | CD | ARG | 1301 | 11.782 | 7.195 | 37.280 | 1.00 | 9.52 | | C |
| ANISOU | 1249 | CD | ARG | 1301 | 969 | 894 | 1753 | 48 | 37 | 138 | C |
| ATOM | 1250 | NE | ARG | 1301 | 10.391 | 7.404 | 37.708 | 1.00 | 9.24 | | N |
| ANISOU | 1250 | NE | ARG | 1301 | 988 | 852 | 1672 | 15 | 9 | 241 | N |
| ATOM | 1251 | CZ | ARG | 1301 | 9.441 | 6.518 | 37.577 | 1.00 | 9.53 | | C |
| ANISOU | 1251 | CZ | ARG | 1301 | 1063 | 837 | 1719 | 1 | 28 | 168 | C |
| ATOM | 1252 | NHI | ARG | 1301 | 9.649 | 5.292 | 37.037 | 1.00 | 10.23 | | N1+ |
| ANISOU | 1252 | NH1 | ARG | 1301 | 1245 | 952 | 1691 | 26 | -19 | -112 | N1+ |
| ATOM | 1253 | NH2 | ARG | 1301 | 8.206 | 6.819 | 37.995 | 1.00 | 10.44 | | N |
| ANISOU | 1253 | NH2 | ARG | 1301 | 1036 | 1088 | 1841 | 69 | 165 | 160 | N |
| ATOM | 1254 | C | ARG | 1301 | 15.914 | 5.784 | 36.277 | 1.00 | 9.87 | | C |
| ANISOU | 1254 | C | ARG | 1301 | 1103 | 945 | 1701 | 58 | 59 | 48 | C |
| ATOM | 1255 | O | ARG | 1301 | 16.764 | 4.954 | 36.627 | 1.00 | 11.76 | | O |
| ANISOU | 1255 | O | ARG | 1301 | 1172 | 1075 | 2222 | 157 | 52 | 343 | O |
| ATOM | 1256 | N | LEU | 1302 | 16.186 | 6.859 | 35.510 | 1.00 | 8.85 | | N |
| ANISOU | 1256 | N | LEU | 1302 | 984 | 1047 | 1334 | -25 | 1 | -10 | N |
| ATOM | 1257 | CA | LEU | 1302 | 17.563 | 7.203 | 35.210 | 1.00 | 8.89 | | C |
| ANISOU | 1257 | CA | LEU | 1302 | 1047 | 1055 | 1276 | -133 | -53 | 0 | C |
| ATOM | 1258 | CB | LEU | 1302 | 17.604 | 8.539 | 34.471 | 1.00 | 9.51 | | C |
| ANISOU | 1258 | CB | LEU | 1302 | 1271 | 1018 | 1323 | -57 | -176 | 26 | C |
| ATOM | 1259 | CG | LEU | 1302 | 17.043 | 8.469 | 33.039 | 1.00 | 8.86 | | C |
| ANISOU | 1259 | CG | LEU | 1302 | 1045 | 1075 | 1247 | -14 | -111 | -56 | C |
| ATOM | 1260 | CD1 | LEU | 1302 | 16.732 | 9.859 | 32.525 | 1.00 | 10.17 | | C |
| ANISOU | 1260 | CD1 | LEU | 1302 | 1507 | 1066 | 1290 | -129 | -225 | 38 | C |
| ATOM | 1261 | CD2 | LEU | 1302 | 18.014 | 7.747 | 32.101 | 1.00 | 10.32 | | C |
| ANISOU | 1261 | CD2 | LEU | 1302 | 1048 | 1389 | 1485 | -147 | 107 | -223 | C |
| ATOM | 1262 | C | LEU | 1302 | 18.413 | 7.312 | 36.483 | 1.00 | 8.91 | | C |
| ANISOU | 1262 | C | LEU | 1302 | 1036 | 977 | 1372 | -40 | -92 | 15 | C |
| ATOM | 1263 | O | LEU | 1302 | 17.951 | 7.839 | 37.487 | 1.00 | 9.40 | | O |
| ANISOU | 1263 | O | LEU | 1302 | 1170 | 1049 | 1354 | -76 | -94 | -93 | O |
| ATOM | 1264 | N | LEU | 1303 | 19.651 | 6.822 | 36.400 | 1.00 | 9.19 | | N |
| ANISOU | 1264 | N | LEU | 1303 | 1092 | 989 | 1411 | 14 | -177 | 22 | N |
| ATOM | 1265 | CA | LEU | 1303 | 20.583 | 6.900 | 37.532 | 1.00 | 9.47 | | C |
| ANISOU | 1265 | CA | LEU | 1303 | 1027 | 1098 | 1472 | 57 | -150 | -77 | C |
| ATOM | 1266 | CB | LEU | 1303 | 21.753 | 5.919 | 37.324 | 1.00 | 11.46 | | C |
| ANISOU | 1266 | CB | LEU | 1303 | 1169 | 1250 | 1935 | 256 | -215 | -145 | C |
| ATOM | 1267 | CG | LEU | 1303 | 21.346 | 4.444 | 37.188 | 1.00 | 14.04 | | C |
| ANISOU | 1267 | CG | LEU | 1303 | 1942 | 1180 | 2213 | 310 | 40 | -75 | C |
| ATOM | 1268 | CD1 | LEU | 1303 | 22.611 | 3.594 | 37.006 | 1.00 | 19.13 | | C |
| ANISOU | 1268 | CD1 | LEU | 1303 | 2562 | 1580 | 3127 | 980 | -95 | 112 | C |
| ATOM | 1269 | CD2 | LEU | 1303 | 20.548 | 4.001 | 38.373 | 1.00 | 20.51 | | C |
| ANISOU | 1269 | CD2 | LEU | 1303 | 3400 | 1600 | 2791 | 27 | 721 | 352 | C |
| ATOM | 1270 | C | LEU | 1303 | 21.131 | 8.306 | 37.698 | 1.00 | 8.89 | | C |
| ANISOU | 1270 | C | LEU | 1303 | 1028 | 1057 | 1293 | -28 | -114 | 79 | C |
| ATOM | 1271 | O | LEU | 1303 | 21.135 | 9.116 | 36.725 | 1.00 | 10.51 | | O |
| ANISOU | 1271 | O | LEU | 1303 | 1467 | 1237 | 1288 | -52 | -280 | 134 | O |
| ATOM | 1272 | N | GLN | 1304 | 21.615 | 8.631 | 38.865 | 1.00 | 8.82 | | N |
| ANISOU | 1272 | N | GLN | 1304 | 1046 | 996 | 1310 | -35 | -217 | 218 | N |
| ATOM | 1273 | CA | GLN | 1304 | 22.186 | 9.937 | 39.120 | 1.00 | 9.13 | | C |
| ANISOU | 1273 | CA | GLN | 1304 | 1059 | 1157 | 1253 | -223 | -2 | 62 | C |
| ATOM | 1274 | CB | GLN | 1304 | 22.413 | 10.071 | 40.635 | 1.00 | 9.74 | | C |
| ANISOU | 1274 | CB | GLN | 1304 | 1245 | 1113 | 1344 | -25 | -138 | 54 | C |
| ATOM | 1275 | CG | GLN | 1304 | 22.960 | 11.467 | 41.000 | 1.00 | 9.72 | | C |
| ANISOU | 1275 | CG | GLN | 1304 | 1160 | 1162 | 1373 | -96 | -289 | 129 | C |
| ATOM | 1276 | CD | GLN | 1304 | 22.981 | 11.664 | 42.514 | 1.00 | 8.88 | | C |
| ANISOU | 1276 | CD | GLN | 1304 | 1080 | 915 | 1381 | 62 | -121 | 151 | C |
| ATOM | 1277 | OE1 | GLN | 1304 | 22.538 | 10.822 | 43.310 | 1.00 | 9.87 | | O |
| ANISOU | 1277 | OE1 | GLN | 1304 | 1267 | 1021 | 1463 | -123 | -72 | 240 | O |
| ATOM | 1278 | NE2 | GLN | 1304 | 23.508 | 12.802 | 42.961 | 1.00 | 9.86 | | N |
| ANISOU | 1278 | NE2 | GLN | 1304 | 1131 | 941 | 1676 | -17 | -194 | 22 | N |
| ATOM | 1279 | C | GLN | 1304 | 23.490 | 10.134 | 38.385 | 1.00 | 9.41 | | C |
| ANISOU | 1279 | C | GLN | 1304 | 932 | 1147 | 1497 | 68 | 1 | 259 | C |
| ATOM | 1280 | O | GLN | 1304 | 24.461 | 9.396 | 38.605 | 1.00 | 11.06 | | O |
| ANISOU | 1280 | O | GLN | 1304 | 1282 | 1192 | 1726 | 172 | 8 | 190 | O |
| ATOM | 1281 | N | PRO | 1305 | 23.633 | 11.119 | 37.492 | 1.00 | 8.95 | | N |
| ANISOU | 1281 | N | PRO | 1305 | 1043 | 1129 | 1227 | -48 | -37 | 124 | N |
| ATOM | 1282 | CD | PRO | 1305 | 22.577 | 12.016 | 36.996 | 1.00 | 10.56 | | C |
| ANISOU | 1282 | CD | PRO | 1305 | 1103 | 1589 | 1320 | -103 | -146 | 626 | C |
| ATOM | 1283 | CA | PRO | 1305 | 24.922 | 11.382 | 36.857 | 1.00 | 10.76 | | C |
| ANISOU | 1283 | CA | PRO | 1305 | 1104 | 1553 | 1430 | -216 | -10 | 328 | C |
| ATOM | 1284 | CB | PRO | 1305 | 24.621 | 12.600 | 35.938 | 1.00 | 11.11 | | C |
| ANISOU | 1284 | CB | PRO | 1305 | 1075 | 1601 | 1547 | -105 | -15 | 416 | C |
| ATOM | 1285 | CG | PRO | 1305 | 23.136 | 12.444 | 35.635 | 1.00 | 10.89 | | C |
| ANISOU | 1285 | CG | PRO | 1305 | 1163 | 1611 | 1363 | -328 | -16 | 436 | C |
| ATOM | 1286 | C | PRO | 1305 | 26.012 | 11.719 | 37.868 | 1.00 | 10.69 | | C |
| ANISOU | 1286 | C | PRO | 1305 | 1186 | 1423 | 1452 | -126 | -66 | 282 | C |
| ATOM | 1287 | O | PRO | 1305 | 25.751 | 12.325 | 38.890 | 1.00 | 10.80 | | O |
| ANISOU | 1287 | O | PRO | 1305 | 1079 | 1439 | 1588 | -109 | -118 | 217 | O |
| ATOM | 1288 | N | GLU | 1306 | 27.241 | 11.302 | 37.532 | 1.00 | 11.59 | | N |
| ANISOU | 1288 | N | GLU | 1306 | 1186 | 1524 | 1694 | 75 | -94 | 361 | N |
| ATOM | 1289 | CA | GLU | 1306 | 28.415 | 11.560 | 38.338 | 1.00 | 13.33 | | C |
| ANISOU | 1289 | CA | GLU | 1306 | 1388 | 1634 | 2044 | 266 | -313 | 206 | C |
| ATOM | 1290 | CB | GLU | 1306 | 29.689 | 11.208 | 37.520 | 1.00 | 18.09 | | C |
| ANISOU | 1290 | CB | GLU | 1306 | 1237 | 2384 | 3251 | 749 | -253 | -189 | C |
| ATOM | 1291 | CG | GLU | 1306 | 30.945 | 11.540 | 38.304 | 1.00 | 25.24 | | C |
| ANISOU | 1291 | CG | GLU | 1306 | 1565 | 4833 | 3192 | -120 | -154 | -240 | C |
| ATOM | 1292 | CD | GLU | 1306 | 32.189 | 11.064 | 37.585 | 1.00 | 29.39 | | C |
| ANISOU | 1292 | CD | GLU | 1306 | 1231 | 5919 | 4015 | 220 | -376 | -574 | C |
| ATOM | 1293 | OE1 | GLU | 1306 | 32.125 | 10.803 | 36.355 | 1.00 | 31.27 | | O1- |
| ANISOU | 1293 | OE1 | GLU | 1306 | 1932 | 5860 | 4090 | 883 | -94 | -983 | O1- |
| ATOM | 1294 | OE2 | GLU | 1306 | 33.236 | 10.966 | 38.267 | 1.00 | 38.16 | | O |
| ANISOU | 1294 | OE2 | GLU | 1306 | 2380 | 6901 | 5219 | 1465 | -1343 | -355 | O |
| ATOM | 1295 | C | GLU | 1306 | 28.502 | 12.993 | 38.805 | 1.00 | 12.43 | | C |
| ANISOU | 1295 | C | GLU | 1306 | 1223 | 1677 | 1823 | -1 | -87 | 248 | C |
| ATOM | 1296 | O | GLU | 1306 | 28.813 | 13.250 | 39.975 | 1.00 | 15.21 | | O |
| ANISOU | 1296 | O | GLU | 1306 | 1833 | 2028 | 1919 | -271 | -470 | 341 | O |
| ATOM | 1297 | N | TYR | 1307 | 28.252 | 13.955 | 37.918 | 1.00 | 12.32 | | N |
| ANISOU | 1297 | N | TYR | 1307 | 1159 | 1615 | 1908 | 17 | -94 | 340 | N |
| ATOM | 1298 | CA | TYR | 1307 | 28.417 | 15.375 | 38.308 | 1.00 | 13.42 | | C |
| ANISOU | 1298 | CA | TYR | 1307 | 1344 | 1605 | 2149 | -156 | 66 | 398 | C |
| ATOM | 1299 | CB | TYR | 1307 | 29.007 | 16.159 | 37.145 | 1.00 | 13.35 | | C |
| ANISOU | 1299 | CB | TYR | 1307 | 1403 | 1724 | 1945 | -183 | 132 | 239 | C |
| ATOM | 1300 | CG | TYR | 1307 | 30.476 | 15.944 | 36.930 | 1.00 | 15.04 | | C |
| ANISOU | 1300 | CG | TYR | 1307 | 1419 | 1756 | 2539 | -319 | 133 | 149 | C |
| ATOM | 1301 | CD1 | TYR | 1307 | 30.941 | 14.947 | 36.108 | 1.00 | 15.62 | | C |
| ANISOU | 1301 | CD1 | TYR | 1307 | 1359 | 2303 | 2274 | 227 | -254 | 3 | C |
| ATOM | 1302 | CE1 | TYR | 1307 | 32.292 | 14.789 | 35.937 | 1.00 | 18.86 | | C |
| ANISOU | 1302 | CEI | TYR | 1307 | 1432 | 2835 | 2901 | 156 | -151 | -36 | C |
| ATOM | 1303 | CD2 | TYR | 1307 | 31.389 | 16.788 | 37.577 | 1.00 | 15.74 | | C |
| ANISOU | 1303 | CD2 | TYR | 1307 | 1409 | 1850 | 2721 | -252 | -19 | 214 | C |
| ATOM | 1304 | CE2 | TYR | 1307 | 32.762 | 16.620 | 37.396 | 1.00 | 17.81 | | C |
| ANISOU | 1304 | CE2 | TYR | 1307 | 1384 | 2404 | 2980 | -41 | -281 | 306 | C |
| ATOM | 1305 | CZ | TYR | 1307 | 33.206 | 15.606 | 36.566 | 1.00 | 18.52 | | C |
| ANISOU | 1305 | CZ | TYR | 1307 | 1213 | 2209 | 3614 | 168 | -205 | 310 | C |
| ATOM | 1306 | OH | TYR | 1307 | 34.574 | 15.479 | 36.418 | 1.00 | 25.92 | | O |
| ANISOU | 1306 | OH | TYR | 1307 | 1175 | 3449 | 5224 | 296 | 39 | 142 | O |
| ATOM | 1307 | C | TYR | 1307 | 27.105 | 16.016 | 38.741 | 1.00 | 13.14 | | C |
| ANISOU | 1307 | C | TYR | 1307 | 1474 | 1486 | 2031 | -163 | 190 | 318 | C |
| ATOM | 1308 | O | TYR | 1307 | 27.031 | 17.255 | 38.955 | 1.00 | 15.30 | | O |
| ANISOU | 1308 | O | TYR | 1307 | 1592 | 1590 | 2632 | -330 | 273 | -25 | O |
| ATOM | 1309 | N | CYS | 1308 | 26.040 | 15.218 | 38.876 | 1.00 | 11.46 | | N |
| ANISOU | 1309 | N | CYS | 1308 | 1222 | 1446 | 1686 | -110 | -96 | 396 | N |
| ATOM | 1310 | CA | CYS | 1308 | 24.771 | 15.785 | 39.316 | 1.00 | 11.80 | | C |
| ANISOU | 1310 | CA | CYS | 1308 | 1281 | 1617 | 1586 | -197 | -162 | 173 | C |
| ATOM | 1311 | CB | CYS | 1308 | 23.612 | 14.867 | 38.858 | 1.00 | 11.30 | | C |
| ANISOU | 1311 | CB | CYS | 1308 | 1251 | 1202 | 1840 | -102 | -115 | -52 | C |
| ATOM | 1312 | SG | CYS | 1308 | 21.987 | 15.498 | 39.385 | 1.00 | 11.19 | | S |
| ANISOU | 1312 | SG | CYS | 1308 | 1269 | 1366 | 1616 | 9 | -170 | 177 | S |
| ATOM | 1313 | C | CYS | 1308 | 24.736 | 15.980 | 40.813 | 1.00 | 10.16 | | C |
| ANISOU | 1313 | C | CYS | 1308 | 1156 | 1110 | 1593 | -102 | -247 | 227 | C |
| ATOM | 1314 | O | CYS | 1308 | 24.838 | 14.939 | 41.502 | 1.00 | 11.07 | | O |
| ANISOU | 1314 | O | CYS | 1308 | 1268 | 1125 | 1812 | -115 | -215 | 290 | O |
| ATOM | 1315 | N | PRO | 1309 | 24.594 | 17.153 | 41.373 | 1.00 | 10.30 | | N |
| ANISOU | 1315 | N | PRO | 1309 | 1090 | 1097 | 1728 | -43 | -103 | 236 | N |
| ATOM | 1316 | CD | PRO | 1309 | 24.472 | 18.483 | 40.727 | 1.00 | 12.79 | | C |
| ANISOU | 1316 | CD | PRO | 1309 | 1717 | 1095 | 2049 | 124 | -295 | 247 | C |
| ATOM | 1317 | CA | PRO | 1309 | 24.520 | 17.230 | 42.852 | 1.00 | 11.57 | | C |
| ANISOU | 1317 | CA | PRO | 1309 | 1300 | 1341 | 1755 | -165 | 74 | 21 | C |
| ATOM | 1318 | CB | PRO | 1309 | 24.522 | 18.734 | 43.165 | 1.00 | 14.62 | | C |
| ANISOU | 1318 | CB | PRO | 1309 | 2182 | 1323 | 2050 | -505 | 271 | -29 | C |
| ATOM | 1319 | CG | PRO | 1309 | 24.151 | 19.357 | 41.899 | 1.00 | 17.02 | | C |
| ANISOU | 1319 | CG | PRO | 1309 | 2591 | 1281 | 2594 | 419 | -887 | -331 | C |
| ATOM | 1320 | C | PRO | 1309 | 23.238 | 16.578 | 43.368 | 1.00 | 10.22 | | C |
| ANISOU | 1320 | C | PRO | 1309 | 1187 | 1169 | 1528 | -118 | -218 | 120 | C |
| ATOM | 1321 | O | PRO | 1309 | 22.194 | 16.536 | 42.688 | 1.00 | 10.38 | | O |
| ANISOU | 1321 | O | PRO | 1309 | 1239 | 1109 | 1596 | -21 | -237 | 52 | O |
| ATOM | 1322 | N | ASP | 1310 | 23.259 | 16.039 | 44.591 | 1.00 | 10.38 | | N |
| ANISOU | 1322 | N | ASP | 1310 | 1297 | 1177 | 1469 | -37 | -156 | 23 | N |
| ATOM | 1323 | CA | ASP | 1310 | 22.125 | 15.312 | 45.114 | 1.00 | 10.23 | | C |
| ANISOU | 1323 | CA | ASP | 1310 | 1361 | 1259 | 1266 | -87 | -200 | 115 | C |
| ATOM | 1324 | CB | ASP | 1310 | 22.414 | 14.859 | 46.563 | 1.00 | 12.93 | | C |
| ANISOU | 1324 | CB | ASP | 1310 | 2098 | 1405 | 1410 | 80 | -361 | 338 | C |
| ATOM | 1325 | CG | ASP | 1310 | 23.570 | 13.919 | 46.709 | 1.00 | 13.66 | | C |
| ANISOU | 1325 | CG | ASP | 1310 | 2192 | 1415 | 1584 | 108 | -452 | 336 | C |
| ATOM | 1326 | OD1 | ASP | 1310 | 24.072 | 13.349 | 45.709 | 1.00 | 13.07 | | O |
| ANISOU | 1326 | OD1 | ASP | 1310 | 1758 | 1415 | 1793 | -134 | -308 | 252 | O |
| ATOM | 1327 | OD2 | ASP | 1310 | 24.003 | 13.733 | 47.901 | 1.00 | 17.38 | | O1- |
| ANISOU | 1327 | OD2 | ASP | 1310 | 2967 | 1849 | 1788 | 40 | -938 | 410 | O1- |
| ATOM | 1328 | C | ASP | 1310 | 20.835 | 16.134 | 45.081 | 1.00 | 9.76 | | C |
| ANISOU | 1328 | C | ASP | 1310 | 1289 | 1159 | 1258 | -177 | -302 | 15 | C |
| ATOM | 1329 | O | ASP | 1310 | 19.821 | 15.556 | 44.686 | 1.00 | 9.91 | | O |
| ANISOU | 1329 | O | ASP | 1310 | 1289 | 1006 | 1469 | -141 | -291 | 28 | O |
| ATOM | 1330 | N | PRO | 1311 | 20.790 | 17.421 | 45.486 | 1.00 | 10.30 | | N |
| ANISOU | 1330 | N | PRO | 1311 | 1361 | 1072 | 1479 | -214 | -317 | 99 | N |
| ATOM | 1331 | CD | PRO | 1311 | 21.873 | 18.217 | 46.106 | 1.00 | 11.37 | | C |
| ANISOU | 1331 | CD | PRO | 1311 | 1669 | 1107 | 1546 | -98 | -518 | -103 | C |
| ATOM | 1332 | CA | PRO | 1311 | 19.514 | 18.134 | 45.406 | 1.00 | 10.38 | | C |
| ANISOU | 1332 | CA | PRO | 1311 | 1501 | 1136 | 1307 | -66 | -172 | -58 | C |
| ATOM | 1333 | CB | PRO | 1311 | 19.838 | 19.527 | 45.957 | 1.00 | 11.67 | | C |
| ANISOU | 1333 | CB | PRO | 1311 | 1637 | 1150 | 1646 | -130 | -309 | -63 | C |
| ATOM | 1334 | CG | PRO | 1311 | 21.053 | 19.277 | 46.804 | 1.00 | 15.64 | | C |
| ANISOU | 1334 | CG | PRO | 1311 | 1744 | 1851 | 2348 | 205 | -659 | -751 | C |
| ATOM | 1335 | C | PRO | 1311 | 18.982 | 18.223 | 43.976 | 1.00 | 9.59 | | C |
| ANISOU | 1335 | C | PRO | 1311 | 1264 | 1067 | 1312 | -192 | -125 | 126 | C |
| ATOM | 1336 | O | PRO | 1311 | 17.741 | 18.267 | 43.809 | 1.00 | 9.87 | | O |
| ANISOU | 1336 | O | PRO | 1311 | 1316 | 1015 | 1419 | -88 | -116 | 199 | O |
| ATOM | 1337 | N | LEU | 1312 | 19.826 | 18.269 | 42.976 | 1.00 | 9.24 | | N |
| ANISOU | 1337 | N | LEU | 1312 | 1278 | 897 | 1337 | -224 | -119 | 90 | N |
| ATOM | 1338 | CA | LEU | 1312 | 19.285 | 18.322 | 41.598 | 1.00 | 9.33 | | C |
| ANISOU | 1338 | CA | LEU | 1312 | 1345 | 915 | 1287 | -224 | -23 | 89 | C |
| ATOM | 1339 | CB | LEU | 1312 | 20.354 | 18.862 | 40.652 | 1.00 | 9.98 | | C |
| ANISOU | 1339 | CB | LEU | 1312 | 1145 | 1234 | 1413 | 47 | 31 | 193 | C |
| ATOM | 1340 | CG | LEU | 1312 | 19.822 | 19.190 | 39.238 | 1.00 | 9.67 | | C |
| ANISOU | 1340 | CG | LEU | 1312 | 1220 | 1265 | 1188 | 246 | 114 | 9 | C |
| ATOM | 1341 | CD1 | LEU | 1312 | 18.846 | 20.363 | 39.265 | 1.00 | 11.14 | | C |
| ANISOU | 1341 | CD1 | LEU | 1312 | 1136 | 1424 | 1674 | 253 | -97 | 137 | C |
| ATOM | 1342 | CD2 | LEU | 1312 | 20.981 | 19.488 | 38.314 | 1.00 | 11.09 | | C |
| ANISOU | 1342 | CD2 | LEU | 1312 | 1288 | 1353 | 1572 | 6 | 227 | 115 | C |
| ATOM | 1343 | C | LEU | 1312 | 18.801 | 16.950 | 41.168 | 1.00 | 8.84 | | C |
| ANISOU | 1343 | C | LEU | 1312 | 1144 | 959 | 1256 | 22 | -107 | -8 | C |
| ATOM | 1344 | O | LEU | 1312 | 17.830 | 16.871 | 40.404 | 1.00 | 8.89 | | O |
| ANISOU | 1344 | O | LEU | 1312 | 1084 | 982 | 1310 | -92 | -144 | 95 | O |
| ATOM | 1345 | N | TYR | 1313 | 19.443 | 15.846 | 41.642 | 1.00 | 8.98 | | N |
| ANISOU | 1345 | N | TYR | 1313 | 1153 | 837 | 1422 | -120 | -191 | 84 | N |
| ATOM | 1346 | CA | TYR | 1313 | 18.859 | 14.550 | 41.343 | 1.00 | 8.53 | | C |
| ANISOU | 1346 | CA | TYR | 1313 | 981 | 862 | 1398 | -5 | -90 | 55 | C |
| ATOM | 1347 | CB | TYR | 1313 | 19.790 | 13.433 | 41.865 | 1.00 | 9.20 | | C |
| ANISOU | 1347 | CB | TYR | 1313 | 1040 | 922 | 1533 | 66 | -188 | 53 | C |
| ATOM | 1348 | CG | TYR | 1313 | 19.374 | 12.050 | 41.369 | 1.00 | 8.64 | | C |
| ANISOU | 1348 | CG | TYR | 1313 | 999 | 926 | 1358 | 128 | -116 | 87 | C |
| ATOM | 1349 | CD1 | TYR | 1313 | 19.154 | 11.775 | 40.030 | 1.00 | 8.50 | | C |
| ANISOU | 1349 | CD1 | TYR | 1313 | 926 | 1001 | 1301 | 101 | -133 | 82 | C |
| ATOM | 1350 | CE1 | TYR | 1313 | 18.780 | 10.515 | 39.592 | 1.00 | 8.61 | | C |
| ANISOU | 1350 | CE1 | TYR | 1313 | 991 | 978 | 1303 | 95 | -154 | 134 | C |
| ATOM | 1351 | CD2 | TYR | 1313 | 19.213 | 11.012 | 42.289 | 1.00 | 8.33 | | C |
| ANISOU | 1351 | CD2 | TYR | 1313 | 842 | 968 | 1356 | 17 | -151 | 135 | C |
| ATOM | 1352 | CE2 | TYR | 1313 | 18.843 | 9.726 | 41.872 | 1.00 | 8.36 | | C |
| ANISOU | 1352 | CE2 | TYR | 1313 | 977 | 965 | 1235 | 119 | -117 | 93 | C |
| ATOM | 1353 | CZ | TYR | 1313 | 18.623 | 9.492 | 40.512 | 1.00 | 8.17 | | C |
| ANISOU | 1353 | CZ | TYR | 1313 | 852 | 977 | 1274 | 90 | -95 | 40 | C |
| ATOM | 1354 | OH | TYR | 1313 | 18.263 | 8.221 | 40.121 | 1.00 | 8.89 | | O |
| ANISOU | 1354 | OH | TYR | 1313 | 1037 | 996 | 1343 | -40 | -72 | 78 | O |
| ATOM | 1355 | C | TYR | 1313 | 17.484 | 14.439 | 41.980 | 1.00 | 8.32 | | C |
| ANISOU | 1355 | C | TYR | 1313 | 970 | 852 | 1340 | -38 | -168 | 59 | C |
| ATOM | 1356 | O | TYR | 1313 | 16.546 | 13.905 | 41.387 | 1.00 | 8.80 | | O |
| ANISOU | 1356 | O | TYR | 1313 | 1027 | 867 | 1449 | -112 | -212 | 170 | O |
| ATOM | 1357 | N | GLU | 1314 | 17.311 | 14.957 | 43.211 | 1.00 | 8.79 | | N |
| ANISOU | 1357 | N | GLU | 1314 | 1128 | 897 | 1315 | -33 | -26 | 92 | N |
| ATOM | 1358 | CA | GLU | 1314 | 15.978 | 14.928 | 43.840 | 1.00 | 9.11 | | C |
| ANISOU | 1358 | CA | GLU | 1314 | 1131 | 1083 | 1247 | 15 | -100 | 102 | C |
| ATOM | 1359 | CB | GLU | 1314 | 16.068 | 15.522 | 45.241 | 1.00 | 10.28 | | C |
| ANISOU | 1359 | CB | GLU | 1314 | 1474 | 1111 | 1319 | -99 | -97 | -58 | C |
| ATOM | 1360 | CG | GLU | 1314 | 16.887 | 14.661 | 46.184 | 1.00 | 13.98 | | C |
| ANISOU | 1360 | CG | GLU | 1314 | 2223 | 1589 | 1500 | -428 | -534 | 490 | C |
| ATOM | 1361 | CD | GLU | 1314 | 17.282 | 15.420 | 47.430 | 1.00 | 17.01 | | C |
| ANISOU | 1361 | CD | GLU | 1314 | 2515 | 2250 | 1698 | -210 | -651 | 108 | C |
| ATOM | 1362 | OE1 | GLU | 1314 | 17.037 | 16.628 | 47.582 | 1.00 | 19.53 | | O1- |
| ANISOU | 1362 | OE1 | GLU | 1314 | 3322 | 2169 | 1930 | -452 | -133 | -7 | O1- |
| ATOM | 1363 | OE2 | GLU | 1314 | 17.877 | 14.756 | 48.312 | 1.00 | 26.91 | | O |
| ANISOU | 1363 | OE2 | GLU | 1314 | 5033 | 2792 | 2398 | -41 | -2136 | 64 | O |
| ATOM | 1364 | C | GLU | 1314 | 14.968 | 15.666 | 42.979 | 1.00 | 8.90 | | C |
| ANISOU | 1364 | C | GLU | 1314 | 1133 | 803 | 1446 | 23 | -189 | -28 | C |
| ATOM | 1365 | O | GLU | 1314 | 13.830 | 15.226 | 42.833 | 1.00 | 9.29 | | O |
| ANISOU | 1365 | O | GLU | 1314 | 1048 | 1026 | 1455 | -7 | -9 | -146 | O |
| ATOM | 1366 | N | VAL | 1315 | 15.397 | 16.803 | 42.388 | 1.00 | 8.90 | | N |
| ANISOU | 1366 | N | VAL | 1315 | 1210 | 781 | 1389 | 63 | -202 | -37 | N |
| ATOM | 1367 | CA | VAL | 1315 | 14.496 | 17.556 | 41.481 | 1.00 | 9.42 | | C |
| ANISOU | 1367 | CA | VAL | 1315 | 1509 | 745 | 1327 | 29 | -258 | -28 | C |
| ATOM | 1368 | CB | VAL | 1315 | 15.153 | 18.885 | 41.074 | 1.00 | 9.87 | | C |
| ANISOU | 1368 | CB | VAL | 1315 | 1542 | 910 | 1298 | -136 | -234 | 68 | C |
| ATOM | 1369 | CG1 | VAL | 1315 | 14.457 | 19.537 | 39.872 | 1.00 | 11.28 | | C |
| ANISOU | 1369 | CG1 | VAL | 1315 | 1424 | 1025 | 1837 | 16 | -470 | 294 | C |
| ATOM | 1370 | CG2 | VAL | 1315 | 15.133 | 19.854 | 42.263 | 1.00 | 12.36 | | C |
| ANISOU | 1370 | CG2 | VAL | 1315 | 1903 | 940 | 1851 | -88 | -252 | -327 | C |
| ATOM | 1371 | C | VAL | 1315 | 14.114 | 16.715 | 40.267 | 1.00 | 8.35 | | C |
| ANISOU | 1371 | C | VAL | 1315 | 1066 | 829 | 1278 | 87 | -133 | 18 | C |
| ATOM | 1372 | O | VAL | 1315 | 12.937 | 16.636 | 39.878 | 1.00 | 8.81 | | O |
| ANISOU | 1372 | O | VAL | 1315 | 1147 | 856 | 1343 | 41 | -257 | 60 | O |
| ATOM | 1373 | N | MET | 1316 | 15.089 | 16.058 | 39.643 | 1.00 | 8.31 | | N |
| ANISOU | 1373 | N | MET | 1316 | 1178 | 737 | 1240 | 55 | -98 | 83 | N |
| ATOM | 1374 | CA | MET | 1316 | 14.806 | 15.179 | 38.519 | 1.00 | 8.12 | | C |
| ANISOU | 1374 | CA | MET | 1316 | 1115 | 780 | 1191 | 30 | -46 | 37 | C |
| ATOM | 1375 | CB | MET | 1316 | 16.081 | 14.451 | 38.033 | 1.00 | 9.57 | | C |
| ANISOU | 1375 | CB | MET | 1316 | 1182 | 1155 | 1298 | 48 | 191 | 39 | C |
| ATOM | 1376 | CG | MET | 1316 | 17.096 | 15.347 | 37.375 | 1.00 | 9.36 | | C |
| ANISOU | 1376 | CG | MET | 1316 | 1139 | 1085 | 1332 | 25 | -87 | 160 | C |
| ATOM | 1377 | SD | MET | 1316 | 18.574 | 14.283 | 37.043 | 1.00 | 10.59 | | S |
| ANISOU | 1377 | SD | MET | 1316 | 1169 | 1286 | 1570 | 183 | 62 | 137 | S |
| ATOM | 1378 | CE | MET | 1316 | 19.767 | 15.577 | 36.652 | 1.00 | 10.80 | | C |
| ANISOU | 1378 | CE | MET | 1316 | 1245 | 1339 | 1519 | -2 | -62 | 357 | C |
| ATOM | 1379 | C | MET | 1316 | 13.769 | 14.138 | 38.905 | 1.00 | 7.73 | | C |
| ANISOU | 1379 | C | MET | 1316 | 876 | 820 | 1241 | 128 | -96 | 116 | C |
| ATOM | 1380 | O | MET | 1316 | 12.778 | 13.897 | 38.204 | 1.00 | 8.51 | | O |
| ANISOU | 1380 | O | MET | 1316 | 1051 | 979 | 1205 | 36 | -125 | 1 | O |
| ATOM | 1381 | N | LEU | 1317 | 14.019 | 13.496 | 40.062 | 1.00 | 7.64 | | N |
| ANISOU | 1381 | N | LEU | 1317 | 1057 | 700 | 1147 | 56 | 3 | 72 | N |
| ATOM | 1382 | CA | LEU | 1317 | 13.110 | 12.414 | 40.479 | 1.00 | 8.30 | | C |
| ANISOU | 1382 | CA | LEU | 1317 | 975 | 890 | 1289 | 5 | -91 | 174 | C |
| ATOM | 1383 | CB | LEU | 1317 | 13.648 | 11.747 | 41.741 | 1.00 | 8.74 | | C |
| ANISOU | 1383 | CB | LEU | 1317 | 956 | 973 | 1391 | 86 | -69 | 344 | C |
| ATOM | 1384 | CG | LEU | 1317 | 14.973 | 11.005 | 41.584 | 1.00 | 8.36 | | C |
| ANISOU | 1384 | CG | LEU | 1317 | 992 | 915 | 1268 | 92 | -119 | 49 | C |
| ATOM | 1385 | CD1 | LEU | 1317 | 15.481 | 10.605 | 42.984 | 1.00 | 9.08 | | C |
| ANISOU | 1385 | CD1 | LEU | 1317 | 1164 | 1025 | 1262 | 136 | -220 | 73 | C |
| ATOM | 1386 | CD2 | LEU | 1317 | 14.845 | 9.800 | 40.669 | 1.00 | 9.69 | | C |
| ANISOU | 1386 | CD2 | LEU | 1317 | 1397 | 1014 | 1269 | 52 | -219 | 29 | C |
| ATOM | 1387 | C | LEU | 1317 | 11.689 | 12.920 | 40.721 | 1.00 | 8.15 | | C |
| ANISOU | 1387 | C | LEU | 1317 | 950 | 856 | 1292 | -7 | -175 | 174 | C |
| ATOM | 1388 | O | LEU | 1317 | 10.719 | 12.215 | 40.427 | 1.00 | 8.88 | | O |
| ANISOU | 1388 | O | LEU | 1317 | 988 | 929 | 1458 | -50 | -109 | 157 | O |
| ATOM | 1389 | N | LYS | 1318 | 11.564 | 14.145 | 41.256 | 1.00 | 8.84 | | N |
| ANISOU | 1389 | N | LYS | 1318 | 986 | 857 | 1516 | 22 | -2 | 115 | N |
| ATOM | 1390 | CA | LYS | 1318 | 10.237 | 14.724 | 41.450 | 1.00 | 9.39 | | C |
| ANISOU | 1390 | CA | LYS | 1318 | 1096 | 1067 | 1405 | 145 | -110 | 90 | C |
| ATOM | 1391 | CB | LYS | 1318 | 10.349 | 16.068 | 42.136 | 1.00 | 11.75 | | C |
| ANISOU | 1391 | CB | LYS | 1318 | 1266 | 1531 | 1669 | 245 | -26 | -489 | C |
| ATOM | 1392 | CG | LYS | 1318 | 10.795 | 16.124 | 43.575 | 1.00 | 17.79 | | C |
| ANISOU | 1392 | CG | LYS | 1318 | 2754 | 2150 | 1858 | 638 | -710 | -484 | C |
| ATOM | 1393 | CD | LYS | 1318 | 11.118 | 17.543 | 44.042 | 1.00 | 25.70 | | C |
| ANISOU | 1393 | CD | LYS | 1318 | 3947 | 2771 | 3047 | 292 | -1417 | -1306 | C |
| ATOM | 1394 | CE | LYS | 1318 | 11.580 | 17.623 | 45.479 | 1.00 | 28.84 | | C |
| ANISOU | 1394 | CE | LYS | 1318 | 4394 | 4108 | 2455 | -814 | -529 | -1296 | C |
| ATOM | 1395 | NZ | LYS | 1318 | 10.462 | 17.760 | 46.451 | 1.00 | 33.92 | | N1+ |
| ANISOU | 1395 | NZ | LYS | 1318 | 4807 | 4444 | 3638 | -410 | 437 | 733 | N1+ |
| ATOM | 1396 | C | LYS | 1318 | 9.517 | 14.911 | 40.115 | 1.00 | 8.18 | | C |
| ANISOU | 1396 | C | LYS | 1318 | 932 | 837 | 1340 | 20 | -1 | 116 | C |
| ATOM | 1397 | O | LYS | 1318 | 8.280 | 14.728 | 40.046 | 1.00 | 8.98 | | O |
| ANISOU | 1397 | O | LYS | 1318 | 916 | 1053 | 1443 | 31 | 3 | 140 | O |
| ATOM | 1398 | N | CYS | 1319 | 10.247 | 15.266 | 39.075 | 1.00 | 8.47 | | N |
| ANISOU | 1398 | N | CYS | 1319 | 1071 | 888 | 1258 | 64 | -19 | 66 | N |
| ATOM | 1399 | CA | CYS | 1319 | 9.677 | 15.412 | 37.751 | 1.00 | 8.55 | | C |
| ANISOU | 1399 | CA | CYS | 1319 | 1125 | 883 | 1240 | -45 | -34 | 100 | C |
| ATOM | 1400 | CB | CYS | 1319 | 10.704 | 15.975 | 36.728 | 1.00 | 7.71 | | C |
| ANISOU | 1400 | CB | CYS | 1319 | 929 | 690 | 1312 | -1 | -39 | 105 | C |
| ATOM | 1401 | SG | CYS | 1319 | 11.231 | 17.678 | 37.125 | 1.00 | 9.29 | | S |
| ANISOU | 1401 | SG | CYS | 1319 | 1184 | 878 | 1467 | -93 | -132 | 88 | S |
| ATOM | 1402 | C | CYS | 1319 | 9.131 | 14.102 | 37.162 | 1.00 | 8.19 | | C |
| ANISOU | 1402 | C | CYS | 1319 | 855 | 866 | 1392 | 10 | -44 | 101 | C |
| ATOM | 1403 | O | CYS | 1319 | 8.314 | 14.142 | 36.246 | 1.00 | 8.74 | | O |
| ANISOU | 1403 | O | CYS | 1319 | 860 | 955 | 1505 | -31 | -92 | 50 | O |
| ATOM | 1404 | N | TRP | 1320 | 9.610 | 12.966 | 37.699 | 1.00 | 8.39 | | N |
| ANISOU | 1404 | N | TRP | 1320 | 928 | 824 | 1437 | 50 | -33 | 96 | N |
| ATOM | 1405 | CA | TRP | 1320 | 9.215 | 11.659 | 37.164 | 1.00 | 8.96 | | C |
| ANISOU | 1405 | CA | TRP | 1320 | 779 | 899 | 1726 | -63 | -4 | 32 | C |
| ATOM | 1406 | CB | TRP | 1320 | 10.442 | 10.786 | 36.861 | 1.00 | 9.04 | | C |
| ANISOU | 1406 | CB | TRP | 1320 | 1064 | 885 | 1485 | 101 | 64 | 135 | C |
| ATOM | 1407 | CG | TRP | 1320 | 11.459 | 11.467 | 35.966 | 1.00 | 7.91 | | C |
| ANISOU | 1407 | CG | TRP | 1320 | 973 | 823 | 1208 | 107 | -79 | -13 | C |
| ATOM | 1408 | CD2 | TRP | 1320 | 12.888 | 11.363 | 36.082 | 1.00 | 7.55 | | C |
| ANISOU | 1408 | CD2 | TRP | 1320 | 990 | 687 | 1191 | -42 | -134 | -100 | C |
| ATOM | 1409 | CE2 | TRP | 1320 | 13.454 | 12.152 | 35.052 | 1.00 | 7.72 | | C |
| ANISOU | 1409 | CE2 | TRP | 1320 | 1053 | 704 | 1176 | 99 | -49 | -25 | C |
| ATOM | 1410 | CE3 | TRP | 1320 | 13.709 | 10.685 | 36.959 | 1.00 | 7.69 | | C |
| ANISOU | 1410 | CE3 | TRP | 1320 | 1016 | 568 | 1337 | 46 | -141 | -73 | C |
| ATOM | 1411 | CD1 | TRP | 1320 | 11.216 | 12.296 | 34.890 | 1.00 | 7.94 | | C |
| ANISOU | 1411 | CD1 | TRP | 1320 | 1124 | 689 | 1203 | 3 | -119 | -59 | C |
| ATOM | 1412 | NE1 | TRP | 1320 | 12.425 | 12.708 | 34.339 | 1.00 | 8.37 | | N |
| ANISOU | 1412 | NE1 | TRP | 1320 | 972 | 865 | 1344 | -47 | -219 | 8 | N |
| ATOM | 1413 | CZ2 | TRP | 1320 | 14.839 | 12.257 | 34.903 | 1.00 | 8.35 | | C |
| ANISOU | 1413 | CZ2 | TRP | 1320 | 1043 | 868 | 1261 | 116 | -80 | 1 | C |
| ATOM | 1414 | CZ3 | TRP | 1320 | 15.082 | 10.786 | 36.816 | 1.00 | 8.23 | | C |
| ANISOU | 1414 | CZ3 | TRP | 1320 | 1003 | 777 | 1348 | -29 | -222 | -13 | C |
| ATOM | 1415 | CH2 | TRP | 1320 | 15.626 | 11.574 | 35.786 | 1.00 | 8.38 | | C |
| ANISOU | 1415 | CH2 | TRP | 1320 | 1036 | 637 | 1512 | -75 | -247 | 65 | C |
| ATOM | 1416 | C | TRP | 1320 | 8.252 | 10.923 | 38.100 | 1.00 | 8.45 | | C |
| ANISOU | 1416 | C | TRP | 1320 | 996 | 848 | 1366 | 29 | -131 | 116 | C |
| ATOM | 1417 | O | TRP | 1320 | 8.082 | 9.721 | 38.024 | 1.00 | 9.72 | | O |
| ANISOU | 1417 | O | TRP | 1320 | 1044 | 862 | 1788 | 3 | -69 | 131 | O |
| ATOM | 1418 | N | HIS | 1321 | 7.583 | 11.669 | 38.980 | 1.00 | 8.92 | | N |
| ANISOU | 1418 | N | HIS | 1321 | 1016 | 1103 | 1272 | -95 | -44 | 49 | N |
| ATOM | 1419 | CA | HIS | 1321 | 6.612 | 11.050 | 39.834 | 1.00 | 9.13 | | C |
| ANISOU | 1419 | CA | HIS | 1321 | 995 | 1057 | 1418 | -101 | -71 | 144 | C |
| ATOM | 1420 | CB | HIS | 1321 | 5.943 | 12.109 | 40.706 | 1.00 | 9.45 | | C |
| ANISOU | 1420 | CB | HIS | 1321 | 906 | 1345 | 1341 | -31 | 2 | 193 | C |
| ATOM | 1421 | CG | HIS | 1321 | 5.210 | 11.590 | 41.890 | 1.00 | 10.89 | | C |
| ANISOU | 1421 | CG | HIS | 1321 | 985 | 1715 | 1439 | -53 | -20 | 254 | C |
| ATOM | 1422 | CD2 | HIS | 1321 | 4.073 | 10.848 | 41.874 | 1.00 | 11.53 | | C |
| ANISOU | 1422 | CD2 | HIS | 1321 | 877 | 1862 | 1643 | -115 | 72 | 651 | C |
| ATOM | 1423 | ND1 | HIS | 1321 | 5.550 | 11.807 | 43.185 | 1.00 | 15.36 | | N |
| ANISOU | 1423 | ND1 | HIS | 1321 | 2062 | 2349 | 1426 | -476 | 76 | 327 | N |
| ATOM | 1424 | CE1 | HIS | 1321 | 4.629 | 11.180 | 43.938 | 1.00 | 14.96 | | C |
| ANISOU | 1424 | CE1 | HIS | 1321 | 1152 | 2956 | 1576 | -61 | 50 | 634 | C |
| ATOM | 1425 | NE2 | HIS | 1321 | 3.746 | 10.604 | 43.152 | 1.00 | 18.70 | | N |
| ANISOU | 1425 | NE2 | HIS | 1321 | 1943 | 3439 | 1723 | -748 | 57 | 688 | N |
| ATOM | 1426 | C | HIS | 1321 | 5.544 | 10.357 | 38.990 | 1.00 | 9.33 | | C |
| ANISOU | 1426 | C | HIS | 1321 | 1131 | 955 | 1460 | -122 | -192 | 345 | C |
| ATOM | 1427 | O | HIS | 1321 | 5.104 | 10.975 | 38.022 | 1.00 | 9.43 | | O |
| ANISOU | 1427 | O | HIS | 1321 | 1204 | 1027 | 1350 | -33 | -172 | 289 | O |
| ATOM | 1428 | N | PRO | 1322 | 5.110 | 9.140 | 39.306 | 1.00 | 10.76 | | N |
| ANISOU | 1428 | N | PRO | 1322 | 1128 | 1010 | 1949 | -160 | -296 | 509 | N |
| ATOM | 1429 | CD | PRO | 1322 | 5.546 | 8.290 | 40.411 | 1.00 | 11.80 | | C |
| ANISOU | 1429 | CD | PRO | 1322 | 1266 | 1147 | 2069 | -152 | -107 | 771 | C |
| ATOM | 1430 | CA | PRO | 1322 | 4.078 | 8.523 | 38.436 | 1.00 | 11.63 | | C |
| ANISOU | 1430 | CA | PRO | 1322 | 1189 | 1067 | 2162 | -252 | -204 | 350 | C |
| ATOM | 1431 | CB | PRO | 1322 | 4.003 | 7.095 | 39.017 | 1.00 | 15.58 | | C |
| ANISOU | 1431 | CB | PRO | 1322 | 1730 | 1065 | 3123 | -277 | -639 | 557 | C |
| ATOM | 1432 | CG | PRO | 1322 | 4.456 | 7.263 | 40.447 | 1.00 | 15.69 | | C |
| ANISOU | 1432 | CG | PRO | 1322 | 1544 | 1507 | 2912 | -409 | -475 | 1060 | C |
| ATOM | 1433 | C | PRO | 1322 | 2.739 | 9.235 | 38.500 | 1.00 | 11.20 | | C |
| ANISOU | 1433 | C | PRO | 1322 | 1211 | 1119 | 1927 | -222 | -241 | 426 | C |
| ATOM | 1434 | O | PRO | 1322 | 1.962 | 9.020 | 37.570 | 1.00 | 12.81 | | O |
| ANISOU | 1434 | O | PRO | 1322 | 1406 | 1443 | 2020 | -85 | -273 | 89 | O |
| ATOM | 1435 | N | LYS | 1323 | 2.450 | 10.021 | 39.534 | 1.00 | 10.65 | | N |
| ANISOU | 1435 | N | LYS | 1323 | 988 | 1354 | 1705 | -267 | -37 | 485 | N |
| ATOM | 1436 | CA | LYS | 1323 | 1.184 | 10.764 | 39.595 | 1.00 | 12.28 | | C |
| ANISOU | 1436 | CA | LYS | 1323 | 1085 | 1704 | 1876 | -93 | -219 | 380 | C |
| ATOM | 1437 | CB | LYS | 1323 | 0.593 | 10.863 | 40.987 | 1.00 | 15.93 | | C |
| ANISOU | 1437 | CB | LYS | 1323 | 1821 | 1865 | 2365 | -254 | 588 | 31 | C |
| ATOM | 1442 | C | LYS | 1323 | 1.469 | 12.162 | 39.063 | 1.00 | 12.40 | | C |
| ANISOU | 1442 | C | LYS | 1323 | 1310 | 1508 | 1894 | 24 | -242 | 227 | C |
| ATOM | 1443 | O | LYS | 1323 | 2.182 | 12.934 | 39.729 | 1.00 | 11.98 | | O |
| ANISOU | 1443 | O | LYS | 1323 | 1356 | 1591 | 1604 | -97 | -17 | 198 | O |
| ATOM | 1444 | N | ALA | 1324 | 0.939 | 12.482 | 37.880 | 1.00 | 11.27 | | N |
| ANISOU | 1444 | N | ALA | 1324 | 981 | 1505 | 1798 | 79 | -103 | 231 | N |
| ATOM | 1445 | CA | ALA | 1324 | 1.272 | 13.747 | 37.248 | 1.00 | 11.18 | | C |
| ANISOU | 1445 | CA | ALA | 1324 | 1262 | 1526 | 1462 | -100 | -94 | 96 | C |
| ATOM | 1446 | CB | ALA | 1324 | 0.424 | 13.928 | 35.982 | 1.00 | 11.94 | | C |
| ANISOU | 1446 | CB | ALA | 1324 | 1319 | 1674 | 1543 | -296 | -244 | 129 | C |
| ATOM | 1447 | C | ALA | 1324 | 1.035 | 14.941 | 38.147 | 1.00 | 11.19 | | C |
| ANISOU | 1447 | C | ALA | 1324 | 1053 | 1641 | 1557 | -228 | -67 | -24 | C |
| ATOM | 1448 | O | ALA | 1324 | 1.830 | 15.903 | 38.165 | 1.00 | 11.22 | | O |
| ANISOU | 1448 | O | ALA | 1324 | 1177 | 1371 | 1714 | -102 | -158 | 219 | O |
| ATOM | 1449 | N | GLU | 1325 | -0.050 | 14.952 | 38.909 | 1.00 | 11.86 | | N |
| ANISOU | 1449 | N | GLU | 1325 | 1005 | 1459 | 2041 | 192 | 90 | 264 | N |
| ATOM | 1450 | CA | GLU | 1325 | -0.377 | 16.098 | 39.768 | 1.00 | 13.69 | | C |
| ANISOU | 1450 | CA | GLU | 1325 | 1113 | 1567 | 2520 | 211 | 159 | 25 | C |
| ATOM | 1451 | CB | GLU | 1325 | -1.831 | 15.883 | 40.233 | 1.00 | 17.24 | | C |
| ANISOU | 1451 | CB | GLU | 1325 | 1083 | 2260 | 3207 | 187 | 431 | -401 | C |
| ATOM | 1456 | C | GLU | 1325 | 0.635 | 16.276 | 40.879 | 1.00 | 13.70 | | C |
| ANISOU | 1456 | C | GLU | 1325 | 1271 | 1598 | 2336 | 72 | 233 | -65 | C |
| ATOM | 1457 | O | GLU | 1325 | 0.685 | 17.375 | 41.467 | 1.00 | 17.90 | | O |
| ANISOU | 1457 | O | GLU | 1325 | 1520 | 1913 | 3370 | 170 | -14 | -547 | O |
| ATOM | 1458 | N | MET | 1326 | 1.454 | 15.287 | 41.216 | 1.00 | 12.21 | | N |
| ANISOU | 1458 | N | MET | 1326 | 1168 | 1477 | 1995 | -238 | 169 | 283 | N |
| ATOM | 1459 | CA | MET | 1326 | 2.418 | 15.414 | 42.307 | 1.00 | 11.67 | | C |
| ANISOU | 1459 | CA | MET | 1326 | 1444 | 1436 | 1553 | -328 | 326 | 251 | C |
| ATOM | 1460 | CB | MET | 1326 | 2.605 | 14.037 | 42.976 | 1.00 | 15.41 | | C |
| ANISOU | 1460 | CB | MET | 1326 | 1916 | 1765 | 2174 | -680 | -197 | 724 | C |
| ATOM | 1461 | CG | MET | 1326 | 1.297 | 13.672 | 43.655 | 1.00 | 21.33 | | C |
| ANISOU | 1461 | CG | MET | 1326 | 2606 | 2259 | 3238 | -914 | 364 | 1411 | C |
| ATOM | 1462 | SD | MET | 1326 | 1.016 | 14.723 | 45.056 | 1.00 | 42.43 | | S |
| ANISOU | 1462 | SD | MET | 1326 | 4873 | 6868 | 4382 | -522 | 1952 | -603 | S |
| ATOM | 1463 | CE | MET | 1326 | 1.072 | 13.580 | 46.440 | 1.00 | 72.67 | | C |
| ANISOU | 1463 | CE | MET | 1326 | 12450 | 12012 | 3148 | -6397 | -96 | 1087 | C |
| ATOM | 1464 | C | MET | 1326 | 3.754 | 15.908 | 41.815 | 1.00 | 10.99 | | C |
| ANISOU | 1464 | C | MET | 1326 | 1259 | 1416 | 1499 | -217 | 148 | 166 | C |
| ATOM | 1465 | O | MET | 1326 | 4.663 | 16.137 | 42.611 | 1.00 | 13.71 | | O |
| ANISOU | 1465 | O | MET | 1326 | 1504 | 2145 | 1560 | -366 | 20 | 45 | O |
| ATOM | 1466 | N | ARG | 1327 | 3.918 | 16.079 | 40.498 | 1.00 | 9.88 | | N |
| ANISOU | 1466 | N | ARG | 1327 | 1112 | 1186 | 1457 | -149 | 139 | 187 | N |
| ATOM | 1467 | CA | ARG | 1327 | 5.145 | 16.649 | 39.965 | 1.00 | 9.45 | | C |
| ANISOU | 1467 | CA | ARG | 1327 | 1081 | 1097 | 1414 | -109 | 191 | -53 | C |
| ATOM | 1468 | CB | ARG | 1327 | 5.193 | 16.418 | 38.449 | 1.00 | 9.35 | | C |
| ANISOU | 1468 | CB | ARG | 1327 | 1252 | 941 | 1359 | 64 | 65 | 0 | C |
| ATOM | 1469 | CG | ARG | 1327 | 5.279 | 14.945 | 38.086 | 1.00 | 9.03 | | C |
| ANISOU | 1469 | CG | ARG | 1327 | 1205 | 813 | 1414 | -61 | -303 | 69 | C |
| ATOM | 1470 | CD | ARG | 1327 | 5.273 | 14.706 | 36.548 | 1.00 | 8.86 | | C |
| ANISOU | 1470 | CD | ARG | 1327 | 996 | 941 | 1428 | 6 | -317 | -4 | C |
| ATOM | 1471 | NE | ARG | 1327 | 4.902 | 13.295 | 36.410 | 1.00 | 9.09 | | N |
| ANISOU | 1471 | NE | ARG | 1327 | 1098 | 958 | 1399 | -60 | -118 | -52 | N |
| ATOM | 1472 | CZ | ARG | 1327 | 4.171 | 12.800 | 35.394 | 1.00 | 8.45 | | C |
| ANISOU | 1472 | CZ | ARG | 1327 | 1106 | 860 | 1244 | 7 | -138 | -1 | C |
| ATOM | 1473 | NH1 | ARG | 1327 | 3.757 | 13.543 | 34.369 | 1.00 | 8.64 | | N1+ |
| ANISOU | 1473 | NH1 | ARG | 1327 | 1014 | 911 | 1357 | 183 | -48 | 51 | N1+ |
| ATOM | 1474 | NH2 | ARG | 1327 | 3.882 | 11.505 | 35.429 | 1.00 | 9.00 | | N |
| ANISOU | 1474 | NH2 | ARG | 1327 | 1002 | 841 | 1575 | 0 | -39 | 59 | N |
| ATOM | 1475 | C | ARG | 1327 | 5.180 | 18.125 | 40.263 | 1.00 | 8.46 | | C |
| ANISOU | 1475 | C | ARG | 1327 | 1010 | 1092 | 1113 | -23 | -52 | 52 | C |
| ATOM | 1476 | O | ARG | 1327 | 4.138 | 18.778 | 40.332 | 1.00 | 10.53 | | O |
| ANISOU | 1476 | O | ARG | 1327 | 1103 | 1088 | 1810 | 60 | 6 | 105 | O |
| ATOM | 1477 | N | PRO | 1328 | 6.341 | 18.733 | 40.403 | 1.00 | 8.99 | | N |
| ANISOU | 1477 | N | PRO | 1328 | 1024 | 973 | 1418 | 23 | -149 | 40 | N |
| ATOM | 1478 | CD | PRO | 1328 | 7.695 | 18.104 | 40.308 | 1.00 | 10.02 | | C |
| ANISOU | 1478 | CD | PRO | 1328 | 1009 | 1033 | 1766 | 58 | -168 | 132 | C |
| ATOM | 1479 | CA | PRO | 1328 | 6.404 | 20.170 | 40.675 | 1.00 | 9.59 | | C |
| ANISOU | 1479 | CA | PRO | 1328 | 1136 | 971 | 1537 | 14 | -184 | 40 | C |
| ATOM | 1480 | CB | PRO | 1328 | 7.905 | 20.445 | 40.874 | 1.00 | 11.74 | | C |
| ANISOU | 1480 | CB | PRO | 1328 | 1172 | 1103 | 2185 | -42 | -211 | -34 | C |
| ATOM | 1481 | CG | PRO | 1328 | 8.611 | 19.279 | 40.284 | 1.00 | 14.90 | | C |
| ANISOU | 1481 | CG | PRO | 1328 | 1095 | 1444 | 3123 | -178 | 78 | -563 | C |
| ATOM | 1482 | C | PRO | 1328 | 5.903 | 20.993 | 39.494 | 1.00 | 9.40 | | C |
| ANISOU | 1482 | C | PRO | 1328 | 1204 | 926 | 1443 | -55 | 48 | 51 | C |
| ATOM | 1483 | O | PRO | 1328 | 5.979 | 20.570 | 38.344 | 1.00 | 11.20 | | O |
| ANISOU | 1483 | O | PRO | 1328 | 1733 | 1113 | 1411 | 203 | -127 | 26 | O |
| ATOM | 1484 | N | SER | 1329 | 5.398 | 22.192 | 39.797 | 1.00 | 8.90 | | N |
| ANISOU | 1484 | N | SER | 1329 | 959 | 866 | 1556 | -99 | -22 | 38 | N |
| ATOM | 1485 | CA | SER | 1329 | 5.189 | 23.154 | 38.720 | 1.00 | 9.51 | | C |
| ANISOU | 1485 | CA | SER | 1329 | 972 | 1064 | 1578 | 46 | 135 | 135 | C |
| ATOM | 1486 | CB | SER | 1329 | 4.347 | 24.325 | 39.215 | 1.00 | 10.36 | | C |
| ANISOU | 1486 | CB | SER | 1329 | 870 | 1104 | 1963 | 107 | 23 | 12 | C |
| ATOM | 1487 | OG | SER | 1329 | 5.083 | 25.028 | 40.210 | 1.00 | 11.39 | | O |
| ANISOU | 1487 | OG | SER | 1329 | 1047 | 1272 | 2010 | 77 | 144 | -215 | O |
| ATOM | 1488 | C | SER | 1329 | 6.499 | 23.697 | 38.209 | 1.00 | 8.13 | | C |
| ANISOU | 1488 | C | SER | 1329 | 856 | 879 | 1352 | 42 | -74 | 26 | C |
| ATOM | 1489 | O | SER | 1329 | 7.547 | 23.638 | 38.880 | 1.00 | 8.32 | | O |
| ANISOU | 1489 | O | SER | 1329 | 952 | 933 | 1274 | 45 | -60 | -24 | O |
| ATOM | 1490 | N | PHE | 1330 | 6.458 | 24.285 | 36.998 | 1.00 | 8.31 | | N |
| ANISOU | 1490 | N | PHE | 1330 | 804 | 861 | 1492 | 38 | -78 | 184 | N |
| ATOM | 1491 | CA | PHE | 1330 | 7.644 | 24.979 | 36.540 | 1.00 | 8.34 | | C |
| ANISOU | 1491 | CA | PHE | 1330 | 893 | 909 | 1367 | -124 | -43 | 29 | C |
| ATOM | 1492 | CB | PHE | 1330 | 7.468 | 25.415 | 35.071 | 1.00 | 8.88 | | C |
| ANISOU | 1492 | CB | PHE | 1330 | 1188 | 879 | 1308 | 10 | 28 | -12 | C |
| ATOM | 1493 | CG | PHE | 1330 | 7.674 | 24.227 | 34.118 | 1.00 | 8.63 | | C |
| ANISOU | 1493 | CG | PHE | 1330 | 973 | 815 | 1491 | 38 | -73 | -2 | C |
| ATOM | 1494 | CD1 | PHE | 1330 | 8.916 | 23.623 | 33.985 | 1.00 | 8.74 | | C |
| ANISOU | 1494 | CD1 | PHE | 1330 | 1069 | 935 | 1316 | 184 | -10 | 189 | C |
| ATOM | 1495 | CD2 | PHE | 1330 | 6.617 | 23.734 | 33.371 | 1.00 | 8.48 | | C |
| ANISOU | 1495 | CD2 | PHE | 1330 | 1136 | 914 | 1173 | -204 | -102 | 205 | C |
| ATOM | 1496 | CE1 | PHE | 1330 | 9.120 | 22.546 | 33.118 | 1.00 | 8.63 | | C |
| ANISOU | 1496 | CE1 | PHE | 1330 | 1275 | 795 | 1211 | -16 | -21 | 113 | C |
| ATOM | 1497 | CE2 | PHE | 1330 | 6.788 | 22.659 | 32.489 | 1.00 | 9.18 | | C |
| ANISOU | 1497 | CE2 | PHE | 1330 | 1220 | 970 | 1297 | -76 | 39 | 130 | C |
| ATOM | 1498 | CZ | PHE | 1330 | 8.052 | 22.079 | 32.374 | 1.00 | 9.20 | | C |
| ANISOU | 1498 | CZ | PHE | 1330 | 1103 | 1052 | 1338 | -133 | 9 | 332 | C |
| ATOM | 1499 | C | PHE | 1330 | 8.011 | 26.180 | 37.434 | 1.00 | 8.35 | | C |
| ANISOU | 1499 | C | PHE | 1330 | 862 | 893 | 1416 | 15 | -80 | 5 | C |
| ATOM | 1500 | O | PHE | 1330 | 9.191 | 26.494 | 37.549 | 1.00 | 8.94 | | O |
| ANISOU | 1500 | O | PHE | 1330 | 927 | 1017 | 1452 | -77 | -106 | -17 | O |
| ATOM | 1501 | N | SER | 1331 | 7.032 | 26.827 | 38.083 | 1.00 | 8.95 | | N |
| ANISOU | 1501 | N | SER | 1331 | 997 | 947 | 1458 | 151 | -23 | 62 | N |
| ATOM | 1502 | CA | SER | 1331 | 7.413 | 27.848 | 39.072 | 1.00 | 10.05 | | C |
| ANISOU | 1502 | CA | SER | 1331 | 1209 | 1164 | 1445 | -71 | 150 | -122 | C |
| ATOM | 1503 | CB | SER | 1331 | 6.140 | 28.511 | 39.611 | 1.00 | 14.00 | | C |
| ANISOU | 1503 | CB | SER | 1331 | 1207 | 1679 | 2434 | 386 | 72 | -399 | C |
| ATOM | 1504 | OG | SER | 1331 | 5.497 | 29.274 | 38.624 | 1.00 | 20.39 | | O |
| ANISOU | 1504 | OG | SER | 1331 | 2307 | 2271 | 3170 | 627 | -286 | 79 | O |
| ATOM | 1505 | C | SER | 1331 | 8.248 | 27.284 | 40.207 | 1.00 | 9.10 | | C |
| ANISOU | 1505 | C | SER | 1331 | 1064 | 1009 | 1385 | -54 | 143 | -244 | C |
| ATOM | 1506 | O | SER | 1331 | 9.250 | 27.877 | 40.592 | 1.00 | 9.80 | | O |
| ANISOU | 1506 | O | SER | 1331 | 1049 | 923 | 1749 | 16 | 91 | -208 | O |
| ATOM | 1507 | N | GLU | 1332 | 7.820 | 26.148 | 40.747 | 1.00 | 9.28 | | N |
| ANISOU | 1507 | N | GLU | 1332 | 1065 | 957 | 1503 | 52 | 54 | -176 | N |
| ATOM | 1508 | CA | GLU | 1332 | 8.616 | 25.507 | 41.796 | 1.00 | 9.73 | | C |
| ANISOU | 1508 | CA | GLU | 1332 | 1137 | 1109 | 1452 | 24 | 115 | -153 | C |
| ATOM | 1509 | CB | GLU | 1332 | 7.881 | 24.270 | 42.319 | 1.00 | 10.32 | | C |
| ANISOU | 1509 | CB | GLU | 1332 | 1157 | 1239 | 1524 | -133 | 191 | -85 | C |
| ATOM | 1510 | CG | GLU | 1332 | 8.757 | 23.552 | 43.390 | 1.00 | 15.56 | | C |
| ANISOU | 1510 | CG | GLU | 1332 | 1949 | 1811 | 2154 | -114 | -104 | 517 | C |
| ATOM | 1511 | CD | GLU | 1332 | 8.107 | 22.358 | 44.053 | 1.00 | 17.02 | | C |
| ANISOU | 1511 | CD | GLU | 1332 | 2266 | 1867 | 2332 | -276 | -121 | 589 | C |
| ATOM | 1512 | OE1 | GLU | 1332 | 6.885 | 22.139 | 43.922 | 1.00 | 23.13 | | O1- |
| ANISOU | 1512 | OE1 | GLU | 1332 | 2459 | 2653 | 3677 | -929 | -272 | 699 | O1- |
| ATOM | 1513 | OE2 | GLU | 1332 | 8.806 | 21.599 | 44.736 | 1.00 | 24.46 | | O |
| ANISOU | 1513 | OE2 | GLU | 1332 | 3352 | 2542 | 3399 | -454 | -763 | 1449 | O |
| ATOM | 1514 | C | GLU | 1332 | 9.996 | 25.115 | 41.297 | 1.00 | 8.05 | | C |
| ANISOU | 1514 | C | GLU | 1332 | 1019 | 785 | 1257 | -115 | 8 | -108 | C |
| ATOM | 1515 | O | GLU | 1332 | 11.011 | 25.264 | 41.978 | 1.00 | 9.15 | | O |
| ANISOU | 1515 | O | GLU | 1332 | 1127 | 1090 | 1258 | -157 | -41 | -28 | O |
| ATOM | 1516 | N | LEU | 1333 | 10.039 | 24.582 | 40.060 | 1.00 | 8.35 | | N |
| ANISOU | 1516 | N | LEU | 1333 | 940 | 935 | 1297 | -105 | 73 | -135 | N |
| ATOM | 1517 | CA | LEU | 1333 | 11.339 | 24.192 | 39.520 | 1.00 | 7.78 | | C |
| ANISOU | 1517 | CA | LEU | 1333 | 882 | 961 | 1113 | -117 | 12 | -112 | C |
| ATOM | 1518 | CB | LEU | 1333 | 11.145 | 23.464 | 38.167 | 1.00 | 8.52 | | C |
| ANISOU | 1518 | CB | LEU | 1333 | 1207 | 780 | 1252 | -8 | -209 | -218 | C |
| ATOM | 1519 | CG | LEU | 1333 | 10.493 | 22.077 | 38.311 | 1.00 | 8.18 | | C |
| ANISOU | 1519 | CG | LEU | 1333 | 863 | 792 | 1451 | 77 | -153 | -145 | C |
| ATOM | 1520 | CD1 | LEU | 1333 | 9.925 | 21.631 | 36.975 | 1.00 | 9.19 | | C |
| ANISOU | 1520 | CD1 | LEU | 1333 | 1204 | 830 | 1456 | -25 | -273 | -204 | C |
| ATOM | 1521 | CD2 | LEU | 1333 | 11.501 | 21.074 | 38.872 | 1.00 | 9.86 | | C |
| ANISOU | 1521 | CD2 | LEU | 1333 | 1184 | 893 | 1670 | 279 | -190 | -65 | C |
| ATOM | 1522 | C | LEU | 1333 | 12.263 | 25.374 | 39.336 | 1.00 | 7.37 | | C |
| ANISOU | 1522 | C | LEU | 1333 | 881 | 844 | 1074 | -32 | -146 | -85 | C |
| ATOM | 1523 | O | LEU | 1333 | 13.466 | 25.290 | 39.641 | 1.00 | 7.98 | | O |
| ANISOU | 1523 | O | LEU | 1333 | 831 | 958 | 1242 | 31 | -120 | -212 | O |
| ATOM | 1524 | N | VAL | 1334 | 11.772 | 26.509 | 38.857 | 1.00 | 7.97 | | N |
| ANISOU | 1524 | N | VAL | 1334 | 1034 | 794 | 1201 | 10 | -184 | -59 | N |
| ATOM | 1525 | CA | VAL | 1334 | 12.628 | 27.715 | 38.762 | 1.00 | 8.35 | | C |
| ANISOU | 1525 | CA | VAL | 1334 | 1117 | 810 | 1244 | -67 | -190 | -110 | C |
| ATOM | 1526 | CB | VAL | 1334 | 11.850 | 28.891 | 38.170 | 1.00 | 8.29 | | C |
| ANISOU | 1526 | CB | VAL | 1334 | 1188 | 745 | 1217 | -39 | -232 | -92 | C |
| ATOM | 1527 | CG1 | VAL | 1334 | 12.602 | 30.211 | 38.360 | 1.00 | 9.62 | | C |
| ANISOU | 1527 | CG1 | VAL | 1334 | 1217 | 775 | 1663 | -96 | -19 | -135 | C |
| ATOM | 1528 | CG2 | VAL | 1334 | 11.575 | 28.593 | 36.689 | 1.00 | 8.88 | | C |
| ANISOU | 1528 | CG2 | VAL | 1334 | 1277 | 985 | 1112 | -24 | -25 | -74 | C |
| ATOM | 1529 | C | VAL | 1334 | 13.172 | 28.074 | 40.130 | 1.00 | 7.99 | | C |
| ANISOU | 1529 | C | VAL | 1334 | 898 | 909 | 1228 | 21 | -163 | -168 | C |
| ATOM | 1530 | O | VAL | 1334 | 14.351 | 28.374 | 40.294 | 1.00 | 8.68 | | O |
| ANISOU | 1530 | O | VAL | 1334 | 948 | 970 | 1380 | -21 | -209 | -158 | O |
| ATOM | 1531 | N | SER | 1335 | 12.293 | 28.068 | 41.153 | 1.00 | 8.46 | | N |
| ANISOU | 1531 | N | SER | 1335 | 1111 | 868 | 1236 | -14 | -66 | -147 | N |
| ATOM | 1532 | CA | SER | 1335 | 12.754 | 28.474 | 42.498 | 1.00 | 8.67 | | C |
| ANISOU | 1532 | CA | SER | 1335 | 1018 | 1019 | 1256 | 39 | -71 | -214 | C |
| ATOM | 1533 | CB | SER | 1335 | 11.500 | 28.530 | 43.364 | 1.00 | 8.71 | | C |
| ANISOU | 1533 | CB | SER | 1335 | 1005 | 1115 | 1189 | 38 | -104 | -248 | C |
| ATOM | 1534 | OG | SER | 1335 | 11.842 | 28.966 | 44.682 | 1.00 | 9.87 | | O |
| ANISOU | 1534 | OG | SER | 1335 | 1275 | 1153 | 1322 | -7 | -124 | -261 | O |
| ATOM | 1535 | C | SER | 1335 | 13.821 | 27.538 | 43.043 | 1.00 | 8.37 | | C |
| ANISOU | 1535 | C | SER | 1335 | 1059 | 909 | 1213 | 58 | -30 | -123 | C |
| ATOM | 1536 | O | SER | 1335 | 14.892 | 27.964 | 43.502 | 1.00 | 9.11 | | O |
| ANISOU | 1536 | O | SER | 1335 | 1132 | 1057 | 1274 | 46 | -106 | -252 | O |
| ATOM | 1537 | N | ARG | 1336 | 13.565 | 26.206 | 42.983 | 1.00 | 8.62 | | N |
| ANISOU | 1537 | N | ARG | 1336 | 1028 | 990 | 1256 | 3 | 26 | -78 | N |
| ATOM | 1538 | CA | ARG | 1336 | 14.515 | 25.252 | 43.542 | 1.00 | 8.14 | | C |
| ANISOU | 1538 | CA | ARG | 1336 | 1174 | 809 | 1111 | 55 | 56 | -84 | C |
| ATOM | 1539 | CB | ARG | 1336 | 13.921 | 23.834 | 43.582 | 1.00 | 9.71 | | C |
| ANISOU | 1539 | CB | ARG | 1336 | 1206 | 974 | 1509 | -146 | -6 | -19 | C |
| ATOM | 1540 | CG | ARG | 1336 | 12.764 | 23.699 | 44.580 | 1.00 | 12.51 | | C |
| ANISOU | 1540 | CG | ARG | 1336 | 1203 | 1574 | 1976 | -295 | 277 | -168 | C |
| ATOM | 1541 | CD | ARG | 1336 | 12.324 | 22.240 | 44.632 | 1.00 | 16.53 | | C |
| ANISOU | 1541 | CD | ARG | 1336 | 1965 | 1828 | 2489 | -794 | 39 | 224 | C |
| ATOM | 1542 | NE | ARG | 1336 | 11.333 | 22.000 | 45.675 | 1.00 | 20.55 | | N |
| ANISOU | 1542 | NE | ARG | 1336 | 2059 | 2710 | 3039 | -569 | 188 | 1093 | N |
| ATOM | 1543 | CZ | ARG | 1336 | 11.602 | 21.702 | 46.960 | 1.00 | 21.12 | | C |
| ANISOU | 1543 | CZ | ARG | 1336 | 2246 | 2809 | 2969 | -365 | 349 | 1111 | C |
| ATOM | 1544 | NH1 | ARG | 1336 | 12.880 | 21.604 | 47.379 | 1.00 | 19.31 | | N1+ |
| ANISOU | 1544 | NH1 | ARG | 1336 | 2479 | 2424 | 2434 | 70 | 177 | -32 | N1+ |
| ATOM | 1545 | NH2 | ARG | 1336 | 10.658 | 21.501 | 47.831 | 1.00 | 24.03 | | N |
| ANISOU | 1545 | NH2 | ARG | 1336 | 2743 | 3291 | 3098 | -790 | 663 | 678 | N |
| ATOM | 1546 | C | ARG | 1336 | 15.809 | 25.197 | 42.747 | 1.00 | 7.85 | | C |
| ANISOU | 1546 | C | ARG | 1336 | 947 | 829 | 1206 | -6 | -140 | -86 | C |
| ATOM | 1547 | O | ARG | 1336 | 16.875 | 25.093 | 43.338 | 1.00 | 8.97 | | O |
| ANISOU | 1547 | O | ARG | 1336 | 1131 | 988 | 1290 | 35 | -222 | -136 | O |
| ATOM | 1548 | N | ILE | 1337 | 15.719 | 25.260 | 41.407 | 1.00 | 8.03 | | N |
| ANISOU | 1548 | N | ILE | 1337 | 979 | 916 | 1157 | 8 | -40 | -62 | N |
| ATOM | 1549 | CA | ILE | 1337 | 16.961 | 25.203 | 40.635 | 1.00 | 8.17 | | C |
| ANISOU | 1549 | CA | ILE | 1337 | 1048 | 857 | 1199 | -46 | -48 | -85 | C |
| ATOM | 1550 | CB | ILE | 1337 | 16.650 | 24.826 | 39.184 | 1.00 | 8.43 | | C |
| ANISOU | 1550 | CB | ILE | 1337 | 851 | 1154 | 1197 | -104 | -87 | -203 | C |
| ATOM | 1551 | CG2 | ILE | 1337 | 17.917 | 24.873 | 38.322 | 1.00 | 9.76 | | C |
| ANISOU | 1551 | CG2 | ILE | 1337 | 1010 | 1299 | 1397 | 61 | 125 | -169 | C |
| ATOM | 1552 | CG1 | ILE | 1337 | 15.973 | 23.446 | 39.108 | 1.00 | 8.62 | | C |
| ANISOU | 1552 | CG1 | ILE | 1337 | 1048 | 956 | 1272 | -49 | -250 | -84 | C |
| ATOM | 1553 | CD1 | ILE | 1337 | 15.426 | 23.048 | 37.743 | 1.00 | 9.65 | | C |
| ANISOU | 1553 | CD1 | ILE | 1337 | 1196 | 1200 | 1269 | -13 | -275 | -167 | C |
| ATOM | 1554 | C | ILE | 1337 | 17.710 | 26.518 | 40.769 | 1.00 | 8.27 | | C |
| ANISOU | 1554 | C | ILE | 1337 | 842 | 1093 | 1209 | -55 | -168 | -302 | C |
| ATOM | 1555 | O | ILE | 1337 | 18.946 | 26.487 | 40.759 | 1.00 | 9.46 | | O |
| ANISOU | 1555 | O | ILE | 1337 | 937 | 1178 | 1477 | -60 | -192 | -228 | O |
| ATOM | 1556 | N | SER | 1338 | 17.043 | 27.673 | 40.942 | 1.00 | 8.80 | | N |
| ANISOU | 1556 | N | SER | 1338 | 1158 | 818 | 1369 | -118 | -97 | -139 | N |
| ATOM | 1557 | CA | SER | 1338 | 17.794 | 28.895 | 41.258 | 1.00 | 9.19 | | C |
| ANISOU | 1557 | CA | SER | 1338 | 1192 | 1059 | 1239 | -250 | -265 | -207 | C |
| ATOM | 1558 | CB | SER | 1338 | 16.805 | 30.044 | 41.512 | 1.00 | 11.88 | | C |
| ANISOU | 1558 | CB | SER | 1338 | 1456 | 600 | 2456 | -125 | -936 | -2 | C |
| ATOM | 1559 | OG | SER | 1338 | 16.175 | 30.499 | 40.359 | 1.00 | 12.96 | | O |
| ANISOU | 1559 | OG | SER | 1338 | 1425 | 1736 | 1763 | 121 | -183 | -133 | O |
| ATOM | 1560 | C | SER | 1338 | 18.651 | 28.684 | 42.483 | 1.00 | 8.30 | | C |
| ANISOU | 1560 | C | SER | 1338 | 949 | 923 | 1281 | 57 | -129 | -157 | C |
| ATOM | 1561 | O | SER | 1338 | 19.817 | 29.078 | 42.554 | 1.00 | 9.12 | | O |
| ANISOU | 1561 | O | SER | 1338 | 951 | 1164 | 1350 | -80 | -120 | -211 | O |
| ATOM | 1562 | N | ALA | 1339 | 18.054 | 28.055 | 43.517 | 1.00 | 8.47 | | N |
| ANISOU | 1562 | N | ALA | 1339 | 1041 | 886 | 1291 | -27 | -135 | -155 | N |
| ATOM | 1563 | CA | ALA | 1339 | 18.793 | 27.824 | 44.748 | 1.00 | 8.28 | | C |
| ANISOU | 1563 | CA | ALA | 1339 | 984 | 980 | 1180 | -13 | -59 | -189 | C |
| ATOM | 1564 | CB | ALA | 1339 | 17.856 | 27.267 | 45.821 | 1.00 | 8.73 | | C |
| ANISOU | 1564 | CB | ALA | 1339 | 1004 | 936 | 1377 | -120 | -30 | -137 | C |
| ATOM | 1565 | C | ALA | 1339 | 19.967 | 26.898 | 44.500 | 1.00 | 8.40 | | C |
| ANISOU | 1565 | C | ALA | 1339 | 1104 | 1079 | 1008 | 64 | -60 | -194 | C |
| ATOM | 1566 | O | ALA | 1339 | 21.086 | 27.149 | 44.966 | 1.00 | 9.08 | | O |
| ANISOU | 1566 | O | ALA | 1339 | 1042 | 1075 | 1334 | 144 | -161 | -198 | O |
| ATOM | 1567 | N | ILE | 1340 | 19.769 | 25.774 | 43.778 | 1.00 | 8.62 | | N |
| ANISOU | 1567 | N | ILE | 1340 | 1053 | 956 | 1268 | 60 | 15 | -132 | N |
| ATOM | 1568 | CA | ILE | 1340 | 20.908 | 24.859 | 43.501 | 1.00 | 8.91 | | C |
| ANISOU | 1568 | CA | ILE | 1340 | 953 | 830 | 1603 | -52 | -57 | -196 | C |
| ATOM | 1569 | CB | ILE | 1340 | 20.360 | 23.612 | 42.790 | 1.00 | 9.84 | | C |
| ANISOU | 1569 | CB | ILE | 1340 | 1108 | 939 | 1693 | -89 | -120 | -286 | C |
| ATOM | 1570 | CG2 | ILE | 1340 | 21.495 | 22.740 | 42.277 | 1.00 | 11.36 | | C |
| ANISOU | 1570 | CG2 | ILE | 1340 | 1209 | 1087 | 2020 | 109 | 58 | -330 | C |
| ATOM | 1571 | CG1 | ILE | 1340 | 19.390 | 22.847 | 43.725 | 1.00 | 10.23 | | C |
| ANISOU | 1571 | CG1 | ILE | 1340 | 1134 | 1089 | 1665 | -271 | -136 | -146 | C |
| ATOM | 1572 | CD1 | ILE | 1340 | 18.582 | 21.793 | 42.957 | 1.00 | 11.89 | | C |
| ANISOU | 1572 | CD1 | ILE | 1340 | 1440 | 1024 | 2054 | -303 | -389 | -101 | C |
| ATOM | 1573 | C | ILE | 1340 | 21.993 | 25.550 | 42.717 | 1.00 | 8.90 | | C |
| ANISOU | 1573 | C | ILE | 1340 | 955 | 1003 | 1425 | 19 | -60 | -112 | C |
| ATOM | 1574 | O | ILE | 1340 | 23.189 | 25.477 | 43.049 | 1.00 | 9.92 | | O |
| ANISOU | 1574 | O | ILE | 1340 | 907 | 1113 | 1750 | 37 | -109 | -204 | O |
| ATOM | 1575 | N | PHE | 1341 | 21.630 | 26.255 | 41.661 | 1.00 | 9.18 | | N |
| ANISOU | 1575 | N | PHE | 1341 | 1280 | 931 | 1277 | -76 | -117 | -186 | N |
| ATOM | 1576 | CA | PHE | 1341 | 22.575 | 27.011 | 40.839 | 1.00 | 8.83 | | C |
| ANISOU | 1576 | CA | PHE | 1341 | 1001 | 1117 | 1239 | -23 | -54 | -175 | C |
| ATOM | 1577 | CB | PHE | 1341 | 21.766 | 27.812 | 39.822 | 1.00 | 9.26 | | C |
| ANISOU | 1577 | CB | PHE | 1341 | 887 | 1351 | 1282 | 84 | 46 | -18 | C |
| ATOM | 1578 | CG | PHE | 1341 | 22.540 | 28.818 | 38.967 | 1.00 | 9.61 | | C |
| ANISOU | 1578 | CG | PHE | 1341 | 1053 | 1261 | 1338 | -61 | 24 | -105 | C |
| ATOM | 1579 | CD1 | PHE | 1341 | 23.266 | 28.379 | 37.890 | 1.00 | 8.96 | | C |
| ANISOU | 1579 | CD1 | PHE | 1341 | 797 | 1429 | 1179 | -207 | -69 | -84 | C |
| ATOM | 1580 | CD2 | PHE | 1341 | 22.508 | 30.172 | 39.267 | 1.00 | 13.06 | | C |
| ANISOU | 1580 | CD2 | PHE | 1341 | 1834 | 1322 | 1805 | -158 | 118 | -291 | C |
| ATOM | 1581 | CE1 | PHE | 1341 | 23.944 | 29.286 | 37.086 | 1.00 | 10.76 | | C |
| ANISOU | 1581 | CE1 | PHE | 1341 | 1084 | 1407 | 1597 | -353 | 72 | -86 | C |
| ATOM | 1582 | CE2 | PHE | 1341 | 23.192 | 31.085 | 38.470 | 1.00 | 15.09 | | C |
| ANISOU | 1582 | CE2 | PHE | 1341 | 2304 | 1223 | 2207 | -114 | 328 | -97 | C |
| ATOM | 1583 | CZ | PHE | 1341 | 23.931 | 30.632 | 37.367 | 1.00 | 12.84 | | C |
| ANISOU | 1583 | CZ | PHE | 1341 | 1360 | 1371 | 2148 | -262 | 26 | -126 | C |
| ATOM | 1584 | C | PHE | 1341 | 23.437 | 27.920 | 41.680 | 1.00 | 8.75 | | C |
| ANISOU | 1584 | C | PHE | 1341 | 880 | 1117 | 1325 | 38 | -16 | -213 | C |
| ATOM | 1585 | O | PHE | 1341 | 24.638 | 28.059 | 41.449 | 1.00 | 11.08 | | O |
| ANISOU | 1585 | O | PHE | 1341 | 991 | 1716 | 1503 | -95 | -44 | -418 | O |
| ATOM | 1586 | N | SER | 1342 | 22.829 | 28.583 | 42.663 | 1.00 | 8.78 | | N |
| ANISOU | 1586 | N | SER | 1342 | 1073 | 929 | 1333 | -47 | -64 | -167 | N |
| ATOM | 1587 | CA | SER | 1342 | 23.508 | 29.588 | 43.467 | 1.00 | 9.79 | | C |
| ANISOU | 1587 | CA | SER | 1342 | 1025 | 1195 | 1499 | 173 | -200 | -453 | C |
| ATOM | 1588 | CB | SER | 1342 | 22.473 | 30.329 | 44.315 | 1.00 | 9.78 | | C |
| ANISOU | 1588 | CB | SER | 1342 | 984 | 1144 | 1586 | 6 | 89 | -299 | C |
| ATOM | 1589 | OG | SER | 1342 | 22.068 | 29.572 | 45.430 | 1.00 | 10.05 | | O |
| ANISOU | 1589 | OG | SER | 1342 | 1176 | 1176 | 1466 | 9 | -97 | -311 | O |
| ATOM | 1590 | C | SER | 1342 | 24.570 | 29.011 | 44.379 | 1.00 | 9.61 | | C |
| ANISOU | 1590 | C | SER | 1342 | 990 | 1195 | 1468 | 19 | -95 | -305 | C |
| ATOM | 1591 | O | SER | 1342 | 25.340 | 29.795 | 44.964 | 1.00 | 11.23 | | O |
| ANISOU | 1591 | O | SER | 1342 | 1090 | 1252 | 1926 | 98 | -301 | -565 | O |
| ATOM | 1592 | N | THR | 1343 | 24.632 | 27.692 | 44.525 | 1.00 | 10.45 | | N |
| ANISOU | 1592 | N | THR | 1343 | 1217 | 1125 | 1629 | 252 | -347 | -428 | N |
| ATOM | 1593 | CA | THR | 1343 | 25.600 | 27.078 | 45.464 | 1.00 | 12.07 | | C |
| ANISOU | 1593 | CA | THR | 1343 | 1479 | 1389 | 1720 | 330 | -356 | -346 | C |
| ATOM | 1594 | CB | THR | 1343 | 25.000 | 25.891 | 46.215 | 1.00 | 13.04 | | C |
| ANISOU | 1594 | CB | THR | 1343 | 1907 | 1482 | 1564 | 405 | -143 | -273 | C |
| ATOM | 1595 | OG1 | THR | 1343 | 24.737 | 24.803 | 45.298 | 1.00 | 12.76 | | O |
| ANISOU | 1595 | OG1 | THR | 1343 | 1902 | 1281 | 1666 | 228 | 104 | -227 | O |
| ATOM | 1596 | CG2 | THR | 1343 | 23.671 | 26.281 | 46.888 | 1.00 | 14.32 | | C |
| ANISOU | 1596 | CG2 | THR | 1343 | 2133 | 1470 | 1839 | 513 | 153 | -243 | C |
| ATOM | 1597 | C | THR | 1343 | 26.881 | 26.637 | 44.743 | 1.00 | 12.17 | | C |
| ANISOU | 1597 | C | THR | 1343 | 1296 | 1324 | 2004 | 355 | -363 | -488 | C |
| ATOM | 1598 | O | THR | 1343 | 27.719 | 26.095 | 45.468 | 1.00 | 16.07 | | O |
| ANISOU | 1598 | O | THR | 1343 | 1607 | 2231 | 2267 | 571 | -656 | -419 | O |
| ATOM | 1599 | N | PHE | 1344 | 26.990 | 26.884 | 43.459 | 1.00 | 12.84 | | N |
| ANISOU | 1599 | N | PHE | 1344 | 1367 | 1612 | 1900 | 399 | -269 | -609 | N |
| ATOM | 1600 | CA | PHE | 1344 | 28.203 | 26.534 | 42.732 | 1.00 | 14.34 | | C |
| ANISOU | 1600 | CA | PHE | 1344 | 1457 | 1713 | 2279 | 145 | -27 | -685 | C |
| ATOM | 1601 | CB | PHE | 1344 | 27.848 | 25.821 | 41.403 | 1.00 | 12.78 | | C |
| ANISOU | 1601 | CB | PHE | 1344 | 1671 | 1415 | 1769 | 121 | 173 | -242 | C |
| ATOM | 1602 | CG | PHE | 1344 | 27.345 | 24.421 | 41.768 | 1.00 | 12.51 | | C |
| ANISOU | 1602 | CG | PHE | 1344 | 1419 | 1411 | 1922 | 200 | -55 | -344 | C |
| ATOM | 1603 | CD1 | PHE | 1344 | 26.058 | 24.180 | 42.147 | 1.00 | 11.90 | | C |
| ANISOU | 1603 | CD1 | PHE | 1344 | 1258 | 1760 | 1502 | -53 | -320 | -351 | C |
| ATOM | 1604 | CD2 | PHE | 1344 | 28.280 | 23.369 | 41.721 | 1.00 | 16.99 | | C |
| ANISOU | 1604 | CD2 | PHE | 1344 | 1844 | 1427 | 3185 | 402 | 306 | -412 | C |
| ATOM | 1605 | CE1 | PHE | 1344 | 25.675 | 22.898 | 42.499 | 1.00 | 14.59 | | C |
| ANISOU | 1605 | CE1 | PHE | 1344 | 1722 | 1904 | 1917 | -105 | -144 | -149 | C |
| ATOM | 1606 | CE2 | PHE | 1344 | 27.903 | 22.099 | 42.055 | 1.00 | 16.79 | | C |
| ANISOU | 1606 | CE2 | PHE | 1344 | 1979 | 1475 | 2924 | 202 | -167 | -261 | C |
| ATOM | 1607 | CZ | PHE | 1344 | 26.589 | 21.858 | 42.445 | 1.00 | 18.76 | | C |
| ANISOU | 1607 | CZ | PHE | 1344 | 2055 | 1812 | 3261 | 54 | -85 | -59 | C |
| ATOM | 1608 | C | PHE | 1344 | 29.076 | 27.730 | 42.403 | 1.00 | 16.20 | | C |
| ANISOU | 1608 | C | PHE | 1344 | 1183 | 1677 | 3296 | 218 | -113 | -754 | C |
| ATOM | 1609 | O | PHE | 1344 | 28.612 | 28.821 | 42.162 | 1.00 | 17.22 | | O |
| ANISOU | 1609 | O | PHE | 1344 | 1411 | 1670 | 3463 | 140 | -108 | -621 | O |
| ATOM | 1610 | N | ILE | 1345 | 30.381 | 27.445 | 42.380 | 1.00 | 22.23 | | N |
| ANISOU | 1610 | N | ILE | 1345 | 1104 | 2594 | 4750 | 315 | -203 | -1085 | N |
| ATOM | 1611 | CA | ILE | 1345 | 31.333 | 28.468 | 41.923 | 1.00 | 28.36 | | C |
| ANISOU | 1611 | CA | ILE | 1345 | 1120 | 4707 | 4948 | -359 | -345 | -102 | C |
| ATOM | 1612 | CB | ILE | 1345 | 32.700 | 28.401 | 42.600 | 1.00 | 31.48 | | C |
| ANISOU | 1612 | CB | ILE | 1345 | 1667 | 4345 | 5949 | -490 | -1170 | 88 | C |
| ATOM | 1613 | CG2 | ILE | 1345 | 33.702 | 29.326 | 41.922 | 1.00 | 34.84 | | C |
| ANISOU | 1613 | CG2 | ILE | 1345 | 1562 | 4401 | 7275 | -865 | -408 | -940 | C |
| ATOM | 1614 | CG1 | ILE | 1345 | 32.633 | 28.692 | 44.101 | 1.00 | 34.37 | | C |
| ANISOU | 1614 | CG1 | ILE | 1345 | 2059 | 5080 | 5918 | 1032 | -1654 | 23 | C |
| ATOM | 1615 | CD1 | ILE | 1345 | 32.799 | 27.504 | 45.004 | 1.00 | 44.90 | | C |
| ANISOU | 1615 | CD1 | ILE | 1345 | 8417 | 3416 | 5227 | 1404 | 551 | -776 | C |
| ATOM | 1616 | C | ILE | 1345 | 31.429 | 28.229 | 40.435 | 1.00 | 32.47 | | C |
| ANISOU | 1616 | C | ILE | 1345 | 743 | 6497 | 5099 | -143 | -91 | -505 | C |
| ATOM | 1617 | O | ILE | 1345 | 31.574 | 27.091 | 39.970 | 1.00 | 52.32 | | O |
| ANISOU | 1617 | O | ILE | 1345 | 4633 | 7954 | 7293 | 636 | -439 | -2761 | O |
| ATOM | 1618 | N | GLY | 1346 | 31.341 | 29.233 | 39.579 | 1.00 | 42.18 | | N |
| ANISOU | 1618 | N | GLY | 1346 | 1564 | 9187 | 5276 | -737 | 634 | 1205 | N |
| ATOM | 1619 | CA | GLY | 1346 | 31.358 | 28.702 | 38.197 | 1.00 | 42.04 | | C |
| ANISOU | 1619 | CA | GLY | 1346 | 1993 | 8390 | 5590 | -288 | 605 | 829 | C |
| ATOM | 1620 | C | GLY | 1346 | 32.744 | 28.804 | 37.605 | 1.00 | 35.42 | | C |
| ANISOU | 1620 | C | GLY | 1346 | 2009 | 5733 | 5715 | -1032 | 594 | -588 | C |
| ATOM | 1621 | O | GLY | 1346 | 32.847 | 29.466 | 36.570 | 1.00 | 29.91 | | O |
| ANISOU | 1621 | O | GLY | 1346 | 2517 | 3128 | 5720 | -609 | 1243 | -1467 | O |
| ATOM | 1622 | N | GLU | 1347 | 33.764 | 28.197 | 38.202 | 1.00 | 32.42 | | N |
| ANISOU | 1622 | N | GLU | 1347 | 1855 | 5078 | 5384 | -1023 | 711 | -1174 | N |
| ATOM | 1623 | CA | GLU | 1347 | 35.102 | 28.380 | 37.583 | 1.00 | 34.52 | | C |
| ANISOU | 1623 | CA | GLU | 1347 | 2028 | 4846 | 6242 | -1021 | 1233 | -2503 | C |
| ATOM | 1624 | CB | GLU | 1347 | 35.866 | 29.445 | 38.387 | 1.00 | 43.54 | | C |
| ANISOU | 1624 | CB | GLU | 1347 | 3110 | 5574 | 7859 | -2136 | 1159 | -2882 | C |
| ATOM | 1625 | CG | GLU | 1347 | 35.257 | 30.837 | 38.232 | 1.00 | 50.27 | | C |
| ANISOU | 1625 | CG | GLU | 1347 | 6099 | 5049 | 7952 | -2109 | 975 | -2900 | C |
| ATOM | 1626 | CD | GLU | 1347 | 36.216 | 31.880 | 38.781 | 1.00 | 59.94 | | C |
| ANISOU | 1626 | CD | GLU | 1347 | 7531 | 5502 | 9744 | -3279 | -210 | -1829 | C |
| ATOM | 1627 | OE1 | GLU | 1347 | 36.704 | 31.654 | 39.911 | 1.00 | 72.63 | | O1- |
| ANISOU | 1627 | OE1 | GLU | 1347 | 7542 | 9402 | 10654 | -3681 | -1627 | -1956 | O1- |
| ATOM | 1628 | OE2 | GLU | 1347 | 36.456 | 32.885 | 38.078 | 1.00 | 80.71 | | O |
| ANISOU | 1628 | OE2 | GLU | 1347 | 11428 | 5956 | 13280 | -4260 | 612 | -625 | O |
| ATOM | 1629 | C | GLU | 1347 | 35.836 | 27.076 | 37.524 | 1.00 | 28.47 | | C |
| ANISOU | 1629 | C | GLU | 1347 | 2371 | 5024 | 3423 | -637 | 340 | -1363 | C |
| ATOM | 1630 | O | GLU | 1347 | 35.699 | 26.251 | 38.444 | 1.00 | 53.72 | | O |
| ANISOU | 1630 | O | GLU | 1347 | 6352 | 8133 | 5926 | 730 | 3277 | 1242 | O |
| ATOM | 1631 | N | HIS | 1348 | 36.626 | 26.832 | 36.477 | 1.00 | 25.55 | | N |
| ANISOU | 1631 | N | HIS | 1348 | 2319 | 4355 | 3034 | 57 | 52 | -915 | N |
| ATOM | 1632 | CA | HIS | 1348 | 37.487 | 25.676 | 36.508 | 1.00 | 26.15 | | C |
| ANISOU | 1632 | CA | HIS | 1348 | 2947 | 4334 | 2655 | 266 | -231 | -331 | C |
| ATOM | 1633 | CB | HIS | 1348 | 37.827 | 25.311 | 35.067 | 1.00 | 22.43 | | C |
| ANISOU | 1633 | CB | HIS | 1348 | 1406 | 4322 | 2793 | 354 | -114 | -334 | C |
| ATOM | 1634 | CG | HIS | 1348 | 36.654 | 24.757 | 34.330 | 1.00 | 20.85 | | C |
| ANISOU | 1634 | CG | HIS | 1348 | 1777 | 3333 | 2813 | 198 | -534 | 199 | C |
| ATOM | 1635 | CD2 | HIS | 1348 | 36.160 | 25.236 | 33.166 | 1.00 | 19.00 | | C |
| ANISOU | 1635 | CD2 | HIS | 1348 | 1957 | 2715 | 2549 | -26 | -196 | 149 | C |
| ATOM | 1636 | ND1 | HIS | 1348 | 35.884 | 23.661 | 34.703 | 1.00 | 20.19 | | N |
| ANISOU | 1636 | ND1 | HIS | 1348 | 2566 | 2754 | 2353 | 394 | -601 | 190 | N |
| ATOM | 1637 | CE1 | HIS | 1348 | 34.960 | 23.526 | 33.759 | 1.00 | 20.25 | | C |
| ANISOU | 1637 | CEI | HIS | 1348 | 2253 | 3002 | 2438 | -199 | -437 | 528 | C |
| ATOM | 1638 | NE2 | HIS | 1348 | 35.105 | 24.478 | 32.810 | 1.00 | 14.15 | | N |
| ANISOU | 1638 | NE2 | HIS | 1348 | 1278 | 2111 | 1989 | 507 | 69 | -48 | N |
| ATOM | 1639 | C | HIS | 1348 | 38.785 | 25.887 | 37.281 | 1.00 | 26.98 | | C |
| ANISOU | 1639 | C | HIS | 1348 | 3381 | 3635 | 3237 | 291 | -787 | -522 | C |
| ATOM | 1640 | O | HIS | 1348 | 39.511 | 24.880 | 37.403 | 1.00 | 32.17 | | O |
| ANISOU | 1640 | O | HIS | 1348 | 3862 | 3955 | 4406 | 527 | -1046 | 318 | O |
| ATOM | 1641 | OXT | HIS | 1348 | 39.104 | 27.002 | 37.772 | 1.00 | 29.89 | | O1- |
| ANISOU | 1641 | OXT | HIS | 1348 | 2610 | 4203 | 4545 | -784 | 1366 | -1301 | O1- |
| ANISOU | 2307 | C01 | AGI | 1 | 1424 | 1244 | 1271 | 107 | -113 | -8 | |
| ATOM | 2308 | C02 | AGI | 1 | 17.248 | 25.870 | 14.705 | 1.00 | 11.23 | | |
| ANISOU | 2308 | C02 | AGI | 1 | 1593 | 1288 | 1385 | 27 | 17 | -42 | |
| ATOM | 2309 | C03 | AGI | 1 | 19.270 | 27.557 | 14.359 | 1.00 | 11.18 | | |
| ANISOU | 2309 | C03 | AGI | 1 | 1576 | 1385 | 1288 | -25 | 177 | -76 | |
| ATOM | 2310 | C04 | AGI | 1 | 17.142 | 28.281 | 14.903 | 1.00 | 10.33 | | |
| ANISOU | 2310 | C04 | AGI | I | 1425 | 1264 | 1237 | 144 | -269 | 177 | |
| ATOM | 2311 | C05 | AGI | 1 | 15.862 | 25.858 | 14.999 | 1.00 | 11.47 | | |
| ANISOU | 2311 | C05 | AGI | 1 | 1622 | 1302 | 1432 | -11 | 124 | 112 | |
| ATOM | 2312 | S06 | AGI | 1 | 17.786 | 24.273 | 14.417 | 1.00 | 11.85 | | |
| ANISOU | 2312 | S06 | AGI | 1 | 1760 | 1225 | 1516 | 96 | -40 | 0 | |
| ATOM | 2313 | C07 | AGI | 1 | 19.293 | 28.958 | 14.483 | 1.00 | 11.57 | | |
| ANISOU | 2313 | C07 | AGI | 1 | 1529 | 1400 | 1469 | -68 | -229 | -9 | |
| ATOM | 2314 | C08 | AGI | 1 | 20.378 | 26.774 | 14.112 | 1.00 | 11.98 | | |
| ANISOU | 2314 | C08 | AGI | 1 | 1525 | 1600 | 1427 | -3 | 111 | -103 | |
| ATOM | 2315 | N09 | AGI | 1 | 18.016 | 29.358 | 14.779 | 1.00 | 11.45 | | |
| ANISOU | 2315 | N09 | AGI | 1 | 1554 | 1301 | 1494 | -37 | -190 | 39 | |
| ATOM | 2316 | C10 | AGI | 1 | 15.805 | 28.308 | 15.186 | 1.00 | 11.35 | | |
| ANISOU | 2316 | C10 | AGI | 1 | 1301 | 1327 | 1685 | 152 | -337 | -83 | |
| ATOM | 2317 | C11 | AGI | 1 | 15.114 | 27.013 | 15.245 | 1.00 | 12.38 | | |
| ANISOU | 2317 | C11 | AGI | 1 | 1642 | 1380 | 1681 | 97 | 25 | 248 | |
| ATOM | 2318 | N12 | AGI | 1 | 15.327 | 24.561 | 15.035 | 1.00 | 11.68 | | |
| ANISOU | 2318 | N12 | AGI | 1 | 1728 | 1354 | 1354 | -82 | -90 | 87 | |
| ATOM | 2319 | C13 | AGI | 1 | 16.275 | 23.604 | 14.744 | 1.00 | 11.95 | | |
| ANISOU | 2319 | C13 | AGI | 1 | 1869 | 1304 | 1369 | 39 | 34 | 202 | |
| ATOM | 2320 | O14 | AGI | 1 | 20.237 | 29.724 | 14.301 | 1.00 | 12.24 | | |
| ANISOU | 2320 | 014 | AGI | 1 | 1692 | 1416 | 1543 | -150 | -48 | -32 | |
| ATOM | 2321 | N15 | AGI | 1 | 21.614 | 27.359 | 14.247 | 1.00 | 12.75 | | |
| AMSOU | 2321 | N15 | AGI | 1 | 1500 | 1840 | 1505 | 50 | -316 | 68 | |
| ATOM | 2322 | C16 | AGI | 1 | 22.834 | 26.782 | 14.157 | 1.00 | 13.67 | | |
| ANISOU | 2322 | C16 | AGI | 1 | 1563 | 1910 | 1719 | 27 | 45 | -28 | |
| ATOM | 2323 | C17 | AGI | 1 | 23.956 | 27.612 | 14.114 | 1.00 | 15.83 | | |
| ANISOU | 2323 | C17 | AGI | 1 | 1525 | 2253 | 2238 | -23 | 258 | -252 | |
| ATOM | 2324 | C18 | AGI | 1 | 22.982 | 25.380 | 14.099 | 1.00 | 14.95 | | |
| ANISOU | 2324 | C18 | AGI | 1 | 2103 | 1915 | 1661 | 270 | 427 | 225 | |
| ATOM | 2325 | C19 | AGI | 1 | 25.237 | 27.058 | 14.005 | 1.00 | 20.32 | | |
| ANISOU | 2325 | C19 | AGI | 1 | 1709 | 2588 | 3424 | 97 | 966 | -237 | |
| ATOM | 2326 | C20 | AGI | 1 | 24.272 | 24.821 | 14.031 | 1.00 | 16.85 | | |
| ANISOU | 2326 | C20 | AGI | 1 | 2237 | 2209 | 1955 | 445 | 278 | 366 | |
| ATOM | 2327 | C21 | AGI | 1 | 25.399 | 25.679 | 13.982 | 1.00 | 20.28 | | |
| ANISOU | 2327 | C21 | AGI | 1 | 2047 | 2603 | 3057 | 394 | 453 | -424 | |
| ATOM | 2328 | S22 | AGI | 1 | 26.986 | 25.032 | 13.876 | 1.00 | 26.64 | | |
| ANISOU | 2328 | S22 | AGI | 1 | 2226 | 3468 | 4428 | 811 | 678 | -405 | |
| ATOM | 2329 | N23 | AGI | 1 | 27.676 | 24.971 | 15.376 | 1.00 | 37.62 | | |
| ANISOU | 2329 | N23 | AGI | 1 | 3078 | 6437 | 4778 | 2628 | 83 | -81 | |
| ATOM | 2330 | 024 | AGI | 1 | 26.827 | 23.716 | 13.311 | 1.00 | 38.19 | | |
| ANISOU | 2330 | 024 | AGI | 1 | 2627 | 3738 | 8146 | 1100 | 929 | -1697 | |
| ATOM | 2331 | 025 | AGI | 1 | 27.805 | 25.956 | 13.104 | 1.00 | 33.13 | | |
| ANISOU | 2331 | 025 | AGI | 1 | 2234 | 4778 | 5575 | 567 | 1078 | 291 | |
| ATOM | 2332 | C26 | AGI | 1 | 27.374 | 25.661 | 16.611 | 1.00 | 37.52 | | |
| ANISOU | 2332 | C26 | AGI | 1 | 2405 | 7263 | 4586 | -168 | 385 | -695 | |
| ATOM | 2281 | C2 | PG1 | 2 | 27.637 | 9.800 | 25.901 | 1.00 | 46.69 | | |
| ANISOU | 2281 | C2 | PG1 | 2 | 1814 | 7160 | 8766 | -1009 | 845 | -92 | |
| ATOM | 2282 | C1 | PG1 | 2 | 26.998 | 8.769 | 23.869 | 1.00 | 59.49 | | |
| ANISOU | 2282 | C1 | PG1 | 2 | 4296 | 8204 | 10103 | -1026 | 2989 | -3292 | |
| ATOM | 2283 | O1 | PG1 | 2 | 27.655 | 9.872 | 24.485 | 1.00 | 49.46 | | |
| ANISOU | 2283 | O1 | PG1 | 2 | 2873 | 7162 | 8759 | -1022 | 848 | -278 | |
| ATOM | 2284 | 02 | PG1 | 2 | 27.875 | 12.238 | 25.632 | 1.00 | 51.26 | | |
| ANISOU | 2284 | 02 | PG1 | 2 | 4605 | 7234 | 7637 | -1392 | 589 | 128 | |
| ATOM | 2285 | C3 | PG1 | 2 | 27.920 | 11.164 | 26.550 | 1.00 | 49.75 | | |
| ANISOU | 2285 | C3 | PG1 | 2 | 3707 | 6963 | 8231 | -940 | 745 | 211 | |
| ATOM | 2286 | C4 | PG1 | 2 | 28.756 | 13.287 | 25.910 | 1.00 | 45.26 | | |
| ANISOU | 2286 | C4 | PG1 | 2 | 4805 | 6323 | 6069 | -685 | 666 | -509 | |
| ATOM | 2287 | C5 | PG1 | 2 | 29.745 | 13.688 | 24.814 | 1.00 | 34.91 | | |
| ANISOU | 2287 | C5 | PG1 | 2 | 3413 | 4955 | 4898 | -386 | -479 | -793 | |
| ATOM | 2288 | 03 | PG1 | 2 | 30.945 | 14.190 | 25.383 | 1.00 | 27.74 | | |
| ANISOU | 2288 | 03 | PG1 | 2 | 3717 | 3007 | 3816 | 462 | -1260 | 173 | |
| ATOM | 2289 | C6 | PG1 | 2 | 30.959 | 15.543 | 25.802 | 1.00 | 24.64 | | |
| ANISOU | 2289 | C6 | PG1 | 2 | 2986 | 2888 | 3489 | 296 | 10 | 477 | |
| ATOM | 2290 | C7 | PG1 | 2 | 32.356 | 15.754 | 26.470 | 1.00 | 23.41 | | |
| ANISOU | 2290 | C7 | PG1 | 2 | 3219 | 2502 | 3172 | 101 | -17 | 354 | |
| ATOM | 2291 | 04 | PG1 | 2 | 32.155 | 15.013 | 27.651 | 1.00 | 21.96 | | |
| ANISOU | 2291 | 04 | PG1 | 2 | 3477 | 2139 | 2726 | 202 | 130 | -176 | |
| ATOM | 2292 | C8 | PG1 | 2 | 33.232 | 15.202 | 28.546 | 1.00 | 20.10 | | |
| ANISOU | 2292 | C8 | PG1 | 2 | 3199 | 2158 | 2279 | -79 | 494 | -644 | |
| ATOM | 2293 | C9 | PG1 | 2 | 32.923 | 14.316 | 29.745 | 1.00 | 17.08 | | |
| ANISOU | 2293 | C9 | PG1 | 2 | 1939 | 1779 | 2771 | -370 | -3 | -461 | |
| ATOM | 2294 | 05 | PG1 | 2 | 31.849 | 14.964 | 30.452 | 1.00 | 17.87 | | |
| ANISOU | 2294 | O5 | PG1 | 2 | 2368 | 1615 | 2808 | -140 | 471 | 62 | |
| ATOM | 2295 | C10 | PG1 | 2 | 31.399 | 14.106 | 31.468 | 1.00 | 18.56 | | |
| ANISOU | 2295 | C10 | PG1 | 2 | 2715 | 1343 | 2995 | -66 | 392 | 161 | |
| ATOM | 2296 | C11 | PG1 | 2 | 30.157 | 14.741 | 32.112 | 1.00 | 16.59 | | |
| ANISOU | 2296 | C11 | PG1 | 2 | 2182 | 1720 | 2403 | -471 | 118 | -26 | |
| ATOM | 2297 | 06 | PG1 | 2 | 30.606 | 15.974 | 32.662 | 1.00 | 14.00 | | |
| ANISOU | 2297 | 06 | PG1 | 2 | 1519 | 1675 | 2125 | -156 | -121 | 0 | |
| ATOM | 2298 | C12 | PG1 | 2 | 29.527 | 16.652 | 33.314 | 1.00 | 13.38 | | |
| ANISOU | 2298 | C12 | PG1 | 2 | 1286 | 2016 | 1780 | -251 | 110 | 217 | |
| ATOM | 2299 | C13 | PG1 | 2 | 30.169 | 17.824 | 34.055 | 1.00 | 13.84 | | |
| ANISOU | 2299 | C13 | PG1 | 2 | 1830 | 1577 | 1853 | 3 | 186 | 239 | |
| ATOM | 2300 | 07 | PG1 | 2 | 30.650 | 18.743 | 33.089 | 1.00 | 12.58 | | |
| ANISOU | 2300 | 07 | PG1 | 2 | 1441 | 1537 | 1802 | 112 | 13 | 189 | |
| ATOM | 2301 | C14 | PG1 | 2 | 31.063 | 19.989 | 33.621 | 1.00 | 13.08 | | |
| ANISOU | 2301 | C14 | PG1 | 2 | 1459 | 1447 | 2063 | 131 | -181 | 62 | |
| ATOM | 2302 | C15 | PG1 | 2 | 32.293 | 19.915 | 34.567 | 1.00 | 13.72 | | |
| ANISOU | 2302 | C15 | PG1 | 2 | 1383 | 1975 | 1853 | 208 | -77 | 126 | |
| ATOM | 2303 | O8 | PG1 | 2 | 33.387 | 19.425 | 33.786 | 1.00 | 12.82 | | |
| ANISOU | 2303 | 08 | PG1 | 2 | 1453 | 1668 | 1751 | 116 | 27 | 224 | |
| ATOM | 2304 | C16 | PG1 | 2 | 34.515 | 18.933 | 34.533 | 1.00 | 20.74 | | |
| ANISOU | 2304 | C16 | PG1 | 2 | 1780 | 3687 | 2413 | 1089 | 122 | 593 | |
| ATOM | 2305 | C17 | PG1 | 2 | 35.616 | 18.454 | 33.843 | 1.00 | 23.49 | | |
| ANISOU | 2305 | C17 | PG1 | 2 | 1639 | 3602 | 3684 | 743 | 777 | 638 | |
| ATOM | 2306 | 09 | PG1 | 2 | 36.710 | 19.364 | 33.366 | 1.00 | 20.22 | | |
| ANISOU | 2306 | 09 | PG1 | 2 | 2338 | 2854 | 2489 | 508 | 381 | 411 | |
| ATOM | 2271 | C6 | PG2 | 3 | 6.745 | 15.876 | 15.090 | 1.00 | 34.33 | | |
| ANISOU | 2271 | C6 | PG2 | 3 | 3183 | 6730 | 3133 | 1454 | 128 | 857 | |
| ATOM | 2272 | C7 | PG2 | 3 | 7.219 | 15.032 | 16.319 | 1.00 | 25.66 | | |
| ANISOU | 2272 | C7 | PG2 | 3 | 2175 | 4321 | 3254 | 456 | 655 | 755 | |
| ATOM | 2273 | 04 | PG2 | 3 | 6.470 | 15.437 | 17.428 | 1.00 | 22.57 | | |
| ANISOU | 2273 | 04 | PG2 | 3 | 2271 | 3181 | 3125 | 785 | 167 | 497 | |
| ATOM | 2274 | C8 | PG2 | 3 | 6.764 | 14.842 | 18.666 | 1.00 | 17.33 | | |
| ANISOU | 2274 | C8 | PG2 | 3 | 1970 | 1841 | 2774 | 275 | -316 | -190 | |
| ATOM | 2275 | C9 | PG2 | 3 | 5.683 | 15.202 | 19.699 | 1.00 | 18.90 | | |
| ANISOU | 2275 | C9 | PG2 | 3 | 2088 | 2289 | 2802 | -96 | -201 | -336 | |
| ATOM | 2276 | O5 | PG2 | 3 | 4.466 | 14.508 | 19.358 | 1.00 | 17.83 | | |
| ANISOU | 2276 | 05 | PG2 | 3 | 2201 | 2137 | 2435 | -230 | -196 | 59 | |
| ATOM | 2277 | C10 | PG2 | 3 | 3.424 | 14.900 | 20.248 | 1.00 | 20.16 | | |
| ANISOU | 2277 | C10 | PG2 | 3 | 2113 | 2721 | 2826 | 76 | -159 | -131 | |
| ATOM | 2278 | C11 | PG2 | 3 | 2.085 | 14.250 | 19.824 | 1.00 | 23.43 | | |
| ANISOU | 2278 | C11 | PG2 | 3 | 2333 | 3640 | 2930 | -505 | 139 | -119 | |
| ATOM | 2279 | 06 | PG2 | 3 | 1.801 | 14.745 | 18.491 | 1.00 | 24.28 | | |
| ANISOU | 2279 | 06 | PG2 | 3 | 2519 | 3696 | 3012 | -374 | -480 | -368 | |
| ATOM | 2280 | C12 | PG2 | 3 | 0.634 | 14.086 | 17.988 | 1.00 | 26.84 | | |
| ANISOU | 2280 | C12 | PG2 | 3 | 2909 | 4233 | 3055 | -1421 | 45 | -373 | |
| ATOM | 2338 | OH2 | HOH | 2001 | 16.059 | 36.368 | 20.087 | 1.00 | 30.04 | | |
| ANISOU | 2338 | OH2 | HOH | 2001 | 3422 | 3777 | 4215 | -1746 | 418 | 47 | |
| ATOM | 2339 | OH2 | HOH | 2002 | 3.991 | 24.550 | 35.564 | 1.00 | 13.01 | | |
| ANISOU | 2339 | OH2 | HOH | 2002 | 1244 | 2095 | 1606 | 247 | -209 | 207 | |
| ATOM | 2340 | OH2 | HOH | 2003 | -1.459 | 12.785 | 29.750 | 1.00 | 14.50 | | |
| ANISOU | 2340 | OH2 | HOH | 2003 | 1560 | 1941 | 2010 | 171 | -329 | -71 | |
| ATOM | 2341 | OH2 | HOH | 2004 | 23.419 | 6.044 | 30.575 | 1.00 | 18.48 | | |
| ANISOU | 2341 | OH2 | HOH | 2004 | 1950 | 2339 | 2732 | 418 | -129 | -49 | |
| ATOM | 2342 | OH2 | HOH | 2005 | 9.610 | 30.646 | 40.439 | 1.00 | 11.74 | | |
| ANISOU | 2342 | OH2 | HOH | 2005 | 1512 | 1159 | 1788 | -73 | 57 | -136 | |
| ATOM | 2343 | OH2 | HOH | 2006 | 7.713 | 17.830 | 26.517 | 1.00 | 8.88 | | |
| ANISOU | 2343 | OH2 | HOH | 2006 | 1017 | 998 | 1358 | 70 | -139 | 28 | |
| ATOM | 2344 | OH2 | HOH | 2007 | 17.870 | 16.008 | 23.308 | 1.00 | 9.47 | | |
| ANISOU | 2344 | OH2 | HOH | 2007 | 1359 | 955 | 1286 | 104 | -189 | 11 | |
| ATOM | 2345 | OH2 | HOH | 2008 | 21.105 | 13.556 | 25.188 | 1.00 | 10.04 | | |
| ANISOU | 2345 | OH2 | HOH | 2008 | 1115 | 1275 | 1425 | 201 | 145 | -1 | |
| ATOM | 2346 | OH2 | HOH | 2009 | 11.978 | 27.393 | 46.893 | 1.00 | 12.79 | | |
| ANISOU | 2346 | OH2 | HOH | 2009 | 2177 | 1306 | 1377 | 53 | -67 | -307 | |
| ATOM | 2347 | OH2 | HOH | 2010 | 3.593 | 23.317 | 29.486 | 1.00 | 10.67 | | |
| ANISOU | 2347 | OH2 | HOH | 2010 | 1035 | 1088 | 1932 | 12 | -67 | -134 | |
| ATOM | 2348 | OH2 | HOH | 2011 | 2.119 | 22.469 | 36.569 | 1.00 | 17.30 | | |
| ANISOU | 2348 | OH2 | HOH | 2011 | 2414 | 2016 | 2143 | 137 | -357 | 141 | |
| ATOM | 2349 | OH2 | HOH | 2012 | 4.387 | 16.752 | 27.179 | 1.00 | 9.09 | | |
| ANISOU | 2349 | OH2 | HOH | 2012 | 1048 | 1035 | 1369 | -34 | -174 | 29 | |
| ATOM | 2350 | OH2 | HOH | 2013 | 13.856 | 8.307 | 34.680 | 1.00 | 9.66 | | |
| ANISOU | 2350 | OH2 | HOH | 2013 | 1010 | 1057 | 1605 | 69 | -39 | -95 | |
| ATOM | 2351 | OH2 | HOH | 2014 | 5.073 | 12.687 | 27.611 | 1.00 | 10.04 | | |
| ANISOU | 2351 | OH2 | HOH | 2014 | 1287 | 1057 | 1471 | 154 | -150 | 49 | |
| ATOM | 2352 | OH2 | HOH | 2015 | -2.674 | 28.202 | 25.274 | 1.00 | 19.18 | | |
| ANISOU | 2352 | OH2 | HOH | 2015 | 2267 | 2160 | 2860 | -20 | -424 | 280 | |
| ATOM | 2353 | OH2 | HOH | 2016 | 9.576 | 34.588 | 22.992 | 1.00 | 14.31 | | |
| ANISOU | 2353 | OH2 | HOH | 2016 | 2070 | 1592 | 1774 | 564 | -448 | -324 | |
| ATOM | 2354 | OH2 | HOH | 2017 | 6.335 | 21.231 | 20.029 | 1.00 | 15.09 | | |
| ANISOU | 2354 | OH2 | HOH | 2017 | 1538 | 2538 | 1658 | -411 | -233 | 287 | |
| ATOM | 2355 | OH2 | HOH | 2018 | 6.469 | 15.014 | 27.130 | 1.00 | 9.55 | | |
| ANISOU | 2355 | OH2 | HOH | 2018 | 1121 | 1016 | 1493 | -27 | -329 | -188 | |
| ATOM | 2356 | OH2 | HOH | 2019 | 7.840 | 14.394 | 24.898 | 1.00 | 10.08 | | |
| ANISOU | 2356 | OH2 | HOH | 2019 | 1286 | 996 | 1547 | -121 | -34 | -133 | |
| ATOM | 2357 | OH2 | HOH | 2020 | 27.816 | 13.613 | 35.155 | 1.00 | 12.76 | | |
| ANISOU | 2357 | OH2 | HOH | 2020 | 1454 | 1523 | 1872 | -58 | 127 | 315 | |
| ATOM | 2358 | OH2 | HOH | 2021 | 10.551 | 15.115 | 24.202 | 1.00 | 10.23 | | |
| ANISOU | 2358 | OH2 | HOH | 2021 | 1239 | 842 | 1806 | -20 | -55 | 11 | |
| ATOM | 2359 | OH2 | HOH | 2022 | 18.390 | 13.963 | 21.493 | 1.00 | 10.05 | | |
| ANISOU | 2359 | OH2 | HOH | 2022 | 1223 | 1093 | 1501 | -18 | -110 | -121 | |
| ATOM | 2360 | OH2 | HOH | 2023 | -0.237 | 14.065 | 31.967 | 1.00 | 11.60 | | |
| ANISOU | 2360 | OH2 | HOH | 2023 | 1335 | 1136 | 1938 | 14 | -417 | 104 | |
| ATOM | 2361 | OH2 | HOH | 2024 | 19.982 | 14.750 | 19.390 | 1.00 | 11.75 | | |
| ANISOU | 2361 | OH2 | HOH | 2024 | 1473 | 1554 | 1438 | -32 | 103 | 28 | |
| ATOM | 2362 | OH2 | HOH | 2025 | 13.228 | -5.984 | 23.216 | 1.00 | 12.24 | | |
| ANISOU | 2362 | OH2 | HOH | 2025 | 1443 | 1475 | 1733 | -90 | 45 | 214 | |
| ATOM | 2363 | OH2 | HOH | 2026 | 19.764 | 12.110 | 23.162 | 1.00 | 11.30 | | |
| ANISOU | 2363 | OH2 | HOH | 2026 | 1385 | 1165 | 1745 | 136 | -1 | 6 | |
| ATOM | 2364 | OH2 | HOH | 2027 | 21.917 | 10.821 | 46.070 | 1.00 | 12.10 | | |
| ANISOU | 2364 | OH2 | HOH | 2027 | 1706 | 1284 | 1606 | -253 | 70 | 41 | |
| ATOM | 2365 | OH2 | HOH | 2028 | 18.224 | 32.221 | 34.277 | 1.00 | 23.49 | | |
| ANISOU | 2365 | OH2 | HOH | 2028 | 3265 | 2841 | 2820 | -537 | -65 | -455 | |
| ATOM | 2366 | OH2 | HOH | 2029 | 9.399 | 32.606 | 33.563 | 1.00 | 14.45 | | |
| ANISOU | 2366 | OH2 | HOH | 2029 | 2294 | 1395 | 1801 | 239 | 35 | -127 | |
| ATOM | 2367 | OH2 | HOH | 2030 | 10.115 | 7.865 | 28.900 | 1.00 | 10.40 | | |
| ANISOU | 2367 | OH2 | HOH | 2030 | 1274 | 957 | 1719 | -31 | -116 | 106 | |
| ATOM | 2368 | OH2 | HOH | 2031 | 11.030 | 33.108 | 36.020 | 1.00 | 14.06 | | |
| ANISOU | 2368 | OH2 | HOH | 2031 | 2205 | 1402 | 1734 | 137 | 81 | 34 | |
| ATOM | 2369 | OH2 | HOH | 2032 | 30.731 | 24.856 | 38.865 | 1.00 | 12.21 | | |
| ANISOU | 2369 | OH2 | HOH | 2032 | 1090 | 1814 | 1734 | 139 | 90 | -184 | |
| ATOM | 2370 | OH2 | HOH | 2033 | 11.845 | 9.197 | 30.391 | 1.00 | 9.58 | | |
| ANISOU | 2370 | OH2 | HOH | 2033 | 1154 | 975 | 1510 | 175 | -170 | -64 | |
| ATOM | 2371 | OH2 | HOH | 2034 | 24.995 | 32.489 | 45.432 | 1.00 | 11.85 | | |
| ANISOU | 2371 | OH2 | HOH | 2034 | 1049 | 1477 | 1975 | 100 | -143 | -163 | |
| ATOM | 2372 | OH2 | HOH | 2035 | 13.731 | 17.090 | 17.376 | 1.00 | 12.56 | | |
| ANISOU | 2372 | OH2 | HOH | 2035 | 1706 | 1581 | 1484 | 23 | 127 | -93 | |
| ATOM | 2373 | OH2 | HOH | 2036 | 32.798 | 17.609 | 31.395 | 1.00 | 11.47 | | |
| ANISOU | 2373 | OH2 | HOH | 2036 | 1144 | 1657 | 1556 | 195 | -179 | 158 | |
| ATOM | 2374 | OH2 | HOH | 2037 | 13.336 | 8.586 | 22.450 | 1.00 | 11.15 | | |
| ANISOU | 2374 | OH2 | HOH | 2037 | 1388 | 1353 | 1496 | -147 | -53 | -94 | |
| ATOM | 2375 | OH2 | HOH | 2038 | 7.261 | 40.042 | 27.357 | 1.00 | 14.02 | | |
| ANISOU | 2375 | OH2 | HOH | 2038 | 1877 | 1236 | 2213 | 161 | -78 | -176 | |
| ATOM | 2376 | OH2 | HOH | 2039 | 8.901 | 18.136 | 17.859 | 1.00 | 14.04 | | |
| ANISOU | 2376 | OH2 | HOH | 2039 | 1783 | 1508 | 2043 | -97 | -293 | 219 | |
| ATOM | 2377 | OH2 | HOH | 2040 | 22.901 | 34.139 | 18.135 | 1.00 | 16.20 | | |
| ANISOU | 2377 | OH2 | HOH | 2040 | 2231 | 1532 | 2391 | -244 | 16 | -168 | |
| ATOM | 2378 | OH2 | HOH | 2041 | 23.996 | 7.240 | 27.986 | 1.00 | 15.72 | | |
| ANISOU | 2378 | OH2 | HOH | 2041 | 1400 | 1337 | 3235 | 14 | -27 | 18 | |
| ATOM | 2379 | OH2 | HOH | 2042 | 24.993 | 23.182 | 18.913 | 1.00 | 16.24 | | |
| ANISOU | 2379 | OH2 | HOH | 2042 | 1807 | 2061 | 2302 | 435 | 320 | 101 | |
| ATOM | 2380 | OH2 | HOH | 2043 | 19.148 | 5.661 | 41.077 | 1.00 | 12.85 | | |
| ANISOU | 2380 | OH2 | HOH | 2043 | 1775 | 1227 | 1881 | 93 | 80 | 301 | |
| ATOM | 2381 | OH2 | HOH | 2044 | 24.501 | 28.878 | 32.387 | 1.00 | 20.46 | | |
| ANISOU | 2381 | OH2 | HOH | 2044 | 2385 | 2138 | 3249 | -544 | 79 | -601 | |
| ATOM | 2382 | OH2 | HOH | 2045 | 11.870 | 34.623 | 33.554 | 1.00 | 22.76 | | |
| ANISOU | 2382 | OH2 | HOH | 2045 | 3018 | 2580 | 3049 | 909 | -557 | -1190 | |
| ATOM | 2383 | OH2 | HOH | 2046 | 22.963 | 30.152 | 47.980 | 1.00 | 10.41 | | |
| ANISOU | 2383 | OH2 | HOH | 2046 | 1456 | 1125 | 1373 | -149 | -122 | -146 | |
| ATOM | 2384 | OH2 | HOH | 2047 | -2.686 | 19.073 | 27.374 | 1.00 | 10.53 | | |
| ANISOU | 2384 | OH2 | HOH | 2047 | 912 | 1254 | 1836 | 26 | -145 | 170 | |
| ATOM | 2385 | OH2 | HOH | 2048 | 17.228 | 5.916 | 21.915 | 1.00 | 20.47 | | |
| ANISOU | 2385 | OH2 | HOH | 2048 | 2683 | 2370 | 2723 | 611 | -208 | -179 | |
| ATOM | 2386 | OH2 | HOH | 2049 | 20.966 | 5.515 | 22.787 | 1.00 | 21.11 | | |
| ANISOU | 2386 | OH2 | HOH | 2049 | 3543 | 2077 | 2402 | 458 | 411 | -168 | |
| ATOM | 2387 | OH2 | HOH | 2050 | 18.808 | 35.973 | 20.701 | 1.00 | 22.96 | | |
| ANISOU | 2387 | OH2 | HOH | 2050 | 3279 | 2099 | 3346 | 296 | -370 | -120 | |
| ATOM | 2388 | OH2 | HOH | 2051 | 22.527 | 22.497 | 16.149 | 1.00 | 15.06 | | |
| ANISOU | 2388 | OH2 | HOH | 2051 | 2078 | 1689 | 1956 | 52 | 53 | -315 | |
| ATOM | 2389 | OH2 | HOH | 2052 | 14.374 | 6.193 | 21.643 | 1.00 | 18.60 | | |
| ANISOU | 2389 | OH2 | HOH | 2052 | 2776 | 1935 | 2354 | 506 | -354 | -250 | |
| ATOM | 2390 | OH2 | HOH | 2053 | 30.488 | 22.065 | 25.530 | 1.00 | 13.36 | | |
| ANISOU | 2390 | OH2 | HOH | 2053 | 1442 | 1894 | 1740 | -203 | -165 | -112 | |
| ATOM | 2391 | OH2 | HOH | 2054 | 24.217 | 9.898 | 21.086 | 1.00 | 23.35 | | |
| ANISOU | 2391 | OH2 | HOH | 2054 | 2905 | 2599 | 3367 | 906 | 63 | -739 | |
| ATOM | 2392 | OH2 | HOH | 2055 | 12.732 | 24.770 | 16.261 | 1.00 | 13.36 | | |
| ANISOU | 2392 | OH2 | HOH | 2055 | 2036 | 1650 | 1389 | -178 | 168 | -96 | |
| ATOM | 2393 | OH2 | HOH | 2056 | 35.310 | 14.193 | 33.653 | 1.00 | 20.60 | | |
| ANISOU | 2393 | OH2 | HOH | 2056 | 1762 | 3205 | 2860 | 356 | 323 | 51 | |
| ATOM | 2394 | OH2 | HOH | 2057 | 15.057 | -2.873 | 25.824 | 1.00 | 18.01 | | |
| ANISOU | 2394 | OH2 | HOH | 2057 | 2788 | 1255 | 2800 | -144 | -775 | 116 | |
| ATOM | 2395 | OH2 | HOH | 2058 | 3.861 | 28.991 | 35.199 | 1.00 | 18.52 | | |
| ANISOU | 2395 | OH2 | HOH | 2058 | 1763 | 2855 | 2420 | 213 | -198 | 961 | |
| ATOM | 2396 | OH2 | HOH | 2059 | 27.541 | 9.738 | 35.095 | 1.00 | 17.78 | | |
| ANISOU | 2396 | OH2 | HOH | 2059 | 1891 | 2061 | 2803 | -223 | 395 | -245 | |
| ATOM | 2397 | OH2 | HOH | 2060 | 25.756 | 16.444 | 46.007 | 1.00 | 17.91 | | |
| ANISOU | 2397 | OH2 | HOH | 2060 | 1771 | 2586 | 2448 | -428 | -487 | 216 | |
| ATOM | 2398 | OH2 | HOH | 2061 | 21.108 | 17.307 | 19.017 | 1.00 | 14.94 | | |
| ANISOU | 2398 | OH2 | HOH | 2061 | 2179 | 1477 | 2019 | -266 | 357 | -88 | |
| ATOM | 2399 | OH2 | HOH | 2062 | 7.279 | 35.459 | 18.975 | 1.00 | 22.51 | | |
| ANISOU | 2399 | OH2 | HOH | 2062 | 2439 | 2916 | 3197 | 792 | -55 | 270 | |
| ATOM | 2400 | OH2 | HOH | 2063 | 22.491 | 30.103 | 32.617 | 1.00 | 21.37 | | |
| ANISOU | 2400 | OH2 | HOH | 2063 | 2255 | 2976 | 2889 | -603 | -705 | 842 | |
| ATOM | 2401 | OH2 | HOH | 2064 | 22.615 | 10.885 | 18.887 | 1.00 | 19.80 | | |
| ANISOU | 2401 | OH2 | HOH | 2064 | 2809 | 1934 | 2782 | 42 | 175 | -707 | |
| ATOM | 2402 | OH2 | HOH | 2065 | 2.269 | 8.129 | 35.106 | 1.00 | 15.91 | | |
| ANISOU | 2402 | OH2 | HOH | 2065 | 2287 | 1527 | 2232 | -220 | -92 | 182 | |
| ATOM | 2403 | OH2 | HOH | 2066 | 9.106 | -3.515 | 39.745 | 1.00 | 26.06 | | |
| ANISOU | 2403 | OH2 | HOH | 2066 | 3641 | 2309 | 3951 | 179 | -1365 | 458 | |
| ATOM | 2404 | OH2 | HOH | 2067 | 20.845 | 22.193 | 14.148 | 1.00 | 18.98 | | |
| ANISOU | 2404 | OH2 | HOH | 2067 | 2479 | 2361 | 2372 | 61 | -17 | 638 | |
| ATOM | 2405 | OH2 | HOH | 2068 | 13.543 | 6.071 | 19.029 | 1.00 | 23.30 | | |
| ANISOU | 2405 | OH2 | HOH | 2068 | 3621 | 2014 | 3217 | -352 | -687 | 605 | |
| ATOM | 2406 | OH2 | HOH | 2069 | 18.565 | 18.981 | 12.787 | 1.00 | 16.91 | | |
| ANISOU | 2406 | OH2 | HOH | 2069 | 2710 | 1821 | 1892 | 123 | -26 | 46 | |
| ATOM | 2407 | OH2 | HOH | 2070 | 18.359 | 21.142 | 14.600 | 1.00 | 14.84 | | |
| ANISOU | 2407 | OH2 | HOH | 2070 | 2166 | 1592 | 1880 | 195 | -73 | -17 | |
| ATOM | 2408 | OH2 | HOH | 2071 | 4.366 | 11.191 | 46.702 | 1.00 | 23.90 | | |
| ANISOU | 2408 | OH2 | HOH | 2071 | 3983 | 2817 | 2280 | 180 | -258 | 479 | |
| ATOM | 2409 | OH2 | HOH | 2072 | 4.072 | 12.730 | 17.146 | 1.00 | 24.94 | | |
| ANISOU | 2409 | OH2 | HOH | 2072 | 3301 | 3045 | 3129 | 319 | -435 | -888 | |
| ATOM | 2410 | OH2 | HOH | 2073 | 35.859 | 26.345 | 23.371 | 1.00 | 17.56 | | |
| ANISOU | 2410 | OH2 | HOH | 2073 | 1939 | 1917 | 2817 | -48 | 90 | 351 | |
| ATOM | 2411 | OH2 | HOH | 2074 | 18.868 | 6.807 | 23.957 | 1.00 | 19.27 | | |
| ANISOU | 2411 | OH2 | HOH | 2074 | 2094 | 2895 | 2334 | -383 | -23 | -44 | |
| ATOM | 2412 | OH2 | HOH | 2075 | 9.091 | -0.413 | 41.963 | 1.00 | 26.22 | | |
| ANISOU | 2412 | OH2 | HOH | 2075 | 4785 | 2662 | 2516 | 921 | 0 | 578 | |
| ATOM | 2413 | OH2 | HOH | 2076 | 16.112 | 2.843 | 21.594 | 1.00 | 19.95 | | |
| ANISOU | 2413 | OH2 | HOH | 2076 | 2733 | 2212 | 2635 | -4 | -260 | 92 | |
| ATOM | 2414 | OH2 | HOH | 2077 | 18.485 | 19.782 | 17.034 | 1.00 | 17.00 | | |
| ANISOU | 2414 | OH2 | HOH | 2077 | 2420 | 2195 | 1846 | 24 | 53 | -391 | |
| ATOM | 2415 | OH2 | HOH | 2078 | 29.025 | 27.077 | 29.091 | 1.00 | 22.18 | | |
| ANISOU | 2415 | OH2 | HOH | 2078 | 2253 | 3097 | 3077 | 598 | 184 | -244 | |
| ATOM | 2416 | OH2 | HOH | 2079 | 19.760 | 17.529 | 16.504 | 1.00 | 27.43 | | |
| ANISOU | 2416 | OH2 | HOH | 2079 | 4340 | 3159 | 2922 | -636 | -664 | -332 | |
| ATOM | 2417 | OH2 | HOH | 2080 | 8.540 | 23.883 | 11.386 | 1.00 | 21.02 | | |
| ANISOU | 2417 | OH2 | HOH | 2080 | 3034 | 2540 | 2414 | 95 | -812 | -497 | |
| ATOM | 2418 | OH2 | HOH | 2081 | 32.474 | 22.826 | 37.215 | 1.00 | 19.51 | | |
| ANISOU | 2418 | OH2 | HOH | 2081 | 1798 | 3082 | 2531 | 67 | -761 | 339 | |
| ATOM | 2419 | OH2 | HOH | 2082 | 21.545 | 11.232 | 16.522 | 1.00 | 26.38 | | |
| ANISOU | 2419 | OH2 | HOH | 2082 | 2902 | 3909 | 3212 | 1513 | 713 | 392 | |
| ATOM | 2420 | OH2 | HOH | 2083 | 16.861 | 32.912 | 39.027 | 1.00 | 24.27 | | |
| ANISOU | 2420 | OH2 | HOH | 2083 | 4041 | 2361 | 2817 | -597 | -538 | -127 | |
| ATOM | 2421 | OH2 | HOH | 2084 | 9.457 | 26.048 | 46.967 | 1.00 | 32.61 | | |
| ANISOU | 2421 | OH2 | HOH | 2084 | 3972 | 3859 | 4559 | -335 | -739 | -194 | |
| ATOM | 2422 | OH2 | HOH | 2085 | 25.255 | 22.321 | 16.382 | 1.00 | 22.54 | | |
| ANISOU | 2422 | OH2 | HOH | 2085 | 2578 | 3324 | 2662 | 797 | 118 | 225 | |
| ATOM | 2423 | OH2 | HOH | 2086 | 7.805 | 33.123 | 21.031 | 1.00 | 22.21 | | |
| ANISOU | 2423 | OH2 | HOH | 2086 | 2474 | 2898 | 3067 | -89 | -7 | 180 | |
| ATOM | 2424 | OH2 | HOH | 2087 | 2.963 | 21.630 | 21.012 | 1.00 | 24.81 | | |
| ANISOU | 2424 | OH2 | HOH | 2087 | 3246 | 3288 | 2892 | 422 | 216 | -522 | |
| ATOM | 2425 | OH2 | HOH | 2088 | 12.611 | -2.156 | 30.006 | 1.00 | 29.02 | | |
| ANISOU | 2425 | OH2 | HOH | 2088 | 5002 | 2469 | 3555 | 1133 | 57 | 319 | |
| ATOM | 2426 | OH2 | HOH | 2089 | 28.315 | 11.728 | 30.582 | 1.00 | 18.77 | | |
| ANISOU | 2426 | OH2 | HOH | 2089 | 2184 | 1833 | 3113 | 424 | -72 | -218 | |
| ATOM | 2427 | OH2 | HOH | 2090 | 0.495 | 6.125 | 34.745 | 1.00 | 25.84 | | |
| ANISOU | 2427 | OH2 | HOH | 2090 | 3436 | 2158 | 4223 | -962 | 713 | -537 | |
| ATOM | 2428 | OH2 | HOH | 2091 | 16.120 | 19.247 | 45.953 | 1.00 | 25.44 | | |
| ANISOU | 2428 | OH2 | HOH | 2091 | 3973 | 2819 | 2875 | 97 | 574 | -277 | |
| ATOM | 2429 | OH2 | HOH | 2092 | 16.726 | 33.514 | 31.779 | 1.00 | 34.52 | | |
| ANISOU | 2429 | OH2 | HOH | 2092 | 4540 | 5165 | 3411 | -2623 | -686 | -581 | |
| ATOM | 2430 | OH2 | HOH | 2093 | 28.925 | 18.611 | 19.416 | 1.00 | 24.08 | | |
| ANISOU | 2430 | OH2 | HOH | 2093 | 2841 | 3620 | 2690 | 596 | 470 | -189 | |
| ATOM | 2431 | OH2 | HOH | 2094 | 29.000 | 18.421 | 40.799 | 1.00 | 30.10 | | |
| ANISOU | 2431 | OH2 | HOH | 2094 | 3522 | 3032 | 4881 | 229 | -35 | -348 | |
| ATOM | 2432 | OH2 | HOH | 2095 | 25.850 | 11.412 | 45.963 | 1.00 | 19.48 | | |
| ANISOU | 2432 | OH2 | HOH | 2095 | 1907 | 2086 | 3408 | 256 | 14 | 909 | |
| ATOM | 2433 | OH2 | HOH | 2096 | 20.009 | 37.070 | 24.551 | 1.00 | 27.14 | | |
| ANISOU | 2433 | OH2 | HOH | 2096 | 3277 | 3716 | 3319 | -9 | -693 | -30 | |
| ATOM | 2434 | OH2 | HOH | 2097 | -0.741 | 10.653 | 36.389 | 1.00 | 27.10 | | |
| ANISOU | 2434 | OH2 | HOH | 2097 | 3762 | 2750 | 3783 | -1375 | -1082 | 1059 | |
| ATOM | 2435 | OH2 | HOH | 2098 | 25.778 | 6.183 | 26.326 | 1.00 | 29.21 | | |
| ANISOU | 2435 | OH2 | HOH | 2098 | 2836 | 3824 | 4439 | 1152 | 1061 | 1265 | |
| ATOM | 2436 | OH2 | HOH | 2099 | 20.635 | 33.299 | -1.826 | 1.00 | 30.80 | | |
| ANISOU | 2436 | OH2 | HOH | 2099 | 5217 | 3154 | 3332 | -1098 | 232 | -415 | |
| ATOM | 2437 | OH2 | HOH | 2100 | 23.679 | 15.862 | 19.292 | 1.00 | 27.46 | | |
| ANISOU | 2437 | OH2 | HOH | 2100 | 3779 | 3378 | 3278 | 503 | 53 | 28 | |
| ATOM | 2438 | OH2 | HOH | 2101 | 28.642 | 11.615 | 33.283 | 1.00 | 18.91 | | |
| ANISOU | 2438 | OH2 | HOH | 2101 | 2364 | 1844 | 2978 | 194 | 182 | 160 | |
| ATOM | 2439 | OH2 | HOH | 2102 | 20.500 | 30.664 | 34.595 | 1.00 | 29.66 | | |
| ANISOU | 2439 | OH2 | HOH | 2102 | 4477 | 3948 | 2846 | 1402 | -136 | -172 | |
| ATOM | 2440 | OH2 | HOH | 2103 | 19.636 | 36.217 | 18.159 | 1.00 | 39.53 | | |
| ANISOU | 2440 | OH2 | HOH | 2103 | 5302 | 3739 | 5977 | 1897 | -584 | -61 | |
| ATOM | 2441 | OH2 | HOH | 2104 | 22.561 | 35.055 | 28.392 | 1.00 | 27.57 | | |
| ANISOU | 2441 | OH2 | HOH | 2104 | 2551 | 3571 | 4355 | -502 | -407 | -353 | |
| ATOM | 2442 | OH2 | HOH | 2105 | 9.812 | 3.300 | 14.696 | 1.00 | 22.81 | | |
| ANISOU | 2442 | OH2 | HOH | 2105 | 2783 | 2692 | 3193 | 393 | -704 | -630 | |
| ATOM | 2443 | OH2 | HOH | 2106 | 17.478 | 13.427 | 11.743 | 1.00 | 24.02 | | |
| ANISOU | 2443 | OH2 | HOH | 2106 | 3894 | 2847 | 2384 | -506 | 379 | 34 | |
| ATOM | 2444 | OH2 | HOH | 2107 | 18.319 | 21.596 | 1.717 | 1.00 | 37.21 | | |
| ANISOU | 2444 | OH2 | HOH | 2107 | 5869 | 3987 | 4284 | -959 | 1727 | -1356 | |
| ATOM | 2445 | OH2 | HOH | 2108 | 0.147 | -1.608 | 12.880 | 1.00 | 27.29 | | |
| ANISOU | 2445 | OH2 | HOH | 2108 | 2843 | 3741 | 3783 | 326 | -894 | -1356 | |
| ATOM | 2446 | OH2 | HOH | 2109 | 6.268 | 18.645 | 18.883 | 1.00 | 22.59 | | |
| ANISOU | 2446 | OH2 | HOH | 2109 | 2918 | 2710 | 2956 | 139 | -216 | -411 | |
| ATOM | 2447 | OH2 | HOH | 2110 | 16.838 | -0.215 | 37.746 | 1.00 | 30.69 | | |
| ANISOU | 2447 | OH2 | HOH | 2110 | 4095 | 2373 | 5193 | -296 | -1720 | 189 | |
| ATOM | 2448 | OH2 | HOH | 2111 | 25.258 | 32.534 | 41.448 | 1.00 | 27.41 | | |
| ANISOU | 2448 | OH2 | HOH | 2111 | 3973 | 1987 | 4456 | -218 | 482 | -926 | |
| ATOM | 2449 | OH2 | HOH | 2112 | 2.223 | 25.051 | 13.896 | 1.00 | 28.20 | | |
| ANISOU | 2449 | OH2 | HOH | 2112 | 3984 | 2395 | 4337 | 6 | -1010 | -511 | |
| ATOM | 2450 | OH2 | HOH | 2113 | 29.267 | 33.574 | 21.744 | 1.00 | 28.16 | | |
| ANISOU | 2450 | OH2 | HOH | 2113 | 2151 | 3744 | 4804 | -674 | 328 | -569 | |
| ATOM | 2451 | OH2 | HOH | 2114 | 14.154 | 32.384 | 41.536 | 1.00 | 30.79 | | |
| ANISOU | 2451 | OH2 | HOH | 2114 | 2509 | 3569 | 5622 | 177 | 209 | -1106 | |
| ATOM | 2452 | OH2 | HOH | 2115 | 25.575 | 23.548 | 4.950 | 1.00 | 27.26 | | |
| ANISOU | 2452 | OH2 | HOH | 2115 | 3885 | 3085 | 3387 | 1053 | -102 | -289 | |
| ATOM | 2453 | OH2 | HOH | 2116 | 21.593 | 7.334 | 20.867 | 1.00 | 27.89 | | |
| ANISOU | 2453 | OH2 | HOH | 2116 | 3321 | 3848 | 3429 | 535 | -417 | 629 | |
| ATOM | 2454 | OH2 | HOH | 2117 | 23.155 | 15.322 | 49.852 | 1.00 | 29.43 | | |
| ANISOU | 2454 | OH2 | HOH | 2117 | 5667 | 2625 | 2888 | -741 | -1410 | -77 | |
| ATOM | 2455 | OH2 | HOH | 2118 | 31.241 | 11.512 | 34.024 | 1.00 | 29.65 | | |
| ANISOU | 2455 | OH2 | HOH | 2118 | 1759 | 4478 | 5029 | -73 | 391 | 1925 | |
| ATOM | 2456 | OH2 | HOH | 2119 | 12.506 | 13.347 | 44.954 | 1.00 | 30.47 | | |
| ANISOU | 2456 | OH2 | HOH | 2119 | 4074 | 3073 | 4432 | -416 | 225 | 29 | |
| ATOM | 2457 | OH2 | HOH | 2120 | 9.137 | 42.977 | 4.644 | 1.00 | 28.80 | | |
| ANISOU | 2457 | OH2 | HOH | 2120 | 4403 | 3030 | 3511 | 268 | 177 | -492 | |
| ATOM | 2458 | OH2 | HOH | 2121 | 10.371 | -4.559 | 22.096 | 1.00 | 25.99 | | |
| ANISOU | 2458 | OH2 | HOH | 2121 | 3180 | 2749 | 3946 | 847 | 777 | 439 | |
| ATOM | 2459 | OH2 | HOH | 2122 | -1.884 | 31.287 | 33.910 | 1.00 | 30.32 | | |
| ANISOU | 2459 | OH2 | HOH | 2122 | 4437 | 3474 | 3610 | 764 | 121 | -284 | |
| ATOM | 2460 | OH2 | HOH | 2123 | 11.926 | 2.902 | 16.437 | 1.00 | 34.58 | | |
| ANISOU | 2460 | OH2 | HOH | 2123 | 3807 | 4138 | 5194 | 1233 | -1452 | -828 | |
| ATOM | 2461 | OH2 | HOH | 2124 | 31.395 | 25.003 | 42.826 | 1.00 | 31.68 | | |
| ANISOU | 2461 | OH2 | HOH | 2124 | 2510 | 3482 | 6045 | 484 | -845 | 81 | |
| ATOM | 2462 | OH2 | HOH | 2125 | 27.526 | 15.878 | 19.127 | 1.00 | 30.88 | | |
| ANISOU | 2462 | OH2 | HOH | 2125 | 4449 | 3902 | 3383 | -518 | -899 | 658 | |
| ATOM | 2463 | OH2 | HOH | 2126 | 15.367 | -0.963 | 15.516 | 1.00 | 49.85 | | |
| ANISOU | 2463 | OH2 | HOH | 2126 | 7256 | 5575 | 6111 | 1754 | -27 | 279 | |
| ATOM | 2464 | OH2 | HOH | 2127 | 26.630 | 29.902 | 34.801 | 1.00 | 29.70 | | |
| ANISOU | 2464 | OH2 | HOH | 2127 | 3399 | 3101 | 4786 | -566 | 1487 | -1057 | |
| ATOM | 2465 | OH2 | HOH | 2128 | 5.907 | 37.376 | 1.732 | 1.00 | 28.32 | | |
| ANISOU | 2465 | OH2 | HOH | 2128 | 4487 | 3014 | 3260 | -536 | -1083 | 189 | |
| ATOM | 2466 | OH2 | HOH | 2129 | 34.255 | 23.638 | 18.132 | 1.00 | 50.15 | | |
| ANISOU | 2466 | OH2 | HOH | 2129 | 6135 | 7075 | 5844 | -476 | 1990 | 903 | |
| ATOM | 2467 | OH2 | HOH | 2130 | 3.318 | 37.511 | 27.388 | 1.00 | 27.03 | | |
| ANISOU | 2467 | OH2 | HOH | 2130 | 2658 | 2481 | 5132 | 212 | -550 | -88 | |
| ATOM | 2468 | OH2 | HOH | 2131 | 1.117 | 29.406 | 35.726 | 1.00 | 30.64 | | |
| ANISOU | 2468 | OH2 | HOH | 2131 | 3294 | 5110 | 3240 | 610 | 20 | 383 | |
| ATOM | 2469 | OH2 | HOH | 2132 | 4.604 | 0.464 | 18.877 | 1.00 | 25.82 | | |
| ANISOU | 2469 | OH2 | HOH | 2132 | 3577 | 2371 | 3862 | -157 | -950 | -139 | |
| ATOM | 2470 | OH2 | HOH | 2133 | -0.956 | 12.842 | 21.057 | 1.00 | 24.47 | | |
| ANISOU | 2470 | OH2 | HOH | 2133 | 3717 | 3027 | 2553 | 385 | -684 | -236 | |
| ATOM | 2471 | OH2 | HOH | 2134 | 27.604 | 15.988 | 43.950 | 1.00 | 45.25 | | |
| ANISOU | 2471 | OH2 | HOH | 2134 | 3755 | 7222 | 6216 | 690 | -1387 | 93 | |
| ATOM | 2472 | OH2 | HOH | 2135 | 10.025 | 0.613 | 18.185 | 1.00 | 42.41 | | |
| ANISOU | 2472 | OH2 | HOH | 2135 | 6266 | 5193 | 4653 | 364 | -513 | -694 | |
| ATOM | 2473 | OH2 | HOH | 2136 | 35.927 | 17.351 | 37.100 | 1.00 | 28.89 | | |
| ANISOU | 2473 | OH2 | HOH | 2136 | 2217 | 3925 | 4833 | -556 | -220 | 1242 | |
| ATOM | 2474 | OH2 | HOH | 2137 | 5.594 | 19.454 | 44.164 | 1.00 | 42.26 | | |
| ANISOU | 2474 | OH2 | HOH | 2137 | 6586 | 5675 | 3798 | -73 | 524 | -39 | |
| ATOM | 2475 | OH2 | HOH | 2138 | 23.132 | 22.600 | 45.476 | 1.00 | 25.88 | | |
| ANISOU | 2475 | OH2 | HOH | 2138 | 2245 | 4904 | 2686 | 346 | -439 | 502 | |
| ATOM | 2476 | OH2 | HOH | 2139 | 20.172 | 31.979 | 41.563 | 1.00 | 32.59 | | |
| ANISOU | 2476 | OH2 | HOH | 2139 | 2979 | 5289 | 4112 | -345 | 255 | -15 | |
| ATOM | 2477 | OH2 | HOH | 2140 | 12.615 | -3.457 | 32.659 | 1.00 | 29.56 | | |
| ANISOU | 2477 | OH2 | HOH | 2140 | 4077 | 3396 | 3758 | 197 | 444 | 282 | |
| ATOM | 2478 | OH2 | HOH | 2141 | 29.893 | 13.167 | 28.439 | 1.00 | 36.82 | | |
| ANISOU | 2478 | OH2 | HOH | 2141 | 5526 | 3816 | 4649 | -1141 | 34 | -237 | |
| ATOM | 2479 | OH2 | HOH | 2142 | 12.410 | 49.301 | 5.202 | 1.00 | 36.70 | | |
| ANISOU | 2479 | OH2 | HOH | 2142 | 5403 | 4305 | 4238 | 835 | 1728 | -452 | |
| ATOM | 2480 | OH2 | HOH | 2143 | 30.715 | 29.052 | 35.394 | 1.00 | 28.01 | | |
| ANISOU | 2480 | OH2 | HOH | 2143 | 3128 | 2641 | 4875 | -241 | -616 | -997 | |
| ATOM | 2481 | OH2 | HOH | 2144 | 17.281 | 34.142 | -1.271 | 1.00 | 45.94 | | |
| ANISOU | 2481 | OH2 | HOH | 2144 | 7491 | 5662 | 4300 | 795 | -289 | 1299 | |
| ATOM | 2482 | OH2 | HOH | 2145 | 4.922 | 22.797 | 42.581 | 1.00 | 19.12 | | |
| ANISOU | 2482 | OH2 | HOH | 2145 | 3396 | 2327 | 1540 | -411 | 509 | -198 | |
| ATOM | 2483 | OH2 | HOH | 2146 | 26.957 | 30.159 | 14.287 | 1.00 | 25.77 | | |
| ANISOU | 2483 | OH2 | HOH | 2146 | 3055 | 3169 | 3566 | 265 | 831 | 406 | |
| ATOM | 2484 | OH2 | HOH | 2147 | 12.422 | 45.401 | 5.036 | 1.00 | 34.03 | | |
| ANISOU | 2484 | OH2 | HOH | 2147 | 3642 | 4631 | 4658 | -181 | 355 | -359 | |
| ATOM | 2485 | OH2 | HOH | 2148 | 33.437 | 14.116 | 23.484 | 1.00 | 31.47 | | |
| ANISOU | 2485 | OH2 | HOH | 2148 | 3522 | 3389 | 5047 | 1058 | 397 | 882 | |
| ATOM | 2486 | OH2 | HOH | 2149 | 0.164 | 4.431 | 23.666 | 1.00 | 33.84 | | |
| ANISOU | 2486 | OH2 | HOH | 2149 | 4637 | 4815 | 3406 | 154 | 538 | -430 | |
| ATOM | 2487 | OH2 | HOH | 2150 | -1.460 | 28.447 | 34.307 | 1.00 | 28.09 | | |
| ANISOU | 2487 | OH2 | HOH | 2150 | 2705 | 3868 | 4101 | 337 | 152 | -315 | |
| ATOM | 2488 | OH2 | HOH | 2151 | 13.771 | 22.521 | 3.113 | 1.00 | 30.67 | | |
| ANISOU | 2488 | OH2 | HOH | 2151 | 5208 | 3547 | 2897 | -542 | -72 | -845 | |
| ATOM | 2489 | OH2 | HOH | 2152 | 28.782 | 29.818 | 29.002 | 1.00 | 47.73 | | |
| ANISOU | 2489 | OH2 | HOH | 2152 | 7348 | 5977 | 4811 | 1228 | -875 | 839 | |
| ATOM | 2490 | OH2 | HOH | 2153 | 7.137 | 15.243 | 42.731 | 1.00 | 29.71 | | |
| ANISOU | 2490 | OH2 | HOH | 2153 | 2540 | 4556 | 4193 | 805 | 148 | 7 | |
| ATOM | 2491 | OH2 | HOH | 2154 | 23.785 | 46.053 | 8.991 | 1.00 | 38.34 | | |
| ANISOU | 2491 | OH2 | HOH | 2154 | 5125 | 4044 | 5399 | -812 | -1016 | 1182 | |
| ATOM | 2492 | OH2 | HOH | 2155 | 33.133 | 20.243 | 38.315 | 1.00 | 36.79 | | |
| ANISOU | 2492 | OH2 | HOH | 2155 | 3714 | 5505 | 4760 | 1076 | -517 | -93 | |
| ATOM | 2493 | OH2 | HOH | 2156 | -0.820 | 9.533 | 18.799 | 1.00 | 41.36 | | |
| ANISOU | 2493 | OH2 | HOH | 2156 | 4360 | 5474 | 5882 | 1636 | 646 | -129 | |
| ATOM | 2494 | OH2 | HOH | 2157 | 18.652 | 5.079 | 19.622 | 1.00 | 29.73 | | |
| ANISOU | 2494 | OH2 | HOH | 2157 | 4286 | 3504 | 3508 | 264 | -349 | 733 | |
| ATOM | 2495 | OH2 | HOH | 2158 | 26.166 | 9.163 | 40.855 | 1.00 | 35.89 | | |
| ANISOU | 2495 | OH2 | HOH | 2158 | 3784 | 5241 | 4612 | 1346 | -144 | 852 | |
| ATOM | 2496 | OH2 | HOH | 2159 | 14.006 | 34.402 | 20.605 | 1.00 | 21.45 | | |
| ANISOU | 2496 | OH2 | HOH | 2159 | 2941 | 3370 | 1838 | -794 | 136 | 154 | |
| ATOM | 2497 | OH2 | HOH | 2160 | 34.840 | 11.129 | 34.976 | 1.00 | 38.19 | | |
| ANISOU | 2497 | OH2 | HOH | 2160 | 3440 | 6588 | 4481 | 697 | -15 | -1011 | |
| ATOM | 2498 | OH2 | HOH | 2161 | -1.988 | 5.242 | 23.285 | 1.00 | 62.11 | | |
| ANISOU | 2498 | OH2 | HOH | 2161 | 8575 | 7078 | 7945 | 192 | 1205 | -866 | |
| ATOM | 2499 | OH2 | HOH | 2162 | 25.277 | 29.730 | 9.669 | 1.00 | 38.83 | | |
| ANISOU | 2499 | OH2 | HOH | 2162 | 3194 | 5683 | 5876 | -287 | 931 | 1477 | |
| ATOM | 2500 | OH2 | HOH | 2163 | 13.817 | 35.649 | 32.918 | 1.00 | 37.06 | | |
| ANISOU | 2500 | OH2 | HOH | 2163 | 5619 | 3812 | 4648 | 1131 | -941 | 1186 | |
| ATOM | 2501 | OH2 | HOH | 2164 | 9.801 | -1.515 | 21.959 | 1.00 | 32.73 | | |
| ANISOU | 2501 | OH2 | HOH | 2164 | 5130 | 3019 | 4287 | 622 | -481 | 422 | |
| ATOM | 2502 | OH2 | HOH | 2165 | 22.948 | 13.966 | 16.940 | 1.00 | 38.75 | | |
| ANISOU | 2502 | OH2 | HOH | 2165 | 5598 | 4725 | 4399 | -470 | 118 | -495 | |
| ATOM | 2503 | OH2 | HOH | 2166 | 24.599 | 43.712 | 23.170 | 1.00 | 41.80 | | |
| ANISOU | 2503 | OH2 | HOH | 2166 | 4078 | 5118 | 6684 | 378 | 1519 | -1220 | |
| ATOM | 2504 | OH2 | HOH | 2167 | 14.615 | 17.699 | 47.486 | 1.00 | 39.85 | | |
| ANISOU | 2504 | OH2 | HOH | 2167 | 6879 | 4803 | 3458 | 1994 | 259 | -707 | |
| ATOM | 2505 | OH2 | HOH | 2168 | 1.445 | 21.717 | 40.470 | 1.00 | 33.88 | | |
| ANISOU | 2505 | OH2 | HOH | 2168 | 3815 | 5217 | 3841 | -697 | 181 | -1633 | |
| ATOM | 2506 | OH2 | HOH | 2169 | 22.327 | -3.798 | 28.767 | 1.00 | 40.30 | | |
| ANISOU | 2506 | OH2 | HOH | 2169 | 5793 | 4158 | 5361 | -118 | -1624 | -544 | |
| ATOM | 2507 | OH2 | HOH | 2170 | -3.301 | 31.835 | 29.570 | 1.00 | 47.68 | | |
| ANISOU | 2507 | OH2 | HOH | 2170 | 4363 | 6351 | 7402 | -268 | 1457 | 665 | |
| ATOM | 2508 | OH2 | HOH | 2171 | 14.099 | 20.235 | 49.732 | 1.00 | 45.20 | | |
| ANISOU | 2508 | OH2 | HOH | 2171 | 5780 | 6657 | 4736 | 223 | -383 | -76 | |
| ATOM | 2509 | OH2 | HOH | 2172 | 33.010 | 29.916 | 24.185 | 1.00 | 36.73 | | |
| ANISOU | 2509 | OH2 | HOH | 2172 | 3842 | 4642 | 5470 | 935 | 604 | 883 | |
| ATOM | 2510 | OH2 | HOH | 2173 | 24.540 | 35.853 | 16.921 | 1.00 | 35.14 | | |
| ANISOU | 2510 | OH2 | HOH | 2173 | 5493 | 4104 | 3754 | -2065 | -882 | 1281 | |
| ATOM | 2511 | OH2 | HOH | 2174 | 22.267 | 39.521 | 13.562 | 1.00 | 38.26 | | |
| ANISOU | 2511 | OH2 | HOH | 2174 | 4832 | 5278 | 4427 | -154 | -1914 | -2304 | |
| ATOM | 2512 | OH2 | HOH | 2175 | 27.448 | 18.787 | 44.469 | 1.00 | 37.82 | | |
| ANISOU | 2512 | OH2 | HOH | 2175 | 3810 | 4082 | 6476 | 300 | -1875 | 1593 | |
| ATOM | 2513 | OH2 | HOH | 2176 | -5.528 | 7.324 | 24.580 | 1.00 | 39.48 | | |
| ANISOU | 2513 | OH2 | HOH | 2176 | 4104 | 5255 | 5641 | -850 | -256 | 33 | |
| ATOM | 2514 | OH2 | HOH | 2177 | -1.271 | 21.631 | 37.322 | 1.00 | 51.77 | | |
| ANISOU | 2514 | OH2 | HOH | 2177 | 6114 | 6078 | 7476 | 95 | -1345 | -992 | |
| ATOM | 2515 | OH2 | HOH | 2178 | 31.545 | 33.928 | 23.456 | 1.00 | 35.45 | | |
| ANISOU | 2515 | OH2 | HOH | 2178 | 2867 | 4757 | 5847 | -1426 | 392 | -613 | |
| ATOM | 2516 | OH2 | HOH | 2179 | 26.809 | 27.791 | 10.386 | 1.00 | 50.04 | | |
| ANISOU | 2516 | OH2 | HOH | 2179 | 8073 | 6525 | 4414 | 92 | -1481 | 1021 | |
| ATOM | 2517 | OH2 | HOH | 2180 | 16.434 | 43.287 | -1.513 | 1.00 | 32.80 | | |
| ANISOU | 2517 | OH2 | HOH | 2180 | 5076 | 4302 | 3084 | -1009 | 378 | -806 | |
| ATOM | 2518 | OH2 | HOH | 2181 | 0.515 | 1.244 | 23.642 | 1.00 | 46.07 | | |
| ANISOU | 2518 | OH2 | HOH | 2181 | 6632 | 5108 | 5766 | -333 | -625 | -365 | |
| ATOM | 2519 | OH2 | HOH | 2182 | 4.462 | 40.555 | 23.430 | 1.00 | 46.89 | | |
| ANISOU | 2519 | OH2 | HOH | 2182 | 5898 | 4762 | 7156 | 1683 | 168 | -724 | |
| ATOM | 2520 | OH2 | HOH | 2183 | 7.693 | 18.637 | 44.649 | 1.00 | 54.29 | | |
| ANISOU | 2520 | OH2 | HOH | 2183 | 5720 | 8062 | 6844 | 444 | 356 | 1599 | |
| ATOM | 2521 | OH2 | HOH | 2184 | 16.308 | 3.941 | 19.161 | 1.00 | 41.90 | | |
| ANISOU | 2521 | OH2 | HOH | 2184 | 5275 | 5467 | 5177 | 722 | 1155 | 423 | |
| ATOM | 2522 | OH2 | HOH | 2185 | 24.284 | 31.246 | 30.071 | 1.00 | 38.99 | | |
| ANISOU | 2522 | OH2 | HOH | 2185 | 3156 | 6579 | 5081 | -671 | 1673 | 405 | |
| ATOM | 2523 | OH2 | HOH | 2186 | 3.278 | 26.568 | 41.478 | 1.00 | 38.84 | | |
| ANISOU | 2523 | OH2 | HOH | 2186 | 4518 | 5116 | 5126 | -518 | 2217 | -1485 | |
| ATOM | 2524 | OH2 | HOH | 2187 | 3.639 | 19.957 | 19.606 | 1.00 | 40.15 | | |
| ANISOU | 2524 | OH2 | HOH | 2187 | 4585 | 5844 | 4825 | -498 | -1941 | 1112 | |
| ATOM | 2525 | OH2 | HOH | 2188 | 21.631 | 33.310 | 34.590 | 1.00 | 63.14 | | |
| ANISOU | 2525 | OH2 | HOH | 2188 | 8075 | 8637 | 7279 | -728 | 183 | -1126 | |
| ATOM | 2526 | OH2 | HOH | 2189 | 32.720 | 29.399 | 21.712 | 1.00 | 53.84 | | |
| ANISOU | 2526 | OH2 | HOH | 2189 | 4431 | 7584 | 8441 | 52 | -467 | -206 | |
| ATOM | 2527 | OH2 | HOH | 2190 | 24.376 | 3.189 | 30.591 | 1.00 | 50.03 | | |
| ANISOU | 2527 | OH2 | HOH | 2190 | 6412 | 6632 | 5966 | 275 | -2344 | 589 | |
| ATOM | 2528 | OH2 | HOH | 2191 | -4.748 | 4.483 | 26.356 | 1.00 | 42.58 | | |
| ANISOU | 2528 | OH2 | HOH | 2191 | 4725 | 4619 | 6834 | -557 | -249 | -1132 | |
| ATOM | 2529 | OH2 | HOH | 2192 | -6.926 | 8.039 | 28.059 | 1.00 | 43.47 | | |
| ANISOU | 2529 | OH2 | HOH | 2192 | 3521 | 5622 | 7375 | 418 | 180 | 553 | |
| ATOM | 2530 | OH2 | HOH | 2193 | 14.237 | 53.526 | 3.775 | 1.00 | 55.14 | | |
| ANISOU | 2530 | OH2 | HOH | 2193 | 7223 | 7276 | 6451 | 657 | 606 | -1397 | |
| ATOM | 2531 | OH2 | HOH | 2194 | 9.458 | 12.921 | 44.597 | 1.00 | 36.74 | | |
| ANISOU | 2531 | OH2 | HOH | 2194 | 6936 | 4199 | 2826 | -1801 | 1015 | 58 | |
| ATOM | 2532 | OH2 | HOH | 2195 | 0.251 | 3.079 | 35.433 | 1.00 | 46.34 | | |
| ANISOU | 2532 | OH2 | HOH | 2195 | 5953 | 7035 | 4619 | 1756 | -784 | -592 | |
| ATOM | 2533 | OH2 | HOH | 2196 | 25.829 | 31.812 | 1.275 | 1.00 | 61.39 | | |
| ANISOU | 2533 | OH2 | HOH | 2196 | 7957 | 7943 | 7426 | -1266 | 689 | -159 | |
| ATOM | 2534 | OH2 | HOH | 2197 | 5.655 | -1.834 | 39.466 | 1.00 | 52.65 | | |
| ANISOU | 2534 | OH2 | HOH | 2197 | 5708 | 7338 | 6960 | 672 | -532 | 941 | |
| ATOM | 2535 | OH2 | HOH | 2198 | 19.706 | 3.198 | 23.260 | 1.00 | 66.22 | | |
| ANISOU | 2535 | OH2 | HOH | 2198 | 7117 | 9716 | 8327 | -157 | 1989 | -532 | |
| ATOM | 2536 | OH2 | HOH | 2199 | 15.408 | 14.624 | 11.007 | 1.00 | 55.81 | | |
| ANISOU | 2536 | OH2 | HOH | 2199 | 8534 | 5674 | 6999 | 1238 | -109 | -1873 | |
| ATOM | 2537 | OH2 | HOH | 2200 | 3.804 | 11.778 | 14.191 | 1.00 | 35.90 | | |
| ANISOU | 2537 | OH2 | HOH | 2200 | 4584 | 3990 | 5068 | -835 | -1773 | 1552 | |
| ATOM | 2538 | OH2 | HOH | 2201 | 10.699 | 20.908 | 50.371 | 1.00 | 48.49 | | |
| ANISOU | 2538 | OH2 | HOH | 2201 | 6598 | 7024 | 4801 | -2364 | -1174 | 534 | |
| ATOM | 2539 | OH2 | HOH | 2202 | 26.392 | 3.610 | 27.263 | 1.00 | 40.02 | | |
| ANISOU | 2539 | OH2 | HOH | 2202 | 3983 | 4878 | 6343 | 1531 | -1650 | -566 | |
| ATOM | 2540 | OH2 | HOH | 2203 | 24.061 | 34.160 | 31.029 | 1.00 | 64.19 | | |
| ANISOU | 2540 | OH2 | HOH | 2203 | 7352 | 8511 | 8525 | -280 | 426 | 1171 | |
| ATOM | 2541 | OH2 | HOH | 2204 | 2.116 | 45.934 | 9.846 | 1.00 | 44.68 | | |
| ANISOU | 2541 | OH2 | HOH | 2204 | 6736 | 4342 | 5899 | -650 | -622 | -484 | |
| ATOM | 2542 | OH2 | HOH | 2205 | 6.445 | 43.337 | 4.182 | 1.00 | 61.78 | | |
| ANISOU | 2542 | OH2 | HOH | 2205 | 8972 | 7223 | 7277 | 1088 | -879 | -922 | |
| ATOM | 2543 | OH2 | HOH | 2206 | 10.542 | 43.839 | 0.866 | 1.00 | 41.73 | | |
| ANISOU | 2543 | OH2 | HOH | 2206 | 5634 | 5406 | 4817 | -452 | -745 | -710 | |
| ATOM | 2544 | OH2 | HOH | 2207 | 14.080 | 18.324 | 4.687 | 1.00 | 51.79 | | |
| ANISOU | 2544 | OH2 | HOH | 2207 | 7044 | 6269 | 6363 | 824 | 1136 | -1751 | |
| ATOM | 2545 | OH2 | HOH | 2208 | 15.234 | 47.925 | 10.270 | 1.00 | 46.92 | | |
| ANISOU | 2545 | OH2 | HOH | 2208 | 7004 | 6456 | 4368 | -1808 | 1022 | -870 | |
| ATOM | 2546 | OH2 | HOH | 2209 | 16.967 | 45.194 | 9.967 | 1.00 | 47.86 | | |
| ANISOU | 2546 | OH2 | HOH | 2209 | 6299 | 5224 | 6664 | -1081 | 1350 | 2059 | |
| ATOM | 2547 | OH2 | HOH | 2210 | 15.117 | 44.426 | 8.848 | 1.00 | 49.55 | | |
| ANISOU | 2547 | OH2 | HOH | 2210 | 7681 | 6916 | 4228 | 537 | 429 | 2327 | |
| ATOM | 2548 | OH2 | HOH | 2211 | 12.361 | 44.070 | 7.558 | 1.00 | 44.73 | | |
| ANISOU | 2548 | OH2 | HOH | 2211 | 5836 | 5538 | 5622 | 45 | 547 | -1911 | |
| ATOM | 2549 | OH2 | HOH | 2212 | 18.430 | 36.878 | 14.596 | 1.00 | 15.94 | | |
| ANISOU | 2549 | OH2 | HOH | 2212 | 2599 | 1411 | 2047 | 20 | 306 | 176 | |
| ATOM | 2550 | OH2 | HOH | 2213 | 1.950 | 23.756 | 18.336 | 1.00 | 54.26 | | |
| ANISOU | 2550 | OH2 | HOH | 2213 | 7029 | 7331 | 6257 | -368 | 1018 | -1458 | |
| ATOM | 2551 | OH2 | HOH | 2214 | 4.070 | 23.111 | 18.945 | 1.00 | 22.11 | | |
| ANISOU | 2551 | OH2 | HOH | 2214 | 4024 | 1919 | 2458 | -603 | -473 | 251 | |
| ATOM | 2552 | OH2 | HOH | 2215 | -4.930 | 30.563 | 15.734 | 1.00 | 47.61 | | |
| ANISOU | 2552 | OH2 | HOH | 2215 | 5138 | 6978 | 5972 | -1286 | 1047 | -490 | |
| ATOM | 2553 | OH2 | HOH | 2216 | 3.493 | 39.603 | 19.806 | 1.00 | 79.97 | | |
| ANISOU | 2553 | OH2 | HOH | 2216 | 9936 | 10609 | 9839 | 1122 | 2485 | -126 | |
| ATOM | 2554 | OH2 | HOH | 2217 | 8.338 | 37.431 | 20.460 | 1.00 | 40.15 | | |
| ANISOU | 2554 | OH2 | HOH | 2217 | 5741 | 4899 | 4614 | -770 | -483 | -246 | |
| ATOM | 2555 | OH2 | HOH | 2218 | 14.473 | 35.604 | 35.443 | 1.00 | 60.51 | | |
| ANISOU | 2555 | OH2 | HOH | 2218 | 9411 | 6844 | 6735 | 1048 | -373 | -2889 | |
| ATOM | 2556 | OH2 | HOH | 2219 | 21.237 | 38.091 | 20.801 | 1.00 | 53.48 | | |
| ANISOU | 2556 | OH2 | HOH | 2219 | 5494 | 7528 | 7298 | -45 | 234 | 965 | |
| ATOM | 2557 | OH2 | HOH | 2220 | 17.262 | 37.265 | 23.919 | 1.00 | 29.72 | | |
| ANISOU | 2557 | OH2 | HOH | 2220 | 2730 | 2018 | 6544 | -555 | -547 | -486 | |
| ATOM | 2558 | OH2 | HOH | 2221 | 31.875 | 27.365 | 22.361 | 1.00 | 38.97 | | |
| ANISOU | 2558 | OH2 | HOH | 2221 | 4117 | 5587 | 5101 | 76 | 284 | 2321 | |
| ATOM | 2559 | OH2 | HOH | 2222 | 33.178 | 17.244 | 20.397 | 1.00 | 27.94 | | |
| ANISOU | 2559 | OH2 | HOH | 2222 | 2657 | 4414 | 3543 | 1272 | -969 | -1929 | |
| ATOM | 2560 | OH2 | HOH | 2223 | 34.367 | 12.125 | 22.041 | 1.00 | 34.43 | | |
| ANISOU | 2560 | OH2 | HOH | 2223 | 4472 | 3185 | 5425 | 358 | -1759 | -77 | |
| ATOM | 2561 | OH2 | HOH | 2224 | 0.095 | 32.901 | 35.929 | 1.00 | 57.21 | | |
| ANISOU | 2561 | OH2 | HOH | 2224 | 7358 | 7653 | 6727 | -800 | 2273 | -457 | |
| ATOM | 2562 | OH2 | HOH | 2225 | -1.851 | 34.924 | 31.401 | 1.00 | 60.17 | | |
| ANISOU | 2562 | OH2 | HOH | 2225 | 8741 | 6000 | 8122 | 1146 | -1427 | 121 | |
| ATOM | 2563 | OH2 | HOH | 2226 | 1.338 | 35.092 | 31.643 | 1.00 | 42.29 | | |
| ANISOU | 2563 | OH2 | HOH | 2226 | 7071 | 3923 | 5073 | 532 | 816 | 457 | |
| ATOM | 2564 | OH2 | HOH | 2227 | 4.978 | 19.377 | 10.874 | 1.00 | 63.72 | | |
| ANISOU | 2564 | OH2 | HOH | 2227 | 8573 | 7361 | 8277 | 1912 | -1119 | 1781 | |
| ATOM | 2565 | OH2 | HOH | 2228 | 20.151 | 13.048 | 12.891 | 1.00 | 51.34 | | |
| ANISOU | 2565 | OH2 | HOH | 2228 | 7243 | 8866 | 3398 | -155 | 176 | -2115 | |
| ATOM | 2566 | OH2 | HOH | 2229 | 3.446 | 6.655 | 10.378 | 1.00 | 43.66 | | |
| ANISOU | 2566 | OH2 | HOH | 2229 | 5444 | 4844 | 6301 | 183 | -922 | 1114 | |
| ATOM | 2567 | OH2 | HOH | 2230 | -1.076 | 11.025 | 16.564 | 1.00 | 49.47 | | |
| ANISOU | 2567 | OH2 | HOH | 2230 | 6690 | 6163 | 5945 | -4 | 453 | -139 | |
| ATOM | 2568 | OH2 | HOH | 2231 | -2.344 | 12.956 | 18.471 | 1.00 | 51.91 | | |
| ANISOU | 2568 | OH2 | HOH | 2231 | 5617 | 6959 | 7149 | -2077 | -1139 | -224 | |
| ATOM | 2569 | OH2 | HOH | 2232 | -3.012 | 10.591 | 30.199 | 1.00 | 23.98 | | |
| ANISOU | 2569 | OH2 | HOH | 2232 | 2569 | 2824 | 3716 | -613 | -569 | -357 | |
| ATOM | 2570 | OH2 | HOH | 2233 | 13.701 | 4.031 | 40.758 | 1.00 | 36.30 | | |
| ANISOU | 2570 | OH2 | HOH | 2233 | 4928 | 4518 | 4348 | -2174 | -1320 | 188 | |
| ATOM | 2571 | OH2 | HOH | 2234 | 15.955 | 2.640 | 38.888 | 1.00 | 28.93 | | |
| ANISOU | 2571 | OH2 | HOH | 2234 | 2375 | 3941 | 4675 | -115 | -130 | 1875 | |
| ATOM | 2572 | OH2 | HOH | 2235 | 20.816 | 5.954 | 33.830 | 1.00 | 22.40 | | |
| ANISOU | 2572 | OH2 | HOH | 2235 | 2205 | 3848 | 2460 | 1125 | 66 | 877 | |
| ATOM | 2573 | OH2 | HOH | 2236 | 20.630 | 5.992 | 31.109 | 1.00 | 15.17 | | |
| ANISOU | 2573 | OH2 | HOH | 2236 | 1772 | 2101 | 1888 | 301 | 135 | -133 | |
| ATOM | 2574 | OH2 | HOH | 2237 | 27.186 | 13.272 | 42.527 | 1.00 | 49.68 | | |
| ANISOU | 2574 | OH2 | HOH | 2237 | 5444 | 6390 | 7043 | 143 | 903 | 1186 | |
| ATOM | 2575 | OH2 | HOH | 2238 | 25.721 | 6.161 | 42.843 | 1.00 | 45.13 | | |
| ANISOU | 2575 | OH2 | HOH | 2238 | 5375 | 7059 | 4712 | -478 | 1664 | 2478 | |
| ATOM | 2576 | OH2 | HOH | 2239 | 3.314 | 19.807 | 43.160 | 1.00 | 43.78 | | |
| ANISOU | 2576 | OH2 | HOH | 2239 | 5811 | 6204 | 4619 | 251 | 129 | -1537 | |
| ATOM | 2577 | OH2 | HOH | 2240 | 4.297 | 27.027 | 37.081 | 1.00 | 17.90 | | |
| ANISOU | 2577 | OH2 | HOH | 2240 | 1612 | 1958 | 3230 | 5 | -222 | 859 | |
| ATOM | 2578 | OH2 | HOH | 2241 | 16.988 | 23.992 | 46.245 | 1.00 | 52.67 | | |
| ANISOU | 2578 | OH2 | HOH | 2241 | 7222 | 5800 | 6992 | 116 | -530 | -1014 | |
| ATOM | 2579 | OH2 | HOH | 2242 | -2.377 | 39.990 | 14.418 | 1.00 | 41.88 | | |
| ANISOU | 2579 | OH2 | HOH | 2242 | 4845 | 5991 | 5078 | -1456 | -193 | -1024 | |
| ATOM | 2580 | OH2 | HOH | 2243 | 3.130 | 42.450 | 7.749 | 1.00 | 42.10 | | |
| ANISOU | 2580 | OH2 | HOH | 2243 | 6306 | 4786 | 4904 | 1497 | -47 | -377 | |
| ATOM | 2581 | OH2 | HOH | 2244 | 2.997 | 44.684 | 6.699 | 1.00 | 56.28 | | |
| ANISOU | 2581 | OH2 | HOH | 2244 | 6235 | 7348 | 7800 | 609 | -1439 | 1125 | |
| ATOM | 2582 | OH2 | HOH | 2245 | 22.890 | 45.174 | 0.987 | 1.00 | 48.36 | | |
| ANISOU | 2582 | OH2 | HOH | 2245 | 5179 | 6800 | 6398 | -116 | -592 | 49 | |
| ATOM | 2583 | OH2 | HOH | 2246 | 15.568 | 38.121 | 17.923 | 1.00 | 30.65 | | |
| ANISOU | 2583 | OH2 | HOH | 2246 | 4597 | 3182 | 3868 | -409 | 196 | 287 | |
| ATOM | 2584 | OH2 | HOH | 2247 | 16.944 | 38.683 | 16.010 | 1.00 | 36.47 | | |
| ANISOU | 2584 | OH2 | HOH | 2247 | 4444 | 4968 | 4443 | 205 | 238 | -1717 | |
| ATOM | 2585 | OH2 | HOH | 2248 | 6.823 | 40.208 | 15.789 | 1.00 | 55.71 | | |
| ANISOU | 2585 | OH2 | HOH | 2248 | 6916 | 7528 | 6725 | 130 | -803 | 312 | |
| ATOM | 2586 | OH2 | HOH | 2249 | 10.350 | 40.659 | 13.664 | 1.00 | 42.28 | | |
| ANISOU | 2586 | OH2 | HOH | 2249 | 6130 | 4786 | 5150 | 530 | -1597 | 354 | |
| ATOM | 2587 | OH2 | HOH | 2250 | 22.047 | 15.825 | 15.658 | 1.00 | 46.32 | | |
| ANISOU | 2587 | OH2 | HOH | 2250 | 6365 | 6760 | 4473 | -442 | 1535 | -48 | |
| ATOM | 2588 | OH2 | HOH | 2251 | 15.675 | 3.412 | 16.780 | 1.00 | 55.34 | | |
| ANISOU | 2588 | OH2 | HOH | 2251 | 7688 | 6918 | 6420 | 188 | -1221 | -87 | |
| ATOM | 2589 | OH2 | HOH | 2252 | 14.677 | 5.481 | 14.790 | 1.00 | 45.59 | | |
| ANISOU | 2589 | OH2 | HOH | 2252 | 6170 | 4087 | 7064 | 112 | -194 | -788 | |
| ATOM | 2590 | OH2 | HOH | 2253 | 6.512 | 4.458 | 37.967 | 1.00 | 16.65 | | |
| ANISOU | 2590 | OH2 | HOH | 2253 | 1744 | 1336 | 3246 | -7 | 65 | -45 | |
| ATOM | 2591 | OH2 | HOH | 2254 | 27.055 | 21.820 | 46.605 | 1.00 | 50.56 | | |
| ANISOU | 2591 | OH2 | HOH | 2254 | 5419 | 7304 | 6488 | -461 | 757 | 1307 | |
| ATOM | 2592 | OH2 | HOH | 2255 | 34.498 | 18.342 | 18.221 | 1.00 | 54.53 | | |
| ANISOU | 2592 | OH2 | HOH | 2255 | 5989 | 6366 | 8364 | -2444 | -1601 | -355 | |
| ATOM | 2593 | OH2 | HOH | 2256 | 35.334 | 16.235 | 18.626 | 1.00 | 54.98 | | |
| ANISOU | 2593 | OH2 | HOH | 2256 | 5757 | 8014 | 7121 | -515 | -1092 | 228 | |
| ATOM | 2594 | OH2 | HOH | 2257 | 31.131 | 14.648 | 22.506 | 1.00 | 36.00 | | |
| ANISOU | 2594 | OH2 | HOH | 2257 | 3978 | 3116 | 6586 | -110 | -1576 | -1470 | |
| ATOM | 2595 | OH2 | HOH | 2258 | 33.181 | 10.274 | 21.383 | 1.00 | 44.81 | | |
| ANISOU | 2595 | OH2 | HOH | 2258 | 5022 | 5790 | 6214 | -1090 | 923 | -1286 | |
| ATOM | 2596 | OH2 | HOH | 2259 | 24.252 | 4.912 | 23.515 | 1.00 | 45.87 | | |
| ANISOU | 2596 | OH2 | HOH | 2259 | 7208 | 4670 | 5550 | 1464 | 1751 | -896 | |
| ATOM | 2597 | OH2 | HOH | 2260 | 1.663 | 22.210 | 43.867 | 1.00 | 70.15 | | |
| ANISOU | 2597 | OH2 | HOH | 2260 | 8197 | 8982 | 9474 | 68 | 337 | -2 | |
| ATOM | 2598 | OH2 | HOH | 2261 | -0.515 | 19.931 | 40.430 | 1.00 | 39.90 | | |
| ANISOU | 2598 | OH2 | HOH | 2261 | 5209 | 3243 | 6710 | 813 | 217 | 505 | |
| ATOM | 2599 | OH2 | HOH | 2262 | -2.457 | 19.610 | 38.621 | 1.00 | 41.31 | | |
| ANISOU | 2599 | OH2 | HOH | 2262 | 4325 | 6734 | 4637 | -249 | -924 | -1772 | |
| ATOM | 2600 | OH2 | HOH | 2263 | -0.166 | 21.593 | 35.310 | 1.00 | 28.83 | | |
| ANISOU | 2600 | OH2 | HOH | 2263 | 4611 | 2357 | 3987 | 429 | -1182 | -689 | |
| ATOM | 2601 | OH2 | HOH | 2264 | 13.689 | 21.920 | 52.636 | 1.00 | 44.27 | | |
| ANISOU | 2601 | OH2 | HOH | 2264 | 4121 | 5106 | 7593 | 836 | -564 | 213 | |
| ATOM | 2602 | OH2 | HOH | 2265 | 19.920 | 1.651 | 29.589 | 1.00 | 21.53 | | |
| ANISOU | 2602 | OH2 | HOH | 2265 | 2354 | 2225 | 3601 | -47 | -357 | -16 | |
| ATOM | 2603 | OH2 | HOH | 2266 | 19.598 | 3.372 | 31.578 | 1.00 | 40.81 | | |
| ANISOU | 2603 | OH2 | HOH | 2266 | 5438 | 4607 | 5460 | 961 | -1985 | 1076 | |
| ATOM | 2604 | OH2 | HOH | 2267 | 22.242 | 1.810 | 32.147 | 1.00 | 92.82 | | |
| ANISOU | 2604 | OH2 | HOH | 2267 | 12020 | 11512 | 11735 | 546 | 661 | -132 | |
| ATOM | 2605 | OH2 | HOH | 2268 | 14.772 | -2.224 | 28.848 | 1.00 | 24.79 | | |
| ANISOU | 2605 | OH2 | HOH | 2268 | 3712 | 2086 | 3622 | 54 | -457 | 149 | |
| ATOM | 2606 | OH2 | HOH | 2269 | 17.749 | -2.449 | 30.658 | 1.00 | 57.30 | | |
| ANISOU | 2606 | OH2 | HOH | 2269 | 8357 | 6705 | 6708 | 1697 | -64 | 1200 | |
| ATOM | 2607 | OH2 | HOH | 2270 | 10.519 | 39.894 | 17.375 | 1.00 | 62.82 | | |
| ANISOU | 2607 | OH2 | HOH | 2270 | 8324 | 6608 | 8938 | 1679 | 1813 | -368 | |
| ATOM | 2608 | OH2 | HOH | 2271 | 0.430 | 11.820 | 14.684 | 1.00 | 45.51 | | |
| ANISOU | 2608 | OH2 | HOH | 2271 | 6282 | 5127 | 5882 | -400 | -439 | -2108 | |
| ATOM | 2609 | OH2 | HOH | 2272 | 5.668 | 12.613 | 13.139 | 1.00 | 70.99 | | |
| ANISOU | 2609 | OH2 | HOH | 2272 | 8991 | 8796 | 9187 | 274 | 94 | -923 | |
| ATOM | 2610 | OH2 | HOH | 2273 | -8.237 | 7.562 | 23.932 | 1.00 | 52.58 | | |
| ANISOU | 2610 | OH2 | HOH | 2273 | 7204 | 6080 | 6693 | -1479 | -2517 | -908 | |
| ATOM | 2611 | OH2 | HOH | 2274 | -5.548 | 10.089 | 31.128 | 1.00 | 50.96 | | |
| ANISOU | 2611 | OH2 | HOH | 2274 | 7124 | 5031 | 7208 | 1698 | -778 | -200 | |
| ATOM | 2612 | OH2 | HOH | 2275 | 6.006 | 3.285 | 39.881 | 1.00 | 59.05 | | |
| ANISOU | 2612 | OH2 | HOH | 2275 | 5232 | 9368 | 7837 | -816 | -907 | -141 | |
| ATOM | 2613 | OH2 | HOH | 2276 | 18.329 | 3.511 | 35.187 | 1.00 | 31.14 | | |
| ANISOU | 2613 | OH2 | HOH | 2276 | 3603 | 3616 | 4612 | -55 | 174 | -18 | |
| ATOM | 2614 | OH2 | HOH | 2277 | 17.197 | 4.413 | 39.613 | 1.00 | 32.09 | | |
| ANISOU | 2614 | OH2 | HOH | 2277 | 3756 | 5397 | 3039 | 1046 | -881 | -147 | |
| ATOM | 2615 | OH2 | HOH | 2278 | 18.186 | 1.261 | 41.660 | 1.00 | 41.10 | | |
| ANISOU | 2615 | OH2 | HOH | 2278 | 5188 | 4409 | 6021 | -602 | 1680 | -703 | |
| ATOM | 2616 | OH2 | HOH | 2279 | 17.739 | 1.394 | 15.973 | 1.00 | 57.56 | | |
| ANISOU | 2616 | OH2 | HOH | 2279 | 7591 | 8946 | 5333 | -164 | 1856 | -1008 | |
| ATOM | 2617 | OH2 | HOH | 2280 | 14.323 | 0.199 | 17.194 | 1.00 | 73.92 | | |
| ANISOU | 2617 | OH2 | HOH | 2280 | 9181 | 9903 | 9003 | 502 | -21 | -2853 | |
| ATOM | 2618 | OH2 | HOH | 2281 | 8.551 | 41.455 | 14.905 | 1.00 | 118.27 | | |
| ANISOU | 2618 | OH2 | HOH | 2281 | 15164 | 14719 | 15054 | -78 | -125 | -146 | |
| ATOM | 2619 | OH2 | HOH | 2282 | 12.963 | 40.910 | 13.215 | 1.00 | 50.68 | | |
| ANISOU | 2619 | OH2 | HOH | 2282 | 5704 | 7547 | 6006 | -155 | 1499 | 562 | |
| ATOM | 2620 | OH2 | HOH | 2283 | 11.294 | 41.629 | 11.806 | 1.00 | 66.06 | | |
| ANISOU | 2620 | OH2 | HOH | 2283 | 7867 | 8521 | 8711 | -456 | 228 | 379 | |
| ATOM | 2621 | OH2 | HOH | 2284 | 16.077 | 39.615 | -1.846 | 1.00 | 40.93 | | |
| ANISOU | 2621 | OH2 | HOH | 2284 | 6295 | 4439 | 4819 | 1068 | -22 | -778 | |
| ATOM | 2622 | OH2 | HOH | 2285 | 26.136 | 25.331 | 7.947 | 1.00 | 44.29 | | |
| ANISOU | 2622 | OH2 | HOH | 2285 | 4768 | 6987 | 5074 | 212 | -40 | -211 | |
| ATOM | 2623 | OH2 | HOH | 2286 | 21.800 | 18.302 | 10.818 | 1.00 | 62.90 | | |
| ANISOU | 2623 | OH2 | HOH | 2286 | 7374 | 8435 | 8089 | -341 | -1040 | 217 | |
| ATOM | 2624 | OH2 | HOH | 2287 | 20.645 | 15.005 | 9.508 | 1.00 | 65.90 | | |
| ANISOU | 2624 | OH2 | HOH | 2287 | 7740 | 8042 | 9257 | 721 | -271 | 672 | |
| ATOM | 2625 | OH2 | HOH | 2288 | 18.387 | 3.421 | 15.099 | 1.00 | 49.21 | | |
| ANISOU | 2625 | OH2 | HOH | 2288 | 6380 | 6528 | 5791 | -510 | 971 | -48 | |
| ATOM | 2626 | OH2 | HOH | 2289 | 24.587 | 9.772 | 18.510 | 1.00 | 57.54 | | |
| ANISOU | 2626 | OH2 | HOH | 2289 | 6543 | 7549 | 7769 | -419 | -226 | 1288 | |
| ATOM | 2627 | OH2 | HOH | 2290 | 25.543 | 11.619 | 19.500 | 1.00 | 71.38 | | |
| ANISOU | 2627 | OH2 | HOH | 2290 | 8517 | 9042 | 9563 | 1529 | -52 | 864 | |
| ATOM | 2628 | OH2 | HOH | 2291 | 33.783 | 12.038 | 33.174 | 1.00 | 35.32 | | |
| ANISOU | 2628 | OH2 | HOH | 2291 | 3767 | 4658 | 4996 | -371 | 1350 | -758 | |
| ATOM | 2629 | OH2 | HOH | 2292 | -3.299 | 8.740 | 32.299 | 1.00 | 39.11 | | |
| ANISOU | 2629 | OH2 | HOH | 2292 | 3342 | 5678 | 5839 | -303 | 342 | -514 | |
| ATOM | 2630 | OH2 | HOH | 2293 | 7.138 | -6.198 | 35.152 | 1.00 | 36.60 | | |
| ANISOU | 2630 | OH2 | HOH | 2293 | 5059 | 3091 | 5757 | -1009 | 1251 | -1152 | |
| ATOM | 2631 | OH2 | HOH | 2294 | 0.994 | 4.645 | 36.942 | 1.00 | 50.65 | | |
| ANISOU | 2631 | OH2 | HOH | 2294 | 6021 | 6739 | 6487 | -816 | 1270 | -1075 | |
| ATOM | 2632 | OH2 | HOH | 2295 | -0.106 | 6.688 | 39.157 | 1.00 | 49.06 | | |
| ANISOU | 2632 | OH2 | HOH | 2295 | 4516 | 7809 | 6315 | -1468 | 564 | -1932 | |
| ATOM | 2633 | OH2 | HOH | 2296 | 21.634 | 37.338 | 18.355 | 1.00 | 50.95 | | |
| ANISOU | 2633 | OH2 | HOH | 2296 | 7051 | 5878 | 6429 | -955 | -1767 | -1051 | |
| ATOM | 2634 | OH2 | HOH | 2297 | 7.045 | 37.657 | 18.368 | 1.00 | 44.09 | | |
| ANISOU | 2634 | OH2 | HOH | 2297 | 4574 | 4722 | 7459 | 242 | 491 | 593 | |
| ATOM | 2635 | OH2 | HOH | 2298 | 18.268 | 34.358 | 30.180 | 1.00 | 37.05 | | |
| ANISOU | 2635 | OH2 | HOH | 2298 | 4092 | 6214 | 3772 | -749 | 165 | 411 | |
| ATOM | 2636 | OH2 | HOH | 2299 | 7.692 | 41.068 | 10.258 | 1.00 | 51.21 | | |
| ANISOU | 2636 | OH2 | HOH | 2299 | 5855 | 8339 | 5263 | 441 | 1330 | -2664 | |
| ATOM | 2637 | OH2 | HOH | 2300 | 6.986 | -7.646 | 33.375 | 1.00 | 32.81 | | |
| ANISOU | 2637 | OH2 | HOH | 2300 | 4060 | 3716 | 4691 | 1275 | -2322 | -704 | |
| ATOM | 2638 | OH2 | HOH | 2301 | -8.782 | 30.474 | 8.939 | 1.00 | 40.24 | | |
| ANISOU | 2638 | OH2 | HOH | 2301 | 4217 | 5588 | 5484 | -1619 | 54 | 693 | |
| ATOM | 2639 | OH2 | HOH | 2302 | 13.681 | 34.760 | -0.596 | 1.00 | 52.86 | | |
| ANISOU | 2639 | OH2 | HOH | 2302 | 6814 | 7897 | 5372 | -621 | -1484 | 1274 | |
| ATOM | 2640 | OH2 | HOH | 2303 | 23.230 | 42.983 | 0.375 | 1.00 | 49.35 | | |
| ANISOU | 2640 | OH2 | HOH | 2303 | 4517 | 8177 | 6057 | 139 | 575 | 1267 | |
| ATOM | 2641 | OH2 | HOH | 2305 | -7.452 | 29.742 | 10.799 | 1.00 | 46.13 | | |
| ANISOU | 2641 | OH2 | HOH | 2305 | 5250 | 5278 | 7000 | 2086 | -1259 | 578 | |
| ATOM | 2642 | OH2 | HOH | 2306 | -6.412 | 30.758 | 28.400 | 1.00 | 53.77 | | |
| ANISOU | 2642 | OH2 | HOH | 2306 | 7895 | 4856 | 7680 | 1834 | -2211 | 1056 | |
| ATOM | 2643 | OH2 | HOH | 2307 | 24.528 | 7.914 | 33.885 | 1.00 | 52.07 | | |
| ANISOU | 2643 | OH2 | HOH | 2307 | 5908 | 6787 | 7090 | 3063 | -2150 | 1040 | |
| ATOM | 2644 | OH2 | HOH | 2308 | 3.162 | 37.461 | 31.203 | 1.00 | 42.70 | | |
| ANISOU | 2644 | OH2 | HOH | 2308 | 3898 | 5376 | 6950 | 553 | 123 | -1855 | |
| ATOM | 2645 | OH2 | HOH | 2309 | 27.344 | 34.345 | 6.979 | 1.00 | 53.05 | | |
| ANISOU | 2645 | OH2 | HOH | 2309 | 7198 | 6157 | 6803 | 524 | 2 | 1342 | |
| ATOM | 2646 | OH2 | HOH | 2310 | 8.352 | 28.846 | 0.587 | 1.00 | 48.08 | | |
| ANISOU | 2646 | OH2 | HOH | 2310 | 7283 | 6918 | 4066 | -1595 | -1926 | -1593 | |
| ATOM | 2647 | OH2 | HOH | 2311 | 27.043 | 12.975 | 47.864 | 1.00 | 57.97 | | |
| ANISOU | 2647 | OH2 | HOH | 2311 | 7623 | 7911 | 6494 | -2096 | 1729 | 2454 | |
| ATOM | 2648 | OH2 | HOH | 2312 | 9.482 | 42.324 | -1.614 | 1.00 | 44.75 | | |
| ANISOU | 2648 | OH2 | HOH | 2312 | 5120 | 4765 | 7117 | 308 | 1106 | 1780 | |
| ATOM | 2649 | OH2 | HOH | 2313 | -8.801 | 35.732 | 7.848 | 1.00 | 57.76 | | |
| ANISOU | 2649 | OH2 | HOH | 2313 | 5517 | 7910 | 8518 | 616 | -382 | -1402 | |
| ATOM | 2650 | OH2 | HOH | 2314 | -0.455 | 18.736 | 16.444 | 1.00 | 43.01 | | |
| ANISOU | 2650 | OH2 | HOH | 2314 | 5291 | 6758 | 4295 | 749 | -1815 | 729 | |
| ATOM | 2651 | OH2 | HOH | 2315 | 7.574 | -1.992 | 20.640 | 1.00 | 46.37 | | |
| ANISOU | 2651 | OH2 | HOH | 2315 | 5569 | 5373 | 6675 | -156 | -41 | 1686 | |
| ATOM | 2652 | OH2 | HOH | 2318 | 3.833 | 31.378 | 36.561 | 1.00 | 53.11 | | |
| ANISOU | 2652 | OH2 | HOH | 2318 | 6350 | 5805 | 8024 | 1840 | -613 | -1091 | |

The variations in coordinates discussed above may be generated because of mathematical manipulations of the HGFR-Compound **1** complex structure coordinates. For example, the structure coordinates set forth in Table 1 could be manipulated by crystallographic permutations of the structure coordinates, fractionalization of the structure coordinates, integer additions or subtractions to sets of the structure coordinates, or combinations thereof.

Alternatively, modifications in the crystal structure due to mutations, additions, substitutions, and/or deletions of amino acids, or other changes in any of the components that make up the crystal could also account for variations in structure coordinates. If such variations are within an acceptable standard error as compared to the original coordinates, the resulting three-dimensional shape is considered to be the same. Thus, for example, a ligand that bound to the binding pocket of the HGFR domain would also be expected to bind to another binding pocket whose structure coordinates when compared to those described have a root mean square difference of equal to or less than about 1.5 Å, more preferably less than about 1.0 Å, and even more preferably less than about 0.5 Å, from the backbone atoms.

Various computational analyses can be performed to determine whether a polypeptide or the binding pocket portion thereof is sufficiently similar to the HGFR binding pocket as described herein. Such analyses may be carried out through the use of known software applications, such as ProMod, SWISS-MODEL (Swiss Institute of Bioinformatics), and the Molecular Similarity application of QUANTA (Accelrys, Inc., San Diego, CA). Programs, such as QUANTA permit comparisons between different structures, different conformations of the same structure, and different parts of the same structure. Comparison of structures using such computer software may involve the following steps: 1) loading the structures to be compared; 2) defining the atom equivalencies in the structures; 3) performing a fitting operation; and 4) analyzing the results. Each structure is identified by a name. One structure is identified as the target (i.e., the fixed structure); all remaining structures are working structures (i.e., moving structures). Since atom equivalency with QUANTA is defined by user input, for the purpose of this invention applicants define equivalent atoms as protein backbone atoms (N, Cα, C, and O) for all conserved residues between the two structures being compared. We will also consider only rigid fitting operations. When a rigid fitting method is used, the working structure is translated and rotated to obtain an optimum fit with the target structure. The fitting operation uses an algorithm that computes the optimum translation and rotation to be applied to the moving structure, such that the root mean square difference of the fit over the specified pairs of equivalent atoms is an absolute minimum. This number, given in angstroms (Å), is reported by software applications, such as QUANTA.

For the purpose of this invention, any HGFR molecule or molecular complex or binding pocket thereof that has a root mean square deviation of conserved residue backbone atoms (N, Cα, C, O) of less than about 1.5 Å, more preferably less than about 1.0 Å, and even more preferably less than about 0.5 Å, when superimposed on the relevant backbone atoms described by structure coordinates listed in Table 1 are considered equivalent.

The term "root mean square deviation" means the square root of the arithmetic mean of the squares of the deviations from the mean. It is a way to express the deviation or variation from a trend or object. For purposes of this invention, the "root mean square deviation" defines the variation in the backbone of a protein from the backbone of the HGFR polypeptides of the invention or the HGFR substrate-binding domain portion thereof, as defined by the structure coordinates described herein.

### E. Computers, Computer Software, Computer Modeling

One embodiment of the invention includes a computer for producing a three-dimensional representation of the HGFR domain of the polypeptides of the invention and complexes of the HGFR domain of such polypeptides with a ligand.

Computers are known in the art and may include a central processing unit (CPU), a working memory, which can be random-access memory, core memory, mass-storage memory, or combinations of all of the aforementioned. The CPU may encode one or more programs. Computers may also include display, and input and output devices, such as one or more cathode-ray tube display terminals, keyboards, modems, input lines and output lines. Persons skilled in the computer art will understand that many variations of a computer exist in the art and all such variations are applicable to the present invention. Further, said computers may be networked to computer servers (the machine on which large calculations can be run in batch), and file servers (the main machine for all the centralized databases).

Machine-readable media containing data, such as the crystal structure coordinates of the polypeptides of the invention may be inputted using various hardware, including modems, CD-ROM drives, disk drives, or keyboards.

Output hardware, such as a CRT display terminal may be used for displaying a graphical representation of the HGFR polypeptide of the invention or the HGFR substrate-binding domain of these polypeptides using a program such as QUANTA. Output hardware may also include a printer, and disk drives.

The CPU coordinates the use of the various input and output devices, coordinates data accesses from storage and accesses to and from working memory, and determines the sequence of data processing steps. A number of programs may be used to process the machine-readable data of this invention. Such programs are discussed in reference to the computational methods of drug discovery as described herein.

Thus, one embodiment of the present invention includes X-ray coordinate data capable of being processed into a three dimensional graphical display of a molecule or molecular complex that comprises an HGFR-like substrate-binding pocket stored in a machine-readable storage medium. The three-dimensional structure of a molecule or molecular complex comprising an HGFR-like substrate-binding pocket may be used for a variety of purposes, such as drug discovery.

For example, the three- dimensional structure derived from the structure coordinate data may be computationally evaluated for its ability to associate with chemical entities. Such entities would be potential drug candidates and would be evaluated for their ability to inhibit or modulate the activity of HGFR.

The term "chemical entity," as used herein, refers to chemical compounds, complexes of at least two chemical compounds, and fragments of such compounds or complexes.

Throughout this section, discussions about the ability of an entity to bind to, or associate with an HGFR-like substrate-binding domain refer to features of the entity alone. Assays to determine if a compound binds to HGFR are known in the art and are exemplified herein.

The design of compounds that bind to HGFR-like substrate-binding domains according to this invention may involve consideration of two factors. First, the entity must be capable of physically and structurally associating with some or the entire HGFR-like substrate-binding domain. Non-covalent molecular interactions important in this association include hydrogen bonding, van der Waals interactions, hydrophobic interactions and electrostatic interactions.

The term "associating with" refers to a condition of proximity between a chemical entity or compound, or portions thereof, and a binding pocket or binding site on a protein. The association may be non-covalent, for example, wherein the juxtaposition is energetically favored by hydrogen bonding of van der Waals or electrostatic interactions, or it may be covalent.

Second, the entity must be able to assume a conformation that allows it to associate with the HGFR-like substrate-binding domain directly. Although certain portions of the entity will not directly participate in these associations, those portions of the entity may still influence the overall conformation of the molecule. This, in turn, may have a significant impact on potency. Such conformational requirements include the overall three-dimensional structure and orientation of the chemical entity in relation to all or a portion of the binding pocket, or the spacing between functional groups of an entity comprising several chemical entities that directly interact with the HGFR-like-binding pocket or homologues thereof.

The potential inhibitory or binding effect of a chemical entity on an HGFR-like substrate-binding domain may be analyzed prior to its actual synthesis and testing through the use of computer-modeling techniques. If the theoretical structure of the given entity suggests insufficient interaction and association between it and the HGFR-like-binding pocket, further testing of the entity may not be necessary. However, if computer modeling indicates a strong interaction, the molecule can be synthesized and tested for its ability to bind to an HGFR-like binding pocket. This may be achieved by testing the ability of the molecule to modulate HGFR activity using the assays described in Examples 3 and 4. Using this scheme, the synthesis of compounds with poor binding activities can be avoided.

A potential inhibitor of an HGFR-like substrate-binding domain may be computationally evaluated by means of a series of steps in which chemical entities or fragments are screened and selected for their ability to associate with the HGFR-like binding pockets. One skilled in the art may use one of several methods to screen chemical entities or fragments for their ability to associate with an HGFR-like substrate-binding domain. For example, one skilled in the art may visually inspect an HGFR-like substrate-binding pocket on a computer screen based on the HGFR structure coordinates reported in Table 1 or other coordinates which define a similar shape generated from the machine-readable storage medium. Selected fragments or chemical entities may then be positioned in a variety of orientations, or docked, within that binding pocket as defined *supra*. Docking may be accomplished using software such as Quanta and Sybyl, followed by energy minimization and molecular dynamics with standard molecular mechanics force fields, such as CHARMM and AMBER. Specialized computer programs to assist in the process of selecting fragments or chemical entities include the following:
1. GRID (Goodford, *J. Med. Chem.* 28:849-857 (1985)). GRID is available from the Oxford University, Oxford, UK.
2. MCSS (Miranker et al., *Proteins: Struct. Funct. and Genet*. 11:29-34 (1991)). MCSS is available from Accelrys, Inc., San Diego, CA.
3. AUTODOCK (Goodsell et al., *Proteins: Struct. Funct. and Genet.* 8:195-20 (1990)). AUTODOCK is available from the Scripps Research Institute, La Jolla, Calif.
4. DOCK (Kuntz et al., *J. Mol. Biol.,* 161:269-288 (1982)). DOCK is available from the University of California, San Francisco, CA.
5. GOLD (Jones et al., *J. Mol. Biol* 267:727-748 (1997)). GOLD is available from the Cambridge Crystallographic Data Centre, UK.
6. GLIDE (Eldridge et al., *J. Comput. Aided Mol. Des.* 11:425-445 (1997)). Glide is available from Schrödinger, Portland OR)
7. AGDOCK (Gehlahaar et al., *Chemsitry & Biol.* 2:317-324 (1995)). In-house software Agouron Pharmaceuticals, Inc./A Pfizer Co.

Once suitable chemical entities or fragments have been selected, they can be assembled into a single compound or complex. Assembly may be preceded by visual inspection of the relationship of the fragments to each other on the three-dimensional image displayed on a computer screen in relation to the structure coordinates of HGFR or an HGFR-ligand complex. This can be followed by manual model building using software such as Quanta or Sybyl (Tripos Associates, St. Louis, Mo.) Useful programs to aid one of skill in the art in connecting the individual chemical entities or fragments include the following:
1. CAVEAT (Bartlett et al, *Molecular Recognition in Chemical and Biological Problems",* Special Pub., Royal Chem. Soc., 78:182-196 (1989); Lauri et al., *J*. *Comput. Aided Mol. Des.* 8:51-66 (1994)). CAVEAT is available from the University of California, Berkeley, CA.
2. 3D Database systems such as ISIS (MDL Information Systems, San Leandro, Calif.). This area is reviewed in Martin, *J. Med. Chem.* 35:2145-2154 (1992)).
3. HOOK (Eisen et al, *Proteins: Struct*., *Funct*., *Genet.,* 19:199-221 (1994)). HOOK is available from Accelrys, Inc.,San Diego, CA.

Instead of proceeding to build an inhibitor of an HGFR-like-binding pocket in a step-wise fashion one fragment or chemical entity at a time as described above, inhibitory or other HGFR-binding compounds may be designed as a whole or *de novo* using either an empty binding site or optionally including some portion(s) of a known inhibitor(s). There are many *de novo* ligand design methods including:
1. LUDI (H.-J. Bohm, *J. Comp. Aid. Molec. Design* 6:61-78 (1992). LUDI is available from Accelrys Incorporated, San Diego, Calif.
2. LEGEND (Y. Nishibata et al., *Tetrahedron* 47:8985 (1991). LEGEND is available from Accelrys Incorporated, San Diego, Calif.
3. LeapFrog (available from Tripos Associates, St. Louis, Mo.).
4. SPROUT (V. Gillet et al, *J. Comput. Aided Mol. Design* 7:127-153(1993). SPROUT is available from the University of Leeds, UK.

Other molecular modeling techniques may also be employed in accordance with this invention (see, e.g., N. C. Cohen et al., *J. Med. Chem.* 33:883-894 (1990); see also, M. A. Navia and M. A. Murcko, *Current Opinions in Structural Biology* 2:202-210 (1992); L. M. Balbes et al., *Reviews in Computational Chemistry,* Vol. 5, K. B. Lipkowitz and D. B. Boyd, Eds., VCH, New York, pp. 337-380 (1994); see also, W. C. Guida, *Curr. Opin. Struct. Biology* 4:777-781 (1994)).

Once a compound has been designed or selected by the above methods, the efficiency with which that entity may bind to an HGFR substrate-binding pocket may be tested and optimized by computational evaluation. For example, an effective HGFR substrate-binding pocket modulator must preferably demonstrate a relatively small difference in energy between its bound and free states (i.e., a small deformation energy of binding). HGFR substrate-binding pocket modulators may interact with the substrate-binding domain in more than one conformation that is similar in overall binding energy. In those cases, the deformation energy of binding is taken to be the difference between the energy of the free entity and the average energy of the conformations observed when the inhibitor binds to the protein.

An entity designed or selected as binding to an HGFR substrate-binding domain may be further computationally optimized so that in its bound state it would preferably lack repulsive electrostatic interaction with the target enzyme and with the surrounding water molecules. Such non-complementary electrostatic interactions include repulsive charge-charge, dipole-dipole and charge-dipole interactions.

Specific computer software is available in the art to evaluate compound deformation energy and electrostatic interactions. Examples of programs designed for such uses include: Gaussian (M. J. Frisch, Gaussian, Inc., Carnegie, PA); AMBER (P. A. Kollman, University of California at San Francisco); Jaguar (Schrödinger, Portland, OR); SPARTAN (Wavefunction, Inc., Irvine, CA); QUANTA/CHARMM (Accelrys, Inc., San Diego, CA.); Impact (Schrödinger, Portland, OR ); Insight II/Discover (Accelrys, Inc., San Diego, CA); MacroModel (Schrödinger, Portland, OR ); Maestro (Schrödinger, Portland, OR ); DelPhi (Accelrys, Inc., San Diego, CA); and AMSOL (Quantum Chemistry Program Exchange, Indiana University). These programs may be implemented, for instance, using a Silicon Graphics workstation, such as an Indigo² with "IMPACT©" graphics. Other hardware systems and software packages will be known to those skilled in the art.

Another approach enabled by this invention, is the computational screening of small molecule databases for chemical entities or compounds that can bind in whole, or in part, to an HGFR substrate-binding pocket. In this screening, the quality of fit of such entities to the binding site may be judged either by shape complementarity or by estimated interaction energy (E. C. Meng et al. *J. Comp. Chem*. 13:505-524 (1992)).

Binding of potential inhibitors can also be assessed biochemically, for example, using isothermal titration calorimetry. See, e.g., Holdgate, *Biotechniques* 30:164-184 (2001).

According to another embodiment, the invention provides compounds that associate with an HGFR-like substrate-binding pocket produced or identified by the methods set forth above.

The structure coordinates of the invention as set forth in Table 1 can be used to obtain structural information about another crystallized molecule or molecular complex. This may be achieved by any of a number of well-known techniques, including molecular replacement. By using molecular replacement, all or part of the structure coordinates of the HGFR polypeptide-Compound 1 complex can be used to determine the structure of a crystallized molecule or molecular complex whose structure is unknown. Molecular replacement provides an accurate estimation of the phases for an unknown structure. Phases are a factor in equations used to solve crystal structures that cannot be determined directly. Obtaining accurate values for the phases, by methods other than molecular replacement, can be more time-consuming. Using molecular replacement methods, when the crystal structure of a protein containing at least a homologous portion has been solved, the phases from the known structure can provide an estimate of the phases for the unknown structure.

The method involves generating a preliminary model of a molecule or molecular complex whose structure coordinates are unknown, by orienting and positioning the relevant portion of the HGFR-Compound 1 complex according to Table 1 within the unit cell of the crystal of the unknown molecule or molecular complex so as best to account for the observed X-ray diffraction data of the crystal of the molecule or molecular complex whose structure is unknown. Phases can then be calculated from this model and combined with the observed X-ray diffraction data amplitudes to generate an electron density map of the structure whose coordinates are unknown. This, in turn, can be subjected to any known model building and structure refinement techniques to provide a final, accurate structure of the unknown crystallized molecule or molecular complex (E. Lattman, "Use of the Rotation and Translation Functions", in *Meth. Enzymol*., 115, pp. 55-77 (1985); Rossmann, ed., "The Molecular Replacement Method", *Int. Sci. Rev. Ser.,* No. 13, Gordon & Breach, New York (1972)). Thus, the structure of any portion of any crystallized molecule or molecular complex that is sufficiently homologous to any portion of the HGFR/Ligand complex can be resolved by this method.

In another aspect, the method of molecular replacement is utilized to obtain structural information about another kinase. The structure coordinates of HGFR as provided by this invention are particularly useful in solving the structure of other isoforms of HGFR or other HGFR containing complexes.

Furthermore, the structure coordinates of HGFR as provided by this invention are useful in solving the structure of HGFR proteins that have amino acid substitutions, additions and/or deletions. These HGFR mutants may optionally be crystallized in co-complex with a chemical entity, such as a Compound 1 analogue. The crystal structures of the protein alone or a series of such complexes may then be solved by molecular replacement and compared with that of wild-type HGFR. Potential sites for modification within the various binding sites of the enzyme may thus be identified. This information provides an additional tool for determining the most efficient binding interactions, for example, increased hydrophobic interactions, between HGFR and a chemical entity.

The structure coordinates of the invention are also useful to solve the structure of crystals of HGFR or HGFR homologues co-complexed with a variety of chemical entities. This approach enables the determination of the optimal sites for interaction between chemical entities, including potential HGFR modulators with the HGFR substrate-binding site. For example, high resolution X-ray diffraction data collected from crystals exposed to different types of solvent allows the determination of where each type of solvent molecule resides. Small molecules that bind tightly to those sites can then be designed and synthesized and tested for their ability to modulate HGFR activity.

All of the complexes referred to above may be studied using well-known X-ray diffraction techniques and may be refined versus X-ray data within a range of about 1.0 Å and about 3.0 Å to an R value of about 0.20 or less using computer software, such as X-PLOR (Yale University, distributed by Accelrys, Inc.; see, e.g., Blundell & Johnson, *supra*; *Meth. Enzymol.*, vol. 114 & 115, H. W. Wyckoff et al., eds., Academic Press (1985)). This information may be used to optimize known HGFR modulators, and to design new HGFR modulators.

### EXAMPLES

### Example 1: The HGFR Kinase Domain

### 1. Identification of the catalytic Domain Sequence

From the complete protein sequence for the human HGFR available from Swissprot: Locus MET_HUMAN, accession P08581, the sequence alignment programs (BLAST and ClustalW) were used to generate multiple sequence alignments with known kinases deposited in the PDB (Protein Data Bank containing deposited coordinates of solved protein structures). The alignments revealed that the insulin receptor tyrosine kinase domain (PDB: 1IR3-A) had the highest CLUSTAL-alignment score. Based on the insulin receptor and HGFR tyrosine kinase alignments, the baculovirus construct for the expression of human HGFR kinase domain (HGFRkd) was designed.

### 2. Cloning

The catalytic domain of the human HGFR kinase (HGFRkd) was cloned by PCR using Expand High Fidelity PCR System (Boehringer Mannheim) and Touchdown PCR (R.H. Don et al., *Nucleic Acids Research* 19: 4008 (1991)) from human liver and kidney Marathon-Ready cDNA (Clontech, Palo Alto, CA) with primers synthesized (Genset, La Jolla, CA) based on the published sequence [SEQ ID NO: 1]. The PCR oligonucleotide primer sequences are listed below in Table 2.

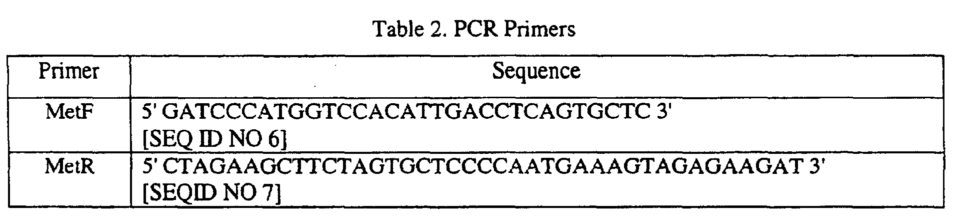

### 3. Expression and Purification of Recombinant HGFR Kinase

The cDNA corresponding to residues 1051-1349 of the HGFR kinase catalytic domain (HGFRkd) was cloned into a pFastBac plasmid (Life Technologies) modified by the introduction of an *Nco*I site. A recombinant baculovirus was generated using the Bacmid system (Life Technologies). The protein was expressed in SF-9 insect cells (Invitrogen, Carlsbad, CA) and purified by conventional column chromatography.

### 4. Generation of Expression Plasmids

Plasmid pFastBac-*Nco*I was modified from the pFastBac1 vector (Life Technologies, Gaithersburg, MD) by *in vitro* site-directed mutagenesis using the QuickChange™ site-directed mutagenesis method (Stratagene, La Jolla, CA). The nucleotide sequence in the vector AATATTCCG was mutated to ACCATGCCG to create a unique *Nco*I site at the original translation start site for the polyhedrin protein. The amplified cDNA fragment was ligated into TopoII PCR vector (Invitrogen) and the recombinant plasmid amplified. The cDNA fragment corresponding to the *Met* kinase domain was excised from the TopoII PCR vector with the restriction enzymes *Nco*I and *Hind*II and subcloned into *Nco*I and *Hind*III digested pFastBac-*Nco*I. Recombinant PfastBac-*Nco*I clones containing the *Met* kinase domain were sequence-verified.

### 5. Generation of Recombinant Virus

The Bac-to-Bac system (Life Technologies) was used to generate recombinant baculovirus for expression of the *Met* kinase. Recombinant virus was confirmed by PCR for the presence of the *Met* kinase encoding insert. SDS-PAGE or Western blot analysis with anti-*Met* polyclonal antibodies confirmed protein expression. 2-3 rounds of amplification in SF9 insect cells generated high titer stocks of recombinant virus.

### 6. Expression in Insect Cells

The titer of the virus stock was 1 to 5 x10⁸ p.f.u./ml. The viral titration was determined by the plaque assay method, with serial 10-fold dilution up to 10⁸-fold. The viral stock was used for large-scale protein production. Sf9 cells were grown in upright roller bottles up to a cell density of 2x10⁶ and subsequently used as seed cells for bioreactor culture. The cells were grown in a 20 L stirred bioreactor with working volume at 18L (Applikon Inc., Foster City, CA). Routinely, the MOI varied from 3-5 and the infection was carried out for 48 hours. After 48 hrs of infection, the infected cells were harvested by centrifugation at 3,000 rpm for 10 min at 4°C. Cell pellets were collected and stored at -80°C.

### 7. Purification of HGFR kinase catalytic domain (HGFRkd)

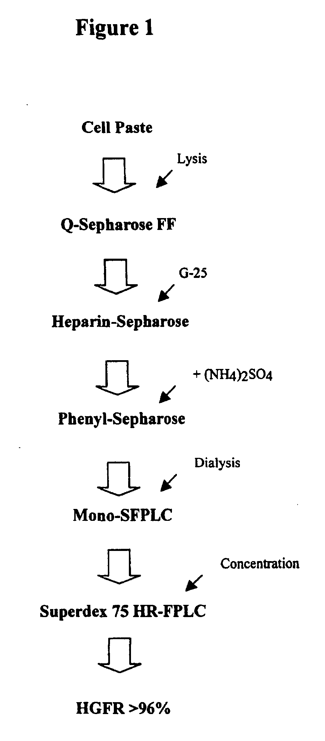

The basic purification scheme is depicted in Figure 1. Frozen cell pellets were thawed, suspended in ice-cold lysis buffer, and lysed by microfluidization (Microfluidics Corp, Newton, MA). All steps were performed at 4°C. A detailed protocol follows:
Buffer A: 50 mM Tris-Cl, pH 7.8, 150 mM NaCI, 5 mM DTT, 10% glycerol.
Buffer B: 50 mM MES pH 6.5, 150 mM NaCl, 3 mM DTT, 10% glycerol.
Buffer C: 50 mM MES pH 6.5, 0.6 M (NH₄)₂SO₄, 3 mM DTT.
Buffer D: 50 mM MES pH 6.5. 50 mM NaCl, 3 mM DTT.
Buffer E: 50 mM MES pH 6.5, 50 mM NaCl, 3 mM DTT, 10% glycerol.

Cell pellets (approx. 50 to 60 grams) were resuspended in 4 x volume of buffer A (50 mM Tris-Cl, pH 7.8, 150 mM NaCl, 5 mM DTT, 10% glycerol). 6 ml of PIC (Protease Inhibitor Cocktail, Sigma) was added to the resuspension and the cells were lysed by passing the solution through the high pressure microfluidizer (Microfluidics Corp, Newton, MA) once. The lysate was cleared at 40,000 rpm, 4°C for 45 min by ultracentrifugation (Beckman Optima LE-80K Ultracentrifuge). The cleared supernatant was loaded onto a Q-FF Sepharose column (Pharmacia XK50/20, 150 ml) equilibrated in buffer A at a rate of 10 ml/min.

The Q-FF Sepharose flow-through was loaded onto an equilibrated G-25 column (Pharmacia XK50/60, 1000 ml) in buffer B (50 mM MES pH 6.5, 150 mM NaCI, 10% glycerol, 3 mM DTT) at a flow rate of 20 ml/min. Combined flow-through fractions from the G-25 column were pooled and applied to an Heparin-Sepharose column (Pharmacia XK 26/20, 60 ml) equilibrated in buffer B. The pool from the G-25 column was loaded onto the Heparin-Sepharose column at 8 ml/min. The column was washed with 300 ml of buffer B and the protein eluted with a 600 ml salt linear gradient spanning from 150 mM NaCl to 600 mM NaCl at 4 ml/min. 8 ml fractions were collected and analyzed by SDS-PAGE. Fractions containing the recombinant *Met* kinase domain (~34,000 Dalton) were collected and pooled.

The pool from the Heparin-Sepharose column was adjusted to a final concentration of 0.6 M ammonium sulfate by the addition of 4 M ammonium sulfate to the slowly stirred solution at 4°C for 30 min. After addition of the ammonium sulfate, the solution was applied to a Phenyl-Sepharose column (Pharmacia XK 16/20, 20 ml) equilibrated in buffer C (50 mM MES pH 6.5, 0.6 M (NH₄)₂SO₄, 3 mM DTT) at a flow rate of 4 ml/min. The column was washed with 100 ml of buffer C and the protein eluted with a 400 ml gradient from 100% buffer C to 100% buffer D (50 mM MES pH 6.5. 50 mM NaCl, 3 mM DTT). The column was further washed with 100 ml of 100% buffer D. 6 ml fractions were collected and analyzed by SDS-PAGE prior to pooling fractions containing the recombinant *Met* kinase domain. The pooled fractions were dialyzed against 4 liters of buffer E (50 mM MES pH 6.5, 50 mM NaCl, 10% glycerol, 3 mM DTT) at 4°C overnight.

The dialyzed pool was filtered through a 0.2 µm filter prior to loading onto an equilibrated (buffer E) mono S column (Pharmacia, mono S HR 10/10, 7.8 ml) using the FPLC system (Pharmacia). The sample was loaded at 2 ml/min. The column was washed with 40 ml of buffer E and the protein eluted with a linear 160 ml salt gradient from 50 mM NaCI to 300 mM NaCl at 2 ml/min. 2 ml fractions were collected and analyzed by SDS-PAGE prior to pooling.

A Superdex 75 gel filtration column (Pharmacia, Superdex HiLoad 16/60, 120 ml) was equilibrated in buffer B. The pool from the mono S column was concentrated to less than 2 ml and the protein sample injected into the column using a FPLC unit. The column was developed with 120 ml of buffer B and 1.5 ml fractions were collected. The monomeric peak was collected and analyzed by SDS-PAGE. The protein at this stage is ∼98% homogenous. The monomeric fraction corresponding to purified *Met* kinase domain (HGFRkd) was concentrated to 6-7 mg/ml for crystallization trials or 5 mg/ml for high-throughput screening (HTS). Alternatively, protein was flash-frozen in liquid N₂ for long-term storage at -80°C.

### Example 2: Crystalization, Crystallography and Three-Dimensional Analysis

### 1. HGFRkd [SEQ ID NO. 4]-Compound 1 crystallization

Extensive screening of crystallization conditions was performed to produce crystals of HGFRkd. Successful crystallization of HGFRkd was performed by first incubating unphosphorylated HGFRkd with Compound **1** (4-[[(Z)-(6,7-dihydro-7-oxo-8H-pyrrolo[2,3-g]benzothiazol-8-ylidene)methyl]amino]-N-[2-(2-hydroxyethoxy)ethyl]-(9Cl).

Compound **1**, which is represented by the following formula, can be prepared in accordance with International Publication No. WO 99/15500, which is hereby incorporated by reference in its entirety:

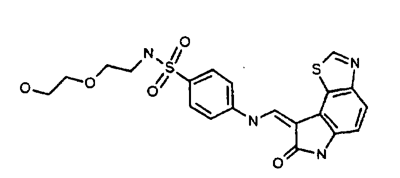

Compound **1** (using a stock solution of 70mM Compound **1** dissolved in dimethyl sulfoxide) was added in a 5:1 molar ratio of Compound **1** to HGFRkd to a solution containing: HGFRkd at a concentration of 6-7 mg/mL, 25mM 2-Morpholinoethanesulfonic acid monohydrate at pH 6.5, 150mM NaCl, and 2mM dithiothreitol. After **1** hour the solution was microfuged to remove any undissolved particles and the solution was mixed with an equal volume of precipitating solutions containing: 27-34% monomethylether polyethylene glycol (average molecular weight 350Da and 50 mM 4-(2-hydroxyethyl)piperazine-1-ethanesulfonic acid at pH 7.5). Using the hanging-drop diffusion method of protein crystallization (Matthews, B.W. (1968). *J. Mol. Biol.* 33, 491-497), 4 µL of the above solution was dispensed onto glass coverslips, inverted, and sealed over a reservoir containing 0.5 - 1.0 mL of the precipitating solution. Crystals of average dimensions 0.4 x 0.4 x 0.05mm grew over 3-5 days at a temperature of 23°C.

### 2. HGFRkd-Compound 1 X-ray diffraction data collection

Two X-ray diffraction data sets were generated and used to solve the three-dimensional structure of the HGFRkd-Compound **1** complex. A 1.9 Å resolution data set was collected using a Rigaku rotating anode X-ray generator (CuKα) and a MAR345 MAR Research image plate detector. A 1.22 Å resolution data set was collected at the Stanford Sunchrotron Radiation Light-source (SSRL) using beam-line 7.1. During both data collections the crystals were kept frozen in a stream of liquid nitrogen. The observed data sets were processed with versions of the programs DENZO and SCALEPACK (Otwinowski, Z. (1993). Oscillation data reduction program. In *Proceedings of the CCP4 Study Weekend: Data Collection and Processing.* (Sawyer, L., Isaacs, N., & Bailey, S., eds), pp. 56-62, SERC Daresbury Laboratory, England). Processing of the data revealed the crystals to belong to the P2(1) crystallographic space group with approximate unit cell constants of a = 37.8 Å, b = 41.6 Å, c = 88.0 Å, alpha = 90°, beta = 92.6°, and gamma=90.0°.

### 3. HGFRkd-Compound 1 three-dimensional structure determination

The approximate rotational and translational position of HGFRkd in the crystal lattice was solved using the program EPMR (Kissinger, C. and Gehlhaar, D (1999). EPMR: A program for crystallographic molecular replacement by evolutionary search. Agouron Pharmaceuticals, Inc., 11099 N. Torrey Pines Rd., La Jolla, CA 92037-1020), a model composed of parts of the structure of lymphocyte kinase (Yamaguchi, H. & Hendrickson, W. A. (1996). Structural basis for activation of human lymphocyte kinase Lck upon tyrosine phosphorylation. *Nature* 384, 484-489, Brookhaven Protein Data Bank entry 3lck) and the 1.9 Å HGFRkd-Compound 1 diffraction data set. The probe model was generated by removing residues of the non-conserved N-terminus, kinase activation loop, and kinase insert region of Lck. After the approximate position of HGFRkd was determined, the individual atomic positions of HGFRkd were fit using the 1.22 Å diffraction data set and the programs *wARP* (Perrakis, A., Morris, R., & Lamzin V. S. (1999) *Nature Struct. Biol.* 6, 496-500) and *SHELXL* (Sheldrick, G. M. & Schneider, T. R. (1997). *Methods Enzymol*. 277, 319-343) with manual fitting to electron density maps. The refined model has an R-factor of 0.1355 calculated using all of the data.

### 4. Description of the HGFRkd-Compound 1 Three-Dimensional (3-D) Structure

### Structural Overview

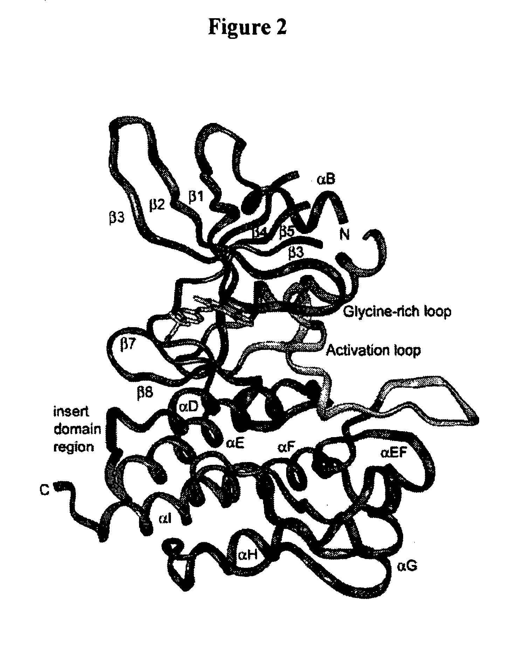

The overall structure of HGFRkd-Compound **1** is similar to that reported for other kinases, however HGFRkd contains unique features. As in other protein kinase structures, the protein is folded into two domains with ATP binding and phosphotransfer occurring in the cleft between the two domains (reviewed in Cox, S., Radzio-Andzelm, E. & Taylor, S. S. (1994). Domain movements in protein kinases. *Curr. Opin. Struct. Biol.* 4, 893-901). Figure 2 depicts the overall fold of HGFRkd with secondary structural elements assigned according to the convention originally given for cyclic AMP-dependent protein kinase (cAPK) (Knighton et al., *Science* 253:407-413 (1991)). The HGFRkd 3-D structural model extends over the range of residues leucine1062 through histidine 1348.

Of the published protein kinase structures, the overall structure of HGFRkd appears to follow most closely those of the insulin receptor kinase (IRK) (Hubbard et al., *Nature* 372:746-754 (1994); Hubbard et al., *Embo J.* 16:5572-5581 (1997)). Certain regions of HGFRkd share more similarity to IRK and other regions follow the phosphorylated IRK (pIRK) structure more closely. While regions of HGFRkd fold share similarity to the IRK and pIRK, the HGFRkd has unique structural elements. For simplicity, the structural differences will be described beginning at the N-terminus.

Residues 1062-1067 form a short helix that does not occur in IRK, IRKP, or in other reported receptor tyrosine kinase structures (McTigue et al., *Structure* 7, 319-330 (1999); Mohammadi et al., *Science* 276:955-960 (1997)). Residues 1079-1082 are an insertion in beta strand 1 relative to IRK and other kinases. This insertion results in a kink in beta strand 1. The position of the glycine-rich loop (residues 1085-1092) follows IRKP much more closely than IRK. The apex of the loop (phenylalanine 1089), however, has a very different conformation from IRKP. Relative to IRKP, Phe 1089 is inserted farther towards alpha helix C and thus affects the position of alpha helix C. The axis of alpha C is approximately 23° farther from the protein core than in IRKP and residues 1113- 1121 at the end of beta strand 3 and the beginning of alpha helix C are disordered (do not have a discreet position) in HGFRkd. The turn between beta strands 4 and 5 (residues 1149 -1152) is disordered in HGFRkd. This disorder, relative to other kinase structures, may be due to the insertion of an additional residue in this turn in HGFR. The kinase insert domain of HGFRkd (residues 1172-1178) folds differently than in other kinase structures. In HGFRkd the kinase insert domain is relatively short and forms an extended loop between alpha helix D and alpha helix E. Following the kinase insert domain the backbone of alpha helix E, the catalytic loop, and beta strands 7 and 8 superimpose reasonably with IRKP. The HGFRkd structure then diverges significantly from other kinase structures in the activation loop region (residues 1221-1244), discussed in more detail below. The rest of the HGFRkd backbone fold follows the same general path as IRKP until alpha helix I. In HGFRkd alpha helix I is oriented so that the C-terminal tail (residues 1342-1348) following this helix veer towards and packs against the kinase insert domain.

### Kinase activation loop conformation

The kinase activation loop (residues 1221-1244) in HGFRkd has a unique inhibitory conformation that prevents ATP binding, peptide substrate binding, affects the position of alpha helix C, and creates a unique binding site for inhibitors. At the beginning of the loop, residues 1223-1227 form a sharp turn or knob that juts into alpha helix C and appears to distort this helix at the point of contact, glycine 1128 (Figure 2). After glycine-1128 residues 1129-1134 are distorted from a normal alpha helix geometry into a 3₁₀ helical geometry. The insertion of residues 1223-1227 towards alpha helix C may also be responsible for pushing this helix away from the protein core and thus away from proper positioning for catalysis. The sidechain of aspartic acid 1228 points towards the glycine-rich loop and appears to force phenylalanine 1089 to adopt a position which would interfere with the correct positioning of alpha helix C for catalysis. The position of tyrosine 1230 occupies the triphosphate binding site of ATP and thus prevents proper ATP binding. The backbone of residues 1234-1238 adopts a position similar to that of the peptide substrate in the IRKP structure, and thus the position of these residues is inhibitory to peptide substrate binding.

### Inhibitor binding site

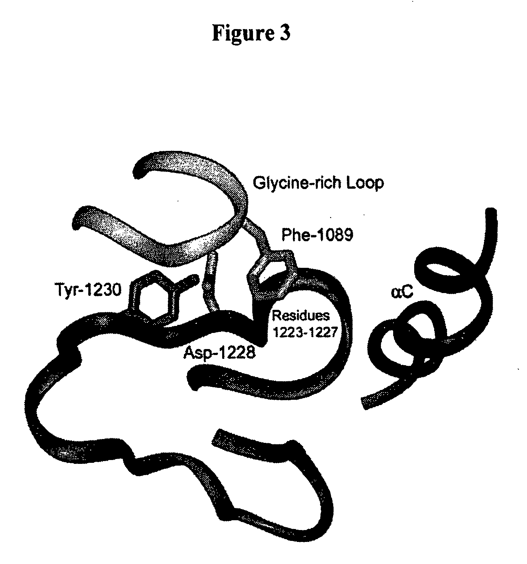

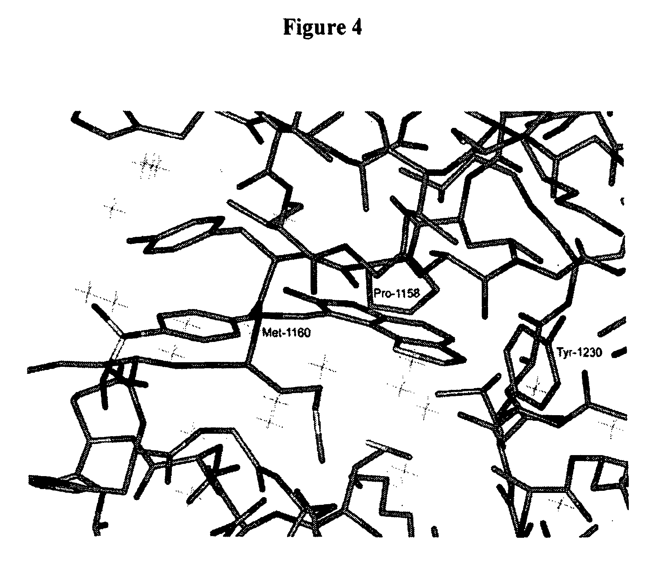

A depiction of the Compound **1** binding site is shown in Figures 3 and 4. The hydroxyethoxymethyl group of Compound **1** is not included in the 3-D structure as the electron density for this group is ambiguous, suggesting that this group adopts many different conformations. The binding site of Compound **1** roughly superimposes with the predicted binding site for the adenine group of ATP. The site is located in a cleft between parts of the N and C-terminal kinase domains (Figure 1). Residues of HGFRkd that form the site include: 1082-1086, 1091-1094, 1107-1110, 1140-1142, 1155-1175, 1208-1213, and 1219-1231. The oxopyrrolo of Compound **1** makes hydrogen bonds to the amino of methione 1160 and the carbonyl of proline 1158. These hydrogen bonds are analogous to those reported in structures of indolinones bound to the fibroblast growth factor receptor kinase (Mohammadi et al., *Science* 276:955-960 (1997)) and cyclin-dependent kinase 2 (Davis et al., *Science* 291:134-137 (2001)). The unique conformation of the kinase activation loop, particularly residues 1226-1231, contributes substantially to the uniqueness of the inhibitor site seen in this HGFRkd structure.

### Example 3: HGFR Biochemical Characterization

### 1. Materials

γ-[³²P]-ATP was purchased from Pharmacia-Amersham. GF/B glass fiber filterplates and Microscint-20 were purchased from Packard. The following reagents were purchased from the Sigma Chemical Company: poly(Glu₄Tyr), dephosphorylated α-casein, lactate dehydrogenase, pyruvate kinase, phosphoenolpyruvate, HEPES (N-[2-hydroxyethyl]piperazine-N'-[4-butanesulfonic acid]), MOPS (3-[N-morpholino]propanesulfonic acid), MES (2-[N-morpholino]ethanesulfonic acid), Bis-Tris Propane (BTP; 1,3-bis[tris(hydroxymethyl)methylamino]propane), NaCI, dithiothreitol, MgCl₂, ATP, and NADH. Met2 peptide (Ac-ARDMYDKEYYSVHNK [SEQ ID NO. 8]) was synthesized on a ABI 433A peptide synthesizer and purified to >95% purity by HPLC.

### 2. Enzymatic Assays

A coupled enzymatic assay format was used to measure both HGFR and phospho-HGFR (pHGFR) activities. The kinase-catalyzed production of ADP from ATP that accompanies phosphate transfer to the random copolymer poly(Glu₄Tyr) or peptide substrate was coupled to the oxidation of NADH through the activities of pyruvate kinase (PK) and lactate dehydrogenase (LDH). NADH conversion to NAD⁺ was monitored by the decrease in absorbance at 340 nm (ε = 6.22 cm⁻¹mM⁻¹) using a Beckman DU650 spectrophotometer. A typical reaction solution contained 1 mM phosphoenolpyruvate, 0.24 mM NADH, 40 mM MgCl₂, 5 mM DTT, poly(Glu₄Tyr), ATP, 15 units/mL PK, 15 units/mL LDH in 100 mM HEPES, pH 7.5. ATP was varied from 0.5 to 2000 µM and poly(Glu₄Tyr) was varied from 0.5 to 20 mg/mL. In addition, Met2 peptide (0-4 mM) could be used instead of poly(Glu₄Tyr). Assays were initiated with the addition of 12 nM phosphorylated HGFR or 125 nM unphosphorylated HGFR. Inhibition studies were performed as described above with following exceptions: 0.075 mM ATP (pHGFR) and 0.375 mM ATP (HGFR). Data was fit to the equation for competitive inhibition by the method of nonlinear least squares (KaleidaGraph).

A second assay format was used to monitor the production of the other kinase product: phospho-protein. This simple radioactive assay was used to monitor the transfer of phosphate from γ-[³²P]-ATP to dephosphorylated α-casein. The glass fiber filterplate assay format measures the capture of TCA-precipitated [³²P]-phosphorylated proteins on glass fiber filters in a 96-well format. The kinetic parameters for HGFR were evaluated in the filtermate assay format and were similar to the values determined in the coupled enzymatic format. Typical assay conditions for measuring inhibition were established: 27.5 nM pHGFR, 0.075 mM ATP, 0.0109 mM dephosphorylated α-casein, 0.5 µCi γ-[³²P]-ATP, 20 mM MgCl₂, 2 mM DTT, 100 mM HEPES pH 7.5, 0.10 mL, RT, and 30 min. These conditions deliver a signal/noise ratio greater than 20.

### 3. HGFR Autophosphorylation

Typical autophosphorylation reactions to produce maximally active pHGFR were performed at 4°C for 4 hours with the following components: 10 µM HGFR, 100 mM HEPES (pH 7.5), 20 mM MgCl₂, 4 mM ATP, and 2 mM DTT. At fixed time points in the autophosphorylation reaction, the extent of autophosphorylation was measured for enhancement of kinase activity: coupled enzymatic assay with 2 mM ATP, variable poly(Glu₄Tyr) (0, 0.38, 0.76, 1.5, 3, 6 mg/mL). The extent of autophosphorylation was also measured by isoelectric focussing gel electrophoresis (IEF) analysis (5 µg/ time point). Finally, mass spectrometry was used to identify the site of phosphate incorporation into HGFR.

### 4. Mass Spectrometry

### Proteolysis experiments.

Unphosphorylated and phosphorylated HGFR samples were digested by trypsin (100ng) overnight in a 100 mM ammonium bicarbonate/30% acetonitrile/3mM Tris-HCl buffer (pH 8) at 37°C. The tryptic peptides were extracted out of the gel using 50% acetonitrile/ 0.1% TFA, concentrated to 10 µl, and subjected to MALDI and nanoESI mass analysis.

### MALDI/MS analysis.

All MALDI-MS analyses were performed in a Voyager-Elite; time-of-flight mass spectrometer with delayed extraction (PerSeptive Biosystems, Inc., Framingham, MA). A volume of 1 µl of digested protein was placed directly on the MALDI analysis plate, mixed with 1 µl of matrix (α-cyano-4-hydroxy-cinnamic acid) in a saturated solution of acetonitrile/water (50:50, v/v) with 0.1% (w:w) trifluoroacetic acid (TFA) and inserted into the MALDI ionization source for analysis. Samples were irradiated with a nitrogen laser (Laser Science Inc.) operated at 337nm. The laser beam was attenuated by a neutral density filter onto the sample target. Ions produced by laser desorption were typically energetically stabilized during a delayed extraction period of 150 nanoseconds and were then accelerated through the time-of-flight mass analyzer with a 20 kV potential. Spectra shown were typically an average of 128 laser pulses.

### NanoESI-MS.

MS/MS analyses were performed on a triple quadrupole mass spectrometer (PE Sciex API III, Alberta, Canada) modified with a nanoESI source from Protana A/S, (Denmark). The ESI voltage was set at 850 V and the orifice settings were maintained at 100 V. A curtain gas of ultrapure nitrogen was pumped into the interface at a rate of 0.6 L/min to aid evaporation of solvent droplets and to prevent particulate matter from entering the analyzer. For precursor ion scan experiment, 3µl of digested protein was mixed with 3µL 25 mM ammonia solution and 3µL methanol (pH=9). For product ion scan experiment, 3 µL of digested protein was mixed with 7µl of methanol and 0.5 µL formic acid. A 4 µL aliquot was loaded into a palladium-coated borosilicate glass capillary and injected into mass spectrometer. Precursor ion scanning was used to generate spectra of its precursors (or "parent" ions) of the phosphate fragment *m*/*z* 79. Product ion scan was also used to obtain the sequence information of phosphopeptides.

### 5. Results

### Autophosphorylation of HGFR

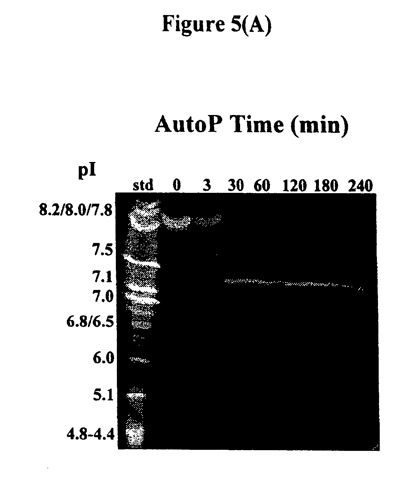

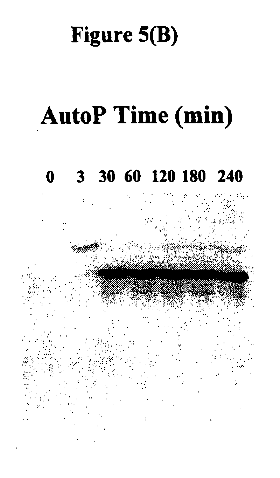

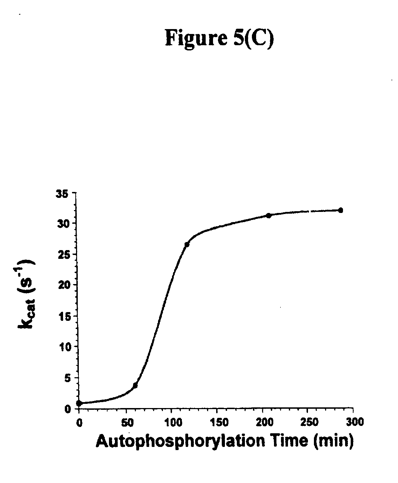

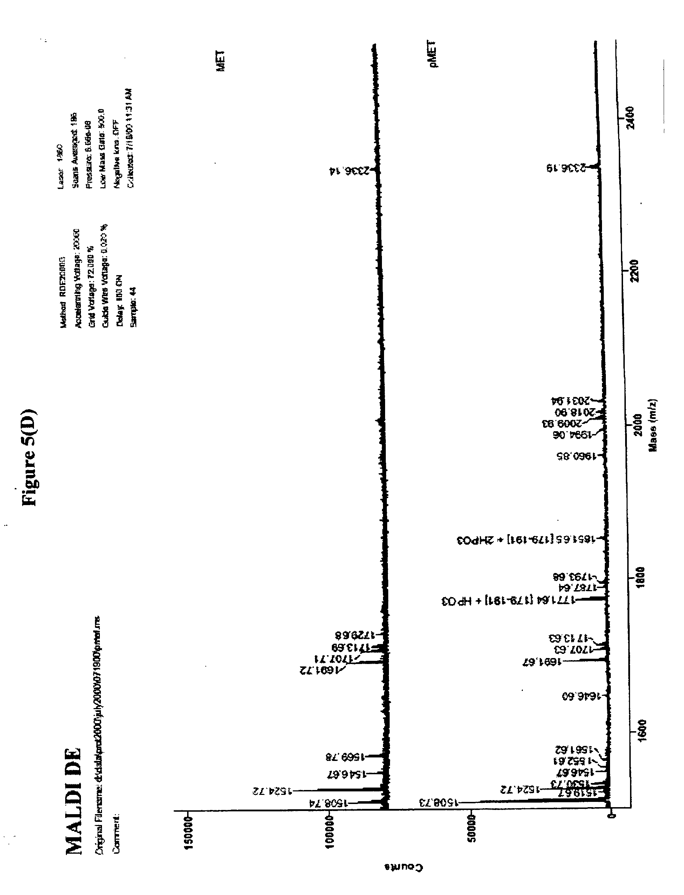

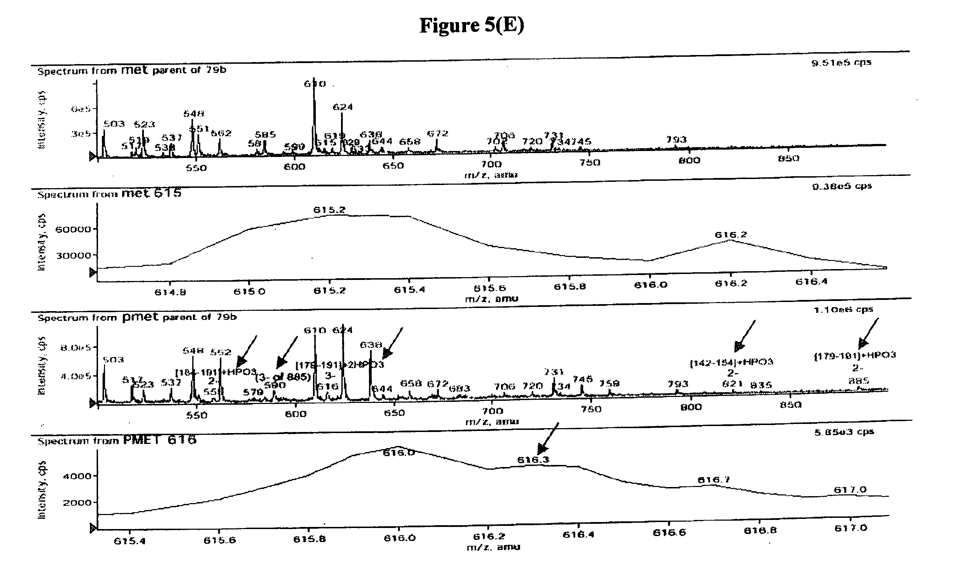

HGFR was activated through an autophosphorylation reaction to produce phospho-HGFR (pHGFR). To achieve high specific activity pHGFR, large-scale autophosphorylation reactions were performed at high HGFR concentration (typically 10 µM), high ATP concentration (4 mM), and low temperature (4°C). The extent of the reaction was monitored by native isoelectric focussing electrophoresis (IEF) with both Coomassie staining for protein (Figure 5(A)) and [³²P]-phosphate incorporation by autoradiography (Figure 5(B)) with small 0.05mL reactions at 20°C. In addition, quantitation of HGFR activity as a function of autophosphorylation time was performed to optimize the reaction in terms of catalytically relevant autophosphorylation. IEF analysis revealed that the HGFR kinase domain was a single species with pI = 7.9 (Figure 5(A)). Limited autophosphorylation shifted HGFR to a second species with pI = 6.6 (Figures 5(A) and 5(B)). Further autophosphorylation shifted HGFR to a third species (pI = 7.2). The enhancement in catalytic efficiency increased as a function of autophosphorylation time. A larger scale reaction (1 mg) was performed at 4°C to produce pHGFR for kinetic studies (Figure 5(C)). Although sequential phosphorylation events can be inferred by the IEF analysis, the precise assignment of these sites was not possible. As such, the determination of the phosphorylation sites and order of phosphate incorporation was investigated by mass spectrometric analysis.

### Determination of Phosphorylation Sites on HGFR.

A preliminary investigation into the HGFR autophosphorylation sites was performed with samples of HGFR and fully phosphorylated HGFR (pHGFR). The samples were proteolyzed with trypsin and subjected to MALDI-TOF mass spectrometry, the results of which are shown in Figure 5(D). One major phosphorylation site was identified. The major phosphopeptide corresponded to HGFR residues 1233-1240 (SYYSVHNK) which is contained in the HGFR activation loop. Parent ion scans using nano-spray EI mass spectrometry, shown in Figure 5(E), confirmed the identified sites. Tyrosine 1235 has been shown to be autophosphorylated (Ferracini, R., Longati, P., Naldini, L., Vigna, E., and Comoglio, P. M. (1991) *J. Biol. Chem 266,* 19558-19564).

### Kinetic Analysis of HGFR and pHGFR

The kinetic consequences of the autophosphorylation were investigated. HGFR and pHGFR activities were evaluated in two distinct and complementing assay formats. Autophosphorylation of HGFR was shown to occur on the activation loop and enhances the kinase specific activity. A coupled, enzymatic assay format (CE format) was used to measure ADP production. HGFR-catalyzed production of ADP from ATP that accompanied phosphate transfer to the random copolymer poly(Glu₄Tyr) was coupled to the oxidation of NADH through the sequential activities of pyruvate kinase (PK) and lactate dehydrogenase (LDH). NADH conversion to NAD⁺ was monitored spectrophotometrically by the decrease in absorbance at 340 nm (ε = 6.22 cm⁻¹mM⁻¹). A second assay format was used to measure the production of [³²P]-labeled phospho-protein (dephosphorylated α-casein). Dephosphorylated α-casein is the substrate for HGFR in the coupled enzymatic format and in a glass fiber filterplate assay format. The K_{m,Dephosphorylated α -casein} was determined to be 28.5 ± 3.5 µM in the coupled, enzymatic format. The filtermate assay format is as follows: kinase reactions run with γ-[³²P]-ATP, dephosphorylated α-casein phosphoacceptor, terminated with trichloroacetic acid, and isolated on glass fiber 96-well filterplates. Both assays produced similar Kₘ values for ATP: 73.7 ± 2.8 µM (coupled enzymatic format) and 78.6 ± 12.1 µM (radioactive filtermate format). By monitoring activity with the coupled enzymatic assay, autophosphorylation of HGFR caused a 32-fold enhancement in turnover number (k_{cat}) and 164-fold enhancement in the specificity constant (k_{cat}/Kₘ) (Table 3). Minor changes in substrate Kₘ values between HGFR and pHGFR were observed (Table 3). As determined in multiple assays with multiple phosphoacceptor substrates, autophosphorylation transformed HGFR into a much more potent catalyst (pHGFR).

### Inhibition of HGFR and pHGFR

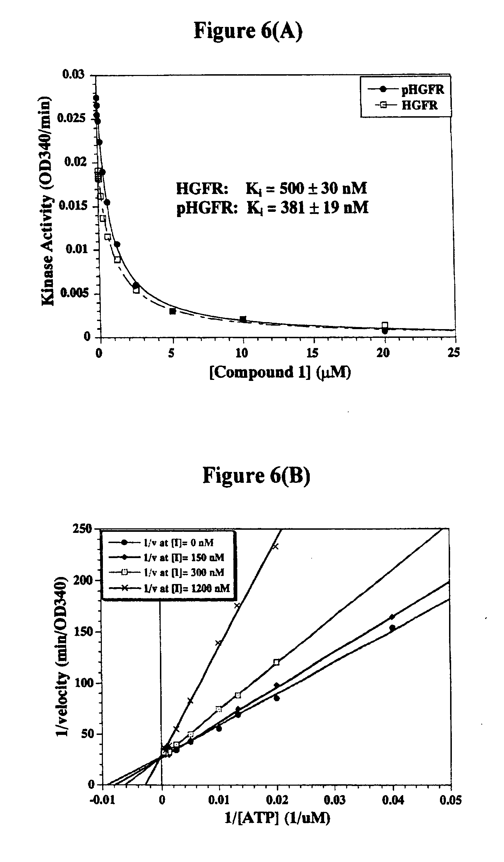

Inhibition of HGFR and pHGFR were monitored in two distinct assay formats to insure valid results. The coupled, enzymatic format (CE) was a continuous assay that measured ADP production through the sequential action of two standard coupling enzymes (pyruvate kinase and lactate dehydrogenase). The radioactive assay format was a discontinuous assay that measured phospho-protein production directly. As part of the assay validation, the inhibition of staurosporine was measured: 132 ± 6 nM (pHGFR/CE format), 97 ± 7 nM (pHGFR/radioactive format), and 260 ± 16 nM (HGFR/CE format). As expected, the general kinase inhibitor staurosporine was a potent HGFR and pHGFR inhibitor. Compound **1** was tested in both assay formats against both HGFR and pHGFR: 128 ± 17 nM (pHGFR/radioactive format), 381 ± 19 nM (pHGFR/CE format), and 500 ± 30 nM (HGFR/CE format). The inhibition of HGFR and pHGFR by Compound **1** as measured in the coupled enzymatic assay is shown in Figure 6(A). To determine the mechanism of pHGFR inhibition by Compound **1**, double reciprocal analysis was undertaken. In the CE assay, ATP (25, 50, 75, 100, 200, 400, 800, 1600, 3200 µM) and Compound **1** (0, 150, 300, 1200 nM) were varied at fixed concentrations of pHGFR (25 nM) and Met2 peptide (0.5 mM). The resulting family of lines had a common y-intercept (1/Vmax) and different slopes (Figure 6(B)). This result is consistent with Compound **1** being an ATP-competitive inhibitor of pHGFR. As measured in both formats, Compound **1** is a potent HGFR and pHGFR inhibitor.

### 6. Discussion

### Autophosphorylation Site

Receptor autophosphorylation is an essential event in many signal transduction pathways and is known to have multiple functions *in vivo* including activation of the kinase domain and creation of recruitment sites for downstream signaling molecules. For many RTKs multiple sites are phosphorylated and the order of phosphorylation of these sites can be either random or sequential. Receptor kinase domains are usually activated by phosphorylation of a tyrosine residue in the activation loop (Hubbard, S. R., and Till, J. H. (2000) *Annu Rev Biochem 69*, 373-98). Autophosphorylation of HGFR results in the enhancement in kinase activity concomitant with the phosphorylation of the activation loop. Furthermore, the identified activation loop sequence contains the reported HGFR autophosphorylation site: Y1235 (Ferracini, R., Longati, P., Naldini, L., Vigna, E., and Comoglio, P. M. (1991) *J. Biol. Chem 266,* 19558-19564). The insulin receptor has been shown to autophosphorylate in the activation loop on three tyrosine residues (White, M. F., Shoelson, S. E., Keutmann, H., and Kahn, C. R. (1988) *J Biol Chem 263,* 2969-80).

### Kinetic Analysis of HGFR and pHGFR

The autophosphorylation can be either cis (intramolecular) or trans (intermolecular). In the trans mechanism, the receptor dimerization increases the effective concentration of the kinase domains which enhances the reaction rate. The HGFR autophosphorylation rate is dependent on the concentration of HGFR. This observation is consistent with a trans autophosphorylation mechanism. Other RTK such as the insulin receptor (Hubbard, S. R., Wei, L., Ellis, L., and Hendrickson, W. A. (1994) *Nature 372*, 746-54; Hubbard, S. R. (1997) *Embo J 16*, 5572-81) and VEGF receptor (Parast, C. V., Mroczkowski, B., Pinko, C., Misialek, S., Khambatta, G., and Appelt, K. (1998) *Biochemistry 37,* 16788-801) have been shown to have a *trans* mechanism for autophosphorylation of the activation loop.

Kinetic analysis of unphosphorylated and autophosphorylated HGFR was undertaken to probe the overall effect of autophosphorylation on the effectiveness and efficiency of HGFR as a catalyst. As observed for other receptor tyrosine kinases, HGFR and pHGFR are robust kinases. The k_{cat} value for HGFR increases from 1.02 s⁻¹ to 31.3 s⁻¹ after autophosphorylation (30-fold enhancement). For example, the VEGFR2 receptor tyrosine kinase has high turnover numbers for both unphosphorylated (k_{cat} = 5 s⁻¹) and autophosphorylated (k_{cat} = 12 s⁻¹) forms. Though the primary effect of autophosphorylation resides on k_{cat}, there is a Kₘ effect. The substrate specificity constant (k_{cat}/Kₘ) for both HGFR (165-fold) and VEGFR2 (10-fold) is increased significantly as a result of autophosphorylation. Others have shown a 7-fold Vₘₐₓ enhancement of HGFR *(c-Met)* (Naldini, L., Vigna, E., Ferracini, R., Longati, P., Gandino, L., Prat, M., and Comoglio, P. M. (1991) *Mol Cell Biol 11*, 1793-803). The insulin receptor has a large 200-fold increase in specific activity as a result of autophosphorylation (Wei, L., Hubbard, S. R., Hendrickson, W. A., and Ellis, L. (1995) *J Biol Chem 270,* 8122-30). It should be noted that the tyrosine residue in the activation loop of the EGF receptor has not been shown to contribute to receptor activation (Gotoh, N., Tojo, A., Hino, M., Yazaki, Y., and Shibuya, M. (1992) *Biochem* *Biophys Res Commun 186*, 768-74). HGFR is a potent kinase that undergoes a large enhancement in activity due to autophosphorylation.

**Table 3**

| | Unphosphorylated HGFR | | | Phospho-HGFR | | |
|---|---|---|---|---|---|---|
| | Kₘ (µM) | k_{cat} (s⁻¹) | k_{cat}/Kₘ (s⁻¹M⁻¹) | Kₘ (µM) | k_{cat} (s⁻¹) | K_{cat}/Kₘ (s⁻¹M⁻¹) |
| MgATP | 373 ± 61 | 1.02 ± 0.013 | 2730 | 73.7 ± 2.8 | 31.3 ± 2.1 | 449,000 |
| Poly(Glu₄Tyr) | 10080 ± 260 | | 48.1 | 8194 ± 651 | | 9,260 |

As seen in the data of Table 3, autophosphorylation of HGFR enhances its catalytic efficiency. The observed enhancement was independent of phosphoacceptor and assay format as measured by steady-state kinetic constants for HGFR and autophosphorylated HGFR. The coupled enzymatic analysis of HGFR processing of ATP and poly(Glu₄Tyr) were measured at 37°C. Variable substrate concentration was measured at a fixed saturating concentration of the other substrate.

### Example 4. Screening-Biochemical and Cell Based Assays: DELIFA HTS (DELIFA High Throughput Screen)

This is a 384-well plate DELFIA assay, a time-resolved fluorescence (TRF) assay for the HGFR kinase. In this assay, the kinase is assayed at room temperature on a Neutravidin-coated white 384-well plate using biotin-gastrin as the peptide substrate in the presence of ATP and with or without inhibitors. The reaction is stopped; the plate is washed and then incubated with europium (Eu) labeled antiphosphotyrosine antibody (anti-PY-Eu Ab), which binds to the phosphotyrosine product. The plate is washed and then incubated with an enhancement solution followed by plate read in a fluorescence plate reader under Eu time-resolved settings to quantify the amount of anti-PY-Eu bound. The Eu counts are directly proportional to the HGFR kinase activity.

### 1. Assay Protocol

### Materials and Reagent

- 384-well flat bottom NeutrAvidin coated microtitre plates (Pierce)
- Substrate: biotin-Gastrin(American Peptide Co.). Stock 10 mM made in DMSO (not very soluble in water)
- HGFR kinase (autophosphorylated, stored at -80°C)
- Anti-PY-Eu antibody (Wallac)
- 1X Enhancement solution (Wallac)
- TBS/Tween-20 (0.05%)
- Antibody diluent: TBS / Tween-20 + 10 mg/ml BSA (Fraction-V, ELISA grade Calbiochem, Cat # 126593)
- ATP (Sigma); Tris (Sigma); MgCl₂ (Fisher); DTT (Calbiochem); NaCl (Fisher); DMSO (Fisher)
- Victor plate reader

### Stock Solutions (in distilled water, unless otherwise stated)

1.0 M HEPES (pH 8); 1.0 M MgCl₂; 2.0 M NaCl; 1.0 M DTT; 0.5 M ATP; 10 mM biotin-Gastrin; 10X TBS/Tween-20; 10X Ab diluent; 50X inhibitor controls in DMSO.

| Reaction Mixture | Assay Buffer |
|---|---|
| 100 mM HEPES | 100 mM HEPES |
| 2 mM DTT | 2 mM DTT |
| 20 mM MgCl₂ | 20 mM MgCl₂ |
| 25 mM NaCI | |
| 100 µg/ml BSA | |

Inhibitor controls: Starosporin at 10 µM and 1 µM.

### 2. Assay Plate Format and Screening Conditions

| | |
|---|---|
| Assay Volume | 50 µl |
| pH | 7.5 |
| HGFR kinase | 0.75 nM |
| Biotin-gastrin | 1 µM |
| ATP | 75 µM |
| Inhibitor controls | Starosporin at 2 µM, 0.5 µM, and 0.4 µM |
| Compounds | 10 µM |
| Reaction time | 11 min |

### 3. 96-Well Plate Set-Up (same set up for each quadrant of a 384-well plate)

a) Columns 1-11, test compounds
b) Column 12, controls: wells A12, B12, C12 are DMSO controls; well D12, E12, F12 inhibitor controls at 2 µM, 1 µM, and 0.4 µM respectively; wells G12 and H12 are without ATP and serve as background controls.

### 4. Screening Protocol

- Wash plate 1 time with TBS Tween-20 buffer with 1 min soak time.
- Dispense 39 µl of assay buffer
- Add 5 µl of 10X biotin-Gastrin mixture to all wells,
- Add 1 µl of test compound
- Add 5 µl 10 HGFR (with/without inhibitor control in column 12)
- Incubate at room temperature for 11 minutes
- Stop reaction by washing plate 3 times with TBS Tween-20
- Add 50 µl of 1:5200 Anti-PY-Eu antibody in Ab diluent and incubate at room temperature for 35 hr.
- Wash plate 3 times with TBS Tween-20
- Add 50 µl enhancement solution and incubate at room temperature for 1 hr.
- Read plate at Ex: 340 nm and Em:615 nm in time-resolved fluorescence (TRF) mode.

### 5. Data Analysis

Percent inhibition for each well is calculated using the wells A12, B12 and C12 as enzyme controls and wells G12 and H12 as background controls.
While the invention has been described in terms of various preferred embodiments and specific examples, the invention should be understood as not being limited by the foregoing detailed description, but as being defined by the appended claims and their equivalents. Accordingly, those skilled in the art will recognize that various changes and modifications can be made without departing from the spirit and scope of the invention. Thus, the invention should be understood as not being limited by the foregoing detailed description, but as being defined by the appended claims and their equivalents.
All U.S. and foreign patents, published patent applications, and other references cited herein are hereby incorporated by reference in their entireties.

## Claims

1. An isolated polynucleotide which encodes the human hepatocyte growth factor receptor or the human hepatocyte gowth factor receptor kinase domain, or a fragment or variant thereof.

2. An isolated polynucleotide according to claim 1, wherein the nucleotide sequence of said polynucleotide corresponds to at least bases 3342 to 4206 of SEQ ID NO: 1.

3. An isolated polynucleotide according to claim 1, wherein the nucleotide sequence of said polynucleotide corresponds to the sequence of SEQ ID NO: 10.

4. An isolated polynucleotide according to claim 1, wherein the nucleotide sequence of said polynucleotide corresponds to the sequence of SEQ ID NO: 11.

5. An isolated polynucleotide according to claim 1, wherein the nucleotide sequence of said polynucleotide corresponds to the sequence of SEQ ID NO: 12.

6. An isolated polynucleotide according to claim 1, wherein the nucleotide sequence of said polynucleotide corresponds to the sequence of SEQ ID NO: 14.

7. A crystal structure comprising the human hepatocyte growth factor receptor kinase.

8. A crystal structure according to claim 7 wherein the amino acid sequence of said kinase corresponds to at least amino acids 1051 to 1348 of SEQ ID NO: 2.

9. A crystal structure according to claim 7 wherein the amino acid sequence of said kinase corresponds to the sequence of SEQ ID NO: 3.

10. A crystal structure according to claim 7 wherein the amino acid sequence of said kinase corresponds to the sequence of SEQ ID NO: 4.

11. A crystal structure according to claim 7 wherein the amino acid sequence of said kinase corresponds to the sequence of SEQ ID NO: 5.

12. A crystal structure according to claim 7 wherein the amino acid sequence of said kinase comprises the sequence of SEQ ID NO: 6.

13. A crystal structure according to claim 7 wherein the amino acid sequence of said kinase comprises the sequence of SEQ ID NO: 7.

14. A crystal structure according to claim 7 wherein the amino acid sequence of said kinase comprises the sequence of SEQ ID NO: 8.

15. A crystal structure according to claim 7 wherein the amino acid sequence of said kinase comprises the sequence of SEQ ID NO: 9.

16. A crystal structure according to claim 7 wherein the amino acid sequence of said kinase comprises the sequence of SEQ ID NO: 13.

17. A crystal structure according to claim 7 wherein the amino acid sequence of said kinase comprises the sequence of SEQ ID NO: 15.

18. A crystal structure according to claim 7 wherein the amino acid sequence of said kinase comprises the sequence of SEQ ID NO: 16.

19. An isolated polypeptide comprising the human hepatocyte growth factor receptor or human hepatocyte growth factor receptor kinase domain, or a variant thereof.

20. A polypeptide according to claim 19 wherein said human hepatocyte growth factor receptor or human hepatocyte growth factor receptor kinase domain comprises a deletion that imparts favorable physical characteristics to the resulting polypeptide.

21. A polypeptide according to claim 19 wherein said polypeptide comprises amino acids 1051 to 1341 of the sequence as set forth in SEQ ID NO. 2 or a conservatively substituted variant thereof.

22. A polypeptide according to claim 19 wherein said polypeptide comprises amino acids 1051 to 1348 of the sequence as set forth in SEQ ID NO. 2 or a conservatively substituted variant thereof.

23. A polypeptide according to claim 19 wherein said polypeptide comprises the amino acid sequence as set forth in SEQ ID NO. 3 or a conservatively substituted variant thereof.

24. A polypeptide according to claim 19 wherein said polypeptide comprises the amino acid sequence as set forth in SEQ ID NO. 4 or a conservatively substituted variant thereof.

25. A polypeptide according to claim 19 wherein said polypeptide comprises the amino acid sequence as set forth in SEQ ID NO. 5 or a conservatively substituted variant thereof.

26. A polypeptide according to claim 19 wherein said polypeptide comprises the amino acid sequence as set forth in SEQ ID NO. 6 or a conservatively substituted variant thereof.

27. A polypeptide according to claim 19 wherein said polypeptide comprises the amino acid sequence as set forth in SEQ ID NO. 7 or a conservatively substituted variant thereof.

28. A polypeptide according to claim 19 wherein said polypeptide comprises the amino acid sequence as set forth in SEQ ID NO. 8 or a conservatively substituted variant thereof.

29. A polypeptide according to claim 19 wherein said polypeptide comprises the amino acid sequence as set forth in SEQ ID NO. 9 or a conservatively substituted variant thereof.

30. A polypeptide according to claim 19 wherein said polypeptide comprises the amino acid sequence as set forth in SEQ ID NO. 13 or a conservatively substituted variant thereof.

31. A polypeptide according to claim 19 wherein said polypeptide comprises the amino acid sequence as set forth in SEQ ID NO. 15 or a conservatively substituted variant thereof.

32. A polypeptide according to claim 19 wherein said polypeptide comprises the amino acid sequence as set forth in SEQ ID NO. 16 or a conservatively substituted variant thereof.

33. An isolated polynucleotide which encodes the catalytically active form of the human hepatocyte growth factor receptor or human hepatocyte growth factor receptor kinase domain, or a fragment or variant thereof.

34. An isolated catalytically active polypeptide comprising the human hepatocyte growth factor receptor or human hepatocyte growth factor receptor kinase domain, or a variant thereof.

35. An isolated polynucleotide which encodes the catalytic domain of the human hepatocyte growth factor receptor kinase, or a fragment or variant thereof.

36. An isolated catalytically active polypeptide comprising the catalytic domain of the human hepatocyte growth factor receptor kinase or a variant thereof.

37. An isolated soluble polypeptide comprising the catalytic domain of the human hepatocyte growth factor receptor kinase or a variant thereof.

38. An expression vector for producing the human hepatocyte growth factor receptor kinase in a host cell, which vector comprises: a polynucleotide encoding the human hepatocyte growth factor receptor kinase or a variant thereof; and regulatory sequences functional in said host cell operably linked to said polynucleotide.

39. A vector according to claim 38 wherein said polynucleotide encodes the active human hepatocyte growth factor receptor kinase, said active kinase comprising bases 3342 to 4206 of SEQ ID NO: 1.

40. A vector according to claim 38 wherein said vector is selected from the group consisting of pET28a, pAcSG2, and pFastBac.

41. A vector according to claim 38 wherein said vector is pFastBac-NcoI.

42. A vector according to claim 38 wherein said host cell is *E*. *coli*.

43. A host cell transformed or transfected with a polynucleotide encoding the human hepatocyte growth factor receptor kinase or a variant thereof.

44. A host cell according to claim 43 wherein said host cell is transformed or transfected with said polynucleotide via an expression vector comprising said polynucleotide; a regulatory sequence functional in said host cell operably linked to said polynucleotide; and a selectable marker.

45. A host cell according to claim 44 wherein said expression vector is selected from the group consisting of: pET28a, pAcSG2, and pFastBac.

46. A host cell according to claim 44 wherein said expression vector is pFastBac-*Nco*I.

47. A host cell according to claim 43 wherein said polynucleotide encodes the human hepatocyte growth factor receptor kinase, said kinase comprising bases 3342 to 4206 of SEQ ID NO: 1.

48. A host cell according to claim 43 wherein said host is *E. coli.*

49. A host cell according to claim 43 wherein said host is infected with a recombinant baculovirus.

50. A host cell according to claim 43 wherein said host is an insect cell.

51. A host cell according to claim 50 wherein said insect cell is Sf9.

52. A method of producing a polypeptide or variant thereof comprising culturing the host cell of claim 43 under conditions such that said polypeptide or variant thereof is expressed, and recovering said polypeptide or variant thereof.

53. A method for assaying a candidate compound for its ability to interact with the human hepatocyte growth factor receptor comprising:
(a) expressing an isolated DNA sequence or variant thereof encoding at least the kinase domain of said human hepatocyte growth factor receptor in a host capable of producing said kinase, said kinase being in a form which may be assayed for interaction of said kinase with said candidate compound;
(b) exposing said kinase to said candidate compound; and
(c) evaluating the interaction of said kinase with said candidate compound.

54. A method according to claim 53 wherein said evaluation step comprises:
(a) crystallizing said kinase in a condition suitable for x-ray crystallography; and
(b) conducting x-ray crystallography on said kinase.

55. A method according to claim 53 wherein the results of said x-ray crystallography step (b) are used to determine the three dimensional molecular structure of the configuration of human hepatocyte growth factor receptor kinase and the binding pockets thereof.

56. A crystal structure comprising a polypeptide encoded by a polynucleotide which encodes at least the human hepatocyte growth factor receptor kinase domain, or a fragment or variant thereof.

57. A crystal structure comprising a polypeptide encoded by a polynucleotide which encodes at least the human hepatocyte growth factor receptor kinase domain, or a fragment or variant thereof, and a ligand complexed thereto.

58. A crystal structure according to claim 57 wherein said ligand modulates the activity of human hepatocyte growth factor kinase.

59. A crystal structure according to claim 58 wherein said ligand is a compound of the formula:

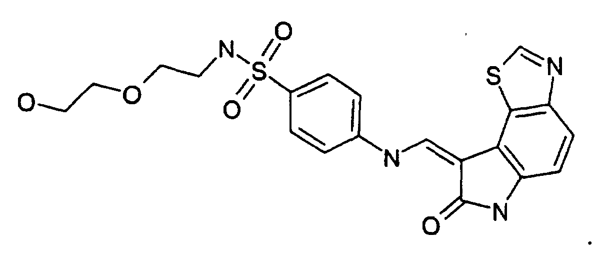

60. A process of drug design for compounds which interact with the human hepatocyte growth factor receptor kinase comprising:
(a) crystallizing said human hepatocyte growth factor receptor kinase;
(b) resolving the x-ray crystallography of said kinase;
(c) applying the data generated from resolving the x-ray crystallography of said kinase to a computer algorithm which generates a model of said kinase suitable for use in designing molecules that will act as agonists or antagonists to said polypeptide; and
(d) applying an interative process whereby various molecular structures are applied to said computer-generated model to identify potential agonists or antagonists of said kinase.

61. A process according to claim 60 wherein said process is utilized to identify modulators of said active kinase, said modulators serving as lead compounds for the design of potentially therapeutic compounds for the treatment of diseases or disorders associated with the hepatocyte growth factor receptor- hepatocyte growth factor signaling pathway.

62. A method of rapidly screening large compound libraries to identify compounds that inhibit human hepatocyte growth factor receptor kinase comprising a non-radioactive immunosorbent assay capable of robotic control.

63. A method according to claim 62 wherein said assay is DELFIA.

64. A method of assessing compounds which are agonists or antagonists of the activity of the hepatocyte growth factor receptor kinase comprising:
(a) crystallizing said hepatocyte growth factor receptor kinase;
(b) obtaining crystallography coordinates for said crystallized hepatocyte growth factor receptor kinase;
(c) applying said crystallography coordinates for said hepatocyte growth factor receptor kinase to a computer algorithm such that said algorithm generates a model of said hepatocyte growth factor receptor kinase, said model suitable for use in designing molecules that will act as agonists or antagonists to said kinase; and
(d) applying an iterative process whereby various molecular structures are applied to said computer-generated model to identify potential agonists or antagonists to said kinase.

65. A method according to claim 64 further comprising the steps of:
(c) synthesizing or obtaining said agonist or antagonist; and
(d) contacting said agonist or antagonist with said molecule to determine the ability of said potential agonist or antagonist to interact with said molecule.

66. A method according to claim 64, wherein the crystallography coordinates comprise coordinates of hepatocyte growth factor receptor kinase amino acids 1082-1086, 1091-1094, 1107-1110, 1140-1142, 1155-1175, 1208-1213, and 1219-1231 which are within about a root mean square deviation of not more than about 1.5 Å from the backbone atoms of said amino acids according to Table 1.

67. A method according to claim 64, wherein the crystallography coordinates comprise coordinates of all the amino acids of hepatocyte growth factor receptor kinase which are within about a root mean square deviation of not more than about 1.5 Å from the backbone atoms of said amino acids according to Table 1.

68. A method for determining the three-dimensional structure of a complex of hepatocyte growth factor receptor kinase with a ligand thereof, comprising:
(a) obtaining x-ray diffraction data for crystals of the complex, and
(b) utilizing a set of atomic coordinates of Table 1 or portions thereof; and coordinates having a root mean square deviation therefrom with respect to conserved protein backbone atoms of not more than about 1.5 Å to define the three-dimensional structure of the complex.

69. A method of using a three-dimensional structure of a polypeptide encoded by a polynucleotide which encodes the human hepatocyte growth factor receptor and a compound of the formula: 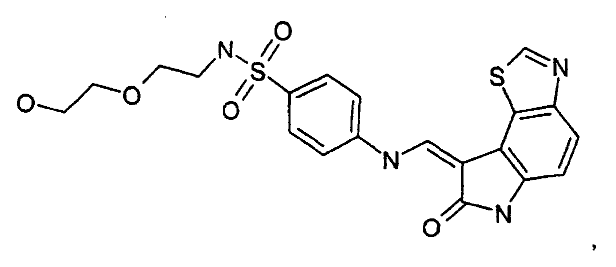 as defined by the structure coordinates of Table 1, or a portion thereof, in a drug-discovery strategy comprising:
(a) selecting a potential drug by performing rational drug design with the three-dimensional structure determined from one or more sets of atomic coordinates in Table 1, wherein said selecting is performed in conjunction with computer modeling;
(b) contacting the potential drug with a polypeptide containing a functional human hepatocyte growth factor receptor; and
(c) determining the binding of the potential drug with said polypeptide.

70. A method of using a three-dimensional structure of a polypeptide encoded by a polynucleotide which encodes the human hepatocyte growth factor receptor kinase domain and a compound of the formula: 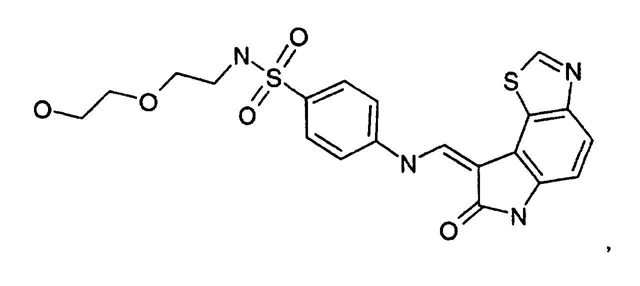 as defined by the structure coordinates of Table 1, or a portion thereof, in a drug-discovery strategy comprising:
(a) selecting a potential drug by performing rational drug design with the three-dimensional structure determined from one or more sets of atomic coordinates in Table 1, wherein said selecting is performed in conjunction with computer modeling;
(b) contacting the potential drug with a polypeptide containing a functional human hepatocyte growth factor receptor; and
(c) determining if the potential drug modulates the activity of the polypeptide.

71. A method for evaluating the potential of a chemical entity to associate with:
(a) a molecule or molecular complex comprising a binding pocket defined by structure coordinates of human hepatocyte growth factor receptor amino acids 1082-1086, 1091-1094, 1107-1110, 1140-1142, 1155-1175, 1208-1213, and 1219-1231, according to Table 1, or
(b) a homologue of said molecule or molecular complex, wherein said homologue comprises a binding pocket that has a root mean square deviation from the backbone atoms of said amino acids of not more than about 1.5 Å comprising the steps of:
(i) employing computational means to perform a fitting operation between the chemical entity and a binding pocket defined by structure coordinates of hepatocyte growth factor receptor amino acids 1082-1086, 1091-1094, 1107-1110, 1140-1142, 1155-1175, 1208-1213, and 1219-1231 which are within about a root mean square deviation of not more than about 1.5 Å from the backbone atoms of said amino acids according to Table 1; and
(ii) analyzing the results of said fitting operation to quantify the association between the chemical entity and the binding pocket.

72. A method according to claim 71, wherein said method evaluates the potential of a chemical entity to associate with a molecule or molecular complex:
(a) defined by structure coordinates of all of the hepatocyte growth factor receptor amino acids, as set forth in Table 1, or
(b) a homologue of said molecule or molecular complex having a root mean square deviation from the backbone atoms of said amino acids of not more than about 1.5 Å

73. A computer for producing a three-dimensional representation of:
(a) a molecule or molecular complex, wherein said molecule or molecular complex comprises a binding pocket defined by the structure coordinates of hepatocyte growth factor receptor kinase amino acids 1082-1086, 1091-1094, 1107-1110, 1140-1142, 1155-1175, 1208-1213, and 1219-1231, according to Table 1; or
(b) a homologue of said molecule or molecular complex, wherein said homologue comprises a binding pocket that has a root mean square deviation from the backbone atoms of said amino acids of not more than about 1.5 Å,
wherein said computer comprises:
(i) a computer-readable data storage medium comprising a data storage material encoded with computer-readable data, wherein said data comprises the structure coordinates of hepatocyte growth factor kinase amino acids 1082-1086, 1091-1094, 1107-1110, 1140-1142, 1155-1175, 1208-1213, and 1219-1231, according to Table 1;
(ii) a working memory for storing instructions for processing said computer-readable data;
(iii) a central-processing unit coupled to said working memory and to said computer-readable data storage medium for processing said computer-machine readable data into said three-dimensional representation; and
(iv) a display coupled to said central-processing unit for displaying said three-dimensional representation.

74. A computer according to claim 73, wherein said computer produces a three-dimensional representation of:
(a) a molecule or molecular complex defined by structure coordinates of all of the hepatocyte growth factor kinase amino acids set forth in Table 1, or
(b) a homologue of said molecule or molecular complex, wherein said homologue comprises a binding pocket that has a root mean square deviation from the backbone atoms of said amino acids of not more than 1.5 Å; and
wherein said computer readable data contains the coordinates of all of the hepatocyte growth factor kinase amino acids set forth in Table 1.

75. A computer for determining at least a portion of the structure coordinates corresponding to the x-ray diffraction data obtained from a molecule or molecular complex, wherein said computer comprises:
(a) a computer-readable data storage medium comprising a data storage material encoded with machine-readable data, wherein said data comprises at least a portion of the structural coordinates of hepatocyte growth factor receptor kinase according to Table 1;
(b) a computer-readable data storage medium comprising a data storage material encoded with computer-readable data, wherein said data comprises x-ray diffraction data obtained from said molecule or molecular complex;
(c) a working memory for storing instructions for processing said computer-readable data of
(a) and (b);
(d) a central-processing unit coupled to said working memory and to said computer-readable data storage medium of (a) and (b) for performing a Fourier transform of the machine readable data of (a) and for processing said computer-readable data of (b) into structure coordinates; and
(e) a display coupled to said central-processing unit for displaying said structure coordinates of said molecule or molecular complex.

76. A computer readable medium having stored thereon data of the structure coordinates of a *Met* ligand-binding site comprising 1082-1086, 1091-1094, 1107-1110, 1140-1142, 1155-1175, 1208-1213, and 1219-1231 according to Table 1.
